# EUROPEAN PATENT APPLICATION

(11) **EP 1 178 052 A2**
(43) Date of publication of application: **06.02.2002**
(21) Application number: 01124215.3
(22) Date of filing: 27.01.2000
(51) Int. Cl.: C07K 14/245, C12N 15/10, C12N 15/11, C12N 15/31

(54) **Genes identified as required for proliferation in Escherichia coli**

(30) Priority: 27.01.1999 US 117405 P
(62) Divisional of application: 00914458.5
(71) Applicant: Elitra Pharmaceuticals, Inc., San Diego, CA 92121 (US)
(72) Inventor: The designation of the inventor has not yet been filed
(74) Representative: VOSSIUS & PARTNER

(57) **Abstract**

The sequences of nucleic acids encoding proteins required for *E.coli* proliferation are disclosed. The nucleic acids can be used to express proteins or portions thereof, to obtain antibodies capable of specifically binding to the expressed proteins, and to use those expressed proteins as a screen to isolate candidate molecules for rational drug discovery programs. The nucleic acids can also be used to screen for homologous genes that are required for proliferation in microorganisms other than *E. coli*. The nucleic acids can also be used to design expression vectors and secretion vectors. The nucleic acids of the present invention can also be used in various assay systems to screen for proliferation required genes in other organisms as well as to screen for antimicrobial agents.

SEQUENCE LISTING

<110> ELITRA PHARMACEUTICALS, INC.
Zyskind, Judith
Ohlsen, Kari L.
Trawick, John
Forsyth, R. Allyn
Froelich, Jamie M.
Carr, Grant J.
Yamamoto, Robert T.
Xu, H. Howard

<120> GENES IDENTIFIED AS REQUIRED FOR PROLIFERATION IN
ESCHERICHIA COLI

<130> ELITRA.001VPC

<160> 485

<170> FastSEQ for Windows Version 3.0

<210> 1
<211> 159
<212> DNA
<213> E. Coli

<400> 1
caggtggtat ggaaacccaa aatggagacg ggaagctgaa ccagatagtt actggaggtg 60
atcaccagca gatgaaataa cgataaccag aacaacgcct tatagcgttg agtttgcgag 120
aaaacgttca tattgtacct ttttgattaa ccattgggg 159

<210> 2
<211> 696
<212> DNA
<213> E. Coli

<220>
<221> misc_feature
<222> (1)...(696)
<223> n = A,T,C or G

<400> 2
gattacatca agcgcgcggt gggtttaccg ggcgataaag tcacttacga tccggtctca 60
aaagagctga cgattcaacc gggatgcagt tccggccagg cgtgtgaaaa cgcgctgccg 120
gtcacctact caaacgtgga accgagcgat ttcgttcaga ccttctcacg ccgtaatggt 180
ggggaagcga ccagcggatt ctttgaagtg ccgaaaaacg aaaccaaaga aaatggaatt 240
cgtctttccg agcgtaaaga gacactgggt gatgtgacgc accgcattct gacagtgccg 300
attgcgcagg atcaggtggg gatgtattac cagcagccag ggcaacaact ggcaacctgg 360
attgttcctc cgggacaata cttcatgatg ggcgacaacc gcgacaacag cgcggacagc 420
cgttactggg gctttgtgcc ngaagcgaat ctggtcggtc nggcaacggc tatctggatg 480
aacttcgata accaagaagg cgaatggccg aatggtctgc cctaantcgc attggcgnnt 540
ccnttaatan ccacttcctt cnctttgtcc ccttatggca acacttaatt tattntaaan 600
taatcncccg tggctnacaa atccccgcct tttnttaaaa atttccccna anttaaggtt 660
ggcctccagt tgcccgnccc aaacactttg gncccc 696

<210> 3
<211> 681
<212> DNA
<213> E. Coli

<220>
<221> misc_feature
<222> (1)...(681)
<223> n = A,T,C or G

<400> 3
ctgcagggta atgtcgccat taaactggcg caggcagcca aagagttgct ccgcttctac 60
ccagtcggca gcgacaactt gcgttaaagt cgcaaaatta tcatctgcac tcactgcgtg 120
acgtaagcgg atggagtggc cggaaacctc atagtgaccg cccaccagtt ggcctgcatc 180
gctttgtagc gtacgcgcgg cattggcaat aagattcaga tactcagact cttccggggc 240
cttcgccagc ataaaagagg aggatgctcg cgtatgcagc aactgctcca gcgcaaattg 300
cagccgcggt tgagtatcac tgaataaagg atcgttttcg tcaatcaaat gtggctgagc 360
aaatatttcc tgatagctat cggtatcagg aaccaggtca cgccatgcaa gtttcgtaat 420
ggtcaaagtt gatgtttttt agtctgttgt caaagccgcn attataccng taaccggcac 480
tacagcacac gtagaaagca cccgacaata ctcctggcat gggcgttaaa gctcacagga 540
tggagatctt ttcttcactg gcctaaaaag ctgatattct gtaaagagtt acacngtaac 600
attgagatcg ctatgaaata tcaacaactt ggaaaatctt gnaaagcngg ttggaaaatg 660
gaaagtatct ggttaagaag c 681

<210> 4
<211> 289
<212> DNA
<213> E. Coli

<400> 4
ggcagaattt tacgctgacc aatgacgcga cgacgtggca tggaaatact ccgttgttaa 60
ttcaggattg tccaaaactc tacgagttta gtttgacatt taagttaaaa cgtttggcct 120
tacttaacgg agaaccatta agccttagga cgcttcacgc catacttgga acgagcctgc 180
ttacggtctt taacgccgga gcagtcaagc gcaccacgta cggtgtggta acgaacaccc 240
gggaggtctt taacacgacc gtcacggatc aggatcacgg agtgctcct 289

<210> 5
<211> 815
<212> DNA
<213> E. Coli

<220>
<221> misc_feature
<222> (1)...(815)
<223> n = A,T,C or G

<400> 5
gggagcttac atcagtaagt gaccgggatg agcgagcgaa gataacgcat ctgcggcgcg 60
aaatatgaag ggggagagcc cttatagacc aggtagtaca cgtttggtta gggggcctgc 120
atatggcccc ctttttcact tttatatctg tgcggtttaa tgccgggcag atcacatctc 180
cgaggatttt agaatggctg aaattaccgc atccctggta aaagagctgc gtgagcgtac 240
tggcgcaggc atgatggatt gcaaaaaagc actgactgaa gctaacggcg acatcgagct 300
ggcaatcgaa aacatgcgta agtccggtgc tattaaagca gcgaaaaaag caggcaacgt 360
tgctgctgac ggcgtgatca aaaccaaaat cgacggcaac tacggcatca ttctggaagt 420
taactgccag actgacttcg ttgcaaaaga cgctggtttc caggcgttcg cagacaaagt 480
tctggacgca gctgttgctg gcaaaatcac tgacgttgaa gttctgaaag cacagttcga 540
agaagaacgt gttgcgctgg tagcgaaaat tggtgaaaac atcaacattc gccgcgttgc 600
tgcgctggaa ggcgacgttc tgggttctta tcagcacggt gcgcgtatcg gccgttctgg 660
ttgctgctaa aagcgctgac gaagaactgg ttaaacacat cgttttgacc tttgttgcaa 720
gccaagccag aattcagaga aactttccgc ttcaccggag gtcccaccca cangganccc 780
cgattttntc agcatggtgg tcttcctncg gagtt 815

<210> 6
<211> 403
<212> DNA

<213> E. Coli

<400> 6
caacactatt ttgttgaccg gaaaatggaa cactttccgc aatgcctgtt gctatcacgc 60
ttaaaccatt tcattgcgat ttacacagaa cggacgtcct gtcgcagtat attaagtcgt 120
cgatagaaac aagcattgaa aggcacagca gtagtcaaac agtgtgaaac gctactggcg 180
ccttacagcg caaaaaggct ggtgactaaa aagtcaccag ccatcagcct gatttctcag 240
gctgcaaccg gaagggttgg cttatttaac ttcaacttca gcgccagctt cttccagagc 300
ttttttcagt gcttctgcgt cgtctttgct cacgccttct ttcagagcag ccggtgcaga 360
ttctaccagg tctttagctt ctttcagacc caggccagtt gcg 403

<210> 7
<211> 149
<212> DNA
<213> E. Coli

<400> 7
gagctttttt cagtgcttct gcgtcgtctt tgctcacgcc ttctttcaga gcagccggtg 60
cagattctac caggtcttta gcttctttca gacccaggcc agttgcgcca cgtactgctt 120
tgataacagc aactttgtta gcgccagca 149

<210> 8
<211> 742
<212> DNA
<213> E. Coli

<220>
<221> misc_feature
<222> (1)...(742)
<223> n = A,T,C or G

<400> 8
ccatctgtcc attgagcgga cagtttgtgc aacactattt tgttgaccgg aaaatggaac 60
actttccgca atgcctgttg ctatcacgct taaaccattt cattgcgatt tacacagaac 120
ggacgtcctg tcgcagtata ttaagtcgtc gatagaaaca agcattgaaa ggcacagcag 180
tagtcaaaca gtgtgaaacg ctactggcgc cttacagcgc aaaaaggctg gtgactaaaa 240
agtcaccagc catcagcctg atttctcagg ctgcaaccgg aagggttggc ttatttaact 300
tcaacttcag cgccagcttc ttccagagct tttttcagtg cttctgcgtc gtctttgctc 360
acgccttctt tcagagcagc cggtgcagat tctaccaggt ctttagcttc tttcagaccc 420
aggccagttg cgccacgtac tgctttgata acagcaactt tgttagcgcc agcagctttc 480
agaattacgt cgaattcagt tntttcttca gcagcttcaa ccgggccagc agctacagct 540
acagcagcag caagcggaaa caccgaattt ttcttccatt gcagagatca gttctacaac 600
cgtccattac agacatagct gcaactgctt caatgatttt gatctttagt ggatagacat 660
ttaaattgtt cctgaattat caagaaataa gtnttatacg taagccgaaa tgcgttaaaa 720
aagataactg ngattaaagc ag 742

<210> 9
<211> 421
<212> DNA
<213> E. Coli

<400> 9
agtagtcaaa cagtgtgaaa cgctactggc gccttacagc gcaaaaaggc tggtgactaa 60
aaagtcacca gccatcagcc tgatttctca ggctgcaacc ggaagggttg gcttatttaa 120
cttcaacttc agcgccagct tcttccagag cttttttcag tgcttctgcg tcgtctttgc 180
tcacgccttc tttcagagca gccggtgcag attctaccag gtctttagct tctttcagac 240
ccaggccagt tgcgccacgt actgctttga taacagcaac tttgttagcg ccagcagctt 300
tcagaattac gtcgaattca gttttttctt cagcagcttc aaccgggcca gcagctacag 360
ctacagcagc agcagcggaa acaccgaatt tttcttccat tgcagagatc agttctacaa 420
c 421

<210> 10
<211> 126
<212> DNA
<213> E. Coli

<400> 10
   agagcttttt tcagtgcttc tgcgtcgtct ttgctcacgc cttctttcag agcagccggt 60
   gcagattcta ccaggtcttt agcttctttc agacccaggc cagttgcgcc acgtactgct 120
   ttgata 126

<210> 11
<211> 262
<212> DNA
<213> E. Coli

<220>
<221> misc_feature
<222> (1)...(262)
<223> n = A,T,C or G

<400> 11
   ctgcaaccgg aagggttggc ttatttaact tcaacttcag cgccagcttc ttccagagct 60
   tttttcagtg cttctgcgtc gtctttgctc acgccttctt tcagagcagc cgntgcagat 120
   tctaccaggt ctttagcttc tttcagaccc aggccagttg cgccacgtac tgctttgata 180
   acagcaactt tgttagcgcc agcagctttc agaattacgt cgaattcagt tttttcttca 240
   gcagcttcaa ccgggccagc ag 262

<210> 12
<211> 202
<212> DNA
<213> E. Coli

<220>
<221> misc_feature
<222> (1)...(202)
<223> n = A,T,C or G

<400> 12
gcgca[?]accc tgcagcatcg gcccgatgga gatcaggtcg gcagaacgct gtaccgcttt 60
gtagg[?]ggtg ttaccggtgn tcagatccgg gaagatgaac acggtagcgc gacctgcaac 120
cggagagttc ggcgctttgg attncgcaac gtcagccatt accgcagcgt cgtactgcag 180
cggac[?]ggcg atcatcaggt ca 202

<210> 13
<211> 261
<212> DNA
<213> E. Coli

<400> 13
tctaggagta agaatagctt caaattcagc agttgacagt ggcataaacg taactggtga 60
cttttgcccg gcatgacgcc gggctttttt tattattccg tgacttccag cgtagtgaag 120
gcaaa[?]ttct cgccatcaaa tagcccctga ctggttagtt ttagcgcggg gatcactggc 180
agagaaagaa acgccatctg aataaacggc tcatcgggta acggaccgca ttcacgggcg 240
gcggc[?]ttca aggcgtcaat t 261

<210> 14
<211> 224
<212> DNA
<213> E. Coli

<400> 14

ttcttttttt cgtcaacggt gtccagaatc attttattta cctcggggta cttatgctga 60
tttttattat tatggggaag gtgttattta tgagtttcat ttatgccgta acgacaatga 120
actcgggaat tagtataagc agcgcgagaa taataatcat tgtgcaaatg ctaatttaat 180
taatactatt taaatattat tttgagcata tgcacataag gttg 224

<210> 15
<211> 232
<212> DNA
<213> E. Coli

<400> 15
aattcccttc tttttttcgt caacggtgtc cagaatcatt ttatttacct cgggtactta 60
tgctgatttt tattattatg gggaaggtgt tatttatgag tttcatttat gccgtaacga 120
caatgaactc gggaattagt ataagcagcg cgagaataat aatcattgtg caaatgctaa 180
tttaattaat actatttaaa tattattttg agcatatgca cataaggttg gg 232

<210> 16
<211> 212
<212> DNA
<213> E. Coli

<400> 16
aatagcgggt atgcacgcct ttcttttttt cgtcaacggt gtccagaatc attttattta 60
cctcgggtac ttatgctgat ttttattatt atggggaagg tgttatttat gagtttcatt 120
tatgccgtaa cgacaatgaa ctcgggaatt agtataagca gcgcgagaat aataatcatt 180
gtgcaaatgc taatttaatt aatactattt aa 212

<210> 17
<211> 433
<212> DNA
<213> E. Coli

<400> 17
ccttgtaaat tatcgcccgt ggcataaaaa ctgcgtccaa acgccgtctt tgccagcagc 60
caggccataa atgccaccag aattatcgtc aaccaaccaa ttgctgaaac gccaagcagc 120
agcggggcgg agagctgttt cagttcggcg ggtaaccctt caatccattt gccgccagtc 180
cacagcaaca tgatgcctct gtacaaccct aacgtgccaa gggtggcaac aatggcaggg 240
atctttagcc acgcgaccag gacaccgttg aaaaatcccg cgagcaaacc aagcagtaaa 300
gtcgcgacac aagcaacagg tagtgaatat cctgcgttca gtaacatccc caacagcacc 360
gcgcacattc cggtaatcga acccactgaa acatcaatat tgcgcgtaag cattaccagc 420
gtcgcgccca ttg 433

<210> 18
<211> 658
<212> DNA
<213> E. Coli

<400> 18
cgtgcgcttc cggttgtggc aacccgcgaa atggcgcggc ggtaagtatg gcggggttat 60
tccttccccg ttgaggacac cgggttgtca ggttgaccat acgcttaagt gacaaccccg 120
ctgcaacgcc ctctgttatc aattttctgg tgacgtttgg cggtatcagt tttactccgt 180
gactgctctg ccgccctttt taaagtgaat tttgtgatgt ggtgaatgcg gctgagcgca 240
cgcggaacag ttaaaaccaa aaacagtgtt atgggtggat tctctgtatc cggcgttaat 300
tgttaactgg ttaacgtcac ctggaggcac caggcactgc atcacaaaat tcattgttga 360
ggacgcgata atgaaaacgt tattaccaaa cgttaatacg tctgaaggtt gttttgaaat 420
tggtgtcact atcagtaacc cagtatttac tgaagatgcc attaacaaga gaaaacaaga 480
acgggagcta ttaaataaaa tatgcattgt ttcaatgctg gctcgtttac gtctgatgcc 540
aaaaggatgt gcacaatgaa ttcagcattt gtgcttgttc tgacagtttt tcttgtttcc 600
ggagagccag ttgatattgc agtcaagtgg tcacaggaca atgcaggagt gtatgact 658

<210> 19

<211> 588
<212> DNA
<213> E. Coli

<220>
<221> misc_feature
<222> (1)...(588)
<223> n = A,T,C or G

<400> 19
gtgactgctc tgccgccctt tttaaagtga attttgtgat gtggtgaatg cggctgagcg 60
cacgcggaac agttaaaacc aaaaacagtg ttatgggtgg attctctgta tccggcgtta 120
attgttaact ggttaacgtc acctggaggc accaggcact gcatcacaaa attcattgtt 180
gaggacgcga taatgaaaac gttattacca aacgttaata cgtctgaagg ttgttttgaa 240
attggtgtca ctatcagtaa cccagtattt actgaagatg ccattaacaa gagaaaacaa 300
gaacgggagc tattaaataa aatatgcatt gtttcaatgc tggctcgttt acgtctgatg 360
ccaaaaggat gtgcacaatg aattcagcat ttgtgcttgt tctgacagtt tttcttgttt 420
ccggagagcc agttgatatt gcagtcagtg ttcacaggac aatgcangag tgtatgactg 480
cagcaacccg aacagaaaat tcccggtaac tgttacccgg tcgataaagt tattcaccag 540
gataatatcg aaatcccggc aggtctttaa aacagttccg taataaat 588

<210> 20
<211> 101
<212> DNA
<213> E. Coli

<400> 20
gatccagcaa gaagatgcgg ttgtaccgtc atcacgcaga tgcgcaaagc tactcagcaa 60
ctgacctttc ttcgcaataa gcacgccatt agcgtcatag a 101

<210> 21
<211> 465
<212> DNA
<213> E. Coli

<400> 21
tcgcgtgttt accttcaaca tcggtaactt tctggcggat agtttcacgg taagcaacct 60
gcggtttacc tacgttcgct tcaacgttga attcacgctt catacggtca acgatgatgt 120
cgaggtgcag ttcgcccata cccgcgatga tggtctggtt agattcttcg tcagtccata 180
cacggaaaga cgggtcttct ttagccagac ggcccagagc cagacccatt ttttcctggt 240
cagctttggt tttcggttca actgcgatgg agattaccgg ctcagggaat tccatacgtt 300
ccagaatgat cggcgcatcc gggtcacaca gggtgtcacc agtggttacg tctttcagac 360
cgatagcagc agcgatgtcg cccgcgcgaa cttctttgat ctcttcacgt ttgttagcgt 420
gcatctgaac gatacgaccg aaacgctcac gtgcagcttt cacgg 465

<210> 22
<211> 859
<212> DNA
<213> E. Coli

<220>
<221> misc_feature
<222> (1)...(859)
<223> n = A,T,C or G

<400> 22
tgatcggctc aagcagaact ggtttcgctt tcttaaagcc ttctttaaag gcgatagaag 60
cagccagttt aaacgccagt tcagaggagt caacgtcatg gtaagaaccg aagtgcagac 120
gaatacccat gtctactacc gggtagcctg ccagcggacc tgctttcagc tgttcctgga 180
tacctttatc aacggccggg atgtattcgc cagggattac accaccttta atgtcgttga 240
tgaactcgta gcctttcggg tttgaacccg gctccagcgg gtacatgtcg ataacaacat 300

gaccatactg accacgacca ccagactgtt tcgcgtgttt accttcaaca tcggtaactt 360
tctggcggat agtttcacgg taagcaacct gcggtttacc tacgttcgct tcaacgttga 420
attcacgctt catacggtca acgatgatgt cgaggtgcag ttcgcccata cccgcgatga 480
tggtctggtt agattcttcg tcagtccata cacggaaaga cgggtcttct ttagccagac 540
gggccanagc cagacccatt ttttcctggt cagctttggt tttcggtcaa ctgcgatgga 600
gattaccggc tcanggaatt tccatacctt ccaggaatga tcggcgcatt ccggtcaaac 660
anggngtacc aggggggtac ntntttttaa nancgattgc cagcancgga tntnncccgn 720
gccnaacttc tttggaacnn tttaccggtt ggtaaccngc cttttnaacn atccaaccga 780
aaaagngtta anngccantt ttccnggngt tnanntncgg nttcccngaa ntaacccncc 840
cggggtnaac ccngnaaaa 859

<210> 23
<211> 269
<212> DNA
<213> E. Coli

<400> 23
ctttcttaaa gccttcttta aaggcgatag aagcagccag tttaaacgcc agttcagagg 60
agtcaacgtc atggtaagaa ccgaagtgca gacgaatacc catgtctact accgggtagc 120
ctgccagcgg acctgctttc agctgttcct ggataccttt atcaacggcc gggatgtatt 180
cgccagggat tacaccacct ttaatgtcgt tgatgaactc gtagcctttc gggtttgaac 240
ccggctccag cgggtacatg tcgataaca 269

<210> 24
<211> 330
<212> DNA
<213> E. Coli

<400> 24
gttttgggga gatgtaaggg ctaatctgaa tggctgcatt ccttgtttaa ggaaaaacga 60
atgactgatt gccgatacct gattaaacgg gtcatcaaaa tcatcattgc tgttttacag 120
ctgatccttc tgttcttata acacaaggaa acgtacttaa ggtgcgtccg gtgaaccagt 180
cggacgcacc tttaataact ataaataagt gtctgggcag atactatata aattaactta 240
gtgaatgatt atgctaatgt catcaattaa ataaatataa tggcgttaag gcttcccagt 300
aatataatta atactctact tccagagtag 330

<210> 25
<211> 471
<212> DNA
<213> E. Coli

<220>
<221> misc_feature
<222> (1)...(471)
<223> n = A,T,C or G

<400> 25
gttttgggga gatgtaaggg ctaatctgaa tggctgcatt ccttgtttaa ggaaaaacga 60
atgactgatt gccgatacct gattaaacgg gtcatcaaaa tcatcattgc tgttttacag 120
ctgatccttc tgttcttata acacaaggaa acgtacttaa ggtgccgtcc ggtgaaccag 180
tcggacgcac ctttaataac tataaataag tgtctgggca gatactatat aaattaactt 240
agtgaatgat tatgctaatg tcatcaatta aataaatata atggcgttaa ggcttcccag 300
taatataatt aatactctac ttccagagta gaatattaaa ttttatccgc gtggtgcatc 360
agcacaaatt tatcccacaa ctgttcttct gtctcgacat gccccccgat ctttnacaaa 420
tantattggg ggattnggcc cncctttttg ncaggttggg gtcntctnat g 471

<210> 26
<211> 379
<212> DNA
<213> E. Coli

<220>
<221> misc_feature
<222> (1)...(379)
<223> n = A,T,C or G

<400> 26
natctgantg gctgcattcc ttgtttaagg aaacccgaat gactgattgc cgatacctga 60
ttaaacgggt catcaaaatc atcattgctg ttttacagct gatccttctg ttcttataac 120
acaaggaaac gtacttaagg tgcgtccggt gaaccagtcg gacgcacctt taataactat 180
aaataagtgt ctgggcagat actatataaa ttaacttagt gaatgattat gctaatgtca 240
tcaattaaat aaatataatg gcgttaaggc ttcccagtaa tataattaat actctacttc 300
cagagtagaa tattaaattt tatccgcgtg gtgcatcagc acaaatttat cccacaactg 360
ttcttctgtc tcgacatgc 379

<210> 27
<211> 799
<212> DNA
<213> E. Coli

<400> 27
aaagatgatg tgatgagaaa gtcaatttga ataagacaat attaagagct aaaaaaatgt 60
caaaaaacac taaatcaaaa aataatggca ttagaaaata taatgcgaaa acggaggtga 120
aattagttta tttcaaatga ggaaaatctc ccggcgaaaa aaccgggaga tgaaagtgtg 180
atgggtatca aataaacaac agaggagaaa tttttaacgc agccattcag gcaaatcgtt 240
taatcccatt gcctggcgga taagttgcgg cttaacgcca ggaagcgtgt cggccagttt 300
caaaccaata tcacgcagca gtttttcgc cggattggta ccggaaaaca gatcgcggaa 360
tccctgcata ccagccagca tcaacgccgc actgtgcttg cggctacgct catagcgacg 420
cagataaatg tactgcccga tgtctgggat ccgtcgacct gcagccaagc ttgggctttt 480
cagcctgata cagattaaat cagaacgcag aagcggtctg ataaaacaga atttgcctgg 540
cggcagtagc gcggtggtcc cacctgaccc catgccgaac tcagaagtga aacgcccgta 600
gcgcccgatg gtagtgtggg gtctccccat gcgagagtag ggaactgcca ggcatcaaat 660
aaaacgaaag gctcagtcga aagactgggc ctttcggttt atctggtggt tgtcggtgaa 720
cgctctctga gtaggacaaa tccgccggga gcggatttgg aacgttgcga aacaaccggc 780
ccggaaaggg gtgggggct 799

<210> 28
<211> 636
<212> DNA
<213> E. Coli

<220>
<221> misc_feature
<222> (1)...(636)
<223> n = A,T,C or G

<400> 28
agggggtttg ttgtgggcaa tgatgcattt aagttatcgt ctgcagatag aggagatatt 60
acaataaaca acgaatcagg gcatttgata gtcaataccg caattctatc aggagatata 120
gtcactctaa gaggaggaga aattaggttg gtattatagc ttgtgcgcgc catgattggc 180
gcgcaattta aacttagtgc tttacatcgc tattgtcttg atttctttga attattttat 240
aaattaaaaa aacgactgtt atgtataagc aaaggtcgaa cgaaaaatac attccaaata 300
aatgcttgct taaatctcta tatccttccc cgaaaaatga cacataaaat tgagatattc 360
caaaaagaga tactacaaat aaagatgcct ttattttatt atttctaata aaaatagaag 420
caataaaaaa taataacaat gatataaatc taatgttttt aaatatattg tcttttatgt 480
tagtaatagt cgttagtatg tttgattctc catatattac gtgtagtttt ttatatacat 540
ggaaataatt ntctttatac tgagacatca caccatcatc aaatggaagt ttgaagatgg 600
tgcttggttt gctaaccaat aaaaagagtg cattcg 636

<210> 29
<211> 757
<212> DNA

<213> E. Coli

<220>
<221> misc_feature
<222> (1)...(757)
<223> n = A,T,C or G

<400> 29
cagcggtcgt atttttagca tggtttttta ttggcggcta tgctgccccg ggagcataaa 60
gatgaaaaaa acaacgatta ttatgatggg tgtggcgatt attgtcgtac tcggcactga 120
gctgggatgg tggtaacgtc acctctaaaa aatagcaaag gctgcctgtg tgcagccttt 180
gtgcaattta agcgttaact tttaatcttc ctgtagataa atagcacgac aatcgcacca 240
ataacggcaa ccacgaagct gccaaaattg aagccatcga ctttaccaaa gccaaacagc 300
gtgctgatcc atccgccgac tacggcaccg actatcccca gcaggatagt cataaagaat 360
ccacctccat ctttacctgg catgatccac ttcgccagaa taccggcaat aagcccaaaa 420
ataatccatg acagaatgcc cattgtttcc tcacttatct gttttgcatt agcgggttag 480
tcgctgataa aaagcatagc acaacatcgg gagggcaaga tttgtgacga gcatcacgga 540
ggtttttttt gcgatggcgc agaaattgcg ccatcaacga tcagtgataa ttaccaacca 600
caaacatcat gttcgttttc cgtgtcataa gaaccgtacg ggattcacca gatcttttat 660
cacttcaagc cggcacttct ggcaccagca aagtcatcgg cgtctctggt tcataatcga 720
ccggaaacgc cattgctggt attggtgacn gtcacgg 757

<210> 30
<211> 392
<212> DNA
<213> E. Coli

<400> 30
aattacagaa aaaggaggca atatcgggta aaggcattag cccgacgaat acgtcgggct 60
acaaatatta ttgtgctgca ggtgttttag cgggttgttg atccacaggt tctaactgga 120
agaccacatc gacctgatca tcaaactgaa tagcggcctg ctcgtaagtt tcctgggcgg 180
acaccggcgc ggcatcggct ttcatcatcc gcaccattgg gctgggctga tagttggaaa 240
catggtagcg cacgctatat accggcccca gtttacgatg aaagccgttc gccagttcct 300
gcgcctgatg aatcgcgtta tcaatcgctg ccttacgcgc tttgtcttta taggcatccg 360
gctgcgccac gcccagcgac acagaacgaa tt 392

<210> 31
<211> 351
<212> DNA
<213> E. Coli

<400> 31
ctatccttga tgaaaccgcg agcaaagata ggtgattacg tcatggtttt acagaaaatt 60
acagaaaaag gaggcaatat cgggtaaagg cattagcccg acgaatacgt cgggctacaa 120
atattattgt gctgcaggtg ttttagcggg ttgttgatcc acaggttcta actggaagac 180
cacatcgacc tgatcatcaa actgaatagc ggcctgctcg taagtttcct gggcggacac 240
cggcgcggca tcggctttca tcatccgcac cattgggctg ggctgatagt tggaaacatg 300
gtagcgcacg ctatataccg gccccagttt acgatgaaag ccgttcgcca g 351

<210> 32
<211> 762
<212> DNA
<213> E. Coli

<220>
<221> misc_feature
<222> (1)...(762)
<223> n = A,T,C or G

<400> 32
aattatgaaa cactgtctgg aatcgtctga atgacgggca catttgcgag cacgcatcca 60

gtaataacac aggaaactat tttatctacg cgttagcgat agactgcttg catggcgaaa 120
ggaggtaagc cgacgatttc agcgggacgc tgaaacggga aagcccctcc cgaggaaggg 180
gccataaata aggaaagggt catgatgaag ctactcatca tcgtggtgct cttagtcata 240
agcttccccg cttactaaga ctaccagggc gggggaaacc ccgctctacc ctcactcctg 300
aaagtatgcc ttcacgataa gattgtcaat ccgcaggctt tgtagtctgc gatcctgcca 360
gcaaatattc tttgcgagtc gttacgcaat aatcacagag gaaactattt tattcacgcg 420
ttagcgatag actgcattca gggcgaaagg aggtaagccg atgatttcag cgggacgctg 480
aaacgggaaa gcctctcccg gagaagaggg cttttaataa ggaaagggtt atgatgaagc 540
acgtcatcat actggtgata ctcttagtga ttagcttcca ggcttactaa gaacaccagg 600
gggaggggga aacctcttcc taaccctcac ttctgaaatt gggtgctatg acgctggcgt 660
tactgcttan cgctaccagt ttgtctgccc tggcggttgt aacgccagat cggtacccgt 720
ttggatattt taatgaaagc cgacaaatca atcancgtga cg 762

<210> 33
<211> 293
<212> DNA
<213> E. Coli

<400> 33
gcacatttgc gagcacgcat ccagtaataa cacaggaaac tattttatct acgcgttagc 60
gatagactgc ttgcatggcg aaaggaggta agccgacgat ttcagcggga cgctgaaacg 120
ggaaagcccc tcccgaggaa ggggccataa ataaggaaag ggtcatgatg aagctactca 180
tcatcgtggt gctcttagtc ataagcttcc ccgcttacta agactaccag ggcgggggaa 240
accccgctct accctcactc ctgaaagtat gccttcacga taagattgtc aat 293

<210> 34
<211> 633
<212> DNA
<213> E. Coli

<220>
<221> misc_feature
<222> (1)...(633)
<223> n = A,T,C or G

<400> 34
atttacactt tttacgaaat catgggatca ctaacaaaat atcgcttgtc agttatattg 60
tatggcagga aagatatgcg actgatatta cagatcccca aagtggagag tttatgacca 120
ttaaaaataa gatgttgctg ggtgcgcttt tgctggttac cagtgccgcc tgggccgcac 180
cagccaccgc gggttcgacc aatacctcgg gaatttctaa gtatgagtta agtagtttca 240
ttgctgactt taagcatttc aaaccagggg acaccgtacc agaaatgtac cgtaccgatg 300
agtacaacat taagcagtgg cagttgcgta acctgcccgc gcctgatgcc gggacgcact 360
ggacctatat gggtggcgcg tacgtgttga tcagcgacac cgacggtaaa atcattaaag 420
cctacgacgg tgagattttt tatcatcgct aaaaaaagcc ccctcatcat gagggggaaa 480
tgcagacacc ttgntatttt ttattattag ccacttgctc gtcttgcttg gtattaagtc 540
gtatttcacg ttgattaatg cnggtggctc cagtgcgcca gattaacttt gtttggatcg 600
aagacgtagt aactggctgg ttatcggaat tgg 633

<210> 35
<211> 569
<212> DNA
<213> E. Coli

<400> 35
tatggcagga aagatatgcg actgatatta cagatcccca aagtggagag tttatgacca 60
ttaaaaataa gatgttgctg ggtgcgcttt tgctggttac cagtgccgcc tgggccgcac 120
cagccaccgc gggttcgacc aatacctcgg gaatttctaa gtatgagtta agtagtttca 180
ttgctgactt taagcatttc aaaccagggg acaccgtacc agaaatgtac cgtaccgatg 240
agtacaacat taagcagtgg cagttgcgta acctgcccgc gcctgatgcc gggacgcact 300
ggacctatat gggtggcgcg tacgtgttga tcagcgacac cgacggtaaa atcattaaag 360
cctacgacgg tgagattttt tatcatcgct aaaaaaagcc ccctcatcat gagggggaaa 420

tgcagacacc ttgttatttt ttattattag ccacttgctc gtcttgcttg ttattagtcg 480
tatttcacgt tgattaatgc ggttgcctcc agtgcgccag atttaacttt gtttgtatcg 540
tagacgtagt aactggctgg tatcggaat 569

<210> 36
<211> 338
<212> DNA
<213> E. Coli

<400> 36
cgtattcaca tccttttgat tggtgataac atgcgaatcg gtattatttt tccggttgta 60
atcttcatta cagcggtcgt atttttagca tggtttttta ttggcggcta tgctgccccg 120
ggagcataaa gatgaaaaaa acaacgatta ttatgatggg tgtggcgatt attgtcgtac 180
tcggcactgc ctgggatggt ggtaacgtca cctctaaaaa atagcaaagg ctgcctgtgt 240
gcagcctttg tgcaatttaa gcgttaactt ttaatcttcc tgtagataaa tagcacgaca 300
atcgcaccaa taacggcaac cacgaagctg ccaaaatt 338

<210> 37
<211> 375
<212> DNA
<213> E. Coli

<400> 37
ctgaatattt aaaaaggaaa acgacatgaa accgaagcac agaatcaaca ttctccaatc 60
ataaaatatt tccgtggagc attttattat tgaatataga ggtttaactc cggtaaaaaa 120
caaagaagca ttgaatgcag ggaaaaataa tatggccata aaaaacatcg aaagaaactc 180
ttttaattta acatgtaaac gcatggttaa tcctcatatc acgggtggag tgttaagaac 240
atacataaat ggagtcatgt tttccctttt ccatttatca agttcctgtt gccgttttag 300
tccatctcta attgcatatt ttaatttttc tgataaatgg cattgagcat cgatttcatt 360
taaaacaact gtaca 375

<210> 38
<211> 446
<212> DNA
<213> E. Coli

<400> 38
ttacgatagc tattagtaaa aatataagag ttagctgtat tgttatgtct gtggcgaaat 60
tgactacctt cgtttttttg attaagaatg attttattat cgtaagtaaa attacatgaa 120
tatttaaaaa ggaaaacgac atgaaaccga agcacagaat caacattctc caatcataaa 180
atatttccgt ggagcatttt attattgaat atagaggttt aactccggta aaaaacaaag 240
aagcattgaa tgcagggaaa aataatatgg ccataaaaaa catcgaaaga aactctttta 300
atttaacatg taaacgcatg gttaatcctc atatcacggg tggagtgtta agaacataca 360
taaatggagt catgtttccc cttttccatt tatcaagttc ctgttgccgt tttagtccat 420
ctctaattgc atattttaat tttct 446

<210> 39
<211> 392
<212> DNA
<213> E. Coli

<220>
<221> misc_feature
<222> (1)...(392)
<223> n = A,T,C or G

<400> 39
tcaccccggt gccgattttc aggcatcctg atttaactta gcacccgcaa cttaactaca 60
ggaaaacaaa gagataaatg tctaatcctg atgcaaatcg agccgatttt ttaatcttta 120
cggactttta cccgcctggt ttattaattg cactgtnatc cgggcgttcg cccgctttaa 180
tcacaatagg ctgtgtagcc tgggcctgtt tctctttcac ccgcgccaga gcggcagcaa 240

tcgcatcttt atctttggct gcaggttgaa cggctgcgct cttatgtcgt tcaaggcgag 300
ccgcttttc gcgctccaga cgagcctggc gcgcttcgaa acgcgctttg gcttctgcgg 360
cncgcctttc ttcctgacga atagccgcaa tt 392

<210> 40
<211> 208
<212> DNA
<213> E. Coli

<400> 40
taataacgct atctgcggat aaagcagaat aggtggttaa ccccagacat aaaccgagga 60
aaataatgtt attgtatttc ataatctatt gttccttagc gacagattgc tgtctgctgg 120
ttcagtaagg taccaggaga aacttcagga agcttgtact cgacaataca gtttgagttt 180
ttatctttgc cccatgaaac ctgtaatt 208

<210> 41
<211> 342
<212> DNA
<213> E. Coli

<400> 41
catcctcaat accgttaaat gcaacccgaa cccccgttgt ccctttgctg cattcactta 60
acgtaatctg aaaagggacg gctggacttg tgctaccggt cgttggaaat tgtctggcac 120
tgtttttttg gagatctacg gtaaaattaa gcgaatccga tgagactgtg cagccataat 180
cgaggacgcg cccgctaatt ttaataacgc tatctgcgga taaagcagaa taggtggtta 240
accccagaca taaaccgagg aaaataatgt tattgtattt cataatctat tgttccttag 300
cgacagattg ctgtctgctg gttcagtaag gtaccaggag aa 342

<210> 42
<211> 841
<212> DNA
<213> E. Coli

<220>
<221> misc_feature
<222> (1)...(841)
<223> n = A,T,C or G

<400> 42
agatttactg ccaatttccg gcagatcgga aagggttaaa ccatattgat ccataagggt 60
acgaatcacg gctataccgc caggcatggc ttgagccatg gcattaaatt ccgcaaattc 120
gggcgctgat tcttcccacg cggttatttt ggcacacacc agatccagca aggggttntc 180
aggatcgttg agcagcagat gatctaccag ttncagcgcc tgggtgtatt gntccttgtt 240
ctgaataccc gnnagaaaag gtgccacagc anttagcttn tctcctgctt gcaagatgtc 300
tggcaatngc aatcattttt tgcacttant acgatgnaca ncngtaaaga aatcgnattt 360
ttntatgccg tcataacttt acgtatgtan cactttttgc nattcnaaaa aagaccattn 420
gctncaacac gtaaatttna ttgnccccna catttanaac ataaatgntt aaaattttcc 480
ccccncnnan ttttaagntn ttnanagaat ngggaattac ctgcttttna atgnactcan 540
anttttttng naataattcc tntatcnaan ctnnttttcn cccaanagnc nnccaaattn 600
cggtttnntn nttnncnngg cnttttttta cccnanaann tttattcaan nccttttttg 660
tagnctattt naagnggnct ttnttnnatt aactttccnn ttggncaaat tttggcnnat 720
ttttatatan aattntctta tntcntaatt tnggnanccc cngatgnaan tttatggngg 780
gantcccnnt ccctntttaa tnnatgntct gggntatttt taaancctnn attaannnan 840
c 841

<210> 43
<211> 215
<212> DNA
<213> E. Coli

<400> 43

aataactttt cgttaggcag ttttgggtgt gagttgcaag aggggagact actgaataac 60
tcaagtttta taatcgaggg gaaaatggtg atggcgttca tagcaaaacg ccctcaacca 120
taaaggtcga gggcgcttaa gatgttaaaa acccgctatc cgttaaaaaa caatgttcaa 180
ctaaggtcag tgacattgcg ctaaaaaagc gaatt 215

<210> 44
<211> 395
<212> DNA
<213> E. Coli

<220>
<221> misc_feature
<222> (1)...(395)
<223> n = A,T,C or G

<400> 44
gcattattca tgagaaatgt gtatcgtaaa tcaactgaaa ttaacgcaac catttgttat 60
ttaaggttta attatctgtg tgtgatattt tattgaatgt tttaaatatt gtttttattg 120
gcattgctat aatattggtt atcatttgct gaatggattc agtcttaatg agtgggtttt 180
taagggacag gcatagagta atgatacgta tgcataacca acatctttac tcattatgtc 240
attgaatgtt gaccctatgt gtttatgaag gagaggtatt ttcagttgat ctggattgnt 300
aaattcatat aatgcgcctt tgctcatgaa tggatgccag tatgtagtgg gaaattataa 360
atattgaaat agtccaacta cttctttatt accaa 395

<210> 45
<211> 883
<212> DNA
<213> E. Coli

<220>
<221> misc_feature
<222> (1)...(883)
<223> n = A,T,C or G

<400> 45
ataatcaggt aagaaaaggt gcgcggagat taccgtgtgt tgcgatatat tttttagttt 60
cgcgtggcaa tacatcagtg gcaataaaac gacatatcca gaaaaatata cactaagtga 120
atgatatctt ccgatttatc ttaatcgttt atggataacg gcaaagggct tcgttttttc 180
ctatacttat tcagcactca caaataaagg aacgccaatg aaaattatac tctgggctgt 240
attgattatt ttcctgattg ggctactggt ggtgactggc gtatttaaga tgatatttta 300
aaattaatta atgtcatcag gtccgaaaat aacgagaata tttcagtctc tcatcctgtt 360
gcgctcctgt catgtgcatt gcttcatata atcactggcg caaggagcgc cgcaggcgna 420
gnntgcncgn cgncccacct naccccatgc cgaacttcag aantgaaaac nccntaacnc 480
cgatngtcgg cgggngcctc cccatgcnan agtangggaa ntgccangcg ncnnattaaa 540
cgaaaggctn attncaaaga ctgggccttn cntttatctg atgtttgtcg gagaacgctc 600
tcctgagnan gacaaatncc gccgggagcg gatttgaacn ttgcgaagca accgncccna 660
agggngnngt cntgacnccc nnctctanct nncngccttc ttttgcttna angncctcct 720
ancngatggc ctttttngcc ntctaccaaa cnntttggtt aatgcttnta aaancctttc 780
cannntncaa tccngtnntn cccatccnnn tnntgaaagn ntncctnccn tgtncantnt 840
anntnngggg gnngngngcc ggcggncccc cccccccccc ccc 883

<210> 46
<211> 1024
<212> DNA
<213> E. Coli

<220>
<221> misc_feature
<222> (1)...(1024)
<223> n = A,T,C or G

<400> 46
gtttatggat aacggcaaag ggcttcgttt tttcctatac ttattcagca ctcacaaata 60
aaggaacgcc aatgaaaatt atactctggg ctgtattgat tattttcctg attgggctac 120
tggtggtgac tggcgtattt aagatgatat tttaaaatta attaatgtca tcaggtccga 180
aaataacgag aatatttcag tctctcatcc tgttgcgctc ctgtcatgtg cattgcttca 240
tataatcact ggcgcaagga gcgcgcagag tnctccnant nnnnntnntt ntntnnctnn 300
nccttcacna tncnnccncn nantnnatag nncaccnntn ttnntcnnnn gnccncctcc 360
nnncnnnnnn ncatnnnatc ccactnnntt tnctccannn nnncnnnntn canccnacaa 420
antncnaccn anntnacctt atacnnannc nancnnnnnn nnccactctn nctcgnnctc 480
cccnttcnac nnccannnnn cancnntcnn ctnnnnccct nncntaattn ttctnnctan 540
ntcctanccn cnnacnnncc cancnatccn nnnatacant cnattnntnn cnntcncntn 600
cnccnnttcc nnctnnncnc tnccncatnc ccnnnannan canntncccc ncctncctna 660
ccncncncnc ccnccatccc nnnccnncnt ccnnantnga caannnnaat cncnnnnncn 720
nnnnnncnnn tnnncncccn gcncnnccnt nccntcacnc tnnncnncta nannnnntac 780
nntnaccnnt cctnncacnc tnccctnnng antccnacna ntnnnnnanc nanaacnctn 840
tnnnnccata atcccacacc acncccntnc ancntntnnt ncntcntccc ttcntatcnc 900
agctnnnnnt nctntnnnnc tnccncccnn cnnactncnn nnacccncnn cccantcagt 960
ccaccntccn cnncnnnntn nnncnancan ctnncacncn cnantaacct nntnncacct 1020
tccc 1024

<210> 47
<211> 236
<212> DNA
<213> E. Coli

<400> 47
atatacacta agtgaatgat atcttccgat ttatcttaat cgtttatgga taacggcaaa 60
gggcttcgtt ttttcctata cttattcagc actcacaaat aaaggaacgc caatgaaaat 120
tatactctgg gctgtattga ttattttcct gattgggcta ctggtggtga ctggcgtatt 180
taagatgata ttttaaaatt aattaatgtc atcaggtccg aaaataacga gaatat 236

<210> 48
<211> 418
<212> DNA
<213> E. Coli

<220>
<221> misc_feature
<222> (1)...(418)
<223> n = A,T,C or G

<400> 48
cggagattac cgtgtgttgc gatatatttt ttagtttcgc gtggcaatac atcagtggca 60
ataaaacgac atatccagaa aaatatacac taagtgaatg atatcttccg attnatctta 120
ntcgtttatg gataacggca aagggcttcg ttttttccta tacttattca gcactcacaa 180
ataaaggaac gccaatgaaa attatactct gggctgtatt gattattttc ctgattgggc 240
tactggtggt gactggcgta tttaagatga tattttaaaa ttaattaatg tcatcaggtc 300
cgaaaataac gagaatattt cagtctctca tcctgttgcg ctcctgtcat gtgcattgct 360
tcatataatc actggcgcaa ggagcgcgca nggggcggcc aatcgccgcc ggcccctg 418

<210> 49
<211> 550
<212> DNA
<213> E. Coli

<400> 49
ctgctagtta cagggaacac taatgacaga cagctaaaag ccctgtttaa ttacgtatta 60
caaacagggg atgcccagcg ttttcgtgca tttattggtg agatagcgga acgcgcacca 120
caagaaaagg agaaactgat gaccattgct gacagattac gtgaagaagg cgcaatgcag 180
ggcaaacacg aagaagccct gcgtattgct caggagatgc tggatagagg tttagacaga 240
gagttagtta tgatggtgac ccgactttca ccagacgatc ttatcgcgca aagccactaa 300

tcctgtaaca ccgggagtta actggcggat gtttgctgta aaccacatca gcgaacgaca 360
tccgccagcg cctcttctaa atcgtaccag cgaaacgcaa aacccgcttc ttccagccgt 420
ttaggcagcg cgcgttgtcc acctaatacc agtactgaag attcgcccat taacagtcga 480
atggcggtcg cggggacgcg caaaatggcc gggcgatgca gcgcatgacc gagcgcatgg 540
gcaaattgtt 550

<210> 50
<211> 99
<212> DNA
<213> E. Coli

<400> 50
ttggcatctc ggtgttgccg atcttcatga tatccagccc gccggaaact tcttcccaaa 60
cggttttgct gttatccatt gagtcacgga actgcccct 99

<210> 51
<211> 259
<212> DNA
<213> E. Coli

<220>
<221> misc_feature
<222> (1)...(259)
<223> n = A,T,C or G

<400> 51
ccgtgccgag atgatcctgt naccatcatc cgttgtgaag tagtgattca cgacttcaag 60
gcgcttttca aaagggtatt ttggctttga catattaggg gctattccat ttcatcgncc 120
aacaaaatgg gtgcagtaca tactcnttgg aaatcaacac aggaggctgg gaatgccgca 180
gaaatataga ttactttctt taatagtgat ntgtttcacg cttttatttt tnaaanaagt 240
tnggcttact tcccgggnn 259

<210> 52
<211> 877
<212> DNA
<213> E. Coli

<220>
<221> misc_feature
<222> (1)...(877)
<223> n = A,T,C or G

<400> 52
cagcagagcg cggccttctt cgtcagattt cgcagtagtg gtaatggtaa tatccaaacc 60
acgaacgcgg tcgactttat cgtagtcgat ttctgggaag atgatctgct cacggacacc 120
catgctgtag ttaccacgac cgtcgaaaga cttagcggac aggccacgga agtcacggat 180
acgaggtaca gcaatagtga tcaggcgctc aaagaactcc cacatgcgtt cgccacgcag 240
agttacttta cagccgatcg gatagccctg acggattttg aagcctgcaa cagatttgcg 300
tgctttggtg atcagcggtt tttgaccgga gattgctgcc aggtctgctg ctgcgttatc 360
cagcagtttt ttgtcagcga tcgcttcacc aacacccatg ttcagggtga tcttctcgac 420
ccgagggact tgcatgacag aattgtagtt aaactcagtc atgagttttt taactacttc 480
gtctttgtag taatcatgca gtttcgccat cgtactactc catgtcggtg aacgctctcc 540
tgagtaggac aaatccgccg ggagcggatt tgaacgttgc gaagcaacgg cccggagggt 600
ggcgggcagg acgcccgcca taaactgcca ggcatcaaat taagcagaag gccatcctga 660
cggatggcct ttttgcgttt ctacaaactc ttttggttat ttttctaaat cattcaaata 720
tgtatccgnt catcccatcc tatcgatgat aagctgtcaa acatgagaat ttaatcaatc 780
taaagtttta tggngttaaa cttgggctgg cagnttncca atggcttaat cagtngaggg 840
ccctatntta acgaactngg ctantttngg tcaatcn 877

<210> 53
<211> 291

<212> DNA
<213> E. Coli

<400> 53
tgaacagcag agatacggcc agtgcggcca atgttttttg tcctttaaac ataacagagt 60
cctttaagga tatagaatag gggtatagct acgccagaat atcgtatttg attattgcta 120
gtttttagtt ttgcttaaaa atattgttag ttttattaaa tgcaaaacta aattattggt 180
atcatgaatt tgttgtatga tgaataaaat ataggggggt atagatagac gtcattttca 240
taggcttata aatgcgacta ccatgaagtt tttaattgaa agtattgggt t 291

<210> 54
<211> 282
<212> DNA
<213> E. Coli

<400> 54
ttattaaatg caaaactaaa ttattggtat catgaatttg ttgtatgatg aataaaatat 60
aggggggtat agatagacgt cattttcata gggttataaa tgcgactacc atgaagtttt 120
taattgaaag tattgggttg ctgataattt gagctgttct attcttttta aatatctata 180
taggtctgtt aatggatttt atttttacaa ttttttgtgt ttaggcatat aaaaatcaac 240
ccgccatatg aacggcgggt taaaatattt acaacttagc aa 282

<210> 55
<211> 293
<212> DNA
<213> E. Coli

<220>
<221> misc_feature
<222> (1)...(293)
<223> n = A,T,C or G

<400> 55
cggggtccgg cgctcatcaa caatcggggg gcagcaaggg gctgaaacgg gaaagcccct 60
cccgaagaag gggccttgta taaggaaagg gttatgatga agctcgtcat catactggtt 120
gtgtngttac tgttaagttt cccgacttac taacaactca tcagaggggg gagaaatcct 180
cccttaccct tgttccttta ctctaggttg aaaaaacaac agcgtcaata ggcctgccat 240
gtacgaagcg agatctgtga accgctttcc ggttagcctt ttttatcctg ttg 293

<210> 56
<211> 300
<212> DNA
<213> E. Coli

<400> 56
   tctgcgttcc gctaaaaggt gcaaatgctc aggacgttgc agcgttttgc gtgaccgctc 60
   ggggaaggca aaattgcctc tgggaaagca ttgcgcgggg tccggcgctc atcaacaatc 120
   ggggggcagc aaggggctga aacgggaaag cccctcccga agaaggggcc ttgtataagg 180
   aaagggttat gatgaagctc gtcatcatac tggttgtgtt gttactgtta agtttcccga 240
   cttactaaca actcatcaga ggggggagaa atcctccctt acccttgttc ctttactcta 300

<210> 57
<211> 359
<212> DNA
<213> E. Coli

<400> 57
caacacagga ggctgggaat gccgcagaaa tatagattac tttctttaat agtgatttgt 60
ttcacgcttt tatttttcac ctggatgata agagattcac tgtgtgaatt gcatattaaa 120
caggagagtt atgagctggc ggcgttttta gcctgcaaat tgaaagagta agagtcttcg 180
gcgggaaatt attcccgcct tacttacggc gttgcgcatt ctcattgcac ccaaatttat 240

tcttcacaaa aataataata gattttatta cgcgatcgat tatttatttc ctgaaaacaa 300
ataaaaaaat ccccgccaaa tggcagggat cttagattct gtgcttttaa gcagagatt 359

<210> 58
<211> 700
<212> DNA
<213> E. Coli

<220>
<221> misc_feature
<222> (1)...(700)
<223> n = A,T,C or G

<400> 58
aaaccttttt ctcctgtttt tcatagaggg caacccatgt cctgacctgg gttcgggga 60
caccaaaacg tgccgagatg atcctgtaac catcatcagt tgtgaagtag tgattcacga 120
cttcaaggcg cttttcaaaa gggtattttg gctttgacat attaggggct attccatttc 180
atcgtccaac aaaatgggtg cagtacatac tcgttggaaa tcaacacagg aggctgggaa 240
tgccgcagaa atatagatta ctttctttaa tagtgatttg tttcacgctt ttatttttca 300
cctggatgat aagagattca ctgtgtgaat tgcatattaa acaggagagt tatgagctgg 360
cggcgttttt agcctgcaaa ttgaaagagt aagagtcttc ggcgggaaat tattcccgcc 420
ttacttacgg cgttgcgcat tctcattgca cccaaattta ttcttcacaa aaataataat 480
agattttatt acgcgatcga ttatttattt cctgaaaaca aataanaaaa tccccgccaa 540
atggcaggga tcttagattc tgtgctttta agcagagatt acaggctggt tacgttacca 600
gctgccgggc ctttaacgcc gctttcgatg gtgaaggaca ctttctgacc ttcgtccaga 660
gattgtaacc atcggtctgg atagccnaga aatgtccaac 700

<210> 59
<211> 631
<212> DNA
<213> E. Coli

<220>
<221> misc_feature
<222> (1)...(631)
<223> n = A,T,C or G

<400> 59
tggtggcatt ggttgctgga gagagaaaac ccccgcacgt tgcaggtatg cacctgacaa 60
caccacgggg gctaatcttg actctagacc actcaagaat agccgcgaaa cgttgtcatt 120
acaacacagg cggctatatg acgttcgcag agctgggcat ggccttctgg catgatttag 180
cggctccggt cattgctggc attcttgcca gtatgatcgt gaactggctg aacaagcgga 240
agtaacgtgt catgcgggcg tcaggctgcc gtaatggcaa tttgcgcccg gaccaggccg 300
caggggggaa actctgcggc ctttttcgtt cttactgcgg gtaaggcacc cagtcgccgc 360
cgttcaggcg aacgtacggt ttatcctggt attgaataac tactgcattt gagttctcgg 420
agaccggtgc tgtttgtggc aacccactgg tgagtttttt ccagtcaaca ttgtcttcgg 480
tgaaaatctt gccatcgaga acgcgaacca ccagatcgga gatagccagg aagctgctcg 540
gttgttcgat gacaatcggt gccccctgat gcggtgcctt catgccgaag aatttcaccc 600
caacggggac gtcngtgata gaccgggcta g 631

<210> 60
<211> 648
<212> DNA
<213> E. Coli

<220>
<221> misc_feature
<222> (1)...(648)
<223> n = A,T,C or G

<400> 60

ggctcaggcn tgctgattgt ttttttgtgc aatggcccng tattagcgtc gttgctgtcg 60
atggagagaa tcataaacgt ggtgaatgat gattgttagc aaggaaaact gtcaaaaatc 120
ttcaaaaaat ttgagggata aggccggaat ggctccggcc agagggaagt taaccgcgaa 180
gctgttgctg cttgagggtc gttttaacca gacgccaggc gctccatacg ccaaaaccgc 240
gtctggccca gcggaccagc atattaggat ggcgaatcgt ccagatcgcc atcacgctac 300
tgccaaccag cgcccaggag cgcagactta gcagcatatt ccancgacga tcgtaagcgc 360
ctgttgtctc cagccattca cgacgactgg cggaagggnc cgcgnctgac caacttgnct 420
tttagnctga tncanattan attnataaac gcagnanncn ggtntgatta atcntatttn 480
gctctngtct ggtagttagc nncggnnngt ctcnttntna cccnnttcnn tttannttac 540
natnngtaan ttatntttnt nngtctnant tntanttgng tactntaagt ntatncgnnn 600
atnntnnnan nnnncagnnc ntntttttta aatnntttnt nanncnnc 648

<210> 61
<211> 737
<212> DNA
<213> E. Coli

<220>
<221> misc_feature
<222> (1)...(737)
<223> n = A,T,C or G

<400> 61
tgctaatatc tttctcattg agatgaaaat taaggtaagc gaggaaacac accacaccat 60
aaacggaggc aaataatgct gggtaatatg aatgttttta tggccgtact gggaataatt 120
ttattttctg gttttctggc cgcgtatttc agccacaaat gggatgacta atgaacggag 180
ataatccctc acctaaccgg ccccttgtta cagttgtgta caaggggcct gatttttatg 240
acggcgaaaa aaaaccgcca gtaaaccggc ggtgaatgct tgcatggata gatttgtgtt 300
ttgcttttac gctaacaggc attttcctgc actgataacg aatcgttgac acagtagcat 360
cagttttctc aatgaatgtt aaacggagct taaactcggt taatcacatt ttgttcgtca 420
ataaacatgc agcgatttct tccggtttgc ttaccctcat acattgcccg gtccgctctt 480
ccaatgacca catccagagg ctcttcagga aatgcgcgac tcacacctgc tgtcacggta 540
atgttgatat gcccttcaga atgtgtgatg gcatggttat cgactaactg gcaaattctg 600
acacctgcac gacatgcttc ttcatcatta gccgctttga caataatgat aaattcttcg 660
cccccgtagc gataaaccgt ttcgtaatna cgcgtccaac tgggntaagt aaagttgcca 720
gggtgccgta atcttac 737

<210> 62
<211> 648
<212> DNA
<213> E. Coli

<220>
<221> misc_feature
<222> (1)...(648)
<223> n = A,T,C or G

<400> 62
tgcttttgaa tatgtgctcg caatcttgag aaggaaatgg cgaccacgaa agaaaaggca 60
aaaaccgata atctgaaaga acccaagtat ttcagtataa gcattgaatg ccgaccagta 120
aactctttcg gattcaccca gaaagtgaan ccaaaatgat aatcgtatac ataagtcttt 180
cgagtggctc gttagcaaaa agtttcaaca atggagtaaa tacatccaac atatcaataa 240
ctctcaactg taaggggatt gaaatggtaa ccccagctct tcgcttgagg ggtatagccg 300
agaccaccga agccccggag gtggtgaaat aaaaccgggc acaacacgaa agggcgcatt 360
tccgatatcc ataaaagaag tcgggtcttt gtctggtaaa attaaattgg tgggaagtgc 420
gcctccgggt tgtaaatacc gactttgctg ggtgtagcct ggcggcatca agtttttttc 480
tggaagttcg ctgatgtccg ccctttttaa agggaatttt ggtgatgccg gtgaatgccg 540
cttaaccccc cgtgggccca gttaaaagtc atggtaagnc ctaatnggtt tggggtggga 600
aaagccnact gnnaattggt tacctggttt gcaagtancc ctggaagg 648

<210> 63

<211> 237
<212> DNA
<213> E. Coli

<220>
<221> misc_feature
<222> (1)...(237)
<223> n = A,T,C or G

<400> 63
ggtgtttant tacaagagat tcatctttgt ntaaancccn gataagtaat tacgcataaa 60
acaacaatga ttataatagc aaaaataaat attatcatct ttgatagatt acttgagata 120
gccagcatct tgtaaagcct ttatcgtttt tttatgctct ggattaatat aatcactaca 180
tctatctgag caatctgttg ttgatggaca tgtcaaccca tggtcattta cagccaa 237

<210> 64
<211> 427
<212> DNA
<213> E. Coli

<400> 64
gataattaga gtttgtcgtc agaaaattga cgttacccat aacaaatgaa aggccaggta 60
aatcatgcca ttagtcattg ttgctatcgg tgtaatcttg ttgttgctcc tgatgatccg 120
cttcaaaatg aacggcttca tcgctctcgt cctcgtggcg cttgctgttg gattaatgca 180
aggaatgccg ctggataaag ttattggctc catcaaagcc ggtgtcggcg ggacgctcgg 240
tagccttgcc ctgatcatgg gttttggcgc aatgctgggc aaaatgctgg cagactgcgg 300
tggcgcacaa cgtatcgcca ccacgctgat tgccaaattt ggtaaaaaac acatccagtg 360
ggcggtggta ctgaccggtt ttaccgttgg ttttgccctg ttctatgaag tgggctttgt 420
gctgatg 427

<210> 65
<211> 261
<212> DNA
<213> E. Coli

<220>
<221> misc_feature
<222> (1)...(261)
<223> n = A,T,C or G

<400> 65
caaagaacct tcaacatgaa aaatatccat ttgtttgcaa aaaaagatta ttaggaagga 60
aattaatgca attatcgaaa attcaaaaaa tatccaaaaa tngtatactt tattccagaa 120
gagttcaata taatgtttgt cttcaatttt tcttacttca gggtaatata gattgctcat 180
tacattgtga gcttcatctt tatttaattt tctgttgact ccagctctcc gtgataacgg 240
ttttataatt agatgcttat c 261

<210> 66
<211> 98
<212> DNA
<213> E. Coli

<400> 66
agatgattgc cgggaacttg ttagcggcac gcaggcggcg gctcgcaccc ttaccctgct 60
ctttacgtac ttctgcgttg atagtaaaca tttctttc 98

<210> 67
<211> 260
<212> DNA
<213> E. Coli

<400> 67
aagcgcgaac gaagtcgatg tgctgcagct tcggtttgta cgggtgacgc tgtacgtcct 60
gagctttaac tttgatttct ttaccgtcaa caacgatggt cagaacttcg ctgtagaatt 120
cagctttagc ttgcatgttc atgactttgt cgtgatccag ctcgatagcc agcggcgctt 180
ctttgccacc gtagatgatt gccgggaact tgttagcggc acgcaggcgg cggctcgcac 240
ccttaccctg ctctttacgt 260

<210> 68
<211> 95
<212> DNA
<213> E. Coli

<400> 68
aaaaacggcg taaagaaagg ttgcaaacat gttaataaaa actcaaattg atcccacgta 60
tatattacgc cgcaaaatcc ttacaataaa caggg 95

<210> 69
<211> 174
<212> DNA
<213> E. Coli

<400> 69
ttaattatta aaatagtgta acgcgattat gtggttatgg gggtaaacat taaataaacc 60
agcggggagg ggaggtaaag tgaaaaaata aaaagcggat aatcttaata agcaggccgg 120
acagcatcgc catccggcac tgatacgagg tttatttcag ctcatcaacc atcg 174

<210> 70
<211> 138
<212> DNA
<213> E. Coli

<400> 70
agtctgtaaa aacgtcaaaa agagtgtttt atcaacagaa gaatggaggt ctgacagata 60
gtagtaatgc aaaaaaatgg agacttaagt tgaatgaacg ggagtaaagc gaaaagacta 120
tagagtgaag gagaaatt 138

<210> 71
<211> 191
<212> DNA
<213> E. Coli

<400> 71
tttgttggct taatattcta ttgttatctt tatttataga tgtttatatt gcatgaggtg 60
gtttttggag agaagaatga ggaagatgcg tcgagccaca gaaacgttag ctttacatat 120
agcggaggtg atgtgaattt aatttacaat agaaataatt tacatatcaa acagttagat 180
gcttttgtcg g 191

<210> 72
<211> 244
<212> DNA
<213> E. Coli

<400> 72
ggccatttat acaggaaaag cctatgtcag aacgtaaaaa ctcaaaatca cgccgtaatt 60
atctcgttaa atgttcctgc ccaaactgca cccaagagtc agaacacagt ttttcaagag 120
tacaaaaagg tgcccttttg atctgccctc attgcaacaa agtattccag acaaatctta 180
aagctgtagc ctgattgatt ttattagtaa caagtatttt ttatatttta ataatatatt 240
taaa 244

<210> 73
<211> 327

<212> DNA
<213> E. Coli

<220>
<221> misc_feature
<222> (1)...(327)
<223> n = A,T,C or G

<400> 73
aaattttcag gtaccttgtc accatacttt tttttctgag cattaatgat attttgagct 60
tcttgaggat ctttaactcc ccacatttgg tggaaagtat tcatattaaa aggaaggntg 120
aataatttgn ctttataaat cgccagtgga gaattagtaa aacgattaaa ttctactaaa 180
tnattaaccg naaaaaaatt cccatatata tttatcattg gtatgaaaaa tatgtgcacc 240
atatttatga atntggatac cctnacagtc ctctgtgtac gcatttccac cgatatgatt 300
tcttttctna atcactaaaa ctttttt 327

<210> 74
<211> 150
<212> DNA
<213> E. Coli

<400> 74
gcagtgatcg aagcgatgac gaagtgtatg gaaaaatcag aaaaactcag caaatcctga 60
tgactttcgc cggacgtcag gccgccactt cggtgcggtt acgtccggct ttctttgctt 120
tgtaaagcgc caaatctgcc gatttcaacc 150

<210> 75
<211> 330
<212> DNA
<213> E. Coli

<400> 75
gaaagtatct tcgttattga catcactgga aaatataact tgcttttcat tattaaactc 60
gaagcgcgta ccgtatctgg acaaacattt atcgagctta ccaaattcct gaagaggttt 120
aactacagat aacatttgcg cgtcctttgc agtaatgccc gtcaaatcct tgacgggcat 180
tatttagatt aaattaccag tatttcttcg gagtgaagaa tattaccagg tatatttaac 240
acccacgttc gcggaccagt cttgatctac gtcaccacca ccgaggtagt tagcatcggt 300
ataggcgctg aagttcttgg tgaagctaaa 330

<210> 76
<211> 194
<212> DNA
<213> E. Coli

<400> 76
tgtttttttc cagcaacgga gcaaaaggtt tgcccttgtg cagctcaggg ttaaccactt 60
taactacgtg gcgacgaccc ggagatgtcg gtttacattt aacaactgcc attgtattac 120
tcctccgact tactcagcgc cgccaacgaa gtccagattc tggccttctt tcagggtgac 180
gtaagctttt ttcc 194

<210> 77
<211> 188
<212> DNA
<213> E. Coli

<400> 77
tccctttaac taccagggtg ttaacgactt cgacttcgac ttcaaacagt ttctgcacag 60
cagctttgat ttctgctttg gtcgcgtctt tagcaacttt gagtactatg gtgttggatt 120
tttccatcgc agtagacgct ttttcagaaa cgtgcggtgc acgcagcacc ttcagcagac 180
gttcttca 188

<210> 78
<211> 173
<212> DNA
<213> E. Coli

<400> 78
acaaaggcga acaaagcctg tgaagcccga aggctccaca gacagtgcta cttgaaggcc 60
ttactgtttc ttcttaggag cgagcaccat gatcatctgg cggccttcga tcttggttgg 120
gaaggattcg accactgcca gttcttgcaa atcgtctttc acgcgattaa gca 173

<210> 79
<211> 272
<212> DNA
<213> E. Coli

<220>
<221> misc_feature
<222> (1)...(272)
<223> n = A,T,C or G

<400> 79
tggagaaaac gggtgattga taaagcaatc atcgttctag gggcgttaat tgcgctgctg 60
gaactgatcc cgctttctgc ttcaagcttc tgaactggat acggaaacgt aatnagggct 120
aaagaagaca ctactcttag ccctttaaca tttaacgcat tgtcacgaac tcttctgccg 180
ccgttgggtg aatggcgacg ggtattggtc gaaatctttt ttgggtggcc ccatctttaa 240
cgcccacccg cgaaaccctg caacatttcg tc 272

<210> 80
<211> 259
<212> DNA
<213> E. Coli

<400> 80
cgcaggcagc tgatggtcaa caggatgaga gaaacccaga gacaggttaa tcacattgcc 60
tttaaccgct gcacggtaac ctacaccaac cagctgcagc ttcttagtga agccttcggt 120
aacaccgata accattgagt tcagcagggc acgcgcggta ccagcctgtg cccaaccgtc 180
tgcgtaacca tcacgcggac cgaaggtcag ggtattatct gcatgtttaa cttcaacagc 240
atcgttgaga gtacgagtc 259

<210> 81
<211> 73
<212> DNA
<213> E. Coli

<400> 81
caggtcggaa cttacccgac aaggaatttc gctaccttag gaccgttata gttacggccg 60
ccgtttaccg ggg 73

<210> 82
<211> 666
<212> DNA
<213> E. Coli

<400> 82
atgaacgttt tctcgcaaac tcaacgctat aaggcgttgt tctggttatc gttatttcat 60
ctgctggtga tcacctccag taactatctg gttcagcttc ccgtctccat tttgggtttc 120
cataccacct ggggcgcgtt tagctttccg tttatttttc ttgctaccga cctgaccgtg 180
cgtatttttg gcgcaccgct ggcccgacgc attatcttcg cggtaatgat ccctgcgtta 240
ttaatctcct acgtcatctc gtcgctattc tatatgggtt cctggcaggg attcggcgca 300
ctcgcccact tcaacctgtt tgtcgcccgt atcgccaccg ccagtttcat ggcctacgcg 360
ctggggcaaa tcctcgacgt gcacgttttt aaccgcctgc gtcagagtcg ccgctggtgg 420

ctggcaccga cagcgtccac actgttcggt aacgtcagcg acacgctggc ctttttcttc 480
attgccttct ggcgtagccc ggatgccttt atggctgaac actggatgga aatcgcgctg 540
gtcgattact gtttcaaagt gttaatcagt atcgttttct tcctgccaat gtatggcgta 600
ttactcaata tgctgttgaa aagactggca gataaatccg aaatcaacgc tttgcaggcg 660
agttaa 666

<210> 83
<211> 612
<212> DNA
<213> E. Coli

<400> 83
gtgataagat ggatgaatga gccgttatgg ccgtttatcg aaaggaagaa gtcaatgcgc 60
aatctggtta aatatgtcgg aattggcctg ctggttatgg ggcttgcggc ctgtgatgat 120
aaagacacta acgctacggc gcagggttcg gtcgcggaaa gtaacgctac cgggaatccc 180
gtcaacctgc ttgatggcaa gttaagtttc tcgctgccag cggatatgac cgaccagagc 240
ggtaagctgg gaacgcaggc caataacatg catgtctggt ccgacgccac cgggcagaaa 300
gcagtcatcg tcatcatggg cgatgatccg aaagaagatc tggcggtgct ggcgaagcgt 360
ctggaagatc agcaacgtag ccgcgatccg cagctgcaag tggtaaccaa taaagccatt 420
gagctgaaag gtcacaaaat gcagcagtta gacagtatta tctccgcgaa aggccagacg 480
gcgtactctt ccgttattct gggtaacgtg ggtaatcaac tgctgaccat gcaaattacg 540
ctgcccgctg acgatcagca aaaagcgcag accaccgcag aaaacatcat taatacgctg 600
gttattcagt aa 612

<210> 84
<211> 975
<212> DNA
<213> E. Coli

<400> 84
atggcgaata tgtttgccct gattctggtg attgccacac tggtgacggg cattttatgg 60
tgcgtggata aattcttttt cgcacctaaa cggcgggaac gtcaggcagc ggcgcaggcg 120
gctgccgggg actcactgga taaagcaacg ttgaaaaagg ttgcgccgaa gcctggctgg 180
ctggaaaccg gtgcttctgt ttttccggta ctggctatcg tattgattgt gcgttcgttt 240
atttatgaac cgttccagat cccgtcaggt tcgatgatgc cgactctgtt aattggtgat 300
tttattctgg tagagaagtt tgcttatggc attaaagatc ctatctacca gaaaacgctg 360
atcgaaaccg gtcatccgaa acgcggcgat atcgtggtct ttaaatatcc ggaagatcca 420
aagcttgatt acatcaagcg cgcggtgggt ttaccgggcg ataaagtcac ttacgatccg 480
gtctcaaaag agctgacgat tcaaccggga tgcagttccg gccaggcgtg tgaaaacgcg 540
ctgccggtca cctactcaaa cgtggaaccg agcgatttcg ttcagacctt ctcacgccgt 600
aatggtgggg aagcgaccag cggattcttt gaagtgccga aaaacgaaac caaagaaaat 660
ggaattcgtc tttccgagcg taaagagaca ctgggtgatg tgacgcaccg cattctgaca 720
gtgccgattg cgcaggatca ggtggggatg tattaccagc agccagggca acaactggca 780
acctggattg ttcctccggg acaatacttc atgatgggcg acaaccgcga caacagcgcg 840
gacagccgtt actggggctt tgtgccggaa gcgaatctgg tcggtcgggc aacggctatc 900
tggatgagct tcgataagca agaaggcgaa tggccgactg gtctgcgctt aagtcgcatt 960
ggcggcatcc attaa 975

<210> 85
<211> 1761
<212> DNA
<213> E. Coli

<400> 85
ttgaccatta cgaaacttgc atggcgtgac ctggttcctg ataccgatag ctatcaggaa 60
atatttgctc agccacattt gattgacgaa aacgatcctt tattcagtga tactcaaccg 120
cggctgcaat ttgcgctgga gcagttgctg catacgcgag catcctcctc ttttatgctg 180
gcgaaggccc cggaagagtc tgagtatctg aatcttattg ccaatgccgc gcgtacgcta 240
caaagcgatg caggccaact ggtgggcggt cactatgagg tttccggcca ctccatccgc 300
ttacgtcacg cagtgagtgc agatgataat tttgcgactt taacgcaagt tgtcgctgcc 360
gactgggtag aagcggagca actctttggc tgcctgcgcc agtttaatgg cgacattacc 420

ctgcagcctg gtctggtgca tcaggcaaat ggcggtattc tcattatctc tttgcgtaca 480
ctgctggcgc aacctctgct gtggatgcgg ctgaaaaata tcgttaaccg cgagcgtttt 540
gactgggttg cgtttgatga gtcgcgccct ctccccgtct ctgtgccttc gatgccattg 600
aagctgaaag tcattctggt aggcgaacgc gaatcattgg ctgatttcca ggagatggag 660
ccagagcttt cagagcaggc tatttatagc gaatttgaag atactctgca gattgtcgat 720
gcggagtcag taacccagtg gtgtcgctgg gtgacattta ccgccagaca taatcactta 780
cctgcaccgg gagcggatgc ctggccgata cttatccgcg aagcagcacg ctacaccggt 840
gaacaagaaa cacttccgct tagcccgcag tggatcctcc gccagtgtaa agaggtcgcc 900
tccctgtgtg atggcgacac cttctccggc gagcagctaa acttaatgct gcagcagcgt 960
gaatggcgcg aaggtttcct cgctgaacgt atgcaggatg agatccttca ggagcaaatc 1020
ctgattgaaa ccgaaggcga acgcatcggg caaattaacg ccctttcggt cattgaattt 1080
ccgggtcatc cacgcgcttt tggcgaacct tctcgcatta gctgcgttgt gcatattggc 1140
gatggtgaat tcaccgacat cgaacgcaaa gcggagcttg gcggcaatat ccatgcgaaa 1200
gggatgatga tcatgcaagc gttcctgatg tcggaactac agcttgagca acagatcccc 1260
ttctcagcat cgctgacatt tgagcagtca tacagtgaag ttgatggaga tagtgcctcg 1320
atggctgaac tctgcgccct gataagcgcc ctcgccgatg tgccggtgaa tcagagtatc 1380
gctatcacag gttcagtcga tcagttcggt cgcgcccagc cggtcggtgg tttaaatgag 1440
aaaatcgaag gcttctttgc tatttgccag caacgtgagt taaccgggaa acaaggtgtc 1500
attatcccca cagctaacgt tcgccattta agtcttcaca gtgaactggt gaaagcggta 1560
gaagaaggca aattcaccat ctgggcagta gacgatgtga ctgacgcact gccgttatta 1620
ttaaatctgg tgtgggatgg cgaaggccaa acgacgctga tgcaaaccat ccaggaacgt 1680
atcgcgcaag catcgcaaca ggaaggacgt caccgttttc catggccatt acgttggctg 1740
aactggttta ttccgaactg a 1761

<210> 86
<211> 1185
<212> DNA
<213> E. Coli

<400> 86
gtgtctaaag aaaaatttga acgtacaaaa ccgcacgtta acgttggtac tatcggccac 60
gttgaccacg gtaaaactac tctgaccgct gcaatcacca ccgtactggc taaaacctac 120
ggcggtgctg ctcgtgcatt cgaccagatc gataacgcgc cggaagaaaa agctcgtggt 180
atcaccatca acacttctca cgttgaatac gacaccccga cccgtcacta cgcacacgta 240
gactgcccgg ggcacgccga ctatgttaaa aacatgatca ccggtgctgc tcagatggac 300
ggcgcgatcc tggtagttgc tgcgactgac ggcccgatgc cgcagactcg tgagcacatc 360
ctgctgggtc gtcaggtagg cgttccgtac atcatcgtgt tcctgaacaa atgcgacatg 420
gttgatgacg aagagctgct ggaactggtt gaaatggaag ttcgtgaact tctgtctcag 480
tacgacttcc cgggcgacga cactccgatc gttcgtggtt ctgctctgaa agcgctggaa 540
ggcgacgcag agtgggaagc gaaaatcctg gaactggctg gcttcctgga ttcttatatt 600
ccggaaccag agcgtgcgat tgacaagccg ttcctgctgc cgatcgaaga cgtattctcc 660
atctccggtc gtggtaccgt tgttaccggt cgtgtagaac gcggtatcat caaagttggt 720
gaagaagttg aaatcgttgg tatcaaagag actcagaagt ctacctgtac tggcgttgaa 780
atgttccgca aactgctgga cgaaggccgt gctggtgaga acgtaggtgt tctgctgcgt 840
ggtatcaaac gtgaagaaat cgaacgtggt caggtactgg ctaagccggg caccatcaag 900
ccgcacacca agttcgaatc tgaagtgtac attctgtcca aagatgaagg cggccgtcat 960
actccgttct tcaaaggcta ccgtccgcag ttctacttcc gtactactga cgtgactggt 1020
accatcgaac tgccggaagg cgtagagatg gtaatgccgg gcgacaacat caaaatggtt 1080
gttaccctga tccacccgat cgcgatggac gacggtctgc gtttcgcaat ccgtgaaggc 1140
ggccgtaccg ttggcgcggg cgttgttgct aaagttctgg gctaa 1185

<210> 87
<211> 2115
<212> DNA
<213> E. Coli

<400> 87
atggctcgta caacacccat cgcacgctac cgtaacatcg gtatcagtgc gcacatcgac 60
gccggtaaaa ccactactac cgaacgtatt ctgttctaca ccggtgtaaa ccataaaatc 120
ggtgaagttc atgacggcgc tgcaaccatg gactggatgg agcaggagca ggaacgtggt 180
attaccatca cttccgctgc gactactgca ttctggtctg gtatggctaa gcagtatgag 240

ccgcatcgca tcaacatcat cgacaccccg gggcacgttg acttcacaat cgaagtagaa 300
cgttccatgc gtgttctcga tggtgcggta atggtttact gcgcagttgg tggtgttcag 360
ccgcagtctg aaaccgtatg gcgtcaggca aacaaatata aagttccgcg cattgcgttc 420
gttaacaaaa tggaccgcat gggtgcgaac ttcctgaaag ttgttaacca gatcaaaacc 480
cgtctgggcg cgaacccggt tccgctgcag ctggcgattg gtgctgaaga acatttcacc 540
ggtgttgttg acctggtgaa aatgaaagct atcaactgga acgacgctga ccagggcgta 600
accttcgaat acgaagatat cccggcagac atggttgaac tggctaacga atggcaccag 660
aacctgatcg aatccgcagc tgaagcttct gaagagctga tggaaaaata cctgggtggt 720
gaagaactga ctgaagcaga aatcaaaggt gctctgcgtc agcgcgttct gaacaacgaa 780
atcatcctgg taacctgtgg ttctgcgttc aagaacaaag gtgttcaggc gatgctggat 840
gcggtaattg attacctgcc atccccggtt gacgtacctg cgatcaacgg tatcctggac 900
gacggtaaag acactccggc tgaacgtcac gcaagtgatg acgagccgtt ctctgcactg 960
gcgttcaaaa tcgctaccga cccgtttgtt ggtaacctga ccttcttccg tgtttactcc 1020
ggtgtggtta actctggtga taccgtactg aactccgtga aagctgcacg tgagcgtttc 1080
ggtcgtatcg ttcagatgca cgctaacaaa cgtgaagaga tcaaagaagt tcgcgcgggc 1140
gacatcgctg ctgctatcgg tctgaaagac gtaaccactg gtgacaccct gtgtgacccg 1200
gatgcgccga tcattctgga acgtatggaa ttccctgagc cggtaatctc catcgcagtt 1260
gaaccgaaaa ccaaagctga ccaggaaaaa atgggtctgg ctctgggccg tctggctaaa 1320
gaagacccgt ctttccgtgt atggactgac gaagaatcta accagaccat catcgcgggt 1380
atgggcgaac tgcacctcga catcatcgtt gaccgtatga agcgtgaatt caacgttgaa 1440
gcgaacgtag gtaaaccgca ggttgcttac cgtgaaacta tccgccagaa agttaccgat 1500
gttgaaggta aacacgcgaa acagtctggt ggtcgtggtc agtatggtca tgttgttatc 1560
gacatgtacc cgctggagcc gggttcaaac ccgaaaggct acgagttcat caacgacatt 1620
aaaggtggtg taatccctgg cgaatacatc ccggccgttg ataaaggtat ccaggaacag 1680
ctgaaagcag gtccgctggc aggctacccg gtagtagaca tgggtattcg tctgcacttc 1740
ggttcttacc atgacgttga ctcctctgaa ctggcgttta aactggctgc ttctatcgcc 1800
tttaaagaag gctttaagaa agcgaaacca gttctgcttg agccgatcat gaaggttgaa 1860
gtagaaactc cggaagagaa caccggtgac gttatcggtg acttgagccg tcgtcgtggt 1920
atgctcaaag gtcaggaatc tgaagttact ggcgttaaga tccacgctga agtaccgctg 1980
tctgaaatgt tcggatacgc aactcagctg cgttctctga ccaaaggtcg tgcatcatac 2040
actatggaat tcctgaagta tgatgaagcg ccgagtaacg ttgctcaggc cgtaattgaa 2100
gcccgtggta aataa 2115

<210> 88
<211> 540
<212> DNA
<213> E. Coli

<400> 88
atgccacgtc gtcgcgtcat tggtcagcgt aaaattctgc cggatccgaa gttcggatca 60
gaactgctgg ctaaatttgt aaatatcctg atggtagatg gtaaaaaatc tactgctgaa 120
tctatcgtat acagcgcgct ggagaccctg gctcagcgct ctggtaaatc tgaactggaa 180
gcattcgaag tagctctcga aaacgtgcgc ccgactgtag aagttaagtc tcgccgcgtt 240
ggtggttcta cttatcaggt accagttgaa gtccgtccgg ttcgtcgtaa tgctctggca 300
atgcgttgga tcgttgaagc tgctcgtaaa cgcggtgata aatccatggc tctgcgcctg 360
gcgaacgaac tttctgatgc tgcagaaaac aaaggtactg cagttaagaa acgtgaagac 420
gttcaccgta tggccgaagc caacaaggcg ttcgcacact accgttggtt atcccttcgg 480
agttttagtc accaggcggg cgcttccagt aagcagcccg ctttgggcta cttaaattga 540

<210> 89
<211> 1549
<212> DNA
<213> E. Coli

<400> 89
aaattgaaga gtttgatcat ggctcagatt gaacgctggc ggcaggccta acacatgcaa 60
gtcgaacggt aacaggaagc agcttgctgc ttcgctgacg agtggcggac gggtgagtaa 120
tgtctgggaa gctgcctgat ggagggggat aactactgga aacggtagct aataccgcat 180
aatgtcgcaa gaccaaagag ggggaccttc gggcctcttg ccatcggatg tgcccagatg 240
ggattagctt gttggtgggg taacggctca ccaaggcgac gatccctagc tggtctgaga 300
ggatgaccag ccacactgga actgagacac ggtccagact cctacgggag gcagcagtgg 360

ggaatattgc acaatgggcg caagcctgat gcagccatgc cgcgtgtatg aagaaggcct 420
tcgggttgta aagtactttc agcggggagg aagggagtaa agttaatacc tttgctcatt 480
gacgttaccc gcagaagaag caccggctaa ctccgtgcca gcagccgcgg taatacggag 540
ggtgcaagcg ttaatcggaa ttactgggcg taaagcgcac gcaggcgggt tggttaagtc 600
agatgtgaaa tccccgggct caacctggga actgcatctg atactggcaa gcttgagtct 660
cgtagagggg ggtagaattc caggtgtagc ggtgaaatgc gtagagatct ggaggaatac 720
cggtggcgaa ggcggccccc tggacgaaga ctgacgctca ggtgcgaaag cgtggggagc 780
aaacaggatt agataccctg gtagtccacg ccgtaaacga tgtcgacttg gaggttgtgc 840
ccttgaggcg tggcttccgg agctaacgcg ttaagtcgac cgcctgggga gtacggccgc 900
aaggttaaaa ctcaaatgaa ttgacggggg cccgcacaag cggtggagca tgtggtttaa 960
ttcgatgcaa cgcgaagaac cttacctggt cttgacatcc acggaagttt tcagagatga 1020
gaatgtgcct tcgggaaccg tgagacaggt gctgcatggc tgtcgtcagc tcgtgttgtg 1080
aaatgttggg ttaagtcccg caacgagcgc aacccttatc ctttgttgcc agcggtccgg 1140
ccgggaactc aaaggagact gccagtgata aactggagga aggtggggat gacgtcaagt 1200
catcatggcc cttacgacca gggctacaca cgtgctacaa tggcgcatac aaagagaagc 1260
gacctcgcga gagcaagcgg acctcataaa gtgcgtcgta gtccggattg gagtctgcaa 1320
ctcgactcca tgaagtcgga atcgctagta atcgtggatc agaatgccac ggtgaatacg 1380
ttcccgggcc ttgtacacac cgcccgtcac accatgggag tgggttgcaa aagaagtagg 1440
tagcttaacc ttcgggaggg cgcttaccac tttgtgattc atgactgggg tgaagtcgta 1500
acaaggtaac cgtaggggaa cctgcggttg gatcacctcc ttaccttaa 1549

<210> 90
<211> 375
<212> DNA
<213> E. Coli

<400> 90
atggcaacag ttaaccagct ggtacgcaaa ccacgtgctc gcaaagttgc gaaaagcaac 60
gtgcctgcgc tggaagcatg cccgcaaaaa cgtggcgtat gtactcgtgt atatactacc 120
actcctaaaa aaccgaactc cgcgctgcgt aaagtatgcc gtgttcgtct gactaacggt 180
ttcgaagtga cttcctacat cggtggtgaa ggtcacaacc tgcaggagca ctccgtgatc 240
ctgatccgtg gcggtcgtgt taaagacctc ccgggtgttc gttaccacac cgtacgtggt 300
gcgcttgact gctccggcgt taaagaccgt aagcaggctc gttccaagta tggcgtgaag 360
cgtcctaagg cttaa 375

<210> 91
<211> 366
<212> DNA
<213> E. Coli

<400> 91
atgtctatca ctaaagatca aatcattgaa gcagttgcag ctatgtctgt aatggacgtt 60
gtagaactga tctctgcaat ggaagaaaaa ttcggtgttt ccgctgctgc tgctgtagct 120
gtagctgctg gcccggttga agctgctgaa gaaaaaactg aattcgacgt aattctgaaa 180
gctgctggcg ctaacaaagt tgctgttatc aaagcagtac gtggcgcaac tggcctgggt 240
ctgaaagaag ctaaagacct ggtagaatct gcaccggctg ctctgaaaga aggcgtgagc 300
aaagacgacg cagaagcact gaaaaaagct ctggaagaag ctggcgctga agttgaagtt 360
aaataa 366

<210> 92
<211> 498
<212> DNA
<213> E. Coli

<400> 92
atggctttaa atcttcaaga caaacaagcg attgttgctg aagtcagcga agtagccaaa 60
ggcgcgctgt ctgcagtagt tgcggattcc cgtggcgtaa ctgtagataa aatgactgaa 120
ctgcgtaaag caggtcgcga agctggcgta tacatgcgtg ttgttcgtaa caccctgctg 180
cgccgtgctg ttgaaggtac tccgttcgag tgcctgaaag acgcgtttgt tggtccgacc 240
ctgattgcat actctatgga acacccgggc gctgctgctc gtctgttcaa agagttcgcg 300
aaagcgaatg caaaatttga ggtcaaagcc gctgcctttg aaggtgagct gatcccggcg 360

tctcagatcg accgcctggc aactctgccg acctacgaag aagcaattgc acgcctgatg 420
gcaaccatga aagaagcttc ggctggcaaa ctggttcgta ctctggctgc tgtacgcgat 480
gcgaaagaag ctgcttaa 498

<210> 93
<211> 2145
<212> DNA
<213> E. Coli

<400> 93
gtgtcccgta ttattatgct gatccctacc ggaaccagcg tcggtctgac cagcgtcagc 60
cttggcgtga tccgtgcaat ggaacgcaaa ggcgttcgtc tgagcgtttt caaacctatc 120
gctcagccgc gtaccggtgg cgatgcgccc gatcagacta cgactatcgt gcgtgcgaac 180
tcttccacca cgacggccgc tgaaccgctg aaaatgagct acgttgaagg tctgctttcc 240
agcaatcaga aagatgtgct gatggaagag atcgtcgcaa actaccacgc taacaccaaa 300
gacgctgaag tcgttctggt tgaaggtctg gtcccgacac gtaagcacca gtttgcccag 360
tctctgaact acgaaatcgc taaaacgctg aatgcggaaa tcgtcttcgt tatgtctcag 420
ggcactgaca ccccggaaca gctgaaagag cgtatcgaac tgacccgcaa cagcttcggc 480
ggtgccaaaa acaccaacat caccggcgtt atcgttaaca aactgaacgc accggttgat 540
gaacagggtc gtactcgccc ggatctgtcc gagattttcg acgactcttc caaagctaaa 600
gtaaacaatg ttgatccggc gaagctgcaa gaatccagcc cgctgccggt tctcggcgct 660
gtgccgtgga gctttgacct gatcgcgact cgtgcgatcg atatggctcg ccacctgaat 720
gcgaccatca tcaacgaagg cgacatcaat actcgccgcg ttaaatccgt cactttctgc 780
gcacgcagca ttccgcacat gctggagcac ttccgtgccg gttctctgct ggtgacttcc 840
gcagaccgtc ctgacgtgct ggtggccgct tgcctggcag ccatgaacgg cgtagaaatc 900
ggtgccctgc tgctgactgg cggttacgaa atggacgcgc gcatttctaa actgtgcgaa 960
cgtgctttcg ctaccggcct gccggtattt atggtgaaca ccaacacctg gcagacctct 1020
ctgagcctgc agagcttcaa cctggaagtt ccggttgacg atcacgaacg tatcgagaaa 1080
gttcaggaat acgttgctaa ctacatcaac gctgactgga tcgaatctct gactgccact 1140
tctgagcgca gccgtcgtct gtctccgcct gcgttccgtt atcagctgac tgaacttgcg 1200
cgcaaagcgg gcaaacgtat cgtactgccg gaaggtgacg aaccgcgtac cgttaaagca 1260
gccgctatct gtgctgaacg tggtatcgca acttgcgtac tgctgggtaa tccggcagag 1320
atcaaccgtg ttgcagcgtc tcagggtgta gaactgggtg cagggattga aatcgttgat 1380
ccagaagtgg ttcgcgaaag ctatgttggt cgtctggtcg aactgcgtaa gaacaaaggc 1440
atgaccgaaa ccgttgcccg cgaacagctg gaagacaacg tggtgctcgg tacgctgatg 1500
ctggaacagg atgaagttga tggtctggtt tccggtgctg ttcacactac cgcaaacacc 1560
atccgtccgc cgctgcagct gatcaaaact gcaccgggca gctccctggt atcttccgtg 1620
ttcttcatgc tgctgccgga acaggtttac gtttacggtg actgtgcgat caacccggat 1680
ccgaccgctg aacagctggc agaaatcgcg attcagtccg ctgattccgc tgcggccttc 1740
ggtatcgaac cgcgcgttgc tatgctctcc tactccaccg gtacttctgg tgcaggtagc 1800
gacgtagaaa aagttcgcga agcaactcgt ctggcgcagg aaaaacgtcc tgacctgatg 1860
atcgacggtc cgctgcagta cgacgctgcg gtaatggctg acgttgcgaa atccaaagcg 1920
ccgaactctc cggttgcagg tcgcgctacc gtgttcatct tcccggatct gaacaccggt 1980
aacaccacct acaaagcggt acagcgttct gccgacctga tctccatcgg gccgatgctg 2040
cagggtatgc gcaagccggt taacgacctg tcccgtggcg cactggttga cgatatcgtc 2100
tacaccatcg cgctgactgc gattcagtct gcacagcagc agtaa 2145

<210> 94
<211> 1767
<212> DNA
<213> E. Coli

<400> 94
atgaataatt ctattaacca taaatttcat cacattagcc gggctgaata ccaggaattg 60
ttagccgttt cccgtggcga cgctgttgcc gattatatta ttgataatgt ctctattctc 120
gacctgatca atggcggaga aatttccggc ccaattgtga ttaaaggacg ttacattgcc 180
ggtgttggcg cagaatacac tgatgctccg gctttgcagc ggattgatgc tcgcggcgca 240
acggcggtgc cagggtttat tgatgctcac ctgcatattg aatccagcat gatgacgccg 300
gtcacttttg aaaccgctac cctgccgcgc ggcctgacga ccgttatttg cgaccctcat 360
gaaatcgtca acgtgatggg cgaagccgga ttcgcctggt ttgcccgctg tgccgaacag 420
gcaaggcaaa accagtactt acaggtcagc tcttgcgtac ccgccctgga aggctgcgat 480

gttaacggtg ccagttttac ccttgaacag atgctcgcct ggcgggacca tccgcaggtt 540
accggccttg cagaaatgat ggactaccct ggcgtaatta gcgggcagaa tgcgctgctc 600
gataaactgg atgcatttcg ccacctgacg ctggacggtc actgcccggg tttgggtggt 660
aaagaactta acgcctatat tactgcgggt attgaaaact gccacgaaag ttatcagctg 720
gaagaaggac gccggaaatt acaactcggc atgtcgttga tgatccgcga agggtccgct 780
gcccgcaatc tcaacgcgct ggcaccgttg atcaacgaat ttaacagccc gcaatgcatg 840
ctctgtaccg atgaccgtaa cccgtgggag atcgcccatg aaggacacat cgatgcctta 900
attcgccgcc tgatcgaaca acacaatgtg ccgctgcatg tggcatatcg cgtcgccagc 960
tggtcgacgg cgcgccactt tggtctgaat cacctcggct tactggcacc cggcaagcag 1020
gccgatatcg tcctgttgag cgatgcgcgt aaggtcacgg tgcagcaggt actggtgaaa 1080
ggcgagccga ttgatgcgca aaccttacag gcggaagagt cggcgagact ggcacaatcc 1140
gctccgccat atggcaacac cattgcccgc cagccagttt ccgccagcga ctttgccctg 1200
caatttacgc ccggaaaacg ctatcgggtc attgacgtca tccataacga attgattacg 1260
cactcccact ccagcgtcta cagcgaaaat ggttttgatc gcgatgatgt gagctttatt 1320
gccgtacttg agcgttacgg gcaacggctg gctccggctt gtggtttgct tggcggcttt 1380
ggactgaatg aaggtgcgct ggctgcgacg gtcagccatg acagccataa tattgtggtg 1440
atcggtcgca gtgccgaaga gatggcgctg gcggtcaatc aggtgattca ggatggcggc 1500
gggctgtgcg tggtacgtaa cggccaggta caaagtcatc tgccgttacc cattgccggg 1560
ctgatgagca ccgacacggc gcagtcgctg gcggaacaaa ttgacgcctt gaaagccgcc 1620
gcccgtgaat gcggtccgtt acccgatgag ccgtttattc agatggcgtt tctttctctg 1680
ccagtgatcc ccgcgctaaa actaaccagt caggggctat ttgatggcga gaagtttgcc 1740
ttcactacgc tggaagtcac ggaataa 1767

<210> 95
<211> 1227
<212> DNA
<213> E. Coli

<400> 95
atggcgtatt gcaatccggg cctggaatcc aggccgaata agagaaacgc cctccggcgt 60
catgtggtaa caggcatagg tatgaaaatc gtaatcgccc cagactctta taaagaaagt 120
ttatctgcca gcgaggttgc gcaggcgata gaaaaaggat ttcgggaaat ttttcctgat 180
gcacagtacg tttctgttcc ggttgccgac ggtggcgaag gaacggtgga agcgatgatt 240
gcagccaccc agggggctga acgtcacgcc tgggttacag ggccgctggg cgagaaagtg 300
aatgccagtt gggggatctc cggcgatggc aaaaccgcgt ttattgaaat ggcggcggcc 360
agtgggctgg agctggtacc tgcggaaaaa cgcgatccac tcgtgaccac ttcacgcggc 420
acaggcgagt taatcctgca ggcgctggag agcggtgcga caaacattat tatcggcatt 480
ggcggcagcg ctacaaatga tggcggcgca ggcatggtac aggcgctggg ggcgaaatta 540
tgcgacgcca acggcaatga aattggtttt ggcggcggta gtcttaatac tctgaatgat 600
attgatattt ccggcctcga tccgcgctta aaagattgcg tcattcgcgt cgcttgtgat 660
gtcaccaatc cgctggtggg cgataacggc gcatcgcgca tctttggccc acaaaaggga 720
gccagtgaag cgatgattgt tgagctggac aataacctct ctcactatgc cgaggtcatt 780
aaaaaagcgc tgcatgttga tgtgaaagat gtccccggtg caggagctgc gggtggtatg 840
ggcgcggcgc taatggcgtt tcttggtgcg gaactgaaaa gtggtattga aatcgtcact 900
acggcgctga atctggagga acatattcac gattgtacgc tggtgatcac cggtgaaggg 960
cgtattgaca gccagagtat tcacgggaag gtaccgattg gtgtcgcaaa cgtggcgaag 1020
aagtaccata aaccggtgat tggcattgcg ggtagcctga ccgatgatgt tggcgttgta 1080
catcagcatg gcattgatgc ggtcttcagc gtattgacca gcataggtac gttggacgaa 1140
gcattccgcg gggcttatga caatatctgc cgtgcttcac gtaatatcgc cgcgacactg 1200
gcgattggaa tgcgcaacgc ggggtga 1227

<210> 96
<211> 900
<212> DNA
<213> E. Coli

<400> 96
atgattgata tgactatgaa agttggtttt attggcctgg ggattatggg taaaccaatg 60
agtaaaaacc ttctgaaagc aggttactcg ctggtggttg ctgaccgtaa cccagaagct 120
attgctgacg tgattgctgc aggtgcagaa acagcgtcta cggctaaagc gatcgctgaa 180
cagtgcgacg tcatcataac catgctgcca aactcccctc atgtgaaaga ggtggcgctg 240

ggtgagaatg gcattattga aggcgcgaag ccaggtacgg tattgatcga tatgagttct 300
atcgcaccgc tggcaagccg tgaaatcagc gaagcgctga aagcgaaagg cattgatatg 360
ctggatgctc cggtgagcgg cggtgaaccg aaagccatcg acggtacgct gtcagtgatg 420
gtgggcggcg acaaggctat tttcgacaaa tactatgatt tgatgaaagc gatggcgggt 480
tccgtggtgc ataccgggga aatcggtgca ggtaacgtca ccaaactggc aaatcaggtc 540
attgtggcgc tgaatattgc cgcgatgtca gaagcgttaa cgctggcaac taaagcgggc 600
gttaacccgg acctggttta tcaggcaatt cgcggtggac tggcgggcag taccgtgctg 660
gatgccaaag cgccgatggt gatggaccgc aacttcaagc cgggcttccg tattgatctg 720
catattaagg atctggcgaa tgcgctggat acttctcacg gcgtcggcgc acaactgccg 780
ctcacagctg cggttatgga gatgatgcag gcactgcgag cagatggttt aggaacggcg 840
gatcatagcg ccctggcgtg ctactacgaa aaactggcga aagtcgaagt tactcgttaa 900

<210> 97
<211> 771
<212> DNA
<213> E. Coli

<400> 97
atgaataacg atgttttccc gaataaattc aaagccgcac tggctgcgaa acaggtacaa 60
attggttgct ggtcagcact ctctaacccg attagcactg aagttcttgg tttggctggg 120
tttgactggc tggtgctgga tggcgaacat gcgccaaacg atatctccac gtttattccg 180
cagttaatgg ccttgaaagg cagcgccagc gcgccagtag tgcgagtgcc gaccaacgag 240
ccggtaatta ttaagcgtct tctggatatc ggtttctata acttcctgat tccttttgta 300
gaaacaaaag aggaagcaga gctggcggtg gcatcaaccc gttacccacc ggaaggcatt 360
cgcggcgtct ccgtttctca ccgcgccaat atgtttggca ccgtggcgga ttatttcgct 420
cagtcgaaca agaacatcac tattctggtc cagatagaaa gtcagcaggg cgtagataac 480
gtcgatgcca ttgccgctac cgaaggcgta gacggcatct tcgtcggccc cagcgatctg 540
gccgcggcat taggccatct cggcaatgca tcacacccgg atgtacaaaa agcaattcag 600
cacattttta accgtgccag cgcgcacggc aaacccagcg gtatcctcgc gccggtcgaa 660
gccgatgcgc gtcgttatct ggaatggggc gcgacgtttg tggctgtcgg cagcgatctc 720
ggcgtcttcc gctctgccac tcagaaactg gctgatacct ttaaaaaata a 771

<210> 98
<211> 1335
<212> DNA
<213> E. Coli

<400> 98
atgattctgg acaccgttga cgaaaaaaag aaaggcgtgc atacccgcta tttaatatta 60
ctgattattt ttattgttac cgccgttaac tacgccgatc gtgcaacgct gtctattgct 120
ggtaccgaag tggcaaaaga gttgcagtta agtgcggttt cgatgggtta catcttctcc 180
gcttttggct gggcctactt gctgatgcaa atccccggcg gctggctgct tgataagttt 240
ggctcgaaaa aagtttacac ctacagcctc tttttctggt cgctattcac cttcctgcaa 300
ggctttgttg atatgttccc gctggcctgg gcagggatct ccatgttctt tatgcgcttt 360
atgctcggct tctcggaagc gccatcattc ccggcgaacg cccgaattgt cgccgcctgg 420
ttcccgacga aagaacgtgg tactgcctcc gccatcttta actcggcgca atatttctcg 480
ctggcgctct tttcgccgct gcttggctgg ctgactttcg cctggggctg ggagcacgtc 540
tttaccgtta tgggggtgat tggttttgtg ctgacggcgc tgtggatcaa gttgattcat 600
aacccgacag atcacccacg tatgtctgcg gaagagctga agtttatctc tgaaaatggc 660
gcggtggtcg atatggacca caaaaagccg ggcagtgcgg cagcaagcgg acccaaactg 720
cattacatca agcaattgct ctctaaccgc atgatgctgg gcgtattttt cggacaatat 780
tttatcaaca ccatcacctg gttcttcctc acctggttcc cgatttatct ggtgcaggaa 840
aaaggcatgt cgattctgaa agtgggtctg gtcgcctcga ttccagcact gtgtggtttt 900
gcgggcggcg tgctgggagg tgtcttctcg gattatctga tcaaacgcgg tttatccctg 960
accctggcac gtaagctacc gattgtgctg ggaatgttgc tggcttccac catcatctta 1020
tgtaactaca ccaacaacac cacgctggtg gtcatgctga tggcgctggc tttctttggc 1080
aaaggatttg gtgcgctggg ctggccggtg atttctgaca ccgcgccgaa agagattgtt 1140
ggcctctgcg gcggcgtctt taacgtcttt ggcaatgttg cctccattgt cactccactg 1200
gtgattggct acctggtaag tgaactgcac tccttcaatg cagcactggt tttcgtggga 1260
tgttcagcgc tgatggcgat ggtctgctac ctcttcgtag ttggcgacat taaacgtatg 1320
gaattgcaga aataa 1335

<210> 99
<211> 1536
<212> DNA
<213> E. Coli

<400> 99
atgcaaacga gtgatacccg cgcgttaccg ctactttgcg cccgctcggt ttataaacag 60
tattcagggg tcaatgtcct gaaaggcatc gattttacgt tgcatcaggg ggaggtccac 120
gccctgctcg gcggcaatgg tgccggtaaa tcgacgttaa tgaagattat tgccggtatt 180
acccctgctg atagcggtac gctggagatt gagggcaaca actacgtcag attaacgcca 240
gttcatgctc atcagctggg tatttatctc gttccccagg aaccgctgct tttcccaagc 300
ctgtcgataa aagaaaacat cctgtttggg ctggcaaaaa aacagctctc catgcagaaa 360
atgaagaact tgctggcggc gctgggctgc cagtttgatc tgcatagtct ggcaggatcg 420
ctggatgtcg ccgatcgcca aatggtggaa atcctccgcg ggctgatgcg cgactcgcgg 480
attctgatcc tcgatgaacc taccgcctcg cttacccctg cggaaaccga acgcttgttt 540
agtcgcttgc aagagctgct tgctactggc gtgggtattg tttttatctc gcataagctg 600
ccggaaattc gccagattgc cgatcgaatt agcgtgatgc gcgacggaac catcgcctta 660
agcggcaaaa ccagcgaact gtctaccgac gacattattc aggccatcac cccagcggta 720
cgggaaaaat cgctctctgc cagccaaaaa ttatggctgg agttacctgg taaccgccca 780
caacatgccg ccggaacgcc ggtgctgaca ctggaaaatc tgaccggcga aggtttcagg 840
aatgtcagcc tgacgctcaa tgccggagaa attctgggcc tggctgggct ggtgggggcc 900
ggacgcacag aactggccga gacgctctat ggtctgcgta ctttgcgtgg cggacgcatt 960
atgctgaatg gtaaagagat caataaatta tccactggag aacgtttact gcgcggtctg 1020
gtttatctgc cggaagatcg ccagtcatcc ggactgaatc tcgatgcttc gctggcctgg 1080
aacgtctgcg cccttactca taaccttcgt ggattctggg cgaaaaccgc gaaagataat 1140
gccaccctgg aacgttatcg tcgggcgctg aatattaaat tcaaccaacc ggaacaagct 1200
gcacggacat tatccggtgg caaccagcaa aaaatcctca ttgccaaatg cttggaagct 1260
tcgccgcaag tattgattgt cgatgagccg acgcgcggcg tggatgtctc ggcccgtaat 1320
gatatctacc agctgttgcg cagcatcgcc gcacaaaatg tggctgtgct gcttatctcc 1380
tccgacctgg aagagatcga actgatggca gatcgtgtgt atgtgatgca tcagggcgaa 1440
attacccact ctgcactgac cgagcgcgat attaatgtcg agactattat gcgcgttgcc 1500
ttcggcgata gtcagcgtca ggaggcgtca tgctga 1536

<210> 100
<211> 1029
<212> DNA
<213> E. Coli

<400> 100
atgctgaagt ttattcagaa caaccgtgaa atcacggcac tgctggcggt ggtgctgctg 60
tttgtattac ccggttttct cgaccgccag tatttaagtg tgcaaacgct gaccatggtt 120
tatagcagcg cgcaaatcct gatcctgctg gcaatgggcg cgacgctggt aatgcttacg 180
cgcaatattg atgtttcagt gggttcgatt accggaatgt gcgcggtgct gttggggatg 240
ttactgaacg caggatattc actacctgtt gcttgtgtcg cgactttact gcttggtttg 300
ctcgcgggat ttttcaacgg tgtcctggtc gcgtggctaa agatccctgc cattgttgcc 360
acccttggca cgttagggtt gtacagaggc atcatgttgc tgtggactgg cggcaaatgg 420
attgaagggt tacccgccga actgaaacag ctctccgccc cgctgctgct tggcgtttca 480
gcaattggtt ggttgacgat aattctggtg gcatttatgg cctggctgct ggcaaagacg 540
gcgtttggac gcagttttta tgccacgggc gataatttac agggcgctcg tcaactgggc 600
gttcgtactg aagccattcg cattgtggca ttttcgttga acggctgcat ggcggcactg 660
gcgggaattg tgtttgcttc gcagattggt tttatcccca accagaccgg taccgggctg 720
gagatgaaag caattgcagc ctgcgtgctg ggcggcatta gtttgctcgg tggttccggt 780
gcgatcattg gtgcggtact cggcgcatgg ttcctgacgc agatcgatag cgtactggtg 840
ctgttgcgca ttccggcatg gtggaatgat tttatcgcgg gtctggttct gctggcggtg 900
ctggtgtttg atggacgcct gcgttgtgcg ctggaacgta atctacggcg gcaaaaatat 960
gcccgcttta tgacgccacc gccatccgtt aaacccgctt cgtcaggtaa aaaacgggag 1020
gccgcataa 1029

<210> 101
<211> 993

<212> DNA
<213> E. Coli

<400> 101
atgcgtattc gctacggttg ggaactggct cttgccgcac tgctcgttat tgagattgtc 60
gcatttggtg caattaaccc gcgaatgtta gatctcaata tgttgctgtt cagcaccagt 120
gactttatct gcattggcat tgtcgcccta ccgctaacga tggtgattgt cagtggcggg 180
atcgatattt cgtttggttc gaccatcggc ctctgcgcca ttgcattggg cgtactgttt 240
caaagtggtg tgccgatgcc gctggcgata ctcctgacct tactgctcgg cgcattgtgc 300
gggctgatca acgccggatt aattatctat accaaagtta acccgctggt gattacgctt 360
ggcacgctgt atctgtttgc cggaagcgct ctgctgcttt ccggtatggc cggagcgacg 420
gggtacgaag gtattggtgg attcccgatg gcgtttacag atttcgctaa cctggatgtg 480
ctgggactcc ccgttccgct gattatcttc ctgatatgtc tcctcgtttt ctggctctgg 540
ctgcataaaa cccatgccgg acgtaatgtg tttttgattg ggcaaagccc gcgcgtggcg 600
ctttatagcg cgattccagt taaccgtacc ttatgtgcgc tctatgccat gacggggctg 660
gcgtctgcgg tcgccgctgt gctgctggta tcgtattttg gttcagcacg ttccgatctc 720
ggtgcgtcgt ttctgatgcc cgccatcacc gccgtggtgc ttggcggggc caatatttat 780
ggtggttccg gttccattat cggcaccgcc attgcggttt tattagtggg atatttgcaa 840
caaggtttgc aaatggcagg agtgccaaat caggtgtcca gcgccctttc cggtgcgcta 900
cttatcgtcg ttgtcgtagg tcgttccgtt agcctgcatc gccagcaaat taaagagtgg 960
ctggcgcgtc gggccaataa cccattgcca taa 993

<210> 102
<211> 1023
<212> DNA
<213> E. Coli

<400> 102
atgacacttc atcgctttaa gaaaatcgcc ttacttagcg ctcttggcat tgccgcaatc 60
tctatgaatg tgcaggccgc agagcgtatt gcatttattc ccaaactggt tggcgtggga 120
ttttttacca gcggtggcaa cggcgcacaa caagcgggta aagagctggg cgttgatgtg 180
acctacgacg ggccgacaga acccagtgtt tctggtcagg tacagttgat taataacttc 240
gtcaatcaag gttataacgc cattatcgtt tctgcggttt cgcctgatgg cttgtgtccg 300
gcactgaaac gcgccatgca acgtggtgtg agagtgctga cctgggactc tgatactaaa 360
ccggagtgcc gctcttacta cattaatcag ggaacgcccg cccagttagg aggtatgttg 420
gtggatatgg cggcgcgtca ggtgaataaa gacaaagcca aagtcgcgtt tttctactca 480
agccccaccg ttacggacca aaaccagtgg gtgaaagaag cgaaagcgaa aatcgccaaa 540
gagcatccag gctgggaaat tgtcactacg cagtttggct ataacgatgc cactaaatcg 600
ttacaaaccg cagaaggaat attaaaagcg tatagcgatc tcgacgccat tatcgccccc 660
gatgccaacg ccctgcccgc tgccgcacaa gccgcagaaa acttgaaaaa tgacaaagta 720
gcgattgtcg gattcagtac gccaaatgtg atgcgcccgt atgtagagcg cggcacggtg 780
aaagaatttg gcctgtggga tgtggttcag caaggcaaaa tttcagtgta tgtcgcggat 840
gcattattga aaaaaggatc aatgaaaacg ggcgacaagc tggatatcaa gggcgtaggt 900
caggttgaag tctcgccaaa cagcgttcag ggctatgact acgaagcgga tggtaatggc 960
atcgtactgt taccggagcg cgtgatattc aacaaagaga atatcggcaa atacgatttc 1020
tga 1023

<210> 103
<211> 876
<212> DNA
<213> E. Coli

<400> 103
atggcagatt tagacgatat taaagatggt aaagattttc gtaccgatca accgcaaaaa 60
aatatccctt ttaccctgaa aggttgcggt gcgctggatt ggggaatgca gtcacgctta 120
tcgcggatat ttaatccgaa aacgggtaaa accgtgatgc tggcttttga ccatggttat 180
tttcagggac cgactaccgg acttgaacgc attgatataa atatcgcccc gctgtttgaa 240
catgccgatg tattaatgtg tacgcgcggc attttgcgca gcgtagttcc ccctgcgacc 300
aataggccgg tggtactgcg ggcgtcaggt gcgaactcta ttctggcgga attaagtaat 360
gaagccgtgg cgttatcgat ggatgacgcc gtgcgcctga acagttgcgc ggtggcggcg 420
caggtttata tcggcagcga atatgaacat cagtcgatca aaaatattat tcagctggtt 480

gatgccggaa tgaaagtggg aatgccgacc atggccgtga ctggcgtggg caaagatatg 540
gtgcgcgatc agcgttattt ctcgctcgcg actcgaatcg ccgctgaaat gggggcgcaa 600
attatcaaaa cctattatgt cgaaaaaggt tttgaacgga ttgttgccgg atgtccggta 660
cccattgtta ttgctggcgg taaaaaatta ccggagcgcg aggcgctgga aatgtgctgg 720
caggctatcg atcagggcgc ttctggtgtg gatatggggc gtaatatttt ccagtctgac 780
catccggtgg cgatgatgaa agccgtacag gcggtggttc accataacga aacggctgat 840
cgggcatatg aactctatct gagtgaaaaa cagtaa 876

<210> 104
<211> 291
<212> DNA
<213> E. Coli

<400> 104
atgcacgtca cactggttga aattaacgtt catgaagaca aggttgacga gtttatcgaa 60
gtttttcgcc agaaccacct gggctctgta caggaagaag gcaatttgcg cttcgatgtc 120
ttacaggacc cggaagtgaa ttcgcgcttt tatatctacg aagcctataa agatgaagac 180
gcagtggcgt tccataaaac cacgccccac tacaaaacct gtgtcgcgaa actggaatct 240
ttaatgaccg ggccgcgtaa aaaacgtctg ttcaatggtt tgatgccgtg a 291

<210> 105
<211> 1152
<212> DNA
<213> E. Coli

<400> 105
atgtttgaac caatggaact taccaatgac gcggtgatta aagtcatcgg cgtcggcggc 60
ggcggcggta atgctgttga acacatggtg cgcgagcgca ttgaaggtgt tgaattcttc 120
gcggtaaata ccgatgcaca agcgctgcgt aaaacagcgg ttggacagac gattcaaatc 180
ggtagcggta tcaccaaagg actgggcgct ggcgctaatc cagaagttgg ccgcaatgcg 240
gctgatgagg atcgcgatgc attgcgtgcg gcgctggaag gtgcagacat ggtctttatt 300
gctgcgggta tgggtggtgg taccggtaca ggtgcagcac cagtcgtcgc tgaagtggca 360
aaagatttgg gtatcctgac cgttgctgtc gtcactaagc ctttcaactt tgaaggcaag 420
aagcgtatgg cattcgcgga gcaggggatc actgaactgt ccaagcatgt ggactctctg 480
atcactatcc cgaacgacaa actgctgaaa gttctgggcc gcggtatctc cctgctggat 540
gcgtttggcg cagcgaacga tgtactgaaa ggcgctgtgc aaggtatcgc tgaactgatt 600
actcgtccgg gtttgatgaa cgtggacttt gcagacgtac gcaccgtaat gtctgagatg 660
ggctacgcaa tgatgggttc tggcgtggcg agcggtgaag accgtgcgga agaagctgct 720
gaaatggcta tctcttctcc gctgctggaa gatatcgacc tgtctggcgc gcgcggcgtg 780
ctggttaaca tcacggcggg cttcgacctg cgtctggatg agttcgaaac ggtaggtaac 840
accatccgtg catttgcttc cgacaacgcg actgtggtta tcggtacttc tcttgacccg 900
gatatgaatg acgagctgcg cgtaaccgtt gttgcgacag gtatcggcat ggacaaacgt 960
cctgaaatca ctctggtgac caataagcag gttcagcagc cagtgatgga tcgctaccag 1020
cagcatggga tggctccgct gacccaggag cagaagccgg ttgctaaagt cgtgaatgac 1080
aatgcgccgc aaactgcgaa agagccggat tatctggata tcccagcatt cctgcgtaag 1140
caagctgatt aa 1152

<210> 106
<211> 3048
<212> DNA
<213> E. Coli

<400> 106
atggacgtca gtcgcagaca attttttaaa atctgcgcgg gcggtatggc tggaacaaca 60
gtagcggcat tgggctttgc cccgaagcaa gcactggctc aggcgcgaaa ctacaaatta 120
ttacgcgcta aagagatccg taacacctgc acatactgtt ccgtaggttg cgggctattg 180
atgtatagcc tgggtgatgg cgcaaaaaac gccagagaag cgatttatca cattgaaggt 240
gacccggatc atccggtaag ccgtggtgcg ctgtgcccga aaggggccgg tttgctggat 300
tacgtcaaca gtgaaaaccg tctgcgctac ccggaatatc gtgcgccagg ttctgacaaa 360
tggcagcgca ttagctggga agaagcattc tcccgtattg cgaagctgat gaaagctgac 420
cgtgacgcta actttattga aaagaacgag cagggcgtaa cggtaaaccg ttggctttct 480

accggtatgc tgtgtgcctc cggtgccagc aacgaaaccg ggatgctgac ccagaaattt 540
gcccgctccc tcgggatgct ggcggtagac aaccaggcgc gcgtctgaca cggaccaacg 600
gtagcaagtc ttgctccaac atttggtcgc ggtgcgatga ccaaccactg ggtggatatc 660
aaaaacgcta acgtcgtgat ggtgatgggc ggtaacgctg ctgaagcgca tcccgtcggt 720
ttccgctggg cgatggaagc gaaaaacaac aacgacgcaa ccttgatcgt tgtcgatccc 780
cgttttacgc gtaccgcttc tgtggcggat atttacgcgc ctattcgttc cggtacggac 840
attacgttcc tgtctggcgt tttgcgctac ctgatcgaaa acaacaaaat caacgccgaa 900
tacgttaagc attacaccaa cgccagcctg ctggtgcgtg atgattttgc tttcgaagac 960
ggtctgttca gcggctacga cgctgaaaaa cgtcaatacg ataaatcgtc ctggaactat 1020
cagctcgatg aaaacggcta tgcgaaacgc gatgaaacac tgactcatcc gcgctgtgtg 1080
tggaacctgc tgaaagagca cgtttcccgc tacacgccgg acgtcgttga aaacatctgc 1140
ggtacgccaa aagccgactt cctgaaagtg tgtgaagtgc tggcctccac cagcgcaccg 1200
gatcgcacaa ccaccttcct gtacgcgctg ggctggacgc agcacactgt gggtgcgcag 1260
aacatccgta ctatggcgat gatccagttg ctgctcggta acatgggtat ggccggtggc 1320
ggcgtgaacg cattgcgtgg tcactccaac attcagggct tgactgactt aggcctgctc 1380
tctaccagcc tgccaggtta tctgacgctg ccgtcagaaa aacaggttga tttgcagtcg 1440
tatctggaag cgaacacgcc gaaagcgacg ctggctgatc aggtgaacta ctggagcaac 1500
tatccgaagt tcttcgttag cctgatgaaa tctttctatg gcgatgccgc gcagaaagag 1560
aacaactggg gctatgactg gctgccgaag tgggaccaga cctacgacgt catcaagtat 1620
ttcaacatga tggatgaagg caaagtcacc ggttatttct gccagggctt taacccggtt 1680
gcgtccttcc cggacaaaaa caaagtggtg agctgcctga gcaagctgaa gtacatggtg 1740
gttatcgatc cgctggtgac tgaaacctct accttctggc agaaccacgg tgagtcgaac 1800
gatgtcgatc cggcgtctat tcagactgaa gtattccgtc tgccttcgac ctgctttgct 1860
gaagaagatg gttctatcgc taactccggt cgctggttgc agtggcactg gaaaggtcag 1920
gacgcgccgg gcgaagcgcg taacgacggt gaaattctgg cgggtatcta ccatcatctg 1980
cgcgagctgt accagtccga aggtggtaaa ggcgtagaac cgctgatgaa gatgagctgg 2040
aactacaagc agccgcacga accgcaatct gacgaagtgg ctaaagagaa caacggctac 2100
gcgctggaag atctctatga cgctaatggc gtgcttattg cgaagaaagg tcagttgctg 2160
agtagctttg cgcatctgcg tgatgacggt acaaccgcat cttcttgctg gatctacacc 2220
ggtagctgga cagagcaggg caaccagatg gctaaccgcg ataactccga cccgtccggt 2280
ctggggaata cgctgggatg ggcctgggcg tggccgctca accgtcgcgt gctgtacaac 2340
cgtgcttcgg cggatatcaa cggtaaaccg tgggatccga aacggatgct gatccagtgg 2400
aacggcagca agtggacggg taacgatatt cctgacttcg gcaatgccgc accgggtacg 2460
ccaaccgggc cgtttatcat gcagccggaa gggatgggac gcctgtttgc catcaacaaa 2520
atggcggaag gtccgttccc ggaacactac gagccgattg aaacgccgct gggcactaac 2580
ccgctgcatc cgaacgtggt gtctaacccg gttgttcgtc tgtatgaaca agacgcgctg 2640
cggatgggta aaaaagagca gttcccgtat gtgggtacga cctatcgtct gaccgagcac 2700
ttccacacct ggaccaagca cgcattgctc aacgcaattg ctcagccgga acagtttgtg 2760
gaaatcagcg aaacgctggc ggcggcgaaa ggcattaata atggcgatcg tgtcactgtc 2820
tccagcaagc gtggctttat ccgcgcggtg gctgtggtaa cgcgtcgtct gaaaccgctg 2880
aatgtaaatg gtcagcaggt tgaaacggtg ggtattccaa tccactgggg ctttgagggt 2940
gtcgcgcgta aaggttatat cgctaacact ctgacgccga atgtcggtga tgcaaactcg 3000
caaacgccgg aatataaagc gttcttagtc aacatcgaga aggcgtaa 3048

<210> 107
<211> 885
<212> DNA
<213> E. Coli

<400> 107
atggctatgg aaacgcagga cattatcaaa aggtccgcaa ctaactccat cacgccgcct 60
tctcaggtgc gtgattacaa agcagaagtc gcaaaactta tcgacgtttc cacctgtatc 120
ggctgtaaag cctgtcaggt ggcgtgttcg gagtggaacg acatccgtga tgaagtgggg 180
cactgcgtcg gggtttacga taaccccgcc gatctgagcg ccaagtcctg gacggtgatg 240
cgctttagcg aaaccgaaca gaacggcaag ctggagtggc tgatccgtaa agacggctgt 300
atgcactgtg aagatcccgg ctgcctgaag gcgtgcccgt ctgctggtgc aatcattcag 360
tacgctaacg ggattgtcga tttccagtcg gaaaactgca tcggctgtgg ttactgcatt 420
gccgggtgtc cgtttaatat tccgcgcctc aacaaagagg ataaccgggt atataaatgc 480
acgctctgcg tcgatcgcgt cagcgtcggc caggaaccgg cttgtgtgaa aacctgtccg 540
accggggcta tccacttcgg caccaagaag gagatgctgg agctggcgga acagcgcgtg 600
gcgaaactga aagcgcgtgg ttacgaacat gctggcgtct acaacccgga aggggtcggt 660

ggtacgcacg ttatgtacgt gctgcatcac gccgatcagc cggagctgta tcacggtctg 720
ccgaaagatc cgaagatcga cacctcggta agcctgtgga aaggcgcgtt gaaaccgctg 780
gcagcggctg gctttattgc cacttttgcc gggttgattt tccactacat cggtattggc 840
ccgaataagg aagtggacga tgacgaggag gatcatcatg agtaa 885

<210> 108
<211> 654
<212> DNA
<213> E. Coli

<400> 108
atgagtaagt cgaaaatgat tgtgcgcacc aaatttattg atcgcgcctg tcactggacc 60
gtggtgattt gcttcttcct ggtggcgctg tccgggattt cgttcttctt cccgacgctg 120
caatggctga cgcaaacctt cggtacgccg cagatgggac gcattttgca cccgttcttc 180
ggcattgcga ttttcgtcgc actgatgttt atgtttgtgc gttttgtgca tcacaacatc 240
ccggataaga aagatattcc gtggctgttg aacattgtcg aagtattgaa aggcaatgag 300
cataaagtgg cggatgtcgg taagtacaac gccgggcaaa agatgatgtt ctggtcgatc 360
atgagcatga ttttcgtgct gctggtgacc ggggtgatta tctggcgtcc gtactttgcg 420
cagtacttcc cgatgcaggt tgttcgctac agcctgctga tccacgcggc tgcgggtatc 480
atcctgatcc acgccatcct gatccatatg tatatggcat tttgggtgaa aggatcgatt 540
aaagggatga tcgaagggaa ggtaagtcgt cgctgggcga agaaacacca tccgcgctgg 600
tatcgtgaaa tcgagaaggc agaagcgaaa aaagagagtg aagaagggat ataa 654

<210> 109
<211> 261
<212> DNA
<213> E. Coli

<400> 109
atggcgttgt taatcactaa aaaatgcatc aattgtgata tgtgtgaacc cgaatgcccg 60
aatgaggcga tttcaatggg agatcatatc tacgagatta acagcgataa gtgtaccgaa 120
tgcgtagggc actacgagac accaacctgc cagaaggtgt gcccgatccc caatactatt 180
gtgaaagatc cggcgcatgt cgagacagaa gaacagttgt gggataaatt tgtgctgatg 240
caccacgcgg ataaaattta a 261

<210> 110
<211> 1203
<212> DNA
<213> E. Coli

<400> 110
atgcaaagtg ttgatgtagc cattgttggc ggcggcatgg tggggctggc ggttgcctgt 60
ggcttacagg ggagcggctt acgcgttgcc gtactggagc agcgcgtaca ggaacctctg 120
gcggcgaatg caccaccaca actgcgcgtt tcggctatca atgccgccag cgaaaaatta 180
ctcacccgtc ttggcgtctg gcaggacatt ctctctcgta gggccagctg ttatcacggt 240
atggaagtgt gggacaaaga cagctttggt cacatttcgt ttgacgatca aagcatgggc 300
tatagccatc ttgggcatat cgttgaaaat tcagtgattc actacgcgct gtggaacaaa 360
gcgcatcagt cgtcagatat cactctgtta gcccccgcag aattacagca ggtcgcctgg 420
ggagaaaatg aaaccttcct gacgctgaaa gatggcagca tgttaacggc gcgtctggtg 480
attggcgcgg acggcgctaa ttcctggttg cgcaacaaag ccgatattcc gctgactttc 540
tgggattatc agcatcacgc gctggtagcg accattcgca cggaagaacc gcatgatgcg 600
gtggcgcggc aggttttcca tggcgaaggc attctggcct ttttaccgct tagcgatccg 660
catctttgct cgattgtctg gtcactgtcg ccagaggaag cgcagcggat gcagcaggca 720
agtgaagacg aatttaatcg cgcgttaaat atcgcttttg ataatcgcct gggcttatgc 780
aaggttgaga gcgcgcgtca ggtgttccca ctgacggggc gttatgcgcg ccagtttgcc 840
tcgcaccgtc tggcgctggt gggcgacgcc gcacatacca ttcacccgct ggcggggcag 900
ggggtaaatc tcggctttat ggatgctgca gagctgattg ccgaactgaa acggttgcat 960
cgtcagggga aagacatcgg gcagtacatt tatctgcgtc gctatgagcg tagccgcaag 1020
cacagtgcgg cgttgatgct ggctggtatg cagggattcc gcgatctgtt ttccggtacc 1080
aatccggcga aaaaactgct gcgtgatatt ggtttgaaac tggccgacac gcttcctggc 1140
gttaagccgc aacttatccg ccaggcaatg ggattaaacg atttgcctga atggctgcgt 1200

taa                                                                1203

<210> 111
<211> 1179
<212> DNA
<213> E. Coli

<400> 111
atgagcgtaa tcatcgtcgg tggcggcatg gcgggcgcga cgctggcgct ggctatttcc      60
cggttaagtc acggggcgct gccggtacat ttgattgaag cgactgcgcc agagtcacat     120
gctcatccgg gctttgatgg acgagcgata gcgctggcgg cgggtacctg tcagcaactg     180
gcgcgcatcg gcgtctggca atctctggcg gattgcgcaa ctgccatcac caccgtgcat     240
gtcagcgatc gtggtcacgc tggatttgtc accctcgccg cagaagatta ccaactggcg     300
gcgctgggac aggttgtcga attgcacaat gtcgggcaac ggctgtttgc attgctgcgt     360
aaagcacctg gcgtaacgct gcattgccct gatcgcgtgg ctaacgttgc ccgtactcag     420
agtcacgttg aagtgacgct ggagagtggc gagacgctga cgggccgcgt gctggtagca     480
gctgatggca cccattcagc gttagccacc gcgtgcggcg ttgactggca gcaggagcct     540
tacgaacaac tggccgtgat tgccaacgtt gctacttccg ttgcgcatga agggcgcgct     600
tttgaacgct ttacgcaaca tggcccgctg gcgatgttgc cgatgtctga cggacgctgt     660
tcgctggtct ggtgtcatcc actggaacgg cgcgaagagg tgttgtcgtg gagtgacgag     720
aagttttgcc gtgaactcca gtcggccttt ggctggcgac ttgggaaaat tacccacgct     780
ggtaaacgca gtgcttatcc gctggcgtta acccacgccg ccagatctat tacccatcgt     840
accgtgctgg tgggcaatgc ggcgcaaact ctgcacccga ttgccgggca agggtttaac     900
ctcggtatgc gagatgtgat gagtcttgcg gaaaccctga ctcaggcgca ggagcgcgga     960
gaagacatgg gggattacgg cgtattgtgc cgttatcagc agcgtcgaca gagcgatcgc    1020
gaagcaacca ttggcgtcac ggacagcctt gtacatcttt ttgccaaccg ttgggcaccg    1080
ctggttgtcg ggcgcaacat cgggctgatg acgatggaat tattcacccc ggcacgcgat    1140
gtgctggcgc agcgcaccct cggttgggtg gcgcgttga                           1179

<210> 112
<211> 1326
<212> DNA
<213> E. Coli

<400> 112
atgagtgaga tatcccggca agagtttcag cgtcgccgtc aggccctggt ggagcaaatg      60
caacccggca gcgccgcgct gatttttgct gcaccagaag taacacgtag cgccgacagc     120
gaatacccct atcgtcagaa cagtgacttc tggtacttca ccggctttaa cgaaccggaa     180
gcggtgctgg tgctgattaa aagcgatgac actcataacc acagcgttct gtttaaccgc     240
gttcgcgacc tgacggcgga gatctggttt ggccgtcgct taggccagga tgccgcgcca     300
gagaaactgg gcgttgaccg cgcactggca ttcagcgaaa tcaatcagca actttatcaa     360
ctacttaacg gcctggatgt ggtttaccat gcccagggcg aatatgcata tgctgatgta     420
atcgtgaaca gtgcgctgga aaaactgcgt aaaggttcgc ggcaaaatct caccgcaccg     480
gcaacgatga tcgactggcg tcctgttgtt catgaaatgc gcctgttcaa atcgccagaa     540
gagattgccg tactccgccg cgcgggagaa atcaccgcca tggcacatac acgggcgatg     600
gaaaaatgcc gtccgggaat gttcgagtac catctggaag gcgaaattca ccacgaattt     660
aaccgccacg gtgcgcgcta tccgtcctat aacaccattg tcggcagcgg tgaaaacggc     720
tgcattctgc actacaccga aaacgagtgt gaaatgcgcg acggcgacct ggtgttgatt     780
gacgcgggtt gtgaatacaa aggttacgct ggcgatatta cccgcacctt cccggtcaac     840
ggcaaattca cccaggccca gcgtgaaatc tacgacattg tgctggagtc tctcgaaacc     900
agcctgcgcc tgtatcgtcc gggaacttcc attctggaag tcactggtga agtggtgcgc     960
atcatggtta gcggcctggt aaaactcggc atcctgaaag gtgatgttga tgaactgatc    1020
gctcagaacg cccatcgtcc tttctttatg catggcctta gccactggtt aggactggat    1080
gtccatgacg tgggtgttta tggtcaggat cgctcgcgca ttctggaacc gggcatggta    1140
ctgaccgtag agccagggct gtatattgcg ccggatgcag aagtgccaga acaatatcgc    1200
ggtatcggca ttcgtattga agacgacatt gtgattaccg aaaccggtaa cgaaaacctc    1260
accgccagcg tggtgaaaaa gccggaagaa atcgaagcgt tgatggttgc tgcgagaaag    1320
caatga                                                               1326

<210> 113
<211> 585

<212> DNA
<213> E. Coli

<400> 113
atgcttatgt ctatacagaa cgaaatgcct ggttacaacg aaatgaacca gtatctgaac 60
caacaaggga cgggtctgac cccagctgag atgcatggtt taatcagcgg gatgatatgt 120
ggcggtaacg atgacagctc atggctaccg ctacttcacg acctgacgaa cgaaggcatg 180
gctttcggtc atgagctggc acaggcactg cgtaaaatgc actctgccac cagcgatgcc 240
ctgcaggatg acggcttcct ttttcagctt tatctgcctg atggcgatga tgtcagcgtt 300
ttcgatcggg ctgatgcatt ggcaggttgg gtcaatcact tcctgcttgg tcttggcgtt 360
acgcaaccga agctggataa agtgaccggc gaaaccggtg aagctatcga cgatctgcgt 420
aacattgcgc aactgggtta cgacgaagac gaagatcagg aagagcttga aatgtcgctt 480
gaagagatca tcgaatacgt tcgtgttgcc gcgctgttat gccacgacac ctttactcat 540
ccgcaaccga ccgcgccaga agtacaaaaa ccgactctac actaa 585

<210> 114
<211> 363
<212> DNA
<213> E. Coli

<400> 114
atgttaaagc tatttgcaaa gtacacctct attggtgtgc tgaacaccct tatacactgg 60
gtggtttttg gtgtttgtat ctatgtcgcg catacaaacc aagctcttgc aaacttcgca 120
ggtttcgttg tggctgtgag ctttagcttc ttcgcgaatg caaaattcac attcaaggca 180
tcgactacaa cgatgcgcta catgctatat gttgggttca tggggacact gagtgctact 240
gttggatggg ctgctgatag atgcgcactt cccccgatga taactcttgt caccttctcc 300
gccatcagcc tggtgtgcgg tttcgtctat tcaaagttca ttgtctttag ggatgcgaaa 360
tga 363

<210> 115
<211> 921
<212> DNA
<213> E. Coli

<400> 115
atgaagatat ctcttgtagt tcctgtcttc aatgaagaag aagcgatacc aatttttat 60
aaaacggtac gtgaattcga agaattgaag tcatatgaag tggaaatcgt tttcataaat 120
gacggcagca aagacgctac ggagtcaatc attaatgctc tggctgtttc agatcctcta 180
gttgttccgc tgtcatttac acgcaacttt ggtaaagaac cagcattgtt tgcagggtta 240
gaccatgcaa ccggggatgc gataatccca attgatgttg acctgcaaga cccgattgag 300
gttattcctc atcttattga aaaatggcaa gcaggtgctg atatggttct tgctaaaaga 360
tctgaccgct caactgatgg acgcctgaag cgaaaaacgg ctgagtggtt ctataagctc 420
cacaataaaa taagcaatcc taaaattgaa gagaatgttg gtgatttcag gctgatgagc 480
cgtgatgttg tcgaaaatat taaacttatg ccagaacgaa accttttcat gaaaggtatt 540
ctgagctggg taggaggaaa gacagatatt gttgaatacg tgcgagcgga aagaattgct 600
ggagatacaa aatttaatgg atggaaactt tggaatttag cacttgaggg tattacaagc 660
ttttccacat tccctcttcg catctggaca tacatagggt tagtggtagc cagtgtagca 720
tttatttatg gggcgtggat gattttagat actatcatat ttggaaatgc tgttagggga 780
tatccttcac tacttgtttc aatactgttt ttaggtggaa ttcagatgat tggaatagga 840
gtattaggtg aatatattgg acgcacatac attgaaacca aaaaacgccc gaaatacatc 900
atcaagagag tcaaaaaatg a 921

<210> 116
<211> 1332
<212> DNA
<213> E. Coli

<400> 116
atgaataaag caataaaagt atcattgtat atatcttttg ttttgattat ttgcgcctta 60
tctaaaaaca taatgatgtt aaatacatct gatttcggaa gagccattaa gccattaatt 120
gaagacatac cagcatttac atatgactta cctttattgt ataaattgaa aggtcatatt 180

gattcaattg atagctatga gtatataagt tcatatagtt atattttgta tacatacgtc 240
ctgtttatta gcatttttac tgaatatctt gatgctaggg tgttatcgtt atttctaaaa 300
gtaatatata tttattcatt atatgcgata tttacttcat atataaaaac agaaaggtat 360
gtaactttat ttacattctt tattttagct tttcttatgt gttcttcatc aacactgtca 420
atgtttgcat cattctatca agagcaaata gttataattt tccttccatt tttggtgtat 480
tcattaacat gcaaaaacaa taaatctatg cttttgctat ttttttcgtt gctaataata 540
tctactgcta aaaatcaatt tatattaacc ccactaatag tgtattcata ttatattttt 600
tttgatagac acaaactaat tattaaatct gtaatatgcg tggtgtgctt gcttgcgtca 660
atatttgcaa tatcttattc aaaaggtgtt gttgaattaa ataagtacca tgcaacatac 720
ttcggtagtt atctttatat gaaaaacaac gggtataaaa tgccatcgta tgttgatgat 780
aagtgtgttg ggttagatgc ctggggtaat aaattcgaca tatcatttgg cgcaacccca 840
acagaagttg gaacggaatg tttcgaatct cataaagatg aaacgttttc gaatgcactc 900
tttttattgg ttagcaaacc aagcaccatc ttcaaacttc catttgatga tggtgtgatg 960
tctcagtata aagaaaatta tttccatgta tataaaaaac tacacgtaat atatggagaa 1020
tcaaacatac taacgactat tactaacata aaagacaata tatttaaaaa cattagattt 1080
atatcattgt tattattttt tattgcttct atttttatta gaaataataa aataaaggca 1140
tctttatttg tagtatctct ttttggaata tctcaatttt atgtgtcatt tttcggggaa 1200
ggatatagag atttaagcaa gcatttattt ggaatgtatt tttcgttcga cctttgctta 1260
tacataacag tcgttttttt aatttataaa ataattcaaa gaaatcaaga caatagcgat 1320
gtaaagcact aa 1332

<210> 117
<211> 249
<212> DNA
<213> E. Coli

<400> 117
atgggcattc tgtcatggat tatttttggg cttattgccg gtattctggc gaagtggatc 60
atgccaggta aagatggagg tggattcttt atgactatcc tgctggggat agtcggtgcc 120
gtagtcggcg gatggatcag cacgctgttt ggctttggta aagtcgatgg cttcaatttt 180
ggcagcttcg tggttgccgt tattggtgcg attgtcgtgc tatttatcta caggaagatt 240
aaaagttaa 249

<210> 118
<211> 183
<212> DNA
<213> E. Coli

<400> 118
atgggcaaag caacgtatac cgtgaccgtc accaataaca gcaatggcgt ttctgtcgat 60
tatgaaacag agacgccgat gactttgctg gtgccagaag tggcggctga agtgataaaa 120
gatctggtga ataccgtacg ttcttatgac acggaaaacg aacatgatgt ttgtggttgg 180
taa 183

<210> 119
<211> 360
<212> DNA
<213> E. Coli

<400> 119
atgcttcaaa tcccacagaa ttatattcat acgcgctcaa cgcctttctg gaataaacaa 60
actgcacctg ccggaatatt cgaacgtcat cttgataaag gaacgcgccc gggggtttac 120
ccacgccttt ccgttatgca tggggcggtc aaatatctcg gctacgctga tgaacacagt 180
gcagagcctg atcaggtgat ccttatcgaa gcggggcagt ttgcggtgtt ccctccagaa 240
aagtggcaca acattgaagc catgactgac gatacttatt tcaacattga cttcttcgtg 300
gctcctgaag tcctgatgga aggtgcgcaa caacggaaag tcattcataa cgggaaatga 360

<210> 120
<211> 741
<212> DNA
<213> E. Coli

<400> 120
gtgaagttca aagttatcgc cctggcggca ttaatgggta ttagcgggat ggcagcgcag 60
gctaacgaat tgccggatgg accgcatatt gtcacctccg gtacggcaag cgtggatgcg 120
gtgccagaca ttgccactct tgcgattgaa gttaacgtgg ccgcgaagga tgccgctact 180
gccaagaaac aggcagatga gcgcgtcgca caatacattt ccttccttga actcaatcag 240
atcgcgaaaa aagatatcag ctcagcgaac ttacgcaccc agccagatta tgattatcag 300
gatggtaaaa gtatccttaa aggctaccgc gctgtgagaa cggtggaagt cacgctccgt 360
cagttagaca aactgaattc cttgctggat ggcgcgctga aggcgggtct taacgaaatt 420
cgttctgtgt cgctgggcgt ggcgcagccg gatgcctata aagacaaagc gcgtaaggca 480
gcgattgata acgcgattca tcaggcgcag gaactggcga acggctttca tcgtaaactg 540
gggccggtat atagcgtgcg ctaccatgtt tccaactatc agcccagccc aatggtgcgg 600
atgatgaaag ccgatgccgc gccggtgtcc gcccaggaaa cttacgagca ggccgctatt 660
cagtttgatg atcaggtcga tgtggtcttc cagttagaac ctgtggatca acaacccgct 720
aaaacacctg cagcacaata a 741

<210> 121
<211> 1395
<212> DNA
<213> E. Coli

<400> 121
gtgttattac tggatgcgtg ctcgcaaatg tgcccgtcat tcagacgatt ccagacagtg 60
tttcataatt cctccatttt tctcccttat tggctggcta cactagtatc attccgcgaa 120
acgtttcagg aagagaaact cttaacgatg aaaggtagtt ataaatcccg ttgggtaatc 180
gtaatcgtgg tggttatcgc cgccatcgcc gcattctggt tctggcaagg ccgcaatgac 240
tcccggagtg cagccccagg ggcgacgaaa caagcgcagc aatcgccagc gggtggtcga 300
cgtggtatgc gttccggccc attagccccg gttcaggcgg cgaccgccgt agaacaggca 360
gttccgcgtt acctcaccgg gcttggcacc attaccgccg ctaataccgt tacggtgcgc 420
agccgcgtgg acggccaact gatagcgtta catttccagg aaggccagca ggtcaaagca 480
ggcgatttac tggcagaaat tgaccccagc cagttcaaag ttgcattagc acaagcccag 540
ggccaactgg caaaagataa agccacgctt gccaacgccc gccgtgacct ggcgcgttat 600
caacaactgg caaaaaccaa tctcgtttcc cgccaggagc tggatgccca acaggcgctg 660
gtcagtgaaa ccgaaggcac cattaaggct gatgaagcaa gcgttgccag cgcgcagctg 720
caactcgact ggagccggat taccgcacca gtcgatggtc gcgttggtct caagcaggtt 780
gatgttggta accaaatctc cagtggtgat accaccggga tcgtggtgat cacccagacg 840
catcctatcg atttagtctt taccctgccg gaaagcgata tcgctaccgt agtgcaggcg 900
cagaaagccg gaaaaccgct ggtggtagaa gcctgggatc gcaccaactc gaagaaatta 960
agtgaaggca cgctgttaag tctagataac caaatcgatg ccactaccgg tacgattaaa 1020
gtgaaagcac gctttaataa tcaggatgat gcgctgtttc ccaatcagtt tgttaacgcg 1080
cgcatgttag tcgacaccga acaaaacgcc gtagtgatcc caacagccgc cctgcaaatg 1140
ggcaatgaag gccattttgt ctgggtgctg aatagcgaaa acaaggtcag caaacatctg 1200
gtgacgccgg gcattcagga cagtcagaaa gtggtgatcc gtgcaggtat ttctgcgggc 1260
gatcgcgtgg tgacagacgg cattgatcgc ctgaccgaag gggcgaaagt ggaagtggtg 1320
gaagcccaga gcgccactac tccggaagag aaagccacca gccgcgaata cgcgaaaaaa 1380
ggagcacgct cctga 1395

<210> 122
<211> 3123
<212> DNA
<213> E. Coli

<400> 122
atgcaggtgt tacccccgag cagcacaggc ggcccgtcgc gcctgtttat tatgcgtcct 60
gtggccacca cgctgctgat ggtggcgatc ttactcgccg ggattatcgg ttatcgcgcc 120
ctgcccgttt cggcgctgcc ggaagtggac tatccgacca ttcaggtggt cacgctctac 180
ccaggtgcca gcccggatgt catgacctct gccgttaccg cgccgctaga acgccagttc 240
gggcagatgt ctggcctgaa acagatgtcg tcgcaaagtt ccggcggtgc gtcagttatc 300
actttgcagt tccagctaac attaccgctc gatgtcgccg agcaggaagt gcaggccgcg 360
attaacgctg cgaccaactt gttgccgagc gatctgccta acccgccggt ttacagcaaa 420
gtgaacccgg cagatccgcc gatcatgacg ctcgccgtca cctcaaccgc catgccgatg 480

acgcaagtgg aagatatggt ggaaacccgc gtcgcgcaga aaatctcgca gatttccggc 540
gtcggcctgg tgacgctttc cggcggtcag cgtccggctg ttcgcgtcaa acttaacgct 600
caggcgattg ccgccctcgg cctgaccagc gaaaccgtgc gcaccgccat taccggcgct 660
aacgttaact cggcaaaagg tagcctcgac ggcccttccc gtgcggtcac gctttccgcg 720
aacgaccaga tgcaatccgc cgaagagtat cgccagctaa tcatcgccta ccagaacggc 780
gcgccaattc gtctgggcga tgtcgcaact gtagagcaag gtgcagaaaa cagctggctc 840
ggcgcgtggg cgaacaaaga acaggccatt gtgatgaatg ttcagcgcca gcccggtgct 900
aacattatct ccaccgccga cagcattcgg cagatgctgc cacagctcac tgagagtctg 960
ccgaaatcgg tgaaggtgac agtgctttcc gatcgcacca ccaatatccg cgcatccgtc 1020
gatgatactc agtttgaatt gatgatggct atcgcgctgg tagtcatgat tatctacctg 1080
tttttgcgca atattccggc gaccatcatt cccggtgttg ctgtaccgct gtcgttaatc 1140
ggcactttcg cggttatggt gtttctcgat ttttcaatca ataacctgac actgatggcg 1200
ttaactatcg ccaccggatt cgtggtcgat gacgccatcg tggtgatcga aaacatttcc 1260
cgctatatcg aaaaaggcga aaaaccgttg gcggcggcgc tcaagggcgc aggtgaaatc 1320
ggctttacca ttatctcgct gaccttctca ctgattgcgg tgttgatccc actgctgttt 1380
atgggcgata tcgtcgggcg actgttccgc gaatttgcta ttaccctggc ggtagcgatt 1440
ttgatctcag cggtggtgtc gctgaccctg acaccgatga tgtgcgcgcg gatgctcagc 1500
caggagtcgt tgcgtaaaca gaaccgcttc tcccgtgcct cggaaaaaat gttcgacagg 1560
ataatcgccg cctatggtcg tggactggcg aaagtgctga atcatccgtg gctgacctta 1620
agcgtggcac tcagcacgct gctgcttagc gtgctgctgt gggtgttcat tccgaaaggt 1680
ttcttcccgg tacaggacaa tggcattatt cagggcactt tgcaggcacc gcaatccagc 1740
tcctttgcca atatggccca gcgacaacgc caggtcgcgg acgtgatttt gcaggatccg 1800
gcagtgcaaa gcctgacctc atttgttggc gttgatggca ctaacccgtc gctgaacagt 1860
gcacgtttac aaatcaacct caaaccgttg gatgaacgtg atgatcgggt gcaaaaagtc 1920
atcgcccgtc tgcaaacggc ggtagataaa gtgccgggcg tcgatctctt cctgcaacca 1980
acgcaggatc tgactattga tactcaggtc agccgcaccc agtaccagtt taccttgcag 2040
gccacgtcac tggatgcgct cagtacctgg gtgccacagt tgatggaaaa actccagcaa 2100
ctgccacagc tttctgatgt ctccagcgac tggcaggaca aagggctggt ggcgtatgtc 2160
aatgttgatc gcgacagcgc cagccgtctg gggatcagca tggcggatgt cgataacgcc 2220
ctgtacaacg cgtttggtca gcggctgatt tccactattt atactcaggc caaccagtat 2280
cgcgtggtgc tggagcacaa caccgaaaat accccaggcc tcgcggcgct ggataccatt 2340
cgcctgacca gcagcgacgg cggcgtggtg ccgctaagct caattgccaa aattgagcag 2400
cgttttgcgc cgctctccat caaccatctg gatcagttcc cggtaacgac catctccttt 2460
aacgtgccgg ataactattc gctgggcgat gcggtgcagg cgattatgga caccgaaaag 2520
acgctgaatc tgccggtgga tatcaccacg cagttccagg gcagcaccct cgccttccag 2580
tcggcgctgg gcagcactgt ctggctgatt gtcgcggcgg tggtggcgat gtatatcgtg 2640
ctcggcattc tgtacgagag ctttattcac ccgatcacca ttctctcgac gctacccacc 2700
gcaggggttg gcgcactgct ggcgttgctg attgctggta gcgaactgga tgtgattgcg 2760
attatcggca ttattttgct gatcggtatc gtgaagaaga acgccatcat gatgatcgac 2820
ttcgcgctgg ctgctgagcg cgagcaaggc atgtcgccgc gcgaggcaat ctaccaggct 2880
tgtctgttgc gttttcgtcc gatcctgatg accactctgg cggctctgct tggcgcgctg 2940
ccgctgatgt tgagtaccgg ggtcggcgcg gaactgcgtc gtccgttagg tatcggcatg 3000
gtcggcggtc tgattgtcag ccaggtgctg acgctgttta ccacgccggt gatttatttg 3060
ctgttcgacc gcctggcatt gtggaccaaa agccgctttg cccgtcatga agaggaggcg 3120
taa 3123

<210> 123
<211> 3078
<212> DNA
<213> E. Coli

<400> 123
gtgaagtttt ttgccctctt catttaccgc ccggtggcga cgattttact gtcggttgcc 60
attaccctgt gcggcatact gggcttccgt atgctgccgg tcgccccgct gccgcaggtc 120
gattttccgg tgattatcgt cagcgcctcg ctgcccggtg cgtcaccaga aacaatggcg 180
tcttccgttg ccacgccgct ggagcgctca cttgggcgca ttgccggagt cagtgaaatg 240
acctccagca gttcgctcgg cagcacgcgt attattttgc agtttgattt tgaccgggat 300
atcaacggcg cagcgcgtga tgtgcaggcg gcgatcaacg ctgcacaaag tttgctgccc 360
agtgggatgc ccagccgccc gacctatcgc aaagcgaacc cgtcggatgc gccaattatg 420
atcctcacgc tgacgtccga tacttattcg cagggtgaac tgtacgattt cgcctcgacg 480
cagctggctc cgacgatttc gcaaatcgac ggtgttggtg atgtcgatgt cggaggcagc 540

tcactgcccg ccgtacgcgt cgggctgaat ccgcaggcgc tgtttaatca gggcgtgtcg 600
ctggacgacg tacgcaccgc cgtcagcaat gccaacgtgc gtaaaccgca gggcgcgctg 660
gaagatggca ctcaccgctg gcagatccag accaatgatg agctaaaaac cgccgctgaa 720
tatcagccgt tgattattca ctacaacaac ggcggcgcgg ttcgtctggg cgatgtggcg 780
acggtgaccg actcagtgca ggatgtgcgc aacgccggga tgaccaacgc caaaccggct 840
attttactga tgatccgcaa actgccggaa gccaatatta tccagacggt tgacagcatc 900
cgggcaaaat taccggagtt gcaggaaacc attccggcgg cgattgatct gcaaattgcc 960
caggatcgct cccccaccat tcgcgcctcg ctggaagaag tcgagcaaac gctgattatc 1020
tcggtggcgc tggtgattct ggtggtgttt ttattcctgc gctcgggtcg cgccactatt 1080
attcccgccg tttcggtgcc ggtttcgctg attggtacgt ttgcggcgat gtacctgtgc 1140
ggattcagtc tcaataacct ttcgttaatg gcgctcacca tcgctactgg tttcgtggtg 1200
gatgacgcca tcgtggtgct ggaaaacatt gcacgtcatc tggaagcggg aatgaaaccg 1260
ttgcaagccg cactgcaagg tactcgcgaa gtcggtttta cggtgctgtc gatgagtctg 1320
tcactggtgg cggtgttcct gccgctgctg ttgatgggcg gattgccggg ccgactgtta 1380
cgcgaatttg ccgtgacgct ttctgtcgcc attggtatat cgttgctggt ttctctgaca 1440
ttaacgccaa tgatgtgtgg ctggatgctg aaagccagca agccgcgcga gcaaaagcga 1500
ctgcgtggtt ttggtcgcat gttggtagcc ctgcaacaag gctacggcaa gtcactaaaa 1560
tgggtgctca atcatacccg tctggtgggc gtggtgctgc ttggcaccat tgcgctgaat 1620
atctggctgt atatctcgat cccgaaaacc ttcttcccgg agcaggacac tggcgtgttg 1680
atgggcggga ttcaggcgga tcagagtatt tcgtttcagg cgatgcgcgg taagttgcag 1740
gatttcatga aaattatccg tgacgatccg gcagtggata atgtcaccgg ctttacaggc 1800
ggttcgcgag tgaacagcgg gatgatgttt atcaccctca agccacgcga cgaacgcagc 1860
gaaacggcgc agcaaattat cgaccgtctg cgcgtaaaac tggcgaaaga accgggggcg 1920
aatctgttcc tgatggcggt acaggatatt cgcgttggtg ggcgtcagtc gaacgccagc 1980
taccagtaca cgttgttatc cgacgacctg gcggcactgc gagaatggga gccgaaaatc 2040
cgcaaaaaac tggcgacgtt gccggaactg gcggacgtga actccgatca gcaggataac 2100
ggcgcggaga tgaatctggt ttacgaccgc gacaccatgg cacggctggg aatcgacgta 2160
caagccgcca acagtctgtt aaataacgcc ttcggtcagc ggcaaatctc gaccatttac 2220
cagccgatga accagtataa agtggtgatg gaagtggatc cgcgctatac ccaggacatc 2280
agtgcgctgg aaaaaatgtt cgttatcaat aacgaaggca aagcgatccc gctgtcgtat 2340
ttcgctaaat ggcaaccggc gaatgcccca ctatcggtga atcatcaggg attatcggcg 2400
gcctcgacca tttcgtttaa cctgccgacc ggaaaatcgc tctcggacgc cagtgcggcg 2460
atcgatcgcg caatgaccca gcttggtgtg ccttcgacgg tgcgcggcag ttttgccggc 2520
acggcgcagg tgttccagga gacgatgaac tcgcaggtga tcctgattat cgccgccatc 2580
gccacggtgt atatcgtgct gggtatcctt tacgagagtt acgtacatcc gctgacgatt 2640
ctctccaccc tgccctcggc gggcgttgga gcgctgttgg cgctggagct gttcaatgcc 2700
ccgttcagcc taatcgccct gatagggatc atgctattaa tcggcatcgt gaagaaaaac 2760
gccattatga tggtcgattt tgcgcttgaa gcccaacggc acggtaacct gacgccgcag 2820
gaagctattt tccaggcctg tctgctgcgt tttcgcccga ttatgatgac taccctggcg 2880
gcgctgtttg gtgcgctgcc gctggtattg tcgggcggcg acggctcgga gctgcggcaa 2940
cccctgggga tcaccattgt cggcggactg gtaatgagcc agctccttac gctgtatacc 3000
acgccggtgg tgtatctctt tttcgaccgt ctgcggctgc gtttttcgcg taaacctaaa 3060
caaacggtaa ccgagtaa 3078

<210> 124
<211> 1416
<212> DNA
<213> E. Coli

<400> 124
atgacagatc ttcccgacag cacccgttgg caattgtgga ttgtggcttt cggcttcttt 60
atgcagtcgc tggacaccac catcgtaaac accgcccttc cctcaatggc gcaaagcctc 120
ggggaaagtc cgttgcatat gcacatggtc attgtctctt atgtgctgac cgtggcggtg 180
atgctgcccg ccagcggctg gctggcggac aaagtcggcg tgcgcaatat tttctttacc 240
gccatcgtgc tgtttactct cggttcactg ttttgcgcgc tttccggcac gctgaacgaa 300
ctgttgctgg cacgcgcgtt acagggcgtt ggcggcgcga tgatggtgcc ggtcggcaga 360
ttgacggtga tgaaaatcgt accgcgcgag caatatatgg cggcgatgac ctttgtcacg 420
ttacccggtc aggtcggtcc gctgctcggt ccggcgctcg gcggtctgct ggtggagtac 480
gcatcgtggc actggatctt tttgatcaac attccggtgg ggattatcgg tgcgatcgcc 540
acattgctgt taatgccgaa ctacaccatg cagacgcggc gctttgatct ctccggattt 600
ttattgctgg cggttggcat ggcggtatta accctggcgc tggacggcag taaaggtaca 660

ggtttatcgc cgctgacgat tgcaggcctg gtcgcagttg gcgtggtggc actggtgctt 720
tatctgctgc acgccagaaa taacaaccgt gccctgttca gtctgaaact gttccgtact 780
cgtacctttt cgctgggcct ggcggggagc tttgccggac gtattggcag tggcatgttg 840
ccctttatga caccggtttt cctgcaaatt ggcctcggtt tctcgccgtt tcatgccgga 900
ctgatgatga tcccgatggt gcttggcagc atgggaatga agcgaattgt ggtacaggtg 960
gtgaatcgct ttggttatcg tcgggtactg gtagcgacca cgctgggtct gtcgctggtc 1020
accctgttgt ttatgactac cgccctgctg ggctggtact acgttttgcc gttcgtcctg 1080
tttttacaag ggatggtcaa ctcgacgcgt ttctcctcca tgaacaccct gacgctgaaa 1140
gatctcccgg acaatctggc gagcagcggc aacagcctgc tgtcgatgat tatgcaattg 1200
tcgatgagta tcggcgtcac tatcgccggg ctgttgctgg gacttttggg ttcacagcat 1260
gtcagcgtcg acagcggcac cacacaaacc gtctttatgt acacctggct tagcatggcg 1320
ttgatcatcg cccttccggc gttcatcttt gccagagtgc cgaacgatac gcatcaaaat 1380
gtagctattt cgcggcgaaa aaggagcgcg caatga 1416

<210> 125
<211> 1035
<212> DNA
<213> E. Coli

<400> 125
atggaaattc gcataatgct atttatatta atgatgatgg ttatgcctgt gagctatgcg 60
gcatgttata gtgagttatc tgttcagcac aacttggttg ttcaggggga ttttgcactt 120
actcaaacac aaatggcgac atatgagcat aattttaatg attcgtcatg cgtaagtaca 180
aatactatca cccctatgag cccgtcggat attattgttg gactttataa cgataccata 240
aaattaaatt tacattttga atggaccaat aaaaacaaca tcacgttgtc aaataatcag 300
accagtttca ccagtggtta ttcagttacg gtgacacctg cggccagtaa tgcaaaagtg 360
aatgtttctg cggggggcgg cggttcagtg atgattaatg gtgttgcgac attatccagt 420
gcttcatcat cgacacgcgg gagtgccgca gtacaatttc tactgtgttt attaggtggc 480
aagtcatggg atgcatgtgt aaatagctac agaaatgcat tggcacaaaa tgcaggtgtc 540
tattccttta atctgacatt gtcatacaac ccgataacca caacctgcaa accggacgat 600
ttattaatta ctttagacag tattcccgtt tcacaattac cagccacagg taacaaagca 660
acaataaata gtaaacaagg ggatattatt ctgcgttgta aaaatttatt aggtcaacaa 720
aatcaaacat cacggaaaat gcaggtgtat ttatcaagtt ctgacttgtt aaccaacagc 780
aacacaatac tgaaaggtgc ggaagataat ggcgtaggat ttattcttga aagtaatggt 840
tcgccagtca cacttttaaa tatcactaac agcagtaaag gatatacaaa tttaaaggaa 900
gttgcggcga agtcaaaact tacagataca acggtttcaa ttccgataac agccagttac 960
tacgtctacg atacaaacaa agttaaatct ggcgcactgg aggcaaccgc attaatcaac 1020
gtgaaatacg actaa 1035

<210> 126
<211> 2481
<212> DNA
<213> E. Coli

<400> 126
atgttgagaa tgaccccact tgcatcagca atcgtagcgt tattgctcgg cattgaagct 60
tatgcagctg aagaaacctt tgatacccat tttatgatag gtggaatgaa agaccagcag 120
gttgcaaata ttcgtcttga tgataatcaa cccttaccgg ggcagtatga catcgatatt 180
tatgtcaata agcaatggcg cgggaaatat gagattattg ttaaagacaa cccgcaagaa 240
acatgtttat caagagaagt tatcaagcgg ttaggcatta atagcgataa cttcgccagc 300
ggtaagcaat gtttaacatt tgagcaactt gttcagggtg ggagctatac ctgggatatc 360
ggggtttttc gtctcgattt cagtgtcccg caggcctggg tggaagaact ggaaagtggc 420
tatgttccac cggaaaactg ggagcggggt attaatgcgt tttatacctc ttattatctg 480
agtcagtatt acagcgacta taaagcgtcg ggtaataaca agagtacata tgtacgtttt 540
aacagcgggt taaatttact ggggtggcaa ctgcattctg atgccagttt cagtaaaaca 600
aataacaatc caggggtgtg gaaaagcaat accctgtatc tggaacgtgg atttgcccaa 660
cttctcggca cgcttcgcgt gggtgatatg tacacatcaa gcgatatttt tgattctgtt 720
cgcttcagag gtgtgcggtt gtttcgtgat atgcagatgt tgcctaactc gaaacaaaat 780
tttacgccac gggtgcaggg gattgctcag agtaacgcgc tggtaactat tgaacagaat 840
ggttttgtgg tttatcagaa agaggttcct cctggcccgt tcgcgattac agatttgcag 900
ttggccggtg gtggagcaga tcttgatgtc agcgtgaaag aggcggacgg ctcggtaacc 960

acctatctgg tgccttatgc agcggtgcca aatatgctgc aacccggcgt gtcgaaatat 1020
gatttagcgg cgggtcgtag ccatattgaa ggggcgagca aacaaagtga ttttgtccag 1080
gcgggttatc agtatggttt taataattta ttgacgctgt atggtggctc gatggtcgcg 1140
aataattatt acgcgtttac tttgggggct ggctggaata cacgcattgg tgccatttcc 1200
gtcgatgcca ctaagtcgca tagtaaacaa gacaacggcg atgtgtttga cgggcaaagt 1260
tatcaaattg cctacaacaa atttgtgagc caaacgtcga cgcgttttgg tctggcggcc 1320
tggcgttatt cgtcgcgtga ttaccggaca tttaacgatc acgtttgggc aaacaataaa 1380
gataattatc gccgtgatga aaacgatgtc tatgacattg ccgattatta ccagaacgat 1440
tttggccgca aaaatagctt ttccgccaat atgagccagt cattgccaga aggttggggg 1500
tctgtgtcat taagtacgtt atggcgagat tactgggggc gtagcggcag tagtaaggat 1560
tatcagttga gttattccaa caacctgcga cggataagct ataccctcgc ggcaagccag 1620
gcttatgacg agaatcatca tgaagagaaa cgttttaata tttttatatc gattcccttt 1680
gattggggtg atgacgtttc gacgcctcgt cggcaaatat atatgtctaa ctcaacgacg 1740
tttgatgatc aggggtttgc ctcaaataat acgggattat caggaacagt agggagtcgg 1800
gatcagttca attatggtgt caacctgagt catcaacatc agggaaatga aacgacagct 1860
ggggcgaatt tgacctggaa cgcgccggtt gcgacagtga atggcagtta tagtcagtcg 1920
agtacttatc gacaggctgg agccagtgtt tcagggggca ttgtcgcctg gtcgggtggc 1980
gttaatctgg cgaaccgtct ttccgaaacg tttgctgtga tgaatgcgcc aggaattaaa 2040
gatgcttatg tcaatgggca aaaatatcgc acaacaaacc gtaatggagt ggtgatatac 2100
gacggaatga caccttatcg ggaaaatcac ctgatgctgg atgtgtcgca aagcgatagc 2160
gaagcagaat tacgtggcaa ccggaaaatt gccgcccctt atcgcggcgc ggttgtactg 2220
gttaattttg ataccgatca gcgcaagcca tggtttataa aagcgttaag agcagatggg 2280
caatcattaa cgtttggtta tgaagtcaat gatatccatg gtcataatat tggcgttgtc 2340
ggccagggaa gtcagttatt tattcgcacc aatgaagtac cgccatcggt taatgtggca 2400
attgataagc aacaaggact ttcatgcaca atcaccttcg gtaaagagat tgatgaaagt 2460
agaaattata tttgccagta a 2481

<210> 127
<211> 720
<212> DNA
<213> E. Coli

<400> 127
   atggccgcta tcccatggcg gccttttaat ttaagaggca ttaaaatgaa aggattatta 60
   tctttactca ttttttctat ggtccttcct gcacatgccg gaattgttat ctacgggacg 120
   cgcattattt acccggcaga aaataaagaa gtgatggtgc agttgatgaa ccagggaaac 180
   cgttcttcgc tgctgcaggc gtggattgat gatggcgata cgtcattacc accagaaaaa 240
   attcaggttc ctttcatgtt aacgccacca gtggcaaaaa taggggcaaa ttccgggcag 300
   caagtaaaaa tcaaaattat gccgaataaa ctgcccacta ataaagaaag cattttttat 360
   ctgaatgttc tggatattcc accaaatagt ccagagcaag aaggtaagaa tgcactgaag 420
   tttgcgatgc aaaacagaat taagttgttt taccggccag cgggtattgc tccggtaaat 480
   aaagcgacat ttaaaaaatt gctggtaaat cgcagtggca atggtttggt gataaaaaat 540
   gactcagcta attgggtgac gatttcggat gtcaaagcta ataatgtcaa agtcaattat 600
   gaaactatta tgattgcccc cttagaaagt cagagtgtta atgtcaaaag taataatgca 660
   aataactggc atctgaccat tatcgatgac catggcaact atattagtga caaaatttaa 720

<210> 128
<211> 543
<212> DNA
<213> E. Coli

<400> 128
atgaaacgtt caattattgc tgccgctgtc ttttcttctt tttttatgag cgctggagta 60
tttgctgcag acgttgatac cggaacatta actattaagg ggaatattgc agaatctccg 120
tgtaaattcg aagcgggtgg tgattcagta agtattaata tgccgactgt accaaccagt 180
gtctttgaag gtaaagctaa atattctacc tatgatgatg cagtcggtgt aaccagcagc 240
atgttaaaaa ttagctgccc gaaagaagtt gctggtgtaa aactctcgtt gattaccaac 300
gataaaataa ccggtaacga taaggcgata gccagtagca acgataccgt gggttactat 360
ctctatttag gtgataacag cgatgtcctg gatgtttctg caccttttaa cattgagagt 420
tataaaacag cggaaggtca atatgctatt ccgtttaaag caaaatacct gaaactgaca 480
gataactcag tgcaatcagg tgatgtgtta tcttctctgg ttatgcgtgt ggcgcaggat 540

taa                                                                543

<210> 129
<211> 339
<212> DNA
<213> E. Coli

<400> 129
atgagttcag agcgagatct ggttaatttt cttggcgatt tttcaatgga tgtggccaaa      60
gcagttatag ccggtggtgt tgcaaccgct attggaagtc tggcttcttt tgcctgtgtt     120
agctttggct ttccagtaat tcttgtcgga ggagcaattt tactgacagg gatagtgtgt     180
acagttgttt taaatgaaat cgatgctcaa tgccatttat cagaaaaatt aaaatatgca     240
attagagatg gactaaaacg gcaacaggaa cttgataaat ggaaaaggga aaacatgact     300
ccatttatgt atgttcttaa cactccaccc gtgatatga                            339

<210> 130
<211> 582
<212> DNA
<213> E. Coli

<400> 130
atgactgact acctgttact gtttgtcgga actgtactgg tcaataactt tgtactggtc      60
aagtttctcg gtctctgtcc gtttatgggg gtttccaaaa aactggaaac cgcgatgggc     120
atggggctgg caacaacgtt tgtgatgacg ctggcgtcta tttgcgcctg gcttatcgat     180
acgtggattt tgatcccact taatctgatt tacctgcgca ccctggcatt tattctggtg     240
attgctgtgg tcgtgcagtt caccgagatg gtggtgcgca aaaccagccc ggtgctttac     300
cgtctgctgg ggattttttt gccgcttatc accaccaact gtgcagtgct cggcgtggcg     360
ttgctgaata tcaatctcgg gcacaatttc ttgcagtcgg cgctgtacgg tttttccgcc     420
gctgtcggtt tctcgctggt gatggtgctc ttcgccgcca tccgcgaacg ccttgctgtg     480
gctgatgtcc cggcaccttt tcgcggtaat gccattgcgt taattaccgc aggtcttatg     540
tctctggcct ttatgggctt tagtggtttg gtgaagttgt aa                        582

<210> 131
<211> 579
<212> DNA
<213> E. Coli

<400> 131
atgaatgcta tctggattgc cgttgccgcc gtgagcctgc tgggcctggc gtttggcgcc      60
attctgggtt atgcctcccg ccgttttgcg gtggaagacg atccggtcgt tgagaaaatt     120
gacgaaatct taccgcagag ccagtgtggt cagtgcggtt atcccggctg tcgcccctac     180
gcggaagcca tcagctgtaa cggtgaaaaa atcaaccgtt gcgccccagg tggcgaagct     240
gtgatgctaa aaattgccga gttgcttaat gtcgagccgc agccgctgga tggcgaagcg     300
caagagataa cgcctgcgcg gatggtggcg gttattgatg aaaataactg tattggctgc     360
actaaatgta ttcaggcgtg tccggtagac gccatcgttg gcgctacccg tgccatgcat     420
acggtaatga gtgatctctg tacgggctgc aatttatgtg ttgatccgtg cccgacgcac     480
tgcatctcgt tgcaaccggt cgcagaaaca cctgactcct ggaaatggga tctgaacacc     540
attcccgtgc gtatcattcc cgtggaacac catgcttaa                            579

<210> 132
<211> 2223
<212> DNA
<213> E. Coli

<400> 132
atgcttaagt tattctctgc attcagaaaa aataaaatct gggatttcaa cggcggcatc      60
catccaccgg agatgaaaac ccagtccaac ggtacacccc tgcgccaggt acccctggcg     120
cagcgttttg ttattccact gaaacagcat attggcgctg aaggtgagtt gtgcgttagc     180
gtcggcgata aagtattgcg cggccagccg cttacccgtg gtcgcggcaa aatgctgcct     240
gttcacgcgc ccacctcggg taccgttacg gctattgcgc cccactctac ggctcatcct     300
tcagctttag ctgaattaag cgtgattatt gatgccgatg gtgaagactg ctggatcccg     360

cgcgacggct gggccgatta tcgcactcgc agtcgcgaag agttaatcga gcgcatacat 420
cagtttggtg ttgccgggct gggcggtgca ggattcccga caggcgttaa attgcagggt 480
ggcggagata agattgaaac gttgattatc aacgcggctg agtgcgagcc gtacattacc 540
gccgatgacc gtttgatgca ggattgcgcg gctcaggtcg tagagggtat tcgcattctt 600
gcgcatattc tgcagccacg cgaaattctt atcggcattg aagataacaa accgcaggcg 660
atttccatgc tgcgcgcggt gctggcggac tctaacgata tttctctgcg ggtgattcca 720
accaaatatc cttctggcgg tgctaaacaa ttaacctaca ttctgaccgg gaagcaggtt 780
ccacatggcg ggcgttcatc cgatatcggc gtattaatgc aaaacgtcgg cactgcttat 840
gcagtgaaac gtgccgttat tgatggcgag ccgattaccg agcgtgttgt aaccctgact 900
ggcgaagcaa tcgctcgccc gggcaacgtc tgggcacggc tggggacgcc agtgcgtcat 960
ttattgaatg atgccggatt ctgcccctct gccgatcaaa tggtgattat gggtggcccg 1020
ctaatgggct ttaccttgcc atggctggat gtcccggtcg taaagattac caactgtctg 1080
ttggctccct ctgccaatga acttggcgaa ccacaggaag aacaaagctg catccggtgt 1140
agcgcctgtg ctgacgcctg ccctgcggat cttttgccgc aacagttgta ctggttcagc 1200
aaaggtcagc aacacgataa agctaccacg cataacattg ctgattgcat tgaatgtggg 1260
gcttgcgcgt gggtttgccc gagcaatatt cccctggtgc aatatttccg tcaggaaaaa 1320
gctgaaattg cggctattcg tcaggaagaa aagcgcgccg cagaagccaa agcgcgtttc 1380
gaagcgcgcc aggctcgtct ggagcgcgaa aaagcggctc gccttgaacg acataagagc 1440
gcagccgttc aacctgcagc caaagataaa gatgcgattg ctgccgctct ggcgcgggtg 1500
aaagagaaac aggcccaggc tacacagcct attgtgatta aagcgggcga acgcccggat 1560
aacagtgcaa ttattgcagc acgggaagcc cgtaaagcgc aagccagagc gaaacaggca 1620
gaactgcagc aaactaacga cgcagcaacc gttgctgatc cacgtaaaac tgccgttgaa 1680
gcagctatcg cccgcgccaa agcgcgcaag ctggaacagc aacaggctaa tgcggaacca 1740
gaacaacagg tcgatccgcg caaagccgcc gtcgaagccg ctattgcccg tgccaaagcg 1800
cgcaagctgg aacagcaaca ggctaatgcg gaaccagaag aacaggtcga tccgcgcaaa 1860
gccgccgtcg aagccgctat tgcccgtgcc aaagcacgca agctggaaca gcaacaggct 1920
aatgccgagc cagaacaaca ggtcgatccg cgcaaagccg ccgtcgaagc cgctattgcc 1980
cgagccaaag cgcgcaaacg ggaacagcaa ccggctaatg cggagccaga agaacaggtt 2040
gatccgcgca aagctgccgt cgaagcggct attgcacgcg ccaaagcacg caagctggaa 2100
cagcaacagg ctaatgcggt accagaagaa caggttgatc cgcgcaaagc ggcagttgcc 2160
gcggctattg cccgcgctca ggccaaaaaa gccgcccagc agaaggttgt aaacgaggac 2220
taa 2223

<210> 133
<211> 1059
<212> DNA
<213> E. Coli

<400> 133
atggtattca gaatagctag ctccccttat acccataacc agcgccagac atcgcgcatt 60
atgctgttgg tgttgctcgc agccgtgcca ggaatcgcag cgcaactgtg gttttttggt 120
tggggtactc tcgttcagat cctgttggca tcggttagtg ctctgttagc cgaagctctc 180
gtactcaaac tacgcaagca gtcggtagcc gcaacgttga aagataactc agcattgctg 240
acaggcttat tgctggcggt aagtattccc cccctcgcgc catggtggat ggtcgtgctg 300
ggtacggtgt ttgcggtgat catcgctaaa cagttgtatg gcggtctggg acaaaacccg 360
tttaatccgg caatgattgg ttatgtggtc ttactgatct ccttccccgt gcagatgacc 420
agctggttac cgccacatga aattgcggtc aacatccctg gttttatcga cgccatccag 480
gttattttta gcggtcatac cgccagtggt ggtgatatga acacactacg cttaggtatt 540
gatggcatta gtcaggcgac accgctggat acatttaaaa cctctgtccg tgccggtcat 600
tcggttgaac agattatgca atatccgatc tacagcggta ttctggcggg cgctggttgg 660
caatgggtaa atctcgcctg gctggctggc ggcgtatggt tgctatggca gaaagcgatt 720
cgctggcata ttcccctcag cttcttagta acgctggcgt tatgcgcaat gttgggctgg 780
ttgttctcac cagaaacact ggcagcaccg caaattcatc tgctgtctgg agcgaccatg 840
ctcggcgcat tctttatttt gactgacccg gttaccgctt ctacgaccaa tcgtggtcgt 900
cttattttcg gcgcgcttgc gggcttatta gtctggttga tccgcagttt cggcggctat 960
cctgacggcg tggcttttgc cgtcctgctg gcgaacatca cggttcctct gatcgattac 1020
tacacgcgtc cgcgcgtcta cggccatcgc aaagggtaa 1059

<210> 134
<211> 621
<212> DNA

<213> E. Coli

<400> 134
   atgctgaaaa ctatccgaaa acacggcatt acgttggcgc tatttgcagc gggttcaaca 60
   gggttaactg cggccatcaa ccagatgacc aaaacgacga ttgctgaaca ggccagtctg 120
   caacaaaagg cgttatttga tcaggtgctg ccagccgaac gctataacaa tgcgctggca 180
   cagagttgct atctggtaac tgcgccagag ttaggtaaag gtgagcatcg ggtttacatc 240
   gccaaacagg atgacaaacc ggtagccgcc gttctggaag caaccgcgcc agatggctat 300
   tccggtgcga ttcagctgct ggtgggagcc gattttaacg gcacggtact tggcacgcgc 360
   gtgacagagc accacgaaac gccagggctt ggcgataaaa tcgaactgcg cctttctgac 420
   tggatcaccc atttgcgggg taaaaaaatc agtggtgcag atgatgcgca ctgggcggtg 480
   aagaaagatg gtggtgattt cgaccagttc accggcgcga cgattactcc ccgcgcggtg 540
   gttaatgcgg taaaacgcgc cggattgtac gctcagacgt taccggcaca actttctcaa 600
   cttcctgcct gtggagaata a 621

<210> 135
<211> 696
<212> DNA
<213> E. Coli

<400> 135
gtgagcgaaa ttaaagacgt tattgttcag gggttgtgga aaaacaactc tgcgctggtc 60
cagttgctcg gcctttgtcc tctgttggcg gtcacgtcca ctgccactaa cgctctgggt 120
ttaggacttg cgactacgct ggtactgacg ctgaccaacc tgaccatttc gacgctgcgt 180
cactggacgc cagccgagat ccgcattccc atttacgtga tgatcatcgc ctcggtggtc 240
agcgctgtac agatgctgat caacgcctac gcctttggcc tgtatcaatc attagggatt 300
tttattccgc tgattgtcac taactgtatc gttgtgggcc gcgctgaagc cttcgccgcc 360
aaaaaaggtc cggcgctttc ggcactggac ggcttttcaa ttggtatggg cgcaacctgc 420
gccatgttcg tgctgggttc actacgcgaa attatcggca atggcacatt gtttgacggt 480
gcagatgcgc tgttaggtag ctgggcaaaa gtattacgcg tggagatttt ccacaccgac 540
tcccctttcc tgctggcgat gctgccacca ggtgcattta ttggcctggg actgatgctg 600
gcaggaaaat acctgattga tgaaagaatg aaaaagcgcc gtgctgaagc agctgcagaa 660
cgtgcattgc caaacggtga aacagggaat gtctga 696

<210> 136
<211> 636
<212> DNA
<213> E. Coli

<400> 136
atgaataaag caaaacgcct ggagatcctc actcgcctgc gtgagaacaa tcctcatccc 60
accaccgagc ttaatttcag ttcgcctttt gaattgctga ttgccgtact gctttccgct 120
caggcgaccg atgtcagtgt taataaggcg acggcgaaac tctacccggt ggcgaatacg 180
cctgcagcga tgcttgaact gggcgttgaa ggggtgaaaa cctatatcaa aacgattggg 240
ctttataaca gcaaagcaga aaatatcatc aaaacctgcc gtatcttgct ggagcagcat 300
aatggcgagg ttccggaaga tcgtgctgcg cttgaagccc tgcccggcgt aggtcgtaaa 360
acagccaacg tcgtattaaa cactgcattc ggctggccga ctattgctgt cgacacgcac 420
attttccgcg tttgtaatcg tactcaattt gcgccgggga aaaacgtcga acaggtagaa 480
gaaaagctac tgaaagtggt tccagcagag tttaaagtcg actgccacca ttggttgatc 540
ctgcacgggc gttatacctg cattgcccgc aagccccgct gtggctcttg tattattgaa 600
gatctttgtg aatacaaaga gaaagttgac atctga 636

<210> 137
<211> 504
<212> DNA
<213> E. Coli

<400> 137
atgaaaagac ttcacaagag gttcctgtta gctacgtttt gcgcgttatt cacagcaact 60
ctccaggccg ccgatgtcac tatcactgtt aatggtcggg tagtcgctaa accctgcact 120
attcaaacca aagaagctaa cgttaatctc ggggatcttt atacgcgcaa tctgcaacaa 180

cctggttctg catctggctg gcacaatatt actttgtcat taaccgattg tccggttgaa 240
acaagtgcag tgacggcaat cgtgacaggt tcaactgaca atacgggtta ttacaaaaat 300
gaaggtactg ccgaaaatat tcagatagag ctgagggatg accaggatgc tgcgttaaaa 360
aatggcgata gcaaaacggt tattgttgat gagatcactc gtaatgcaca gtttccactt 420
aaggcaagag ctatcacggt gaatggaaac gcaagccagg gaacgatcga ggcgctaatc 480
aatgtgatct acacctggca ataa 504

<210> 138
<211> 531
<212> DNA
<213> E. Coli

<400> 138
atgaaataca ataacattat tttcctcggt ttatgtctgg ggttaaccac ctattctgct 60
ttatccgcag atagcgttat taaaattagc gggcgcgtcc tcgattatgg ctgcacagtc 120
tcatcggatt cgcttaattt taccgtagat ctccaaaaaa acagtgccag acaatttcca 180
acgaccggta gcacaagtcc agccgtccct tttcagatta cgttaagtga atgcagcaaa 240
gggacaacgg gggttcgggt tgcatttaac ggtattgagg atgcagaaaa taatactttg 300
ttgaaactgg atgaaggaag caatacggcc tccggtttgg gtatagaaat attggacgca 360
aatatgcgtc cggtgaaact gaatgatctt catgccggga tgcagtggat cccactggta 420
ccagaacaga acaatatttt gccttactcc gctcgtctga agtcaactca gaagtccgtc 480
aatccgggac tggtgagggc ttcggcaacc tttacccttg aatttcaata a 531

<210> 139
<211> 1149
<212> DNA
<213> E. Coli

<400> 139
atgagtggtt acaccgtcaa gcctcctacc ggagacacca atgagcagac acaatttatt 60
gattatttta atctgttcta cagtaagcgt ggtcaggaac aaataagcat ctctcagcag 120
cttggaaatt acggtacgac atttttcagt gccagtcgcc aaagttactg gaacacgtca 180
cgcagcgacc agcaaatatc atttggatta aatgtgccgt ttggtgatat tacgacttcg 240
ctgaattaca gctattccaa taatatatgg caaaacgatc gggatcattt actcgctttt 300
acgcttaatg ttcccttcag tcattggatg cgtacagaca gtcagtcggc atttcgtaat 360
tcaaacgcca gttacagtat gtcaaacgat ttgaaaggcg gcatgaccaa tctatcgggg 420
gtttatggca ctctgctgcc ggataataac ctgaattata gcgttcaggt cggtaacacc 480
cacggaggta atacatcgtc tggcaccagt ggttacagtt ctcttaatta tcgtggagct 540
tatggtaata ctaatgtcgg ttacagtcgg agtggtgaca gcagccagat ttattacgga 600
atgagtggtg ggattattgc tcatgctgat ggcatcacct ttggacagcc gctgggcgac 660
acaatggttc tggttaaggc tcctggtgct gataatgtca aaatagagaa ccagaccgga 720
attcataccg actggcgtgg ctatgccata ttaccatttg cgacagaata tagagaaaac 780
cgtgttgctc ttaacgcgaa ttcccttgca gataatgttg aactggatga aaccgtggtc 840
actgtcatcc caactcacgg tgctattgcc agagcaacat ttaatgcaca aatcggcggg 900
aaagtattaa tgacgttgaa gtacggtaat aagagcgttc cattcggtgc aattgtcaca 960
cacggagaga ataaaaatgg cagcattgtc gcggaaaatg gtcaggttta tctgactgga 1020
cttccacagt cagggcaatt acaggtttca tggggcaaag ataaaaactc aaactgtatt 1080
gtcgagtaca agcttcctga agtttctcct ggtaccttac tgaaccagca gacagcaatc 1140
tgtcgctaa 1149

<210> 140
<211> 417
<212> DNA
<213> E. Coli

<400> 140
atgattgcga ttgccgacat cttgcaagca ggagaaaagc taactgctgt ggcacctttt 60
ctggcgggta ttcagaacga ggaacaatac acccaggcgc tggaactggt agatcatctg 120
ctgctcaacg atcctgaaaa ccccttgctg gatctggtgt gtgccaaaat aaccgcgtgg 180
gaagaatcag cgcccgaatt tgcggaattt aatgccatgg ctcaagccat gcctggcggt 240
atagccgtga ttcgtaccct tatggatcaa tatggtttaa ccctttccga tctgccggaa 300

attggcagta aatctatggt gtcacgcgtt ttgagcggga agaggaaatt aacgctggaa 360
cacgctaaaa aattggcaac gcgattcggc atttctcccg ccttgtttat tgattaa 417

<210> 141
<211> 315
<212> DNA
<213> E. Coli

<400> 141
atgcacctga taactcaaaa agcattgaaa gatgctgcgg aaaaataccc gcaacataaa 60
acggagttgg tggctctggg gaacacgatt gctaagggat atttcaaaaa acctgagtca 120
ttaaaagcag tattcccatc tctggataac ttcaaatatc tggataagca ttatgttttc 180
aatgttgggg gcaatgaatt acgtgttgta gcaatggtct tttttgaatc gcaaaagtgc 240
tacatacgtg aagttatgac gcataaagaa tacgatttct ttaccgctgt tcatcgtact 300
aaggggaaaa aatga 315

<210> 142
<211> 7152
<212> DNA
<213> E. Coli

<400> 142
ttgctatcag tatttacatt ttttcgctgt gctagaaagg gcgcatttat gttagctcgt 60
tcagggaagg taagcatggc tacgaagaag agaagtggag aagaaataaa tgaccgacaa 120
atattatgcg ggatgggaat taaactacgc cgcttaactg cgggtatctg tctgataact 180
caacttgcgt tccctatggc tgcggcagca caaggtgtgg taaacgccgc aacccaacaa 240
ccagttcctg cacaaattgc cattgcaaat gccaatacgg tgccctacac ccttggagcg 300
ttggaatcgg cccaaagcgt tgccgaacgt ttcggtattt cggtggctga gttacgcaaa 360
ctcaaccagt ttcgtacgtt tgctcgaagt tttgataatg tccgccaggg tgatgaactg 420
gatgtcccgg cacaagttag tgaaaaaaaa ttaaccccgc cgccgggtaa tagcagtgac 480
aacctcgagc aacagatagc cagtacttca cagcaaatcg ggtctctgct cgccgaagat 540
atgaacagcg agcaagcggc aaatatggcg cgtggatggg cctcttctca ggcttcaggc 600
gcaatgacag actggttaag ccgcttcggt accgcaagaa tcacgctggg cgtggatgaa 660
gattttagcc tgaagaactc ccagttcgat tttctccatc cgtggtatga aacgcctgat 720
aatctctttt tcagtcagca tactctccat cgtactgacg agcgtacgca gattaacaac 780
ggcttaggtt ggcgtcattt cactcccaca tggatgtcgg gcatcaactt ctttttcgac 840
cacgatctta gccgttacca ctcccgcgcc ggcattggcg cggagtactg gcgcgactat 900
ctaaaattaa gcagtaacgg ctatttgcga ctgaccaact ggcgcagcgc acctgaactg 960
gacaacgatt atgaagcacg cccggccaat ggctgggatg tacgcgcaga aagctggcta 1020
cccgcctggc cgcaccttgg cggtaaactg gtctatgaac agtattatgg cgatgaagtg 1080
gccctgttcg ataaagacga tcggcaaagt aatcctcatg ccataaccgc tggacttaac 1140
tataccccct tcccgctgat gaccttcagc gcggagcaac gccagggtaa acagggcgaa 1200
aatgacaccc gttttgccgt cgattttacc tggcaacctg gcagcgcaat gcagaaacag 1260
cttgacccga atgaagtcgc tgcacggcgt agccttgcag gcagccgtta tgatctggtg 1320
gatcgcaaca acaatatcgt tctggaatat cgcaaaaaag aactggttcg cctgaccctg 1380
acagaccccg tgacagggaa gtcaggagaa gtgaaatcac tggtttcgtc gctacaaacc 1440
aaatatgccc tgaaaggcta taacgtcgaa gccaccgcac tggaagctgc cggtggcaaa 1500
gtggtcacaa cgggtaaaga tattctggtt accctgccgg cttaccggtt caccagtacg 1560
ccagaaaccg ataacacctg gccgattgaa gtcaccgccg aagatgtcaa aggcaatttg 1620
tcgaatcgtg aacagagcat ggtggtcgtt caggcaccta cgctaagcca gaaagattcc 1680
tcggtatcgt taagtaccca aacattgaac gcggattccc attcaaccgc cacactgact 1740
tttattgcgc atgatgcagc aggtaatcct gttgtcgggc tggtgctctc gacgcgtcac 1800
gaaggtgttc aggacatcac cctttctgac tggaaagata atggtgacgg aagctatacc 1860
cagatcctga ccacaggagc gatgtctggc acgctgacgc tgatgccaca gctgaatggt 1920
gtggatgcgg ctaaagcccc cgccgtggtg aatatcattt ctgtttcgtc atcccgaact 1980
cactcgtcaa ttaagattga taaggaccgt tatctctccg gcaatcctat cgaggtgacg 2040
gtagaactga gagatgaaaa tgacaaacct gttaaggaac aaaaacagca actgaataac 2100
gcagtcagca tcgacaacgt gaaaccagga gtcactacag actggaaaga aaccgcagat 2160
ggcgtctata aggcgaccta taccgcctat accaaaggca gtggacttac tgcgaagcta 2220
ttaatgcaaa actggaatga agatttgcat accgctggat ttatcatcga cgccaacccg 2280
cagtcagcga aaattgcgac attatctgcc agcaataatg gtgtgctcgc caatgagaat 2340

gcagcaaaca ccgtctcggt caatgtcgct gatgaaggaa gcaacccaat caatgatcat 2400
accgtcacgt ttgcggtatt aagcggatcg gcaacttcct tcaacaatca aaacaccgca 2460
aaaacggatg ttaatggtct ggcgactttt gatctgaaaa gtagtaagca ggaagacaac 2520
acggttgaag tcacccttga aaatggcgtg aaacaaacgt taatcgtcag ttttgtcggc 2580
gactcgagta ctgcgcaggt tgatctgcag aagtcgaaaa atgaagtggt tgctgacggc 2640
aatgacagcg tcacaatgac cgcgaccgtc cgggatgcaa aaggcaacct gctcaatgac 2700
gtcatggtca ctttcaatgt taattcagca gaggcgaaac tgagccaaac cgaagtgaat 2760
agccacgacg ggatcgccac agctacgctg accagtttga aaaatggtga ttatagggtt 2820
acggcctctg tgagctctgg ttcccaggct aatcaacagg tgaattttat cggtgatcaa 2880
agtactgctg ccctgaccct cagtgtgcct tcaggtgata tcaccgtcac caacacagct 2940
ccgcaatata tgactgcaac cttgcaggat aaaaatggca acccactaaa agataaagaa 3000
atcaccttct ctgtgccaaa cgacgtcgca agtaagttct cgattagcaa cggaggaaaa 3060
ggcatgacgg atagtaacgg ggttgcaatc gcctccctga ccggcacgtt agcgggcacg 3120
catatgatca tggctcgtct ggctaacagc aatgtcagcg atgcacagcc aatgacgttt 3180
gtggcggata aagacagagc ggttgtcgtt ttgcaaacat cgaaagcgga aatcattggg 3240
aatggcgtgg atgagacaac tctgacagca acagtgaaag atccgtcgaa tcatccggtg 3300
gcggggataa cggtaaactt caccatgcca caggacgttg cggcaaactt tacccttgaa 3360
aataacggta ttgccatcac tcaggccaat ggggaagcgc atgtcacgct gaaaggtaaa 3420
aaagcgggca cgcatacggt taccgcaacg ctgggtaata acaataccag tgattcgcag 3480
ccggtaacat ttgtggcgga caaagcctcg gctcaggttg tcctgcagat atcaaaagat 3540
gagatcacag gtaatggcgt cgatagcgca acgctaactg caacggttaa agatcagttc 3600
gacaatgagg tgaataatct tccggtaaca ttcagctcag cctcttcagg actcaccctg 3660
accccgggag taagtaatac caacgagtct ggcatcgcgc aggccactct cgcaggcgtt 3720
gcctttggtg agaagacggt tactgcatca ctggctaata atggtgccag cgacaacaaa 3780
actgtgcatt ttattggcga cacagcggcg gcaaaaatta tcgagttggc gcctgtccca 3840
gacagcataa tcgccggtac cccgcagaac agctccggca gcgtcatcac cgccacagtc 3900
gttgataata atggctttcc ggtgaaaggt gtgactgtga acttcaccag caacgcagcg 3960
acagccgaaa tgacgaacgg cggtcaagcc gtgacgaacg aacagggtaa ggctaccgtc 4020
acttatacca atacccgctc ctcgatagaa tcaggagcga gaccggatac cgttgaggcc 4080
agtctggaaa atggtagctc cacgcttagc acatcaatta atgtcaacgc tgatgcgtct 4140
acggcacatc tcaccttgct acaggcactt tttgatacag tctccgcagg cgagacaacc 4200
agtctgtata ttgaggtgaa ggataattac ggcaacggtg tcccccagca ggaggtaacc 4260
ctcagcgttt caccaagtga aggcgtgacc cccagtaata acgctatata tactaccaac 4320
cacgacggca atttttacgc aagctttacc gctacaaaag ccggggttta tcaattgacg 4380
gcaaccctcg aaaatggcga ttcgatgcaa caaacagtga cctatgtgcc gaacgtcgcg 4440
aatgctgaaa tcacgctggc agcctcgaag gatccggtga ttgccgacaa taacgatctc 4500
acgacactaa cagcaacagt cgctgataca gagggcaatg cgatagccaa cactgaggta 4560
acatttactc tgccggaaga tgtgaaggcg aacttcacgc tgagcgatgg cggtaaagtg 4620
attactgatg ctgaaggcaa agcgaaagtc acgctgaaag gtacaaaagc aggcgctcat 4680
actgttacag catcgatgac tggcggtaag agtgagcagt tggtggtgaa ctttattgcg 4740
gatacgctca ctgcgcaggt taatcttaac gttaccgagg acaattttat cgctaataac 4800
gtcgggatga ccaggctgca ggcaacagtg actgatggaa acggcaaccc gttagccaat 4860
gaggcggtga cattcacgct accggcagat gtgagcgcaa gctttactct cggacaaggc 4920
ggttccgcca ttactgatat caacggcaag gctgaagtta cactgagcgg tacaaaatcc 4980
ggcacctacc ccgtgacagt tagcgtgaac aattatggtg tcagtgatac gaaacaggtg 5040
actttgattg ccgatgctgg taccgcaaaa ctagcctcct taacctctgt atactcattc 5100
gtcgtcagca cgaccgaggg cgcaaccatg acggcaagcg tcactgacgc taacggcaac 5160
ccggtagaag gcataaaagt taatttccgc ggaacctccg tcacgctaag cagcaccagc 5220
gttgaaacgg atgatcgggg tttcgctgaa attcttgtga caagcaccga ggtcggactg 5280
aaaacagttt cagcctctct ggcagataaa cctactgaag tcatctcgcg attactgaat 5340
gccagtgcag atgttaattc tgcgacgatt accagtctgg agataccgga aggtcaggta 5400
atggtcgcac aagacgtagc agttaaagct cacgttaacg accagtttgg caacccggtt 5460
gcgcatcaac ccgtgacatt cagtgcagag ccatcctcgc aaatgatcat cagccagaat 5520
acggtctcta ctaatacgca gggtgtagcc gaggtcacca tgacgcccga aagaaacggt 5580
tcgtatatgg tgaaagcatc cctgccgaat ggagcctcac ttgagaaaca actggaggct 5640
attgatgaaa aactgacact cacggcgtcc agtccgctta tcggtgtcta tgcccctaca 5700
ggcgctactc tgacggcaac gctaacctct gcaaatggca ctccagtgga gggtcaggtc 5760
atcaacttta gcgtaacgcc agaaggggcg acgttaagtg gcggaaaagt gagaactaac 5820
tcttcaggtc aggctccagt cgttttgacc agcaataaag tcggtacata tacggtgact 5880
gcatctttcc ataacggcgt aacaatacag acacagacaa ccgtgaaagt cactggcaac 5940
tcaagcaccg cccatgttgc tagctttatc gctgatccat cgactatcgc cgccaccaac 6000

actgatttaa gtaccttaaa ggcaacggtt gaggatggca gtggtaacct gatcgaaggt 6060
ctcactgtgt acttcgcctt aaaaagcggc tctgccacat taacgtcatt aacagcggtg 6120
accgatcaaa acggaatcgc gacaacaagc gtgaaaggag cgatgacagg tagcgtcacg 6180
gtaagcgcag tcacgaccgc tggtggaatg caaacagtag atataacgct ggtggctggc 6240
ccggcagaca cctcgcagtc cgtccttaag agcaatcggt catcactgaa aggggactat 6300
accgatagtg ctgaattacg tcttgttctg cacgatatat caggcaatcc gatcaaagtt 6360
tctgaaggga tggaatttgt gcaatcaggt actaacgtgc cctatataaa aattagcgca 6420
attgattaca gtctaaatat caacggtgat tacaaagcca ctgttacagg aggcggagag 6480
ggtatcgcaa cgctgatccc tgtattgaat ggtgttcatc aagctggtct gagtaccaca 6540
atacaattca ctcgcgcaga agacaaaata atgagcggta cagtatcagt caatggtact 6600
gacctaccga caactacatt cccttcgcag gggttcaccg gggcgtatta tcagttgaat 6660
aatgacaact ttgccccagg aaaaacggcg gctgattatg agttttcaag ctctgcctcc 6720
tgggtcgatg ttgatgctac cggtaaagtg acatttaaaa atgtcggcag caattcggaa 6780
aggattacgg cgacgccaaa atcaggaggc cctagctatg tatacgaaat ccgtgtgaag 6840
agttggtggg tgaacgccgg cgaggctttc atgatataca gccttgctga aaatttttgc 6900
agcagcaatg gctacacgct ccccagagca aactatttaa accactgtag ttcccgaggc 6960
atcgggtcac tgtacagtga atggggagat atggggcatt acacgactga cgctggtttt 7020
caatcaaata tgtattggtc atctagtccc gcaaactcaa gcgaacaata cgtagtttcc 7080
ctggcaacag gtgatcaaag cgtatttgaa aagcttgggt ttgcttatgc gacatgttat 7140
aaaaacctgt ga 7152
<210> 143
<211> 186
<212> DNA
<213> E. Coli

<400> 143
atgagcaaag gcgcattata tgaatttaac aatccagatc aactgaaaat acctctccct 60
cataaacaca tagcgtcaac attcaatgac ataatgagta aagatgttgg ttatgcatac 120
gtatcattac tctatgcctg tcccttaaaa acccactcat taagactgaa tccattcagc 180
aaatga 186

<210> 144
<211> 1197
<212> DNA
<213> E. Coli

<400> 144
atgcaggtgg ctgaacagcg cattcagcta gctgaagccc aggcgaaggc agttgccact 60
caggatggtc cgcagatcga cttttcggcg gatatggagc ggcaaaaaat gtcggcagaa 120
ggcttaatgg ggccgtttgc tctgaacgat ccggccgcag gtacgaccgg cccgtggtac 180
accaacggta cttttggctt aacggcgggc tggcatctcg atatctgggg aaagaatcgg 240
gcggaggtta ctgcccgcct gggtacggtt aaagcacggg cggcggaacg cgagcaaacc 300
cgccaattgc tggctggcag cgtagcccgc ctgtactggg agtggcaaac ccaggcggcg 360
ttaaacacgg tcttgcagca aatagaaaaa gagcagaaca ccattatcgc gaccgatcgc 420
cagctatatc agaacgggat tacttcttca gttgaaggtg tggaaaccga tattaatgcc 480
agcaaaaccc ggcagcagct caacgatgtc gcggggaaaa tgaaaattat tgaggcacgg 540
ttaagcgcac ttacaaataa ccagacaaag tcattgaagc ttaaaccggt cgcgttgccg 600
aaagtggcaa gccagcttcc tgatgaactg gggtactcct tactggcccg gcgggcagat 660
ttgcaggcgg cgcactggta cgttgagtca tcgctaagca ccattgatgc ggcaaaagcg 720
gcattttatc ctgacatcaa cctgatggcc ttcctgcaac aggatgcgtt gcacttaagc 780
gatctgttcc gtcattccgc gcagcaaatg ggcgttacgg caggcctgac gctacccatt 840
ttcgatagtg gtcgtcttaa cgccaatctc gatatcgcaa aagccgaaag caacttgtct 900
atcgccagct acaacaaagc ggtggttgaa gcggtgaatg acgtggcgcg ggcagccagt 960
caggttcaga cactggcgga gaaaaaccag catcaggcgc aaattgagcg cgatgccttg 1020
cgtgtggtag gtcttgcgca ggcgcgcttt aacgcgggca tcattgctgg ttcccgcgtc 1080
agcgaagcca gaatccccgc gctgcgtgag cgggccaatg gcctgttatt gcaagggcag 1140
tggctggatg cctccattca actcactggt gcgttgggcg gggggtacaa acgctga 1197

<210> 145
<211> 291
<212> DNA

<213> E. Coli

<400> 145
atgtattgcc acgcgaaact aaaaaatata tcgcaacaca cggtaatctc cgcgcacctt 60
ttcttacctg attattcccc catgaatcgt gattcctttt atccagccat cgcctgtttt 120
ccgctgttac tgatgctggc cgggtgtgcg cctatgcatg aaacccgcca ggcgttaagc 180
cagcaaacgc ccgctgcaca agttgacacc gcattaccca cggcgctgaa aatggttggc 240
cagacagcca atggtggctg gagtatcacg ataatcaact cacttcctta a 291

<210> 146
<211> 948
<212> DNA
<213> E. Coli

<400> 146
atgcgtgtgt tactggcacc gatggaggga gtgcttgact ctctggtgcg tgaattgctg 60
accgaagtta acgactacga tctgtgcatc accgagtttg tccgcgtggt ggatcaactg 120
ctgccggtaa aagtctttca tcgcatttgc cctgagctac aaaacgccag ccggacacca 180
tctggtacgc tggtgcgcgt gcagttgtta ggtcagttcc cacaatggct ggcagagaac 240
gccgcccgtg cggtggagtt aggttcctgg ggcgtggatc tcaattgcgg ctgcccgtcg 300
aaaacggtta acggtagcgg cggcggggcg acgttactca aagatcctga actcatctac 360
cagggtgcaa aagcgatgcg tgaagctgta ccggcgcatt tgcccgtcag cgtgaaagtg 420
cgtctgggct gggacagcgg tgagaagaaa tttgaaatcg ccgatgcggt tcaacaggct 480
ggcgctacgg agctggtggt gcatgggcgg acgaaagagc agggttaccg cgcggagcat 540
attgactggc aggcgattgg cgatattcgc cagcggctga atattccggt gattgccaac 600
ggtgaaatct gggactggca gagcgcgcaa caatgcatgg cgatcagcgg ctgcgacgca 660
gtgatgattg gtcgcggggc gctcaatatt cccaacctga gccgggtggt aaaatataac 720
gaaccgcgaa tgccgtggcc ggaggtggtt gctttgctgc aaaaatatac ccgtctggaa 780
aagcagggcg ataccgggtt atatcacgtt gcgcggatta aacagtggtt gagttatttg 840
cgtaaagaat acgatgaagc aacggaatta tttcagcatg ttcgggtgtt gaataattcc 900
cctgatattg caagggctat tcaggcaatt gatatcgaga aactctaa 948

<210> 147
<211> 891
<212> DNA
<213> E. Coli

<400> 147
atgacaatat cgacaacttc cacgccgcat gatgcggtat ttaaatcttt tttacgccat 60
ccagacaccg cgcgggattt tattgatatt catcttcccg cgccgctgcg caaactgtgt 120
gatttaacga cgcttaaact ggaaccaaac agttttattg atgaagacct gcggcaatat 180
tattccgacc tcttgtggtc tgtgaaaacg caggagggag tgggttatat ttatgtagtg 240
atagagcacc aaagtaagcc ggaagaatta atggcttttc gcatgatgcg ttattccatt 300
gcggcaatgc aaaaccatct tgatgcgggc tataaagagc ttccattggt gctcccgatg 360
ctgttttatc atggttgcag aagtccttat ccttattcac tctgctggct tgatgaattt 420
gccgagcctg ctatagcccg caaaatatat tcatcggctt ttccgttggt ggatattacc 480
gtggtgccgg atgacgagat tatgcaacac cgcaaaatgg cgctgttgga gttaattcag 540
aaacatattc gtcagcgcga tctgttggga ttagtcgacc aaattgtttc gctgctagtt 600
acagggaaca ctaatgacag acagctaaaa gccctgttta attacgtatt acaaacaggg 660
gatgcccagc gttttcgtgc atttattggt gagatagcgg aacgcgcacc acaagaaaag 720
gagaaactga tgaccattgc tgacagatta cgtgaagaag gcgcaatgca gggcaaacac 780
gaagaagccc tgcgtattgc tcaggagatg ctggatagag gtttagacag agagttagtt 840
atgatggtga cccgactttc accagacgat cttatcgcgc aaagccacta a 891

<210> 148
<211> 1668
<212> DNA
<213> E. Coli

<400> 148
gtggctcaat tcgtttatac catgcatcgt gtcggcaaag ttgttccgcc gaaacgtcat 60

attttgaaaa acatctctct gagtttcttc cctggggcaa aaattggtgt cctgggtctg 120
aatggcgcgg gtaagtccac cctgctgcgc attatggcgg gcattgataa agacatcgaa 180
ggtgaagcgc gtccgcagcc agacatcaag attggttatc tgccgcagga accgcagctg 240
aacccggaac acaccgtgcg tgagtccatt gaagaagcgg tttcagaagt ggttaacgcc 300
ctgaaacgcc tggatgaagt gtatgcgctg tacgccgatc cggatgccga ttttgacaag 360
ctggccgctg aacaaggccg tctggaagag atcattcagg ctcacgacgg tcataatctg 420
aacgtacagc tggagcgtgc ggcggatgcg ctacgtctgc cggactggga cgcgaaaatc 480
gctaacctct ccggtggtga acgtcgtcgc gtagcgttgt gccgcctgct gctggaaaaa 540
ccagacatgc tgctgctcga cgaaccgacc aaccacctgg atgccgaatc cgtggcctgg 600
ctggaacgct tcctgcacga cttcgaaggc accgttgtgg cgattaccca cgaccgttac 660
ttcctcgata acgttgcagg ctggatcctc gaacttgacc gcggtgaagg tattccgtgg 720
gaaggtaact actcctcctg gctggagcag aaagatcagc gcctggcgca ggaagcttca 780
caagaagcgg cgcgtcgtaa gtcgattgag aaagagctgg aatgggtacg tcaaggtact 840
aaaggccgtc agtcgaaagg taaagcacgt ctggcgcgct ttgaagaact gaacagcacc 900
gaatatcaga aacgtaacga aaccaacgaa ctgtttattc cacctggacc gcgtctgggc 960
gataaagtgc tggaagtcag caacctgcgt aaatcctatg gcgatcgtct gctgattgat 1020
gacctgagct tctcgatccc gaaaggagcg atcgtcggga tcatcggtcc gaacggtgcg 1080
ggtaaatcga ccctgttccg tatgatctct ggtcaggaac agccggacag cggcaccatc 1140
actttgggtg aaacggtgaa actggcgtcg gttgatcagt tccgtgactc aatggataac 1200
agcaaaaccg tttgggaaga agtttccggc gggctggata tcatgaagat cggcaacacc 1260
gagatgccaa gccgcgccta cgttggccgc tttaacttta aaggggttga tcagggtaaa 1320
cgcgttggtg aactctccgg tggtgagcgc ggtcgtctgc atctggcgaa gctgctgcag 1380
gttggcggca acatgctgct gctcgacgaa ccaaccaacg acctggatat cgaaaccctg 1440
cgcgcgctgg aaaacgccct gctggagttc ccgggctgtg cgatggttat ctcgcacgac 1500
cgttggttcc tcgaccgtat cgccacgcac attctggatt accaggatga aggtaaagtt 1560
gagttcttcg aaggtaactt taccgagtac gaagagtaca agaaacgcac gctgggcgca 1620
gacgcgctgg agccgaagcg tatcaagtac aagcgtattg cgaagtaa 1668

<210> 149
<211> 522
<212> DNA
<213> E. Coli

<400> 149
atgtcaaagc caaaataccc ttttgaaaag cgccttgaag tcgtgaatca ctacttcaca 60
actgatgatg gttacaggat catctcggca cgttttggtg tcccccgaac ccaggtcagg 120
acatgggttg ccctctatga aaaacatgga gaaaaaggtt taattcccaa acctaaaggc 180
gttagtgctg atccagagtt gcgtattaag gtcgtgaaag ctgtgatcga gcagcacatg 240
tcccttaatc aggctgctgc tcactttatg cttgctggta gtggttctgt agccaggtgg 300
ctgaaggtct atgaagagcg cggagaagct ggtttacgcg cgctcaagat tggcaccaaa 360
agaaacattg caatatcagt tgatccagaa aaagcggcat cagcattgga gctgtcaaaa 420
gaccgacgca ttgaggatct tgaaaggcaa gttcgatttc ttgaaacgcg gcttatgtat 480
ctaaaaaagc tgaaagcctt agctcatccc acgaaaaagt ga 522

<210> 150
<211> 852
<212> DNA
<213> E. Coli

<400> 150
   gtgaaagtac tcaacgagct aaggcagttt tatcctcttg atgagcttct cagggctgcg 60
   gagataccgc gcagtacgtt ttattatcat ctaaaggctc tcagcaagcc tgacaagtat 120
   gcggacgtta aaaagcgtat tagtgagatt tatcacgaga atagaggccg atacggatac 180
   cgtagggtaa cgctgtctct tcatcgagaa gggaaacaga ttaaccataa agctgttcag 240
   cgcctgatgg gaaccctctc acttaaagca gcgattaagg tcaagcgata ccgctcttac 300
   agaggagagg tagggcaaac cgcccctaat gttctccaaa gagatttcaa ggctacgcgg 360
   ccaaacgaga agtgggttac cgatgttact gaatttgcag tcaatggcgg caagctgtat 420
   ttgtctccag taatagatct cttcaacaac gaagttattt cttacagcct ttcggaaaga 480
   ccagtgatga acatggttga gaatatgctc gatcaggcat tcaaaaagct taatcctcac 540
   gagcatcctg ttctgcactc tgaccaggga tggcagtatc gtatgagaag atatcaaaat 600
   atccttaaag aacatggtat taaacaaagc atgtccagaa aaggcaattg tctggataat 660
   gctgtggtgg agtgtttctt tggaacctta aagtcggagt gtttttatct tgatgagttc 720
   agtaatataa gcgaactgaa ggatgctgtt acggaatata ttgaatacta caacagcaga 780
   agaattagcc tgaaattaaa aggtctgact ccaattgaat atcggaatca gacctatatg 840
   cctcgtgttt aa 852

<210> 151
<211> 117
<212> DNA
<213> E. Coli

<400> 151
atgaaagttc gtgcttccgt caagaaatta tgccgtaact gcaaaatcgt taagcgtgat 60
ggtgtcatcc gtgtgatttg cagtgccgag ccgaagcata aacagcgcca aggctga 117

<210> 152
<211> 1332
<212> DNA
<213> E. Coli

<400> 152
atggctaaac aaccgggatt agattttcaa agtgccaaag gtggcttagg cgagctgaaa 60
cgcagactgc tgtttgttat cggtgcgctg attgtgttcc gtattggctc ttttattccg 120
atccctggta ttgatgccgc tgtacttgcc aaactgcttg agcaacagcg aggcaccatc 180
attgagatgt ttaacatgtt ctctggtggt gctctcagcc gtgcttctat ctttgctctg 240
gggatcatgc cgtatatttc ggcgtcgatc attatccagc tgctgacggt ggttcaccca 300
acgttggcag aaattaagaa agaaggggag tctggtcgtc gtaagatcag ccagtacacc 360
cgctacggta ctctggtgct ggcaatattc cagtcgatcg gtattgctac cggtctgccg 420
aatatgcctg gtatgcaagg cctggtgatt aacccgggct ttgcattcta cttcaccgct 480
gttgtaagtc tggtcacagg aaccatgttc ctgatgtggt tgggcgaaca gattactgaa 540
cgaggtatcg gcaacggtat ttcaatcatt atcttcgccg gtattgtcgc gggactcccg 600
ccagccattg cccatactat cgagcaagcg cgtcaaggcg acctgcactt cctcgtgttg 660
ctgttggttg cagtattagt atttgcagtg acgttctttg ttgtatttgt tgagcgtggt 720
caacgccgca ttgtggtaaa ctacgcgaaa cgtcagcaag gtcgtcgtgt ctatgctgca 780
cagagcacac atttaccgct gaaagtgaat atggcggggg taatcccggc aatcttcgct 840
tccagtatta ttctgttccc ggcgaccatc gcgtcatggt tcgggggcgg tactggttgg 900
aactggctga caacaatttc gctgtatttg cagcctgggc aaccgcttta tgtgttactc 960
tatgcgtctg caatcatctt cttctgtttc ttctacacgg cgttggtttt caacccgcgt 1020
gaaacagcag ataacctgaa gaagtccggt gcatttgtac caggaattcg tccgggagag 1080
caaacggcga agtatatcga taaagtaatg acccgcctga ccctggttgg tgcgctgtat 1140
attaccttta tctgcctgat cccggagttc atgcgtgatg caatgaaagt accgttctac 1200
ttcggtggga cctcactgct tatcgttgtt gtcgtgatta tggactttat ggctcaagtg 1260
caaactctga tgatgtccag tcagtatgag tctgcattga agaaggcgaa cctgaaaggc 1320
tacggccgat aa 1332

<210> 153
<211> 435
<212> DNA
<213> E. Coli

<400> 153
atgcgtttaa atactctgtc tccggccgaa ggctccaaaa aggcgggtaa acgcctgggt 60
cgtggtatcg gttctggcct cggtaaaacc ggtggtcgtg gtcacaaagg tcagaagtct 120
cgttctggcg gtggcgtacg tcgcggtttc gagggtggtc agatgcctct gtaccgtcgt 180
ctgccgaaat tcggcttcac ttctcgtaaa gcagcgatta cagccgaaat tcgtctgtct 240
gacctggcta aagtagaagg cggtgtagta gacctgaaca cgctgaaagc ggctaacatt 300
atcggtatcc agatcgagtt cgcgaaagtg atcctggctg gcgaagtaac gactccggta 360
actgttcgtg gcctgcgtgt tactaaaggc gctcgtgctg ctatcgaagc tgctggcggt 420
aaaatcgagg aataa 435

<210> 154

<211> 180
<212> DNA
<213> E. Coli

<400> 154
atggcaaaga ctattaaaat tactcaaacc cgcagtgcaa tcggtcgtct gccgaaacac 60
aaggcaacgc tgcttggcct gggtctgcgt cgtattggtc acaccgtaga gcgcgaggat 120
actcctgcta ttcgcggtat gatcaacgcg gtttccttca tggttaaagt tgaggagtaa 180

<210> 155
<211> 504
<212> DNA
<213> E. Coli

<400> 155
atggctcaca tcgaaaaaca agctggcgaa ctgcaggaaa agctgatcgc ggtaaaccgc 60
gtatctaaaa ccgttaaagg tggtcgtatt ttctccttca cagctctgac tgtagttggc 120
gatggtaacg gtcgcgttgg ttttggttac ggtaaagcgc gtgaagttcc agcagcgatc 180
cagaaagcga tggaaaaagc ccgtcgcaat atgattaacg tcgcgctgaa taacggcact 240
ctgcaacacc ctgttaaagg tgttcacacg ggttctcgcg tattcatgca gccggcttcc 300
gaaggtaccg gtatcatcgc cggtggtgca atgcgcgccg ttctggaagt cgctggggtt 360
cataacgttc tggctaaagc ctatggttcc accaacccga tcaacgtggt tcgtgcaact 420
attgatggcc tggaaaatat gaattctcca gaaatggtcg ctgccaagcg tggtaaatcc 480
gttgaagaaa ttctggggaa ataa 504

<210> 156
<211> 354
<212> DNA
<213> E. Coli

<400> 156
atggataaga aatctgctcg tatccgtcgt gcgacccgcg cacgccgcaa gctccaggag 60
ctgggcgcaa ctcgcctggt ggtacatcgt accccgcgtc acatttacgc acaggtaatt 120
gcaccgaacg gttctgaagt tctggtagct gcttctactg tagaaaaagc tatcgctgaa 180
caactgaagt acaccggtaa caaagacgcg gctgcagctg tgggtaaagc tgtcgctgaa 240
cgcgctctgg aaaaaggcat caaagatgta tcctttgacc gttccgggtt ccaatatcat 300
ggtcgtgtcc aggcactggc agatgctgcc cgtgaagctg gccttcagtt ctaa 354

<210> 157
<211> 534
<212> DNA
<213> E. Coli

<400> 157
atgtctcgtg ttgctaaagc accggtcgtt gttcctgccg gcgttgacgt aaaaatcaac 60
ggtcaggtta ttacgatcaa aggtaaaaac ggcgagctga ctcgtactct caacgatgct 120
gttgaagtta aacatgcaga taataccctg accttcggtc cgcgtgatgg ttacgcagac 180
ggttgggcac aggctggtac cgcgcgtgcc ctgctgaact caatggttat cggtgttacc 240
gaaggcttca ctaagaagct gcagctggtt ggtgtaggtt accgtgcagc ggttaaaggc 300
aatgtgatta acctgtctct gggtttctct catcctgttg accatcagct gcctgcgggt 360
atcactgctg aatgtccgac tcagactgaa atcgtgctga aaggcgctga taagcaggtg 420
atcggccagg ttgcagcgga tctgcgcgcc taccgtcgtc ctgagcctta taaaggcaag 480
ggtgttcgtt acgccgacga agtcgtgcgt accaaagagg ctaagaagaa gtaa 534

<210> 158
<211> 393
<212> DNA
<213> E. Coli

<400> 158
atgagcatgc aagatccgat cgcggatatg ctgacccgta tccgtaacgg tcaggccgcg 60

aacaaagctg cggtcaccat gccttcctcc aagctgaaag tggcaatcgc caacgtgctg 120
aaggaagaag gttttattga agattttaaa gttgaaggcg acaccaagcc tgaactggaa 180
cttactctga agtatttcca gggcaaagct gttgtagaaa gcattcagcg tgtcagccgc 240
ccaggtctgc gcatctataa acgtaaagat gagctgccga aagttatggc gggtctgggt 300
atcgcagttg tttctacctc taaaggtgtt atgactgatc gtgcagcgcg ccaggctggt 360
cttggtggcg aaattatctg ctacgtagcc taa 393

<210> 159
<211> 306
<212> DNA
<213> E. Coli

<400> 159
atggctaagc aatcaatgaa agcacgcgaa gtaaaacgcg tagctttagc tgataaatac 60
ttcgcgaaac gcgctgaact gaaagcgatc atctctgatg tgaacgcttc cgacgaagat 120
cgttggaacg ctgttctcaa gctgcagact ctgccgcgtg attccagccc gtctcgtcag 180
cgtaaccgct gccgtcaaac aggtcgtccg catggtttcc tgcggaagtt cgggttgagc 240
cgtattaagg tccgtgaagc cgctatgcgc ggtgaaatcc cgggtctgaa aaaggctagc 300
tggtaa 306

<210> 160
<211> 540
<212> DNA
<213> E. Coli

<400> 160
atggcgaaac tgcatgatta ctacaaagac gaagtagtta aaaaactcat gactgagttt 60
aactacaatt ctgtcatgca agtccctcgg gtcgagaaga tcaccctgaa catgggtgtt 120
ggtgaagcga tcgctgacaa aaaactgctg gataacgcag cagcagacct ggcagcaatc 180
tccggtcaaa aaccgctgat caccaaagca cgcaaatctg ttgcaggctt caaaatccgt 240
cagggctatc cgatcggctg taaagtaact ctgcgtggcg aacgcatgtg ggagttcttt 300
gagcgcctga tcactattgc tgtacctcgt atccgtgact tccgtggcct gtccgctaag 360
tctttcgacg gtcgtggtaa ctacagcatg ggtgtccgtg agcagatcat cttcccagaa 420
atcgactacg ataaagtcga ccgcgttcgt ggtttggata ttaccattac cactactgcg 480
aaatctgacg aagaaggccg cgctctgctg gctgcctttg acttcccgtt ccgcaagtaa 540

<210> 161
<211> 315
<212> DNA
<213> E. Coli

<400> 161
atggcagcga aaatccgtcg tgatgacgaa gttatcgtgt taaccggtaa agataaaggt 60
aaacgcggta aagttaagaa tgtcctgtct tccggcaagg tcattgttga aggtatcaac 120
ctggttaaga aacatcagaa gccggttccg gccctgaacc aaccgggtgg catcgttgaa 180
aaagaagccg ctattcaggt ttccaacgta gcaatcttca atgcggcaac cggcaaggct 240
gaccgtgtag gctttagatt cgaagacggt aaaaaagtcc gtttcttcaa gtctaacagc 300
gaaactatca agtaa 315

<210> 162
<211> 372
<212> DNA
<213> E. Coli

<400> 162
atgatccaag aacagactat gctgaacgtc gccgacaact ccggtgcacg tcgcgtaatg 60
tgtatcaagg ttctgggtgg ctcgcaccgt cgctacgcag gcgtaggcga catcatcaag 120
atcaccatca aagaagcaat tccgcgtggt aaggtcaaaa aaggtgatgt gctgaaggcg 180
gtagtggtgc gcaccaagaa gggtgttcgt cgcccggacg gttctgtcat tcgcttcgat 240
ggtaatgctt gtgttcttct gaacaacaac agcgagcagc ctatcggtac gcgtattttt 300
gggccggtaa ctcgtgagct tcgtagtgag aagttcatga aaattatctc tctggcacca 360

gaagtactct aa                                                          372

<210> 163
<211> 567
<212> DNA
<213> E. Coli

<400> 163
atgtttaaag gacaaaaaac attggccgca ctggccgtat ctctgctgtt cactgcacct      60
gtttatgctg ctgatgaagg ttctggcgaa attcacttta agggggaggt tattgaagca      120
ccttgtgaaa ttcatccaga agatattgat aaaaacatag atcttggaca agtcacgaca      180
acccatataa accgggagca tcatagcaat aaagtggccg tcgacattcg cttgatcaac      240
tgtgatctgc ctgcttctga caacggtagc ggaatgccgg tatccaaagt tggcgtaacc      300
ttcgatagca cggctaagac aactggtgct acgcctttgt tgagcaacac cagtgcaggc      360
gaagcaactg gggtcggtgt acgactgatg gacaaaaatg acggtaacat cgtattaggt      420
tcagccgcgc cagatcttga cctggatgca agctcatcag aacagacgct gaactttttc      480
gcctggatgg aacaaattga taatgcagtc gatgtcacgg caggtgaagt aaccgctaac      540
gcaacctacg tgctggatta taaataa                                          567

<210> 164
<211> 1284
<212> DNA
<213> E. Coli

<400> 164
atggctgata caaaagcaaa actcaccctc aacggggata cagctgttga actggatgtg      60
ctgaaaggca cgctgggtca agatgttatt gatatccgta ctctcggttc aaaaggtgtg      120
ttcacctttg acccaggctt cacttcaacc gcatcctgcg aatctaaaat tacttttatt      180
gatggtgatg aaggtatttt gctgcaccgc ggtttcccga tcgatcagct ggcgaccgat      240
tctaactacc tggaagtttg ttacatcctg ctgaatggtg aaaaaccgac tcaggaacag      300
tatgacgaat ttaaaactac ggtgacccgt cataccatga tccacgagca gattacccgt      360
ctgttccatg ctttccgtcg cgactcgcat ccaatggcag tcatgtgtgg tattaccggc      420
gcgctggcgg cgttctatca cgactcgctg gatgttaaca atcctcgtca ccgtgaaatt      480
gccgcgttcc gcctgctgtc gaaaatgccg accatggccg cgatgtgtta caagtattcc      540
attggtcagc catttgttta cccgcgcaac gatctctcct acgccggtaa cttcctgaat      600
atgatgttct ccacgccgtg cgaaccgtat gaagttaatc cgattctgga acgtgctatg      660
gaccgtattc tgatcctgca cgctgaccat gaacagaacg cctctacctc caccgtgcgt      720
accgctggct cttcgggtgc gaacccgttt gcctgtatcg cagcaggtat tgcttcactg      780
tggggacctg cgcacggcgg tgctaacgaa gcggcgctga aaatgctgga agaaatcagc      840
tccgttaaac acattccgga atttgttcgt cgtgcgaaag acaaaaatga ttctttccgc      900
ctgatgggct tcggtcaccg cgtgtacaaa aattacgacc cgcgcgccac cgtaatgcgt      960
gaaacctgcc atgaagtgct gaaagagctg ggcacgaagg atgacctgct ggaagtggct      1020
atggagctgg aaaacatcgc gctgaacgac ccgtacttta tcgagaagaa actgtacccg      1080
aacgtcgatt tctactctgg tatcatcctg aaagcgatgg gtattccgtc ttccatgttc      1140
accgtcattt tcgcaatggc acgtaccgtt ggctggatcg cccactggag cgaaatgcac      1200
agtgacggta tgaagattgc ccgtccgcgt cagctgtata caggatatga aaaacgcgac      1260
tttaaaagcg atatcaagcg ttaa                                             1284

<210> 165
<211> 1434
<212> DNA
<213> E. Coli

<400> 165
atgaaagtaa cgctgccaga gtttgaacgt gcaggagtga tggtggttgg tgatgtgatg      60
ctggatcgtt actggtacgg ccccaccagt cgtatctcgc cggaagcgcc ggtgcccgtg      120
gttaaagtga ataccatcga agaacgtccg ggcggcgcgg ctaacgtggc gatgaatatc      180
gcttctctcg gtgctaatgc acgcctggtc gggttgacgg gcattgacga tgcagcgcgc      240
gcgctgagta aatctctggc cgacgtcaac gtcaaatgcg acttcgtttc tgtaccgacg      300
catccgacca ttaccaaatt acgggtactt tcccgcaacc aacagctgat ccgtctggat      360
tttgaagaag gtttcgaagg tgttgatccg cagccgctgc acgagcggat taatcaggcg      420

ctgagttcga ttggcgcgct ggtgctttct gactacgcca aaggtgcgct ggcaagcgta 480
cagcagatga tccaactggc gcgtaaagcg ggtgttccgg tgctgattga tccaaaaggt 540
accgattttg agcgctaccg cggcgctacg ctgttaacgc cgaatctctc ggaatttgaa 600
gctgttgtcg gtaaatgtaa gaccgaagaa gagattgttg agcgcggcat gaaactgatt 660
gccgattacg aactctcggc tctgttagtg acccgttccg aacagggtat gtcgctgctg 720
caaccgggta aagcgccgct gcatatgcca acccaagcgc aggaagtgta tgacgttacc 780
ggtgcgggcg acacggtgat tggcgtcctg gcggcaacgc tggcagcggg taattcgctg 840
gaagaagcct gcttctttgc caatgcggcg gctggcgtgg tggtcggcaa actgggaacc 900
tccacggttt cgccgatcga gctggaaaat gctgtacgtg gacgtgcaga tacaggcttt 960
ggcgtgatga ccgaagagga actgaagctg gccgtagcgg cagcgcgtaa acgtggtgaa 1020
aaagtggtga tgaccaacgg tgtctttgac atcctgcacg ccgggcacgt ctcttatctg 1080
gcaaatgccc gcaagctggg tgaccgcttg attgttgccg tcaacagcga tgcctccacc 1140
aaacggctga aaggggattc ccgcccggta aacccactcg aacagcgtat gattgtgctg 1200
ggcgcactgg aagcggtcga ctgggtagtg tcgtttgaag aggacacgcc gcagcgcttg 1260
atcgccggga tcttgccaga tctgctggtg aaaggcggcg actataaacc agaagagatt 1320
gccgggagta aagaagtctg ggccaacggt ggcgaagtgt tggtgctcaa ctttgaagac 1380
ggttgctcga cgaccaacat catcaagaag atccaacagg ataaaaaagg ctaa 1434

<210> 166
<211> 2841
<212> DNA
<213> E. Coli

<400> 166
atgaagccgc tctcttcacc gttacagcag tactggcaga ccgttgttga gcggctgcca 60
gagcctttag ccgaggaatc acttagcgca caggcgaagt cagtacttac ttttagtgat 120
tttgtgcagg acagcgtgat tgcgcatcca gagtggctga cggaactgga aagccaaccg 180
ccgcaggccg acgaatggca gcattacgcg gcatggttgc aggaggcgct ctgtaatgtg 240
agtgacgaag ccgggttaat gcgcgagctg cggctattcc ggcggcgcat tatggtgcgc 300
atcgcctggg cgcaaacgct ggcactggtt actgaagaga gcatattgca gcagctcagc 360
tatctggcgg agacgctgat tgttgcggcg cgtgactggc tgtatgacgc ctgctgccgc 420
gagtggggaa cgccgtgcaa tgcgcagggc gaagcgcaac cgctgctgat tttaggcatg 480
ggtaagctgg gcggtgggga gctgaatttc tcctctgata tcgatctgat ttttgcctgg 540
ccggaacatg gttgtacgca gggtggacgc cgggaactgg ataacgcgca gttttttacc 600
cgcatggggc agcggctgat taaagtgctg gatcaaccaa cgcaggatgg cttcgtctat 660
cgcgtggata tgcggctgcg tccgtttggc gaaagtggcc cgctggtgct gagctttgcc 720
gcgttggaag attattacca ggagcagggg cgcgactggg agcgttacgc gatggtcaag 780
gcgcggatta tgggcgatag cgaaggcgtc tatgctaacg agttgcgtgc gatgctgcgc 840
ccgtttgttt tccgtcgtta catcgatttc agcgtgattc agtcgctgcg caacatgaaa 900
gggatgattg cccgtgaagt gcgtcgacgt ggtttgaccg acaatatcaa actcggcgca 960
ggcggcattc gcgaaattga atttatcgtt caggtgttcc agctcattcg cggcggacgc 1020
gaaccgtcgc tgcaatcgcg ctctttactg ccaacgctca gcgccattgc cgagctgcat 1080
ctgctttctg aaaacgatgc tgaacaattg cgagtggcgt atctgttcct gcggcgtctg 1140
gaaaacctgc tgcaaagcat taacgacgaa caaacccaga cgcttccttc tgatgagctt 1200
aatcgtgcgc ggctggcgtg ggcgatggac tttgctgact ggccgcaact gaccggggcg 1260
ctgaccgcac atatgaccaa tgtgcgccgg gtgtttaatg aattgattgg cgacgatgaa 1320
agtgaaactc aggaagagtc gctgtcggaa cagtggcgtg agctgtggca ggatgcgttg 1380
caggaagatg acactacgcc agtgctggcg catcttagcg aggatgatcg caaacaggtg 1440
ctaacgctga ttgccgattt ccgcaaagag ctggataagc gcaccatcgg gccgcgagga 1500
cgtcaggtgc tcgaccatct gatgccgcat ctgctaagtg atgtctgtgc gcgtgaagac 1560
gctgccgtta cgctgtcgcg cattaccgcc ttgctggtgg ggattgttac ccgcaccacc 1620
tatttagaat tgctcagtga attccccgcg gcgcttaaac atttgatttc tctgtgtgcc 1680
gcgtcgccga tgattgccag ccagctggcg cgttatccat tattgctgga tgaattgctc 1740
gatccaaaca ccctttacca gccgacggcg accgatgcct accgcgatga gttgcgccag 1800
tatttgctgc gcgtgccgga agatgacgaa gagcaacagc ttgaggcgct gcgtcagttc 1860
aaacaggcgc agctgttacg catcgccgca gcggatatcg ccggtacgct accggtgatg 1920
aaagtgagcg atcacttaac ctggctggcg gaagccatga tagatgccgt cgttcagcag 1980
gcgtgggttc aaatggttgc ccgctacggt aagccgaatc acctgaacga acgcgaaggg 2040
cgtggttttg cggtggtcgg ctacggcaag ctgggcggct gggagttagg ctacagttcc 2100
gatcttgacc ttatcttcct ccatgattgc ccaatggatg cgatgactga cggtgagcgg 2160
gaaatcgacg ggcggcagtt ttatctgcgt ctggcgcaac gcattatgca tctgttcagt 2220

acgcgtacct cttccggcat tttgtatgaa gtggatgctc gactgcgtcc gtccggggcg 2280
gcgggaatgc tggtgacatc cgcagaagca tttgccgatt atcagaaaaa cgaggcctgg 2340
acgtgggaac atcaggcgct ggtgcgtgcg cgtgtagtgt acggcgatcc gcagctcacc 2400
gcgcactttg acgcagtgcg tcgcgagatt atgacgctgc cgcgtgaagg taaaactctg 2460
caaacggaag tgcgggaaat gcgcgagaaa atgcgcgctc atctcggcaa taaacatcgc 2520
gatcgctttg atatcaaagc tgatgaaggg ggaattaccg atatcgaatt tattacccaa 2580
tatctggtgt tgcgctacgc tcatgaaaaa ccgaagttaa cgcgctggtc agacaacgtg 2640
cgtattctgg aactactggc gcaaaacgac attatggaag agcaggaagc gatggcgctg 2700
acccgtgctt acactacgct tcgcgatgaa cttcatcatc tggcattaca ggaattgccg 2760
ggccatgtgt cggaggattg cttcaccgca gagcgtgaac tggtgcgggc aagctggcag 2820
aagtggctgg tggaagaatg a 2841

<210> 167
<211> 1302
<212> DNA
<213> E. Coli

<400> 167
atggctcagg aaatcgaatt aaagtttatt gttaatcaca gtgccgttga ggcgttgcgt 60
gaccatctca atacgctggg cggcgagcac catgaccccg tgcagttgct gaatatttac 120
tacgaaacgc cggataactg gctgcgtggg cacgatatgg gcttacgtat tcgtggcgaa 180
aacggtcgct atgagatgac catgaaagtt gcaggaagag tgacaggcgg cttacatcag 240
cgcccggaat ataacgtggc gttgagcgaa ccgacgctcg acctggcgca gttaccgacg 300
gaagtctggc cgaacggcga attgcccgcc gatctcgcct cccgcgtgca gccgctgttc 360
agcaccgatt tttatcgcga aaaatggctg gtggcggtcg atggtagcca aattgaaatc 420
gccctcgacc agggggaagt gaaagcgggt gaatttgctg aacctatctg tgagctggaa 480
ctggaactgc ttagcggcga cacgcgcgcg gtgctgaaac tggcgaacca actggtatcg 540
caaaccggat tacgccaggg cagcctgagc aaagcggcgc gtggctatca tctggcgcag 600
ggcaatccgg cgcgtgaaat caaaccgacc accattttgc atgttgcggc aaaagccgat 660
gtggaacagg ggctggaagc ggcgctcgag ctggcgttag cgcaatggca gtatcatgaa 720
gaactgtggg tacgcggcaa cgatgcggcg aaagaacagg tgctggcagc cattagcctg 780
gtccgtcata cgctgatgct gttcggtggt attgtgccgc gtaaagcgag cactcactta 840
cgtgatctgc tgactcaatg cgaggcgacc attgcttctg cggtgtctgc cgtgacggcg 900
gtctactcta ccgaaacggc aatggcgaag ctggcgttga ccgaatggtt ggtaagcaaa 960
gcatggcagc catttttaga tgccaaagcg cagggcaaaa tcagcgactc cttcaaacgc 1020
tttgccgata tccatctttc ccgccatgcc gctgaactga aaagcgtttt ctgccagccg 1080
ttaggcgatc gctaccgtga ccagttgcca cgcctgacgc gtgatattga ctcaatactg 1140
ttgctggcgg gttactatga tcctgtcgtc gcgcaagcct ggctggagaa ctggcagggg 1200
ctgcatcacg ctattgcgac cgggcaacgc atcgaaattg aacatttccg taatgaggca 1260
aacaatcagg aaccgttctg gttgcacagc ggaaaacgtt aa 1302

<210> 168
<211> 213
<212> DNA
<213> E. Coli

<400> 168
atgtccggta aaatgactgg tatcgtaaaa tggttcaacg ctgacaaagg cttcggcttc 60
atcactcctg acgatggctc taaagatgtg ttcgtacact tctctgctat ccagaacgat 120
ggttacaaat ctctggacga aggtcagaaa gtgtccttca ccatcgaaag cggcgctaaa 180
ggcccggcag ctggtaacgt aaccagcctg taa 213

<210> 169
<211> 1572
<212> DNA
<213> E. Coli

<400> 169
atgagggaca ttgtggaccc tgtattctct atcggtatct catcattatg ggatgagctg 60
cgacatatgc cagcaggcgg cgtctggtgg tttaacgtcg atcgccatga agatgctatc 120
agtctggcga atcaaacaat tgcatcccag gctgaaaccg cacacgtcgc ggtcattagc 180

atggacagcg atccggcgaa aatctttcaa ttagatgatt ctcaagggcc ggaaaaaata 240
aaattatttt caatgctaaa tcatgaaaaa ggtctatact atttgacccg tgatttgcag 300
tgttctattg atccccataa ttaccttttt attcttgttt gcgcaaataa cgcatggcaa 360
aacattcctg ccgagcggct tcgctcatgg ttggataaaa tgaataaatg gagcaggtta 420
aaccattgtt cgcttttggt aattaatccc ggaaataata acgataaaca attttcattg 480
ttgcttgagg aataccgttc actttttggt cttgccagtt tgcgttttca gggtgaccaa 540
catttgctgg atattgcctt ctggtgcaac gaaaaagggg tcagcgcccg tcagcagctt 600
agcgttcagc aacaaaatgg tatctggaca ttagttcaaa gcgaagaggc ggagatccaa 660
ccacgcagcg acgaaaaacg cattctgagt aatgttgctg tactggaagg tgcgccgccg 720
ctatcggaac actggcaact gttcaacaat aacgaagtcc tgttcaatga agcccgtacc 780
gctcaggcgg cgacggtggt cttttcttta cagcaaaatg cgcaaatcga gccactggcc 840
cgcagcattc ataccctgcg tcgccagcgc ggtagtgcga tgaaaatcct cgtgcgggaa 900
aataccgcta gcctgcgcgc caccgatgaa cgtttgttat tggcctgcgg tgcaaatatg 960
gttattccgt ggaatgcgcc actctcccgt tgtctgacga tgatcgaaag cgtgcaaggg 1020
cagaagttta gtcgctatgt gccggaagat atcactacct tgctgtcaat gacccagccg 1080
ctcaaactgc gtggtttcca gaagtgggat gtgttctgta atgccgtcaa caacatgatg 1140
aataaccctc tattacctgc ccacggtaaa ggcgttctgg ttgccctacg tccggtaccg 1200
ggtatccgcg ttgaacaagc cctgacgctg tgtcgcccta accgtaccgg cgatatcatg 1260
accattggcg gtaatcggct ggtgctgttt ctctcattct gtcggattaa cgatctggat 1320
accgcgttga atcatatttt cccattgcct actggcgaca ttttctcaaa ccgtatggtc 1380
tggtttgaag atgatcaaat cagtgccgag ctggtgcaga tgcgcttgct tgccccagaa 1440
caatggggca tgccgctgcc tttaacgcaa agttctaaac cggtcatcaa tgccgagcac 1500
gatggtcgcc actggcgacg aataccagaa cccatgcgac tgttagatga tgctgtggag 1560
cgctcatcat ga 1572

<210> 170
<211> 189
<212> DNA
<213> E. Coli

<400> 170
atgaccatca gcgatatcat tgaaattatt gtcgtttgcg cactgatatt tttcccgctg 60
ggctatctgg cgcggcactc tttgcgacgc attcgcgaca ccttacgttt gttctttgct 120
aaacctcgtt atgttaaacc ggccgggacg ttacgccgca cggaaaaagc cagggcaacc 180
aaaaaatga 189

<210> 171
<211> 1680
<212> DNA
<213> E. Coli

<400> 171
atgactcaat ttacgcaaaa taccgccatg ccttcttccc tctggcaata ctggcgcggc 60
ctttccggct ggaacttcta ttttctggtt aagttcggcc tgttgtgggc gggatatctt 120
aacttccatc cgctcctcaa tttggtgttt gccgcgtttc tgctgatgcc ccttccgcgc 180
tacagcctgc atcgcttgcg ccactggatt gccctgccga tcggctttgc tttgttctgg 240
catgacacct ggttgcctgg cccggaaagc ataatgagcc agggttcgca ggtggcgggg 300
ttcagtaccg attatttaat cgaccttgtc acacgcttta ttaactggca gatgattggg 360
gccattttgg ttttattagt ggcctggtta ttcctgtcac aatggattcg cattaccgtt 420
tttgtggttg ccatactgct atggctgaac gtacttaccc tggcgggacc aagtttctcc 480
ttgtggccag ccggacaacc gacgaccact gtaacaacga cgggtggtaa cgcagcggca 540
accgttgcgg cgacgggtgg cgcaccggta gtgggtgata tgcccgcaca aactgcaccg 600
ccaacaacgg cgaaccttaa cgcctggctg aataatttct ataacgcgga ggcgaaacgt 660
aaatcgacct tcccgtcttc gctgcccgct gatgctcagc catttgaact actggtgatt 720
aacatctgtt cgctttcctg gtcggatata gaagccgccg ggttgatgtc gcatccactg 780
tggtcgcatt tcgatattga gttcaagaac tttaactccg ccacctccta cagtggcccg 840
gcggcgatcc gtttactgcg cgccagctgc gggcagactt cgcacactaa tctgtatcaa 900
ccggcaaata acgactgcta tctgtttgat aacctttcga aactgggctt tacccagcac 960
ctgatgatgg gacataacgg ccagttcggc ggttttttga aagaagttcg cgaaaatggc 1020
ggcatgcaga gcgaattgat ggatcaaaca aatctgccgg ttattttgct gggctttgat 1080
ggttcgccgg tttatgacga taccgctgtg cttaaccgct ggctggacgt taccgaaaaa 1140

gataaaaaca gccgtagtgc cacgttctac aacacgcttc cactgcatga cggcaaccat 1200
tatccggggg tcagcaaaac agcggattac aaagcgcggg cgcagaaatt ctttgatgaa 1260
ctggacgcct tctttactga acttgagaaa tcgggtcgta aagtgatggt ggtcgtggtg 1320
ccggaacacg gcggcgcgct gaagggcgac agaatgcagg tatctggcct acgtgatatc 1380
cctagcccgt ctatcaccga cgtccccgtt ggggtgaaat tcttcggcat gaaggcaccg 1440
catcaggggg caccgattgt catcgaacaa ccgagcagct tcctggctat ctccgatctg 1500
gtggttcgcg ttctcgatgg caagattttc accgaagaca atgttgactg gaaaaaactc 1560
accagtgggt tgccacaaac agcaccggtc tccgagaact caaatgcagt agttattcaa 1620
taccaggata aaccgtacgt tcgcctgaac ggcggcgact gggtgcctta cccgcagtaa 1680

<210> 172
<211> 384
<212> DNA
<213> E. Coli

<400> 172
atggaaggtt caagaatgaa ataccgcatc gctttagctg tttctctctt tgctcttagt 60
gccggtagtt atgccactac cctgtgtcag gaaaaggagc aaaatatcct taaggagatc 120
agctatgccg aaaaacacca aaaccagaat cgtattgacg gtctgaataa agccctgagt 180
gaagtccggg ccaactgttc agatagccag ctgcgtgccg atcatcagaa gaaaatcgca 240
aagcagaaag atgaggtggc ggaacgccag caagatttag ccgaggcgaa gcaaaaaggc 300
gatgccgata agattgccaa acgcgaacgg aaactggcag aagcgcagga agagctgaaa 360
aagctggaag cgcgcgacta ctaa 384

<210> 173
<211> 306
<212> DNA
<213> E. Coli

<400> 173
atgtcgaaag aacacactac ggaacatctg cgtgctgagt tgaaatccct ttccgatacg 60
ctggaagagg tgcttagctc atctggcgag aagtcgaaag aagagttgag taagattcgt 120
agcaaagcgg agcaggcact gaaacagagc cgttatcgcc tgggtgaaac cggtgatgcc 180
attgccaaac aaacccgtgt cgcggcggcg cgtgccgatg agtatgtgcg cgaaaatccg 240
tggacgggcg tgggcattgg cgctgcaatc ggtgtagtgc tcggcgttct gctgtcgcgt 300
cgttaa 306

<210> 174
<211> 405
<212> DNA
<213> E. Coli

<400> 174
atggcggaca ctcatcacgc acaagggccc ggtaaaagcg ttctgggcat cgggcagcga 60
attgtttcta tcatggttga aatggtagag acacgtctgc ggctggcggt ggtggagctg 120
gaagaggaaa aagcgaatct ctttcaactt ttactgatgc tgggcctgac gatgcttttc 180
gctgcatttg gtcttatgag cctgatggtg ctaattattt gggcggttga cccgcaatat 240
cgcctgaatg cgatgattgc caccaccgtg gtgttgctgc tactggcact gattggcggt 300
atctggacgc tacgtaaatc gcgtaagtct acgttgctgc gccatacacg ccatgagtta 360
gcaaacgatc ggcagctgct cgaggaggag tcccgtgagc agtaa 405

<210> 175
<211> 300
<212> DNA
<213> E. Coli

<400> 175
gtgagcagta aagtcgaacg tgaacgacgt aaggcgcaac tgcttagcca gatccagcaa 60
caacggctgg atctttccgc cagtcgtcgt gaatggctgg agacaacagg cgcttacgat 120
cgtcgctgga atatgctgct aagtctgcgc tcctggcgcc tggttggcag tagcgtgatg 180
gcgatctgga cgattcgcca tcctaatatg ctggtccgct gggccagacg cggttttggc 240

gtatggagcg cctggcgtct ggttaaaacg accctcaagc agcaacagct tcgcggttaa 300

<210> 176
<211> 483
<212> DNA
<213> E. Coli

<400> 176
atgattctct ccatcgacag caacgacgct aataccgcgc cattgcacaa aaaaacaatc 60
agcagcctga gtggcgcagt ggagagtatg atgaaaaaat tagaagatgt tggtgtactg 120
gtagcgcgca ttttaatgcc gattctgttt attaccgctg gctggggaaa aattactggc 180
tacgcgggta cccaacaata tatggaagca atgggcgtcc cgggttttat gctgccactg 240
gtgattctgc ttgagtttgg tggtggtctg gcaatcctgt tcggtttcct gactcgcacc 300
acagccctgt ttactgcggg ctttacgctg ctgacggcat ttttatttca cagcaacttt 360
gctgaaggcg tcaactcgct gatgttcatg aaaaacctga caatttctgg cggattcctg 420
ctgctggcaa ttaccggtcc gggcgcgtat agcatcgacc gcctgctgaa taaaaagtgg 480
taa 483

<210> 177
<211> 891
<212> DNA
<213> E. Coli

<400> 177
atgatcaaga agacaacgga aattgatgcc atcttgttaa atctcaataa ggctatcgat 60
gcccactacc agtggctggt gagtatgttt cacagcgtgg tcgcgagaga tgccagtaag 120
ccagaaataa cggataacca ttcttatgga ctgtgccagt ttggtcggtg gattgatcat 180
ctggggccac tcgataacga tgaattacct tacgttcggc taatggattc tgcccatcaa 240
catatgcata actgtggtcg ggaattaatg ctggctattg ttgaaaatca ctggcaggac 300
gcgcatttcg acgcctttca ggaggggttg ctttctttta ctgcggcatt aaccgattac 360
aaaatttatt tgctgacgat ccgtagcaat atggatgttt tgacgggatt gccgggtcgt 420
cgggttcttg atgaatcctt tgatcatcag ttacgcaacg ctgagcctct gaatctttat 480
ttaatgttgt tggatattga ccgatttaaa ttggttaatg atacctacgg gcatttaatc 540
ggcgatgtag tattacgcac cctggcaact tacttagcca gttggacgcg tgattacgaa 600
acggtttatc gctacggggg cgaagaattt atcattattg tcaaagcggc taatgatgaa 660
gaagcatgtc gtgcaggtgt cagaatttgc cagttagtcg ataaccatgc catcacacat 720
tctgaaggcc atatcaacat taccgtgaca gcaggtgtga gtcgcgcatt tcctgaagag 780
cctctggatg tggtcattgg aagagcggac cgggcaatgt atgagggtaa gcaaaccgga 840
agaaatcgct gcatgtttat tgacgaacaa aatgtgatta accgagttta a 891

<210> 178
<211> 612
<212> DNA
<213> E. Coli

<400> 178
atgcgccttc gtgttgtgcc cggttttatt tcaccacctc cgggcttcgg tggtctcggc 60
tataccccta cagcgagagc ttgtgttaac atttcaatac ccttacagtt gagagttatt 120
gatatgttgg atgtatttac tccattgttg aaactttttg ctaacgagcc actcgaaaga 180
cttatgtata cgattatcat ttttggtctc actctctggc tgataccgaa agagtttact 240
gtcgcattca atgcttatac tgaaatacct tggctctttc agattatcgt ttttgccttt 300
tctttcgtgg tcgccatttc cttctcaaga ttgcgagcac atattcaaaa gcattattca 360
ttactaccag agcaacgagt attgcttcgt ttatctgaga aagaaatcgc tgtatttaaa 420
gatttcctta aaacaggaaa tcttattatc acttctcctt gccgtaaccc ggttatgaaa 480
aaattagaac ggaagggcat cattcaacat cagagtgata gcgcaaactg ttcttattat 540
ctcgtcaccg aaaaatactc ccattttatg aagttattct ggaacagcag gagtagacgt 600
tttaatcgtt ag 612

<210> 179
<211> 177
<212> DNA

<213> E. Coli

<400> 179
gtgcttctcc aaccatcggc gcgcaccagt ttcggtttta aatgttttgc ttttggtata 60
cgtcatggca gtgaacgttc catcctggtt ggggaacacg ccgcacacca gggattcgtt 120
gttgccgagg tcgatttttt gcattttgcg aatctcacat cttgttgcta cgtatag 177

<210> 180
<211> 4281
<212> DNA
<213> E. Coli

<400> 180
atgagcggaa aaccagcggc gcgtcaggga gatatgactc agtatggcgg tcccattgtc 60
cagggttcgg caggtgtaag aattggcgcg cccaccggcg tggcgtgctc ggtgtgtccg 120
ggcgggatga cttcgggcaa cccggtaaat ccgctgctgg gggcgaaggt gctgcccggc 180
gagacggacc ttgcgctgcc cggcccgctg ccgttcattc tctcccgcac ctacagcagc 240
taccggacga agacgcctgc accggtgggc gttttcggcc ccggctggaa agcgccttct 300
gatatccgct tacagctacg tgatgacgga ctgatactca acgacaacgg cgggcggagc 360
attcactttg agccgctgct gccgggggag gcggtgtaca gccgcagtga gtcaatgtgg 420
ctggtgcgcg gtggtaaggc agcacagccg gacggccata cgctggcgcg gctgtggggg 480
gcgctgccgc cggatatccg gttaagcccg catctttacc tggcgaccaa cagcgcacag 540
gggccgtggt ggatactggg gtggtctgag cgggtgccgg gtgctgagga cgtactgcca 600
gcgccgctgc cgccgtaccg ggtgcttacc gggatggcgg accgcttcgg gcggacgctg 660
acgtaccggc gtgaggccgc cggtgacctg gccggggaaa tcaccggcgt gacggacggt 720
gccgggcggg agttccgtct ggtgctgacc acgcaggcgc agcgtgcgga agaggcccgc 780
acctcttcgc tatcttcttc tgacagttcc cgccctctct cagcctcagc gttccccgac 840
acactgcccg gtaccgaata cggccccgac aggggtatcc gcctttcggc ggtgtggctg 900
atgcacgacc cggcataccc ggagagcctg cccgctgcgc cactggtgcg gtacacgtat 960
acggaagccg gtgaactgct ggcggtatat gaccgcagca atacgcaggt gcgcgctttc 1020
acgtatgacg cgcagcaccc gggccggatg gtggcgcacc gttacgcggg aaggccggag 1080
atgcgctacc gctacgacga tacggggcgg gtggtggagc aactgaaccc ggcagggtta 1140
agctaccgct atctttatga gcaggaccgc atcaccgtca ccgacagcct gaaccggcgt 1200
gaggtgctgc atacagaagg cggggccggg ctgaaacggg tggtgaaaaa agaactggcg 1260
gacggcagcg tcacgcgcag cgggtatgac gcggcaggaa ggctcacggc gcagacggac 1320
gcggcgggac ggaggacaga gtacggtctg aatgtggtgt ccggcgatat cacggacatc 1380
accacaccgg acgggcggga gacgaaattt tactataacg acggaaccca gctgacggcg 1440
gtggtgtccc cggacgggct ggagagccgc cgggaatatg atgaaccggg caggctggta 1500
tcggagacat cgcgcagcgg ggagacagta cgctaccgct acgatgacgc gcacagtgag 1560
ttaccggcga cgacaacgga tgcgacgggc agcacccggc agatgacctg gagccgctac 1620
gggcagttgc tggcgttcac cgactgctcg ggctaccaga cccgttatga atacgaccgc 1680
ttcggccaga tgacggcggt ccaccgcgag gaaggcatca gcctttaccg ccgctatgac 1740
aaccgtggcc ggttaacctc ggtgaaagac gcacagggcc gtgaaacgcg gtatgaatac 1800
aacgccgcag gcgacctgac tgccgttatc accccggacg gcaaccggag cgagacacag 1860
tacgatgcgt ggggaaaggc ggtcagcacc acgcagggcg ggctgacgcg cagtatggag 1920
tacgatgctg ccggacgtgt catcagcctg accaacgaga acggcagcca cagcgtcttc 1980
agttacgatg cgctggaccg gctggtacag cagggcggct ttgacggcgg gacgcaacgt 2040
tatcattatg acctgaccgg aaaactcaca cagagtgagg atgagggact tgtcatcctc 2100
tggtactacg atgaatcgga ccgtatcact caccgcacgg tgaacggcga accggcagag 2160
cagtggcagt atgatggcca cggctggctg acagacatca gccacctgag cgaaggccac 2220
cgtgttgccg tccactatgg ctatgacgat aaaggccgcc tgaccggcga atgccagacg 2280
gtggagaacc cggagacggg ggaactgctg tggcagcatg agacgaaaca cgcatacaac 2340
gagcaggggc tggcaaaccg cgtcacgccg gacagcctgc cgccggtgga gtggctgacg 2400
tatggcagcg gttacctggc gggaatgaag ctgggcggga cgccgctggt cgagtatacg 2460
cgggacaggc tgcaccgtga gacggtgcgc agcttcggca gcatggcagg cagtaatgcc 2520
gcatacgaac tgaccagcac atacaccccc gcaggccagt tacagagcca gcacctgaac 2580
agcctggtat atgaccgtga ctacgggtgg agtgacaacg gcgacctggt gcgcatcagc 2640
ggcccgcgac agacgcggga atacggctac agcgccacgg gcaggctgga gagtgtgcgc 2700
accctcgcac cagacctgga catccgcatc ccgtatgcca cggacccggc gggcaaccgg 2760
ctgccggacc cggagctgca cccggacagt acactcacag tgtggccgga taaccgcatc 2820
gcggaggatg cgcactatgt ctaccgccac gatgaatacg gcaggctgac ggagaagacg 2880

gaccgcatcc cggcgggtgt gatacggacg gacgacgagc ggacccacca ctaccactac 2940
gacagccagc accgcctggt gttctacacg cggatacagc atggcgagcc actggtcgag 3000
agccgctacc tctacgaccc gctgggacgg cgaatggcaa aacgggtctg gcggcgggag 3060
cgtgacctga cggggtggat gtcgctgtcg cgtaaaccgg aggtgacgtg gtatggctgg 3120
gacggagaca ggctgacgac ggtgcagact gacaccacac gtatccagac ggtatacgag 3180
ccgggaagct tcacgccgct catccgggtc gagacagaga acggcgagcg ggaaaaagcg 3240
cagcggcgca gcctggcaga gacgctccag caggaaggga gtgagaacgg ccacggcgtg 3300
gtgttcccgg ctgaactggt gcggctgctg gacaggctgg aggaagaaat ccgggcagac 3360
cgcgtgagca gtgaaagccg ggcgtggctt gcgcagtgcg ggctgacggt ggagcaactg 3420
gccagacagg tggagccgga atacacaccg gcgcgaaaag ctcatcttta tcactgcgac 3480
caccggggac tgccgctggc gcttatcagc gaagacggca atacggcgtg gagcgcggaa 3540
tatgatgaat ggggcaacca gcttaatgag gagaacccgc atcatgtgta tcagccgtac 3600
cgtctgccag ggcagcagca tgatgaggaa tcagggctgt actataaccg tcaccggtac 3660
tacgatccgt tgcaggggcg gtatattact caggacccga tggggttgaa agggggatgg 3720
aatttatatc agtatccttt aaatccacta caacaaattg accctatggg attattgcag 3780
acttgggatg atgccagatc tggagcatgt acggggggag tttgtggtgt tctttcacgt 3840
ataataggac caagtaaatt tgatagtact gcagatgctg cgttagatgc tttgaaagaa 3900
acgcagaata gatctctatg taatgatatg gaatactctg gtattgtctg taaagatact 3960
aatggaaaat attttgcatc taaggcagaa actgataatt taagaaagga gtcatatcct 4020
ctgaaaagaa aatgtcccac aggtacagat agagttgctg cttatcatac tcacggtgca 4080
gatagtcatg gcgattatgt tgatgaattt ttttcaagta gcgataaaaa tcttgtaaga 4140
agtaaagata ataatcttga agcattttat ctcgcaacac ctgatggacg atttgaggcg 4200
cttaataata aaggagaata tatttttatc agaaatagtg tcccgggatt gagttcagta 4260
tgcataccgt atcatgatta a 4281

<210> 181
<211> 369
<212> DNA
<213> E. Coli

<400> 181
atgaaatata gttcaatatt ttcgatgctt tcatttttta tactatttgc ctgtaatgag 60
acagctgttt acggttctga tgaaaacatt atttttatga ggtatgtgga aaaattacat 120
ttagataaat actctgttaa aaatacggta aaaactgaaa caatggcgat acaattagct 180
gaaatatatg ttaggtatcg ctatggcgaa cggattgcag aagaagaaaa accatattta 240
attacggaac taccagatag ttgggttgtt gagggagcaa agttacctta tgaagttgcg 300
ggtggtgtat ttattataga aattaataag aaaaatggat gtgttttgaa tttcctacat 360
agtaaataa 369

<210> 182
<211> 711
<212> DNA
<213> E. Coli

<400> 182
atgctggcgc tgatggatgc ggatggaaac attgcgtgga gcggggagta tgatgagtgg 60
ggcaaccagc tgaatgaaga gaacccgcat cacctgcacc agccgtaccg gctgccgggg 120
cagcagtatg ataaggagtc ggggctgtac tacaaccgga accggtacta cgatccgttg 180
caggggcggt atatcactca ggacccgata gggctggagg ggggatggag tctgtatgcg 240
tatccgctga atccggtgaa tggtattgat ccattagggt taagtcccgc agatgtagcg 300
ctaataagaa gaaaagatca actaaaccat caaagagcat gggatatatt atctgatact 360
tatgaagata tgaagagatt aaatttaggt gggactgatc aatttttcca ttgtatggca 420
ttttgtcgag tgtctaaatt aaatgacgct ggtgttagcc gatcggcgaa agggctgggt 480
tatgaaaaag agattagaga ttacgggtta aatctgttcg gtatgtacgg cagaaaagta 540
aagctatccc attctgaaat gattgaagat aataaaaaag acttggctgt aaatgaccat 600
gggttgacat gtccatcaac aacagattgc tcagatagat gtagtgatta tattaatcca 660
gagcataaaa aaacgataaa ggctttacaa gatgctggct atctcaagta a 711

<210> 183
<211> 261
<212> DNA

<213> E. Coli

<400> 183
   atgctggcta tctcaagtaa tctatcaaag atgataatat ttatttttgc tattataatc 60
   attgttgttt tatgcgtaat tacttatctt tatttataca aagatgaatc tcttgtaagt 120
   aaacattaca taaactatat ggcaatacca gaaaatgatg gagtttttac atggctccca 180
   gatttttttc cgcacgtagc ggtggatata tcaatataca caaatgtaga agatgattat 240
   ttttttctta tttttcccta a 261

<210> 184
<211> 192
<212> DNA
<213> E. Coli

<400> 184
gtgagggcca gggaacaagt ggcgaaaatc gtatcaaaga atgatccaga tacaaaaaaa 60
gtgtggtgta aatatggtaa gataccaggg caaggggatg gtgtaaacct tttttttgtt 120
ggtgaaatta atgttacgca ttattttata acaaatattg gagctggatt gcctgatgct 180
tgtgcagagt aa 192

<210> 185
<211> 504
<212> DNA
<213> E. Coli

<400> 185
atgccgggca acagcccgca ttatggcgtt tggcctcaac acgattttac gtcacttaaa 60
aaactcaggc cgcagtcggt aacctcgcgc atacagccgg gcagtgacgt catcgtctgc 120
gcggaaatgg acgaacagtg gggctatgtc ggggctaaat cgcgccagcg ctggctgttt 180
tacgcgtatg acagtctccg gaagacggtt gttgcgcacg tattcggtga acgcactatg 240
gcgacgctgg ggcgtcttat gagcctgctg tcaccctttg acgtggtgat atggatgacg 300
gatggctggc cgctgtatga atcccgcctg aagggaaagc tgcacgtaat cagcaagcga 360
tatacgcagc gaattgagcg gcataacctg aatctgaggc agcacctggc acggctggga 420
cggaagtcgc tgtcgttctc aaaatcggtg gagctgcatg acaaagtcat cgggcattat 480
ctgaacataa aacactatca ataa 504

<210> 186
<211> 276
<212> DNA
<213> E. Coli

<400> 186
gtggcttctg tttctatcag ctgtccctcc tgttcagcta ctgacggggt ggtgcgtaac 60
ggcaaaagca ccgccggaca tcagcgctat ctctgctctc actgccgtaa aacatggcaa 120
ctgcagttca cttacaccgc ttctcaaccc ggtacgcacc agaaaatcat tgatatggcc 180
atgaatggcg ttggatgccg ggcaacagcc cgcattatgg gcgttggcct caacacgatt 240
ttacgtcact taaaaaactc aggccgcagt cggtaa 276

<210> 187
<211> 417
<212> DNA
<213> E. Coli

<400> 187
atgatgacta aaacccaaat aaataaatta ataaaaatga tgaatgattt agactatcca 60
tttgaagcac cgctcaagga atcatttatt gaaagtataa tccaaataga atttaattct 120
aattcaacta attgcctgga gaagttatgt aatgaagtta gtattctttt taagaatcaa 180
cctgattatc ttactttttt aagagcaatg gatggattcg aagttaatgg attacgatta 240
tttagcctct cgattccaga accttcagtt aaaaaccttt ttgccgtaaa tgaattttat 300
agaaataatg atgatttcat aaaccctgat ctacaagaac ggttagtgat cggggattat 360
agcatttcaa tatttactta tgacattaaa ggtgatgctg ccaacttact gatttag 417

<210> 188
<211> 1179
<212> DNA
<213> E. Coli

<400> 188
atgagtaata ttgtttacct gacagtaacg ggagaacaac aaggaagcat ctccgcaggt 60
tgtgggactt ctgagtctac aggtaatcgt tggcagagcg ggcatgagga tgaaatattt 120
acattctcac tcttaaataa tattaataat acggggcttg gttcacagtt ccatggtata 180
acattttgta aattaattga taaaagcact ccattattta ttaattccat taacaataat 240
gaacaattat ttatgggatt tgacttctat cgaataaata gatttggtag attggaaaag 300
tattattata tacaactaag aggcgctttt ttatcggcta ttcatcacca gatcattgaa 360
aaccaactgg atacagaaac aataactatt agttatgaat ttatcctctg tcaacatctt 420
atcgcaaata ccgagttcag ctatttggca ctccctgaaa attataaccg tttgttttta 480
ccaaattcaa aaaaccaaac aaataatcgt ttcaaaacgt taaacagcaa agctattggc 540
aggctacttg ctgctggtgg cgtatacaat gggaacattg aaggattcag agatactgcg 600
gaaaaactgg gtggagatgc aataaaaggc tatgatcaaa tactaaatga aaaaacagcg 660
ggcatagcga tagcaacagc atctattctt ttaacaaagc gttctaatgt tgatacatat 720
acagaaataa atagttactt aggcaaactt agaggtcaac aaaaacttct tgatggtata 780
gacataatag aaataatata cattaagaga ccttcaaaag acttagctaa cttacgaaag 840
gagtttaata aaactgtaag aaaaaatttt cttatcaaac ttgcaaaaac ctccgaagca 900
tctggaagat tcaacgccga agacctttta agaatgagaa agggcaatgt tcctctaaat 960
tataatgttc accataaact atctctagat gatggtggta ctaatgattt cgaaaattta 1020
gtattaatcg aaaacgaacc atatcataaa gtttttacta acatgcaatc acgaatagct 1080
aagggaatat tagtaggtga aagcaaaatc actccctggg ccattccatc tggctcaatt 1140
tatcctccca tgaaaaatat tatggaccac acaaaatga 1179

<210> 189
<211> 666
<212> DNA
<213> E. Coli

<400> 189
atggtacttg ctttgaacta taatatgcac ggagttaata ttcgctcaga gaatgcagca 60
aaacctcata cgatgccctc tagatatctt tgcgagtata ttagaagcat tgagaaaaat 120
ggccacgccc ttgattttgg ctgcggaaaa cttagatatt ctgatgaatt aatcagtaaa 180
tttgatgaag ttacttttct agactcgaaa aggcaacttg aaagagagca aattattaga 240
ggaattaaaa ctaaaattat tgactatgtc ccacgatatt ataaaaatgc aaatacagtt 300
gctttcgagg atgtcgacaa aataattggg ggttacgatt tcatcctttg ctctaatgtt 360
ctctctgccg ttccttgtcg ggatacaatc gacaaaatag ttcttagcat caagagatta 420
ctaaaatcag gaggtgagac tcttattgta aatcaatata aaagctcata cttcaaaaaa 480
tacgaaacag gaagaaaaca tctttacgga tacatttaca aaaattcaaa aagtgtttct 540
tactatggat tactcgatga actcgcagtg caagaaatat gttcttcaca tggccttgaa 600
atattaaagt cgtggagtaa agcaggaagt tcatatgtca ctgttgggag ttgtaatgca 660
atataa 666

<210> 190
<211> 705
<212> DNA
<213> E. Coli

<400> 190
gtgaataata tgttcgaacc ccccaaaaat tataatgaaa tgttgcctaa acttcataaa 60
gcaactttct taaatacgct aatatattgc atacttctag ttatttacga atacatccct 120
ttaataacat taccaaccaa gtatgtccca cctattaaag atcatgagag ctttattaat 180
tgggcactat cttttggtat attaccttgt gcttttgcga tttttgcata tttaattagc 240
ggtgcgttag acctacataa caatgcagcc aaactacttc gggtgcgata tctttgggat 300
aagcatctaa ttataaaacc gttatcacgg agagctggag tcaacagaaa attaaataaa 360
gatgaagctc acaatgtaat gagcaatcta tattaccctg aagtaagaaa aattgaagac 420
aaacattata ttgaactctt ctggaataaa gtatactatt tttggatatt ttttgaattt 480

tcgataattg cattaatttc cttcctaata atcttttttt gcaaacaaat ggatattttt 540
catgttgaag gttctttgct gtctttattc ttttttgtaa ttttatcatt ctcagtgagt 600
ggtattatct ttgctttgac agttaagccc agaactgaaa gtcaagtcgg aaaaatcccg 660
gacgataaaa taaaagaatt tttcactaaa aataacatta attga 705

<210> 191
<211> 285
<212> DNA
<213> E. Coli

<400> 191
atgtttacta tcaacgcaga agtacgtaaa gagcagggta agggtgcgag ccgccgcctg 60
cgtgccgcta acaagttccc ggcaatcatc tacggtggca aagaagcgcc gctggctatc 120
gagctggatc acgacaaagt catgaacatg caagctaaag ctgaattcta cagcgaagtt 180
ctgaccatcg ttgttgacgg taaagaaatc aaagttaaag ctcaggacgt acagcgtcac 240
ccgtacaaac cgaagctgca gcacatcgac ttcgttcgcg cttaa 285

<210> 192
<211> 1977
<212> DNA
<213> E. Coli

<400> 192
atggtattgt tttatcgggc acactggcgc gactataaaa acgatcaagt gaggatcatg 60
atgaatctga cgactctgac ccaccgcgat gcgttgtgtc tgaatgcgcg ctttaccagc 120
cgtgaagagg ccatccacgc gttgactcaa cgtcttgctg ctctggggaa aatttccagt 180
actgagcaat ttctggaaga agtgtatcgc cgtgaaagcc ttggcccgac cgccttaggt 240
gaagggttgg ctgtgccgca tggcaaaact gctgcggtaa aagaagcggc gtttgcggtc 300
gccacactca gcgagccgct tcagtgggaa ggcgttgatg gcccggaagc agttgattta 360
gtggtgctgc tggcgattcc ccccaatgaa gcgggtacta cgcatatgca actgctgaca 420
gcgctgacca cgcgccttgc ggatgatgag attcgggcgc gtatacagtc ggcgacgacg 480
cctgatgagt tgctctcggc gctggatgac aagggaggca cgcaaccttc tgcctctttt 540
tccaacgcgc caactatcgt ctgcgtaacg gcctgtccgg cgggtattgc tcacacctat 600
atggctgcgg aatatctgga aaaagccgga cgcaaactcg gcgtaaatgt ttacgttgaa 660
aaacaaggcg ctaacggcat tgaagggcgt ttaacggcgg atcaactcaa tagtgcaacc 720
gcctgtattt ttgcggctga agtcgccatc aaggagagtg agcgttttaa tggcattccc 780
gcgctttcag tgcctgttgc cgagccgatt cgccatgcag aagcgttgat ccaacaagcg 840
cttaccctca agcgtagcga tgagacgcgt accgtacagc aagatacgca accggtgaaa 900
agtgtcaaaa cggagctgaa acaggcactg ttgagcggaa tctcttttgc cgtaccgttg 960
attgtcgcgg ggggcacggt gctggcggtc gcggtattac tgtcgcaaat cttcggccta 1020
caagatctgt ttaatgaaga aaactcctgg ctgtggatgt accgcaagct gggcggcggg 1080
ctgctcggaa ttttgatggt accggtgctc gcggcctata ccgcctattc tctggcagat 1140
aaaccggcgt tagcgccagg ctttgcggct ggacttgccg ccaacatgat cggctccggg 1200
tttctcggcg cggtcgttgg cggattgata gccggttact tgatgcgctg ggtgaaaaat 1260
cacttgcgtc ttagcagtaa attcaatgga ttcctgactt tttatctcta cccggtgctc 1320
ggtacgttgg gagcgggcag tctgatgctg tttgtggtgg gggaacctgt cgcctggatc 1380
aataactcgc ttaccgcctg gctgaacggt ctgtcaggaa gtaacgcgct gttgctgggt 1440
gccattctcg gttttatgtg ttcctttgac cttggagggc cagtgaataa agccgcttat 1500
gcattctgcc tgggcgcaat ggcgaacggc gtttacggcc cgtatgccat tttcgcctcc 1560
gtcaaaatgg tttcggcatt taccgtaacc gcttccacga tgctcgcacc gcgcctgttt 1620
aaagagtttg aaattgagac cgggaaatcc acctggctgt tagggctggc aggtattacc 1680
gaaggggcga tcccgatggc gattgaagat ccgctgcggg ttattggttc gtttgtgctg 1740
ggctctatgg taacgggcgc tattgtcggt gcgatgaata tcggcctttc gacacccggt 1800
gccggcattt tctcgctctt tttacttcat gataatggcg cgggcggtgt tatggcggca 1860
attggctggt ttggcgcggc attggtgggg gctgcaatct cgactgcaat tctcctgatg 1920
tggcggcgtc acgcggttaa gcatggcaac tatctgactg atggcgtaat gccataa 1977

<210> 193
<211> 2634
<212> DNA
<213> E. Coli

<400> 193
atgaaagcag tatctcgcgt tcacatcacc ccgcatatgc actgggatcg agagtggtat 60
ttcaccaccg aagagtcacg tattctgctg gtcaataata tggaagagat cctgtgccga 120
ctggaacagg acaacgaata caaatattac gtactcgacg ggcaaacggc gatcctcgaa 180
gattatttcg cggtgaaacc ggaaaacaaa gaccgtgtga agaaacaggt agaagccggc 240
aagttgatta tcggcccctg gtatacccag accgatacca cgattgtttc tgcggaatcc 300
atcgtccgta atctgatgta cggaatgcgt gactgcctcg cgtttggcga gccgatgaaa 360
ataggttatt taccagattc ctttggcatg tccgggcaac tgccgcatat ctacaatgga 420
tttggcatta cccgcaccat gttctggcgc ggatgttcgg agcgccacgg tactgataaa 480
accgagtttt tgtggcaaag cagtgacggt agcgaagtga cggcgcaggt gctgccgctg 540
ggctacgcca tcggtaagta cttacctgcc gacgaaaacg gattacgtaa acgcctcgac 600
agttattttg acgtgctgga aaaagcgtct gtaaccaaag agattttgct gccgaatggg 660
catgaccaga tgccattgca gcaaaatatc ttcgaagtga tggataagct acgtgagatc 720
taccctcaac gtaagtttgt gatgagccgc tttgaagagg tatttgagaa gatcgaagcg 780
cagcgagata atctggcaac cctgaaaggg gaatttattg atggcaaata tatgcgcgtg 840
catcgcacca tcggttctac gcgtatggat atcaaaattg cccacgcgcg tattgaaaat 900
aagattgtta atctgctgga accgctggca acactggcct ggacgttggg ttttgaatac 960
caccacggct tgctggagaa aatgtggaaa gagatcttaa aaaatcatgc ccacgacagt 1020
atcggctgct gctgtagtga caaagttcat cgcgaaatcg tcgcccgctt cgaactggct 1080
gaagacatgg cggataatct gattcgtttc tacatgcgca aaattgccga caacatgccg 1140
cagagcgacg ccgacaaact cgtcctgttt aacctgatgc cctggccgcg tgaagaagtt 1200
atcaacacca ctgtgcggct gcgcgccagc cagtttaatt tgcgggacga tcgcggtcag 1260
cctgtaccgt attttattcg ccatgcccgt gagatcgatc caggcctaat cgatcggcaa 1320
atagttcatt acggtaatta cgatcccttt atggagtttg atatacagat caaccagatt 1380
gtcccttcta tgggctatcg cacgctttat atcgaagcga atcagcctgg caacgtaatt 1440
gcggcaaaaa gtgacgctga agggatactg gaaaatgctt tctggcaaat tgcgctcaat 1500
gaggatggtt ctctgcaact ggtagataaa gacagcggtg tgcgctatga ccgggtattg 1560
caaattgaag aaagctctga tgatggtgat gaatatgact attcacccgc aaaagaagag 1620
tgggtaatta ccgcagcgaa cgcgaaaccg caatgcgata ttattcatga agcctggcag 1680
agcagggctg ttatccgcta tgacatggca gtgccgctca atttgtcaga acgcagcgcc 1740
cggcaatcca ctggcagagt aggggtggtg ttggttgtca ctcttagtca taacagcagg 1800
cgtattgatg tggatatcaa tcttgataac caggctgacg atcatcgcct tcgtgtcctg 1860
gtccctacac cttttaacac cgacagtgtt ctggcagata cgcagtttgg ttcgctaacg 1920
cgccccgtga acgacagtgc aatgaacaac tggcagcaag aaggctggaa agaagcgccg 1980
gttccggtat ggaatatgct caactatgtt gccttacagg aagggcgtaa cggcatggct 2040
gtctttagcg aagggttacg tgaatttgaa gtcatcggtg aagagaagaa aacctttgcc 2100
attacgttgc tgcgtggcgt gggcttactg ggcaaagaag atctgctttt aaggcctggg 2160
cggccttcgg gaattaaaat gccagtcccg gactcacaac tacgtggtct gctttcttgt 2220
cgcctaagtt tattgagtta taccggtacg ccaaccgccg ctggtgtagc tcagcaggcg 2280
cgagcatggc tgactccagt acagtgttac aacaaaatcc catgggatgt gatgaagctc 2340
aacaaagccg gattcaacgt gccggaaagt tatagtttgt tgaaaatgcc cccagtggga 2400
tgcctgataa gcgcacttaa gaaagctgaa gaccgacaag aagtgatttt acggctgttt 2460
aatccggctg aatcagcaac ctgtgatgcg actgttgctt tcagtcgcga ggtgatttct 2520
tgctcagaaa cgatgatgga tgaacacatt accaccgagg aaaatcaagg ttcaaatcta 2580
tcggggcctt ttttacccgg ccagtcacgg acgttcagtt accggcttgc ctga 2634

<210> 194
<211> 1572
<212> DNA
<213> E. Coli

<400> 194
atgatgttag atatagtcga actgtcgcgc ttacagtttg ccttgaccgc gatgtaccac 60
ttcctttttg tgccactgac gctcggtatg gcgttcctgc tggccattat ggaaacggtc 120
tacgtcctct ccggcaaaca gatttataaa gatatgacca agttctgggg caagttgttt 180
ggtatcaact tcgctctggg tgtggctacc ggtctgacca tggagttcca gttcgggact 240
aactggtctt actattccca ctatgtaggg gatatcttcg gtgcgccgct ggcaatcgaa 300
ggtctgatgg ccttcttcct cgaatccacc tttgtaggtc tgttcttctt cggttgggat 360
cgtctgggta aagttcagca tatgtgtgtc acctggctgg tggcgctcgg ttcaaacctg 420
tccgcactgt ggattctggt tgcgaacggc tggatgcaaa acccaatcgc gtccgatttc 480

aactttgaaa ctatgcgtat ggagatggtg agcttctccg agctggtgct taacccggtt 540
gctcaggtga aattcgttca cactgtagcg tctggttatg tgactggcgc gatgttcatc 600
ctcggtatca gcgcatggta tatgctgaaa ggtcgtgact tcgccttcgc taaacgctcc 660
tttgctatcg ctgccagctt cggtatggct gctgttctgt ctgttattgt tctgggtgat 720
gaatccggct acgaaatggg cgacgtgcag aaaaccaaac tggctgctat tgaagccgag 780
tgggaaacgc aacctgcgcc tgctgccttt actctgttcg gcattcctga tcaggaagag 840
gagacgaaca aatttgcgat tcagatccct tacgcactgg gcatcattgc aacgcgttcc 900
gtggataccc cggttatcgg cctgaaagag ctgatggtgc agcatgaaga acgcattcgt 960
aacgggatga aggcgtactc tctgctcgaa caactgcgtt ctggttctac cgaccaggcg 1020
gttcgtgacc agttcaatag catgaagaaa gacctcggtt acggtctgct gctgaaacgc 1080
tatacgccaa acgtggctga tgcgactgaa gcgcagattc aacaggcaac caaagactcc 1140
atcccgcgtg tagcgccgct gtactttgcg ttccgtatca tggtggcgtg tggcttcctg 1200
cttctggcaa tcatcgcgct ctctttctgg agtgtcatcc gcaaccgcat tggcgagaaa 1260
aaatggcttc tgcgcgccgc gctgtacggt attccgctgc cgtggattgc tgtagaagcg 1320
ggctggttcg tggctgaata tggccgccaa ccgtgggcta tcggtgaagt gctgccgaca 1380
gctgtggcga actcgtcact gaccgcaggc gatctcatct tctcaatggt gctgatttgc 1440
ggcctgtata ccctgttcct ggtggcagaa ttgttcttaa tgttcaagtt tgcacgcctc 1500
ggcccaagca gcctgaaaac cggtcgctat cactttgagc agtcttccac gactactcag 1560
ccggcacgct aa 1572

<210> 195
<211> 1140
<212> DNA
<213> E. Coli

<400> 195
atgatcgatt atgaagtatt gcgttttatc tggtggctgc tggttggcgt tctgctgatt 60
ggttttgcag tcactgacgg tttcgacatg ggggtgggca tgctcacccg tttcctcggt 120
cgtaacgaca ccgagcgtcg aattatgatt aactccattg caccacactg ggacggtaac 180
caggtttggc tgatcaccgc gggcggcgca ctctttgctg cctggccgat ggtctatgcc 240
gctgcgttct ccggcttcta tgtggcgatg atcctcgtgc tggcgtcttt gttcttccgt 300
ccggtcggtt ttgactaccg ctccaagatt gaagaaaccc gctggcgtaa catgtgggac 360
tggggcatct tcattggtag cttcgttccg ccgctggtaa ttggtgtagc gttcggtaac 420
ctgttgcagg gcgtaccgtt caacgttgat gaatatctgc gtctgtacta caccggtaac 480
ttcttccagt tgcttaaccc gttcggcctg ctggcaggcg tggtgagcgt agggatgatc 540
attactcagg gcgcaaccta tctgcaaatg cgtaccgtgg gcgaactgca cctgcgtacc 600
cgtgcaacgg ctcaggtggc tgcgctggtg acactggtct gtttcgcact ggctggcgta 660
tgggtgatgt acggtatcga tggttatgtc gtgaaatcga caatggacca ttacgcagcc 720
tctaacccac tgaataaaga agtggttcgt gaagctggcg catggctggt taacttcaac 780
aacacgccaa ttctgtgggc tattccggca ctgggtgtgg ttctgccgct gctgaccatc 840
ctgactgcac gtatggataa agccgcgtgg gcgtttgtgt tctcctccct gacgctggcc 900
tgcatcatcc tgacagccgg tatcgcaatg ttcccgtttg tgatgccgtc cagcaccatg 960
atgaacgcaa gtctgacaat gtgggatgca acttccagcc agctgacgct taacgtcatg 1020
acctgggttg cggtggttct ggtaccgatc attctgctct acaccgcctg gtgttactgg 1080
aaaatgttcg gtcgtatcac caaagaagat attgaacgta acacccactc tctgtactaa 1140

<210> 196
<211> 1371
<212> DNA
<213> E. Coli

<400> 196
atggaattat cctcactgac cgccgtttcc cctgtcgatg gacgctacgg cgataaagtc 60
agcgcgctgc gcgggatttt cagcgaatat ggtttgctga aattccgtgt acaagttgaa 120
gtacgttggc tgcaaaaact ggccgcgcac gcagcgatca aggaagttcc tgcttttgct 180
gccgacgcaa tcggttacct tgatgcaatc gtcgccagtt tcagcgaaga agatgcggcg 240
cgcatcaaaa ctatcgagcg taccactaac cacgacgtta aagcggttga gtatttcctg 300
aaagaaaaag tggcggagat cccggaactg cacgcggttt ctgaattcat ccactttgcc 360
tgtacttcgg aagatatcaa taacctctcc cacgcattaa tgctgaaaac cgcgcgtgat 420
gaagtgatcc tgccatactg gcgtcaactg attgatggca ttaaagatct cgccgttcag 480
tatcgcgata tcccgctgct gtctcgtacc cacggtcagc cagccacgcc gtcaaccatc 540

ggtaaagaga tggcaaacgt cgcctaccgt atggagcgcc agtaccgcca gcttaaccag 600
gtggagatcc tcggcaaaat caacggcgcg gtcggtaact ataacgccca catcgccgct 660
tacccggaag ttgactggca tcagttcagc gaagagttcg tcacctcgct gggtattcag 720
tggaacccgt acaccaccca gatcgaaccg cacgactaca ttgccgaact gtttgattgc 780
gttgcgcgct tcaacactat tctgatcgac tttgaccgtg acgtctgggg ttatatcgcc 840
cttaaccact tcaaacagaa aaccattgct ggtgagattg gttcttccac catgccgcat 900
aaagttaacc cgatcgactt cgaaaactcc gaagggaatc tgggcctttc caacgcggta 960
ttgcagcatc tggcaagcaa actgccggtt tcccgctggc agcgtgacct gaccgactct 1020
accgtgctgc gtaacctcgg cgtgggtatc ggttatgcct tgattgcata tcaatccacc 1080
ctgaaaggcg tgagcaaact ggaagtgaac cgtgaccatc tgctggatga actggatcac 1140
aactgggaag tgctggctga accaatccag acagttatgc gtcgctatgg catcgaaaaa 1200
ccgtacgaga agctgaaaga gctgactcgc ggtaagcgcg ttgacgccga aggcatgaag 1260
cagtttatcg atggtctggc gttgccagaa gaagagaaag cccgcctgaa agcgatgacg 1320
ccggctaact atattggtcg agctatcacg atggttgatg agctgaaata a 1371

<210> 197
<211> 186
<212> DNA
<213> E. Coli

<400> 197
atgctgattc tgactcgtcg agttggtgag accctcatga ttggggatga ggtcaccgtg 60
acagttttag gggtaaaggg caaccaggta cgtattggcg taaatgcccc gaaggaagtt 120
tctgttcacc gtgaagagat ctaccagcgt atccaggctg aaaaatccca gcagtccagt 180
tactaa 186

<210> 198
<211> 93
<212> DNA
<213> E. Coli

<400> 198
ggtgaggtgg ccgagaggct gaaggcgctc ccctgctaag ggagtatgcg gtcaaaagct 60
gcatccgggg ttcgaatccc cgcctcaccg cca 93

<210> 199
<211> 603
<212> DNA
<213> E. Coli

<400> 199
atgaagaata aggctgataa caaaaaaagg aacttcctga cccatagtga aatcgaatca 60
ctccttaaag cagcaaatac cgggcctcat gcagcacgta attattgtct gactttgctt 120
tgttttattc atggtttccg ggcgagtgaa atttgtcgat tgaggatttc ggatattgat 180
cttaaggcaa agtgtatata tatccatcga ttaaaaaaag gcttttcaac aacgcacccg 240
ctattgaata aagaagttca ggctttaaaa aactggttga gtatccgtac ttcgtacccg 300
catgctgaga gcgagtgggt atttttatca cgtaagggga atccgctttc tcggcaacag 360
ttttaccata ttatctcgac ttccggtggt aatgccgggt tgtcactgga gattcatccg 420
cacatgttac gccattcgtg tggttttgct ttggcgaata tgggaataga tacgcgactt 480
atccaggatt atcttgggca tcgcaatatt cgtcatactg tctggtatac cgccagcaat 540
gcagggcgtt tttacggcat ctgggataga gccagaggac gacagcgtca cgctgtttta 600
tag 603

<210> 200
<211> 597
<212> DNA
<213> E. Coli

<400> 200
gtgagtaaac gtcgttatct taccggtaaa gaagttcagg ccatgatgca ggcggtttgt 60
tacggggcaa cgggagccag agattattgt cttattctgt tggcatatcg gcatgggatg 120

cgtattagtg aactgcttga tctgcattat caggaccttg accttaatga aggtagaata 180
aatattcgcc gactgaagaa cggattttct accgttcacc cgttacgttt tgatgagcgt 240
gaagccgtgg aacgctggac ccaggaacgt gctaactgga aaggcgctga ccggactgac 300
gctatattta tttctcgccg cgggagtcgg ctttctcgcc agcaggccta tcgcattatt 360
cgcgatgccg gtattgaagc tggaaccgta acgcagactc atcctcatat gttaaggcat 420
gcttgcggtt atgaattggc ggagcgtggt gcagatactc gtttaattca ggattatctc 480
gggcatcgaa atattcgcca tactgtgcgt tataccgcca gtaatgctgc tcgttttgcc 540
ggattatggg aaagaaataa tctcataaac gaaaaattaa aaagagaaga ggtttga 597

<210> 201
<211> 549
<212> DNA
<213> E. Coli

<400> 201
atgaaaatta aaactctggc aatcgttgtt ctgtcggctc tgtccctcag ttctacagcg 60
gctctggccg ctgccacgac ggttaatggt gggaccgttc actttaaagg ggaagttgtt 120
aacgccgctt gcgcagttga tgcaggctct gttgatcaaa ccgttcagtt aggacaggtt 180
cgtaccgcat cgctggcaca ggaaggagca accagttctg ctgtcggttt taacattcag 240
ctgaatgatt gcgataccaa tgttgcatct aaagccgctg ttgccttttt aggtacggcg 300
attgatgcgg gtcataccaa cgttctggct ctgcagagtt cagctgcggg tagcgcaaca 360
aacgttggtg tgcagatcct ggacagaacg ggtgctgcgc tgacgctgga tggtgcgaca 420
tttagttcag aaacaaccct gaataacgga accaatacca ttccgttcca ggcgcgttat 480
tttgcaaccg gggccgcaac cccgggtgct gctaatgcgg atgcgacctt caaggttcag 540
tatcaataa 549

<210> 202
<211> 648
<212> DNA
<213> E. Coli

<400> 202
gtgctgctaa tgcggatgcg accttcaagg ttcagtatca ataacctacc caggttcagg 60
gacgtcatta cgggcaggga tgcccaccct tgtgcgataa aaataacgat gaaaaggaag 120
agattatttc tattagcgtc gttgctgcca atgtttgctc tggccggaaa taaatggaat 180
accacgttgc ccggcggaaa tatgcaattt cagggcgtca ttattgcgga aacttgccgg 240
attgaagccg gtgataaaca aatgacggtc aatatggggc aaatcagcag taaccggttt 300
catgcggttg gggaagatag cgcaccggtg ccttttgtta ttcatttacg ggaatgtagc 360
acggtggtga gtgaacgtgt aggtgtggcg tttcacggtg tcgcggatgg taaaaatccg 420
gatgtgcttt ccgtgggaga ggggccaggg atagccacca atattggcgt agcgttgttt 480
gatgatgaag gaaacctcgt accgattaat cgtcctccag caaactggaa acggctttat 540
tcaggctcta cttcgctaca tttcatcgcc aaatatcgtg ctaccgggcg tcgggttact 600
ggcggcatcg ccaatgccca ggcctggttc tctttaacct atcagtaa 648

<210> 203
<211> 726
<212> DNA
<213> E. Coli

<400> 203
gtgagtaata aaaacgtcaa tgtaaggaaa tcgcaggaaa taacattctg cttgctggca 60
ggtatcctga tgttcatggc aatgatggtt gccggacgcg ctgaagcggg agtggcctta 120
ggtgcgactc gcgtaattta tccggcaggg caaaaacaag agcaacttgc cgtgacaaat 180
aatgatgaaa atagtaccta tttaattcaa tcatgggtgg aaaatgccga tggtgtaaag 240
gatggtcgtt ttatcgtgac gcctcctctg tttgcgatga agggaaaaaa agagaatacc 300
ttacgtattc ttgatgcaac aaataaccaa ttgccacagg accgggaaag tttattctgg 360
atgaacgtta aagcgattcc gtcaatggat aaatcaaaat tgactgagaa tacgctacag 420
ctcgcaatta tcagccgcat taaactgtac tatcgcccgg ctaaattagc gttgccaccc 480
gatcaggccg cagaaaaatt aagatttcgt cgtagcgcga attctctgac gctgattaac 540
ccgacaccct attacctgac ggtaacagag ttgaatgccg gaacccgggt tcttgaaaat 600
gcattggtgc ctccaatggg cgaaagcacg gttaaattgc cttctgatgc aggaagcaat 660

attacttacc gaacaataaa tgattatggc gcacttaccc ccaaaatgac gggcgtaatg 720
gaataa 726

<210> 204
<211> 2637
<212> DNA
<213> E. Coli

<400> 204
atgtcatatc tgaatttaag actttaccag cgaaacacac aatgcttgca tattcgtaag 60
catcgtttgg ctggtttttt tgtccgactc gttgtcgcct gtgcttttgc cgcacaggca 120
cctttgtcat ctgccgacct ctattttaat ccgcgctttt tagcggatga tccccaggct 180
gtggccgatt tatcgcgttt tgaaaatggg caagaattac cgccagggac gtatcgcgtc 240
gatatctatt tgaataatgg ttatatggca acgcgtgatg tcacatttaa tacgggcgac 300
agtgaacaag ggattgttcc ctgcctgaca cgcgcgcaac tcgccagtat ggggctgaat 360
acggcttctg tcgccggtat gaatctgctg gcggatgatg cctgtgtgcc attaaccaca 420
atggtccagg acgctactgc gcatctggat gttggtcagc agcgactgaa cctgacgatc 480
cctcaggcat ttatgagtaa tcgcgcgcgt ggttatattc ctcctgagtt atgggatccc 540
ggtattaatg ccggattgct caattataat ttcagcggaa atagtgtaca gaatcggatt 600
gggggtaaca gccattatgc atatttaaac ctacagagtg ggttaaatat tggtgcgtgg 660
cgtttacgcg acaataccac ctggagttat aacagtagcg acagatcatc aggtagcaaa 720
aataaatggc agcatatcaa tacctggctt gagcgagaca taataccgtt acgttcccgg 780
ctgacgctgg gtgatggtta tactcagggc gatattttcg atggtattaa ctttcgcggc 840
gcacaattgg cctcagatga caatatgtta cccgatagtc aaagaggatt tgccccggtg 900
atccacggta ttgctcgtgg tactgcacag gtcactatta aacaaaatgg gtatgacatt 960
tataatagta cggtgccacc ggggcctttt accatcaacg atatctatgc cgcaggtaat 1020
agtggtgact tgcaggtaac gatcaaagag gctgacggca gcacgcagat ttttaccgta 1080
ccctattcgt cagtcccgct tttgcaacgt gaagggcata ctcgttattc cattacggca 1140
ggagaatacc gtagtggaaa tgcgcagcag gaaaaaaccc gctttttcca gagtacatta 1200
ctccacggcc ttccggctgg ctggacaata tatggtggaa cgcaactggc ggatcgttat 1260
cgtgctttta atttcggtat cgggaaaaac atgggggcac tgggcgctct gtctgtggat 1320
atgacgcagg ctaattccac acttcccgat gacagtcagc atgacggaca atcggtgcgt 1380
tttctctata acaaatcgct caatgaatca ggcacgaata ttcagttagt gggttaccgt 1440
tattcgacca gcggatattt taatttcgct gatacaacat acagtcgaat gaatggctac 1500
aacattgaaa cacaggacgg agttattcag gttaagccga aattcaccga ctattacaac 1560
ctcgcttata acaaacgcgg gaaattacaa ctcaccgtta ctcagcaact cgggcgcaca 1620
tcaacactgt atttgagtgg tagccatcaa acttattggg gaacgagtaa tgtcgatgag 1680
caattccagg ctggattaaa tactgcgttc gaagatatca actggacgct cagctatagc 1740
ctgacgaaaa acgcctggca aaaaggacgg gatcagatgt tagcgcttaa cgtcaatatt 1800
cctttcagcc actggctgcg ttctgacagt aaatctcagt ggcgacatgc cagtgccagc 1860
tacagcatgt cacacgatct caacggtcgg atgaccaatc tggctggtgt atacggtacg 1920
ttgctggaag acaacaacct cagctatagc gtgcaaaccg gctatgccgg gggaggcgat 1980
ggaaatagcg gaagtacagg ctacgccacg ctgaattatc gcggtggtta cggcaatgcc 2040
aatatcggtt acagccatag cgatgatatt aagcagctct attacggagt cagcggtggg 2100
gtactggctc atgccaatgg cgtaacgctg gggcagccgt taaacgatac ggtggtgctt 2160
gttaaagcgc ctggcgcaaa agatgcaaaa gtcgaaaacc agacgggggt gcgtaccgac 2220
tggcgtggtt atgccgtgct gccttatgcc actgaatatc gggaaaatag agtggcgctg 2280
gataccaata ccctggctga taacgtcgat ttagataacg cggttgctaa cgttgttccc 2340
actcgtgggg cgatcgtgcg agcagagttt aaagcgcgcg ttgggataaa actgctcatg 2400
acgctgaccc acaataataa gccgctgccg tttggggcga tggtgacatc agagagtagc 2460
cagagtagcg gcattgttgc ggataatggt caggtttacc tcagcggaat gcctttagcg 2520
ggaaaagttc aggtgaaatg gggagaagag gaaaatgctc actgtgtcgc caattatcaa 2580
ctgccaccag agagtcagca gcagttatta acccagctat cagctgaatg tcgttaa 2637

<210> 205
<211> 531
<212> DNA
<213> E. Coli

<400> 205
atgagaaaca aaccttttta tcttctgtgc gcttttttgt ggctggcggt gagtcacgct 60

ttggctgcgg atagcacgat tactatccgc ggctatgtca gggataacgg ctgtagtgtg 120
gccgctgaat caaccaattt tactgttgat ctgatggaaa acgcggcgaa gcaatttaac 180
aacattggcg cgacgactcc tgttgttcca tttcgtattt tgctgtcacc ctgtggtaat 240
gccgtttctg ccgtaaaggt tgggtttact ggcgttgcag atagccacaa tgccaacctg 300
cttgcacttg aaaatacggt gtcagcggct tcgggactgg gaatacagct tctgaatgag 360
cagcaaaatc aaatacccct taatgctcca tcgtccgcgc tttcgtggac gaccctgacg 420
ccgggtaaac caaatacgct gaatttttac gcccggctaa tggcgacaca ggtgcctgtc 480
actgcggggc atatcaatgc cacggctacc ttcactcttg aatatcagta a 531

<210> 206
<211> 504
<212> DNA
<213> E. Coli

<400> 206
atgaaatggt gcaaacgtgg gtatgtattg gcggcaatat tggcgctcgc aagtgcgacg 60
atacaggcag ccgatgtcac catcacggtg aacggtaagg tcgtcgccaa accgtgtacg 120
gtttccacca ccaatgccac ggttgatctc ggcgatcttt attctttcag tcttatgtct 180
gccggggcgg catcggcctg gcatgatgtt gcgcttgagt tgactaattg tccggtggga 240
acgtcgaggg tcactgccag cttcagcggg gcagccgaca gtaccggata ttataaaaac 300
caggggaccg cgcaaaacat ccagttagag ctacaggatg acagtggcaa cacattgaat 360
actggcgcaa ccaaaacagt tcaggtggat gattcctcac aatcagcgca cttcccgtta 420
caggtcagag cattgacagt aaatggcgga gccactcagg gaaccattca ggcagtgatt 480
agcatcacct atacctacag ctga 504

<210> 207
<211> 903
<212> DNA
<213> E. Coli

<400> 207
atgaaacgag ttattaccct gtttgctgta ctgctgatgg gctggtcggt aaatgcctgg 60
tcattcgcct gtaaaaccgc caatggtacc gctatcccta ttggcggtgg cagcgccaat 120
gtttatgtaa accttgcgcc cgtcgtgaat gtggggcaaa acctggtcgt ggatctttcg 180
acgcaaatct tttgccataa cgattatccg gaaaccatta cagactatgt cacactgcaa 240
cgaggctcgg cttatggcgg cgtgttatct aatttttccg ggaccgtaaa atatagtggc 300
agtagctatc catttcctac caccagcgaa acgccgcgcg ttgtttataa ttcgagaacg 360
gataagccgt ggccggtggc gctttatttg acgcctgtga gcagtgcggg cggggtggcg 420
attaaagctg gctcattaat tgccgtgctt attttgcgac agaccaacaa ctataacagc 480
gatgatttcc agtttgtgtg gaatatttac gccaataatg atgtggtggt gcctactggc 540
ggctgcgatg tttctgctcg tgatgtcacc gttactctgc cggactaccc tggttcagtg 600
ccaattcctc ttaccgttta ttgtgcgaaa agccaaaacc tggggtatta cctctccggc 660
acaaccgcag atgcgggcaa ctcgattttc accaataccg cgtcgttttc acctgcacag 720
ggcgtcggcg tacagttgac gcgcaacggt acgattattc cagcgaataa cacggtatcg 780
ttaggagcag tagggacttc ggcggtgagt ctgggattaa cggcaaatta tgcacgtacc 840
ggagggcagg tgactgcagg gaatgtgcaa tcgattattg gcgtgacttt tgtttatcaa 900
taa 903

<210> 208
<211> 1631
<212> DNA
<213> E. Coli

<400> 208
gtgctgtcaa aactaccccg tagactccga tcttttcaaa catattgcac catccgtgta 60
catcggggtg aggatatgaa atcaatggat aagttaacaa caggtgttgc ctatggcaca 120
tcggcgggta atgctggttt ctgggcattg cagttactcg ataaagtaac tccgtcacag 180
tgggctgcaa tcggtgtgct gggtagcctg gtttttggcc tgctgacgta tctgacaaat 240
ctttatttca agattaaaga agacaggcgt aaggctgcga gaggagagta atccaatgac 300
tcaagactat gaactggttg tgaaaggagt ccgtaatttt gagaataaag ttacggtaac 360
tgtagcctta caggacaaag aacgctttga cggtgaaatt tttgacctgg atgtcgccat 420

ggaccgtgtt gaaggagctg cgctggagtt ttatgaggca gcagccagaa ggagcgtccg 480
gcaagtcttc ctggaagtag cagaaaaatt gtcagaaaaa gttgagtctt atctgcagca 540
tcagtactcc tttaagattg aaaatcctgc caataagcac gagcgtcctc atcataaata 600
tctatgaaca caaaaatcag atacggcctg tcggctgccg ttctggcgct gattggtgct 660
ggcgcatctg ctcctcagat acttgaccag tttctggacg aaaaagaagg taaccacaca 720
atggcatacc gcgatggttc tggcatatgg accatctgtc ggggtgccac agtggtggat 780
ggaaaaaccg tttttcccaa tatgaaactg tcgaaggaaa aatgcgacca ggtcaacgcc 840
attgagcgtg ataaggcgct ggcatgggtg gagcgcaata ttaaagtacc actgaccgaa 900
ccacaaaaag cgggtatcgc gtcattttgt ccctataaca ttggccccgg taagtgtttc 960
ccgtcgacgt tttataagcg gctgaatgct ggtgatcgta aaggtgcatg cgaagcgatt 1020
cgctggtgga ttaaggatgg cggacgcgat tgccgcattc gttcaaataa ctgttacggt 1080
caggttattc gtcgtgacca ggagagcgca ttaacctgct gggggataga acagtgaatc 1140
agatattcat ggtgattttt ctcgtgttgt caggatttat cgtcggaaat gtctggagcg 1200
accgaggatg gcaaaaaaaa tgggcggaac gtgatgctgc cgcattatca caagaggtaa 1260
atgctcaatt tgctgctcga ataattgaac aggggcgaac tatagcccgt gatgaggctg 1320
ttaaagatgc gcaacagaaa tctgctgaaa tttctgccag ggctgcttat ctgtctgata 1380
gtgttaacca gttgcgtgcc gaagcaaaaa aatatgccat acgccttgac gcagcgaagc 1440
ataccgcaga tcttgccgct gccgtcagag gcaaaacaac caaaaccgcc gaaggaatgc 1500
tcaccaacat gctcggagat attgcagcag aagctcagct ttatgctgaa attgctgacg 1560
aacgctacat cgcaggagtg acttgtcaac agatctatga atctttaaga gataaaaagc 1620
atcaaatgta g 1631

<210> 209
<211> 534
<212> DNA
<213> E. Coli

<400> 209
atgaacacaa aaatcagata cggcctgtcg gctgccgttc tggcgctgat tggtgctggc 60
gcatctgctc ctcagatact tgaccagttt ctggacgaaa aagaaggtaa ccacacaatg 120
gcataccgcg atggttctgg catatggacc atctgtcggg gtgccacagt ggtggatgga 180
aaaaccgttt ttcccaatat gaaactgtcg aaggaaaaat gcgaccaggt caacgccatt 240
gagcgtgata aggcgctggc atgggtggag cgcaatatta aagtaccact gaccgaacca 300
caaaaagcgg gtatcgcgtc attttgtccc tataacattg gccccggtaa gtgtttcccg 360
tcgacgtttt ataagcggct gaatgctggt gatcgtaaag gtgcatgcga agcgattcgc 420
tggtggatta aggatggcgg acgcgattgc cgcattcgtt caaataactg ttacggtcag 480
gttattcgtc gtgaccagga gagcgcatta acctgctggg ggatagaaca gtga 534

<210> 210
<211> 312
<212> DNA
<213> E. Coli

<400> 210
atgactcaag actatgaact ggttgtgaaa ggagtccgta attttgagaa taaagttacg 60
gtaactgtag ccttacagga caaagaacgc tttgacggtg aaatttttga cctggatgtc 120
gccatggacc gtgttgaagg agctgcgctg gagttttatg aggcagcagc cagaaggagc 180
gtccggcaag tcttcctgga agtagcagaa aaattgtcag aaaaagttga gtcttatctg 240
cagcatcagt actcctttaa gattgaaaat cctgccaata agcacgagcg tcctcatcat 300
aaatatctat ga 312

<210> 211
<211> 291
<212> DNA
<213> E. Coli

<400> 211
gtgctgtcaa aactaccccg tagactccga tcttttcaaa catattgcac catccgtgta 60
catcggggtg aggatatgaa atcaatggat aagttaacaa caggtgttgc ctatggcaca 120
tcggcgggta atgctggttt ctgggcattg cagttactcg ataaagtaac tccgtcacag 180
tgggctgcaa tcggtgtgct gggtagcctg gtttttggcc tgctgacgta tctgacaaat 240

ctttatttca agattaaaga agacaggcgt aaggctgcga gaggagagta a 291

<210> 212
<211> 216
<212> DNA
<213> E. Coli

<400> 212
atgtcaaata aaatgactgg tttagtaaaa tggtttaacg ctgataaagg tttcggcttt 60
atttctcctg ttgatggtag taaagatgtg tttgtgcatt tttctgcgat tcagaatgat 120
aattatcgaa ccttatttga aggtcaaaag gttaccttct ctatagagag tggtgctaaa 180
ggtcctgcag cagcaaatgt catcattact gattaa 216

<210> 213
<211> 1017
<212> DNA
<213> E. Coli

<400> 213
atgtttgtca tctggagcca tagaacaggg ttcatcatga gtcatcaact taccttcgcc 60
gacagtgaat tcagcagtaa gcgccgtcag accagaaaag agattttctt gtcccgcatg 120
gagcagattc tgccatggca aaacatggtg gaagtcatcg agccgtttta ccccaaggct 180
ggtaatggcc ggcgacctta tccgctggaa accatgctac gcattcactg catgcagcat 240
tggtacaacc tgagcgatgg cgcgatggaa gatgctctgt acgaaatcgc ctccatgcgt 300
ctgtttgccc ggttatccct ggatagcgcc ttgccggacc gcaccaccat catgaatttc 360
cgccacctgc tggagcagca tcaactggcc cgccaattgt tcaagaccat caatcgctgg 420
ctggccgaag caggcgtcat gatgactcaa ggcaccttgg tcgatgccac catcattgag 480
gcacccagct cgaccaagaa caaagagcag caacgcgatc cggagatgca tcagaccaag 540
aaaggcaatc agtggcactt tggcatgaag gcccacattg gtgtcgatgc caagagtggc 600
ctgacccaca gcctggtcac caccgcggcc aacgagcatg acctcaatca gctgggtaat 660
ctgctgcatg gagaggagca atttgtctca gccgatgccg gctaccaagg ggcgccacag 720
cgcgaggagc tggccgaggt ggatgtggac tggctgatcg ccgagcgccc cggcaaggta 780
agaaccttga aacagcatcc acgcaagaac aaaacggcca tcaacatcga atacatgaaa 840
gccagcatcc gggccagggt ggagcaccca tttcgcatca tcaagcgaca gttcggcttc 900
gtgaaagcca gatacaaggg gttgctgaaa aacgataacc aactggcgat gttattcacg 960
ctggccaacc tgtttcgggc ggaccaaatg atacgtcagt gggagagatc tcactaa 1017

<210> 214
<211> 474
<212> DNA
<213> E. Coli

<400> 214
atggtatata taataatcgt ttcccacgga catgaagact acatcaaaaa attactcgaa 60
aatcttaatg ctgacgatga gcactacaag attatcgtac gcgacaacaa agactctcta 120
ttattgaaac aaatatgcca gcattatgca ggcctggact atattagtgg aggtgtatac 180
ggctttggtc ataataataa tattgcggtg gcgtatgtaa aggaaaaata tagacccgca 240
gatgatgatt acattttgtt tttgaatccc gatatcatca tgaagcatga tgatttgctg 300
acatatatta aatatgtcga aagtaagcgt tatgctttta gtacattatg cctgttccga 360
gatgaagcga aatctttaca tgattattcc gtaagaaaat ttcctgtgct ttctgatttt 420
attgtgtcat ttatgttagg gattaaggaa ggtgcgaaca agtccctgat atga 474

<210> 215
<211> 1119
<212> DNA
<213> E. Coli

<400> 215
atgggaaaaa gcatagtcgt tgtttctgcg gtcaatttta ccactggcgg tccatttacc 60
attttgaaaa aatttttggc agcaactaat aataaagaaa atgtcagttt tatcgcatta 120
gtccattctg ctaaagagtt aaaagaaagt tatccatggg ttaaattcat tgagtttcct 180

gaggttaaag ggtcgtggct aaaacgtttg cactttgaat atgtagtttg taaaaaactt 240
tcaaaagagc tgaatgctac gcattggatt tgtctgcatg atattacggc caatgtcgtc 300
actaaaaaaa gatatgtgta ttgtcataac cctgcacctt tttataaagg aattttattc 360
cgtgaaattc ttatggagcc tagctttttc ttatttaaaa tgctatacgg gctgatatat 420
aaaataaaca ttaaaaaaaa tactgcagtg tttgttcaac aattctggat gaaagaaaaa 480
tttatcaaga aatattctat aaataacatc attgtcagtc ggccagaaat taaattatct 540
gataaaagcc aacttactga tgatgattct caatttaaga ataacccttc tgagttgaca 600
atattttacc ctgctgttcc acgagtattt aaaaattacg agcttattat tagtgcagca 660
aggaaattga aagaacaatc caatattaaa tttctgctta ctatcagtgg tacagaaaat 720
gcgtatgcaa aatatattat cagtcttgca gaaggactgg ataatgttca tttcctcggg 780
tacttggata aagaaaaaat cgatcattgt tataatattt cagatatagt ttgttttccc 840
tctaggttag aaacatgggg attgccgttg tctgaagcta aagagcgagg taagtgggta 900
ttagcatcag atttcccatt tactagagaa actcttggta gttatgaaaa gaaagctttt 960
tttgattcta ataacgatga catgttagtt aaacttatta ttgacttcaa aaaaggtaac 1020
ctcaaaaaag atatctctga tgcaaatttc atttatcgta atgaaaatgt attagttggg 1080
tttgatgaac tagttaattt tattactgaa gaacattga 1119

<210> 216
<211> 591
<212> DNA
<213> E. Coli

<400> 216
atgatcttaa aactcgctaa acgatatggt ctctgtggtt ttattcggct tgttagagat 60
gtcttattga ctcgtgtatt ttaccggaac tgtagaatta ttcgatttcc ctgctatatt 120
cgcaatgatg gtagcattaa ttttggtgaa aatttcacaa gtggagtcgg tctcaggctg 180
gatgcatttg gacgtggcgt gatttttttt tccgataatg tgcaagttaa cgactatgtt 240
catatcgcct caattgagag cgttacgata ggtcgggata cgcttattgc aagtaaagta 300
tttattaccg atcataatca cggttccttt aagcactctg atccaatgag ttcgccaaat 360
atacctccag acatgcgcac gttggaatct tcagctgttg taattggcca gagggtttgg 420
ttgggtgaga atgtgacggt tttgcctgga acaattattg gtaatggagt cgtagtcggc 480
gccaattctg ttgttagagg ttctattccc gaaaatactg tcattgcggg agtaccagca 540
aaaatcataa agaaatacaa tcatgagacc aaattatggg aaaaagcata g 591

<210> 217
<211> 993
<212> DNA
<213> E. Coli

<400> 217
atgtattttt tgaatgattt aaatttctct agacgcgatg ctggatttaa agcaagaaaa 60
gatgcactgg acattgcttc agattatgaa aacatttctg ttgttaacat tcctctatgg 120
ggtggagtag tccagagaat tattagttct gttaagctta gtacatttct ctgcggtctt 180
gaaaataaag atgttttaat tttcaatttc ccgatggcca aaccattttg gcatatattg 240
tcattctttc accgccttct aaaatttaga atagtacctc tgattcatga tattgatgaa 300
ttaagaggag gagggggtag tgattctgtg cggcttgcta cctgtgatat ggtcataagt 360
cacaatccac aaatgacaaa gtaccttagt aaatatatgt ctcaggataa aatcaaagac 420
ataaaaatat ttgattacct cgtctcatct gatgtggagc atcgagatgt tacggataag 480
caacgagggg tcatatatgc tggcaacctt tctaggcata aatgttcttt catatatact 540
gaaggatgcg attttactct ctttggtgtc aactatgaaa ataaagataa tcctaaatat 600
cttggaagtt ttgatgctca atctccggaa aagattaacc tcccaggcat gcaatttgga 660
ctcatttggg atggagattc tgtcgaaacc tgtagtggtg cctttggcga ctatttaaag 720
tttaataacc ctcataagac atctctttat ctttcaatgg aacttccagt atttatatgg 780
gataaagccg cccttgcgga tttcattgta gataatagaa taggatatgc agtgggatca 840
atcaaagaaa tgcaagagat tgttgactcc atgacaatag aaacttataa gcaaattagt 900
gagaatacaa aaattatttc tcagaaaatt cgaacaggaa gttacttcag ggatgttctt 960
gaagaggtga tcgatgatct taaaactcgc taa 993

<210> 218
<211> 1167
<212> DNA

<213> E. Coli

<400> 218
atgatctatc ttgtaattag tgtctttctc attacagcat ttatctgttt atatcttaag 60
aaggatatat tttatccagc cgtatgcgtt aatatcatct tcgcactggt cttattggga 120
tatgaaataa cgtcagatat atatgctttt cagttaaatg acgctacgtt gatttttcta 180
ctttgcaatg ttttgacatt taccctgtca tgtttattga cggaaagtgt attagatcta 240
aatatcagaa aagtcaataa tgctatttat agcataccat cgaagaaagt gcataatgta 300
ggcttgttag ttatttcttt ttcgatgata tatatatgca tgaggttaag taactaccag 360
ttcgggacta gcttacttag ctatatgaat ttgataagag atgctgatgt tgaagacaca 420
tcaagaaatt tctcagcata catgcagcca atcattctaa ctacttttgc tttatttatt 480
tggtctaaaa aatttactaa tacaaaggta agtaaaacat ttactttact tgtttttatt 540
gtattcatct ttgcaattat actgaatact ggtaagcaaa ttgtctttat ggttatcatc 600
tcttatgcat tcatcgtagg tgttaataga gtaaaacatt atgtttatct tattacagct 660
gtaggtgttc tattctcctt gtatatgctc tttttacgtg gactgcctgg ggggatggca 720
tattatctat ccatgtattt ggtcagccct ataatcgcgt ttcaggagtt ttattttcag 780
caagtatcta actctgccag ttctcatgtc ttttggtttt ttgaaaggct gatggggcta 840
ttaacaggtg gagtctctat gtcgttgcat aaagaatttg tgtgggtggg tttgccaaca 900
aatgtttata ctgctttttc ggattatgtt tatatttccg cggagctaag ctatttgatg 960
atggttattc atggctgtat ttcaggtgtt ttatggagat tgtctcgaaa ttacatatct 1020
gtgaaaatat tttattcata ttttatttat accttttctt tcattttta tcatgaaagc 1080
ttcatgacta atattagcag ttggatacaa ataactcttt gtatcatagt attctctcaa 1140
tttcttaagg cccagaaaat aaagtga 1167

<210> 219
<211> 1104
<212> DNA
<213> E. Coli

<400> 219
atgtacgatt atatcattgt tggttctggt ttgtttggtg ccgtttgtgc gaatgagtta 60
aaaaagctaa acaaaaaagt tttagtgatt gagaaaagaa atcatatcgg tggaaatgcg 120
tacacagagg actgtgaggg tatccagatt cataaatatg gtgcacatat ttttcatacc 180
aatgataaat atatatggga ttacgttaat gatttagtag aatttaatcg ttttactaat 240
tctccactgg cgatttataa agacaaatta ttcaaccttc cttttaatat gaatactttc 300
caccaaatgt ggggagttaa agatcctcaa gaagctcaaa atatcattaa tgctcagaaa 360
aaaaagtatg gtgacaaggt acctgaaaat ttggaggagc aggcgatttc attagttggg 420
gaggacttat accaagcatt gataaagggt tatacggaga agcagtgggg aagaagtgca 480
aaagaattgc ctgcatttat tattaagcga atcccagtga gatttacgtt tgataacaat 540
tatttttccg atcgctatca aggtattccg gtgggaggct acactaagct tattgaaaaa 600
atgcttgaag gtgtggacgt aaaattaggc attgattttt tgaaagacaa agattctcta 660
gcgagtaaag cccatagaat catctacact ggacccattg atcagtactt cgactatagg 720
tttggagcgt tagaatatcg ctctttaaaa tttgagacgg aacgccatga atttccaaac 780
ttccaaggga atgcagtaat aaatttcact gatgctaatg taccatatac cagaataatt 840
gagcataaac attttgacta tgttgagaca aagcatacgg ttgttacaaa agaatatcca 900
ttagagtgga aagttggcga cgaaccctac tatccagtta atgataataa aaacatggag 960
ctttttaaga aatatagaga gttagctagc agagaagaca aggttatatt tggcgggcgt 1020
ttggccgagt ataaatatta tgatatgcat caagtgatat ctgccgctct ttatcaagtg 1080
aaaaatataa tgagtacgga ttaa 1104

<210> 220
<211> 1116
<212> DNA
<213> E. Coli

<400> 220
atgttcccaa aaataatgaa tgatgaaaac tttttcaaaa aagcggcggc gcacggggag 60
gaacctcctt taactcctca aaacgaacat cagcggtccg ggctgcgctt cgcccgtcgc 120
gtcagactac cccgtgcggt tggcctggct ggcatgttct taccgattgc ttcaacgctg 180
gtttcacacc cgccgccggg ctggtggtgg ctggtgttgg tcggctgggc gttcgtctgg 240
ccgcatttag cctggcagat agcgagcagg gccgtcgatc cgcttagccg ggaaatttac 300

aacttaaaaa ccgatgcagt attagcggga atgtgggtag gcgtaatggg cgtaaacgtg 360
ctgccttcca ccgcgatgtt gatgattatg tgtctgaatt tgatgggggc aggcggcccc 420
cgtctgtttg tcgcgggtct ggtgttgatg gtggtttcct gccttgtcac cctcgagctg 480
acgggcatta ccgtgtcgtt caatagtgcg ccgctggaat ggtggctctc ccttcccatt 540
attgtcattt atcctctgct gtttggctgg gtcagctacc agacggcaac caaactggcg 600
gaacataaac gcaggttgca ggtcatgagt acccgcgacg gcatgacggg cgtgtataac 660
cgacgtcatt gggaaactat gttacgcaat gaatttgata actgtcggcg gcataatcgc 720
gatgcaacgt tactgattat cgatatcgac catttcaaga gcatcaacga tacctggggc 780
catgatgtgg gcgatgaagc gattgtggcg cttacccgac agttacaaat taccctgcgc 840
ggtagcgatg tgattggtcg gtttggcggc gatgagtttg cagtaatcat gtccggtacg 900
ccagctgaga gcgccattac cgccatgtta cgggtgcatg aagggctaaa tacattacgt 960
ttgccgaata cgccacaggt aactttacgg attagtgtgg gggttgcgcc gctgaaccca 1020
caaatgagtc actatcgtga gtggttgaaa tcggcagatt tggcgcttta caaagcaaag 1080
aaagccggac gtaaccgcac cgaagtggcg gcctga 1116

<210> 221
<211> 1404
<212> DNA
<213> E. Coli

<400> 221
ttggatgtga acgttgatca gttcgatact gaagctttcc gtactgacaa actggaactg 60
accagcggca acatcgctga ccataacggt aacgtagtat ctggtgtgtt cgatatccat 120
agcagcgatt acgttctgaa cgctgatctg gtgaacgacc gtacctggga tacttccaag 180
tctaactacg gttacggtat tgttgctatg aactctgatg gtcacctgac tatcaacggt 240
aacggcgacg tagacaacgg tactgaactg gataacagct ctgtagacaa tgttgttgct 300
gcaaccggta actacaaagt tcgtatcgac aacgcaactg gcgctggcgc tatcgctgat 360
tacaaagata aagaaattat ctacgtaaac gacgtcaaca gcaacgcgac cttctctgct 420
gctaacaaag ctgacctggg tgcatacacc tatcaggctg aacagcgcgg taacaccgtt 480
gttctgcaac agatggagct gaccgactac gctaacatgg cgctgagcat cccgtctgcg 540
aacaccaata tctggaacct ggaacaagac accgttggta ctcgtctgac caactctcgt 600
catggcctgg ctgataacgg cggcgcatgg gtaagctact tcggtggtaa cttcaacggc 660
gacaacggca ccatcaacta tgatcaggat gttaacggca tcatggtcgg tgttgatacc 720
aaaattgacg gtaacaacgc taagtggatc gtcggtgcgg ctgcaggctt cgctaaaggt 780
gacatgaatg accgttctgg tcaggtggat caagacagcc agactgccta catctactct 840
tctgctcact tcgcgaacaa cgtctttgtt gatggtagct tgagctactc tcacttcaac 900
aacgacctgt ctgcaaccat gagcaacggt acttacgttg acggtagcac caactccgac 960
gcttggggct tcggtttgaa agccggttac gacttcaaac tgggtgatgc tggttacgtg 1020
actccttacg gcagcgtttc tggtctgttc cagtctggtg atgactacca gctgagcaac 1080
gacatgaaag ttgacggtca gtcttacgac agcatgcgtt atgaactggg tgtagatgca 1140
ggttatacct tcacctacag cgaagatcag gctctgactc cgtacttcaa actggcttac 1200
gtctacgacg actctaacaa cgataacgat gtgaacggcg attccatcga taacggtact 1260
gaagggtctg cggtacgtgt tggtctgggt actcagttta gcttcaccaa gaacttcagc 1320
gcctataccg atgctaacta cctcggtggt ggtgacgtag atcaagactg gtccgcgaac 1380
gtgggtgtta aatatacctg gtaa 1404

<210> 222
<211> 669
<212> DNA
<213> E. Coli

<400> 222
atgcccgtca aggatttgac gggcattact gcaaaggacg cgcaaatgtt atctgtagtt 60
aaacctcttc aggaatttgg taagctcgat aaatgtttgt ccagatacgg tacgcgcttc 120
gagtttaata atgaaaagca agttatattt tccagtgatg tcaataacga agatactttc 180
gttattttag agggagttat ctctctgcgt agagaagaaa acgtacttat cggtattacc 240
caggctcctt atattatggg gctggctgat ggtttaatga aaaacgatat accatacaaa 300
ttaatatcag aaggaaattg tacgggatat catctaccag ccaaacaaac cattacgctt 360
attgaacaaa atcaactctg gcgagacgct ttttactggt tagcctggca aaatagaatt 420
ctggaattac gcgacgtgca gctcattggg cataattcct acgaacaaat ccgcgcaaca 480
ttattatcaa tgattgactg gaatgaagaa ttgcgatcac gtattggtgt gatgaattat 540

atccatcaac gtacacgcat atcgcgttct gtcgtcgcag aagttctcgc tgctttgcgt 600
aaaggcggct atatcgaaat gaataaaggc aaactggtcg ctatcaaccg tttgccttca 660
gagtattaa 669

<210> 223
<211> 255
<212> DNA
<213> E. Coli

<400> 223
atgaccgata aaatccgtac tctgcaaggt cgcgttgtta gcgacaaaat ggagaaatcc 60
attgttgttg ctatcgaacg ttttgtgaaa cacccgatct acggtaaatt catcaagcgt 120
acgaccaaac tgcacgtaca tgacgagaac aacgaatgcg gtatcggtga cgtggttgaa 180
atccgcgaat gccgtccgct gtccaagact aaatcctgga cgctggttcg cgttgtagag 240
aaagcggttc tgtaa 255

<210> 224
<211> 192
<212> DNA
<213> E. Coli

<400> 224
atgaaagcaa aagagctgcg tgagaagagc gttgaagagc tgaacaccga gctgctgaac 60
ctgctgcgtg agcagttcaa cctgcgtatg caggctgcaa gtggccagct gcaacagtct 120
cacctgttga agcaagtgcg tcgcgatgtc gcacgcgtta agactttact gaacgagaag 180
gcgggtgcgt aa 192

<210> 225
<211> 411
<212> DNA
<213> E. Coli

<400> 225
atgttacaac caaagcgtac aaaattccgt aaaatgcaca aaggccgtaa ccgcggtctg 60
gcgcagggta cggatgttag cttcggcagc ttcggtctga aagctgttgg ccgtggtcgt 120
ctgactgccc gtcagatcga agcagcacgt cgtgctatga cccgtgcagt taagcgtcaa 180
ggtaagatct ggatccgtgt gttcccggac aaaccgatca ctgaaaagcc gctggcagtg 240
cgtatgggta aaggtaaagg taacgtggag tattgggttg ccttgattca gccgggtaaa 300
gtcctgtatg aaatggacgg tgttccggaa gagctggccc gtgaagcatt caagctggca 360
gcagcgaaac tgccgattaa aaccaccttt gtaactaaga cggtgatgta a 411

<210> 226
<211> 702
<212> DNA
<213> E. Coli

<400> 226
atgggtcaga aagtacatcc taatggtatt cgcctgggta ttgtaaaacc atggaactct 60
acctggtttg cgaacaccaa agaattcgct gacaacctgg acagcgattt taaagtacgt 120
cagtacctga ctaaggaact ggctaaagcg tccgtatctc gtatcgttat cgagcgtccg 180
gctaagagca tccgtgtaac cattcacact gctcgcccgg gtatcgttat cggtaaaaaa 240
ggtgaagacg tagaaaaact gcgtaaggtc gtagcggaca tcgctggcgt tcctgcacag 300
atcaacatcg ccgaagttcg taagcctgaa ctggacgcaa aactggttgc tgacagcatc 360
acttctcagc tggaacgtcg cgttatgttc cgtcgtgcta tgaagcgtgc tgtacagaac 420
gcaatgcgtc tgggcgctaa aggtattaaa gttgaagtta gcggccgtct gggcggcgcg 480
gaaatcgcac gtaccgaatg gtaccgcgaa ggtcgcgtac cgctgcacac tctgcgtgct 540
gacatcgact acaacacctc tgaagcgcac accacttacg gtgtaatcgg cgttaaagtg 600
tggatcttca aaggcgagat cctgggtggt atggctgctg ttgaacaacc ggaaaaaccg 660
gctgctcagc ctaaaaagca gcagcgtaaa ggccgtaaat aa 702

<210> 227

<211> 333
<212> DNA
<213> E. Coli

<400> 227
atggaaacta tcgctaaaca tcgccatgct cgttcttctg ctcagaaggt tcgccttgtt 60
gctgacctga ttcgcggtaa gaaagtgtcg caggctctgg atattttgac ctacaccaac 120
aagaaagcgg ctgtactggt caagaaagtt ctggaatctg ccattgctaa cgctgaacac 180
aacgatggcg ctgacattga cgatctgaaa gttacgaaaa ttttcgtaga cgaaggcccg 240
agcatgaagc gcattatgcc gcgtgcaaaa ggtcgtgcag atcgcatcct gaagcgcacc 300
agccacatca ctgtggttgt gtccgatcgc tga 333

<210> 228
<211> 279
<212> DNA
<213> E. Coli

<400> 228
atgccacgtt ctctcaagaa aggtcctttt attgacctgc acttgctgaa gaaggtagag 60
aaagcggtgg aaagcggaga caagaagccc ctgcgcactt ggtcccgtcg ttcaacgatc 120
tttcctaaca tgatcggttt gaccatcgct gtccataatg gtcgtcagca cgttccggta 180
tttgtaaccg acgaaatggt tggtcacaaa ctgggtgaat tcgcaccgac tcgtacttat 240
cgcggccacg ctgctgataa aaaagcgaag aagaaataa 279

<210> 229
<211> 822
<212> DNA
<213> E. Coli

<400> 229
atggcagttg ttaaatgtaa accgacatct ccgggtcgtc gccacgtagt taaagtggtt 60
aaccctgagc tgcacaaggg caaacctttt gctccgttgc tggaaaaaaa cagcaaatcc 120
ggtggtcgta acaacaatgg ccgtatcacc actcgtcata tcggtggtgg ccacaagcag 180
gcttaccgta ttgttgactt caaacgcaac aaagacggta tcccggcagt tgttgaacgt 240
cttgagtacg atccgaaccg ttccgcgaac atcgcgctgg ttctgtacaa agacggtgaa 300
cgccgttaca tcctggcccc taaaggcctg aaagctggcg accagattca gtctggcgtt 360
gatgctgcaa tcaaaccagg taacaccctg ccgatgcgca acatcccggt tggttctact 420
gttcataacg tagaaatgaa accaggtaaa ggcggtcagc tggcacgttc cgctggtact 480
tacgttcaga tcgttgctcg tgatggtgct tatgtcaccc tgcgtctgcg ttctggtgaa 540
atgcgtaaag tagaagcaga ctgccgtgca actctgggcg aagttggcaa tgctgagcat 600
atgctgcgcg ttctgggtaa agcaggtgct gcacgctggc gtggtgttcg tccgaccgtt 660
cgcggtaccg cgatgaaccc ggtagaccac ccacatggtg gtggtgaagg tcgtaacttt 720
ggtaagcacc cggtaactcc gtggggcgtt cagaccaaag gtaagaagac ccgcagcaac 780
aagcgtactg ataaattcat cgtacgtcgc cgtagcaaat aa 822

<210> 230
<211> 303
<212> DNA
<213> E. Coli

<400> 230
atgattcgtg aagaacgtct gctgaaggtg ctgcgtgcac cgcacgtttc tgaaaaagcg 60
tctactgcga tggaaaaatc caacaccatc gtactcaaag ttgctaaaga cgcgaccaaa 120
gcagaaatca aagctgctgt gcagaaactg tttgaagtcg aagtcgaagt cgttaacacc 180
ctggtagtta aaggaaaagt taaacgtcac ggacagcgta tcggtcgtcg tagcgactgg 240
aaaaaagctt acgtcaccct gaaagaaggc cagaatctgg acttcgttgg cggcgctgag 300
taa 303

<210> 231
<211> 630
<212> DNA

<213> E. Coli

<400> 231
atgattggtt tagtcggtaa aaaagtgggt atgacccgta tcttcacaga agacggcgtt 60
tctatcccag taaccgtaat cgaagttgaa gcaaaccgcg ttactcaggt taaagacctg 120
gctaacgatg gctaccgtgc tattcaggtg accaccggtg ctaaaaaagc taaccgtgtg 180
accaagcctg aagctggcca cttcgctaaa gctggcgtag aagctggccg tggtctgtgg 240
gaattccgcc tggctgaagg cgaagagttc actgtaggtc agagcattag cgttgaactg 300
tttgctgacg ttaaaaaagt tgacgtaact ggcacctcta aaggtaaagg tttcgcaggt 360
accgttaagc gctggaactt ccgtacccag gacgctactc acggtaactc cttgtctcac 420
cgcgttccgg gttctatcgg tcagaaccag actccgggca aagtgttcaa aggcaagaaa 480
atggcaggtc agatgggtaa cgaacgtgta accgttcaga gccttgacgt agtacgcgtt 540
gacgctgagc gcaacctgct gctggttaaa ggtgctgtcc cgggtgcaac cggtagcgac 600
ctgatcgtta aaccagctgt gaaggcgtaa 630

<210> 232
<211> 606
<212> DNA
<213> E. Coli

<400> 232
atggaattag tattgaaaga cgcgcagagc gcgctgactg tttccgaaac taccttcggt 60
cgtgatttca acgaagcgct ggttcaccag gttgttgttg cttatgcagc tggtgctcgt 120
cagggtactc gtgctcagaa gactcgtgct gaagtaactg gttccggtaa aaaaccgtgg 180
cgccagaaag gcaccggccg tgcgcgttct ggttctatca agagcccgat ctggcgttct 240
ggtggcgtga cctttgctgc tcgtccgcag gaccacagtc aaaaagttaa caagaagatg 300
taccgcggcg cgctgaaaag catcctgtcc gaactggtac gtcaggatcg tctgatcgtt 360
gtcgagaagt tctctgtaga agcgccgaaa actaagctgc tggcacagaa actgaaagac 420
atggctctgg aagatgtgct gatcatcacc ggtgagctgg acgaaaacct gttcctggct 480
gcgcgcaacc tgcacaaggt tgacgtacgc gatgcaactg gtatcgaccc ggttagcctg 540
atcgccttcg acaaagtcgt aatgactgct gatgctgtta agcaagttga ggagatgctg 600
gcatga 606

<210> 233
<211> 312
<212> DNA
<213> E. Coli

<400> 233
atgcagaacc aaagaatccg tatccgcctg aaagcgtttg atcatcgtct gatcgatcaa 60
gcaaccgcgg aaatcgtcga gactgccaag cgcactggtg cgcaggtccg tggtccgatc 120
ccgctgccga cacgcaaaga gcgcttcact gttctgatct ccccgcacgt caacaaagac 180
gcgcgcgatc agtacgaaat ccgtactcac ttgcgtctgg ttgacatcgt tgagccaacc 240
gagaaaaccg ttgatgctct gatgcgtctg gatctggctg ccggtgtaga cgtgcagatc 300
agcctgggtt aa 312

<210> 234
<211> 357
<212> DNA
<213> E. Coli

<400> 234
atggctcgcg taaaacgtgg tgttattgca cgtgcacgtc acaagaaaat tttgaaacaa 60
gctaaaggct actacggtgc gcgttctcgc gtataccgcg ttgccttcca ggctgttatc 120
aaagctggtc agtatgctta ccgtgaccgt cgtcaacgta agcgtcagtt ccgtcaactg 180
tggattgcgc gtatcaacgc agcagcacgt cagaacggta tttcttacag caaattcatc 240
aatggcctga aaaaagcctc tgttgaaatc gaccgtaaga tcctggctga tatcgcagta 300
ttcgacaaag tagcgttcac cgctctggtt gaaaaagcga aagcagctct ggcataa 357

<210> 235
<211> 198

<212> DNA
<213> E. Coli

<400> 235
atgccaaaaa ttaagaccgt acgcggtgct gctaagcgct tcaaaaaaac cggtaaaggt 60
ggttttaagc acaagcacgc taacctgcgt cacattctga ccaaaaaagc gaccaaacgt 120
aaacgtcacc tgcgtccgaa agccatggtt tccaaaggcg atctgggcct ggtaatcgcg 180
tgcctgccgt acgcataa 198

<210> 236
<211> 543
<212> DNA
<213> E. Coli

<400> 236
attaaaggcg gaaaacgagt tcaaacggcg cgccctaacc gtatcaatgg cgaaattcgc 60
gcccaggaag ttcgcttaac aggtctggaa ggcgagcagc ttggtattgt gagtctgaga 120
gaagctctgg agaaagcaga agaagccgga gtagacttag tcgagatcag ccctaacgcc 180
gagccgccgg tttgtcgtat aatggattac ggcaaattcc tctatgaaaa gagcaagtct 240
tctaaggaac agaagaaaaa gcaaaaagtt atccaggtta aggaaattaa attccgtcct 300
ggtacagatg aaggcgacta tcaggtaaaa ctccgcagcc tgattcgctt tctcgaagag 360
ggtgataaag ccaaaatcac gctgcgtttc cgcggtcgtg agatggcgca ccagcaaatc 420
ggtatggaag tgcttaatcg cgtgaaagac gatttgcaag aactggcagt ggtcgaatcc 480
ttcccaacga agatcgaagg ccgccagatg atcatggtgc tcgctcctaa gaagaaacag 540
taa 543

<210> 237
<211> 1929
<212> DNA
<213> E. Coli

<400> 237
atgcctgtta taactcttcc tgatggcagc caacgccatt acgatcacgc tgtaagcccc 60
atggatgttg cgctggacat tggtccaggt ctggcgaaag cctgtatcgc agggcgcgtt 120
aatggcgaac tggttgatgc ttgcgatctg attgaaaacg acgcacaact gtcgatcatt 180
accgccaaag acgaagaagg tctggagatc attcgtcact cctgtgcgca cctgttaggg 240
cacgcgatta aacaactttg gccgcatacc aaaatggcaa tcggcccggt tattgacaac 300
ggtttttatt acgacgttga tcttgaccgc acgttaaccc aggaagatgt cgaagcactc 360
gagaagcgga tgcatgagct tgctgagaaa aactacgacg tcattaagaa gaaagtcagc 420
tggcacgaag cgcgtgaaac tttcgccaac cgtggggaga gctacaaagt ctccattctt 480
gacgaaaaca tcgcccatga tgacaagcca ggtctgtact tccatgaaga atatgtcgat 540
atgtgccgcg gtccgcacgt accgaacatg cgtttctgcc atcatttcaa actaatgaaa 600
acggcagggg cttactggcg tggcgacagc aacaacaaaa tgttgcaacg tatttacggt 660
acggcgtggg cagacaaaaa agcacttaac gcttacctgc agcgcctgga agaagccgcg 720
aaacgcgacc accgtaaaat cggtaaacag ctcgacctgt accatatgca ggaagaagcg 780
ccgggtatgg tattctggca caacgacggc tggaccatct tccgtgaact ggaagtgttt 840
gttcgttcta aactgaaaga gtaccagtat caggaagtta aaggtccgtt catgatggac 900
cgtgtcctgt gggaaaaaac cggtcactgg gacaactaca aagatgcaat gttcaccaca 960
tcttctgaga accgtgaata ctgcattaag ccgatgaact gcccgggtca cgtacaaatt 1020
ttcaaccagg ggctgaagtc ttatcgcgat ctgccgctgc gtatggccga gtttggtagc 1080
tgccaccgta acgagccgtc aggttcgctg catggcctga tgcgcgtgcg tggatttacc 1140
caggatgacg cgcatatctt ctgtactgaa gaacaaattc gcgatgaagt taacggatgt 1200
atccgtttag tctatgatat gtacagcact tttggcttcg agaagatcgt cgtcaaactc 1260
tccactcgtc ctgaaaaacg tattggcagc gacgaaatgt gggatcgtgc tgaggcggac 1320
ctggcggttg cgctggaaga aaacaacatc ccgtttgaat atcaactggg tgaaggcgct 1380
ttctacggtc cgaaaattga atttaccctg tatgactgcc tcgatcgtgc atggcagtgc 1440
ggtacagtac agctggactt ctctttgccg tctcgtctga gcgcttctta tgtaggcgaa 1500
gacaatgaac gtaaagtacc ggtaatgatt caccgcgcaa ttctggggtc gatggaacgt 1560
ttcatcggta tcctgaccga agagttcgct ggtttcttcc cgacctggct tgcgccggtt 1620
caggttgtta tcatgaatat taccgattca cagtctgaat acgttaacga attgacgcaa 1680
aaactatcaa atgcgggcat tcgtgttaaa gcagacttga gaaatgagaa gattggcttt 1740

aaaatccgcg agcacacttt gcgtcgcgtc ccatatatgc tggtctgtgg tgataaagag 1800
gtggaatcag gcaaagttgc cgttcgcacc cgccgtggta aagacctggg aagcatggac 1860
gtaaatgaag tgatcgagaa gctgcaacaa gagattcgca gccgcagtct taaacaattg 1920
gaggaataa 1929

<210> 238
<211> 1353
<212> DNA
<213> E. Coli

<400> 238
atgactaaac actatgatta catcgccatc ggcggcggca gcggcggtat cgcctccatc 60
aaccgcgcgg ctatgtacgg ccagaaatgt gcgctgattg aagccaaaga gctgggcggc 120
acctgcgtaa atgttggctg tgtgccgaaa aaagtgatgt ggcacgcggc gcaaatccgt 180
gaagcgatcc atatgtacgg cccggattat ggttttgata ccactatcaa taaattcaac 240
tgggaaacgt tgatcgccag ccgtaccgcc tatatcgacc gtattcatac ttcctatgaa 300
aacgtgctcg gtaaaaataa cgttgatgta atcaaaggct ttgcccgctt cgttgatgcc 360
aaaacgctgg aggtaaacgg cgaaaccatc acggccgatc atattctgat cgccacaggc 420
ggtcgtccga gccacccgga tattccgggc gtggaatacg gtattgattc tgatggcttc 480
ttcgcccttc ctgctttgcc agagcgcgtg gcggttgttg gcgcgggtta catcgccgtt 540
gagctggcgg gcgtgattaa cggcctcggc gcgaaaacgc atctgtttgt gcgtaaacat 600
gcgccgctgc gcagcttcga cccgatgatt tccgaaacgc tggtcgaagt gatgaacgcc 660
gaaggcccgc agctgcacac caacgccatc ccgaaagcgg tagtgaaaaa taccgatggt 720
agcctgacgc tggagctgga agatggtcgc agtgaaacgg tggattgcct gatttgggcg 780
attggtcgcg agcctgccaa tgacaacatc aacctggaag ccgctggcgt taaaactaac 840
gaaaaaggct atatcgtcgt cgataaatat caaaacacca atattgaagg tatttacgcg 900
gtgggcgata acacgggtgc agtggagctg acaccggtgg cagttgcagc gggtcgccgt 960
ctctctgaac gcctgtttaa taacaagccg gatgagcatc tggattacag caacattccg 1020
accgtggtct tcagccatcc gccgattggt actgttggtt taacggaacc gcaggcgcgc 1080
gagcagtatg gcgacgatca ggtgaaagtg tataaatcct ctttcaccgc gatgtatacc 1140
gccgtcacca ctcaccgcca gccgtgccgc atgaagctgg tgtgcgttgg atcggaagag 1200
aagattgtcg gtattcacgg cattggcttt ggtatggacg aaatgttgca gggcttcgcg 1260
gtggcgctga agatgggggc aaccaaaaaa gacttcgaca ataccgtcgc cattcaccca 1320
acggcggcag aagagttcgt gacaatgcgt taa 1353

<210> 239
<211> 2904
<212> DNA
<213> E. Coli

<400> 239
aaggttaagc ctcacggttc attagtaccg gttagctcaa cgcatcgctg cgcttacaca 60
cccggcctat caacgtcgtc gtcttcaacg ttccttcagg acccttaaag ggtcagggag 120
aactcatctc ggggcaagtt tcgtgcttag atgctttcag cacttatctc ttccgcattt 180
agctaccggg cagtgccatt ggcatgacaa cccgaacacc agtgatgcgt ccactccggt 240
cctctcgtac taggagcagc ccccctcagt tctccagcgc ccacggcaga tagggaccga 300
actgtctcac gacgttctaa acccagctcg cgtaccactt taaatggcga acagccatac 360
ccttgggacc tacttcagcc ccaggatgtg atgagccgac atcgaggtgc caaacaccgc 420
cgtcgatatg aactcttggg cggtatcagc ctgttatccc cggagtacct tttatccgtt 480
gagcgatggc ccttccattc agaaccaccg gatcactatg acctgctttc gcacctgctc 540
gcgccgtcac gctcgcagtc aagctggctt atgccattgc actaacctcc tgatgtccga 600
ccaggattag ccaaccttcg tgctcctccg ttactcttta ggaggagacc gccccagtca 660
aactacccac cagacactgt ccgcaacccg gattacgggt caacgttaga acatcaaaca 720
ttaaagggtg gtatttcaag gtcggctcca tgcagactgg cgtccacact tcaaagcctc 780
ccacctatcc tacacatcaa ggctcaatgt tcagtgtcaa gctatagtaa aggttcacgg 840
ggtctttccg tcttgccgcg ggtacactgc atcttcacag cgagttcaat ttcactgagt 900
ctcgggtgga gacagcctgg ccatcattac gccattcgtg caggtcggaa cttacccgac 960
aaggaatttc gctaccttag gaccgttata gttacggccg ccgtttaccg gggcttcgat 1020
caagagcttc gcttgcgcta accccatcaa ttaaccttcc ggcaccgggc aggcgtcaca 1080
ccgtatacgt ccactttcgt gtttgcacag tgctgtgttt ttaataaaca gttgcagcca 1140
gctggtatct tcgactgatt tcagctccat ccgcgaggga cctcacctac atatcagcgt 1200

gccttctccc gaagttacgg caccattttg cctagttcct tcacccgagt tctctcaagc 1260
gccttggtat tctctacctg accacctgtg tcggtttggg gtacgatttg atgttacctg 1320
atgcttagag gcttttcctg gaagcagggc atttgttgct tcagcaccgt agtgcctcgt 1380
catcacgcct cagccttgat tttccggatt tgcctggaaa accagcctac acgcttaaac 1440
cgggacaacc gtcgcccggc caacatagcc ttctccgtcc ccccttcgca gtaacaccaa 1500
gtacaggaat attaacctgt ttcccatcga ctacgccttt cggcctcgcc ttaggggtcg 1560
actcaccctg ccccgattaa cgttggacag gaacccttgg tcttccggcg agcgggcttt 1620
tcacccgctt tatcgttact tatgtcagca ttcgcacttc tgatacctcc agcatgcctc 1680
acagcacacc ttcgcaggct tacagaacgc tcccctaccc aacaacgcat aagcgtcgct 1740
gccgcagctt cggtgcatgg tttagccccg ttacatcttc cgcgcaggcc gactcgacca 1800
gtgagctatt acgctttctt taaatgatgg ctgcttctaa gccaacatcc tggctgtctg 1860
ggccttccca catcgtttcc cacttaacca tgactttggg accttagctg gcggtctggg 1920
ttgtttccct cttcacgacg gacgttagca cccgccgtgt gtctcccgtg ataacattct 1980
ccggtattcg cagtttgcat cgggttggta agtcgggatg acccccttgc cgaaacagtg 2040
ctctaccccc ggagatgaat tcacgaggcg ctacctaaat agctttcggg gagaaccagc 2100
tatctcccgg tttgattggc ctttcacccc cagccacaag tcatccgcta atttttcaac 2160
attagtcggt tcggtcctcc agttagtgtt acccaacctt caacctgccc atggctagat 2220
caccgggttt cgggtctata ccctgcaact taacgcccag ttaagactcg gtttcccttc 2280
ggctccccta ttcggttaac cttgctacag aatataagtc gctgacccat tatacaaaag 2340
gtacgcagtc acacgcctaa gcgtgctccc actgcttgta cgtacacggt ttcaggttct 2400
ttttcactcc cctcgccggg gttcttttcg cctttccctc acggtactgg ttcactatcg 2460
gtcagtcagg agtatttagc cttggaggat ggtcccccca tattcagaca ggataccacg 2520
tgtcccgccc tactcatcga gctcacagca tgtgcatttt tgtgtacggg gctgtcaccc 2580
tgtatcgcgc gcctttccag acgcttccac taacacacac actgattcag gctctgggct 2640
gctccccgtt cgctcgccgc tactggggga atctcggttg atttcttttc ctcggggtac 2700
ttagatgttt cagttccccc ggttcgcctc attaacctat ggattcagtt aatgatagtg 2760
tgtcgaaaca cactgggttt ccccattcgg aaatcgccgg ttataacggt tcatatcacc 2820
ttaccgacgc ttatcgcaga ttagcacgtc cttcatcgcc tctgactgcc agggcatcca 2880
ccgtgtacgc ttagtcgctt aacc 2904

<210> 240
<211> 120
<212> DNA
<213> E. Coli

<400> 240
atgcctggca gttccctact ctcgcatggg gagaccccac actaccatcg gcgctacggc 60
gtttcacttc tgagttcggc atggggtcag gtgggaccac cgcgctacgg ccgccaggca 120

<210> 241
<211> 76
<212> DNA
<213> E. Coli

<400> 241
gtccccttcg tctagaggcc caggacaccg ccctttcacg gcggtaacag gggttcgaat 60
cccctagggg acgcca 76

<210> 242
<211> 1549
<212> DNA
<213> E. Coli

<400> 242
aaattgaaga gtttgatcat ggctcagatt gaacgctggc ggcaggccta acacatgcaa 60
gtcgaacggt aacaggaagc agcttgctgc ttcgctgacg agtggcggac gggtgagtaa 120
tgtctgggaa gctgcctgat ggagggggat aactactgga aacggtagct aataccgcat 180
aatgtcgcaa gaccaaagag ggggaccttc gggcctcttg ccatcggatg tgcccagatg 240
ggattagctt gttggtgggg taacggctca ccaaggcgac gatccctagc tggtctgaga 300
ggatgaccag ccacactgga actgagacac ggtccagact cctacgggag gcagcagtgg 360
ggaatattgc acaatgggcg caagcctgat gcagccatgc cgcgtgtatg aagaaggcct 420

tcgggttgta aagtactttc agcggggagg aagggagtaa agttaatacc tttgctcatt 480
gacgttaccc gcagaagaag caccggctaa ctccgtgcca gcagccgcgg taatacggag 540
ggtgcaagcg ttaatcggaa ttactgggcg taaagcgcac gcaggcgggt tggttaagtc 600
agatgtgaaa tccccgggct caacctggga actgcatctg atactggcaa gcttgagtct 660
cgtagagggg ggtagaattc caggtgtagc ggtgaaatgc gtagagatct ggaggaatac 720
cggtggcgaa ggcggccccc tggacgaaga ctgacgctca ggtgcgaaag cgtggggagc 780
aaacaggatt agataccctg gtagtccacg ccgtaaacga tgtcgacttg gaggttgtgc 840
ccttgaggcg tggcttccgg agctaacgcg ttaagtcgac cgcctgggga gtacggccgc 900
aaggttaaaa ctcaaatgaa ttgacggggg cccgcacaag cggtggagca tgtggtttaa 960
ttcgatgcaa cgcgaagaac cttacctggt cttgacatcc acggaagttt tcagagatga 1020
gaatgtgcct tcgggaaccg tgagacaggt gctgcatggc tgtcgtcagc tcgtgttgtg 1080
aaatgttggg ttaagtcccg caacgagcgc aacccttatc ctttgttgcc agcggtccgg 1140
ccgggaactc aaaggagact gccagtgata aactggagga aggtggggat gacgtcaagt 1200
catcatggcc cttacgacca gggctacaca cgtgctacaa tggcgcatac aaagagaagc 1260
gacctcgcga gagcaagcgg acctcataaa gtgcgtcgta gtccggattg gagtctgcaa 1320
ctcgactcca tgaagtcgga atcgctagta atcgtggatc agaatgccac ggtgaatacg 1380
ttcccgggcc ttgtacacac cgcccgtcac accatgggag tgggttgcaa aagaagtagg 1440
tagcttaacc ttcgggaggg cgcttaccac tttgtgattc atgactgggg tgaagtcgta 1500
acaaggtaac cgtaggggaa cctgcggttg gatcacctcc ttaccttaa 1549

<210> 243
<211> 221
<212> PRT
<213> E. Coli

<400> 243
Met Asn Val Phe Ser Gln Thr Gln Arg Tyr Lys Ala Leu Phe Trp Leu
1               5                   10                  15
Ser Leu Phe His Leu Leu Val Ile Thr Ser Ser Asn Tyr Leu Val Gln
            20                  25                  30
Leu Pro Val Ser Ile Leu Gly Phe His Thr Thr Trp Gly Ala Phe Ser
        35                  40                  45
Phe Pro Phe Ile Phe Leu Ala Thr Asp Leu Thr Val Arg Ile Phe Gly
    50                  55                  60
Ala Pro Leu Ala Arg Arg Ile Ile Phe Ala Val Met Ile Pro Ala Leu
65                  70                  75                  80
Leu Ile Ser Tyr Val Ile Ser Ser Leu Phe Tyr Met Gly Ser Trp Gln
                85                  90                  95
Gly Phe Gly Ala Leu Ala His Phe Asn Leu Phe Val Ala Arg Ile Ala
            100                 105                 110
Thr Ala Ser Phe Met Ala Tyr Ala Leu Gly Gln Ile Leu Asp Val His
        115                 120                 125
Val Phe Asn Arg Leu Arg Gln Ser Arg Arg Trp Trp Leu Ala Pro Thr
    130                 135                 140
Ala Ser Thr Leu Phe Gly Asn Val Ser Asp Thr Leu Ala Phe Phe Phe
145                 150                 155                 160
Ile Ala Phe Trp Arg Ser Pro Asp Ala Phe Met Ala Glu His Trp Met
                165                 170                 175
Glu Ile Ala Leu Val Asp Tyr Cys Phe Lys Val Leu Ile Ser Ile Val
            180                 185                 190
Phe Phe Leu Pro Met Tyr Gly Val Leu Leu Asn Met Leu Leu Lys Arg
        195                 200                 205
Leu Ala Asp Lys Ser Glu Ile Asn Ala Leu Gln Ala Ser
    210                 215                 220

<210> 244
<211> 203
<212> PRT
<213> E. Coli

<400> 244
Met Ile Arg Trp Met Asn Glu Pro Leu Trp Pro Phe Ile Glu Arg Lys
1               5                   10                  15
Lys Ser Met Arg Asn Leu Val Lys Tyr Val Gly Ile Gly Leu Leu Val
            20                  25                  30
Met Gly Leu Ala Ala Cys Asp Asp Lys Asp Thr Asn Ala Thr Ala Gln
        35                  40                  45
Gly Ser Val Ala Glu Ser Asn Ala Thr Gly Asn Pro Val Asn Leu Leu
    50                  55                  60
Asp Gly Lys Leu Ser Phe Ser Leu Pro Ala Asp Met Thr Asp Gln Ser
65                  70                  75                  80
Gly Lys Leu Gly Thr Gln Ala Asn Asn Met His Val Trp Ser Asp Ala
                85                  90                  95
Thr Gly Gln Lys Ala Val Ile Val Ile Met Gly Asp Asp Pro Lys Glu
            100                 105                 110
Asp Leu Ala Val Leu Ala Lys Arg Leu Glu Asp Gln Gln Arg Ser Arg
        115                 120                 125
Asp Pro Gln Leu Gln Val Val Thr Asn Lys Ala Ile Glu Leu Lys Gly
    130                 135                 140
His Lys Met Gln Gln Leu Asp Ser Ile Ile Ser Ala Lys Gly Gln Thr
145                 150                 155                 160
Ala Tyr Ser Ser Val Ile Leu Gly Asn Val Gly Asn Gln Leu Leu Thr
                165                 170                 175
Met Gln Ile Thr Leu Pro Ala Asp Asp Gln Gln Lys Ala Gln Thr Thr
            180                 185                 190
Ala Glu Asn Ile Ile Asn Thr Leu Val Ile Gln
        195                 200

<210> 245
<211> 324
<212> PRT
<213> E. Coli

<400> 245
Met Ala Asn Met Phe Ala Leu Ile Leu Val Ile Ala Thr Leu Val Thr
1               5                   10                  15
Gly Ile Leu Trp Cys Val Asp Lys Phe Phe Phe Ala Pro Lys Arg Arg
            20                  25                  30
Glu Arg Gln Ala Ala Ala Gln Ala Ala Ala Gly Asp Ser Leu Asp Lys
        35                  40                  45
Ala Thr Leu Lys Lys Val Ala Pro Lys Pro Gly Trp Leu Glu Thr Gly
    50                  55                  60
Ala Ser Val Phe Pro Val Leu Ala Ile Val Leu Ile Val Arg Ser Phe
65                  70                  75                  80
Ile Tyr Glu Pro Phe Gln Ile Pro Ser Gly Ser Met Met Pro Thr Leu
                85                  90                  95
Leu Ile Gly Asp Phe Ile Leu Val Glu Lys Phe Ala Tyr Gly Ile Lys
            100                 105                 110
Asp Pro Ile Tyr Gln Lys Thr Leu Ile Glu Thr Gly His Pro Lys Arg
        115                 120                 125
Gly Asp Ile Val Val Phe Lys Tyr Pro Glu Asp Pro Lys Leu Asp Tyr
    130                 135                 140
Ile Lys Arg Ala Val Gly Leu Pro Gly Asp Lys Val Thr Tyr Asp Pro
145                 150                 155                 160
Val Ser Lys Glu Leu Thr Ile Gln Pro Gly Cys Ser Ser Gly Gln Ala
                165                 170                 175
Cys Glu Asn Ala Leu Pro Val Thr Tyr Ser Asn Val Glu Pro Ser Asp
            180                 185                 190
Phe Val Gln Thr Phe Ser Arg Arg Asn Gly Gly Glu Ala Thr Ser Gly

195                 200                 205
Phe Phe Glu Val Pro Lys Asn Glu Thr Lys Glu Asn Gly Ile Arg Leu
    210                 215                 220
Ser Glu Arg Lys Glu Thr Leu Gly Asp Val Thr His Arg Ile Leu Thr
225                 230                 235                 240
Val Pro Ile Ala Gln Asp Gln Val Gly Met Tyr Tyr Gln Gln Pro Gly
                245                 250                 255
Gln Gln Leu Ala Thr Trp Ile Val Pro Pro Gly Gln Tyr Phe Met Met
            260                 265                 270
Gly Asp Asn Arg Asp Asn Ser Ala Asp Ser Arg Tyr Trp Gly Phe Val
        275                 280                 285
Pro Glu Ala Asn Leu Val Gly Arg Ala Thr Ala Ile Trp Met Ser Phe
    290                 295                 300
Asp Lys Gln Glu Gly Glu Trp Pro Thr Gly Leu Arg Leu Ser Arg Ile
305                 310                 315                 320
Gly Gly Ile His

<210> 246
<211> 586
<212> PRT
<213> E. Coli

<400> 246
Met Thr Ile Thr Lys Leu Ala Trp Arg Asp Leu Val Pro Asp Thr Asp
1               5                   10                  15
Ser Tyr Gln Glu Ile Phe Ala Gln Pro His Leu Ile Asp Glu Asn Asp
            20                  25                  30
Pro Leu Phe Ser Asp Thr Gln Pro Arg Leu Gln Phe Ala Leu Glu Gln
        35                  40                  45
Leu Leu His Thr Arg Ala Ser Ser Ser Phe Met Leu Ala Lys Ala Pro
    50                  55                  60
Glu Glu Ser Glu Tyr Leu Asn Leu Ile Ala Asn Ala Ala Arg Thr Leu
65                  70                  75                  80
Gln Ser Asp Ala Gly Gln Leu Val Gly Gly His Tyr Glu Val Ser Gly
                85                  90                  95
His Ser Ile Arg Leu Arg His Ala Val Ser Ala Asp Asp Asn Phe Ala
            100                 105                 110
Thr Leu Thr Gln Val Val Ala Ala Asp Trp Val Glu Ala Glu Gln Leu
        115                 120                 125
Phe Gly Cys Leu Arg Gln Phe Asn Gly Asp Ile Thr Leu Gln Pro Gly
    130                 135                 140
Leu Val His Gln Ala Asn Gly Gly Ile Leu Ile Ile Ser Leu Arg Thr
145                 150                 155                 160
Leu Leu Ala Gln Pro Leu Leu Trp Met Arg Leu Lys Asn Ile Val Asn
                165                 170                 175
Arg Glu Arg Phe Asp Trp Val Ala Phe Asp Glu Ser Arg Pro Leu Pro
            180                 185                 190
Val Ser Val Pro Ser Met Pro Leu Lys Leu Lys Val Ile Leu Val Gly
        195                 200                 205
Glu Arg Glu Ser Leu Ala Asp Phe Gln Glu Met Glu Pro Glu Leu Ser
    210                 215                 220
Glu Gln Ala Ile Tyr Ser Glu Phe Glu Asp Thr Leu Gln Ile Val Asp
225                 230                 235                 240
Ala Glu Ser Val Thr Gln Trp Cys Arg Trp Val Thr Phe Thr Ala Arg
                245                 250                 255
His Asn His Leu Pro Ala Pro Gly Ala Asp Ala Trp Pro Ile Leu Ile
            260                 265                 270
Arg Glu Ala Ala Arg Tyr Thr Gly Glu Gln Glu Thr Leu Pro Leu Ser

275                 280                 285
Pro Gln Trp Ile Leu Arg Gln Cys Lys Glu Val Ala Ser Leu Cys Asp
    290                 295                 300
Gly Asp Thr Phe Ser Gly Glu Gln Leu Asn Leu Met Leu Gln Gln Arg
305                 310                 315                 320
Glu Trp Arg Glu Gly Phe Leu Ala Glu Arg Met Gln Asp Glu Ile Leu
                325                 330                 335
Gln Glu Gln Ile Leu Ile Glu Thr Glu Gly Glu Arg Ile Gly Gln Ile
            340                 345                 350
Asn Ala Leu Ser Val Ile Glu Phe Pro Gly His Pro Arg Ala Phe Gly
        355                 360                 365
Glu Pro Ser Arg Ile Ser Cys Val Val His Ile Gly Asp Gly Glu Phe
    370                 375                 380
Thr Asp Ile Glu Arg Lys Ala Glu Leu Gly Gly Asn Ile His Ala Lys
385                 390                 395                 400
Gly Met Met Ile Met Gln Ala Phe Leu Met Ser Glu Leu Gln Leu Glu
                405                 410                 415
Gln Gln Ile Pro Phe Ser Ala Ser Leu Thr Phe Glu Gln Ser Tyr Ser
            420                 425                 430
Glu Val Asp Gly Asp Ser Ala Ser Met Ala Glu Leu Cys Ala Leu Ile
        435                 440                 445
Ser Ala Leu Ala Asp Val Pro Val Asn Gln Ser Ile Ala Ile Thr Gly
    450                 455                 460
Ser Val Asp Gln Phe Gly Arg Ala Gln Pro Val Gly Gly Leu Asn Glu
465                 470                 475                 480
Lys Ile Glu Gly Phe Phe Ala Ile Cys Gln Gln Arg Glu Leu Thr Gly
                485                 490                 495
Lys Gln Gly Val Ile Ile Pro Thr Ala Asn Val Arg His Leu Ser Leu
            500                 505                 510
His Ser Glu Leu Val Lys Ala Val Glu Glu Gly Lys Phe Thr Ile Trp
        515                 520                 525
Ala Val Asp Asp Val Thr Asp Ala Leu Pro Leu Leu Leu Asn Leu Val
    530                 535                 540
Trp Asp Gly Glu Gly Gln Thr Thr Leu Met Gln Thr Ile Gln Glu Arg
545                 550                 555                 560
Ile Ala Gln Ala Ser Gln Gln Glu Gly Arg His Arg Phe Pro Trp Pro
                565                 570                 575
Leu Arg Trp Leu Asn Trp Phe Ile Pro Asn
            580                 585

<210> 247
<211> 394
<212> PRT
<213> E. Coli

<400> 247

<210> 248
<211> 704
<212> PRT
<213> E. Coli

<400> 248
Met Ala Arg Thr Thr Pro Ile Ala Arg Tyr Arg Asn Ile Gly Ile Ser
 1               5                  10                  15
Ala His Ile Asp Ala Gly Lys Thr Thr Thr Thr Glu Arg Ile Leu Phe
            20                  25                  30
Tyr Thr Gly Val Asn His Lys Ile Gly Glu Val His Asp Gly Ala Ala
        35                  40                  45
Thr Met Asp Trp Met Glu Gln Glu Gln Glu Arg Gly Ile Thr Ile Thr
    50                  55                  60
Ser Ala Ala Thr Thr Ala Phe Trp Ser Gly Met Ala Lys Gln Tyr Glu
65                  70                  75                  80
Pro His Arg Ile Asn Ile Ile Asp Thr Pro Gly His Val Asp Phe Thr
                85                  90                  95
Ile Glu Val Glu Arg Ser Met Arg Val Leu Asp Gly Ala Val Met Val
            100                 105                 110
Tyr Cys Ala Val Gly Gly Val Gln Pro Gln Ser Glu Thr Val Trp Arg
        115                 120                 125

Gln Ala Asn Lys Tyr Lys Val Pro Arg Ile Ala Phe Val Asn Lys Met
        130                 135                 140
Asp Arg Met Gly Ala Asn Phe Leu Lys Val Val Asn Gln Ile Lys Thr
145                 150                 155                 160
Arg Leu Gly Ala Asn Pro Val Pro Leu Gln Leu Ala Ile Gly Ala Glu
                165                 170                 175
Glu His Phe Thr Gly Val Val Asp Leu Val Lys Met Lys Ala Ile Asn
            180                 185                 190
Trp Asn Asp Ala Asp Gln Gly Val Thr Phe Glu Tyr Glu Asp Ile Pro
        195                 200                 205
Ala Asp Met Val Glu Leu Ala Asn Glu Trp His Gln Asn Leu Ile Glu
    210                 215                 220
Ser Ala Ala Glu Ala Ser Glu Glu Leu Met Glu Lys Tyr Leu Gly Gly
225                 230                 235                 240
Glu Glu Leu Thr Glu Ala Glu Ile Lys Gly Ala Leu Arg Gln Arg Val
                245                 250                 255
Leu Asn Asn Glu Ile Ile Leu Val Thr Cys Gly Ser Ala Phe Lys Asn
            260                 265                 270
Lys Gly Val Gln Ala Met Leu Asp Ala Val Ile Asp Tyr Leu Pro Ser
        275                 280                 285
Pro Val Asp Val Pro Ala Ile Asn Gly Ile Leu Asp Asp Gly Lys Asp
    290                 295                 300
Thr Pro Ala Glu Arg His Ala Ser Asp Asp Glu Pro Phe Ser Ala Leu
305                 310                 315                 320
Ala Phe Lys Ile Ala Thr Asp Pro Phe Val Gly Asn Leu Thr Phe Phe
                325                 330                 335
Arg Val Tyr Ser Gly Val Val Asn Ser Gly Asp Thr Val Leu Asn Ser
            340                 345                 350
Val Lys Ala Ala Arg Glu Arg Phe Gly Arg Ile Val Gln Met His Ala
        355                 360                 365
Asn Lys Arg Glu Glu Ile Lys Glu Val Arg Ala Gly Asp Ile Ala Ala
    370                 375                 380
Ala Ile Gly Leu Lys Asp Val Thr Thr Gly Asp Thr Leu Cys Asp Pro
385                 390                 395                 400
Asp Ala Pro Ile Ile Leu Glu Arg Met Glu Phe Pro Glu Pro Val Ile
                405                 410                 415
Ser Ile Ala Val Glu Pro Lys Thr Lys Ala Asp Gln Glu Lys Met Gly
            420                 425                 430
Leu Ala Leu Gly Arg Leu Ala Lys Glu Asp Pro Ser Phe Arg Val Trp
        435                 440                 445
Thr Asp Glu Glu Ser Asn Gln Thr Ile Ile Ala Gly Met Gly Glu Leu
    450                 455                 460
His Leu Asp Ile Ile Val Asp Arg Met Lys Arg Glu Phe Asn Val Glu
465                 470                 475                 480
Ala Asn Val Gly Lys Pro Gln Val Ala Tyr Arg Glu Thr Ile Arg Gln
                485                 490                 495
Lys Val Thr Asp Val Glu Gly Lys His Ala Lys Gln Ser Gly Gly Arg
            500                 505                 510
Gly Gln Tyr Gly His Val Val Ile Asp Met Tyr Pro Leu Glu Pro Gly
        515                 520                 525
Ser Asn Pro Lys Gly Tyr Glu Phe Ile Asn Asp Ile Lys Gly Gly Val
    530                 535                 540
Ile Pro Gly Glu Tyr Ile Pro Ala Val Asp Lys Gly Ile Gln Glu Gln
545                 550                 555                 560
Leu Lys Ala Gly Pro Leu Ala Gly Tyr Pro Val Val Asp Met Gly Ile
                565                 570                 575
Arg Leu His Phe Gly Ser Tyr His Asp Val Asp Ser Ser Glu Leu Ala
            580                 585                 590
Phe Lys Leu Ala Ala Ser Ile Ala Phe Lys Glu Gly Phe Lys Lys Ala
        595                 600                 605
Lys Pro Val Leu Leu Glu Pro Ile Met Lys Val Glu Val Glu Thr Pro

610                 615                 620
Glu Glu Asn Thr Gly Asp Val Ile Gly Asp Leu Ser Arg Arg Arg Gly
625                 630                 635                 640
Met Leu Lys Gly Gln Glu Ser Glu Val Thr Gly Val Lys Ile His Ala
                645                 650                 655
Glu Val Pro Leu Ser Glu Met Phe Gly Tyr Ala Thr Gln Leu Arg Ser
            660                 665                 670
Leu Thr Lys Gly Arg Ala Ser Tyr Thr Met Glu Phe Leu Lys Tyr Asp
        675                 680                 685
Glu Ala Pro Ser Asn Val Ala Gln Ala Val Ile Glu Ala Arg Gly Lys
    690                 695                 700

<210> 249
<211> 179
<212> PRT
<213> E. Coli

<400> 249
Met Pro Arg Arg Arg Val Ile Gly Gln Arg Lys Ile Leu Pro Asp Pro
1               5                   10                  15
Lys Phe Gly Ser Glu Leu Leu Ala Lys Phe Val Asn Ile Leu Met Val
            20                  25                  30
Asp Gly Lys Lys Ser Thr Ala Glu Ser Ile Val Tyr Ser Ala Leu Glu
        35                  40                  45
Thr Leu Ala Gln Arg Ser Gly Lys Ser Glu Leu Glu Ala Phe Glu Val
    50                  55                  60
Ala Leu Glu Asn Val Arg Pro Thr Val Glu Val Lys Ser Arg Arg Val
65                  70                  75                  80
Gly Gly Ser Thr Tyr Gln Val Pro Val Glu Val Arg Pro Val Arg Arg
                85                  90                  95
Asn Ala Leu Ala Met Arg Trp Ile Val Glu Ala Ala Arg Lys Arg Gly
            100                 105                 110
Asp Lys Ser Met Ala Leu Arg Leu Ala Asn Glu Leu Ser Asp Ala Ala
        115                 120                 125
Glu Asn Lys Gly Thr Ala Val Lys Lys Arg Glu Asp Val His Arg Met
    130                 135                 140
Ala Glu Ala Asn Lys Ala Phe Ala His Tyr Arg Trp Leu Ser Leu Arg
145                 150                 155                 160
Ser Phe Ser His Gln Ala Gly Ala Ser Ser Lys Gln Pro Ala Leu Gly
                165                 170                 175
Tyr Leu Asn

<210> 250
<211> 124
<212> PRT
<213> E. Coli

<400> 250
Met Ala Thr Val Asn Gln Leu Val Arg Lys Pro Arg Ala Arg Lys Val
1               5                   10                  15
Ala Lys Ser Asn Val Pro Ala Leu Glu Ala Cys Pro Gln Lys Arg Gly
            20                  25                  30
Val Cys Thr Arg Val Tyr Thr Thr Thr Pro Lys Lys Pro Asn Ser Ala
        35                  40                  45
Leu Arg Lys Val Cys Arg Val Arg Leu Thr Asn Gly Phe Glu Val Thr
    50                  55                  60
Ser Tyr Ile Gly Gly Glu Gly His Asn Leu Gln Glu His Ser Val Ile
65                  70                  75                  80

Leu Ile Arg Gly Gly Arg Val Lys Asp Leu Pro Gly Val Arg Tyr His
                85                  90                  95
Thr Val Arg Gly Ala Leu Asp Cys Ser Gly Val Lys Asp Arg Lys Gln
            100                 105                 110
Ala Arg Ser Lys Tyr Gly Val Lys Arg Pro Lys Ala
        115                 120

<210> 251
<211> 165
<212> PRT
<213> E. Coli

<400> 251
Met Ala Leu Asn Leu Gln Asp Lys Gln Ala Ile Val Ala Glu Val Ser
 1               5                  10                  15
Glu Val Ala Lys Gly Ala Leu Ser Ala Val Val Ala Asp Ser Arg Gly
            20                  25                  30
Val Thr Val Asp Lys Met Thr Glu Leu Arg Lys Ala Gly Arg Glu Ala
        35                  40                  45
Gly Val Tyr Met Arg Val Val Arg Asn Thr Leu Leu Arg Arg Ala Val
    50                  55                  60
Glu Gly Thr Pro Phe Glu Cys Leu Lys Asp Ala Phe Val Gly Pro Thr
65                  70                  75                  80
Leu Ile Ala Tyr Ser Met Glu His Pro Gly Ala Ala Ala Arg Leu Phe
                85                  90                  95
Lys Glu Phe Ala Lys Ala Asn Ala Lys Phe Glu Val Lys Ala Ala Ala
            100                 105                 110
Phe Glu Gly Glu Leu Ile Pro Ala Ser Gln Ile Asp Arg Leu Ala Thr
        115                 120                 125
Leu Pro Thr Tyr Glu Glu Ala Ile Ala Arg Leu Met Ala Thr Met Lys
    130                 135                 140
Glu Ala Ser Ala Gly Lys Leu Val Arg Thr Leu Ala Ala Val Arg Asp
145                 150                 155                 160
Ala Lys Glu Ala Ala
                165

<210> 252
<211> 121
<212> PRT
<213> E. Coli

<400> 252
Met Ser Ile Thr Lys Asp Gln Ile Ile Glu Ala Val Ala Ala Met Ser
 1               5                  10                  15
Val Met Asp Val Val Glu Leu Ile Ser Ala Met Glu Glu Lys Phe Gly
            20                  25                  30
Val Ser Ala Ala Ala Ala Val Ala Val Ala Ala Gly Pro Val Glu Ala
        35                  40                  45
Ala Glu Glu Lys Thr Glu Phe Asp Val Ile Leu Lys Ala Ala Gly Ala
    50                  55                  60
Asn Lys Val Ala Val Ile Lys Ala Val Arg Gly Ala Thr Gly Leu Gly
65                  70                  75                  80
Leu Lys Glu Ala Lys Asp Leu Val Glu Ser Ala Pro Ala Ala Leu Lys
                85                  90                  95
Glu Gly Val Ser Lys Asp Asp Ala Glu Ala Leu Lys Lys Ala Leu Glu
            100                 105                 110
Glu Ala Gly Ala Glu Val Glu Val Lys
        115                 120

<210> 253
<211> 714
<212> PRT
<213> E. Coli

<400> 253
Met Ser Arg Ile Ile Met Leu Ile Pro Thr Gly Thr Ser Val Gly Leu
1               5                   10                  15
Thr Ser Val Ser Leu Gly Val Ile Arg Ala Met Glu Arg Lys Gly Val
            20                  25                  30
Arg Leu Ser Val Phe Lys Pro Ile Ala Gln Pro Arg Thr Gly Gly Asp
        35                  40                  45
Ala Pro Asp Gln Thr Thr Thr Ile Val Arg Ala Asn Ser Ser Thr Thr
    50                  55                  60
Thr Ala Ala Glu Pro Leu Lys Met Ser Tyr Val Glu Gly Leu Leu Ser
65                  70                  75                  80
Ser Asn Gln Lys Asp Val Leu Met Glu Glu Ile Val Ala Asn Tyr His
                85                  90                  95
Ala Asn Thr Lys Asp Ala Glu Val Val Leu Val Glu Gly Leu Val Pro
            100                 105                 110
Thr Arg Lys His Gln Phe Ala Gln Ser Leu Asn Tyr Glu Ile Ala Lys
        115                 120                 125
Thr Leu Asn Ala Glu Ile Val Phe Val Met Ser Gln Gly Thr Asp Thr
    130                 135                 140
Pro Glu Gln Leu Lys Glu Arg Ile Glu Leu Thr Arg Asn Ser Phe Gly
145                 150                 155                 160
Gly Ala Lys Asn Thr Asn Ile Thr Gly Val Ile Val Asn Lys Leu Asn
                165                 170                 175
Ala Pro Val Asp Glu Gln Gly Arg Thr Arg Pro Asp Leu Ser Glu Ile
            180                 185                 190
Phe Asp Asp Ser Ser Lys Ala Lys Val Asn Asn Val Asp Pro Ala Lys
        195                 200                 205
Leu Gln Glu Ser Ser Pro Leu Pro Val Leu Gly Ala Val Pro Trp Ser
    210                 215                 220
Phe Asp Leu Ile Ala Thr Arg Ala Ile Asp Met Ala Arg His Leu Asn
225                 230                 235                 240
Ala Thr Ile Ile Asn Glu Gly Asp Ile Asn Thr Arg Arg Val Lys Ser
                245                 250                 255
Val Thr Phe Cys Ala Arg Ser Ile Pro His Met Leu Glu His Phe Arg
            260                 265                 270
Ala Gly Ser Leu Leu Val Thr Ser Ala Asp Arg Pro Asp Val Leu Val
        275                 280                 285
Ala Ala Cys Leu Ala Ala Met Asn Gly Val Glu Ile Gly Ala Leu Leu
    290                 295                 300
Leu Thr Gly Gly Tyr Glu Met Asp Ala Arg Ile Ser Lys Leu Cys Glu
305                 310                 315                 320
Arg Ala Phe Ala Thr Gly Leu Pro Val Phe Met Val Asn Thr Asn Thr
                325                 330                 335
Trp Gln Thr Ser Leu Ser Leu Gln Ser Phe Asn Leu Glu Val Pro Val
            340                 345                 350
Asp Asp His Glu Arg Ile Glu Lys Val Gln Glu Tyr Val Ala Asn Tyr
        355                 360                 365
Ile Asn Ala Asp Trp Ile Glu Ser Leu Thr Ala Thr Ser Glu Arg Ser
    370                 375                 380
Arg Arg Leu Ser Pro Pro Ala Phe Arg Tyr Gln Leu Thr Glu Leu Ala
385                 390                 395                 400
Arg Lys Ala Gly Lys Arg Ile Val Leu Pro Glu Gly Asp Glu Pro Arg
                405                 410                 415
Thr Val Lys Ala Ala Ala Ile Cys Ala Glu Arg Gly Ile Ala Thr Cys
            420                 425                 430

Val Leu Leu Gly Asn Pro Ala Glu Ile Asn Arg Val Ala Ala Ser Gln
        435                 440                 445
Gly Val Glu Leu Gly Ala Gly Ile Glu Ile Val Asp Pro Glu Val Val
    450                 455                 460
Arg Glu Ser Tyr Val Gly Arg Leu Val Glu Leu Arg Lys Asn Lys Gly
465                 470                 475                 480
Met Thr Glu Thr Val Ala Arg Glu Gln Leu Glu Asp Asn Val Val Leu
                485                 490                 495
Gly Thr Leu Met Leu Glu Gln Asp Glu Val Asp Gly Leu Val Ser Gly
            500                 505                 510
Ala Val His Thr Thr Ala Asn Thr Ile Arg Pro Pro Leu Gln Leu Ile
        515                 520                 525
Lys Thr Ala Pro Gly Ser Ser Leu Val Ser Ser Val Phe Phe Met Leu
    530                 535                 540
Leu Pro Glu Gln Val Tyr Val Tyr Gly Asp Cys Ala Ile Asn Pro Asp
545                 550                 555                 560
Pro Thr Ala Glu Gln Leu Ala Glu Ile Ala Ile Gln Ser Ala Asp Ser
                565                 570                 575
Ala Ala Ala Phe Gly Ile Glu Pro Arg Val Ala Met Leu Ser Tyr Ser
            580                 585                 590
Thr Gly Thr Ser Gly Ala Gly Ser Asp Val Glu Lys Val Arg Glu Ala
        595                 600                 605
Thr Arg Leu Ala Gln Glu Lys Arg Pro Asp Leu Met Ile Asp Gly Pro
    610                 615                 620
Leu Gln Tyr Asp Ala Ala Val Met Ala Asp Val Ala Lys Ser Lys Ala
625                 630                 635                 640
Pro Asn Ser Pro Val Ala Gly Arg Ala Thr Val Phe Ile Phe Pro Asp
                645                 650                 655
Leu Asn Thr Gly Asn Thr Thr Tyr Lys Ala Val Gln Arg Ser Ala Asp
            660                 665                 670
Leu Ile Ser Ile Gly Pro Met Leu Gln Gly Met Arg Lys Pro Val Asn
        675                 680                 685
Asp Leu Ser Arg Gly Ala Leu Val Asp Asp Ile Val Tyr Thr Ile Ala
    690                 695                 700
Leu Thr Ala Ile Gln Ser Ala Gln Gln Gln
705                 710

<210> 254
<211> 588
<212> PRT
<213> E. Coli

<400> 254
Met Asn Asn Ser Ile Asn His Lys Phe His His Ile Ser Arg Ala Glu
 1               5                  10                  15
Tyr Gln Glu Leu Leu Ala Val Ser Arg Gly Asp Ala Val Ala Asp Tyr
            20                  25                  30
Ile Ile Asp Asn Val Ser Ile Leu Asp Leu Ile Asn Gly Gly Glu Ile
        35                  40                  45
Ser Gly Pro Ile Val Ile Lys Gly Arg Tyr Ile Ala Gly Val Gly Ala
    50                  55                  60
Glu Tyr Thr Asp Ala Pro Ala Leu Gln Arg Ile Asp Ala Arg Gly Ala
65                  70                  75                  80
Thr Ala Val Pro Gly Phe Ile Asp Ala His Leu His Ile Glu Ser Ser
                85                  90                  95
Met Met Thr Pro Val Thr Phe Glu Thr Ala Thr Leu Pro Arg Gly Leu
            100                 105                 110
Thr Thr Val Ile Cys Asp Pro His Glu Ile Val Asn Val Met Gly Glu
        115                 120                 125
Ala Gly Phe Ala Trp Phe Ala Arg Cys Ala Glu Gln Ala Arg Gln Asn

130                 135                 140
Gln Tyr Leu Gln Val Ser Ser Cys Val Pro Ala Leu Glu Gly Cys Asp
145                 150                 155                 160
Val Asn Gly Ala Ser Phe Thr Leu Glu Gln Met Leu Ala Trp Arg Asp
                165                 170                 175
His Pro Gln Val Thr Gly Leu Ala Glu Met Met Asp Tyr Pro Gly Val
            180                 185                 190
Ile Ser Gly Gln Asn Ala Leu Leu Asp Lys Leu Asp Ala Phe Arg His
        195                 200                 205
Leu Thr Leu Asp Gly His Cys Pro Gly Leu Gly Gly Lys Glu Leu Asn
    210                 215                 220
Ala Tyr Ile Thr Ala Gly Ile Glu Asn Cys His Glu Ser Tyr Gln Leu
225                 230                 235                 240
Glu Glu Gly Arg Arg Lys Leu Gln Leu Gly Met Ser Leu Met Ile Arg
                245                 250                 255
Glu Gly Ser Ala Ala Arg Asn Leu Asn Ala Leu Ala Pro Leu Ile Asn
            260                 265                 270
Glu Phe Asn Ser Pro Gln Cys Met Leu Cys Thr Asp Asp Arg Asn Pro
        275                 280                 285
Trp Glu Ile Ala His Glu Gly His Ile Asp Ala Leu Ile Arg Arg Leu
    290                 295                 300
Ile Glu Gln His Asn Val Pro Leu His Val Ala Tyr Arg Val Ala Ser
305                 310                 315                 320
Trp Ser Thr Ala Arg His Phe Gly Leu Asn His Leu Gly Leu Leu Ala
                325                 330                 335
Pro Gly Lys Gln Ala Asp Ile Val Leu Leu Ser Asp Ala Arg Lys Val
            340                 345                 350
Thr Val Gln Gln Val Leu Val Lys Gly Glu Pro Ile Asp Ala Gln Thr
        355                 360                 365
Leu Gln Ala Glu Glu Ser Ala Arg Leu Ala Gln Ser Ala Pro Pro Tyr
    370                 375                 380
Gly Asn Thr Ile Ala Arg Gln Pro Val Ser Ala Ser Asp Phe Ala Leu
385                 390                 395                 400
Gln Phe Thr Pro Gly Lys Arg Tyr Arg Val Ile Asp Val Ile His Asn
                405                 410                 415
Glu Leu Ile Thr His Ser His Ser Ser Val Tyr Ser Glu Asn Gly Phe
            420                 425                 430
Asp Arg Asp Asp Val Ser Phe Ile Ala Val Leu Glu Arg Tyr Gly Gln
        435                 440                 445
Arg Leu Ala Pro Ala Cys Gly Leu Leu Gly Gly Phe Gly Leu Asn Glu
    450                 455                 460
Gly Ala Leu Ala Ala Thr Val Ser His Asp Ser His Asn Ile Val Val
465                 470                 475                 480
Ile Gly Arg Ser Ala Glu Glu Met Ala Leu Ala Val Asn Gln Val Ile
                485                 490                 495
Gln Asp Gly Gly Gly Leu Cys Val Val Arg Asn Gly Gln Val Gln Ser
            500                 505                 510
His Leu Pro Leu Pro Ile Ala Gly Leu Met Ser Thr Asp Thr Ala Gln
        515                 520                 525
Ser Leu Ala Glu Gln Ile Asp Ala Leu Lys Ala Ala Ala Arg Glu Cys
    530                 535                 540
Gly Pro Leu Pro Asp Glu Pro Phe Ile Gln Met Ala Phe Leu Ser Leu
545                 550                 555                 560
Pro Val Ile Pro Ala Leu Lys Leu Thr Ser Gln Gly Leu Phe Asp Gly
                565                 570                 575
Glu Lys Phe Ala Phe Thr Thr Leu Glu Val Thr Glu
            580                 585

<210> 255
<211> 408

<212> PRT
<213> E. Coli

<400> 255
Met Ala Tyr Cys Asn Pro Gly Leu Glu Ser Arg Pro Asn Lys Arg Asn
1               5                   10                  15
Ala Leu Arg Arg His Val Val Thr Gly Ile Gly Met Lys Ile Val Ile
            20                  25                  30
Ala Pro Asp Ser Tyr Lys Glu Ser Leu Ser Ala Ser Glu Val Ala Gln
        35                  40                  45
Ala Ile Glu Lys Gly Phe Arg Glu Ile Phe Pro Asp Ala Gln Tyr Val
    50                  55                  60
Ser Val Pro Val Ala Asp Gly Gly Glu Gly Thr Val Glu Ala Met Ile
65                  70                  75                  80
Ala Ala Thr Gln Gly Ala Glu Arg His Ala Trp Val Thr Gly Pro Leu
                85                  90                  95
Gly Glu Lys Val Asn Ala Ser Trp Gly Ile Ser Gly Asp Gly Lys Thr
            100                 105                 110
Ala Phe Ile Glu Met Ala Ala Ala Ser Gly Leu Glu Leu Val Pro Ala
        115                 120                 125
Glu Lys Arg Asp Pro Leu Val Thr Thr Ser Arg Gly Thr Gly Glu Leu
    130                 135                 140
Ile Leu Gln Ala Leu Glu Ser Gly Ala Thr Asn Ile Ile Ile Gly Ile
145                 150                 155                 160
Gly Gly Ser Ala Thr Asn Asp Gly Gly Ala Gly Met Val Gln Ala Leu
                165                 170                 175
Gly Ala Lys Leu Cys Asp Ala Asn Gly Asn Glu Ile Gly Phe Gly Gly
            180                 185                 190
Gly Ser Leu Asn Thr Leu Asn Asp Ile Asp Ile Ser Gly Leu Asp Pro
        195                 200                 205
Arg Leu Lys Asp Cys Val Ile Arg Val Ala Cys Asp Val Thr Asn Pro
    210                 215                 220
Leu Val Gly Asp Asn Gly Ala Ser Arg Ile Phe Gly Pro Gln Lys Gly
225                 230                 235                 240
Ala Ser Glu Ala Met Ile Val Glu Leu Asp Asn Asn Leu Ser His Tyr
                245                 250                 255
Ala Glu Val Ile Lys Lys Ala Leu His Val Asp Val Lys Asp Val Pro
            260                 265                 270
Gly Ala Gly Ala Ala Gly Gly Met Gly Ala Ala Leu Met Ala Phe Leu
        275                 280                 285
Gly Ala Glu Leu Lys Ser Gly Ile Glu Ile Val Thr Thr Ala Leu Asn
    290                 295                 300
Leu Glu Glu His Ile His Asp Cys Thr Leu Val Ile Thr Gly Glu Gly
305                 310                 315                 320
Arg Ile Asp Ser Gln Ser Ile His Gly Lys Val Pro Ile Gly Val Ala
                325                 330                 335
Asn Val Ala Lys Lys Tyr His Lys Pro Val Ile Gly Ile Ala Gly Ser
            340                 345                 350
Leu Thr Asp Asp Val Gly Val Val His Gln His Gly Ile Asp Ala Val
        355                 360                 365
Phe Ser Val Leu Thr Ser Ile Gly Thr Leu Asp Glu Ala Phe Arg Gly
    370                 375                 380
Ala Tyr Asp Asn Ile Cys Arg Ala Ser Arg Asn Ile Ala Ala Thr Leu
385                 390                 395                 400
Ala Ile Gly Met Arg Asn Ala Gly
                405

<210> 256
<211> 299
<212> PRT

<213> E. Coli

<400> 256
Met Ile Asp Met Thr Met Lys Val Gly Phe Ile Gly Leu Gly Ile Met
1               5                   10                  15
Gly Lys Pro Met Ser Lys Asn Leu Leu Lys Ala Gly Tyr Ser Leu Val
            20                  25                  30
Val Ala Asp Arg Asn Pro Glu Ala Ile Ala Asp Val Ile Ala Ala Gly
        35                  40                  45
Ala Glu Thr Ala Ser Thr Ala Lys Ala Ile Ala Glu Gln Cys Asp Val
    50                  55                  60
Ile Ile Thr Met Leu Pro Asn Ser Pro His Val Lys Glu Val Ala Leu
65                  70                  75                  80
Gly Glu Asn Gly Ile Ile Glu Gly Ala Lys Pro Gly Thr Val Leu Ile
                85                  90                  95
Asp Met Ser Ser Ile Ala Pro Leu Ala Ser Arg Glu Ile Ser Glu Ala
            100                 105                 110
Leu Lys Ala Lys Gly Ile Asp Met Leu Asp Ala Pro Val Ser Gly Gly
        115                 120                 125
Glu Pro Lys Ala Ile Asp Gly Thr Leu Ser Val Met Val Gly Gly Asp
    130                 135                 140
Lys Ala Ile Phe Asp Lys Tyr Tyr Asp Leu Met Lys Ala Met Ala Gly
145                 150                 155                 160
Ser Val Val His Thr Gly Glu Ile Gly Ala Gly Asn Val Thr Lys Leu
                165                 170                 175
Ala Asn Gln Val Ile Val Ala Leu Asn Ile Ala Ala Met Ser Glu Ala
            180                 185                 190
Leu Thr Leu Ala Thr Lys Ala Gly Val Asn Pro Asp Leu Val Tyr Gln
        195                 200                 205
Ala Ile Arg Gly Gly Leu Ala Gly Ser Thr Val Leu Asp Ala Lys Ala
    210                 215                 220
Pro Met Val Met Asp Arg Asn Phe Lys Pro Gly Phe Arg Ile Asp Leu
225                 230                 235                 240
His Ile Lys Asp Leu Ala Asn Ala Leu Asp Thr Ser His Gly Val Gly
                245                 250                 255
Ala Gln Leu Pro Leu Thr Ala Ala Val Met Glu Met Met Gln Ala Leu
            260                 265                 270
Arg Ala Asp Gly Leu Gly Thr Ala Asp His Ser Ala Leu Ala Cys Tyr
        275                 280                 285
Tyr Glu Lys Leu Ala Lys Val Glu Val Thr Arg
    290                 295

<210> 257
<211> 256
<212> PRT
<213> E. Coli

<400> 257

<210> 258
<211> 444
<212> PRT
<213> E. Coli

<400> 258
Met Ile Leu Asp Thr Val Asp Glu Lys Lys Lys Gly Val His Thr Arg
 1               5                  10                  15
Tyr Leu Ile Leu Leu Ile Ile Phe Ile Val Thr Ala Val Asn Tyr Ala
            20                  25                  30
Asp Arg Ala Thr Leu Ser Ile Ala Gly Thr Glu Val Ala Lys Glu Leu
        35                  40                  45
Gln Leu Ser Ala Val Ser Met Gly Tyr Ile Phe Ser Ala Phe Gly Trp
    50                  55                  60
Ala Tyr Leu Leu Met Gln Ile Pro Gly Gly Trp Leu Leu Asp Lys Phe
65                  70                  75                  80
Gly Ser Lys Lys Val Tyr Thr Tyr Ser Leu Phe Phe Trp Ser Leu Phe
                85                  90                  95
Thr Phe Leu Gln Gly Phe Val Asp Met Phe Pro Leu Ala Trp Ala Gly
            100                 105                 110
Ile Ser Met Phe Phe Met Arg Phe Met Leu Gly Phe Ser Glu Ala Pro
        115                 120                 125
Ser Phe Pro Ala Asn Ala Arg Ile Val Ala Ala Trp Phe Pro Thr Lys
    130                 135                 140
Glu Arg Gly Thr Ala Ser Ala Ile Phe Asn Ser Ala Gln Tyr Phe Ser
145                 150                 155                 160
Leu Ala Leu Phe Ser Pro Leu Leu Gly Trp Leu Thr Phe Ala Trp Gly
                165                 170                 175
Trp Glu His Val Phe Thr Val Met Gly Val Ile Gly Phe Val Leu Thr
            180                 185                 190
Ala Leu Trp Ile Lys Leu Ile His Asn Pro Thr Asp His Pro Arg Met
        195                 200                 205
Ser Ala Glu Glu Leu Lys Phe Ile Ser Glu Asn Gly Ala Val Val Asp
    210                 215                 220
Met Asp His Lys Lys Pro Gly Ser Ala Ala Ala Ser Gly Pro Lys Leu
225                 230                 235                 240
His Tyr Ile Lys Gln Leu Leu Ser Asn Arg Met Met Leu Gly Val Phe
                245                 250                 255

Phe Gly Gln Tyr Phe Ile Asn Thr Ile Thr Trp Phe Phe Leu Thr Trp
            260                 265                 270
Phe Pro Ile Tyr Leu Val Gln Glu Lys Gly Met Ser Ile Leu Lys Val
        275                 280                 285
Gly Leu Val Ala Ser Ile Pro Ala Leu Cys Gly Phe Ala Gly Gly Val
    290                 295                 300
Leu Gly Gly Val Phe Ser Asp Tyr Leu Ile Lys Arg Gly Leu Ser Leu
305                 310                 315                 320
Thr Leu Ala Arg Lys Leu Pro Ile Val Leu Gly Met Leu Leu Ala Ser
                325                 330                 335
Thr Ile Ile Leu Cys Asn Tyr Thr Asn Asn Thr Thr Leu Val Val Met
            340                 345                 350
Leu Met Ala Leu Ala Phe Phe Gly Lys Gly Phe Gly Ala Leu Gly Trp
        355                 360                 365
Pro Val Ile Ser Asp Thr Ala Pro Lys Glu Ile Val Gly Leu Cys Gly
    370                 375                 380
Gly Val Phe Asn Val Phe Gly Asn Val Ala Ser Ile Val Thr Pro Leu
385                 390                 395                 400
Val Ile Gly Tyr Leu Val Ser Glu Leu His Ser Phe Asn Ala Ala Leu
                405                 410                 415
Val Phe Val Gly Cys Ser Ala Leu Met Ala Met Val Cys Tyr Leu Phe
            420                 425                 430
Val Val Gly Asp Ile Lys Arg Met Glu Leu Gln Lys
        435                 440

<210> 259
<211> 511
<212> PRT
<213> E. Coli

<400> 259
Met Gln Thr Ser Asp Thr Arg Ala Leu Pro Leu Leu Cys Ala Arg Ser
1               5                   10                  15
Val Tyr Lys Gln Tyr Ser Gly Val Asn Val Leu Lys Gly Ile Asp Phe
            20                  25                  30
Thr Leu His Gln Gly Glu Val His Ala Leu Leu Gly Gly Asn Gly Ala
        35                  40                  45
Gly Lys Ser Thr Leu Met Lys Ile Ile Ala Gly Ile Thr Pro Ala Asp
    50                  55                  60
Ser Gly Thr Leu Glu Ile Glu Gly Asn Asn Tyr Val Arg Leu Thr Pro
65                  70                  75                  80
Val His Ala His Gln Leu Gly Ile Tyr Leu Val Pro Gln Glu Pro Leu
                85                  90                  95
Leu Phe Pro Ser Leu Ser Ile Lys Glu Asn Ile Leu Phe Gly Leu Ala
            100                 105                 110
Lys Lys Gln Leu Ser Met Gln Lys Met Lys Asn Leu Leu Ala Ala Leu
        115                 120                 125
Gly Cys Gln Phe Asp Leu His Ser Leu Ala Gly Ser Leu Asp Val Ala
    130                 135                 140
Asp Arg Gln Met Val Glu Ile Leu Arg Gly Leu Met Arg Asp Ser Arg
145                 150                 155                 160
Ile Leu Ile Leu Asp Glu Pro Thr Ala Ser Leu Thr Pro Ala Glu Thr
                165                 170                 175
Glu Arg Leu Phe Ser Arg Leu Gln Glu Leu Leu Ala Thr Gly Val Gly
            180                 185                 190
Ile Val Phe Ile Ser His Lys Leu Pro Glu Ile Arg Gln Ile Ala Asp
        195                 200                 205
Arg Ile Ser Val Met Arg Asp Gly Thr Ile Ala Leu Ser Gly Lys Thr
    210                 215                 220
Ser Glu Leu Ser Thr Asp Asp Ile Ile Gln Ala Ile Thr Pro Ala Val

225                 230                 235                 240
Arg Glu Lys Ser Leu Ser Ala Ser Gln Lys Leu Trp Leu Glu Leu Pro
                245                 250                 255
Gly Asn Arg Pro Gln His Ala Ala Gly Thr Pro Val Leu Thr Leu Glu
            260                 265                 270
Asn Leu Thr Gly Glu Gly Phe Arg Asn Val Ser Leu Thr Leu Asn Ala
        275                 280                 285
Gly Glu Ile Leu Gly Leu Ala Gly Leu Val Gly Ala Gly Arg Thr Glu
    290                 295                 300
Leu Ala Glu Thr Leu Tyr Gly Leu Arg Thr Leu Arg Gly Gly Arg Ile
305                 310                 315                 320
Met Leu Asn Gly Lys Glu Ile Asn Lys Leu Ser Thr Gly Glu Arg Leu
                325                 330                 335
Leu Arg Gly Leu Val Tyr Leu Pro Glu Asp Arg Gln Ser Ser Gly Leu
            340                 345                 350
Asn Leu Asp Ala Ser Leu Ala Trp Asn Val Cys Ala Leu Thr His Asn
        355                 360                 365
Leu Arg Gly Phe Trp Ala Lys Thr Ala Lys Asp Asn Ala Thr Leu Glu
    370                 375                 380
Arg Tyr Arg Arg Ala Leu Asn Ile Lys Phe Asn Gln Pro Glu Gln Ala
385                 390                 395                 400
Ala Arg Thr Leu Ser Gly Gly Asn Gln Gln Lys Ile Leu Ile Ala Lys
                405                 410                 415
Cys Leu Glu Ala Ser Pro Gln Val Leu Ile Val Asp Glu Pro Thr Arg
            420                 425                 430
Gly Val Asp Val Ser Ala Arg Asn Asp Ile Tyr Gln Leu Leu Arg Ser
        435                 440                 445
Ile Ala Ala Gln Asn Val Ala Val Leu Leu Ile Ser Ser Asp Leu Glu
    450                 455                 460
Glu Ile Glu Leu Met Ala Asp Arg Val Tyr Val Met His Gln Gly Glu
465                 470                 475                 480
Ile Thr His Ser Ala Leu Thr Glu Arg Asp Ile Asn Val Glu Thr Ile
                485                 490                 495
Met Arg Val Ala Phe Gly Asp Ser Gln Arg Gln Glu Ala Ser Cys
            500                 505                 510

<210> 260
<211> 342
<212> PRT
<213> E. Coli

<400> 260
Met Leu Lys Phe Ile Gln Asn Asn Arg Glu Ile Thr Ala Leu Leu Ala
1               5                   10                  15
Val Val Leu Leu Phe Val Leu Pro Gly Phe Leu Asp Arg Gln Tyr Leu
            20                  25                  30
Ser Val Gln Thr Leu Thr Met Val Tyr Ser Ser Ala Gln Ile Leu Ile
        35                  40                  45
Leu Leu Ala Met Gly Ala Thr Leu Val Met Leu Thr Arg Asn Ile Asp
    50                  55                  60
Val Ser Val Gly Ser Ile Thr Gly Met Cys Ala Val Leu Leu Gly Met
65                  70                  75                  80
Leu Leu Asn Ala Gly Tyr Ser Leu Pro Val Ala Cys Val Ala Thr Leu
                85                  90                  95
Leu Leu Gly Leu Leu Ala Gly Phe Phe Asn Gly Val Leu Val Ala Trp
            100                 105                 110
Leu Lys Ile Pro Ala Ile Val Ala Thr Leu Gly Thr Leu Gly Leu Tyr
        115                 120                 125
Arg Gly Ile Met Leu Leu Trp Thr Gly Gly Lys Trp Ile Glu Gly Leu
    130                 135                 140

Pro Ala Glu Leu Lys Gln Leu Ser Ala Pro Leu Leu Leu Gly Val Ser
145                 150                 155                 160
Ala Ile Gly Trp Leu Thr Ile Ile Leu Val Ala Phe Met Ala Trp Leu
                165                 170                 175
Leu Ala Lys Thr Ala Phe Gly Arg Ser Phe Tyr Ala Thr Gly Asp Asn
            180                 185                 190
Leu Gln Gly Ala Arg Gln Leu Gly Val Arg Thr Glu Ala Ile Arg Ile
        195                 200                 205
Val Ala Phe Ser Leu Asn Gly Cys Met Ala Ala Leu Ala Gly Ile Val
    210                 215                 220
Phe Ala Ser Gln Ile Gly Phe Ile Pro Asn Gln Thr Gly Thr Gly Leu
225                 230                 235                 240
Glu Met Lys Ala Ile Ala Ala Cys Val Leu Gly Gly Ile Ser Leu Leu
                245                 250                 255
Gly Gly Ser Gly Ala Ile Ile Gly Ala Val Leu Gly Ala Trp Phe Leu
            260                 265                 270
Thr Gln Ile Asp Ser Val Leu Val Leu Leu Arg Ile Pro Ala Trp Trp
        275                 280                 285
Asn Asp Phe Ile Ala Gly Leu Val Leu Leu Ala Val Leu Val Phe Asp
    290                 295                 300
Gly Arg Leu Arg Cys Ala Leu Glu Arg Asn Leu Arg Arg Gln Lys Tyr
305                 310                 315                 320
Ala Arg Phe Met Thr Pro Pro Pro Ser Val Lys Pro Ala Ser Ser Gly
                325                 330                 335
Lys Lys Arg Glu Ala Ala
            340

<210> 261
<211> 330
<212> PRT
<213> E. Coli

<400> 261
Met Arg Ile Arg Tyr Gly Trp Glu Leu Ala Leu Ala Ala Leu Leu Val
 1               5                  10                  15
Ile Glu Ile Val Ala Phe Gly Ala Ile Asn Pro Arg Met Leu Asp Leu
            20                  25                  30
Asn Met Leu Leu Phe Ser Thr Ser Asp Phe Ile Cys Ile Gly Ile Val
        35                  40                  45
Ala Leu Pro Leu Thr Met Val Ile Val Ser Gly Gly Ile Asp Ile Ser
    50                  55                  60
Phe Gly Ser Thr Ile Gly Leu Cys Ala Ile Ala Leu Gly Val Leu Phe
65                  70                  75                  80
Gln Ser Gly Val Pro Met Pro Leu Ala Ile Leu Leu Thr Leu Leu Leu
                85                  90                  95
Gly Ala Leu Cys Gly Leu Ile Asn Ala Gly Leu Ile Ile Tyr Thr Lys
            100                 105                 110
Val Asn Pro Leu Val Ile Thr Leu Gly Thr Leu Tyr Leu Phe Ala Gly
        115                 120                 125
Ser Ala Leu Leu Leu Ser Gly Met Ala Gly Ala Thr Gly Tyr Glu Gly
    130                 135                 140
Ile Gly Gly Phe Pro Met Ala Phe Thr Asp Phe Ala Asn Leu Asp Val
145                 150                 155                 160
Leu Gly Leu Pro Val Pro Leu Ile Ile Phe Leu Ile Cys Leu Leu Val
                165                 170                 175
Phe Trp Leu Trp Leu His Lys Thr His Ala Gly Arg Asn Val Phe Leu
            180                 185                 190
Ile Gly Gln Ser Pro Arg Val Ala Leu Tyr Ser Ala Ile Pro Val Asn
        195                 200                 205
Arg Thr Leu Cys Ala Leu Tyr Ala Met Thr Gly Leu Ala Ser Ala Val
    210                 215                 220

Ala Ala Val Leu Leu Val Ser Tyr Phe Gly Ser Ala Arg Ser Asp Leu
225                 230                 235                 240
Gly Ala Ser Phe Leu Met Pro Ala Ile Thr Ala Val Val Leu Gly Gly
                245                 250                 255
Ala Asn Ile Tyr Gly Gly Ser Gly Ser Ile Ile Gly Thr Ala Ile Ala
            260                 265                 270
Val Leu Leu Val Gly Tyr Leu Gln Gln Gly Leu Gln Met Ala Gly Val
        275                 280                 285
Pro Asn Gln Val Ser Ser Ala Leu Ser Gly Ala Leu Leu Ile Val Val
    290                 295                 300
Val Val Gly Arg Ser Val Ser Leu His Arg Gln Gln Ile Lys Glu Trp
305                 310                 315                 320
Leu Ala Arg Arg Ala Asn Asn Pro Leu Pro
                325                 330

<210> 262
<211> 340
<212> PRT
<213> E. Coli

<400> 262
Met Thr Leu His Arg Phe Lys Lys Ile Ala Leu Leu Ser Ala Leu Gly
1               5                   10                  15
Ile Ala Ala Ile Ser Met Asn Val Gln Ala Ala Glu Arg Ile Ala Phe
            20                  25                  30
Ile Pro Lys Leu Val Gly Val Gly Phe Phe Thr Ser Gly Gly Asn Gly
        35                  40                  45
Ala Gln Gln Ala Gly Lys Glu Leu Gly Val Asp Val Thr Tyr Asp Gly
    50                  55                  60
Pro Thr Glu Pro Ser Val Ser Gly Gln Val Gln Leu Ile Asn Asn Phe
65                  70                  75                  80
Val Asn Gln Gly Tyr Asn Ala Ile Ile Val Ser Ala Val Ser Pro Asp
                85                  90                  95
Gly Leu Cys Pro Ala Leu Lys Arg Ala Met Gln Arg Gly Val Arg Val
            100                 105                 110
Leu Thr Trp Asp Ser Asp Thr Lys Pro Glu Cys Arg Ser Tyr Tyr Ile
        115                 120                 125
Asn Gln Gly Thr Pro Ala Gln Leu Gly Gly Met Leu Val Asp Met Ala
    130                 135                 140
Ala Arg Gln Val Asn Lys Asp Lys Ala Lys Val Ala Phe Phe Tyr Ser
145                 150                 155                 160
Ser Pro Thr Val Thr Asp Gln Asn Gln Trp Val Lys Glu Ala Lys Ala
                165                 170                 175
Lys Ile Ala Lys Glu His Pro Gly Trp Glu Ile Val Thr Thr Gln Phe
            180                 185                 190
Gly Tyr Asn Asp Ala Thr Lys Ser Leu Gln Thr Ala Glu Gly Ile Leu
        195                 200                 205
Lys Ala Tyr Ser Asp Leu Asp Ala Ile Ile Ala Pro Asp Ala Asn Ala
    210                 215                 220
Leu Pro Ala Ala Ala Gln Ala Ala Glu Asn Leu Lys Asn Asp Lys Val
225                 230                 235                 240
Ala Ile Val Gly Phe Ser Thr Pro Asn Val Met Arg Pro Tyr Val Glu
                245                 250                 255
Arg Gly Thr Val Lys Glu Phe Gly Leu Trp Asp Val Val Gln Gln Gly
            260                 265                 270
Lys Ile Ser Val Tyr Val Ala Asp Ala Leu Leu Lys Lys Gly Ser Met
        275                 280                 285
Lys Thr Gly Asp Lys Leu Asp Ile Lys Gly Val Gly Gln Val Glu Val

290                 295                 300
Ser Pro Asn Ser Val Gln Gly Tyr Asp Tyr Glu Ala Asp Gly Asn Gly
305                 310                 315                 320
Ile Val Leu Leu Pro Glu Arg Val Ile Phe Asn Lys Glu Asn Ile Gly
                325                 330                 335
Lys Tyr Asp Phe
            340

<210> 263
<211> 291
<212> PRT
<213> E. Coli

<400> 263
Met Ala Asp Leu Asp Asp Ile Lys Asp Gly Lys Asp Phe Arg Thr Asp
1               5                   10                  15
Gln Pro Gln Lys Asn Ile Pro Phe Thr Leu Lys Gly Cys Gly Ala Leu
            20                  25                  30
Asp Trp Gly Met Gln Ser Arg Leu Ser Arg Ile Phe Asn Pro Lys Thr
        35                  40                  45
Gly Lys Thr Val Met Leu Ala Phe Asp His Gly Tyr Phe Gln Gly Pro
    50                  55                  60
Thr Thr Gly Leu Glu Arg Ile Asp Ile Asn Ile Ala Pro Leu Phe Glu
65                  70                  75                  80
His Ala Asp Val Leu Met Cys Thr Arg Gly Ile Leu Arg Ser Val Val
                85                  90                  95
Pro Pro Ala Thr Asn Arg Pro Val Val Leu Arg Ala Ser Gly Ala Asn
            100                 105                 110
Ser Ile Leu Ala Glu Leu Ser Asn Glu Ala Val Ala Leu Ser Met Asp
        115                 120                 125
Asp Ala Val Arg Leu Asn Ser Cys Ala Val Ala Ala Gln Val Tyr Ile
    130                 135                 140
Gly Ser Glu Tyr Glu His Gln Ser Ile Lys Asn Ile Ile Gln Leu Val
145                 150                 155                 160
Asp Ala Gly Met Lys Val Gly Met Pro Thr Met Ala Val Thr Gly Val
                165                 170                 175
Gly Lys Asp Met Val Arg Asp Gln Arg Tyr Phe Ser Leu Ala Thr Arg
            180                 185                 190
Ile Ala Ala Glu Met Gly Ala Gln Ile Ile Lys Thr Tyr Tyr Val Glu
        195                 200                 205
Lys Gly Phe Glu Arg Ile Val Ala Gly Cys Pro Val Pro Ile Val Ile
    210                 215                 220
Ala Gly Gly Lys Lys Leu Pro Glu Arg Glu Ala Leu Glu Met Cys Trp
225                 230                 235                 240
Gln Ala Ile Asp Gln Gly Ala Ser Gly Val Asp Met Gly Arg Asn Ile
                245                 250                 255
Phe Gln Ser Asp His Pro Val Ala Met Met Lys Ala Val Gln Ala Val
            260                 265                 270
Val His His Asn Glu Thr Ala Asp Arg Ala Tyr Glu Leu Tyr Leu Ser
        275                 280                 285
Glu Lys Gln
    290

<210> 264
<211> 96
<212> PRT
<213> E. Coli

<400> 264

Met His Val Thr Leu Val Glu Ile Asn Val His Glu Asp Lys Val Asp
1               5                   10                  15
Glu Phe Ile Glu Val Phe Arg Gln Asn His Leu Gly Ser Val Gln Glu
            20                  25                  30
Glu Gly Asn Leu Arg Phe Asp Val Leu Gln Asp Pro Glu Val Asn Ser
        35                  40                  45
Arg Phe Tyr Ile Tyr Glu Ala Tyr Lys Asp Glu Asp Ala Val Ala Phe
    50                  55                  60
His Lys Thr Thr Pro His Tyr Lys Thr Cys Val Ala Lys Leu Glu Ser
65                  70                  75                  80
Leu Met Thr Gly Pro Arg Lys Lys Arg Leu Phe Asn Gly Leu Met Pro
                85                  90                  95

<210> 265
<211> 383
<212> PRT
<213> E. Coli

<400> 265
Met Phe Glu Pro Met Glu Leu Thr Asn Asp Ala Val Ile Lys Val Ile
1               5                   10                  15
Gly Val Gly Gly Gly Gly Gly Asn Ala Val Glu His Met Val Arg Glu
            20                  25                  30
Arg Ile Glu Gly Val Glu Phe Phe Ala Val Asn Thr Asp Ala Gln Ala
        35                  40                  45
Leu Arg Lys Thr Ala Val Gly Gln Thr Ile Gln Ile Gly Ser Gly Ile
    50                  55                  60
Thr Lys Gly Leu Gly Ala Gly Ala Asn Pro Glu Val Gly Arg Asn Ala
65                  70                  75                  80
Ala Asp Glu Asp Arg Asp Ala Leu Arg Ala Ala Leu Glu Gly Ala Asp
                85                  90                  95
Met Val Phe Ile Ala Ala Gly Met Gly Gly Gly Thr Gly Thr Gly Ala
            100                 105                 110
Ala Pro Val Val Ala Glu Val Ala Lys Asp Leu Gly Ile Leu Thr Val
        115                 120                 125
Ala Val Val Thr Lys Pro Phe Asn Phe Glu Gly Lys Lys Arg Met Ala
    130                 135                 140
Phe Ala Glu Gln Gly Ile Thr Glu Leu Ser Lys His Val Asp Ser Leu
145                 150                 155                 160
Ile Thr Ile Pro Asn Asp Lys Leu Leu Lys Val Leu Gly Arg Gly Ile
                165                 170                 175
Ser Leu Leu Asp Ala Phe Gly Ala Ala Asn Asp Val Leu Lys Gly Ala
            180                 185                 190
Val Gln Gly Ile Ala Glu Leu Ile Thr Arg Pro Gly Leu Met Asn Val
        195                 200                 205
Asp Phe Ala Asp Val Arg Thr Val Met Ser Glu Met Gly Tyr Ala Met
    210                 215                 220
Met Gly Ser Gly Val Ala Ser Gly Glu Asp Arg Ala Glu Glu Ala Ala
225                 230                 235                 240
Glu Met Ala Ile Ser Ser Pro Leu Leu Glu Asp Ile Asp Leu Ser Gly
                245                 250                 255
Ala Arg Gly Val Leu Val Asn Ile Thr Ala Gly Phe Asp Leu Arg Leu
            260                 265                 270
Asp Glu Phe Glu Thr Val Gly Asn Thr Ile Arg Ala Phe Ala Ser Asp
        275                 280                 285
Asn Ala Thr Val Val Ile Gly Thr Ser Leu Asp Pro Asp Met Asn Asp
    290                 295                 300
Glu Leu Arg Val Thr Val Val Ala Thr Gly Ile Gly Met Asp Lys Arg
305                 310                 315                 320
Pro Glu Ile Thr Leu Val Thr Asn Lys Gln Val Gln Gln Pro Val Met

325                 330                 335
Asp Arg Tyr Gln Gln His Gly Met Ala Pro Leu Thr Gln Glu Gln Lys
            340                 345                 350
Pro Val Ala Lys Val Val Asn Asp Asn Ala Pro Gln Thr Ala Lys Glu
        355                 360                 365
Pro Asp Tyr Leu Asp Ile Pro Ala Phe Leu Arg Lys Gln Ala Asp
    370                 375                 380

<210> 266
<211> 1014
<212> PRT
<213> E. Coli

<400> 266
Met Asp Val Ser Arg Arg Gln Phe Phe Lys Ile Cys Ala Gly Gly Met
1               5                   10                  15
Ala Gly Thr Thr Val Ala Ala Leu Gly Phe Ala Pro Lys Gln Ala Leu
            20                  25                  30
Ala Gln Ala Arg Asn Tyr Lys Leu Leu Arg Ala Lys Glu Ile Arg Asn
        35                  40                  45
Thr Cys Thr Tyr Cys Ser Val Gly Cys Gly Leu Leu Met Tyr Ser Leu
    50                  55                  60
Gly Asp Gly Ala Lys Asn Ala Arg Glu Ala Ile Tyr His Ile Glu Gly
65                  70                  75                  80
Asp Pro Asp His Pro Val Ser Arg Gly Ala Leu Cys Pro Lys Gly Ala
                85                  90                  95
Gly Leu Leu Asp Tyr Val Asn Ser Glu Asn Arg Leu Arg Tyr Pro Glu
            100                 105                 110
Tyr Arg Ala Pro Gly Ser Asp Lys Trp Gln Arg Ile Ser Trp Glu Glu
        115                 120                 125
Ala Phe Ser Arg Ile Ala Lys Leu Met Lys Ala Asp Arg Asp Ala Asn
    130                 135                 140
Phe Ile Glu Lys Asn Glu Gln Gly Val Thr Val Asn Arg Trp Leu Ser
145                 150                 155                 160
Thr Gly Met Leu Cys Ala Ser Gly Ala Ser Asn Glu Thr Gly Met Leu
                165                 170                 175
Thr Gln Lys Phe Ala Arg Ser Leu Gly Met Leu Ala Val Asp Asn Gln
            180                 185                 190
Ala Arg Val His Gly Pro Thr Val Ala Ser Leu Ala Pro Thr Phe Gly
        195                 200                 205
Arg Gly Ala Met Thr Asn His Trp Val Asp Ile Lys Asn Ala Asn Val
    210                 215                 220
Val Met Val Met Gly Gly Asn Ala Ala Glu Ala His Pro Val Gly Phe
225                 230                 235                 240
Arg Trp Ala Met Glu Ala Lys Asn Asn Asn Asp Ala Thr Leu Ile Val
                245                 250                 255
Val Asp Pro Arg Phe Thr Arg Thr Ala Ser Val Ala Asp Ile Tyr Ala
            260                 265                 270
Pro Ile Arg Ser Gly Thr Asp Ile Thr Phe Leu Ser Gly Val Leu Arg
        275                 280                 285
Tyr Leu Ile Glu Asn Asn Lys Ile Asn Ala Glu Tyr Val Lys His Tyr
    290                 295                 300
Thr Asn Ala Ser Leu Leu Val Arg Asp Asp Phe Ala Phe Glu Asp Gly
305                 310                 315                 320
Leu Phe Ser Gly Tyr Asp Ala Glu Lys Arg Gln Tyr Asp Lys Ser Ser
                325                 330                 335
Trp Asn Tyr Gln Leu Asp Glu Asn Gly Tyr Ala Lys Arg Asp Glu Thr
            340                 345                 350
Leu Thr His Pro Arg Cys Val Trp Asn Leu Leu Lys Glu His Val Ser
        355                 360                 365

Arg Tyr Thr Pro Asp Val Val Glu Asn Ile Cys Gly Thr Pro Lys Ala
    370                 375                 380
Asp Phe Leu Lys Val Cys Glu Val Leu Ala Ser Thr Ser Ala Pro Asp
385                 390                 395                 400
Arg Thr Thr Thr Phe Leu Tyr Ala Leu Gly Trp Thr Gln His Thr Val
                405                 410                 415
Gly Ala Gln Asn Ile Arg Thr Met Ala Met Ile Gln Leu Leu Leu Gly
            420                 425                 430
Asn Met Gly Met Ala Gly Gly Gly Val Asn Ala Leu Arg Gly His Ser
        435                 440                 445
Asn Ile Gln Gly Leu Thr Asp Leu Gly Leu Leu Ser Thr Ser Leu Pro
    450                 455                 460
Gly Tyr Leu Thr Leu Pro Ser Glu Lys Gln Val Asp Leu Gln Ser Tyr
465                 470                 475                 480
Leu Glu Ala Asn Thr Pro Lys Ala Thr Leu Ala Asp Gln Val Asn Tyr
                485                 490                 495
Trp Ser Asn Tyr Pro Lys Phe Phe Val Ser Leu Met Lys Ser Phe Tyr
            500                 505                 510
Gly Asp Ala Ala Gln Lys Glu Asn Asn Trp Gly Tyr Asp Trp Leu Pro
        515                 520                 525
Lys Trp Asp Gln Thr Tyr Asp Val Ile Lys Tyr Phe Asn Met Met Asp
    530                 535                 540
Glu Gly Lys Val Thr Gly Tyr Phe Cys Gln Gly Phe Asn Pro Val Ala
545                 550                 555                 560
Ser Phe Pro Asp Lys Asn Lys Val Val Ser Cys Leu Ser Lys Leu Lys
                565                 570                 575
Tyr Met Val Val Ile Asp Pro Leu Val Thr Glu Thr Ser Thr Phe Trp
            580                 585                 590
Gln Asn His Gly Glu Ser Asn Asp Val Asp Pro Ala Ser Ile Gln Thr
        595                 600                 605
Glu Val Phe Arg Leu Pro Ser Thr Cys Phe Ala Glu Glu Asp Gly Ser
    610                 615                 620
Ile Ala Asn Ser Gly Arg Trp Leu Gln Trp His Trp Lys Gly Gln Asp
625                 630                 635                 640
Ala Pro Gly Glu Ala Arg Asn Asp Gly Glu Ile Leu Ala Gly Ile Tyr
                645                 650                 655
His His Leu Arg Glu Leu Tyr Gln Ser Glu Gly Gly Lys Gly Val Glu
            660                 665                 670
Pro Leu Met Lys Met Ser Trp Asn Tyr Lys Gln Pro His Glu Pro Gln
        675                 680                 685
Ser Asp Glu Val Ala Lys Glu Asn Asn Gly Tyr Ala Leu Glu Asp Leu
    690                 695                 700
Tyr Asp Ala Asn Gly Val Leu Ile Ala Lys Lys Gly Gln Leu Leu Ser
705                 710                 715                 720
Ser Phe Ala His Leu Arg Asp Asp Gly Thr Thr Ala Ser Ser Cys Trp
                725                 730                 735
Ile Tyr Thr Gly Ser Trp Thr Glu Gln Gly Asn Gln Met Ala Asn Arg
            740                 745                 750
Asp Asn Ser Asp Pro Ser Gly Leu Gly Asn Thr Leu Gly Trp Ala Trp
        755                 760                 765
Ala Trp Pro Leu Asn Arg Arg Val Leu Tyr Asn Arg Ala Ser Ala Asp
    770                 775                 780
Ile Asn Gly Lys Pro Trp Asp Pro Lys Arg Met Leu Ile Gln Trp Asn
785                 790                 795                 800
Gly Ser Lys Trp Thr Gly Asn Asp Ile Pro Asp Phe Gly Asn Ala Ala
                805                 810                 815
Pro Gly Thr Pro Thr Gly Pro Phe Ile Met Gln Pro Glu Gly Met Gly
            820                 825                 830
Arg Leu Phe Ala Ile Asn Lys Met Ala Glu Gly Pro Phe Pro Glu His
        835                 840                 845
Tyr Glu Pro Ile Glu Thr Pro Leu Gly Thr Asn Pro Leu His Pro Asn

850                 855                 860
Val Val Ser Asn Pro Val Val Arg Leu Tyr Glu Gln Asp Ala Leu Arg
865                 870                 875                 880
Met Gly Lys Lys Glu Gln Phe Pro Tyr Val Gly Thr Thr Tyr Arg Leu
                885                 890                 895
Thr Glu His Phe His Thr Trp Thr Lys His Ala Leu Leu Asn Ala Ile
            900                 905                 910
Ala Gln Pro Glu Gln Phe Val Glu Ile Ser Glu Thr Leu Ala Ala Ala
        915                 920                 925
Lys Gly Ile Asn Asn Gly Asp Arg Val Thr Val Ser Ser Lys Arg Gly
    930                 935                 940
Phe Ile Arg Ala Val Ala Val Val Thr Arg Arg Leu Lys Pro Leu Asn
945                 950                 955                 960
Val Asn Gly Gln Gln Val Glu Thr Val Gly Ile Pro Ile His Trp Gly
                965                 970                 975
Phe Glu Gly Val Ala Arg Lys Gly Tyr Ile Ala Asn Thr Leu Thr Pro
            980                 985                 990
Asn Val Gly Asp Ala Asn Ser Gln Thr Pro Glu Tyr Lys Ala Phe Leu
        995                 1000                1005
Val Asn Ile Glu Lys Ala
    1010

<210> 267
<211> 294
<212> PRT
<213> E. Coli

<400> 267
Met Ala Met Glu Thr Gln Asp Ile Ile Lys Arg Ser Ala Thr Asn Ser
 1               5                  10                  15
Ile Thr Pro Pro Ser Gln Val Arg Asp Tyr Lys Ala Glu Val Ala Lys
            20                  25                  30
Leu Ile Asp Val Ser Thr Cys Ile Gly Cys Lys Ala Cys Gln Val Ala
        35                  40                  45
Cys Ser Glu Trp Asn Asp Ile Arg Asp Glu Val Gly His Cys Val Gly
    50                  55                  60
Val Tyr Asp Asn Pro Ala Asp Leu Ser Ala Lys Ser Trp Thr Val Met
65                  70                  75                  80
Arg Phe Ser Glu Thr Glu Gln Asn Gly Lys Leu Glu Trp Leu Ile Arg
                85                  90                  95
Lys Asp Gly Cys Met His Cys Glu Asp Pro Gly Cys Leu Lys Ala Cys
            100                 105                 110
Pro Ser Ala Gly Ala Ile Ile Gln Tyr Ala Asn Gly Ile Val Asp Phe
        115                 120                 125
Gln Ser Glu Asn Cys Ile Gly Cys Gly Tyr Cys Ile Ala Gly Cys Pro
    130                 135                 140
Phe Asn Ile Pro Arg Leu Asn Lys Glu Asp Asn Arg Val Tyr Lys Cys
145                 150                 155                 160
Thr Leu Cys Val Asp Arg Val Ser Val Gly Gln Glu Pro Ala Cys Val
                165                 170                 175
Lys Thr Cys Pro Thr Gly Ala Ile His Phe Gly Thr Lys Lys Glu Met
            180                 185                 190
Leu Glu Leu Ala Glu Gln Arg Val Ala Lys Leu Lys Ala Arg Gly Tyr
        195                 200                 205
Glu His Ala Gly Val Tyr Asn Pro Glu Gly Val Gly Gly Thr His Val
    210                 215                 220
Met Tyr Val Leu His His Ala Asp Gln Pro Glu Leu Tyr His Gly Leu
225                 230                 235                 240
Pro Lys Asp Pro Lys Ile Asp Thr Ser Val Ser Leu Trp Lys Gly Ala
                245                 250                 255
Leu Lys Pro Leu Ala Ala Ala Gly Phe Ile Ala Thr Phe Ala Gly Leu

260                 265                 270
Ile Phe His Tyr Ile Gly Ile Gly Pro Asn Lys Glu Val Asp Asp Asp
        275                 280                 285
Glu Glu Asp His His Glu
    290

<210> 268
<211> 217
<212> PRT
<213> E. Coli

<400> 268
Met Ser Lys Ser Lys Met Ile Val Arg Thr Lys Phe Ile Asp Arg Ala
 1               5                  10                  15
Cys His Trp Thr Val Val Ile Cys Phe Phe Leu Val Ala Leu Ser Gly
            20                  25                  30
Ile Ser Phe Phe Phe Pro Thr Leu Gln Trp Leu Thr Gln Thr Phe Gly
        35                  40                  45
Thr Pro Gln Met Gly Arg Ile Leu His Pro Phe Phe Gly Ile Ala Ile
    50                  55                  60
Phe Val Ala Leu Met Phe Met Phe Val Arg Phe Val His His Asn Ile
65                  .70                 75                  80
Pro Asp Lys Lys Asp Ile Pro Trp Leu Leu Asn Ile Val Glu Val Leu
                85                  90                  95
Lys Gly Asn Glu His Lys Val Ala Asp Val Gly Lys Tyr Asn Ala Gly
            100                 105                 110
Gln Lys Met Met Phe Trp Ser Ile Met Ser Met Ile Phe Val Leu Leu
        115                 120                 125
Val Thr Gly Val Ile Ile Trp Arg Pro Tyr Phe Ala Gln Tyr Phe Pro
    130                 135                 140
Met Gln Val Val Arg Tyr Ser Leu Leu Ile His Ala Ala Ala Gly Ile
145                 150                 155                 160
Ile Leu Ile His Ala Ile Leu Ile His Met Tyr Met Ala Phe Trp Val
                165                 170                 175
Lys Gly Ser Ile Lys Gly Met Ile Glu Gly Lys Val Ser Arg Arg Trp
            180                 185                 190
Ala Lys Lys His His Pro Arg Trp Tyr Arg Glu Ile Glu Lys Ala Glu
        195                 200                 205
Ala Lys Lys Glu Ser Glu Glu Gly Ile
    210                 215

<210> 269
<211> 86
<212> PRT
<213> E. Coli

<400> 269
Met Ala Leu Leu Ile Thr Lys Lys Cys Ile Asn Cys Asp Met Cys Glu
 1               5                  10                  15
Pro Glu Cys Pro Asn Glu Ala Ile Ser Met Gly Asp His Ile Tyr Glu
            20                  25                  30
Ile Asn Ser Asp Lys Cys Thr Glu Cys Val Gly His Tyr Glu Thr Pro
        35                  40                  45
Thr Cys Gln Lys Val Cys Pro Ile Pro Asn Thr Ile Val Lys Asp Pro
    50                  55                  60
Ala His Val Glu Thr Glu Glu Gln Leu Trp Asp Lys Phe Val Leu Met
65                  70                  75                  80
His His Ala Asp Lys Ile
                85

<210> 270
<211> 400
<212> PRT
<213> E. Coli

<400> 270

<210> 271

<211> 392
<212> PRT
<213> E. Coli

<400> 271

<210> 272
<211> 441
<212> PRT
<213> E. Coli

<400> 272
Met Ser Glu Ile Ser Arg Gln Glu Phe Gln Arg Arg Arg Gln Ala Leu
1               5                   10                  15
Val Glu Gln Met Gln Pro Gly Ser Ala Ala Leu Ile Phe Ala Ala Pro
            20                  25                  30
Glu Val Thr Arg Ser Ala Asp Ser Glu Tyr Pro Tyr Arg Gln Asn Ser
        35                  40                  45
Asp Phe Trp Tyr Phe Thr Gly Phe Asn Glu Pro Glu Ala Val Leu Val
    50                  55                  60
Leu Ile Lys Ser Asp Asp Thr His Asn His Ser Val Leu Phe Asn Arg
65                  70                  75                  80
Val Arg Asp Leu Thr Ala Glu Ile Trp Phe Gly Arg Arg Leu Gly Gln
                85                  90                  95
Asp Ala Ala Pro Glu Lys Leu Gly Val Asp Arg Ala Leu Ala Phe Ser
            100                 105                 110
Glu Ile Asn Gln Gln Leu Tyr Gln Leu Leu Asn Gly Leu Asp Val Val
        115                 120                 125
Tyr His Ala Gln Gly Glu Tyr Ala Tyr Ala Asp Val Ile Val Asn Ser
    130                 135                 140
Ala Leu Glu Lys Leu Arg Lys Gly Ser Arg Gln Asn Leu Thr Ala Pro
145                 150                 155                 160
Ala Thr Met Ile Asp Trp Arg Pro Val Val His Glu Met Arg Leu Phe
                165                 170                 175
Lys Ser Pro Glu Glu Ile Ala Val Leu Arg Arg Ala Gly Glu Ile Thr
            180                 185                 190
Ala Met Ala His Thr Arg Ala Met Glu Lys Cys Arg Pro Gly Met Phe
        195                 200                 205
Glu Tyr His Leu Glu Gly Glu Ile His His Glu Phe Asn Arg His Gly
    210                 215                 220
Ala Arg Tyr Pro Ser Tyr Asn Thr Ile Val Gly Ser Gly Glu Asn Gly
225                 230                 235                 240
Cys Ile Leu His Tyr Thr Glu Asn Glu Cys Glu Met Arg Asp Gly Asp
                245                 250                 255
Leu Val Leu Ile Asp Ala Gly Cys Glu Tyr Lys Gly Tyr Ala Gly Asp
            260                 265                 270
Ile Thr Arg Thr Phe Pro Val Asn Gly Lys Phe Thr Gln Ala Gln Arg
        275                 280                 285
Glu Ile Tyr Asp Ile Val Leu Glu Ser Leu Glu Thr Ser Leu Arg Leu
    290                 295                 300
Tyr Arg Pro Gly Thr Ser Ile Leu Glu Val Thr Gly Glu Val Val Arg
305                 310                 315                 320
Ile Met Val Ser Gly Leu Val Lys Leu Gly Ile Leu Lys Gly Asp Val
                325                 330                 335
Asp Glu Leu Ile Ala Gln Asn Ala His Arg Pro Phe Phe Met His Gly
            340                 345                 350
Leu Ser His Trp Leu Gly Leu Asp Val His Asp Val Gly Val Tyr Gly
        355                 360                 365
Gln Asp Arg Ser Arg Ile Leu Glu Pro Gly Met Val Leu Thr Val Glu
    370                 375                 380
Pro Gly Leu Tyr Ile Ala Pro Asp Ala Glu Val Pro Glu Gln Tyr Arg
385                 390                 395                 400
Gly Ile Gly Ile Arg Ile Glu Asp Asp Ile Val Ile Thr Glu Thr Gly
                405                 410                 415
Asn Glu Asn Leu Thr Ala Ser Val Val Lys Lys Pro Glu Glu Ile Glu
            420                 425                 430
Ala Leu Met Val Ala Ala Arg Lys Gln
        435                 440

<210> 273

<211> 194
<212> PRT
<213> E. Coli

<400> 273
Met Leu Met Ser Ile Gln Asn Glu Met Pro Gly Tyr Asn Glu Met Asn
1               5                   10                  15
Gln Tyr Leu Asn Gln Gln Gly Thr Gly Leu Thr Pro Ala Glu Met His
            20                  25                  30
Gly Leu Ile Ser Gly Met Ile Cys Gly Gly Asn Asp Asp Ser Ser Trp
        35                  40                  45
Leu Pro Leu Leu His Asp Leu Thr Asn Glu Gly Met Ala Phe Gly His
    50                  55                  60
Glu Leu Ala Gln Ala Leu Arg Lys Met His Ser Ala Thr Ser Asp Ala
65                  70                  75                  80
Leu Gln Asp Asp Gly Phe Leu Phe Gln Leu Tyr Leu Pro Asp Gly Asp
                85                  90                  95
Asp Val Ser Val Phe Asp Arg Ala Asp Ala Leu Ala Gly Trp Val Asn
            100                 105                 110
His Phe Leu Leu Gly Leu Gly Val Thr Gln Pro Lys Leu Asp Lys Val
        115                 120                 125
Thr Gly Glu Thr Gly Glu Ala Ile Asp Asp Leu Arg Asn Ile Ala Gln
    130                 135                 140
Leu Gly Tyr Asp Glu Asp Glu Asp Gln Glu Glu Leu Glu Met Ser Leu
145                 150                 155                 160
Glu Glu Ile Ile Glu Tyr Val Arg Val Ala Ala Leu Leu Cys His Asp
                165                 170                 175
Thr Phe Thr His Pro Gln Pro Thr Ala Pro Glu Val Gln Lys Pro Thr
            180                 185                 190
Leu His

<210> 274
<211> 120
<212> PRT
<213> E. Coli

<400> 274
Met Leu Lys Leu Phe Ala Lys Tyr Thr Ser Ile Gly Val Leu Asn Thr
1               5                   10                  15
Leu Ile His Trp Val Val Phe Gly Val Cys Ile Tyr Val Ala His Thr
            20                  25                  30
Asn Gln Ala Leu Ala Asn Phe Ala Gly Phe Val Val Ala Val Ser Phe
        35                  40                  45
Ser Phe Phe Ala Asn Ala Lys Phe Thr Phe Lys Ala Ser Thr Thr Thr
    50                  55                  60
Met Arg Tyr Met Leu Tyr Val Gly Phe Met Gly Thr Leu Ser Ala Thr
65                  70                  75                  80
Val Gly Trp Ala Ala Asp Arg Cys Ala Leu Pro Pro Met Ile Thr Leu
                85                  90                  95
Val Thr Phe Ser Ala Ile Ser Leu Val Cys Gly Phe Val Tyr Ser Lys
            100                 105                 110
Phe Ile Val Phe Arg Asp Ala Lys
        115                 120

<210> 275
<211> 306
<212> PRT
<213> E. Coli

<400> 275
Met Lys Ile Ser Leu Val Val Pro Val Phe Asn Glu Glu Glu Ala Ile
1               5                   10                  15
Pro Ile Phe Tyr Lys Thr Val Arg Glu Phe Glu Glu Leu Lys Ser Tyr
            20                  25                  30
Glu Val Glu Ile Val Phe Ile Asn Asp Gly Ser Lys Asp Ala Thr Glu
        35                  40                  45
Ser Ile Ile Asn Ala Leu Ala Val Ser Asp Pro Leu Val Val Pro Leu
    50                  55                  60
Ser Phe Thr Arg Asn Phe Gly Lys Glu Pro Ala Leu Phe Ala Gly Leu
65                  70                  75                  80
Asp His Ala Thr Gly Asp Ala Ile Ile Pro Ile Asp Val Asp Leu Gln
                85                  90                  95
Asp Pro Ile Glu Val Ile Pro His Leu Ile Glu Lys Trp Gln Ala Gly
            100                 105                 110
Ala Asp Met Val Leu Ala Lys Arg Ser Asp Arg Ser Thr Asp Gly Arg
        115                 120                 125
Leu Lys Arg Lys Thr Ala Glu Trp Phe Tyr Lys Leu His Asn Lys Ile
    130                 135                 140
Ser Asn Pro Lys Ile Glu Glu Asn Val Gly Asp Phe Arg Leu Met Ser
145                 150                 155                 160
Arg Asp Val Val Glu Asn Ile Lys Leu Met Pro Glu Arg Asn Leu Phe
                165                 170                 175
Met Lys Gly Ile Leu Ser Trp Val Gly Gly Lys Thr Asp Ile Val Glu
            180                 185                 190
Tyr Val Arg Ala Glu Arg Ile Ala Gly Asp Thr Lys Phe Asn Gly Trp
        195                 200                 205
Lys Leu Trp Asn Leu Ala Leu Glu Gly Ile Thr Ser Phe Ser Thr Phe
    210                 215                 220
Pro Leu Arg Ile Trp Thr Tyr Ile Gly Leu Val Val Ala Ser Val Ala
225                 230                 235                 240
Phe Ile Tyr Gly Ala Trp Met Ile Leu Asp Thr Ile Ile Phe Gly Asn
                245                 250                 255
Ala Val Arg Gly Tyr Pro Ser Leu Leu Val Ser Ile Leu Phe Leu Gly
            260                 265                 270
Gly Ile Gln Met Ile Gly Ile Gly Val Leu Gly Glu Tyr Ile Gly Arg
        275                 280                 285
Thr Tyr Ile Glu Thr Lys Lys Arg Pro Lys Tyr Ile Ile Lys Arg Val
    290                 295                 300
Lys Lys
305

<210> 276
<211> 443
<212> PRT
<213> E. Coli

<400> 276
Met Asn Lys Ala Ile Lys Val Ser Leu Tyr Ile Ser Phe Val Leu Ile
1               5                   10                  15
Ile Cys Ala Leu Ser Lys Asn Ile Met Met Leu Asn Thr Ser Asp Phe
            20                  25                  30
Gly Arg Ala Ile Lys Pro Leu Ile Glu Asp Ile Pro Ala Phe Thr Tyr
        35                  40                  45
Asp Leu Pro Leu Leu Tyr Lys Leu Lys Gly His Ile Asp Ser Ile Asp
    50                  55                  60
Ser Tyr Glu Tyr Ile Ser Ser Tyr Ser Tyr Ile Leu Tyr Thr Tyr Val
65                  70                  75                  80
Leu Phe Ile Ser Ile Phe Thr Glu Tyr Leu Asp Ala Arg Val Leu Ser
                85                  90                  95

Leu Phe Leu Lys Val Ile Tyr Ile Tyr Ser Leu Tyr Ala Ile Phe Thr
            100                 105                 110
Ser Tyr Ile Lys Thr Glu Arg Tyr Val Thr Leu Phe Thr Phe Phe Ile
        115                 120                 125
Leu Ala Phe Leu Met Cys Ser Ser Ser Thr Leu Ser Met Phe Ala Ser
    130                 135                 140
Phe Tyr Gln Glu Gln Ile Val Ile Ile Phe Leu Pro Phe Leu Val Tyr
145                 150                 155                 160
Ser Leu Thr Cys Lys Asn Asn Lys Ser Met Leu Leu Leu Phe Phe Ser
                165                 170                 175
Leu Leu Ile Ile Ser Thr Ala Lys Asn Gln Phe Ile Leu Thr Pro Leu
            180                 185                 190
Ile Val Tyr Ser Tyr Tyr Ile Phe Phe Asp Arg His Lys Leu Ile Ile
        195                 200                 205
Lys Ser Val Ile Cys Val Val Cys Leu Leu Ala Ser Ile Phe Ala Ile
    210                 215                 220
Ser Tyr Ser Lys Gly Val Val Glu Leu Asn Lys Tyr His Ala Thr Tyr
225                 230                 235                 240
Phe Gly Ser Tyr Leu Tyr Met Lys Asn Asn Gly Tyr Lys Met Pro Ser
                245                 250                 255
Tyr Val Asp Asp Lys Cys Val Gly Leu Asp Ala Trp Gly Asn Lys Phe
            260                 265                 270
Asp Ile Ser Phe Gly Ala Thr Pro Thr Glu Val Gly Thr Glu Cys Phe
        275                 280                 285
Glu Ser His Lys Asp Glu Thr Phe Ser Asn Ala Leu Phe Leu Leu Val
    290                 295                 300
Ser Lys Pro Ser Thr Ile Phe Lys Leu Pro Phe Asp Asp Gly Val Met
305                 310                 315                 320
Ser Gln Tyr Lys Glu Asn Tyr Phe His Val Tyr Lys Lys Leu His Val
                325                 330                 335
Ile Tyr Gly Glu Ser Asn Ile Leu Thr Thr Ile Thr Asn Ile Lys Asp
            340                 345                 350
Asn Ile Phe Lys Asn Ile Arg Phe Ile Ser Leu Leu Leu Phe Phe Ile
        355                 360                 365
Ala Ser Ile Phe Ile Arg Asn Asn Lys Ile Lys Ala Ser Leu Phe Val
    370                 375                 380
Val Ser Leu Phe Gly Ile Ser Gln Phe Tyr Val Ser Phe Phe Gly Glu
385                 390                 395                 400
Gly Tyr Arg Asp Leu Ser Lys His Leu Phe Gly Met Tyr Phe Ser Phe
                405                 410                 415
Asp Leu Cys Leu Tyr Ile Thr Val Val Phe Leu Ile Tyr Lys Ile Ile
            420                 425                 430
Gln Arg Asn Gln Asp Asn Ser Asp Val Lys His
        435                 440

<210> 277
<211> 82
<212> PRT
<213> E. Coli

<400> 277
Met Gly Ile Leu Ser Trp Ile Ile Phe Gly Leu Ile Ala Gly Ile Leu
1               5                   10                  15
Ala Lys Trp Ile Met Pro Gly Lys Asp Gly Gly Gly Phe Phe Met Thr
            20                  25                  30
Ile Leu Leu Gly Ile Val Gly Ala Val Val Gly Gly Trp Ile Ser Thr
        35                  40                  45
Leu Phe Gly Phe Gly Lys Val Asp Gly Phe Asn Phe Gly Ser Phe Val
    50                  55                  60

Val Ala Val Ile Gly Ala Ile Val Val Leu Phe Ile Tyr Arg Lys Ile
65                  70                  75                  80
Lys Ser

<210> 278
<211> 60
<212> PRT
<213> E. Coli

<400> 278
Met Gly Lys Ala Thr Tyr Thr Val Thr Val Thr Asn Asn Ser Asn Gly
1               5                   10                  15
Val Ser Val Asp Tyr Glu Thr Glu Thr Pro Met Thr Leu Leu Val Pro
            20                  25                  30
Glu Val Ala Ala Glu Val Ile Lys Asp Leu Val Asn Thr Val Arg Ser
        35                  40                  45
Tyr Asp Thr Glu Asn Glu His Asp Val Cys Gly Trp
    50                  55                  60

<210> 279
<211> 119
<212> PRT
<213> E. Coli

<400> 279
Met Leu Gln Ile Pro Gln Asn Tyr Ile His Thr Arg Ser Thr Pro Phe
1               5                   10                  15
Trp Asn Lys Gln Thr Ala Pro Ala Gly Ile Phe Glu Arg His Leu Asp
            20                  25                  30
Lys Gly Thr Arg Pro Gly Val Tyr Pro Arg Leu Ser Val Met His Gly
        35                  40                  45
Ala Val Lys Tyr Leu Gly Tyr Ala Asp Glu His Ser Ala Glu Pro Asp
    50                  55                  60
Gln Val Ile Leu Ile Glu Ala Gly Gln Phe Ala Val Phe Pro Pro Glu
65                  70                  75                  80
Lys Trp His Asn Ile Glu Ala Met Thr Asp Asp Thr Tyr Phe Asn Ile
                85                  90                  95
Asp Phe Phe Val Ala Pro Glu Val Leu Met Glu Gly Ala Gln Gln Arg
            100                 105                 110
Lys Val Ile His Asn Gly Lys
        115

<210> 280
<211> 246
<212> PRT
<213> E. Coli

<400> 280
Met Lys Phe Lys Val Ile Ala Leu Ala Ala Leu Met Gly Ile Ser Gly
1               5                   10                  15
Met Ala Ala Gln Ala Asn Glu Leu Pro Asp Gly Pro His Ile Val Thr
            20                  25                  30
Ser Gly Thr Ala Ser Val Asp Ala Val Pro Asp Ile Ala Thr Leu Ala
        35                  40                  45
Ile Glu Val Asn Val Ala Ala Lys Asp Ala Ala Thr Ala Lys Lys Gln
    50                  55                  60
Ala Asp Glu Arg Val Ala Gln Tyr Ile Ser Phe Leu Glu Leu Asn Gln

65                  70                  75                  80
Ile Ala Lys Lys Asp Ile Ser Ser Ala Asn Leu Arg Thr Gln Pro Asp
                85                  90                  95
Tyr Asp Tyr Gln Asp Gly Lys Ser Ile Leu Lys Gly Tyr Arg Ala Val
            100                 105                 110
Arg Thr Val Glu Val Thr Leu Arg Gln Leu Asp Lys Leu Asn Ser Leu
        115                 120                 125
Leu Asp Gly Ala Leu Lys Ala Gly Leu Asn Glu Ile Arg Ser Val Ser
    130                 135                 140
Leu Gly Val Ala Gln Pro Asp Ala Tyr Lys Asp Lys Ala Arg Lys Ala
145                 150                 155                 160
Ala Ile Asp Asn Ala Ile His Gln Ala Gln Glu Leu Ala Asn Gly Phe
                165                 170                 175
His Arg Lys Leu Gly Pro Val Tyr Ser Val Arg Tyr His Val Ser Asn
            180                 185                 190
Tyr Gln Pro Ser Pro Met Val Arg Met Met Lys Ala Asp Ala Ala Pro
        195                 200                 205
Val Ser Ala Gln Glu Thr Tyr Glu Gln Ala Ala Ile Gln Phe Asp Asp
    210                 215                 220
Gln Val Asp Val Val Phe Gln Leu Glu Pro Val Asp Gln Gln Pro Ala
225                 230                 235                 240
Lys Thr Pro Ala Ala Gln
                245

<210> 281
<211> 464
<212> PRT
<213> E. Coli

<400> 281
Met Leu Leu Leu Asp Ala Cys Ser Gln Met Cys Pro Ser Phe Arg Arg
 1               5                  10                  15
Phe Gln Thr Val Phe His Asn Ser Ser Ile Phe Leu Pro Tyr Trp Leu
            20                  25                  30
Ala Thr Leu Val Ser Phe Arg Glu Thr Phe Gln Glu Glu Lys Leu Leu
        35                  40                  45
Thr Met Lys Gly Ser Tyr Lys Ser Arg Trp Val Ile Val Ile Val Val
    50                  55                  60
Val Ile Ala Ala Ile Ala Ala Phe Trp Phe Trp Gln Gly Arg Asn Asp
65                  70                  75                  80
Ser Arg Ser Ala Ala Pro Gly Ala Thr Lys Gln Ala Gln Gln Ser Pro
                85                  90                  95
Ala Gly Gly Arg Arg Gly Met Arg Ser Gly Pro Leu Ala Pro Val Gln
            100                 105                 110
Ala Ala Thr Ala Val Glu Gln Ala Val Pro Arg Tyr Leu Thr Gly Leu
        115                 120                 125
Gly Thr Ile Thr Ala Ala Asn Thr Val Thr Val Arg Ser Arg Val Asp
    130                 135                 140
Gly Gln Leu Ile Ala Leu His Phe Gln Glu Gly Gln Gln Val Lys Ala
145                 150                 155                 160
Gly Asp Leu Leu Ala Glu Ile Asp Pro Ser Gln Phe Lys Val Ala Leu
                165                 170                 175
Ala Gln Ala Gln Gly Gln Leu Ala Lys Asp Lys Ala Thr Leu Ala Asn
            180                 185                 190
Ala Arg Arg Asp Leu Ala Arg Tyr Gln Gln Leu Ala Lys Thr Asn Leu
        195                 200                 205
Val Ser Arg Gln Glu Leu Asp Ala Gln Gln Ala Leu Val Ser Glu Thr
    210                 215                 220
Glu Gly Thr Ile Lys Ala Asp Glu Ala Ser Val Ala Ser Ala Gln Leu
225                 230                 235                 240

Gln Leu Asp Trp Ser Arg Ile Thr Ala Pro Val Asp Gly Arg Val Gly
                245                 250                 255
Leu Lys Gln Val Asp Val Gly Asn Gln Ile Ser Ser Gly Asp Thr Thr
            260                 265                 270
Gly Ile Val Val Ile Thr Gln Thr His Pro Ile Asp Leu Val Phe Thr
        275                 280                 285
Leu Pro Glu Ser Asp Ile Ala Thr Val Val Gln Ala Gln Lys Ala Gly
    290                 295                 300
Lys Pro Leu Val Val Glu Ala Trp Asp Arg Thr Asn Ser Lys Lys Leu
305                 310                 315                 320
Ser Glu Gly Thr Leu Leu Ser Leu Asp Asn Gln Ile Asp Ala Thr Thr
                325                 330                 335
Gly Thr Ile Lys Val Lys Ala Arg Phe Asn Asn Gln Asp Asp Ala Leu
            340                 345                 350
Phe Pro Asn Gln Phe Val Asn Ala Arg Met Leu Val Asp Thr Glu Gln
        355                 360                 365
Asn Ala Val Val Ile Pro Thr Ala Ala Leu Gln Met Gly Asn Glu Gly
    370                 375                 380
His Phe Val Trp Val Leu Asn Ser Glu Asn Lys Val Ser Lys His Leu
385                 390                 395                 400
Val Thr Pro Gly Ile Gln Asp Ser Gln Lys Val Val Ile Arg Ala Gly
                405                 410                 415
Ile Ser Ala Gly Asp Arg Val Val Thr Asp Gly Ile Asp Arg Leu Thr
            420                 425                 430
Glu Gly Ala Lys Val Glu Val Val Glu Ala Gln Ser Ala Thr Thr Pro
        435                 440                 445
Glu Glu Lys Ala Thr Ser Arg Glu Tyr Ala Lys Lys Gly Ala Arg Ser
    450                 455                 460

<210> 282
<211> 1040
<212> PRT
<213> E. Coli

<400> 282
Met Gln Val Leu Pro Pro Ser Ser Thr Gly Gly Pro Ser Arg Leu Phe
1               5                   10                  15
Ile Met Arg Pro Val Ala Thr Thr Leu Leu Met Val Ala Ile Leu Leu
            20                  25                  30
Ala Gly Ile Ile Gly Tyr Arg Ala Leu Pro Val Ser Ala Leu Pro Glu
        35                  40                  45
Val Asp Tyr Pro Thr Ile Gln Val Val Thr Leu Tyr Pro Gly Ala Ser
    50                  55                  60
Pro Asp Val Met Thr Ser Ala Val Thr Ala Pro Leu Glu Arg Gln Phe
65                  70                  75                  80
Gly Gln Met Ser Gly Leu Lys Gln Met Ser Ser Gln Ser Ser Gly Gly
                85                  90                  95
Ala Ser Val Ile Thr Leu Gln Phe Gln Leu Thr Leu Pro Leu Asp Val
            100                 105                 110
Ala Glu Gln Glu Val Gln Ala Ala Ile Asn Ala Ala Thr Asn Leu Leu
        115                 120                 125
Pro Ser Asp Leu Pro Asn Pro Pro Val Tyr Ser Lys Val Asn Pro Ala
    130                 135                 140
Asp Pro Pro Ile Met Thr Leu Ala Val Thr Ser Thr Ala Met Pro Met
145                 150                 155                 160
Thr Gln Val Glu Asp Met Val Glu Thr Arg Val Ala Gln Lys Ile Ser
                165                 170                 175
Gln Ile Ser Gly Val Gly Leu Val Thr Leu Ser Gly Gly Gln Arg Pro
            180                 185                 190
Ala Val Arg Val Lys Leu Asn Ala Gln Ala Ile Ala Ala Leu Gly Leu

195                 200                 205
Thr Ser Glu Thr Val Arg Thr Ala Ile Thr Gly Ala Asn Val Asn Ser
    210                 215                 220
Ala Lys Gly Ser Leu Asp Gly Pro Ser Arg Ala Val Thr Leu Ser Ala
225                 230                 235                 240
Asn Asp Gln Met Gln Ser Ala Glu Glu Tyr Arg Gln Leu Ile Ile Ala
                245                 250                 255
Tyr Gln Asn Gly Ala Pro Ile Arg Leu Gly Asp Val Ala Thr Val Glu
            260                 265                 270
Gln Gly Ala Glu Asn Ser Trp Leu Gly Ala Trp Ala Asn Lys Glu Gln
        275                 280                 285
Ala Ile Val Met Asn Val Gln Arg Gln Pro Gly Ala Asn Ile Ile Ser
    290                 295                 300
Thr Ala Asp Ser Ile Arg Gln Met Leu Pro Gln Leu Thr Glu Ser Leu
305                 310                 315                 320
Pro Lys Ser Val Lys Val Thr Val Leu Ser Asp Arg Thr Thr Asn Ile
                325                 330                 335
Arg Ala Ser Val Asp Asp Thr Gln Phe Glu Leu Met Met Ala Ile Ala
            340                 345                 350
Leu Val Val Met Ile Ile Tyr Leu Phe Leu Arg Asn Ile Pro Ala Thr
        355                 360                 365
Ile Ile Pro Gly Val Ala Val Pro Leu Ser Leu Ile Gly Thr Phe Ala
    370                 375                 380
Val Met Val Phe Leu Asp Phe Ser Ile Asn Asn Leu Thr Leu Met Ala
385                 390                 395                 400
Leu Thr Ile Ala Thr Gly Phe Val Val Asp Asp Ala Ile Val Val Ile
                405                 410                 415
Glu Asn Ile Ser Arg Tyr Ile Glu Lys Gly Glu Lys Pro Leu Ala Ala
            420                 425                 430
Ala Leu Lys Gly Ala Gly Glu Ile Gly Phe Thr Ile Ile Ser Leu Thr
        435                 440                 445
Phe Ser Leu Ile Ala Val Leu Ile Pro Leu Leu Phe Met Gly Asp Ile
    450                 455                 460
Val Gly Arg Leu Phe Arg Glu Phe Ala Ile Thr Leu Ala Val Ala Ile
465                 470                 475                 480
Leu Ile Ser Ala Val Val Ser Leu Thr Leu Thr Pro Met Met Cys Ala
                485                 490                 495
Arg Met Leu Ser Gln Glu Ser Leu Arg Lys Gln Asn Arg Phe Ser Arg
            500                 505                 510
Ala Ser Glu Lys Met Phe Asp Arg Ile Ile Ala Ala Tyr Gly Arg Gly
        515                 520                 525
Leu Ala Lys Val Leu Asn His Pro Trp Leu Thr Leu Ser Val Ala Leu
    530                 535                 540
Ser Thr Leu Leu Leu Ser Val Leu Leu Trp Val Phe Ile Pro Lys Gly
545                 550                 555                 560
Phe Phe Pro Val Gln Asp Asn Gly Ile Ile Gln Gly Thr Leu Gln Ala
                565                 570                 575
Pro Gln Ser Ser Ser Phe Ala Asn Met Ala Gln Arg Gln Arg Gln Val
            580                 585                 590
Ala Asp Val Ile Leu Gln Asp Pro Ala Val Gln Ser Leu Thr Ser Phe
        595                 600                 605
Val Gly Val Asp Gly Thr Asn Pro Ser Leu Asn Ser Ala Arg Leu Gln
    610                 615                 620
Ile Asn Leu Lys Pro Leu Asp Glu Arg Asp Asp Arg Val Gln Lys Val
625                 630                 635                 640
Ile Ala Arg Leu Gln Thr Ala Val Asp Lys Val Pro Gly Val Asp Leu
                645                 650                 655
Phe Leu Gln Pro Thr Gln Asp Leu Thr Ile Asp Thr Gln Val Ser Arg
            660                 665                 670
Thr Gln Tyr Gln Phe Thr Leu Gln Ala Thr Ser Leu Asp Ala Leu Ser
        675                 680                 685

Thr Trp Val Pro Gln Leu Met Glu Lys Leu Gln Gln Leu Pro Gln Leu
    690                 695                 700
Ser Asp Val Ser Ser Asp Trp Gln Asp Lys Gly Leu Val Ala Tyr Val
705                 710                 715                 720
Asn Val Asp Arg Asp Ser Ala Ser Arg Leu Gly Ile Ser Met Ala Asp
                725                 730                 735
Val Asp Asn Ala Leu Tyr Asn Ala Phe Gly Gln Arg Leu Ile Ser Thr
            740                 745                 750
Ile Tyr Thr Gln Ala Asn Gln Tyr Arg Val Val Leu Glu His Asn Thr
        755                 760                 765
Glu Asn Thr Pro Gly Leu Ala Ala Leu Asp Thr Ile Arg Leu Thr Ser
    770                 775                 780
Ser Asp Gly Gly Val Val Pro Leu Ser Ser Ile Ala Lys Ile Glu Gln
785                 790                 795                 800
Arg Phe Ala Pro Leu Ser Ile Asn His Leu Asp Gln Phe Pro Val Thr
                805                 810                 815
Thr Ile Ser Phe Asn Val Pro Asp Asn Tyr Ser Leu Gly Asp Ala Val
            820                 825                 830
Gln Ala Ile Met Asp Thr Glu Lys Thr Leu Asn Leu Pro Val Asp Ile
        835                 840                 845
Thr Thr Gln Phe Gln Gly Ser Thr Leu Ala Phe Gln Ser Ala Leu Gly
    850                 855                 860
Ser Thr Val Trp Leu Ile Val Ala Ala Val Val Ala Met Tyr Ile Val
865                 870                 875                 880
Leu Gly Ile Leu Tyr Glu Ser Phe Ile His Pro Ile Thr Ile Leu Ser
                885                 890                 895
Thr Leu Pro Thr Ala Gly Val Gly Ala Leu Leu Ala Leu Leu Ile Ala
            900                 905                 910
Gly Ser Glu Leu Asp Val Ile Ala Ile Ile Gly Ile Ile Leu Leu Ile
        915                 920                 925
Gly Ile Val Lys Lys Asn Ala Ile Met Met Ile Asp Phe Ala Leu Ala
    930                 935                 940
Ala Glu Arg Glu Gln Gly Met Ser Pro Arg Glu Ala Ile Tyr Gln Ala
945                 950                 955                 960
Cys Leu Leu Arg Phe Arg Pro Ile Leu Met Thr Thr Leu Ala Ala Leu
                965                 970                 975
Leu Gly Ala Leu Pro Leu Met Leu Ser Thr Gly Val Gly Ala Glu Leu
            980                 985                 990
Arg Arg Pro Leu Gly Ile Gly Met Val Gly Gly Leu Ile Val Ser Gln
        995                 1000                1005
Val Leu Thr Leu Phe Thr Thr Pro Val Ile Tyr Leu Leu Phe Asp Arg
    1010                1015                1020
Leu Ala Leu Trp Thr Lys Ser Arg Phe Ala Arg His Glu Glu Glu Ala
1025                1030                1035                1040

<210> 283
<211> 1025
<212> PRT
<213> E. Coli

<400> 283
Met Lys Phe Phe Ala Leu Phe Ile Tyr Arg Pro Val Ala Thr Ile Leu
 1               5                  10                  15
Leu Ser Val Ala Ile Thr Leu Cys Gly Ile Leu Gly Phe Arg Met Leu
            20                  25                  30
Pro Val Ala Pro Leu Pro Gln Val Asp Phe Pro Val Ile Ile Val Ser
        35                  40                  45
Ala Ser Leu Pro Gly Ala Ser Pro Glu Thr Met Ala Ser Ser Val Ala
    50                  55                  60
Thr Pro Leu Glu Arg Ser Leu Gly Arg Ile Ala Gly Val Ser Glu Met

65                  70                  75                  80
Thr Ser Ser Ser Ser Leu Gly Ser Thr Arg Ile Ile Leu Gln Phe Asp
                85                  90                  95
Phe Asp Arg Asp Ile Asn Gly Ala Ala Arg Asp Val Gln Ala Ala Ile
            100                 105                 110
Asn Ala Ala Gln Ser Leu Leu Pro Ser Gly Met Pro Ser Arg Pro Thr
        115                 120                 125
Tyr Arg Lys Ala Asn Pro Ser Asp Ala Pro Ile Met Ile Leu Thr Leu
    130                 135                 140
Thr Ser Asp Thr Tyr Ser Gln Gly Glu Leu Tyr Asp Phe Ala Ser Thr
145                 150                 155                 160
Gln Leu Ala Pro Thr Ile Ser Gln Ile Asp Gly Val Gly Asp Val Asp
                165                 170                 175
Val Gly Gly Ser Ser Leu Pro Ala Val Arg Val Gly Leu Asn Pro Gln
            180                 185                 190
Ala Leu Phe Asn Gln Gly Val Ser Leu Asp Asp Val Arg Thr Ala Val
        195                 200                 205
Ser Asn Ala Asn Val Arg Lys Pro Gln Gly Ala Leu Glu Asp Gly Thr
    210                 215                 220
His Arg Trp Gln Ile Gln Thr Asn Asp Glu Leu Lys Thr Ala Ala Glu
225                 230                 235                 240
Tyr Gln Pro Leu Ile Ile His Tyr Asn Asn Gly Gly Ala Val Arg Leu
                245                 250                 255
Gly Asp Val Ala Thr Val Thr Asp Ser Val Gln Asp Val Arg Asn Ala
            260                 265                 270
Gly Met Thr Asn Ala Lys Pro Ala Ile Leu Leu Met Ile Arg Lys Leu
        275                 280                 285
Pro Glu Ala Asn Ile Ile Gln Thr Val Asp Ser Ile Arg Ala Lys Leu
    290                 295                 300
Pro Glu Leu Gln Glu Thr Ile Pro Ala Ala Ile Asp Leu Gln Ile Ala
305                 310                 315                 320
Gln Asp Arg Ser Pro Thr Ile Arg Ala Ser Leu Glu Glu Val Glu Gln
                325                 330                 335
Thr Leu Ile Ile Ser Val Ala Leu Val Ile Leu Val Val Phe Leu Phe
            340                 345                 350
Leu Arg Ser Gly Arg Ala Thr Ile Ile Pro Ala Val Ser Val Pro Val
        355                 360                 365
Ser Leu Ile Gly Thr Phe Ala Ala Met Tyr Leu Cys Gly Phe Ser Leu
    370                 375                 380
Asn Asn Leu Ser Leu Met Ala Leu Thr Ile Ala Thr Gly Phe Val Val
385                 390                 395                 400
Asp Asp Ala Ile Val Val Leu Glu Asn Ile Ala Arg His Leu Glu Ala
                405                 410                 415
Gly Met Lys Pro Leu Gln Ala Ala Leu Gln Gly Thr Arg Glu Val Gly
            420                 425                 430
Phe Thr Val Leu Ser Met Ser Leu Ser Leu Val Ala Val Phe Leu Pro
        435                 440                 445
Leu Leu Leu Met Gly Gly Leu Pro Gly Arg Leu Leu Arg Glu Phe Ala
    450                 455                 460
Val Thr Leu Ser Val Ala Ile Gly Ile Ser Leu Leu Val Ser Leu Thr
465                 470                 475                 480
Leu Thr Pro Met Met Cys Gly Trp Met Leu Lys Ala Ser Lys Pro Arg
                485                 490                 495
Glu Gln Lys Arg Leu Arg Gly Phe Gly Arg Met Leu Val Ala Leu Gln
            500                 505                 510
Gln Gly Tyr Gly Lys Ser Leu Lys Trp Val Leu Asn His Thr Arg Leu
        515                 520                 525
Val Gly Val Val Leu Leu Gly Thr Ile Ala Leu Asn Ile Trp Leu Tyr
    530                 535                 540
Ile Ser Ile Pro Lys Thr Phe Phe Pro Glu Gln Asp Thr Gly Val Leu
545                 550                 555                 560

Met Gly Gly Ile Gln Ala Asp Gln Ser Ile Ser Phe Gln Ala Met Arg
                565                 570                 575
Gly Lys Leu Gln Asp Phe Met Lys Ile Ile Arg Asp Asp Pro Ala Val
            580                 585                 590
Asp Asn Val Thr Gly Phe Thr Gly Gly Ser Arg Val Asn Ser Gly Met
        595                 600                 605
Met Phe Ile Thr Leu Lys Pro Arg Asp Glu Arg Ser Glu Thr Ala Gln
    610                 615                 620
Gln Ile Ile Asp Arg Leu Arg Val Lys Leu Ala Lys Glu Pro Gly Ala
625                 630                 635                 640
Asn Leu Phe Leu Met Ala Val Gln Asp Ile Arg Val Gly Gly Arg Gln
                645                 650                 655
Ser Asn Ala Ser Tyr Gln Tyr Thr Leu Leu Ser Asp Asp Leu Ala Ala
            660                 665                 670
Leu Arg Glu Trp Glu Pro Lys Ile Arg Lys Lys Leu Ala Thr Leu Pro
        675                 680                 685
Glu Leu Ala Asp Val Asn Ser Asp Gln Gln Asp Asn Gly Ala Glu Met
    690                 695                 700
Asn Leu Val Tyr Asp Arg Asp Thr Met Ala Arg Leu Gly Ile Asp Val
705                 710                 715                 720
Gln Ala Ala Asn Ser Leu Leu Asn Asn Ala Phe Gly Gln Arg Gln Ile
                725                 730                 735
Ser Thr Ile Tyr Gln Pro Met Asn Gln Tyr Lys Val Val Met Glu Val
            740                 745                 750
Asp Pro Arg Tyr Thr Gln Asp Ile Ser Ala Leu Glu Lys Met Phe Val
        755                 760                 765
Ile Asn Asn Glu Gly Lys Ala Ile Pro Leu Ser Tyr Phe Ala Lys Trp
    770                 775                 780
Gln Pro Ala Asn Ala Pro Leu Ser Val Asn His Gln Gly Leu Ser Ala
785                 790                 795                 800
Ala Ser Thr Ile Ser Phe Asn Leu Pro Thr Gly Lys Ser Leu Ser Asp
                805                 810                 815
Ala Ser Ala Ala Ile Asp Arg Ala Met Thr Gln Leu Gly Val Pro Ser
            820                 825                 830
Thr Val Arg Gly Ser Phe Ala Gly Thr Ala Gln Val Phe Gln Glu Thr
        835                 840                 845
Met Asn Ser Gln Val Ile Leu Ile Ile Ala Ala Ile Ala Thr Val Tyr
    850                 855                 860
Ile Val Leu Gly Ile Leu Tyr Glu Ser Tyr Val His Pro Leu Thr Ile
865                 870                 875                 880
Leu Ser Thr Leu Pro Ser Ala Gly Val Gly Ala Leu Leu Ala Leu Glu
                885                 890                 895
Leu Phe Asn Ala Pro Phe Ser Leu Ile Ala Leu Ile Gly Ile Met Leu
            900                 905                 910
Leu Ile Gly Ile Val Lys Lys Asn Ala Ile Met Met Val Asp Phe Ala
        915                 920                 925
Leu Glu Ala Gln Arg His Gly Asn Leu Thr Pro Gln Glu Ala Ile Phe
    930                 935                 940
Gln Ala Cys Leu Leu Arg Phe Arg Pro Ile Met Met Thr Thr Leu Ala
945                 950                 955                 960
Ala Leu Phe Gly Ala Leu Pro Leu Val Leu Ser Gly Gly Asp Gly Ser
                965                 970                 975
Glu Leu Arg Gln Pro Leu Gly Ile Thr Ile Val Gly Gly Leu Val Met
            980                 985                 990
Ser Gln Leu Leu Thr Leu Tyr Thr Thr Pro Val Val Tyr Leu Phe Phe
        995                 1000                1005
Asp Arg Leu Arg Leu Arg Phe Ser Arg Lys Pro Lys Gln Thr Val Thr
    1010                1015                1020
Glu
1025

<210> 284
<211> 471
<212> PRT
<213> E. Coli

<400> 284
Met Thr Asp Leu Pro Asp Ser Thr Arg Trp Gln Leu Trp Ile Val Ala
1               5                   10                  15
Phe Gly Phe Phe Met Gln Ser Leu Asp Thr Thr Ile Val Asn Thr Ala
            20                  25                  30
Leu Pro Ser Met Ala Gln Ser Leu Gly Glu Ser Pro Leu His Met His
        35                  40                  45
Met Val Ile Val Ser Tyr Val Leu Thr Val Ala Val Met Leu Pro Ala
    50                  55                  60
Ser Gly Trp Leu Ala Asp Lys Val Gly Val Arg Asn Ile Phe Phe Thr
65                  70                  75                  80
Ala Ile Val Leu Phe Thr Leu Gly Ser Leu Phe Cys Ala Leu Ser Gly
                85                  90                  95
Thr Leu Asn Glu Leu Leu Leu Ala Arg Ala Leu Gln Gly Val Gly Gly
            100                 105                 110
Ala Met Met Val Pro Val Gly Arg Leu Thr Val Met Lys Ile Val Pro
        115                 120                 125
Arg Glu Gln Tyr Met Ala Ala Met Thr Phe Val Thr Leu Pro Gly Gln
    130                 135                 140
Val Gly Pro Leu Leu Gly Pro Ala Leu Gly Gly Leu Leu Val Glu Tyr
145                 150                 155                 160
Ala Ser Trp His Trp Ile Phe Leu Ile Asn Ile Pro Val Gly Ile Ile
                165                 170                 175
Gly Ala Ile Ala Thr Leu Leu Leu Met Pro Asn Tyr Thr Met Gln Thr
            180                 185                 190
Arg Arg Phe Asp Leu Ser Gly Phe Leu Leu Leu Ala Val Gly Met Ala
        195                 200                 205
Val Leu Thr Leu Ala Leu Asp Gly Ser Lys Gly Thr Gly Leu Ser Pro
    210                 215                 220
Leu Thr Ile Ala Gly Leu Val Ala Val Gly Val Val Ala Leu Val Leu
225                 230                 235                 240
Tyr Leu Leu His Ala Arg Asn Asn Asn Arg Ala Leu Phe Ser Leu Lys
                245                 250                 255
Leu Phe Arg Thr Arg Thr Phe Ser Leu Gly Leu Ala Gly Ser Phe Ala
            260                 265                 270
Gly Arg Ile Gly Ser Gly Met Leu Pro Phe Met Thr Pro Val Phe Leu
        275                 280                 285
Gln Ile Gly Leu Gly Phe Ser Pro Phe His Ala Gly Leu Met Met Ile
    290                 295                 300
Pro Met Val Leu Gly Ser Met Gly Met Lys Arg Ile Val Val Gln Val
305                 310                 315                 320
Val Asn Arg Phe Gly Tyr Arg Arg Val Leu Val Ala Thr Thr Leu Gly
                325                 330                 335
Leu Ser Leu Val Thr Leu Leu Phe Met Thr Thr Ala Leu Leu Gly Trp
            340                 345                 350
Tyr Tyr Val Leu Pro Phe Val Leu Phe Leu Gln Gly Met Val Asn Ser
        355                 360                 365
Thr Arg Phe Ser Ser Met Asn Thr Leu Thr Leu Lys Asp Leu Pro Asp
    370                 375                 380
Asn Leu Ala Ser Ser Gly Asn Ser Leu Leu Ser Met Ile Met Gln Leu
385                 390                 395                 400
Ser Met Ser Ile Gly Val Thr Ile Ala Gly Leu Leu Leu Gly Leu Phe
                405                 410                 415
Gly Ser Gln His Val Ser Val Asp Ser Gly Thr Thr Gln Thr Val Phe
            420                 425                 430

Met Tyr Thr Trp Leu Ser Met Ala Leu Ile Ile Ala Leu Pro Ala Phe
        435                 440                 445
Ile Phe Ala Arg Val Pro Asn Asp Thr His Gln Asn Val Ala Ile Ser
    450                 455                 460
Arg Arg Lys Arg Ser Ala Gln
465                 470

<210> 285
<211> 344
<212> PRT
<213> E. Coli

<400> 285
Met Glu Ile Arg Ile Met Leu Phe Ile Leu Met Met Met Val Met Pro
 1               5                  10                  15
Val Ser Tyr Ala Ala Cys Tyr Ser Glu Leu Ser Val Gln His Asn Leu
            20                  25                  30
Val Val Gln Gly Asp Phe Ala Leu Thr Gln Thr Gln Met Ala Thr Tyr
        35                  40                  45
Glu His Asn Phe Asn Asp Ser Ser Cys Val Ser Thr Asn Thr Ile Thr
    50                  55                  60
Pro Met Ser Pro Ser Asp Ile Ile Val Gly Leu Tyr Asn Asp Thr Ile
65                  70                  75                  80
Lys Leu Asn Leu His Phe Glu Trp Thr Asn Lys Asn Asn Ile Thr Leu
                85                  90                  95
Ser Asn Asn Gln Thr Ser Phe Thr Ser Gly Tyr Ser Val Thr Val Thr
            100                 105                 110
Pro Ala Ala Ser Asn Ala Lys Val Asn Val Ser Ala Gly Gly Gly Gly
        115                 120                 125
Ser Val Met Ile Asn Gly Val Ala Thr Leu Ser Ser Ala Ser Ser Ser
    130                 135                 140
Thr Arg Gly Ser Ala Ala Val Gln Phe Leu Leu Cys Leu Leu Gly Gly
145                 150                 155                 160
Lys Ser Trp Asp Ala Cys Val Asn Ser Tyr Arg Asn Ala Leu Ala Gln
                165                 170                 175
Asn Ala Gly Val Tyr Ser Phe Asn Leu Thr Leu Ser Tyr Asn Pro Ile
            180                 185                 190
Thr Thr Thr Cys Lys Pro Asp Asp Leu Leu Ile Thr Leu Asp Ser Ile
        195                 200                 205
Pro Val Ser Gln Leu Pro Ala Thr Gly Asn Lys Ala Thr Ile Asn Ser
    210                 215                 220
Lys Gln Gly Asp Ile Ile Leu Arg Cys Lys Asn Leu Leu Gly Gln Gln
225                 230                 235                 240
Asn Gln Thr Ser Arg Lys Met Gln Val Tyr Leu Ser Ser Ser Asp Leu
                245                 250                 255
Leu Thr Asn Ser Asn Thr Ile Leu Lys Gly Ala Glu Asp Asn Gly Val
            260                 265                 270
Gly Phe Ile Leu Glu Ser Asn Gly Ser Pro Val Thr Leu Leu Asn Ile
        275                 280                 285
Thr Asn Ser Ser Lys Gly Tyr Thr Asn Leu Lys Glu Val Ala Ala Lys
    290                 295                 300
Ser Lys Leu Thr Asp Thr Thr Val Ser Ile Pro Ile Thr Ala Ser Tyr
305                 310                 315                 320
Tyr Val Tyr Asp Thr Asn Lys Val Lys Ser Gly Ala Leu Glu Ala Thr
                325                 330                 335
Ala Leu Ile Asn Val Lys Tyr Asp
            340

<210> 286

<211> 826
<212> PRT
<213> E. Coli

<400> 286
Met Leu Arg Met Thr Pro Leu Ala Ser Ala Ile Val Ala Leu Leu Leu
1               5                   10                  15
Gly Ile Glu Ala Tyr Ala Ala Glu Glu Thr Phe Asp Thr His Phe Met
            20                  25                  30
Ile Gly Gly Met Lys Asp Gln Gln Val Ala Asn Ile Arg Leu Asp Asp
        35                  40                  45
Asn Gln Pro Leu Pro Gly Gln Tyr Asp Ile Asp Ile Tyr Val Asn Lys
    50                  55                  60
Gln Trp Arg Gly Lys Tyr Glu Ile Ile Val Lys Asp Asn Pro Gln Glu
65                  70                  75                  80
Thr Cys Leu Ser Arg Glu Val Ile Lys Arg Leu Gly Ile Asn Ser Asp
                85                  90                  95
Asn Phe Ala Ser Gly Lys Gln Cys Leu Thr Phe Glu Gln Leu Val Gln
            100                 105                 110
Gly Gly Ser Tyr Thr Trp Asp Ile Gly Val Phe Arg Leu Asp Phe Ser
        115                 120                 125
Val Pro Gln Ala Trp Val Glu Glu Leu Glu Ser Gly Tyr Val Pro Pro
    130                 135                 140
Glu Asn Trp Glu Arg Gly Ile Asn Ala Phe Tyr Thr Ser Tyr Tyr Leu
145                 150                 155                 160
Ser Gln Tyr Tyr Ser Asp Tyr Lys Ala Ser Gly Asn Asn Lys Ser Thr
                165                 170                 175
Tyr Val Arg Phe Asn Ser Gly Leu Asn Leu Leu Gly Trp Gln Leu His
            180                 185                 190
Ser Asp Ala Ser Phe Ser Lys Thr Asn Asn Asn Pro Gly Val Trp Lys
        195                 200                 205
Ser Asn Thr Leu Tyr Leu Glu Arg Gly Phe Ala Gln Leu Leu Gly Thr
    210                 215                 220
Leu Arg Val Gly Asp Met Tyr Thr Ser Ser Asp Ile Phe Asp Ser Val
225                 230                 235                 240
Arg Phe Arg Gly Val Arg Leu Phe Arg Asp Met Gln Met Leu Pro Asn
                245                 250                 255
Ser Lys Gln Asn Phe Thr Pro Arg Val Gln Gly Ile Ala Gln Ser Asn
            260                 265                 270
Ala Leu Val Thr Ile Glu Gln Asn Gly Phe Val Val Tyr Gln Lys Glu
        275                 280                 285
Val Pro Pro Gly Pro Phe Ala Ile Thr Asp Leu Gln Leu Ala Gly Gly
    290                 295                 300
Gly Ala Asp Leu Asp Val Ser Val Lys Glu Ala Asp Gly Ser Val Thr
305                 310                 315                 320
Thr Tyr Leu Val Pro Tyr Ala Ala Val Pro Asn Met Leu Gln Pro Gly
                325                 330                 335
Val Ser Lys Tyr Asp Leu Ala Ala Gly Arg Ser His Ile Glu Gly Ala
            340                 345                 350
Ser Lys Gln Ser Asp Phe Val Gln Ala Gly Tyr Gln Tyr Gly Phe Asn
        355                 360                 365
Asn Leu Leu Thr Leu Tyr Gly Gly Ser Met Val Ala Asn Asn Tyr Tyr
    370                 375                 380
Ala Phe Thr Leu Gly Ala Gly Trp Asn Thr Arg Ile Gly Ala Ile Ser
385                 390                 395                 400
Val Asp Ala Thr Lys Ser His Ser Lys Gln Asp Asn Gly Asp Val Phe
                405                 410                 415
Asp Gly Gln Ser Tyr Gln Ile Ala Tyr Asn Lys Phe Val Ser Gln Thr
            420                 425                 430
Ser Thr Arg Phe Gly Leu Ala Ala Trp Arg Tyr Ser Ser Arg Asp Tyr
        435                 440                 445

Arg Thr Phe Asn Asp His Val Trp Ala Asn Asn Lys Asp Asn Tyr Arg
    450                 455                 460
Arg Asp Glu Asn Asp Val Tyr Asp Ile Ala Asp Tyr Tyr Gln Asn Asp
465                 470                 475                 480
Phe Gly Arg Lys Asn Ser Phe Ser Ala Asn Met Ser Gln Ser Leu Pro
                485                 490                 495
Glu Gly Trp Gly Ser Val Ser Leu Ser Thr Leu Trp Arg Asp Tyr Trp
            500                 505                 510
Gly Arg Ser Gly Ser Ser Lys Asp Tyr Gln Leu Ser Tyr Ser Asn Asn
        515                 520                 525
Leu Arg Arg Ile Ser Tyr Thr Leu Ala Ala Ser Gln Ala Tyr Asp Glu
    530                 535                 540
Asn His His Glu Glu Lys Arg Phe Asn Ile Phe Ile Ser Ile Pro Phe
545                 550                 555                 560
Asp Trp Gly Asp Asp Val Ser Thr Pro Arg Arg Gln Ile Tyr Met Ser
                565                 570                 575
Asn Ser Thr Thr Phe Asp Asp Gln Gly Phe Ala Ser Asn Asn Thr Gly
            580                 585                 590
Leu Ser Gly Thr Val Gly Ser Arg Asp Gln Phe Asn Tyr Gly Val Asn
        595                 600                 605
Leu Ser His Gln His Gln Gly Asn Glu Thr Thr Ala Gly Ala Asn Leu
    610                 615                 620
Thr Trp Asn Ala Pro Val Ala Thr Val Asn Gly Ser Tyr Ser Gln Ser
625                 630                 635                 640
Ser Thr Tyr Arg Gln Ala Gly Ala Ser Val Ser Gly Gly Ile Val Ala
                645                 650                 655
Trp Ser Gly Gly Val Asn Leu Ala Asn Arg Leu Ser Glu Thr Phe Ala
            660                 665                 670
Val Met Asn Ala Pro Gly Ile Lys Asp Ala Tyr Val Asn Gly Gln Lys
        675                 680                 685
Tyr Arg Thr Thr Asn Arg Asn Gly Val Val Ile Tyr Asp Gly Met Thr
    690                 695                 700
Pro Tyr Arg Glu Asn His Leu Met Leu Asp Val Ser Gln Ser Asp Ser
705                 710                 715                 720
Glu Ala Glu Leu Arg Gly Asn Arg Lys Ile Ala Ala Pro Tyr Arg Gly
                725                 730                 735
Ala Val Val Leu Val Asn Phe Asp Thr Asp Gln Arg Lys Pro Trp Phe
            740                 745                 750
Ile Lys Ala Leu Arg Ala Asp Gly Gln Ser Leu Thr Phe Gly Tyr Glu
        755                 760                 765
Val Asn Asp Ile His Gly His Asn Ile Gly Val Val Gly Gln Gly Ser
    770                 775                 780
Gln Leu Phe Ile Arg Thr Asn Glu Val Pro Pro Ser Val Asn Val Ala
785                 790                 795                 800
Ile Asp Lys Gln Gln Gly Leu Ser Cys Thr Ile Thr Phe Gly Lys Glu
                805                 810                 815
Ile Asp Glu Ser Arg Asn Tyr Ile Cys Gln
            820                 825

<210> 287
<211> 239
<212> PRT
<213> E. Coli

<400> 287
Met Ala Ala Ile Pro Trp Arg Pro Phe Asn Leu Arg Gly Ile Lys Met
 1               5                  10                  15
Lys Gly Leu Leu Ser Leu Leu Ile Phe Ser Met Val Leu Pro Ala His
            20                  25                  30
Ala Gly Ile Val Ile Tyr Gly Thr Arg Ile Ile Tyr Pro Ala Glu Asn

35                  40                  45
Lys Glu Val Met Val Gln Leu Met Asn Gln Gly Asn Arg Ser Ser Leu
    50                  55                  60
Leu Gln Ala Trp Ile Asp Asp Gly Asp Thr Ser Leu Pro Pro Glu Lys
65                  70                  75                  80
Ile Gln Val Pro Phe Met Leu Thr Pro Pro Val Ala Lys Ile Gly Ala
                85                  90                  95
Asn Ser Gly Gln Gln Val Lys Ile Lys Ile Met Pro Asn Lys Leu Pro
            100                 105                 110
Thr Asn Lys Glu Ser Ile Phe Tyr Leu Asn Val Leu Asp Ile Pro Pro
        115                 120                 125
Asn Ser Pro Glu Gln Glu Gly Lys Asn Ala Leu Lys Phe Ala Met Gln
    130                 135                 140
Asn Arg Ile Lys Leu Phe Tyr Arg Pro Ala Gly Ile Ala Pro Val Asn
145                 150                 155                 160
Lys Ala Thr Phe Lys Lys Leu Leu Val Asn Arg Ser Gly Asn Gly Leu
                165                 170                 175
Val Ile Lys Asn Asp Ser Ala Asn Trp Val Thr Ile Ser Asp Val Lys
            180                 185                 190
Ala Asn Asn Val Lys Val Asn Tyr Glu Thr Ile Met Ile Ala Pro Leu
        195                 200                 205
Glu Ser Gln Ser Val Asn Val Lys Ser Asn Asn Ala Asn Asn Trp His
    210                 215                 220
Leu Thr Ile Ile Asp Asp His Gly Asn Tyr Ile Ser Asp Lys Ile
225                 230                 235

<210> 288
<211> 180
<212> PRT
<213> E. Coli

<400> 288
Met Lys Arg Ser Ile Ile Ala Ala Ala Val Phe Ser Ser Phe Phe Met
1               5                   10                  15
Ser Ala Gly Val Phe Ala Ala Asp Val Asp Thr Gly Thr Leu Thr Ile
            20                  25                  30
Lys Gly Asn Ile Ala Glu Ser Pro Cys Lys Phe Glu Ala Gly Gly Asp
        35                  40                  45
Ser Val Ser Ile Asn Met Pro Thr Val Pro Thr Ser Val Phe Glu Gly
    50                  55                  60
Lys Ala Lys Tyr Ser Thr Tyr Asp Asp Ala Val Gly Val Thr Ser Ser
65                  70                  75                  80
Met Leu Lys Ile Ser Cys Pro Lys Glu Val Ala Gly Val Lys Leu Ser
                85                  90                  95
Leu Ile Thr Asn Asp Lys Ile Thr Gly Asn Asp Lys Ala Ile Ala Ser
            100                 105                 110
Ser Asn Asp Thr Val Gly Tyr Tyr Leu Tyr Leu Gly Asp Asn Ser Asp
        115                 120                 125
Val Leu Asp Val Ser Ala Pro Phe Asn Ile Glu Ser Tyr Lys Thr Ala
    130                 135                 140
Glu Gly Gln Tyr Ala Ile Pro Phe Lys Ala Lys Tyr Leu Lys Leu Thr
145                 150                 155                 160
Asp Asn Ser Val Gln Ser Gly Asp Val Leu Ser Ser Leu Val Met Arg
                165                 170                 175
Val Ala Gln Asp
            180

<210> 289

<211> 112
<212> PRT
<213> E. Coli

<400> 289
Met Ser Ser Glu Arg Asp Leu Val Asn Phe Leu Gly Asp Phe Ser Met
1               5                   10                  15
Asp Val Ala Lys Ala Val Ile Ala Gly Gly Val Ala Thr Ala Ile Gly
            20                  25                  30
Ser Leu Ala Ser Phe Ala Cys Val Ser Phe Gly Phe Pro Val Ile Leu
        35                  40                  45
Val Gly Gly Ala Ile Leu Leu Thr Gly Ile Val Cys Thr Val Val Leu
    50                  55                  60
Asn Glu Ile Asp Ala Gln Cys His Leu Ser Glu Lys Leu Lys Tyr Ala
65                  70                  75                  80
Ile Arg Asp Gly Leu Lys Arg Gln Gln Glu Leu Asp Lys Trp Lys Arg
                85                  90                  95
Glu Asn Met Thr Pro Phe Met Tyr Val Leu Asn Thr Pro Pro Val Ile
            100                 105                 110

<210> 290
<211> 193
<212> PRT
<213> E. Coli

<400> 290
Met Thr Asp Tyr Leu Leu Leu Phe Val Gly Thr Val Leu Val Asn Asn
1               5                   10                  15
Phe Val Leu Val Lys Phe Leu Gly Leu Cys Pro Phe Met Gly Val Ser
            20                  25                  30
Lys Lys Leu Glu Thr Ala Met Gly Met Gly Leu Ala Thr Thr Phe Val
        35                  40                  45
Met Thr Leu Ala Ser Ile Cys Ala Trp Leu Ile Asp Thr Trp Ile Leu
    50                  55                  60
Ile Pro Leu Asn Leu Ile Tyr Leu Arg Thr Leu Ala Phe Ile Leu Val
65                  70                  75                  80
Ile Ala Val Val Val Gln Phe Thr Glu Met Val Val Arg Lys Thr Ser
                85                  90                  95
Pro Val Leu Tyr Arg Leu Leu Gly Ile Phe Leu Pro Leu Ile Thr Thr
            100                 105                 110
Asn Cys Ala Val Leu Gly Val Ala Leu Leu Asn Ile Asn Leu Gly His
        115                 120                 125
Asn Phe Leu Gln Ser Ala Leu Tyr Gly Phe Ser Ala Ala Val Gly Phe
    130                 135                 140
Ser Leu Val Met Val Leu Phe Ala Ala Ile Arg Glu Arg Leu Ala Val
145                 150                 155                 160
Ala Asp Val Pro Ala Pro Phe Arg Gly Asn Ala Ile Ala Leu Ile Thr
                165                 170                 175
Ala Gly Leu Met Ser Leu Ala Phe Met Gly Phe Ser Gly Leu Val Lys
            180                 185                 190
Leu

<210> 291
<211> 192
<212> PRT
<213> E. Coli

<400> 291
Met Asn Ala Ile Trp Ile Ala Val Ala Ala Val Ser Leu Leu Gly Leu
1               5                   10                  15
Ala Phe Gly Ala Ile Leu Gly Tyr Ala Ser Arg Arg Phe Ala Val Glu
            20                  25                  30
Asp Asp Pro Val Val Glu Lys Ile Asp Glu Ile Leu Pro Gln Ser Gln
        35                  40                  45
Cys Gly Gln Cys Gly Tyr Pro Gly Cys Arg Pro Tyr Ala Glu Ala Ile
    50                  55                  60
Ser Cys Asn Gly Glu Lys Ile Asn Arg Cys Ala Pro Gly Gly Glu Ala
65                  70                  75                  80
Val Met Leu Lys Ile Ala Glu Leu Leu Asn Val Glu Pro Gln Pro Leu
                85                  90                  95
Asp Gly Glu Ala Gln Glu Ile Thr Pro Ala Arg Met Val Ala Val Ile
            100                 105                 110
Asp Glu Asn Asn Cys Ile Gly Cys Thr Lys Cys Ile Gln Ala Cys Pro
        115                 120                 125
Val Asp Ala Ile Val Gly Ala Thr Arg Ala Met His Thr Val Met Ser
    130                 135                 140
Asp Leu Cys Thr Gly Cys Asn Leu Cys Val Asp Pro Cys Pro Thr His
145                 150                 155                 160
Cys Ile Ser Leu Gln Pro Val Ala Glu Thr Pro Asp Ser Trp Lys Trp
                165                 170                 175
Asp Leu Asn Thr Ile Pro Val Arg Ile Ile Pro Val Glu His His Ala
            180                 185                 190

<210> 292
<211> 740
<212> PRT
<213> E. Coli

<400> 292
Met Leu Lys Leu Phe Ser Ala Phe Arg Lys Asn Lys Ile Trp Asp Phe
1               5                   10                  15
Asn Gly Gly Ile His Pro Pro Glu Met Lys Thr Gln Ser Asn Gly Thr
            20                  25                  30
Pro Leu Arg Gln Val Pro Leu Ala Gln Arg Phe Val Ile Pro Leu Lys
        35                  40                  45
Gln His Ile Gly Ala Glu Gly Glu Leu Cys Val Ser Val Gly Asp Lys
    50                  55                  60
Val Leu Arg Gly Gln Pro Leu Thr Arg Gly Arg Gly Lys Met Leu Pro
65                  70                  75                  80
Val His Ala Pro Thr Ser Gly Thr Val Thr Ala Ile Ala Pro His Ser
                85                  90                  95
Thr Ala His Pro Ser Ala Leu Ala Glu Leu Ser Val Ile Ile Asp Ala
            100                 105                 110
Asp Gly Glu Asp Cys Trp Ile Pro Arg Asp Gly Trp Ala Asp Tyr Arg
        115                 120                 125
Thr Arg Ser Arg Glu Glu Leu Ile Glu Arg Ile His Gln Phe Gly Val
    130                 135                 140
Ala Gly Leu Gly Gly Ala Gly Phe Pro Thr Gly Val Lys Leu Gln Gly
145                 150                 155                 160
Gly Gly Asp Lys Ile Glu Thr Leu Ile Ile Asn Ala Ala Glu Cys Glu
                165                 170                 175
Pro Tyr Ile Thr Ala Asp Asp Arg Leu Met Gln Asp Cys Ala Ala Gln
            180                 185                 190
Val Val Glu Gly Ile Arg Ile Leu Ala His Ile Leu Gln Pro Arg Glu
        195                 200                 205
Ile Leu Ile Gly Ile Glu Asp Asn Lys Pro Gln Ala Ile Ser Met Leu
    210                 215                 220

Arg Ala Val Leu Ala Asp Ser Asn Asp Ile Ser Leu Arg Val Ile Pro
225                 230                 235                 240
Thr Lys Tyr Pro Ser Gly Gly Ala Lys Gln Leu Thr Tyr Ile Leu Thr
                245                 250                 255
Gly Lys Gln Val Pro His Gly Gly Arg Ser Ser Asp Ile Gly Val Leu
            260                 265                 270
Met Gln Asn Val Gly Thr Ala Tyr Ala Val Lys Arg Ala Val Ile Asp
        275                 280                 285
Gly Glu Pro Ile Thr Glu Arg Val Val Thr Leu Thr Gly Glu Ala Ile
    290                 295                 300
Ala Arg Pro Gly Asn Val Trp Ala Arg Leu Gly Thr Pro Val Arg His
305                 310                 315                 320
Leu Leu Asn Asp Ala Gly Phe Cys Pro Ser Ala Asp Gln Met Val Ile
                325                 330                 335
Met Gly Gly Pro Leu Met Gly Phe Thr Leu Pro Trp Leu Asp Val Pro
            340                 345                 350
Val Val Lys Ile Thr Asn Cys Leu Leu Ala Pro Ser Ala Asn Glu Leu
        355                 360                 365
Gly Glu Pro Gln Glu Glu Gln Ser Cys Ile Arg Cys Ser Ala Cys Ala
    370                 375                 380
Asp Ala Cys Pro Ala Asp Leu Leu Pro Gln Gln Leu Tyr Trp Phe Ser
385                 390                 395                 400
Lys Gly Gln Gln His Asp Lys Ala Thr Thr His Asn Ile Ala Asp Cys
                405                 410                 415
Ile Glu Cys Gly Ala Cys Ala Trp Val Cys Pro Ser Asn Ile Pro Leu
            420                 425                 430
Val Gln Tyr Phe Arg Gln Glu Lys Ala Glu Ile Ala Ala Ile Arg Gln
        435                 440                 445
Glu Glu Lys Arg Ala Ala Glu Ala Lys Ala Arg Phe Glu Ala Arg Gln
    450                 455                 460
Ala Arg Leu Glu Arg Glu Lys Ala Ala Arg Leu Glu Arg His Lys Ser
465                 470                 475                 480
Ala Ala Val Gln Pro Ala Ala Lys Asp Lys Asp Ala Ile Ala Ala Ala
                485                 490                 495
Leu Ala Arg Val Lys Glu Lys Gln Ala Gln Ala Thr Gln Pro Ile Val
            500                 505                 510
Ile Lys Ala Gly Glu Arg Pro Asp Asn Ser Ala Ile Ile Ala Ala Arg
        515                 520                 525
Glu Ala Arg Lys Ala Gln Ala Arg Ala Lys Gln Ala Glu Leu Gln Gln
    530                 535                 540
Thr Asn Asp Ala Ala Thr Val Ala Asp Pro Arg Lys Thr Ala Val Glu
545                 550                 555                 560
Ala Ala Ile Ala Arg Ala Lys Ala Arg Lys Leu Glu Gln Gln Gln Ala
                565                 570                 575
Asn Ala Glu Pro Glu Gln Gln Val Asp Pro Arg Lys Ala Ala Val Glu
            580                 585                 590
Ala Ala Ile Ala Arg Ala Lys Ala Arg Lys Leu Glu Gln Gln Gln Ala
        595                 600                 605
Asn Ala Glu Pro Glu Glu Gln Val Asp Pro Arg Lys Ala Ala Val Glu
    610                 615                 620
Ala Ala Ile Ala Arg Ala Lys Ala Arg Lys Leu Glu Gln Gln Gln Ala
625                 630                 635                 640
Asn Ala Glu Pro Glu Gln Gln Val Asp Pro Arg Lys Ala Ala Val Glu
                645                 650                 655
Ala Ala Ile Ala Arg Ala Lys Ala Arg Lys Arg Glu Gln Gln Pro Ala
            660                 665                 670
Asn Ala Glu Pro Glu Glu Gln Val Asp Pro Arg Lys Ala Ala Val Glu
        675                 680                 685
Ala Ala Ile Ala Arg Ala Lys Ala Arg Lys Leu Glu Gln Gln Gln Ala
    690                 695                 700
Asn Ala Val Pro Glu Glu Gln Val Asp Pro Arg Lys Ala Ala Val Ala

705                 710                 715                 720
Ala Ala Ile Ala Arg Ala Gln Ala Lys Lys Ala Ala Gln Gln Lys Val
                725                 730                 735
Val Asn Glu Asp
            740

<210> 293
<211> 352
<212> PRT
<213> E. Coli

<400> 293
Met Val Phe Arg Ile Ala Ser Ser Pro Tyr Thr His Asn Gln Arg Gln
1               5                   10                  15
Thr Ser Arg Ile Met Leu Leu Val Leu Leu Ala Ala Val Pro Gly Ile
            20                  25                  30
Ala Ala Gln Leu Trp Phe Phe Gly Trp Gly Thr Leu Val Gln Ile Leu
        35                  40                  45
Leu Ala Ser Val Ser Ala Leu Leu Ala Glu Ala Leu Val Leu Lys Leu
    50                  55                  60
Arg Lys Gln Ser Val Ala Ala Thr Leu Lys Asp Asn Ser Ala Leu Leu
65                  70                  75                  80
Thr Gly Leu Leu Leu Ala Val Ser Ile Pro Pro Leu Ala Pro Trp Trp
                85                  90                  95
Met Val Val Leu Gly Thr Val Phe Ala Val Ile Ile Ala Lys Gln Leu
            100                 105                 110
Tyr Gly Gly Leu Gly Gln Asn Pro Phe Asn Pro Ala Met Ile Gly Tyr
        115                 120                 125
Val Val Leu Leu Ile Ser Phe Pro Val Gln Met Thr Ser Trp Leu Pro
    130                 135                 140
Pro His Glu Ile Ala Val Asn Ile Pro Gly Phe Ile Asp Ala Ile Gln
145                 150                 155                 160
Val Ile Phe Ser Gly His Thr Ala Ser Gly Gly Asp Met Asn Thr Leu
                165                 170                 175
Arg Leu Gly Ile Asp Gly Ile Ser Gln Ala Thr Pro Leu Asp Thr Phe
            180                 185                 190
Lys Thr Ser Val Arg Ala Gly His Ser Val Glu Gln Ile Met Gln Tyr
        195                 200                 205
Pro Ile Tyr Ser Gly Ile Leu Ala Gly Ala Gly Trp Gln Trp Val Asn
    210                 215                 220
Leu Ala Trp Leu Ala Gly Gly Val Trp Leu Leu Trp Gln Lys Ala Ile
225                 230                 235                 240
Arg Trp His Ile Pro Leu Ser Phe Leu Val Thr Leu Ala Leu Cys Ala
                245                 250                 255
Met Leu Gly Trp Leu Phe Ser Pro Glu Thr Leu Ala Ala Pro Gln Ile
            260                 265                 270
His Leu Leu Ser Gly Ala Thr Met Leu Gly Ala Phe Phe Ile Leu Thr
        275                 280                 285
Asp Pro Val Thr Ala Ser Thr Thr Asn Arg Gly Arg Leu Ile Phe Gly
    290                 295                 300
Ala Leu Ala Gly Leu Leu Val Trp Leu Ile Arg Ser Phe Gly Gly Tyr
305                 310                 315                 320
Pro Asp Gly Val Ala Phe Ala Val Leu Leu Ala Asn Ile Thr Val Pro
                325                 330                 335
Leu Ile Asp Tyr Tyr Thr Arg Pro Arg Val Tyr Gly His Arg Lys Gly
            340                 345                 350

<210> 294

<211> 206
<212> PRT
<213> E. Coli

<400> 294
Met Leu Lys Thr Ile Arg Lys His Gly Ile Thr Leu Ala Leu Phe Ala
1               5                   10                  15
Ala Gly Ser Thr Gly Leu Thr Ala Ala Ile Asn Gln Met Thr Lys Thr
            20                  25                  30
Thr Ile Ala Glu Gln Ala Ser Leu Gln Gln Lys Ala Leu Phe Asp Gln
        35                  40                  45
Val Leu Pro Ala Glu Arg Tyr Asn Asn Ala Leu Ala Gln Ser Cys Tyr
    50                  55                  60
Leu Val Thr Ala Pro Glu Leu Gly Lys Gly Glu His Arg Val Tyr Ile
65                  70                  75                  80
Ala Lys Gln Asp Asp Lys Pro Val Ala Ala Val Leu Glu Ala Thr Ala
                85                  90                  95
Pro Asp Gly Tyr Ser Gly Ala Ile Gln Leu Leu Val Gly Ala Asp Phe
            100                 105                 110
Asn Gly Thr Val Leu Gly Thr Arg Val Thr Glu His His Glu Thr Pro
        115                 120                 125
Gly Leu Gly Asp Lys Ile Glu Leu Arg Leu Ser Asp Trp Ile Thr His
    130                 135                 140
Phe Ala Gly Lys Lys Ile Ser Gly Ala Asp Asp Ala His Trp Ala Val
145                 150                 155                 160
Lys Lys Asp Gly Gly Asp Phe Asp Gln Phe Thr Gly Ala Thr Ile Thr
                165                 170                 175
Pro Arg Ala Val Val Asn Ala Val Lys Arg Ala Gly Leu Tyr Ala Gln
            180                 185                 190
Thr Leu Pro Ala Gln Leu Ser Gln Leu Pro Ala Cys Gly Glu
        195                 200                 205

<210> 295
<211> 231
<212> PRT
<213> E. Coli

<400> 295
Met Ser Glu Ile Lys Asp Val Ile Val Gln Gly Leu Trp Lys Asn Asn
1               5                   10                  15
Ser Ala Leu Val Gln Leu Leu Gly Leu Cys Pro Leu Leu Ala Val Thr
            20                  25                  30
Ser Thr Ala Thr Asn Ala Leu Gly Leu Gly Leu Ala Thr Thr Leu Val
        35                  40                  45
Leu Thr Leu Thr Asn Leu Thr Ile Ser Thr Leu Arg His Trp Thr Pro
    50                  55                  60
Ala Glu Ile Arg Ile Pro Ile Tyr Val Met Ile Ile Ala Ser Val Val
65                  70                  75                  80
Ser Ala Val Gln Met Leu Ile Asn Ala Tyr Ala Phe Gly Leu Tyr Gln
                85                  90                  95
Ser Leu Gly Ile Phe Ile Pro Leu Ile Val Thr Asn Cys Ile Val Val
            100                 105                 110
Gly Arg Ala Glu Ala Phe Ala Ala Lys Lys Gly Pro Ala Leu Ser Ala
        115                 120                 125
Leu Asp Gly Phe Ser Ile Gly Met Gly Ala Thr Cys Ala Met Phe Val
    130                 135                 140
Leu Gly Ser Leu Arg Glu Ile Ile Gly Asn Gly Thr Leu Phe Asp Gly
145                 150                 155                 160
Ala Asp Ala Leu Leu Gly Ser Trp Ala Lys Val Leu Arg Val Glu Ile
                165                 170                 175

Phe His Thr Asp Ser Pro Phe Leu Leu Ala Met Leu Pro Pro Gly Ala
            180                 185                 190
Phe Ile Gly Leu Gly Leu Met Leu Ala Gly Lys Tyr Leu Ile Asp Glu
        195                 200                 205
Arg Met Lys Lys Arg Arg Ala Glu Ala Ala Ala Glu Arg Ala Leu Pro
    210                 215                 220
Asn Gly Glu Thr Gly Asn Val
225                 230

<210> 296
<211> 211
<212> PRT
<213> E. Coli

<400> 296
Met Asn Lys Ala Lys Arg Leu Glu Ile Leu Thr Arg Leu Arg Glu Asn
1               5                   10                  15
Asn Pro His Pro Thr Thr Glu Leu Asn Phe Ser Ser Pro Phe Glu Leu
            20                  25                  30
Leu Ile Ala Val Leu Leu Ser Ala Gln Ala Thr Asp Val Ser Val Asn
        35                  40                  45
Lys Ala Thr Ala Lys Leu Tyr Pro Val Ala Asn Thr Pro Ala Ala Met
    50                  55                  60
Leu Glu Leu Gly Val Glu Gly Val Lys Thr Tyr Ile Lys Thr Ile Gly
65                  70                  75                  80
Leu Tyr Asn Ser Lys Ala Glu Asn Ile Ile Lys Thr Cys Arg Ile Leu
                85                  90                  95
Leu Glu Gln His Asn Gly Glu Val Pro Glu Asp Arg Ala Ala Leu Glu
            100                 105                 110
Ala Leu Pro Gly Val Gly Arg Lys Thr Ala Asn Val Val Leu Asn Thr
        115                 120                 125
Ala Phe Gly Trp Pro Thr Ile Ala Val Asp Thr His Ile Phe Arg Val
    130                 135                 140
Cys Asn Arg Thr Gln Phe Ala Pro Gly Lys Asn Val Glu Gln Val Glu
145                 150                 155                 160
Glu Lys Leu Leu Lys Val Val Pro Ala Glu Phe Lys Val Asp Cys His
                165                 170                 175
His Trp Leu Ile Leu His Gly Arg Tyr Thr Cys Ile Ala Arg Lys Pro
            180                 185                 190
Arg Cys Gly Ser Cys Ile Ile Glu Asp Leu Cys Glu Tyr Lys Glu Lys
        195                 200                 205
Val Asp Ile
    210

<210> 297
<211> 167
<212> PRT
<213> E. Coli

<400> 297
Met Lys Arg Leu His Lys Arg Phe Leu Leu Ala Thr Phe Cys Ala Leu
1               5                   10                  15
Phe Thr Ala Thr Leu Gln Ala Ala Asp Val Thr Ile Thr Val Asn Gly
            20                  25                  30
Arg Val Val Ala Lys Pro Cys Thr Ile Gln Thr Lys Glu Ala Asn Val
        35                  40                  45
Asn Leu Gly Asp Leu Tyr Thr Arg Asn Leu Gln Gln Pro Gly Ser Ala
    50                  55                  60
Ser Gly Trp His Asn Ile Thr Leu Ser Leu Thr Asp Cys Pro Val Glu

65                  70                  75                  80
Thr Ser Ala Val Thr Ala Ile Val Thr Gly Ser Thr Asp Asn Thr Gly
                85                  90                  95
Tyr Tyr Lys Asn Glu Gly Thr Ala Glu Asn Ile Gln Ile Glu Leu Arg
            100                 105                 110
Asp Asp Gln Asp Ala Ala Leu Lys Asn Gly Asp Ser Lys Thr Val Ile
        115                 120                 125
Val Asp Glu Ile Thr Arg Asn Ala Gln Phe Pro Leu Lys Ala Arg Ala
    130                 135                 140
Ile Thr Val Asn Gly Asn Ala Ser Gln Gly Thr Ile Glu Ala Leu Ile
145                 150                 155                 160
Asn Val Ile Tyr Thr Trp Gln
                165

<210> 298
<211> 176
<212> PRT
<213> E. Coli

<400> 298
Met Lys Tyr Asn Asn Ile Ile Phe Leu Gly Leu Cys Leu Gly Leu Thr
 1               5                  10                  15
Thr Tyr Ser Ala Leu Ser Ala Asp Ser Val Ile Lys Ile Ser Gly Arg
            20                  25                  30
Val Leu Asp Tyr Gly Cys Thr Val Ser Ser Asp Ser Leu Asn Phe Thr
        35                  40                  45
Val Asp Leu Gln Lys Asn Ser Ala Arg Gln Phe Pro Thr Thr Gly Ser
    50                  55                  60
Thr Ser Pro Ala Val Pro Phe Gln Ile Thr Leu Ser Glu Cys Ser Lys
65                  70                  75                  80
Gly Thr Thr Gly Val Arg Val Ala Phe Asn Gly Ile Glu Asp Ala Glu
                85                  90                  95
Asn Asn Thr Leu Leu Lys Leu Asp Glu Gly Ser Asn Thr Ala Ser Gly
            100                 105                 110
Leu Gly Ile Glu Ile Leu Asp Ala Asn Met Arg Pro Val Lys Leu Asn
        115                 120                 125
Asp Leu His Ala Gly Met Gln Trp Ile Pro Leu Val Pro Glu Gln Asn
    130                 135                 140
Asn Ile Leu Pro Tyr Ser Ala Arg Leu Lys Ser Thr Gln Lys Ser Val
145                 150                 155                 160
Asn Pro Gly Leu Val Arg Ala Ser Ala Thr Phe Thr Leu Glu Phe Gln
                165                 170                 175

<210> 299
<211> 382
<212> PRT
<213> E. Coli

<400> 299

<210> 300
<211> 138
<212> PRT
<213> E. Coli

<400> 300
Met Ile Ala Ile Ala Asp Ile Leu Gln Ala Gly Glu Lys Leu Thr Ala
1               5                   10                  15
Val Ala Pro Phe Leu Ala Gly Ile Gln Asn Glu Glu Gln Tyr Thr Gln
            20                  25                  30
Ala Leu Glu Leu Val Asp His Leu Leu Leu Asn Asp Pro Glu Asn Pro
        35                  40                  45
Leu Leu Asp Leu Val Cys Ala Lys Ile Thr Ala Trp Glu Glu Ser Ala
    50                  55                  60
Pro Glu Phe Ala Glu Phe Asn Ala Met Ala Gln Ala Met Pro Gly Gly
65                  70                  75                  80
Ile Ala Val Ile Arg Thr Leu Met Asp Gln Tyr Gly Leu Thr Leu Ser
                85                  90                  95
Asp Leu Pro Glu Ile Gly Ser Lys Ser Met Val Ser Arg Val Leu Ser

100                 105                 110
Gly Lys Arg Lys Leu Thr Leu Glu His Ala Lys Lys Leu Ala Thr Arg
        115                 120                 125
Phe Gly Ile Ser Pro Ala Leu Phe Ile Asp
    130                 135

<210> 301
<211> 104
<212> PRT
<213> E. Coli

<400> 301
Met His Leu Ile Thr Gln Lys Ala Leu Lys Asp Ala Ala Glu Lys Tyr
 1               5                  10                  15
Pro Gln His Lys Thr Glu Leu Val Ala Leu Gly Asn Thr Ile Ala Lys
            20                  25                  30
Gly Tyr Phe Lys Lys Pro Glu Ser Leu Lys Ala Val Phe Pro Ser Leu
        35                  40                  45
Asp Asn Phe Lys Tyr Leu Asp Lys His Tyr Val Phe Asn Val Gly Gly
    50                  55                  60
Asn Glu Leu Arg Val Val Ala Met Val Phe Phe Glu Ser Gln Lys Cys
65                  70                  75                  80
Tyr Ile Arg Glu Val Met Thr His Lys Glu Tyr Asp Phe Phe Thr Ala
                85                  90                  95
Val His Arg Thr Lys Gly Lys Lys
            100

<210> 302
<211> 2383
<212> PRT
<213> E. Coli

<400> 302
Met Leu Ser Val Phe Thr Phe Phe Arg Cys Ala Arg Lys Gly Ala Phe
 1               5                  10                  15
Met Leu Ala Arg Ser Gly Lys Val Ser Met Ala Thr Lys Lys Arg Ser
            20                  25                  30
Gly Glu Glu Ile Asn Asp Arg Gln Ile Leu Cys Gly Met Gly Ile Lys
        35                  40                  45
Leu Arg Arg Leu Thr Ala Gly Ile Cys Leu Ile Thr Gln Leu Ala Phe
    50                  55                  60
Pro Met Ala Ala Ala Ala Gln Gly Val Val Asn Ala Ala Thr Gln Gln
65                  70                  75                  80
Pro Val Pro Ala Gln Ile Ala Ile Ala Asn Ala Asn Thr Val Pro Tyr
                85                  90                  95
Thr Leu Gly Ala Leu Glu Ser Ala Gln Ser Val Ala Glu Arg Phe Gly
            100                 105                 110
Ile Ser Val Ala Glu Leu Arg Lys Leu Asn Gln Phe Arg Thr Phe Ala
        115                 120                 125
Arg Ser Phe Asp Asn Val Arg Gln Gly Asp Glu Leu Asp Val Pro Ala
    130                 135                 140
Gln Val Ser Glu Lys Lys Leu Thr Pro Pro Pro Gly Asn Ser Ser Asp
145                 150                 155                 160
Asn Leu Glu Gln Gln Ile Ala Ser Thr Ser Gln Gln Ile Gly Ser Leu
                165                 170                 175
Leu Ala Glu Asp Met Asn Ser Glu Gln Ala Ala Asn Met Ala Arg Gly
            180                 185                 190
Trp Ala Ser Ser Gln Ala Ser Gly Ala Met Thr Asp Trp Leu Ser Arg

195                 200                 205
Phe Gly Thr Ala Arg Ile Thr Leu Gly Val Asp Glu Asp Phe Ser Leu
    210                 215                 220
Lys Asn Ser Gln Phe Asp Phe Leu His Pro Trp Tyr Glu Thr Pro Asp
225                 230                 235                 240
Asn Leu Phe Phe Ser Gln His Thr Leu His Arg Thr Asp Glu Arg Thr
                245                 250                 255
Gln Ile Asn Asn Gly Leu Gly Trp Arg His Phe Thr Pro Thr Trp Met
            260                 265                 270
Ser Gly Ile Asn Phe Phe Phe Asp His Asp Leu Ser Arg Tyr His Ser
        275                 280                 285
Arg Ala Gly Ile Gly Ala Glu Tyr Trp Arg Asp Tyr Leu Lys Leu Ser
    290                 295                 300
Ser Asn Gly Tyr Leu Arg Leu Thr Asn Trp Arg Ser Ala Pro Glu Leu
305                 310                 315                 320
Asp Asn Asp Tyr Glu Ala Arg Pro Ala Asn Gly Trp Asp Val Arg Ala
                325                 330                 335
Glu Ser Trp Leu Pro Ala Trp Pro His Leu Gly Gly Lys Leu Val Tyr
            340                 345                 350
Glu Gln Tyr Tyr Gly Asp Glu Val Ala Leu Phe Asp Lys Asp Asp Arg
        355                 360                 365
Gln Ser Asn Pro His Ala Ile Thr Ala Gly Leu Asn Tyr Thr Pro Phe
    370                 375                 380
Pro Leu Met Thr Phe Ser Ala Glu Gln Arg Gln Gly Lys Gln Gly Glu
385                 390                 395                 400
Asn Asp Thr Arg Phe Ala Val Asp Phe Thr Trp Gln Pro Gly Ser Ala
                405                 410                 415
Met Gln Lys Gln Leu Asp Pro Asn Glu Val Ala Ala Arg Arg Ser Leu
            420                 425                 430
Ala Gly Ser Arg Tyr Asp Leu Val Asp Arg Asn Asn Asn Ile Val Leu
        435                 440                 445
Glu Tyr Arg Lys Lys Glu Leu Val Arg Leu Thr Leu Thr Asp Pro Val
    450                 455                 460
Thr Gly Lys Ser Gly Glu Val Lys Ser Leu Val Ser Ser Leu Gln Thr
465                 470                 475                 480
Lys Tyr Ala Leu Lys Gly Tyr Asn Val Glu Ala Thr Ala Leu Glu Ala
                485                 490                 495
Ala Gly Gly Lys Val Val Thr Thr Gly Lys Asp Ile Leu Val Thr Leu
            500                 505                 510
Pro Ala Tyr Arg Phe Thr Ser Thr Pro Glu Thr Asp Asn Thr Trp Pro
        515                 520                 525
Ile Glu Val Thr Ala Glu Asp Val Lys Gly Asn Leu Ser Asn Arg Glu
    530                 535                 540
Gln Ser Met Val Val Val Gln Ala Pro Thr Leu Ser Gln Lys Asp Ser
545                 550                 555                 560
Ser Val Ser Leu Ser Thr Gln Thr Leu Asn Ala Asp Ser His Ser Thr
                565                 570                 575
Ala Thr Leu Thr Phe Ile Ala His Asp Ala Ala Gly Asn Pro Val Val
            580                 585                 590
Gly Leu Val Leu Ser Thr Arg His Glu Gly Val Gln Asp Ile Thr Leu
        595                 600                 605
Ser Asp Trp Lys Asp Asn Gly Asp Gly Ser Tyr Thr Gln Ile Leu Thr
    610                 615                 620
Thr Gly Ala Met Ser Gly Thr Leu Thr Leu Met Pro Gln Leu Asn Gly
625                 630                 635                 640
Val Asp Ala Ala Lys Ala Pro Ala Val Val Asn Ile Ile Ser Val Ser
                645                 650                 655
Ser Ser Arg Thr His Ser Ser Ile Lys Ile Asp Lys Asp Arg Tyr Leu
            660                 665                 670
Ser Gly Asn Pro Ile Glu Val Thr Val Glu Leu Arg Asp Glu Asn Asp
        675                 680                 685

Lys Pro Val Lys Glu Gln Lys Gln Gln Leu Asn Asn Ala Val Ser Ile
    690                 695                 700
Asp Asn Val Lys Pro Gly Val Thr Thr Asp Trp Lys Glu Thr Ala Asp
705                 710                 715                 720
Gly Val Tyr Lys Ala Thr Tyr Thr Ala Tyr Thr Lys Gly Ser Gly Leu
                725                 730                 735
Thr Ala Lys Leu Leu Met Gln Asn Trp Asn Glu Asp Leu His Thr Ala
            740                 745                 750
Gly Phe Ile Ile Asp Ala Asn Pro Gln Ser Ala Lys Ile Ala Thr Leu
        755                 760                 765
Ser Ala Ser Asn Asn Gly Val Leu Ala Asn Glu Asn Ala Ala Asn Thr
    770                 775                 780
Val Ser Val Asn Val Ala Asp Glu Gly Ser Asn Pro Ile Asn Asp His
785                 790                 795                 800
Thr Val Thr Phe Ala Val Leu Ser Gly Ser Ala Thr Ser Phe Asn Asn
                805                 810                 815
Gln Asn Thr Ala Lys Thr Asp Val Asn Gly Leu Ala Thr Phe Asp Leu
            820                 825                 830
Lys Ser Ser Lys Gln Glu Asp Asn Thr Val Glu Val Thr Leu Glu Asn
        835                 840                 845
Gly Val Lys Gln Thr Leu Ile Val Ser Phe Val Gly Asp Ser Ser Thr
    850                 855                 860
Ala Gln Val Asp Leu Gln Lys Ser Lys Asn Glu Val Val Ala Asp Gly
865                 870                 875                 880
Asn Asp Ser Val Thr Met Thr Ala Thr Val Arg Asp Ala Lys Gly Asn
                885                 890                 895
Leu Leu Asn Asp Val Met Val Thr Phe Asn Val Asn Ser Ala Glu Ala
            900                 905                 910
Lys Leu Ser Gln Thr Glu Val Asn Ser His Asp Gly Ile Ala Thr Ala
        915                 920                 925
Thr Leu Thr Ser Leu Lys Asn Gly Asp Tyr Arg Val Thr Ala Ser Val
    930                 935                 940
Ser Ser Gly Ser Gln Ala Asn Gln Gln Val Asn Phe Ile Gly Asp Gln
945                 950                 955                 960
Ser Thr Ala Ala Leu Thr Leu Ser Val Pro Ser Gly Asp Ile Thr Val
                965                 970                 975
Thr Asn Thr Ala Pro Gln Tyr Met Thr Ala Thr Leu Gln Asp Lys Asn
            980                 985                 990
Gly Asn Pro Leu Lys Asp Lys Glu Ile Thr Phe Ser Val Pro Asn Asp
        995                 1000                1005
Val Ala Ser Lys Phe Ser Ile Ser Asn Gly Gly Lys Gly Met Thr Asp
    1010                1015                1020
Ser Asn Gly Val Ala Ile Ala Ser Leu Thr Gly Thr Leu Ala Gly Thr
1025                1030                1035                1040
His Met Ile Met Ala Arg Leu Ala Asn Ser Asn Val Ser Asp Ala Gln
                1045                1050                1055
Pro Met Thr Phe Val Ala Asp Lys Asp Arg Ala Val Val Val Leu Gln
            1060                1065                1070
Thr Ser Lys Ala Glu Ile Ile Gly Asn Gly Val Asp Glu Thr Thr Leu
        1075                1080                1085
Thr Ala Thr Val Lys Asp Pro Ser Asn His Pro Val Ala Gly Ile Thr
    1090                1095                1100
Val Asn Phe Thr Met Pro Gln Asp Val Ala Ala Asn Phe Thr Leu Glu
1105                1110                1115                1120
Asn Asn Gly Ile Ala Ile Thr Gln Ala Asn Gly Glu Ala His Val Thr
                1125                1130                1135
Leu Lys Gly Lys Lys Ala Gly Thr His Thr Val Thr Ala Thr Leu Gly
            1140                1145                1150
Asn Asn Asn Thr Ser Asp Ser Gln Pro Val Thr Phe Val Ala Asp Lys
        1155                1160                1165
Ala Ser Ala Gln Val Val Leu Gln Ile Ser Lys Asp Glu Ile Thr Gly

1170                1175                1180
Asn Gly Val Asp Ser Ala Thr Leu Thr Ala Thr Val Lys Asp Gln Phe
1185                1190                1195                1200
Asp Asn Glu Val Asn Asn Leu Pro Val Thr Phe Ser Ser Ala Ser Ser
                1205                1210                1215
Gly Leu Thr Leu Thr Pro Gly Val Ser Asn Thr Asn Glu Ser Gly Ile
            1220                1225                1230
Ala Gln Ala Thr Leu Ala Gly Val Ala Phe Gly Glu Lys Thr Val Thr
        1235                1240                1245
Ala Ser Leu Ala Asn Asn Gly Ala Ser Asp Asn Lys Thr Val His Phe
    1250                1255                1260
Ile Gly Asp Thr Ala Ala Ala Lys Ile Ile Glu Leu Ala Pro Val Pro
1265                1270                1275                1280
Asp Ser Ile Ile Ala Gly Thr Pro Gln Asn Ser Ser Gly Ser Val Ile
                1285                1290                1295
Thr Ala Thr Val Val Asp Asn Asn Gly Phe Pro Val Lys Gly Val Thr
            1300                1305                1310
Val Asn Phe Thr Ser Asn Ala Ala Thr Ala Glu Met Thr Asn Gly Gly
        1315                1320                1325
Gln Ala Val Thr Asn Glu Gln Gly Lys Ala Thr Val Thr Tyr Thr Asn
    1330                1335                1340
Thr Arg Ser Ser Ile Glu Ser Gly Ala Arg Pro Asp Thr Val Glu Ala
1345                1350                1355                1360
Ser Leu Glu Asn Gly Ser Ser Thr Leu Ser Thr Ser Ile Asn Val Asn
                1365                1370                1375
Ala Asp Ala Ser Thr Ala His Leu Thr Leu Leu Gln Ala Leu Phe Asp
            1380                1385                1390
Thr Val Ser Ala Gly Glu Thr Thr Ser Leu Tyr Ile Glu Val Lys Asp
        1395                1400                1405
Asn Tyr Gly Asn Gly Val Pro Gln Gln Glu Val Thr Leu Ser Val Ser
    1410                1415                1420
Pro Ser Glu Gly Val Thr Pro Ser Asn Asn Ala Ile Tyr Thr Thr Asn
1425                1430                1435                1440
His Asp Gly Asn Phe Tyr Ala Ser Phe Thr Ala Thr Lys Ala Gly Val
                1445                1450                1455
Tyr Gln Leu Thr Ala Thr Leu Glu Asn Gly Asp Ser Met Gln Gln Thr
            1460                1465                1470
Val Thr Tyr Val Pro Asn Val Ala Asn Ala Glu Ile Thr Leu Ala Ala
        1475                1480                1485
Ser Lys Asp Pro Val Ile Ala Asp Asn Asn Asp Leu Thr Thr Leu Thr
    1490                1495                1500
Ala Thr Val Ala Asp Thr Glu Gly Asn Ala Ile Ala Asn Thr Glu Val
1505                1510                1515                1520
Thr Phe Thr Leu Pro Glu Asp Val Lys Ala Asn Phe Thr Leu Ser Asp
                1525                1530                1535
Gly Gly Lys Val Ile Thr Asp Ala Glu Gly Lys Ala Lys Val Thr Leu
            1540                1545                1550
Lys Gly Thr Lys Ala Gly Ala His Thr Val Thr Ala Ser Met Thr Gly
        1555                1560                1565
Gly Lys Ser Glu Gln Leu Val Val Asn Phe Ile Ala Asp Thr Leu Thr
    1570                1575                1580
Ala Gln Val Asn Leu Asn Val Thr Glu Asp Asn Phe Ile Ala Asn Asn
1585                1590                1595                1600
Val Gly Met Thr Arg Leu Gln Ala Thr Val Thr Asp Gly Asn Gly Asn
                1605                1610                1615
Pro Leu Ala Asn Glu Ala Val Thr Phe Thr Leu Pro Ala Asp Val Ser
            1620                1625                1630
Ala Ser Phe Thr Leu Gly Gln Gly Gly Ser Ala Ile Thr Asp Ile Asn
        1635                1640                1645
Gly Lys Ala Glu Val Thr Leu Ser Gly Thr Lys Ser Gly Thr Tyr Pro
    1650                1655                1660

Val Thr Val Ser Val Asn Asn Tyr Gly Val Ser Asp Thr Lys Gln Val
1665                1670                1675                1680
Thr Leu Ile Ala Asp Ala Gly Thr Ala Lys Leu Ala Ser Leu Thr Ser
                1685                1690                1695
Val Tyr Ser Phe Val Val Ser Thr Thr Glu Gly Ala Thr Met Thr Ala
            1700                1705                1710
Ser Val Thr Asp Ala Asn Gly Asn Pro Val Glu Gly Ile Lys Val Asn
        1715                1720                1725
Phe Arg Gly Thr Ser Val Thr Leu Ser Ser Thr Ser Val Glu Thr Asp
    1730                1735                1740
Asp Arg Gly Phe Ala Glu Ile Leu Val Thr Ser Thr Glu Val Gly Leu
1745                1750                1755                1760
Lys Thr Val Ser Ala Ser Leu Ala Asp Lys Pro Thr Glu Val Ile Ser
                1765                1770                1775
Arg Leu Leu Asn Ala Ser Ala Asp Val Asn Ser Ala Thr Ile Thr Ser
            1780                1785                1790
Leu Glu Ile Pro Glu Gly Gln Val Met Val Ala Gln Asp Val Ala Val
        1795                1800                1805
Lys Ala His Val Asn Asp Gln Phe Gly Asn Pro Val Ala His Gln Pro
    1810                1815                1820
Val Thr Phe Ser Ala Glu Pro Ser Ser Gln Met Ile Ile Ser Gln Asn
1825                1830                1835                1840
Thr Val Ser Thr Asn Thr Gln Gly Val Ala Glu Val Thr Met Thr Pro
                1845                1850                1855
Glu Arg Asn Gly Ser Tyr Met Val Lys Ala Ser Leu Pro Asn Gly Ala
            1860                1865                1870
Ser Leu Glu Lys Gln Leu Glu Ala Ile Asp Glu Lys Leu Thr Leu Thr
        1875                1880                1885
Ala Ser Ser Pro Leu Ile Gly Val Tyr Ala Pro Thr Gly Ala Thr Leu
    1890                1895                1900
Thr Ala Thr Leu Thr Ser Ala Asn Gly Thr Pro Val Glu Gly Gln Val
1905                1910                1915                1920
Ile Asn Phe Ser Val Thr Pro Glu Gly Ala Thr Leu Ser Gly Gly Lys
                1925                1930                1935
Val Arg Thr Asn Ser Ser Gly Gln Ala Pro Val Val Leu Thr Ser Asn
            1940                1945                1950
Lys Val Gly Thr Tyr Thr Val Thr Ala Ser Phe His Asn Gly Val Thr
        1955                1960                1965
Ile Gln Thr Gln Thr Thr Val Lys Val Thr Gly Asn Ser Ser Thr Ala
    1970                1975                1980
His Val Ala Ser Phe Ile Ala Asp Pro Ser Thr Ile Ala Ala Thr Asn
1985                1990                1995                2000
Thr Asp Leu Ser Thr Leu Lys Ala Thr Val Glu Asp Gly Ser Gly Asn
                2005                2010                2015
Leu Ile Glu Gly Leu Thr Val Tyr Phe Ala Leu Lys Ser Gly Ser Ala
            2020                2025                2030
Thr Leu Thr Ser Leu Thr Ala Val Thr Asp Gln Asn Gly Ile Ala Thr
        2035                2040                2045
Thr Ser Val Lys Gly Ala Met Thr Gly Ser Val Thr Val Ser Ala Val
    2050                2055                2060
Thr Thr Ala Gly Gly Met Gln Thr Val Asp Ile Thr Leu Val Ala Gly
2065                2070                2075                2080
Pro Ala Asp Thr Ser Gln Ser Val Leu Lys Ser Asn Arg Ser Ser Leu
                2085                2090                2095
Lys Gly Asp Tyr Thr Asp Ser Ala Glu Leu Arg Leu Val Leu His Asp
            2100                2105                2110
Ile Ser Gly Asn Pro Ile Lys Val Ser Glu Gly Met Glu Phe Val Gln
        2115                2120                2125
Ser Gly Thr Asn Val Pro Tyr Ile Lys Ile Ser Ala Ile Asp Tyr Ser
    2130                2135                2140
Leu Asn Ile Asn Gly Asp Tyr Lys Ala Thr Val Thr Gly Gly Gly Glu

2145                2150                2155                2160
Gly Ile Ala Thr Leu Ile Pro Val Leu Asn Gly Val His Gln Ala Gly
                2165                2170                2175
Leu Ser Thr Thr Ile Gln Phe Thr Arg Ala Glu Asp Lys Ile Met Ser
            2180                2185                2190
Gly Thr Val Ser Val Asn Gly Thr Asp Leu Pro Thr Thr Thr Phe Pro
        2195                2200                2205
Ser Gln Gly Phe Thr Gly Ala Tyr Tyr Gln Leu Asn Asn Asp Asn Phe
    2210                2215                2220
Ala Pro Gly Lys Thr Ala Ala Asp Tyr Glu Phe Ser Ser Ser Ala Ser
2225                2230                2235                2240
Trp Val Asp Val Asp Ala Thr Gly Lys Val Thr Phe Lys Asn Val Gly
                2245                2250                2255
Ser Asn Ser Glu Arg Ile Thr Ala Thr Pro Lys Ser Gly Gly Pro Ser
            2260                2265                2270
Tyr Val Tyr Glu Ile Arg Val Lys Ser Trp Trp Val Asn Ala Gly Glu
        2275                2280                2285
Ala Phe Met Ile Tyr Ser Leu Ala Glu Asn Phe Cys Ser Ser Asn Gly
    2290                2295                2300
Tyr Thr Leu Pro Arg Ala Asn Tyr Leu Asn His Cys Ser Ser Arg Gly
2305                2310                2315                2320
Ile Gly Ser Leu Tyr Ser Glu Trp Gly Asp Met Gly His Tyr Thr Thr
                2325                2330                2335
Asp Ala Gly Phe Gln Ser Asn Met Tyr Trp Ser Ser Ser Pro Ala Asn
            2340                2345                2350
Ser Ser Glu Gln Tyr Val Val Ser Leu Ala Thr Gly Asp Gln Ser Val
        2355                2360                2365
Phe Glu Lys Leu Gly Phe Ala Tyr Ala Thr Cys Tyr Lys Asn Leu
    2370                2375                2380

<210> 303
<211> 61
<212> PRT
<213> E. Coli

<400> 303
Met Ser Lys Gly Ala Leu Tyr Glu Phe Asn Asn Pro Asp Gln Leu Lys
1               5                   10                  15
Ile Pro Leu Pro His Lys His Ile Ala Ser Thr Phe Asn Asp Ile Met
            20                  25                  30
Ser Lys Asp Val Gly Tyr Ala Tyr Val Ser Leu Leu Tyr Ala Cys Pro
        35                  40                  45
Leu Lys Thr His Ser Leu Arg Leu Asn Pro Phe Ser Lys
    50                  55                  60

<210> 304
<211> 398
<212> PRT
<213> E. Coli

<400> 304
Met Gln Val Ala Glu Gln Arg Ile Gln Leu Ala Glu Ala Gln Ala Lys
1               5                   10                  15
Ala Val Ala Thr Gln Asp Gly Pro Gln Ile Asp Phe Ser Ala Asp Met
            20                  25                  30
Glu Arg Gln Lys Met Ser Ala Glu Gly Leu Met Gly Pro Phe Ala Leu
        35                  40                  45
Asn Asp Pro Ala Ala Gly Thr Thr Gly Pro Trp Tyr Thr Asn Gly Thr
    50                  55                  60

Phe Gly Leu Thr Ala Gly Trp His Leu Asp Ile Trp Gly Lys Asn Arg
65                  70                  75                  80
Ala Glu Val Thr Ala Arg Leu Gly Thr Val Lys Ala Arg Ala Ala Glu
                85                  90                  95
Arg Glu Gln Thr Arg Gln Leu Leu Ala Gly Ser Val Ala Arg Leu Tyr
            100                 105                 110
Trp Glu Trp Gln Thr Gln Ala Ala Leu Asn Thr Val Leu Gln Gln Ile
        115                 120                 125
Glu Lys Glu Gln Asn Thr Ile Ile Ala Thr Asp Arg Gln Leu Tyr Gln
    130                 135                 140
Asn Gly Ile Thr Ser Ser Val Glu Gly Val Glu Thr Asp Ile Asn Ala
145                 150                 155                 160
Ser Lys Thr Arg Gln Gln Leu Asn Asp Val Ala Gly Lys Met Lys Ile
                165                 170                 175
Ile Glu Ala Arg Leu Ser Ala Leu Thr Asn Asn Gln Thr Lys Ser Leu
            180                 185                 190
Lys Leu Lys Pro Val Ala Leu Pro Lys Val Ala Ser Gln Leu Pro Asp
        195                 200                 205
Glu Leu Gly Tyr Ser Leu Leu Ala Arg Arg Ala Asp Leu Gln Ala Ala
    210                 215                 220
His Trp Tyr Val Glu Ser Ser Leu Ser Thr Ile Asp Ala Ala Lys Ala
225                 230                 235                 240
Ala Phe Tyr Pro Asp Ile Asn Leu Met Ala Phe Leu Gln Gln Asp Ala
                245                 250                 255
Leu His Leu Ser Asp Leu Phe Arg His Ser Ala Gln Gln Met Gly Val
            260                 265                 270
Thr Ala Gly Leu Thr Leu Pro Ile Phe Asp Ser Gly Arg Leu Asn Ala
        275                 280                 285
Asn Leu Asp Ile Ala Lys Ala Glu Ser Asn Leu Ser Ile Ala Ser Tyr
    290                 295                 300
Asn Lys Ala Val Val Glu Ala Val Asn Asp Val Ala Arg Ala Ala Ser
305                 310                 315                 320
Gln Val Gln Thr Leu Ala Glu Lys Asn Gln His Gln Ala Gln Ile Glu
                325                 330                 335
Arg Asp Ala Leu Arg Val Val Gly Leu Ala Gln Ala Arg Phe Asn Ala
            340                 345                 350
Gly Ile Ile Ala Gly Ser Arg Val Ser Glu Ala Arg Ile Pro Ala Leu
        355                 360                 365
Arg Glu Arg Ala Asn Gly Leu Leu Leu Gln Gly Gln Trp Leu Asp Ala
    370                 375                 380
Ser Ile Gln Leu Thr Gly Ala Leu Gly Gly Gly Tyr Lys Arg
385                 390                 395

<210> 305
<211> 96
<212> PRT
<213> E. Coli

<400> 305
Met Tyr Cys His Ala Lys Leu Lys Asn Ile Ser Gln His Thr Val Ile
1               5                   10                  15
Ser Ala His Leu Phe Leu Pro Asp Tyr Ser Pro Met Asn Arg Asp Ser
            20                  25                  30
Phe Tyr Pro Ala Ile Ala Cys Phe Pro Leu Leu Leu Met Leu Ala Gly
        35                  40                  45
Cys Ala Pro Met His Glu Thr Arg Gln Ala Leu Ser Gln Gln Thr Pro
    50                  55                  60
Ala Ala Gln Val Asp Thr Ala Leu Pro Thr Ala Leu Lys Met Val Gly
65                  70                  75                  80
Gln Thr Ala Asn Gly Gly Trp Ser Ile Thr Ile Ile Asn Ser Leu Pro

85                  90                  95

<210> 306
<211> 315
<212> PRT
<213> E. Coli

<400> 306
Met Arg Val Leu Leu Ala Pro Met Glu Gly Val Leu Asp Ser Leu Val
1               5                   10                  15
Arg Glu Leu Leu Thr Glu Val Asn Asp Tyr Asp Leu Cys Ile Thr Glu
            20                  25                  30
Phe Val Arg Val Val Asp Gln Leu Leu Pro Val Lys Val Phe His Arg
        35                  40                  45
Ile Cys Pro Glu Leu Gln Asn Ala Ser Arg Thr Pro Ser Gly Thr Leu
    50                  55                  60
Val Arg Val Gln Leu Leu Gly Gln Phe Pro Gln Trp Leu Ala Glu Asn
65                  70                  75                  80
Ala Ala Arg Ala Val Glu Leu Gly Ser Trp Gly Val Asp Leu Asn Cys
                85                  90                  95
Gly Cys Pro Ser Lys Thr Val Asn Gly Ser Gly Gly Gly Ala Thr Leu
            100                 105                 110
Leu Lys Asp Pro Glu Leu Ile Tyr Gln Gly Ala Lys Ala Met Arg Glu
        115                 120                 125
Ala Val Pro Ala His Leu Pro Val Ser Val Lys Val Arg Leu Gly Trp
    130                 135                 140
Asp Ser Gly Glu Lys Lys Phe Glu Ile Ala Asp Ala Val Gln Gln Ala
145                 150                 155                 160
Gly Ala Thr Glu Leu Val Val His Gly Arg Thr Lys Glu Gln Gly Tyr
                165                 170                 175
Arg Ala Glu His Ile Asp Trp Gln Ala Ile Gly Asp Ile Arg Gln Arg
            180                 185                 190
Leu Asn Ile Pro Val Ile Ala Asn Gly Glu Ile Trp Asp Trp Gln Ser
        195                 200                 205
Ala Gln Gln Cys Met Ala Ile Ser Gly Cys Asp Ala Val Met Ile Gly
    210                 215                 220
Arg Gly Ala Leu Asn Ile Pro Asn Leu Ser Arg Val Val Lys Tyr Asn
225                 230                 235                 240
Glu Pro Arg Met Pro Trp Pro Glu Val Val Ala Leu Leu Gln Lys Tyr
                245                 250                 255
Thr Arg Leu Glu Lys Gln Gly Asp Thr Gly Leu Tyr His Val Ala Arg
            260                 265                 270
Ile Lys Gln Trp Leu Ser Tyr Leu Arg Lys Glu Tyr Asp Glu Ala Thr
        275                 280                 285
Glu Leu Phe Gln His Val Arg Val Leu Asn Asn Ser Pro Asp Ile Ala
    290                 295                 300
Arg Ala Ile Gln Ala Ile Asp Ile Glu Lys Leu
305                 310                 315

<210> 307
<211> 296
<212> PRT
<213> E. Coli

<400> 307
Met Thr Ile Ser Thr Thr Ser Thr Pro His Asp Ala Val Phe Lys Ser
1               5                   10                  15
Phe Leu Arg His Pro Asp Thr Ala Arg Asp Phe Ile Asp Ile His Leu
            20                  25                  30

Pro Ala Pro Leu Arg Lys Leu Cys Asp Leu Thr Thr Leu Lys Leu Glu
        35                  40                  45
Pro Asn Ser Phe Ile Asp Glu Asp Leu Arg Gln Tyr Tyr Ser Asp Leu
    50                  55                  60
Leu Trp Ser Val Lys Thr Gln Glu Gly Val Gly Tyr Ile Tyr Val Val
65                  70                  75                  80
Ile Glu His Gln Ser Lys Pro Glu Glu Leu Met Ala Phe Arg Met Met
                85                  90                  95
Arg Tyr Ser Ile Ala Ala Met Gln Asn His Leu Asp Ala Gly Tyr Lys
            100                 105                 110
Glu Leu Pro Leu Val Leu Pro Met Leu Phe Tyr His Gly Cys Arg Ser
        115                 120                 125
Pro Tyr Pro Tyr Ser Leu Cys Trp Leu Asp Glu Phe Ala Glu Pro Ala
    130                 135                 140
Ile Ala Arg Lys Ile Tyr Ser Ser Ala Phe Pro Leu Val Asp Ile Thr
145                 150                 155                 160
Val Val Pro Asp Asp Glu Ile Met Gln His Arg Lys Met Ala Leu Leu
                165                 170                 175
Glu Leu Ile Gln Lys His Ile Arg Gln Arg Asp Leu Leu Gly Leu Val
            180                 185                 190
Asp Gln Ile Val Ser Leu Leu Val Thr Gly Asn Thr Asn Asp Arg Gln
        195                 200                 205
Leu Lys Ala Leu Phe Asn Tyr Val Leu Gln Thr Gly Asp Ala Gln Arg
    210                 215                 220
Phe Arg Ala Phe Ile Gly Glu Ile Ala Glu Arg Ala Pro Gln Glu Lys
225                 230                 235                 240
Glu Lys Leu Met Thr Ile Ala Asp Arg Leu Arg Glu Glu Gly Ala Met
                245                 250                 255
Gln Gly Lys His Glu Glu Ala Leu Arg Ile Ala Gln Glu Met Leu Asp
            260                 265                 270
Arg Gly Leu Asp Arg Glu Leu Val Met Met Val Thr Arg Leu Ser Pro
        275                 280                 285
Asp Asp Leu Ile Ala Gln Ser His
    290                 295

<210> 308
<211> 555
<212> PRT
<213> E. Coli

<400> 308
<400> 3
Met Ala Gln Phe Val Tyr Thr Met His Arg Val Gly Lys Val Val Pro
1               5                   10                  15
Pro Lys Arg His Ile Leu Lys Asn Ile Ser Leu Ser Phe Phe Pro Gly
            20                  25                  30
Ala Lys Ile Gly Val Leu Gly Leu Asn Gly Ala Gly Lys Ser Thr Leu
        35                  40                  45
Leu Arg Ile Met Ala Gly Ile Asp Lys Asp Ile Glu Gly Glu Ala Arg
    50                  55                  60
Pro Gln Pro Asp Ile Lys Ile Gly Tyr Leu Pro Gln Glu Pro Gln Leu
65                  70                  75                  80
Asn Pro Glu His Thr Val Arg Glu Ser Ile Glu Glu Ala Val Ser Glu
                85                  90                  95
Val Val Asn Ala Leu Lys Arg Leu Asp Glu Val Tyr Ala Leu Tyr Ala
            100                 105                 110
Asp Pro Asp Ala Asp Phe Asp Lys Leu Ala Ala Glu Gln Gly Arg Leu
        115                 120                 125
Glu Glu Ile Ile Gln Ala His Asp Gly His Asn Leu Asn Val Gln Leu
    130                 135                 140

Glu Arg Ala Ala Asp Ala Leu Arg Leu Pro Asp Trp Asp Ala Lys Ile
145                 150                 155                 160
Ala Asn Leu Ser Gly Gly Glu Arg Arg Arg Val Ala Leu Cys Arg Leu
                165                 170                 175
Leu Leu Glu Lys Pro Asp Met Leu Leu Leu Asp Glu Pro Thr Asn His
            180                 185                 190
Leu Asp Ala Glu Ser Val Ala Trp Leu Glu Arg Phe Leu His Asp Phe
        195                 200                 205
Glu Gly Thr Val Val Ala Ile Thr His Asp Arg Tyr Phe Leu Asp Asn
    210                 215                 220
Val Ala Gly Trp Ile Leu Glu Leu Asp Arg Gly Glu Gly Ile Pro Trp
225                 230                 235                 240
Glu Gly Asn Tyr Ser Ser Trp Leu Glu Gln Lys Asp Gln Arg Leu Ala
                245                 250                 255
Gln Glu Ala Ser Gln Glu Ala Ala Arg Arg Lys Ser Ile Glu Lys Glu
            260                 265                 270
Leu Glu Trp Val Arg Gln Gly Thr Lys Gly Arg Gln Ser Lys Gly Lys
        275                 280                 285
Ala Arg Leu Ala Arg Phe Glu Glu Leu Asn Ser Thr Glu Tyr Gln Lys
    290                 295                 300
Arg Asn Glu Thr Asn Glu Leu Phe Ile Pro Pro Gly Pro Arg Leu Gly
305                 310                 315                 320
Asp Lys Val Leu Glu Val Ser Asn Leu Arg Lys Ser Tyr Gly Asp Arg
                325                 330                 335
Leu Leu Ile Asp Asp Leu Ser Phe Ser Ile Pro Lys Gly Ala Ile Val
            340                 345                 350
Gly Ile Ile Gly Pro Asn Gly Ala Gly Lys Ser Thr Leu Phe Arg Met
        355                 360                 365
Ile Ser Gly Gln Glu Gln Pro Asp Ser Gly Thr Ile Thr Leu Gly Glu
    370                 375                 380
Thr Val Lys Leu Ala Ser Val Asp Gln Phe Arg Asp Ser Met Asp Asn
385                 390                 395                 400
Ser Lys Thr Val Trp Glu Glu Val Ser Gly Gly Leu Asp Ile Met Lys
                405                 410                 415
Ile Gly Asn Thr Glu Met Pro Ser Arg Ala Tyr Val Gly Arg Phe Asn
            420                 425                 430
Phe Lys Gly Val Asp Gln Gly Lys Arg Val Gly Glu Leu Ser Gly Gly
        435                 440                 445
Glu Arg Gly Arg Leu His Leu Ala Lys Leu Leu Gln Val Gly Gly Asn
    450                 455                 460
Met Leu Leu Leu Asp Glu Pro Thr Asn Asp Leu Asp Ile Glu Thr Leu
465                 470                 475                 480
Arg Ala Leu Glu Asn Ala Leu Leu Glu Phe Pro Gly Cys Ala Met Val
                485                 490                 495
Ile Ser His Asp Arg Trp Phe Leu Asp Arg Ile Ala Thr His Ile Leu
            500                 505                 510
Asp Tyr Gln Asp Glu Gly Lys Val Glu Phe Phe Glu Gly Asn Phe Thr
        515                 520                 525
Glu Tyr Glu Glu Tyr Lys Lys Arg Thr Leu Gly Ala Asp Ala Leu Glu
    530                 535                 540
Pro Lys Arg Ile Lys Tyr Lys Arg Ile Ala Lys
545                 550

<210> 309
<211> 173
<212> PRT
<213> E. Coli

<400> 309

Met Ser Lys Pro Lys Tyr Pro Phe Glu Lys Arg Leu Glu Val Val Asn
1               5                   10                  15
His Tyr Phe Thr Thr Asp Asp Gly Tyr Arg Ile Ile Ser Ala Arg Phe
            20                  25                  30
Gly Val Pro Arg Thr Gln Val Arg Thr Trp Val Ala Leu Tyr Glu Lys
        35                  40                  45
His Gly Glu Lys Gly Leu Ile Pro Lys Pro Lys Gly Val Ser Ala Asp
    50                  55                  60
Pro Glu Leu Arg Ile Lys Val Val Lys Ala Val Ile Glu Gln His Met
65                  70                  75                  80
Ser Leu Asn Gln Ala Ala Ala His Phe Met Leu Ala Gly Ser Gly Ser
                85                  90                  95
Val Ala Arg Trp Leu Lys Val Tyr Glu Glu Arg Gly Glu Ala Gly Leu
            100                 105                 110
Arg Ala Leu Lys Ile Gly Thr Lys Arg Asn Ile Ala Ile Ser Val Asp
        115                 120                 125
Pro Glu Lys Ala Ala Ser Ala Leu Glu Leu Ser Lys Asp Arg Arg Ile
    130                 135                 140
Glu Asp Leu Glu Arg Gln Val Arg Phe Leu Glu Thr Arg Leu Met Tyr
145                 150                 155                 160
Leu Lys Lys Leu Lys Ala Leu Ala His Pro Thr Lys Lys
                165                 170

<210> 310
<211> 283
<212> PRT
<213> E. Coli

<400> 310
Met Lys Val Leu Asn Glu Leu Arg Gln Phe Tyr Pro Leu Asp Glu Leu
1               5                   10                  15
Leu Arg Ala Ala Glu Ile Pro Arg Ser Thr Phe Tyr Tyr His Leu Lys
            20                  25                  30
Ala Leu Ser Lys Pro Asp Lys Tyr Ala Asp Val Lys Lys Arg Ile Ser
        35                  40                  45
Glu Ile Tyr His Glu Asn Arg Gly Arg Tyr Gly Tyr Arg Arg Val Thr
    50                  55                  60
Leu Ser Leu His Arg Glu Gly Lys Gln Ile Asn His Lys Ala Val Gln
65                  70                  75                  80
Arg Leu Met Gly Thr Leu Ser Leu Lys Ala Ala Ile Lys Val Lys Arg
                85                  90                  95
Tyr Arg Ser Tyr Arg Gly Glu Val Gly Gln Thr Ala Pro Asn Val Leu
            100                 105                 110
Gln Arg Asp Phe Lys Ala Thr Arg Pro Asn Glu Lys Trp Val Thr Asp
        115                 120                 125
Val Thr Glu Phe Ala Val Asn Gly Arg Lys Leu Tyr Leu Ser Pro Val
    130                 135                 140
Ile Asp Leu Phe Asn Asn Glu Val Ile Ser Tyr Ser Leu Ser Glu Arg
145                 150                 155                 160
Pro Val Met Asn Met Val Glu Asn Met Leu Asp Gln Ala Phe Lys Lys
                165                 170                 175
Leu Asn Pro His Glu His Pro Val Leu His Ser Asp Gln Gly Trp Gln
            180                 185                 190
Tyr Arg Met Arg Arg Tyr Gln Asn Ile Leu Lys Glu His Gly Ile Lys
        195                 200                 205
Gln Ser Met Ser Arg Lys Gly Asn Cys Leu Asp Asn Ala Val Val Glu
    210                 215                 220
Cys Phe Phe Gly Thr Leu Lys Ser Glu Cys Phe Tyr Leu Asp Glu Phe
225                 230                 235                 240
Ser Asn Ile Ser Glu Leu Lys Asp Ala Val Thr Glu Tyr Ile Glu Tyr

245                 250                 255
Tyr Asn Ser Arg Arg Ile Ser Leu Lys Leu Lys Gly Leu Thr Pro Ile
            260                 265                 270
Glu Tyr Arg Asn Gln Thr Tyr Met Pro Arg Val
        275                 280

<210> 311
<211> 38
<212> PRT
<213> E. Coli

<400> 311

<210> 312
<211> 443
<212> PRT
<213> E. Coli

<400> 312
Met Ala Lys Gln Pro Gly Leu Asp Phe Gln Ser Ala Lys Gly Gly Leu
 1               5                  10                  15
Gly Glu Leu Lys Arg Arg Leu Leu Phe Val Ile Gly Ala Leu Ile Val
            20                  25                  30
Phe Arg Ile Gly Ser Phe Ile Pro Ile Pro Gly Ile Asp Ala Ala Val
        35                  40                  45
Leu Ala Lys Leu Leu Glu Gln Gln Arg Gly Thr Ile Ile Glu Met Phe
    50                  55                  60
Asn Met Phe Ser Gly Gly Ala Leu Ser Arg Ala Ser Ile Phe Ala Leu
65                  70                  75                  80
Gly Ile Met Pro Tyr Ile Ser Ala Ser Ile Ile Ile Gln Leu Leu Thr
                85                  90                  95
Val Val His Pro Thr Leu Ala Glu Ile Lys Lys Glu Gly Glu Ser Gly
            100                 105                 110
Arg Arg Lys Ile Ser Gln Tyr Thr Arg Tyr Gly Thr Leu Val Leu Ala
        115                 120                 125
Ile Phe Gln Ser Ile Gly Ile Ala Thr Gly Leu Pro Asn Met Pro Gly
    130                 135                 140
Met Gln Gly Leu Val Ile Asn Pro Gly Phe Ala Phe Tyr Phe Thr Ala
145                 150                 155                 160
Val Val Ser Leu Val Thr Gly Thr Met Phe Leu Met Trp Leu Gly Glu
                165                 170                 175
Gln Ile Thr Glu Arg Gly Ile Gly Asn Gly Ile Ser Ile Ile Ile Phe
            180                 185                 190
Ala Gly Ile Val Ala Gly Leu Pro Pro Ala Ile Ala His Thr Ile Glu
        195                 200                 205
Gln Ala Arg Gln Gly Asp Leu His Phe Leu Val Leu Leu Leu Val Ala
    210                 215                 220
Val Leu Val Phe Ala Val Thr Phe Phe Val Val Phe Val Glu Arg Gly
225                 230                 235                 240
Gln Arg Arg Ile Val Val Asn Tyr Ala Lys Arg Gln Gln Gly Arg Arg
                245                 250                 255
Val Tyr Ala Ala Gln Ser Thr His Leu Pro Leu Lys Val Asn Met Ala
            260                 265                 270
Gly Val Ile Pro Ala Ile Phe Ala Ser Ser Ile Ile Leu Phe Pro Ala

275                 280                 285
Thr Ile Ala Ser Trp Phe Gly Gly Gly Thr Gly Trp Asn Trp Leu Thr
    290                 295                 300
Thr Ile Ser Leu Tyr Leu Gln Pro Gly Gln Pro Leu Tyr Val Leu Leu
305                 310                 315                 320
Tyr Ala Ser Ala Ile Ile Phe Phe Cys Phe Phe Tyr Thr Ala Leu Val
                325                 330                 335
Phe Asn Pro Arg Glu Thr Ala Asp Asn Leu Lys Lys Ser Gly Ala Phe
            340                 345                 350
Val Pro Gly Ile Arg Pro Gly Glu Gln Thr Ala Lys Tyr Ile Asp Lys
        355                 360                 365
Val Met Thr Arg Leu Thr Leu Val Gly Ala Leu Tyr Ile Thr Phe Ile
    370                 375                 380
Cys Leu Ile Pro Glu Phe Met Arg Asp Ala Met Lys Val Pro Phe Tyr
385                 390                 395                 400
Phe Gly Gly Thr Ser Leu Leu Ile Val Val Val Val Ile Met Asp Phe
                405                 410                 415
Met Ala Gln Val Gln Thr Leu Met Met Ser Ser Gln Tyr Glu Ser Ala
            420                 425                 430
Leu Lys Lys Ala Asn Leu Lys Gly Tyr Gly Arg
        435                 440

<210> 313
<211> 144
<212> PRT
<213> E. Coli

<400> 313

<210> 314
<211> 59
<212> PRT
<213> E. Coli

<400> 314

<210> 315
<211> 167
<212> PRT
<213> E. Coli

<400> 315
Met Ala His Ile Glu Lys Gln Ala Gly Glu Leu Gln Glu Lys Leu Ile
 1               5                  10                  15
Ala Val Asn Arg Val Ser Lys Thr Val Lys Gly Gly Arg Ile Phe Ser
                20                  25                  30
Phe Thr Ala Leu Thr Val Val Gly Asp Gly Asn Gly Arg Val Gly Phe
        35                  40                  45
Gly Tyr Gly Lys Ala Arg Glu Val Pro Ala Ala Ile Gln Lys Ala Met
    50                  55                  60
Glu Lys Ala Arg Arg Asn Met Ile Asn Val Ala Leu Asn Asn Gly Thr
65                  70                  75                  80
Leu Gln His Pro Val Lys Gly Val His Thr Gly Ser Arg Val Phe Met
                85                  90                  95
Gln Pro Ala Ser Glu Gly Thr Gly Ile Ile Ala Gly Gly Ala Met Arg
                100                 105                 110
Ala Val Leu Glu Val Ala Gly Val His Asn Val Leu Ala Lys Ala Tyr
        115                 120                 125
Gly Ser Thr Asn Pro Ile Asn Val Val Arg Ala Thr Ile Asp Gly Leu
    130                 135                 140
Glu Asn Met Asn Ser Pro Glu Met Val Ala Ala Lys Arg Gly Lys Ser
145                 150                 155                 160
Val Glu Glu Ile Leu Gly Lys
                165

<210> 316
<211> 117
<212> PRT
<213> E. Coli

<400> 316

<210> 317
<211> 177

<212> PRT
<213> E. Coli

<400> 317

<210> 318
<211> 130
<212> PRT
<213> E. Coli

<400> 318
Met Ser Met Gln Asp Pro Ile Ala Asp Met Leu Thr Arg Ile Arg Asn
1               5                   10                  15
Gly Gln Ala Ala Asn Lys Ala Ala Val Thr Met Pro Ser Ser Lys Leu
            20                  25                  30
Lys Val Ala Ile Ala Asn Val Leu Lys Glu Glu Gly Phe Ile Glu Asp
        35                  40                  45
Phe Lys Val Glu Gly Asp Thr Lys Pro Glu Leu Glu Leu Thr Leu Lys
    50                  55                  60
Tyr Phe Gln Gly Lys Ala Val Val Glu Ser Ile Gln Arg Val Ser Arg
65                  70                  75                  80
Pro Gly Leu Arg Ile Tyr Lys Arg Lys Asp Glu Leu Pro Lys Val Met
                85                  90                  95
Ala Gly Leu Gly Ile Ala Val Val Ser Thr Ser Lys Gly Val Met Thr
            100                 105                 110
Asp Arg Ala Ala Arg Gln Ala Gly Leu Gly Gly Glu Ile Ile Cys Tyr
        115                 120                 125
Val Ala
    130

<210> 319
<211> 101
<212> PRT
<213> E. Coli

<400> 319
Met Ala Lys Gln Ser Met Lys Ala Arg Glu Val Lys Arg Val Ala Leu
1               5                   10                  15
Ala Asp Lys Tyr Phe Ala Lys Arg Ala Glu Leu Lys Ala Ile Ile Ser
            20                  25                  30
Asp Val Asn Ala Ser Asp Glu Asp Arg Trp Asn Ala Val Leu Lys Leu
        35                  40                  45
Gln Thr Leu Pro Arg Asp Ser Ser Pro Ser Arg Gln Arg Asn Arg Cys
    50                  55                  60
Arg Gln Thr Gly Arg Pro His Gly Phe Leu Arg Lys Phe Gly Leu Ser
65                  70                  75                  80
Arg Ile Lys Val Arg Glu Ala Ala Met Arg Gly Glu Ile Pro Gly Leu
                85                  90                  95
Lys Lys Ala Ser Trp
            100

<210> 320
<211> 179
<212> PRT
<213> E. Coli

<400> 320

<210> 321Z
<211> 104
<212> PRT
<213> E. Coli

<400> 321

<210> 322
<211> 123
<212> PRT
<213> E. Coli

<400> 322

<210> 323
<211> 188
<212> PRT
<213> E. Coli

<400> 323
Met Phe Lys Gly Gln Lys Thr Leu Ala Ala Leu Ala Val Ser Leu Leu
1               5                   10                  15
Phe Thr Ala Pro Val Tyr Ala Ala Asp Glu Gly Ser Gly Glu Ile His
            20                  25                  30
Phe Lys Gly Glu Val Ile Glu Ala Pro Cys Glu Ile His Pro Glu Asp
        35                  40                  45
Ile Asp Lys Asn Ile Asp Leu Gly Gln Val Thr Thr Thr His Ile Asn
    50                  55                  60
Arg Glu His His Ser Asn Lys Val Ala Val Asp Ile Arg Leu Ile Asn
65                  70                  75                  80
Cys Asp Leu Pro Ala Ser Asp Asn Gly Ser Gly Met Pro Val Ser Lys
                85                  90                  95
Val Gly Val Thr Phe Asp Ser Thr Ala Lys Thr Thr Gly Ala Thr Pro
            100                 105                 110
Leu Leu Ser Asn Thr Ser Ala Gly Glu Ala Thr Gly Val Gly Val Arg
        115                 120                 125
Leu Met Asp Lys Asn Asp Gly Asn Ile Val Leu Gly Ser Ala Ala Pro
    130                 135                 140
Asp Leu Asp Leu Asp Ala Ser Ser Ser Glu Gln Thr Leu Asn Phe Phe

145                 150                 155                 160
Ala Trp Met Glu Gln Ile Asp Asn Ala Val Asp Val Thr Ala Gly Glu
                165                 170                 175
Val Thr Ala Asn Ala Thr Tyr Val Leu Asp Tyr Lys
            180                 185

<210> 324
<211> 427
<212> PRT
<213> E. Coli

<400> 324
Met Ala Asp Thr Lys Ala Lys Leu Thr Leu Asn Gly Asp Thr Ala Val
1               5                   10                  15
Glu Leu Asp Val Leu Lys Gly Thr Leu Gly Gln Asp Val Ile Asp Ile
            20                  25                  30
Arg Thr Leu Gly Ser Lys Gly Val Phe Thr Phe Asp Pro Gly Phe Thr
        35                  40                  45
Ser Thr Ala Ser Cys Glu Ser Lys Ile Thr Phe Ile Asp Gly Asp Glu
    50                  55                  60
Gly Ile Leu Leu His Arg Gly Phe Pro Ile Asp Gln Leu Ala Thr Asp
65                  70                  75                  80
Ser Asn Tyr Leu Glu Val Cys Tyr Ile Leu Leu Asn Gly Glu Lys Pro
                85                  90                  95
Thr Gln Glu Gln Tyr Asp Glu Phe Lys Thr Thr Val Thr Arg His Thr
            100                 105                 110
Met Ile His Glu Gln Ile Thr Arg Leu Phe His Ala Phe Arg Arg Asp
        115                 120                 125
Ser His Pro Met Ala Val Met Cys Gly Ile Thr Gly Ala Leu Ala Ala
    130                 135                 140
Phe Tyr His Asp Ser Leu Asp Val Asn Asn Pro Arg His Arg Glu Ile
145                 150                 155                 160
Ala Ala Phe Arg Leu Leu Ser Lys Met Pro Thr Met Ala Ala Met Cys
                165                 170                 175
Tyr Lys Tyr Ser Ile Gly Gln Pro Phe Val Tyr Pro Arg Asn Asp Leu
            180                 185                 190
Ser Tyr Ala Gly Asn Phe Leu Asn Met Met Phe Ser Thr Pro Cys Glu
        195                 200                 205
Pro Tyr Glu Val Asn Pro Ile Leu Glu Arg Ala Met Asp Arg Ile Leu
    210                 215                 220
Ile Leu His Ala Asp His Glu Gln Asn Ala Ser Thr Ser Thr Val Arg
225                 230                 235                 240
Thr Ala Gly Ser Ser Gly Ala Asn Pro Phe Ala Cys Ile Ala Ala Gly
                245                 250                 255
Ile Ala Ser Leu Trp Gly Pro Ala His Gly Gly Ala Asn Glu Ala Ala
            260                 265                 270
Leu Lys Met Leu Glu Glu Ile Ser Ser Val Lys His Ile Pro Glu Phe
        275                 280                 285
Val Arg Arg Ala Lys Asp Lys Asn Asp Ser Phe Arg Leu Met Gly Phe
    290                 295                 300
Gly His Arg Val Tyr Lys Asn Tyr Asp Pro Arg Ala Thr Val Met Arg
305                 310                 315                 320
Glu Thr Cys His Glu Val Leu Lys Glu Leu Gly Thr Lys Asp Asp Leu
                325                 330                 335
Leu Glu Val Ala Met Glu Leu Glu Asn Ile Ala Leu Asn Asp Pro Tyr
            340                 345                 350
Phe Ile Glu Lys Lys Leu Tyr Pro Asn Val Asp Phe Tyr Ser Gly Ile
        355                 360                 365
Ile Leu Lys Ala Met Gly Ile Pro Ser Ser Met Phe Thr Val Ile Phe
    370                 375                 380

Ala Met Ala Arg Thr Val Gly Trp Ile Ala His Trp Ser Glu Met His
385                 390                 395                 400
Ser Asp Gly Met Lys Ile Ala Arg Pro Arg Gln Leu Tyr Thr Gly Tyr
                405                 410                 415
Glu Lys Arg Asp Phe Lys Ser Asp Ile Lys Arg
            420                 425

<210> 325
<211> 477
<212> PRT
<213> E. Coli

<400> 325
Met Lys Val Thr Leu Pro Glu Phe Glu Arg Ala Gly Val Met Val Val
1               5                   10                  15
Gly Asp Val Met Leu Asp Arg Tyr Trp Tyr Gly Pro Thr Ser Arg Ile
            20                  25                  30
Ser Pro Glu Ala Pro Val Pro Val Val Lys Val Asn Thr Ile Glu Glu
        35                  40                  45
Arg Pro Gly Gly Ala Ala Asn Val Ala Met Asn Ile Ala Ser Leu Gly
    50                  55                  60
Ala Asn Ala Arg Leu Val Gly Leu Thr Gly Ile Asp Asp Ala Ala Arg
65                  70                  75                  80
Ala Leu Ser Lys Ser Leu Ala Asp Val Asn Val Lys Cys Asp Phe Val
                85                  90                  95
Ser Val Pro Thr His Pro Thr Ile Thr Lys Leu Arg Val Leu Ser Arg
            100                 105                 110
Asn Gln Gln Leu Ile Arg Leu Asp Phe Glu Glu Gly Phe Glu Gly Val
        115                 120                 125
Asp Pro Gln Pro Leu His Glu Arg Ile Asn Gln Ala Leu Ser Ser Ile
    130                 135                 140
Gly Ala Leu Val Leu Ser Asp Tyr Ala Lys Gly Ala Leu Ala Ser Val
145                 150                 155                 160
Gln Gln Met Ile Gln Leu Ala Arg Lys Ala Gly Val Pro Val Leu Ile
                165                 170                 175
Asp Pro Lys Gly Thr Asp Phe Glu Arg Tyr Arg Gly Ala Thr Leu Leu
            180                 185                 190
Thr Pro Asn Leu Ser Glu Phe Glu Ala Val Val Gly Lys Cys Lys Thr
        195                 200                 205
Glu Glu Glu Ile Val Glu Arg Gly Met Lys Leu Ile Ala Asp Tyr Glu
    210                 215                 220
Leu Ser Ala Leu Leu Val Thr Arg Ser Glu Gln Gly Met Ser Leu Leu
225                 230                 235                 240
Gln Pro Gly Lys Ala Pro Leu His Met Pro Thr Gln Ala Gln Glu Val
                245                 250                 255
Tyr Asp Val Thr Gly Ala Gly Asp Thr Val Ile Gly Val Leu Ala Ala
            260                 265                 270
Thr Leu Ala Ala Gly Asn Ser Leu Glu Glu Ala Cys Phe Phe Ala Asn
        275                 280                 285
Ala Ala Ala Gly Val Val Val Gly Lys Leu Gly Thr Ser Thr Val Ser
    290                 295                 300
Pro Ile Glu Leu Glu Asn Ala Val Arg Gly Arg Ala Asp Thr Gly Phe
305                 310                 315                 320
Gly Val Met Thr Glu Glu Glu Leu Lys Leu Ala Val Ala Ala Ala Arg
                325                 330                 335
Lys Arg Gly Glu Lys Val Val Met Thr Asn Gly Val Phe Asp Ile Leu
            340                 345                 350
His Ala Gly His Val Ser Tyr Leu Ala Asn Ala Arg Lys Leu Gly Asp
        355                 360                 365
Arg Leu Ile Val Ala Val Asn Ser Asp Ala Ser Thr Lys Arg Leu Lys

370                 375                 380
Gly Asp Ser Arg Pro Val Asn Pro Leu Glu Gln Arg Met Ile Val Leu
385                 390                 395                 400
Gly Ala Leu Glu Ala Val Asp Trp Val Val Ser Phe Glu Glu Asp Thr
                405                 410                 415
Pro Gln Arg Leu Ile Ala Gly Ile Leu Pro Asp Leu Leu Val Lys Gly
            420                 425                 430
Gly Asp Tyr Lys Pro Glu Glu Ile Ala Gly Ser Lys Glu Val Trp Ala
        435                 440                 445
Asn Gly Gly Glu Val Leu Val Leu Asn Phe Glu Asp Gly Cys Ser Thr
    450                 455                 460
Thr Asn Ile Ile Lys Lys Ile Gln Gln Asp Lys Lys Gly
465                 470                 475

<210> 326
<211> 946
<212> PRT
<213> E. Coli

<400> 326
Met Lys Pro Leu Ser Ser Pro Leu Gln Gln Tyr Trp Gln Thr Val Val
1               5                   10                  15
Glu Arg Leu Pro Glu Pro Leu Ala Glu Glu Ser Leu Ser Ala Gln Ala
            20                  25                  30
Lys Ser Val Leu Thr Phe Ser Asp Phe Val Gln Asp Ser Val Ile Ala
        35                  40                  45
His Pro Glu Trp Leu Thr Glu Leu Glu Ser Gln Pro Pro Gln Ala Asp
    50                  55                  60
Glu Trp Gln His Tyr Ala Ala Trp Leu Gln Glu Ala Leu Cys Asn Val
65                  70                  75                  80
Ser Asp Glu Ala Gly Leu Met Arg Glu Leu Arg Leu Phe Arg Arg Arg
                85                  90                  95
Ile Met Val Arg Ile Ala Trp Ala Gln Thr Leu Ala Leu Val Thr Glu
            100                 105                 110
Glu Ser Ile Leu Gln Gln Leu Ser Tyr Leu Ala Glu Thr Leu Ile Val
        115                 120                 125
Ala Ala Arg Asp Trp Leu Tyr Asp Ala Cys Cys Arg Glu Trp Gly Thr
    130                 135                 140
Pro Cys Asn Ala Gln Gly Glu Ala Gln Pro Leu Leu Ile Leu Gly Met
145                 150                 155                 160
Gly Lys Leu Gly Gly Gly Glu Leu Asn Phe Ser Ser Asp Ile Asp Leu
                165                 170                 175
Ile Phe Ala Trp Pro Glu His Gly Cys Thr Gln Gly Gly Arg Arg Glu
            180                 185                 190
Leu Asp Asn Ala Gln Phe Phe Thr Arg Met Gly Gln Arg Leu Ile Lys
        195                 200                 205
Val Leu Asp Gln Pro Thr Gln Asp Gly Phe Val Tyr Arg Val Asp Met
    210                 215                 220
Arg Leu Arg Pro Phe Gly Glu Ser Gly Pro Leu Val Leu Ser Phe Ala
225                 230                 235                 240
Ala Leu Glu Asp Tyr Tyr Gln Glu Gln Gly Arg Asp Trp Glu Arg Tyr
                245                 250                 255
Ala Met Val Lys Ala Arg Ile Met Gly Asp Ser Glu Gly Val Tyr Ala
            260                 265                 270
Asn Glu Leu Arg Ala Met Leu Arg Pro Phe Val Phe Arg Arg Tyr Ile
        275                 280                 285
Asp Phe Ser Val Ile Gln Ser Leu Arg Asn Met Lys Gly Met Ile Ala
    290                 295                 300
Arg Glu Val Arg Arg Arg Gly Leu Thr Asp Asn Ile Lys Leu Gly Ala
305                 310                 315                 320

Gly Gly Ile Arg Glu Ile Glu Phe Ile Val Gln Val Phe Gln Leu Ile
                325                 330                 335
Arg Gly Gly Arg Glu Pro Ser Leu Gln Ser Arg Ser Leu Leu Pro Thr
            340                 345                 350
Leu Ser Ala Ile Ala Glu Leu His Leu Leu Ser Glu Asn Asp Ala Glu
        355                 360                 365
Gln Leu Arg Val Ala Tyr Leu Phe Leu Arg Arg Leu Glu Asn Leu Leu
    370                 375                 380
Gln Ser Ile Asn Asp Glu Gln Thr Gln Thr Leu Pro Ser Asp Glu Leu
385                 390                 395                 400
Asn Arg Ala Arg Leu Ala Trp Ala Met Asp Phe Ala Asp Trp Pro Gln
                405                 410                 415
Leu Thr Gly Ala Leu Thr Ala His Met Thr Asn Val Arg Arg Val Phe
            420                 425                 430
Asn Glu Leu Ile Gly Asp Asp Glu Ser Glu Thr Gln Glu Glu Ser Leu
        435                 440                 445
Ser Glu Gln Trp Arg Glu Leu Trp Gln Asp Ala Leu Gln Glu Asp Asp
    450                 455                 460
Thr Thr Pro Val Leu Ala His Leu Ser Glu Asp Asp Arg Lys Gln Val
465                 470                 475                 480
Leu Thr Leu Ile Ala Asp Phe Arg Lys Glu Leu Asp Lys Arg Thr Ile
                485                 490                 495
Gly Pro Arg Gly Arg Gln Val Leu Asp His Leu Met Pro His Leu Leu
            500                 505                 510
Ser Asp Val Cys Ala Arg Glu Asp Ala Ala Val Thr Leu Ser Arg Ile
        515                 520                 525
Thr Ala Leu Leu Val Gly Ile Val Thr Arg Thr Thr Tyr Leu Glu Leu
    530                 535                 540
Leu Ser Glu Phe Pro Ala Ala Leu Lys His Leu Ile Ser Leu Cys Ala
545                 550                 555                 560
Ala Ser Pro Met Ile Ala Ser Gln Leu Ala Arg Tyr Pro Leu Leu Leu
                565                 570                 575
Asp Glu Leu Leu Asp Pro Asn Thr Leu Tyr Gln Pro Thr Ala Thr Asp
            580                 585                 590
Ala Tyr Arg Asp Glu Leu Arg Gln Tyr Leu Leu Arg Val Pro Glu Asp
        595                 600                 605
Asp Glu Glu Gln Gln Leu Glu Ala Leu Arg Gln Phe Lys Gln Ala Gln
    610                 615                 620
Leu Leu Arg Ile Ala Ala Ala Asp Ile Ala Gly Thr Leu Pro Val Met
625                 630                 635                 640
Lys Val Ser Asp His Leu Thr Trp Leu Ala Glu Ala Met Ile Asp Ala
                645                 650                 655
Val Val Gln Gln Ala Trp Val Gln Met Val Ala Arg Tyr Gly Lys Pro
            660                 665                 670
Asn His Leu Asn Glu Arg Glu Gly Arg Gly Phe Ala Val Val Gly Tyr
        675                 680                 685
Gly Lys Leu Gly Gly Trp Glu Leu Gly Tyr Ser Ser Asp Leu Asp Leu
    690                 695                 700
Ile Phe Leu His Asp Cys Pro Met Asp Ala Met Thr Asp Gly Glu Arg
705                 710                 715                 720
Glu Ile Asp Gly Arg Gln Phe Tyr Leu Arg Leu Ala Gln Arg Ile Met
                725                 730                 735
His Leu Phe Ser Thr Arg Thr Ser Ser Gly Ile Leu Tyr Glu Val Asp
            740                 745                 750
Ala Arg Leu Arg Pro Ser Gly Ala Ala Gly Met Leu Val Thr Ser Ala
        755                 760                 765
Glu Ala Phe Ala Asp Tyr Gln Lys Asn Glu Ala Trp Thr Trp Glu His
    770                 775                 780
Gln Ala Leu Val Arg Ala Arg Val Val Tyr Gly Asp Pro Gln Leu Thr
785                 790                 795                 800
Ala His Phe Asp Ala Val Arg Arg Glu Ile Met Thr Leu Pro Arg Glu

                    805                     810                     815
Gly Lys Thr Leu Gln Thr Glu Val Arg Glu Met Arg Glu Lys Met Arg
                820                     825                     830
Ala His Leu Gly Asn Lys His Arg Asp Arg Phe Asp Ile Lys Ala Asp
        835                     840                     845
Glu Gly Gly Ile Thr Asp Ile Glu Phe Ile Thr Gln Tyr Leu Val Leu
    850                     855                     860
Arg Tyr Ala His Glu Lys Pro Lys Leu Thr Arg Trp Ser Asp Asn Val
865                     870                     875                     880
Arg Ile Leu Glu Leu Leu Ala Gln Asn Asp Ile Met Glu Glu Gln Glu
                885                     890                     895
Ala Met Ala Leu Thr Arg Ala Tyr Thr Thr Leu Arg Asp Glu Leu His
            900                     905                     910
His Leu Ala Leu Gln Glu Leu Pro Gly His Val Ser Glu Asp Cys Phe
        915                     920                     925
Thr Ala Glu Arg Glu Leu Val Arg Ala Ser Trp Gln Lys Trp Leu Val
    930                     935                     940
Glu Glu
945

<210> 327
<211> 433
<212> PRT
<213> E. Coli

<400> 327

<210> 328
<211> 70
<212> PRT
<213> E. Coli

<400> 328
Met Ser Gly Lys Met Thr Gly Ile Val Lys Trp Phe Asn Ala Asp Lys
 1               5                  10                  15
Gly Phe Gly Phe Ile Thr Pro Asp Asp Gly Ser Lys Asp Val Phe Val
            20                  25                  30
His Phe Ser Ala Ile Gln Asn Asp Gly Tyr Lys Ser Leu Asp Glu Gly
        35                  40                  45
Gln Lys Val Ser Phe Thr Ile Glu Ser Gly Ala Lys Gly Pro Ala Ala
    50                  55                  60
Gly Asn Val Thr Ser Leu
65                  70

<210> 329
<211> 523
<212> PRT
<213> E. Coli

<400> 329
Met Arg Asp Ile Val Asp Pro Val Phe Ser Ile Gly Ile Ser Ser Leu
 1               5                  10                  15
Trp Asp Glu Leu Arg His Met Pro Ala Gly Gly Val Trp Trp Phe Asn
            20                  25                  30
Val Asp Arg His Glu Asp Ala Ile Ser Leu Ala Asn Gln Thr Ile Ala
        35                  40                  45
Ser Gln Ala Glu Thr Ala His Val Ala Val Ile Ser Met Asp Ser Asp
    50                  55                  60
Pro Ala Lys Ile Phe Gln Leu Asp Asp Ser Gln Gly Pro Glu Lys Ile
65                  70                  75                  80

Lys Leu Phe Ser Met Leu Asn His Glu Lys Gly Leu Tyr Tyr Leu Thr
                85                  90                  95
Arg Asp Leu Gln Cys Ser Ile Asp Pro His Asn Tyr Leu Phe Ile Leu
            100                 105                 110
Val Cys Ala Asn Asn Ala Trp Gln Asn Ile Pro Ala Glu Arg Leu Arg
        115                 120                 125
Ser Trp Leu Asp Lys Met Asn Lys Trp Ser Arg Leu Asn His Cys Ser
    130                 135                 140
Leu Leu Val Ile Asn Pro Gly Asn Asn Asn Asp Lys Gln Phe Ser Leu
145                 150                 155                 160
Leu Leu Glu Glu Tyr Arg Ser Leu Phe Gly Leu Ala Ser Leu Arg Phe
                165                 170                 175
Gln Gly Asp Gln His Leu Leu Asp Ile Ala Phe Trp Cys Asn Glu Lys
            180                 185                 190
Gly Val Ser Ala Arg Gln Gln Leu Ser Val Gln Gln Gln Asn Gly Ile
        195                 200                 205
Trp Thr Leu Val Gln Ser Glu Glu Ala Glu Ile Gln Pro Arg Ser Asp
    210                 215                 220
Glu Lys Arg Ile Leu Ser Asn Val Ala Val Leu Glu Gly Ala Pro Pro
225                 230                 235                 240
Leu Ser Glu His Trp Gln Leu Phe Asn Asn Asn Glu Val Leu Phe Asn
                245                 250                 255
Glu Ala Arg Thr Ala Gln Ala Ala Thr Val Val Phe Ser Leu Gln Gln
            260                 265                 270
Asn Ala Gln Ile Glu Pro Leu Ala Arg Ser Ile His Thr Leu Arg Arg
        275                 280                 285
Gln Arg Gly Ser Ala Met Lys Ile Leu Val Arg Glu Asn Thr Ala Ser
    290                 295                 300
Leu Arg Ala Thr Asp Glu Arg Leu Leu Leu Ala Cys Gly Ala Asn Met
305                 310                 315                 320
Val Ile Pro Trp Asn Ala Pro Leu Ser Arg Cys Leu Thr Met Ile Glu
                325                 330                 335
Ser Val Gln Gly Gln Lys Phe Ser Arg Tyr Val Pro Glu Asp Ile Thr
            340                 345                 350
Thr Leu Leu Ser Met Thr Gln Pro Leu Lys Leu Arg Gly Phe Gln Lys
        355                 360                 365
Trp Asp Val Phe Cys Asn Ala Val Asn Asn Met Met Asn Asn Pro Leu
    370                 375                 380
Leu Pro Ala His Gly Lys Gly Val Leu Val Ala Leu Arg Pro Val Pro
385                 390                 395                 400
Gly Ile Arg Val Glu Gln Ala Leu Thr Leu Cys Arg Pro Asn Arg Thr
                405                 410                 415
Gly Asp Ile Met Thr Ile Gly Gly Asn Arg Leu Val Leu Phe Leu Ser
            420                 425                 430
Phe Cys Arg Ile Asn Asp Leu Asp Thr Ala Leu Asn His Ile Phe Pro
        435                 440                 445
Leu Pro Thr Gly Asp Ile Phe Ser Asn Arg Met Val Trp Phe Glu Asp
    450                 455                 460
Asp Gln Ile Ser Ala Glu Leu Val Gln Met Arg Leu Leu Ala Pro Glu
465                 470                 475                 480
Gln Trp Gly Met Pro Leu Pro Leu Thr Gln Ser Ser Lys Pro Val Ile
                485                 490                 495
Asn Ala Glu His Asp Gly Arg His Trp Arg Arg Ile Pro Glu Pro Met
            500                 505                 510
Arg Leu Leu Asp Asp Ala Val Glu Arg Ser Ser
        515                 520

<210> 330
<211> 62
<212> PRT

<213> E. Coli

<400> 330
Met Thr Ile Ser Asp Ile Ile Glu Ile Ile Val Val Cys Ala Leu Ile
1               5                   10                  15
Phe Phe Pro Leu Gly Tyr Leu Ala Arg His Ser Leu Arg Arg Ile Arg
            20                  25                  30
Asp Thr Leu Arg Leu Phe Phe Ala Lys Pro Arg Tyr Val Lys Pro Ala
        35                  40                  45
Gly Thr Leu Arg Arg Thr Glu Lys Ala Arg Ala Thr Lys Lys
    50                  55                  60

<210> 331
<211> 559
<212> PRT
<213> E. Coli

<400> 331
Met Thr Gln Phe Thr Gln Asn Thr Ala Met Pro Ser Ser Leu Trp Gln
1               5                   10                  15
Tyr Trp Arg Gly Leu Ser Gly Trp Asn Phe Tyr Phe Leu Val Lys Phe
            20                  25                  30
Gly Leu Leu Trp Ala Gly Tyr Leu Asn Phe His Pro Leu Leu Asn Leu
        35                  40                  45
Val Phe Ala Ala Phe Leu Leu Met Pro Leu Pro Arg Tyr Ser Leu His
    50                  55                  60
Arg Leu Arg His Trp Ile Ala Leu Pro Ile Gly Phe Ala Leu Phe Trp
65                  70                  75                  80
His Asp Thr Trp Leu Pro Gly Pro Glu Ser Ile Met Ser Gln Gly Ser
                85                  90                  95
Gln Val Ala Gly Phe Ser Thr Asp Tyr Leu Ile Asp Leu Val Thr Arg
            100                 105                 110
Phe Ile Asn Trp Gln Met Ile Gly Ala Ile Phe Val Leu Leu Val Ala
        115                 120                 125
Trp Leu Phe Leu Ser Gln Trp Ile Arg Ile Thr Val Phe Val Val Ala
    130                 135                 140
Ile Leu Leu Trp Leu Asn Val Leu Thr Leu Ala Gly Pro Ser Phe Ser
145                 150                 155                 160
Leu Trp Pro Ala Gly Gln Pro Thr Thr Thr Val Thr Thr Thr Gly Gly
                165                 170                 175
Asn Ala Ala Ala Thr Val Ala Ala Thr Gly Gly Ala Pro Val Val Gly
            180                 185                 190
Asp Met Pro Ala Gln Thr Ala Pro Pro Thr Thr Ala Asn Leu Asn Ala
        195                 200                 205
Trp Leu Asn Asn Phe Tyr Asn Ala Glu Ala Lys Arg Lys Ser Thr Phe
    210                 215                 220
Pro Ser Ser Leu Pro Ala Asp Ala Gln Pro Phe Glu Leu Leu Val Ile
225                 230                 235                 240
Asn Ile Cys Ser Leu Ser Trp Ser Asp Ile Glu Ala Ala Gly Leu Met
                245                 250                 255
Ser His Pro Leu Trp Ser His Phe Asp Ile Glu Phe Lys Asn Phe Asn
            260                 265                 270
Ser Ala Thr Ser Tyr Ser Gly Pro Ala Ala Ile Arg Leu Leu Arg Ala
        275                 280                 285
Ser Cys Gly Gln Thr Ser His Thr Asn Leu Tyr Gln Pro Ala Asn Asn
    290                 295                 300
Asp Cys Tyr Leu Phe Asp Asn Leu Ser Lys Leu Gly Phe Thr Gln His
305                 310                 315                 320
Leu Met Met Gly His Asn Gly Gln Phe Gly Gly Phe Leu Lys Glu Val
                325                 330                 335

Arg Glu Asn Gly Gly Met Gln Ser Glu Leu Met Asp Gln Thr Asn Leu
            340                 345                 350
Pro Val Ile Leu Leu Gly Phe Asp Gly Ser Pro Val Tyr Asp Asp Thr
        355                 360                 365
Ala Val Leu Asn Arg Trp Leu Asp Val Thr Glu Lys Asp Lys Asn Ser
    370                 375                 380
Arg Ser Ala Thr Phe Tyr Asn Thr Leu Pro Leu His Asp Gly Asn His
385                 390                 395                 400
Tyr Pro Gly Val Ser Lys Thr Ala Asp Tyr Lys Ala Arg Ala Gln Lys
                405                 410                 415
Phe Phe Asp Glu Leu Asp Ala Phe Phe Thr Glu Leu Glu Lys Ser Gly
            420                 425                 430
Arg Lys Val Met Val Val Val Val Pro Glu His Gly Gly Ala Leu Lys
        435                 440                 445
Gly Asp Arg Met Gln Val Ser Gly Leu Arg Asp Ile Pro Ser Pro Ser
    450                 455                 460
Ile Thr Asp Val Pro Val Gly Val Lys Phe Phe Gly Met Lys Ala Pro
465                 470                 475                 480
His Gln Gly Ala Pro Ile Val Ile Glu Gln Pro Ser Ser Phe Leu Ala
                485                 490                 495
Ile Ser Asp Leu Val Val Arg Val Leu Asp Gly Lys Ile Phe Thr Glu
            500                 505                 510
Asp Asn Val Asp Trp Lys Lys Leu Thr Ser Gly Leu Pro Gln Thr Ala
        515                 520                 525
Pro Val Ser Glu Asn Ser Asn Ala Val Val Ile Gln Tyr Gln Asp Lys
    530                 535                 540
Pro Tyr Val Arg Leu Asn Gly Gly Asp Trp Val Pro Tyr Pro Gln
545                 550                 555

<210> 332
<211> 127
<212> PRT
<213> E. Coli

<400> 332
Met Glu Gly Ser Arg Met Lys Tyr Arg Ile Ala Leu Ala Val Ser Leu
1               5                   10                  15
Phe Ala Leu Ser Ala Gly Ser Tyr Ala Thr Thr Leu Cys Gln Glu Lys
            20                  25                  30
Glu Gln Asn Ile Leu Lys Glu Ile Ser Tyr Ala Glu Lys His Gln Asn
        35                  40                  45
Gln Asn Arg Ile Asp Gly Leu Asn Lys Ala Leu Ser Glu Val Arg Ala
    50                  55                  60
Asn Cys Ser Asp Ser Gln Leu Arg Ala Asp His Gln Lys Lys Ile Ala
65                  70                  75                  80
Lys Gln Lys Asp Glu Val Ala Glu Arg Gln Gln Asp Leu Ala Glu Ala
                85                  90                  95
Lys Gln Lys Gly Asp Ala Asp Lys Ile Ala Lys Arg Glu Arg Lys Leu
            100                 105                 110
Ala Glu Ala Gln Glu Glu Leu Lys Lys Leu Glu Ala Arg Asp Tyr
        115                 120                 125

<210> 333
<211> 101
<212> PRT
<213> E. Coli

<400> 333
Met Ser Lys Glu His Thr Thr Glu His Leu Arg Ala Glu Leu Lys Ser

1               5                   10                  15
Leu Ser Asp Thr Leu Glu Glu Val Leu Ser Ser Ser Gly Glu Lys Ser
            20                  25                  30
Lys Glu Glu Leu Ser Lys Ile Arg Ser Lys Ala Glu Gln Ala Leu Lys
        35                  40                  45
Gln Ser Arg Tyr Arg Leu Gly Glu Thr Gly Asp Ala Ile Ala Lys Gln
    50                  55                  60
Thr Arg Val Ala Ala Ala Arg Ala Asp Glu Tyr Val Arg Glu Asn Pro
65                  70                  75                  80
Trp Thr Gly Val Gly Ile Gly Ala Ala Ile Gly Val Val Leu Gly Val
                85                  90                  95
Leu Leu Ser Arg Arg
            100

<210> 334
<211> 134
<212> PRT
<213> E. Coli

<400> 334
Met Ala Asp Thr His His Ala Gln Gly Pro Gly Lys Ser Val Leu Gly
1               5                   10                  15
Ile Gly Gln Arg Ile Val Ser Ile Met Val Glu Met Val Glu Thr Arg
            20                  25                  30
Leu Arg Leu Ala Val Val Glu Leu Glu Glu Glu Lys Ala Asn Leu Phe
        35                  40                  45
Gln Leu Leu Leu Met Leu Gly Leu Thr Met Leu Phe Ala Ala Phe Gly
    50                  55                  60
Leu Met Ser Leu Met Val Leu Ile Ile Trp Ala Val Asp Pro Gln Tyr
65                  70                  75                  80
Arg Leu Asn Ala Met Ile Ala Thr Thr Val Val Leu Leu Leu Leu Ala
                85                  90                  95
Leu Ile Gly Gly Ile Trp Thr Leu Arg Lys Ser Arg Lys Ser Thr Leu
            100                 105                 110
Leu Arg His Thr Arg His Glu Leu Ala Asn Asp Arg Gln Leu Leu Glu
        115                 120                 125
Glu Glu Ser Arg Glu Gln
    130

<210> 335
<211> 99
<212> PRT
<213> E. Coli

<400> 335
Met Ser Ser Lys Val Glu Arg Glu Arg Arg Lys Ala Gln Leu Leu Ser
1               5                   10                  15
Gln Ile Gln Gln Gln Arg Leu Asp Leu Ser Ala Ser Arg Arg Glu Trp
            20                  25                  30
Leu Glu Thr Thr Gly Ala Tyr Asp Arg Arg Trp Asn Met Leu Leu Ser
        35                  40                  45
Leu Arg Ser Trp Ala Leu Val Gly Ser Ser Val Met Ala Ile Trp Thr
    50                  55                  60
Ile Arg His Pro Asn Met Leu Val Arg Trp Ala Arg Arg Gly Phe Gly
65                  70                  75                  80
Val Trp Ser Ala Trp Arg Leu Val Lys Thr Thr Leu Lys Gln Gln Gln
                85                  90                  95
Leu Arg Gly

<210> 336
<211> 160
<212> PRT
<213> E. Coli

<400> 336
Met Ile Leu Ser Ile Asp Ser Asn Asp Ala Asn Thr Ala Pro Leu His
1               5                   10                  15
Lys Lys Thr Ile Ser Ser Leu Ser Gly Ala Val Glu Ser Met Met Lys
            20                  25                  30
Lys Leu Glu Asp Val Gly Val Leu Val Ala Arg Ile Leu Met Pro Ile
        35                  40                  45
Leu Phe Ile Thr Ala Gly Trp Gly Lys Ile Thr Gly Tyr Ala Gly Thr
    50                  55                  60
Gln Gln Tyr Met Glu Ala Met Gly Val Pro Gly Phe Met Leu Pro Leu
65                  70                  75                  80
Val Ile Leu Leu Glu Phe Gly Gly Gly Leu Ala Ile Leu Phe Gly Phe
                85                  90                  95
Leu Thr Arg Thr Thr Ala Leu Phe Thr Ala Gly Phe Thr Leu Leu Thr
            100                 105                 110
Ala Phe Leu Phe His Ser Asn Phe Ala Glu Gly Val Asn Ser Leu Met
        115                 120                 125
Phe Met Lys Asn Leu Thr Ile Ser Gly Gly Phe Leu Leu Leu Ala Ile
    130                 135                 140
Thr Gly Pro Gly Ala Tyr Ser Ile Asp Arg Leu Leu Asn Lys Lys Trp
145                 150                 155                 160

<210> 337
<211> 296
<212> PRT
<213> E. Coli

<400> 337
Met Ile Lys Lys Thr Thr Glu Ile Asp Ala Ile Leu Leu Asn Leu Asn
1               5                   10                  15
Lys Ala Ile Asp Ala His Tyr Gln Trp Leu Val Ser Met Phe His Ser
            20                  25                  30
Val Val Ala Arg Asp Ala Ser Lys Pro Glu Ile Thr Asp Asn His Ser
        35                  40                  45
Tyr Gly Leu Cys Gln Phe Gly Arg Trp Ile Asp His Leu Gly Pro Leu
    50                  55                  60
Asp Asn Asp Glu Leu Pro Tyr Val Arg Leu Met Asp Ser Ala His Gln
65                  70                  75                  80
His Met His Asn Cys Gly Arg Glu Leu Met Leu Ala Ile Val Glu Asn
                85                  90                  95
His Trp Gln Asp Ala His Phe Asp Ala Phe Gln Glu Gly Leu Leu Ser
            100                 105                 110
Phe Thr Ala Ala Leu Thr Asp Tyr Lys Ile Tyr Leu Leu Thr Ile Arg
        115                 120                 125
Ser Asn Met Asp Val Leu Thr Gly Leu Pro Gly Arg Arg Val Leu Asp
    130                 135                 140
Glu Ser Phe Asp His Gln Leu Arg Asn Ala Glu Pro Leu Asn Leu Tyr
145                 150                 155                 160
Leu Met Leu Leu Asp Ile Asp Arg Phe Lys Leu Val Asn Asp Thr Tyr
                165                 170                 175

Gly His Leu Ile Gly Asp Val Val Leu Arg Thr Leu Ala Thr Tyr Leu
            180                 185                 190
Ala Ser Trp Thr Arg Asp Tyr Glu Thr Val Tyr Arg Tyr Gly Gly Glu
        195                 200                 205
Glu Phe Ile Ile Ile Val Lys Ala Ala Asn Asp Glu Glu Ala Cys Arg
    210                 215                 220
Ala Gly Val Arg Ile Cys Gln Leu Val Asp Asn His Ala Ile Thr His
225                 230                 235                 240
Ser Glu Gly His Ile Asn Ile Thr Val Thr Ala Gly Val Ser Arg Ala
                245                 250                 255
Phe Pro Glu Glu Pro Leu Asp Val Val Ile Gly Arg Ala Asp Arg Ala
            260                 265                 270
Met Tyr Glu Gly Lys Gln Thr Gly Arg Asn Arg Cys Met Phe Ile Asp
        275                 280                 285
Glu Gln Asn Val Ile Asn Arg Val
    290                 295

<210> 338
<211> 203
<212> PRT
<213> E. Coli

<400> 338
Met Arg Leu Arg Val Val Pro Gly Phe Ile Ser Pro Pro Pro Gly Phe
1               5                   10                  15
Gly Gly Leu Gly Tyr Thr Pro Thr Ala Arg Ala Cys Val Asn Ile Ser
            20                  25                  30
Ile Pro Leu Gln Leu Arg Val Ile Asp Met Leu Asp Val Phe Thr Pro
        35                  40                  45
Leu Leu Lys Leu Phe Ala Asn Glu Pro Leu Glu Arg Leu Met Tyr Thr
    50                  55                  60
Ile Ile Ile Phe Gly Leu Thr Leu Trp Leu Ile Pro Lys Glu Phe Thr
65                  70                  75                  80
Val Ala Phe Asn Ala Tyr Thr Glu Ile Pro Trp Leu Phe Gln Ile Ile
                85                  90                  95
Val Phe Ala Phe Ser Phe Val Val Ala Ile Ser Phe Ser Arg Leu Arg
            100                 105                 110
Ala His Ile Gln Lys His Tyr Ser Leu Leu Pro Glu Gln Arg Val Leu
        115                 120                 125
Leu Arg Leu Ser Glu Lys Glu Ile Ala Val Phe Lys Asp Phe Leu Lys
    130                 135                 140
Thr Gly Asn Leu Ile Ile Thr Ser Pro Cys Arg Asn Pro Val Met Lys
145                 150                 155                 160
Lys Leu Glu Arg Lys Gly Ile Ile Gln His Gln Ser Asp Ser Ala Asn
                165                 170                 175
Cys Ser Tyr Tyr Leu Val Thr Glu Lys Tyr Ser His Phe Met Lys Leu
            180                 185                 190
Phe Trp Asn Ser Arg Ser Arg Arg Phe Asn Arg
        195                 200

<210> 339
<211> 58
<212> PRT
<213> E. Coli

<400> 339
Met Leu Leu Gln Pro Ser Ala Arg Thr Ser Phe Gly Phe Lys Cys Phe

1               5                   10                  15
Ala Phe Gly Ile Arg His Gly Ser Glu Arg Ser Ile Leu Val Gly Glu
            20                  25                  30
His Ala Ala His Gln Gly Phe Val Val Ala Glu Val Asp Phe Leu His
        35                  40                  45
Phe Ala Asn Leu Thr Ser Cys Cys Tyr Val
    50                  55

<210> 340
<211> 1426
<212> PRT
<213> E. Coli

<400> 340
Met Ser Gly Lys Pro Ala Ala Arg Gln Gly Asp Met Thr Gln Tyr Gly
 1               5                  10                  15
Gly Pro Ile Val Gln Gly Ser Ala Gly Val Arg Ile Gly Ala Pro Thr
            20                  25                  30
Gly Val Ala Cys Ser Val Cys Pro Gly Gly Met Thr Ser Gly Asn Pro
        35                  40                  45
Val Asn Pro Leu Leu Gly Ala Lys Val Leu Pro Gly Glu Thr Asp Leu
    50                  55                  60
Ala Leu Pro Gly Pro Leu Pro Phe Ile Leu Ser Arg Thr Tyr Ser Ser
65                  70                  75                  80
Tyr Arg Thr Lys Thr Pro Ala Pro Val Gly Val Phe Gly Pro Gly Trp
                85                  90                  95
Lys Ala Pro Ser Asp Ile Arg Leu Gln Leu Arg Asp Asp Gly Leu Ile
            100                 105                 110
Leu Asn Asp Asn Gly Gly Arg Ser Ile His Phe Glu Pro Leu Leu Pro
        115                 120                 125
Gly Glu Ala Val Tyr Ser Arg Ser Glu Ser Met Trp Leu Val Arg Gly
    130                 135                 140
Gly Lys Ala Ala Gln Pro Asp Gly His Thr Leu Ala Arg Leu Trp Gly
145                 150                 155                 160
Ala Leu Pro Pro Asp Ile Arg Leu Ser Pro His Leu Tyr Leu Ala Thr
                165                 170                 175
Asn Ser Ala Gln Gly Pro Trp Trp Ile Leu Gly Trp Ser Glu Arg Val
            180                 185                 190
Pro Gly Ala Glu Asp Val Leu Pro Ala Pro Leu Pro Pro Tyr Arg Val
        195                 200                 205
Leu Thr Gly Met Ala Asp Arg Phe Gly Arg Thr Leu Thr Tyr Arg Arg
    210                 215                 220
Glu Ala Ala Gly Asp Leu Ala Gly Glu Ile Thr Gly Val Thr Asp Gly
225                 230                 235                 240
Ala Gly Arg Glu Phe Arg Leu Val Leu Thr Thr Gln Ala Gln Arg Ala
                245                 250                 255
Glu Glu Ala Arg Thr Ser Ser Leu Ser Ser Ser Asp Ser Ser Arg Pro
            260                 265                 270
Leu Ser Ala Ser Ala Phe Pro Asp Thr Leu Pro Gly Thr Glu Tyr Gly
        275                 280                 285
Pro Asp Arg Gly Ile Arg Leu Ser Ala Val Trp Leu Met His Asp Pro
    290                 295                 300
Ala Tyr Pro Glu Ser Leu Pro Ala Ala Pro Leu Val Arg Tyr Thr Tyr
305                 310                 315                 320
Thr Glu Ala Gly Glu Leu Leu Ala Val Tyr Asp Arg Ser Asn Thr Gln
                325                 330                 335
Val Arg Ala Phe Thr Tyr Asp Ala Gln His Pro Gly Arg Met Val Ala
            340                 345                 350
His Arg Tyr Ala Gly Arg Pro Glu Met Arg Tyr Arg Tyr Asp Asp Thr
        355                 360                 365

Gly Arg Val Val Glu Gln Leu Asn Pro Ala Gly Leu Ser Tyr Arg Tyr
    370                 375                 380
Leu Tyr Glu Gln Asp Arg Ile Thr Val Thr Asp Ser Leu Asn Arg Arg
385                 390                 395                 400
Glu Val Leu His Thr Glu Gly Gly Ala Gly Leu Lys Arg Val Val Lys
                405                 410                 415
Lys Glu Leu Ala Asp Gly Ser Val Thr Arg Ser Gly Tyr Asp Ala Ala
            420                 425                 430
Gly Arg Leu Thr Ala Gln Thr Asp Ala Ala Gly Arg Arg Thr Glu Tyr
        435                 440                 445
Gly Leu Asn Val Val Ser Gly Asp Ile Thr Asp Ile Thr Thr Pro Asp
    450                 455                 460
Gly Arg Glu Thr Lys Phe Tyr Tyr Asn Asp Gly Asn Gln Leu Thr Ala
465                 470                 475                 480
Val Val Ser Pro Asp Gly Leu Glu Ser Arg Arg Glu Tyr Asp Glu Pro
                485                 490                 495
Gly Arg Leu Val Ser Glu Thr Ser Arg Ser Gly Glu Thr Val Arg Tyr
            500                 505                 510
Arg Tyr Asp Asp Ala His Ser Glu Leu Pro Ala Thr Thr Thr Asp Ala
        515                 520                 525
Thr Gly Ser Thr Arg Gln Met Thr Trp Ser Arg Tyr Gly Gln Leu Leu
    530                 535                 540
Ala Phe Thr Asp Cys Ser Gly Tyr Gln Thr Arg Tyr Glu Tyr Asp Arg
545                 550                 555                 560
Phe Gly Gln Met Thr Ala Val His Arg Glu Glu Gly Ile Ser Leu Tyr
                565                 570                 575
Arg Arg Tyr Asp Asn Arg Gly Arg Leu Thr Ser Val Lys Asp Ala Gln
            580                 585                 590
Gly Arg Glu Thr Arg Tyr Glu Tyr Asn Ala Ala Gly Asp Leu Thr Ala
        595                 600                 605
Val Ile Thr Pro Asp Gly Asn Arg Ser Glu Thr Gln Tyr Asp Ala Trp
    610                 615                 620
Gly Lys Ala Val Ser Thr Thr Gln Gly Gly Leu Thr Arg Ser Met Glu
625                 630                 635                 640
Tyr Asp Ala Ala Gly Arg Val Ile Ser Leu Thr Asn Glu Asn Gly Ser
                645                 650                 655
His Ser Val Phe Ser Tyr Asp Ala Leu Asp Arg Leu Val Gln Gln Gly
            660                 665                 670
Gly Phe Asp Gly Arg Thr Gln Arg Tyr His Tyr Asp Leu Thr Gly Lys
        675                 680                 685
Leu Thr Gln Ser Glu Asp Glu Gly Leu Val Ile Leu Trp Tyr Tyr Asp
    690                 695                 700
Glu Ser Asp Arg Ile Thr His Arg Thr Val Asn Gly Glu Pro Ala Glu
705                 710                 715                 720
Gln Trp Gln Tyr Asp Gly His Gly Trp Leu Thr Asp Ile Ser His Leu
                725                 730                 735
Ser Glu Gly His Arg Val Ala Val His Tyr Gly Tyr Asp Asp Lys Gly
            740                 745                 750
Arg Leu Thr Gly Glu Cys Gln Thr Val Glu Asn Pro Glu Thr Gly Glu
        755                 760                 765
Leu Leu Trp Gln His Glu Thr Lys His Ala Tyr Asn Glu Gln Gly Leu
    770                 775                 780
Ala Asn Arg Val Thr Pro Asp Ser Leu Pro Pro Val Glu Trp Leu Thr
785                 790                 795                 800
Tyr Gly Ser Gly Tyr Leu Ala Gly Met Lys Leu Gly Gly Thr Pro Leu
                805                 810                 815
Val Glu Tyr Thr Arg Asp Arg Leu His Arg Glu Thr Val Arg Ser Phe
            820                 825                 830
Gly Ser Met Ala Gly Ser Asn Ala Ala Tyr Glu Leu Thr Ser Thr Tyr
        835                 840                 845
Thr Pro Ala Gly Gln Leu Gln Ser Gln His Leu Asn Ser Leu Val Tyr

    850                 855                 860
Asp Arg Asp Tyr Gly Trp Ser Asp Asn Gly Asp Leu Val Arg Ile Ser
865                 870                 875                 880
Gly Pro Arg Gln Thr Arg Glu Tyr Gly Tyr Ser Ala Thr Gly Arg Leu
                885                 890                 895
Glu Ser Val Arg Thr Leu Ala Pro Asp Leu Asp Ile Arg Ile Pro Tyr
            900                 905                 910
Ala Thr Asp Pro Ala Gly Asn Arg Leu Pro Asp Pro Glu Leu His Pro
        915                 920                 925
Asp Ser Thr Leu Thr Val Trp Pro Asp Asn Arg Ile Ala Glu Asp Ala
    930                 935                 940
His Tyr Val Tyr Arg His Asp Glu Tyr Gly Arg Leu Thr Glu Lys Thr
945                 950                 955                 960
Asp Arg Ile Pro Ala Gly Val Ile Arg Thr Asp Asp Glu Arg Thr His
                965                 970                 975
His Tyr His Tyr Asp Ser Gln His Arg Leu Val Phe Tyr Thr Arg Ile
            980                 985                 990
Gln His Gly Glu Pro Leu Val Glu Ser Arg Tyr Leu Tyr Asp Pro Leu
        995                 1000                1005
Gly Arg Arg Met Ala Lys Arg Val Trp Arg Arg Glu Arg Asp Leu Thr
    1010                1015                1020
Gly Trp Met Ser Leu Ser Arg Lys Pro Glu Val Thr Trp Tyr Gly Trp
1025                1030                1035                1040
Asp Gly Asp Arg Leu Thr Thr Val Gln Thr Asp Thr Thr Arg Ile Gln
                1045                1050                1055
Thr Val Tyr Glu Pro Gly Ser Phe Thr Pro Leu Ile Arg Val Glu Thr
            1060                1065                1070
Glu Asn Gly Glu Arg Glu Lys Ala Gln Arg Arg Ser Leu Ala Glu Thr
        1075                1080                1085
Leu Gln Gln Glu Gly Ser Glu Asn Gly His Gly Val Val Phe Pro Ala
    1090                1095                1100
Glu Leu Val Arg Leu Leu Asp Arg Leu Glu Glu Glu Ile Arg Ala Asp
1105                1110                1115                1120
Arg Val Ser Ser Glu Ser Arg Ala Trp Leu Ala Gln Cys Gly Leu Thr
                1125                1130                1135
Val Glu Gln Leu Ala Arg Gln Val Glu Pro Glu Tyr Thr Pro Ala Arg
            1140                1145                1150
Lys Ala His Leu Tyr His Cys Asp His Arg Gly Leu Pro Leu Ala Leu
        1155                1160                1165
Ile Ser Glu Asp Gly Asn Thr Ala Trp Ser Ala Glu Tyr Asp Glu Trp
    1170                1175                1180
Gly Asn Gln Leu Asn Glu Glu Asn Pro His His Val Tyr Gln Pro Tyr
1185                1190                1195                1200
Arg Leu Pro Gly Gln Gln His Asp Glu Glu Ser Gly Leu Tyr Tyr Asn
                1205                1210                1215
Arg His Arg Tyr Tyr Asp Pro Leu Gln Gly Arg Tyr Ile Thr Gln Asp
            1220                1225                1230
Pro Met Gly Leu Lys Gly Gly Trp Asn Leu Tyr Gln Tyr Pro Leu Asn
        1235                1240                1245
Pro Leu Gln Gln Ile Asp Pro Met Gly Leu Leu Gln Thr Trp Asp Asp
    1250                1255                1260
Ala Arg Ser Gly Ala Cys Thr Gly Gly Val Cys Gly Val Leu Ser Arg
1265                1270                1275                1280
Ile Ile Gly Pro Ser Lys Phe Asp Ser Thr Ala Asp Ala Ala Leu Asp
                1285                1290                1295
Ala Leu Lys Glu Thr Gln Asn Arg Ser Leu Cys Asn Asp Met Glu Tyr
            1300                1305                1310
Ser Gly Ile Val Cys Lys Asp Thr Asn Gly Lys Tyr Phe Ala Ser Lys
        1315                1320                1325
Ala Glu Thr Asp Asn Leu Arg Lys Glu Ser Tyr Pro Leu Lys Arg Lys
    1330                1335                1340

Cys Pro Thr Gly Thr Asp Arg Val Ala Ala Tyr His Thr His Gly Ala
1345                1350                1355                1360
Asp Ser His Gly Asp Tyr Val Asp Glu Phe Phe Ser Ser Ser Asp Lys
                1365                1370                1375
Asn Leu Val Arg Ser Lys Asp Asn Asn Leu Glu Ala Phe Tyr Leu Ala
            1380                1385                1390
Thr Pro Asp Gly Arg Phe Glu Ala Leu Asn Asn Lys Gly Glu Tyr Ile
        1395                1400                1405
Phe Ile Arg Asn Ser Val Pro Gly Leu Ser Ser Val Cys Ile Pro Tyr
    1410                1415                1420
His Asp
1425

<210> 341
<211> 122
<212> PRT
<213> E. Coli

<400> 341
Met Lys Tyr Ser Ser Ile Phe Ser Met Leu Ser Phe Phe Ile Leu Phe
1               5                   10                  15
Ala Cys Asn Glu Thr Ala Val Tyr Gly Ser Asp Glu Asn Ile Ile Phe
            20                  25                  30
Met Arg Tyr Val Glu Lys Leu His Leu Asp Lys Tyr Ser Val Lys Asn
        35                  40                  45
Thr Val Lys Thr Glu Thr Met Ala Ile Gln Leu Ala Glu Ile Tyr Val
    50                  55                  60
Arg Tyr Arg Tyr Gly Glu Arg Ile Ala Glu Glu Glu Lys Pro Tyr Leu
65                  70                  75                  80
Ile Thr Glu Leu Pro Asp Ser Trp Val Val Glu Gly Ala Lys Leu Pro
                85                  90                  95
Tyr Glu Val Ala Gly Gly Val Phe Ile Ile Glu Ile Asn Lys Lys Asn
            100                 105                 110
Gly Cys Val Leu Asn Phe Leu His Ser Lys
        115                 120

<210> 342
<211> 236
<212> PRT
<213> E. Coli

<400> 342
Met Leu Ala Leu Met Asp Ala Asp Gly Asn Ile Ala Trp Ser Gly Glu
1               5                   10                  15
Tyr Asp Glu Trp Gly Asn Gln Leu Asn Glu Glu Asn Pro His His Leu
            20                  25                  30
His Gln Pro Tyr Arg Leu Pro Gly Gln Gln Tyr Asp Lys Glu Ser Gly
        35                  40                  45
Leu Tyr Tyr Asn Arg Asn Arg Tyr Tyr Asp Pro Leu Gln Gly Arg Tyr
    50                  55                  60
Ile Thr Gln Asp Pro Ile Gly Leu Glu Gly Gly Trp Ser Leu Tyr Ala
65                  70                  75                  80
Tyr Pro Leu Asn Pro Val Asn Gly Ile Asp Pro Leu Gly Leu Ser Pro
                85                  90                  95
Ala Asp Val Ala Leu Ile Arg Arg Lys Asp Gln Leu Asn His Gln Arg
            100                 105                 110
Ala Trp Asp Ile Leu Ser Asp Thr Tyr Glu Asp Met Lys Arg Leu Asn
        115                 120                 125
Leu Gly Gly Thr Asp Gln Phe Phe His Cys Met Ala Phe Cys Arg Val

130                 135                 140
Ser Lys Leu Asn Asp Ala Gly Val Ser Arg Ser Ala Lys Gly Leu Gly
145                 150                 155                 160
Tyr Glu Lys Glu Ile Arg Asp Tyr Gly Leu Asn Leu Phe Gly Met Tyr
                165                 170                 175
Gly Arg Lys Val Lys Leu Ser His Ser Glu Met Ile Glu Asp Asn Lys
            180                 185                 190
Lys Asp Leu Ala Val Asn Asp His Gly Leu Thr Cys Pro Ser Thr Thr
        195                 200                 205
Asp Cys Ser Asp Arg Cys Ser Asp Tyr Ile Asn Pro Glu His Lys Lys
    210                 215                 220
Thr Ile Lys Ala Leu Gln Asp Ala Gly Tyr Leu Lys
225                 230                 235

<210> 343
<211> 86
<212> PRT
<213> E. Coli

<400> 343
Met Leu Ala Ile Ser Ser Asn Leu Ser Lys Met Ile Ile Phe Ile Phe
 1               5                  10                  15
Ala Ile Ile Ile Ile Val Val Leu Cys Val Ile Thr Tyr Leu Tyr Leu
            20                  25                  30
Tyr Lys Asp Glu Ser Leu Val Ser Lys His Tyr Ile Asn Tyr Met Ala
        35                  40                  45
Ile Pro Glu Asn Asp Gly Val Phe Thr Trp Leu Pro Asp Phe Phe Pro
    50                  55                  60
His Val Ala Val Asp Ile Ser Ile Tyr Thr Asn Val Glu Asp Asp Tyr
65                  70                  75                  80
Phe Phe Leu Ile Phe Pro
                85

<210> 344
<211> 63
<212> PRT
<213> E. Coli

<400> 344
Met Arg Ala Arg Glu Gln Val Ala Lys Ile Val Ser Lys Asn Asp Pro
 1               5                  10                  15
Asp Thr Lys Lys Val Trp Cys Lys Tyr Gly Lys Ile Pro Gly Gln Gly
            20                  25                  30
Asp Gly Val Asn Leu Phe Phe Val Gly Glu Ile Asn Val Thr His Tyr
        35                  40                  45
Phe Ile Thr Asn Ile Gly Ala Gly Leu Pro Asp Ala Cys Ala Glu
    50                  55                  60

<210> 345
<211> 167
<212> PRT
<213> E. Coli

<400> 345
Met Pro Gly Asn Ser Pro His Tyr Gly Arg Trp Pro Gln His Asp Phe
 1               5                  10                  15

Thr Ser Leu Lys Lys Leu Arg Pro Gln Ser Val Thr Ser Arg Ile Gln
            20                  25                  30
Pro Gly Ser Asp Val Ile Val Cys Ala Glu Met Asp Glu Gln Trp Gly
        35                  40                  45
Tyr Val Gly Ala Lys Ser Arg Gln Arg Trp Leu Phe Tyr Ala Tyr Asp
    50                  55                  60
Ser Leu Arg Lys Thr Val Val Ala His Val Phe Gly Glu Arg Thr Met
65                  70                  75                  80
Ala Thr Leu Gly Arg Leu Met Ser Leu Leu Ser Pro Phe Asp Val Val
                85                  90                  95
Ile Trp Met Thr Asp Gly Trp Pro Leu Tyr Glu Ser Arg Leu Lys Gly
            100                 105                 110
Lys Leu His Val Ile Ser Lys Arg Tyr Thr Gln Arg Ile Glu Arg His
        115                 120                 125
Asn Leu Asn Leu Arg Gln His Leu Ala Arg Leu Gly Arg Lys Ser Leu
    130                 135                 140
Ser Phe Ser Lys Ser Val Glu Leu His Asp Lys Val Ile Gly His Tyr
145                 150                 155                 160
Leu Asn Ile Lys His Tyr Gln
                165

<210> 346
<211> 91
<212> PRT
<213> E. Coli

<400> 346
Met Ala Ser Val Ser Ile Ser Cys Pro Ser Cys Ser Ala Thr Asp Gly
 1               5                  10                  15
Val Val Arg Asn Gly Lys Ser Thr Ala Gly His Gln Arg Tyr Leu Cys
            20                  25                  30
Ser His Cys Arg Lys Thr Trp Gln Leu Gln Phe Thr Tyr Thr Ala Ser
        35                  40                  45
Gln Pro Gly Thr His Gln Lys Ile Ile Asp Met Ala Met Asn Gly Val
    50                  55                  60
Gly Cys Arg Ala Thr Ala Arg Ile Met Gly Val Gly Leu Asn Thr Ile
65                  70                  75                  80
Leu Arg His Leu Lys Asn Ser Gly Arg Ser Arg
                85                  90

<210> 347
<211> 138
<212> PRT
<213> E. Coli

<400> 347
Met Met Thr Lys Thr Gln Ile Asn Lys Leu Ile Lys Met Met Asn Asp
 1               5                  10                  15
Leu Asp Tyr Pro Phe Glu Ala Pro Leu Lys Glu Ser Phe Ile Glu Ser
            20                  25                  30
Ile Ile Gln Ile Glu Phe Asn Ser Asn Ser Thr Asn Cys Leu Glu Lys
        35                  40                  45
Leu Cys Asn Glu Val Ser Ile Leu Phe Lys Asn Gln Pro Asp Tyr Leu
    50                  55                  60
Thr Phe Leu Arg Ala Met Asp Gly Phe Glu Val Asn Gly Leu Arg Leu
65                  70                  75                  80
Phe Ser Leu Ser Ile Pro Glu Pro Ser Val Lys Asn Leu Phe Ala Val
                85                  90                  95

Asn Glu Phe Tyr Arg Asn Asn Asp Asp Phe Ile Asn Pro Asp Leu Gln
            100                 105                 110
Glu Arg Leu Val Ile Gly Asp Tyr Ser Ile Ser Ile Phe Thr Tyr Asp
        115                 120                 125
Ile Lys Gly Asp Ala Ala Asn Leu Leu Ile
    130                 135

<210> 348
<211> 392
<212> PRT
<213> E. Coli

<400> 348
Met Ser Asn Ile Val Tyr Leu Thr Val Thr Gly Glu Gln Gln Gly Ser
1               5                   10                  15
Ile Ser Ala Gly Cys Gly Thr Ser Glu Ser Thr Gly Asn Arg Trp Gln
            20                  25                  30
Ser Gly His Glu Asp Glu Ile Phe Thr Phe Ser Leu Leu Asn Asn Ile
        35                  40                  45
Asn Asn Thr Gly Leu Gly Ser Gln Phe His Gly Ile Thr Phe Cys Lys
    50                  55                  60
Leu Ile Asp Lys Ser Thr Pro Leu Phe Ile Asn Ser Ile Asn Asn Asn
65                  70                  75                  80
Glu Gln Leu Phe Met Gly Phe Asp Phe Tyr Arg Ile Asn Arg Phe Gly
                85                  90                  95
Arg Leu Glu Lys Tyr Tyr Tyr Ile Gln Leu Arg Gly Ala Phe Leu Ser
            100                 105                 110
Ala Ile His His Gln Ile Ile Glu Asn Gln Leu Asp Thr Glu Thr Ile
        115                 120                 125
Thr Ile Ser Tyr Glu Phe Ile Leu Cys Gln His Leu Ile Ala Asn Thr
    130                 135                 140
Glu Phe Ser Tyr Leu Ala Leu Pro Glu Asn Tyr Asn Arg Leu Phe Leu
145                 150                 155                 160
Pro Asn Ser Lys Asn Gln Thr Asn Asn Arg Phe Lys Thr Leu Asn Ser
                165                 170                 175
Lys Ala Ile Gly Arg Leu Leu Ala Ala Gly Gly Val Tyr Asn Gly Asn
            180                 185                 190
Ile Glu Gly Phe Arg Asp Thr Ala Glu Lys Leu Gly Gly Asp Ala Ile
        195                 200                 205
Lys Gly Tyr Asp Gln Ile Leu Asn Glu Lys Thr Ala Gly Ile Ala Ile
    210                 215                 220
Ala Thr Ala Ser Ile Leu Leu Thr Lys Arg Ser Asn Val Asp Thr Tyr
225                 230                 235                 240
Thr Glu Ile Asn Ser Tyr Leu Gly Lys Leu Arg Gly Gln Gln Lys Leu
                245                 250                 255
Leu Asp Gly Ile Asp Ile Ile Glu Ile Ile Tyr Ile Lys Arg Pro Ser
            260                 265                 270
Lys Asp Leu Ala Asn Leu Arg Lys Glu Phe Asn Lys Thr Val Arg Lys
        275                 280                 285
Asn Phe Leu Ile Lys Leu Ala Lys Thr Ser Glu Ala Ser Gly Arg Phe
    290                 295                 300
Asn Ala Glu Asp Leu Leu Arg Met Arg Lys Gly Asn Val Pro Leu Asn
305                 310                 315                 320
Tyr Asn Val His His Lys Leu Ser Leu Asp Asp Gly Gly Thr Asn Asp
                325                 330                 335
Phe Glu Asn Leu Val Leu Ile Glu Asn Glu Pro Tyr His Lys Val Phe
            340                 345                 350
Thr Asn Met Gln Ser Arg Ile Ala Lys Gly Ile Leu Val Gly Glu Ser
        355                 360                 365
Lys Ile Thr Pro Trp Ala Ile Pro Ser Gly Ser Ile Tyr Pro Pro Met

370                 375                 380
Lys Asn Ile Met Asp His Thr Lys
385                 390

<210> 349
<211> 221
<212> PRT
<213> E. Coli

<400> 349
Met Val Leu Ala Leu Asn Tyr Asn Met His Gly Val Asn Ile Arg Ser
1               5                   10                  15
Glu Asn Ala Ala Lys Pro His Thr Met Pro Ser Arg Tyr Leu Cys Glu
            20                  25                  30
Tyr Ile Arg Ser Ile Glu Lys Asn Gly His Ala Leu Asp Phe Gly Cys
        35                  40                  45
Gly Lys Leu Arg Tyr Ser Asp Glu Leu Ile Ser Lys Phe Asp Glu Val
    50                  55                  60
Thr Phe Leu Asp Ser Lys Arg Gln Leu Glu Arg Glu Gln Ile Ile Arg
65                  70                  75                  80
Gly Ile Lys Thr Lys Ile Ile Asp Tyr Val Pro Arg Tyr Tyr Lys Asn
                85                  90                  95
Ala Asn Thr Val Ala Phe Glu Asp Val Asp Lys Ile Ile Gly Gly Tyr
            100                 105                 110
Asp Phe Ile Leu Cys Ser Asn Val Leu Ser Ala Val Pro Cys Arg Asp
        115                 120                 125
Thr Ile Asp Lys Ile Val Leu Ser Ile Lys Arg Leu Leu Lys Ser Gly
    130                 135                 140
Gly Glu Thr Leu Ile Val Asn Gln Tyr Lys Ser Ser Tyr Phe Lys Lys
145                 150                 155                 160
Tyr Glu Thr Gly Arg Lys His Leu Tyr Gly Tyr Ile Tyr Lys Asn Ser
                165                 170                 175
Lys Ser Val Ser Tyr Tyr Gly Leu Leu Asp Glu Leu Ala Val Gln Glu
            180                 185                 190
Ile Cys Ser Ser His Gly Leu Glu Ile Leu Lys Ser Trp Ser Lys Ala
        195                 200                 205
Gly Ser Ser Tyr Val Thr Val Gly Ser Cys Asn Ala Ile
    210                 215                 220

<210> 350
<211> 234
<212> PRT
<213> E. Coli

<400> 350
Met Asn Asn Met Phe Glu Pro Pro Lys Asn Tyr Asn Glu Met Leu Pro
1               5                   10                  15
Lys Leu His Lys Ala Thr Phe Leu Asn Thr Leu Ile Tyr Cys Ile Leu
            20                  25                  30
Leu Val Ile Tyr Glu Tyr Ile Pro Leu Ile Thr Leu Pro Thr Lys Tyr
        35                  40                  45
Val Pro Pro Ile Lys Asp His Glu Ser Phe Ile Asn Trp Ala Leu Ser
    50                  55                  60
Phe Gly Ile Leu Pro Cys Ala Phe Ala Ile Phe Ala Tyr Leu Ile Ser
65                  70                  75                  80
Gly Ala Leu Asp Leu His Asn Asn Ala Ala Lys Leu Leu Arg Val Arg
                85                  90                  95
Tyr Leu Trp Asp Lys His Leu Ile Ile Lys Pro Leu Ser Arg Arg Ala

            100                 105                 110
Gly Val Asn Arg Lys Leu Asn Lys Asp Glu Ala His Asn Val Met Ser
        115                 120                 125
Asn Leu Tyr Tyr Pro Glu Val Arg Lys Ile Glu Asp Lys His Tyr Ile
    130                 135                 140
Glu Leu Phe Trp Asn Lys Val Tyr Tyr Phe Trp Ile Phe Phe Glu Phe
145                 150                 155                 160
Ser Ile Ile Ala Leu Ile Ser Phe Leu Ile Ile Phe Phe Cys Lys Gln
                165                 170                 175
Met Asp Ile Phe His Val Glu Gly Ser Leu Leu Ser Leu Phe Phe Phe
            180                 185                 190
Val Ile Leu Ser Phe Ser Val Ser Gly Ile Ile Phe Ala Leu Thr Val
        195                 200                 205
Lys Pro Arg Thr Glu Ser Gln Val Gly Lys Ile Pro Asp Asp Lys Ile
    210                 215                 220
Lys Glu Phe Phe Thr Lys Asn Asn Ile Asn
225                 230


      <210> 351
      <211> 94
      <212> PRT
      <213> E. Coli

      <400> 351


      <210> 352
      <211> 658
      <212> PRT
      <213> E. Coli

      <400> 352
Met Val Leu Phe Tyr Arg Ala His Trp Arg Asp Tyr Lys Asn Asp Gln
 1               5                  10                  15
Val Arg Ile Met Met Asn Leu Thr Thr Leu Thr His Arg Asp Ala Leu
            20                  25                  30
Cys Leu Asn Ala Arg Phe Thr Ser Arg Glu Glu Ala Ile His Ala Leu
        35                  40                  45
Thr Gln Arg Leu Ala Ala Leu Gly Lys Ile Ser Ser Thr Glu Gln Phe
    50                  55                  60
Leu Glu Glu Val Tyr Arg Arg Glu Ser Leu Gly Pro Thr Ala Leu Gly
65                  70                  75                  80
Glu Gly Leu Ala Val Pro His Gly Lys Thr Ala Ala Val Lys Glu Ala
                85                  90                  95
Ala Phe Ala Val Ala Thr Leu Ser Glu Pro Leu Gln Trp Glu Gly Val
            100                 105                 110
Asp Gly Pro Glu Ala Val Asp Leu Val Val Leu Leu Ala Ile Pro Pro

115                 120                 125
Asn Glu Ala Gly Thr Thr His Met Gln Leu Leu Thr Ala Leu Thr Thr
    130                 135                 140
Arg Leu Ala Asp Asp Glu Ile Arg Ala Arg Ile Gln Ser Ala Thr Thr
145                 150                 155                 160
Pro Asp Glu Leu Leu Ser Ala Leu Asp Asp Lys Gly Gly Thr Gln Pro
                165                 170                 175
Ser Ala Ser Phe Ser Asn Ala Pro Thr Ile Val Cys Val Thr Ala Cys
            180                 185                 190
Pro Ala Gly Ile Ala His Thr Tyr Met Ala Ala Glu Tyr Leu Glu Lys
        195                 200                 205
Ala Gly Arg Lys Leu Gly Val Asn Val Tyr Val Glu Lys Gln Gly Ala
    210                 215                 220
Asn Gly Ile Glu Gly Arg Leu Thr Ala Asp Gln Leu Asn Ser Ala Thr
225                 230                 235                 240
Ala Cys Ile Phe Ala Ala Glu Val Ala Ile Lys Glu Ser Glu Arg Phe
                245                 250                 255
Asn Gly Ile Pro Ala Leu Ser Val Pro Val Ala Glu Pro Ile Arg His
            260                 265                 270
Ala Glu Ala Leu Ile Gln Gln Ala Leu Thr Leu Lys Arg Ser Asp Glu
        275                 280                 285
Thr Arg Thr Val Gln Gln Asp Thr Gln Pro Val Lys Ser Val Lys Thr
    290                 295                 300
Glu Leu Lys Gln Ala Leu Leu Ser Gly Ile Ser Phe Ala Val Pro Leu
305                 310                 315                 320
Ile Val Ala Gly Gly Thr Val Leu Ala Val Ala Val Leu Leu Ser Gln
                325                 330                 335
Ile Phe Gly Leu Gln Asp Leu Phe Asn Glu Glu Asn Ser Trp Leu Trp
            340                 345                 350
Met Tyr Arg Lys Leu Gly Gly Gly Leu Leu Gly Ile Leu Met Val Pro
        355                 360                 365
Val Leu Ala Ala Tyr Thr Ala Tyr Ser Leu Ala Asp Lys Pro Ala Leu
    370                 375                 380
Ala Pro Gly Phe Ala Ala Gly Leu Ala Ala Asn Met Ile Gly Ser Gly
385                 390                 395                 400
Phe Leu Gly Ala Val Val Gly Gly Leu Ile Ala Gly Tyr Leu Met Arg
                405                 410                 415
Trp Val Lys Asn His Leu Arg Leu Ser Ser Lys Phe Asn Gly Phe Leu
            420                 425                 430
Thr Phe Tyr Leu Tyr Pro Val Leu Gly Thr Leu Gly Ala Gly Ser Leu
        435                 440                 445
Met Leu Phe Val Val Gly Glu Pro Val Ala Trp Ile Asn Asn Ser Leu
    450                 455                 460
Thr Ala Trp Leu Asn Gly Leu Ser Gly Ser Asn Ala Leu Leu Leu Gly
465                 470                 475                 480
Ala Ile Leu Gly Phe Met Cys Ser Phe Asp Leu Gly Gly Pro Val Asn
                485                 490                 495
Lys Ala Ala Tyr Ala Phe Cys Leu Gly Ala Met Ala Asn Gly Val Tyr
            500                 505                 510
Gly Pro Tyr Ala Ile Phe Ala Ser Val Lys Met Val Ser Ala Phe Thr
        515                 520                 525
Val Thr Ala Ser Thr Met Leu Ala Pro Arg Leu Phe Lys Glu Phe Glu
    530                 535                 540
Ile Glu Thr Gly Lys Ser Thr Trp Leu Leu Gly Leu Ala Gly Ile Thr
545                 550                 555                 560
Glu Gly Ala Ile Pro Met Ala Ile Glu Asp Pro Leu Arg Val Ile Gly
                565                 570                 575
Ser Phe Val Leu Gly Ser Met Val Thr Gly Ala Ile Val Gly Ala Met
            580                 585                 590
Asn Ile Gly Leu Ser Thr Pro Gly Ala Gly Ile Phe Ser Leu Phe Leu
        595                 600                 605

Leu His Asp Asn Gly Ala Gly Gly Val Met Ala Ala Ile Gly Trp Phe
    610                 615                 620
Gly Ala Ala Leu Val Gly Ala Ala Ile Ser Thr Ala Ile Leu Leu Met
625                 630                 635                 640
Trp Arg Arg His Ala Val Lys His Gly Asn Tyr Leu Thr Asp Gly Val
                645                 650                 655
Met Pro

<210> 353
<211> 877
<212> PRT
<213> E. Coli

<400> 353
Met Lys Ala Val Ser Arg Val His Ile Thr Pro His Met His Trp Asp
 1               5                  10                  15
Arg Glu Trp Tyr Phe Thr Thr Glu Glu Ser Arg Ile Leu Leu Val Asn
            20                  25                  30
Asn Met Glu Glu Ile Leu Cys Arg Leu Glu Gln Asp Asn Glu Tyr Lys
        35                  40                  45
Tyr Tyr Val Leu Asp Gly Gln Thr Ala Ile Leu Glu Asp Tyr Phe Ala
    50                  55                  60
Val Lys Pro Glu Asn Lys Asp Arg Val Lys Lys Gln Val Glu Ala Gly
65                  70                  75                  80
Lys Leu Ile Ile Gly Pro Trp Tyr Thr Gln Thr Asp Thr Thr Ile Val
                85                  90                  95
Ser Ala Glu Ser Ile Val Arg Asn Leu Met Tyr Gly Met Arg Asp Cys
            100                 105                 110
Leu Ala Phe Gly Glu Pro Met Lys Ile Gly Tyr Leu Pro Asp Ser Phe
        115                 120                 125
Gly Met Ser Gly Gln Leu Pro His Ile Tyr Asn Gly Phe Gly Ile Thr
    130                 135                 140
Arg Thr Met Phe Trp Arg Gly Cys Ser Glu Arg His Gly Thr Asp Lys
145                 150                 155                 160
Thr Glu Phe Leu Trp Gln Ser Ser Asp Gly Ser Glu Val Thr Ala Gln
                165                 170                 175
Val Leu Pro Leu Gly Tyr Ala Ile Gly Lys Tyr Leu Pro Ala Asp Glu
            180                 185                 190
Asn Gly Leu Arg Lys Arg Leu Asp Ser Tyr Phe Asp Val Leu Glu Lys
        195                 200                 205
Ala Ser Val Thr Lys Glu Ile Leu Leu Pro Asn Gly His Asp Gln Met
    210                 215                 220
Pro Leu Gln Gln Asn Ile Phe Glu Val Met Asp Lys Leu Arg Glu Ile
225                 230                 235                 240
Tyr Pro Gln Arg Lys Phe Val Met Ser Arg Phe Glu Glu Val Phe Glu
                245                 250                 255
Lys Ile Glu Ala Gln Arg Asp Asn Leu Ala Thr Leu Lys Gly Glu Phe
            260                 265                 270
Ile Asp Gly Lys Tyr Met Arg Val His Arg Thr Ile Gly Ser Thr Arg
        275                 280                 285
Met Asp Ile Lys Ile Ala His Ala Arg Ile Glu Asn Lys Ile Val Asn
    290                 295                 300
Leu Leu Glu Pro Leu Ala Thr Leu Ala Trp Thr Leu Gly Phe Glu Tyr
305                 310                 315                 320
His His Gly Leu Leu Glu Lys Met Trp Lys Glu Ile Leu Lys Asn His
                325                 330                 335
Ala His Asp Ser Ile Gly Cys Cys Cys Ser Asp Lys Val His Arg Glu
            340                 345                 350
Ile Val Ala Arg Phe Glu Leu Ala Glu Asp Met Ala Asp Asn Leu Ile

        355                 360                 365
Arg Phe Tyr Met Arg Lys Ile Ala Asp Asn Met Pro Gln Ser Asp Ala
    370                 375                 380
Asp Lys Leu Val Leu Phe Asn Leu Met Pro Trp Pro Arg Glu Glu Val
385                 390                 395                 400
Ile Asn Thr Thr Val Arg Leu Arg Ala Ser Gln Phe Asn Leu Arg Asp
                405                 410                 415
Asp Arg Gly Gln Pro Val Pro Tyr Phe Ile Arg His Ala Arg Glu Ile
            420                 425                 430
Asp Pro Gly Leu Ile Asp Arg Gln Ile Val His Tyr Gly Asn Tyr Asp
        435                 440                 445
Pro Phe Met Glu Phe Asp Ile Gln Ile Asn Gln Ile Val Pro Ser Met
    450                 455                 460
Gly Tyr Arg Thr Leu Tyr Ile Glu Ala Asn Gln Pro Gly Asn Val Ile
465                 470                 475                 480
Ala Ala Lys Ser Asp Ala Glu Gly Ile Leu Glu Asn Ala Phe Trp Gln
                485                 490                 495
Ile Ala Leu Asn Glu Asp Gly Ser Leu Gln Leu Val Asp Lys Asp Ser
            500                 505                 510
Gly Val Arg Tyr Asp Arg Val Leu Gln Ile Glu Glu Ser Ser Asp Asp
        515                 520                 525
Gly Asp Glu Tyr Asp Tyr Ser Pro Ala Lys Glu Glu Trp Val Ile Thr
    530                 535                 540
Ala Ala Asn Ala Lys Pro Gln Cys Asp Ile Ile His Glu Ala Trp Gln
545                 550                 555                 560
Ser Arg Ala Val Ile Arg Tyr Asp Met Ala Val Pro Leu Asn Leu Ser
                565                 570                 575
Glu Arg Ser Ala Arg Gln Ser Thr Gly Arg Val Gly Val Val Leu Val
            580                 585                 590
Val Thr Leu Ser His Asn Ser Arg Arg Ile Asp Val Asp Ile Asn Leu
        595                 600                 605
Asp Asn Gln Ala Asp Asp His Arg Leu Arg Val Leu Val Pro Thr Pro
    610                 615                 620
Phe Asn Thr Asp Ser Val Leu Ala Asp Thr Gln Phe Gly Ser Leu Thr
625                 630                 635                 640
Arg Pro Val Asn Asp Ser Ala Met Asn Asn Trp Gln Gln Glu Gly Trp
                645                 650                 655
Lys Glu Ala Pro Val Pro Val Trp Asn Met Leu Asn Tyr Val Ala Leu
            660                 665                 670
Gln Glu Gly Arg Asn Gly Met Ala Val Phe Ser Glu Gly Leu Arg Glu
        675                 680                 685
Phe Glu Val Ile Gly Glu Glu Lys Lys Thr Phe Ala Ile Thr Leu Leu
    690                 695                 700
Arg Gly Val Gly Leu Leu Gly Lys Glu Asp Leu Leu Leu Arg Pro Gly
705                 710                 715                 720
Arg Pro Ser Gly Ile Lys Met Pro Val Pro Asp Ser Gln Leu Arg Gly
                725                 730                 735
Leu Leu Ser Cys Arg Leu Ser Leu Leu Ser Tyr Thr Gly Thr Pro Thr
            740                 745                 750
Ala Ala Gly Val Ala Gln Gln Ala Arg Ala Trp Leu Thr Pro Val Gln
        755                 760                 765
Cys Tyr Asn Lys Ile Pro Trp Asp Val Met Lys Leu Asn Lys Ala Gly
    770                 775                 780
Phe Asn Val Pro Glu Ser Tyr Ser Leu Leu Lys Met Pro Pro Val Gly
785                 790                 795                 800
Cys Leu Ile Ser Ala Leu Lys Lys Ala Glu Asp Arg Gln Glu Val Ile
                805                 810                 815
Leu Arg Leu Phe Asn Pro Ala Glu Ser Ala Thr Cys Asp Ala Thr Val
            820                 825                 830
Ala Phe Ser Arg Glu Val Ile Ser Cys Ser Glu Thr Met Met Asp Glu
        835                 840                 845

His Ile Thr Thr Glu Glu Asn Gln Gly Ser Asn Leu Ser Gly Pro Phe
    850                 855                 860
Leu Pro Gly Gln Ser Arg Thr Phe Ser Tyr Arg Leu Ala
865                 870                 875

<210> 354
<211> 523
<212> PRT
<213> E. Coli

<400> 354
Met Met Leu Asp Ile Val Glu Leu Ser Arg Leu Gln Phe Ala Leu Thr
 1               5                  10                  15
Ala Met Tyr His Phe Leu Phe Val Pro Leu Thr Leu Gly Met Ala Phe
            20                  25                  30
Leu Leu Ala Ile Met Glu Thr Val Tyr Val Leu Ser Gly Lys Gln Ile
        35                  40                  45
Tyr Lys Asp Met Thr Lys Phe Trp Gly Lys Leu Phe Gly Ile Asn Phe
    50                  55                  60
Ala Leu Gly Val Ala Thr Gly Leu Thr Met Glu Phe Gln Phe Gly Thr
65                  70                  75                  80
Asn Trp Ser Tyr Tyr Ser His Tyr Val Gly Asp Ile Phe Gly Ala Pro
                85                  90                  95
Leu Ala Ile Glu Gly Leu Met Ala Phe Phe Leu Glu Ser Thr Phe Val
            100                 105                 110
Gly Leu Phe Phe Phe Gly Trp Asp Arg Leu Gly Lys Val Gln His Met
        115                 120                 125
Cys Val Thr Trp Leu Val Ala Leu Gly Ser Asn Leu Ser Ala Leu Trp
    130                 135                 140
Ile Leu Val Ala Asn Gly Trp Met Gln Asn Pro Ile Ala Ser Asp Phe
145                 150                 155                 160
Asn Phe Glu Thr Met Arg Met Glu Met Val Ser Phe Ser Glu Leu Val
                165                 170                 175
Leu Asn Pro Val Ala Gln Val Lys Phe Val His Thr Val Ala Ser Gly
            180                 185                 190
Tyr Val Thr Gly Ala Met Phe Ile Leu Gly Ile Ser Ala Trp Tyr Met
        195                 200                 205
Leu Lys Gly Arg Asp Phe Ala Phe Ala Lys Arg Ser Phe Ala Ile Ala
    210                 215                 220
Ala Ser Phe Gly Met Ala Ala Val Leu Ser Val Ile Val Leu Gly Asp
225                 230                 235                 240
Glu Ser Gly Tyr Glu Met Gly Asp Val Gln Lys Thr Lys Leu Ala Ala
                245                 250                 255
Ile Glu Ala Glu Trp Glu Thr Gln Pro Ala Pro Ala Ala Phe Thr Leu
            260                 265                 270
Phe Gly Ile Pro Asp Gln Glu Glu Glu Thr Asn Lys Phe Ala Ile Gln
        275                 280                 285
Ile Pro Tyr Ala Leu Gly Ile Ile Ala Thr Arg Ser Val Asp Thr Pro
    290                 295                 300
Val Ile Gly Leu Lys Glu Leu Met Val Gln His Glu Glu Arg Ile Arg
305                 310                 315                 320
Asn Gly Met Lys Ala Tyr Ser Leu Leu Glu Gln Leu Arg Ser Gly Ser
                325                 330                 335
Thr Asp Gln Ala Val Arg Asp Gln Phe Asn Ser Met Lys Lys Asp Leu
            340                 345                 350
Gly Tyr Gly Leu Leu Leu Lys Arg Tyr Thr Pro Asn Val Ala Asp Ala
        355                 360                 365
Thr Glu Ala Gln Ile Gln Gln Ala Thr Lys Asp Ser Ile Pro Arg Val
    370                 375                 380
Ala Pro Leu Tyr Phe Ala Phe Arg Ile Met Val Ala Cys Gly Phe Leu

385                 390                 395                 400
Leu Leu Ala Ile Ile Ala Leu Ser Phe Trp Ser Val Ile Arg Asn Arg
                405                 410                 415
Ile Gly Glu Lys Lys Trp Leu Leu Arg Ala Ala Leu Tyr Gly Ile Pro
            420                 425                 430
Leu Pro Trp Ile Ala Val Glu Ala Gly Trp Phe Val Ala Glu Tyr Gly
        435                 440                 445
Arg Gln Pro Trp Ala Ile Gly Glu Val Leu Pro Thr Ala Val Ala Asn
    450                 455                 460
Ser Ser Leu Thr Ala Gly Asp Leu Ile Phe Ser Met Val Leu Ile Cys
465                 470                 475                 480
Gly Leu Tyr Thr Leu Phe Leu Val Ala Glu Leu Phe Leu Met Phe Lys
                485                 490                 495
Phe Ala Arg Leu Gly Pro Ser Ser Leu Lys Thr Gly Arg Tyr His Phe
            500                 505                 510
Glu Gln Ser Ser Thr Thr Thr Gln Pro Ala Arg
        515                 520

<210> 355
<211> 379
<212> PRT
<213> E. Coli

<400> 355
Met Ile Asp Tyr Glu Val Leu Arg Phe Ile Trp Trp Leu Leu Val Gly
1               5                   10                  15
Val Leu Leu Ile Gly Phe Ala Val Thr Asp Gly Phe Asp Met Gly Val
            20                  25                  30
Gly Met Leu Thr Arg Phe Leu Gly Arg Asn Asp Thr Glu Arg Arg Ile
        35                  40                  45
Met Ile Asn Ser Ile Ala Pro His Trp Asp Gly Asn Gln Val Trp Leu
    50                  55                  60
Ile Thr Ala Gly Gly Ala Leu Phe Ala Ala Trp Pro Met Val Tyr Ala
65                  70                  75                  80
Ala Ala Phe Ser Gly Phe Tyr Val Ala Met Ile Leu Val Leu Ala Ser
                85                  90                  95
Leu Phe Phe Arg Pro Val Gly Phe Asp Tyr Arg Ser Lys Ile Glu Glu
            100                 105                 110
Thr Arg Trp Arg Asn Met Trp Asp Trp Gly Ile Phe Ile Gly Ser Phe
        115                 120                 125
Val Pro Pro Leu Val Ile Gly Val Ala Phe Gly Asn Leu Leu Gln Gly
    130                 135                 140
Val Pro Phe Asn Val Asp Glu Tyr Leu Arg Leu Tyr Tyr Thr Gly Asn
145                 150                 155                 160
Phe Phe Gln Leu Leu Asn Pro Phe Gly Leu Leu Ala Gly Val Val Ser
                165                 170                 175
Val Gly Met Ile Ile Thr Gln Gly Ala Thr Tyr Leu Gln Met Arg Thr
            180                 185                 190
Val Gly Glu Leu His Leu Arg Thr Arg Ala Thr Ala Gln Val Ala Ala
        195                 200                 205
Leu Val Thr Leu Val Cys Phe Ala Leu Ala Gly Val Trp Val Met Tyr
    210                 215                 220
Gly Ile Asp Gly Tyr Val Val Lys Ser Thr Met Asp His Tyr Ala Ala
225                 230                 235                 240
Ser Asn Pro Leu Asn Lys Glu Val Val Arg Glu Ala Gly Ala Trp Leu
                245                 250                 255
Val Asn Phe Asn Asn Thr Pro Ile Leu Trp Ala Ile Pro Ala Leu Gly
            260                 265                 270
Val Val Leu Pro Leu Leu Thr Ile Leu Thr Ala Arg Met Asp Lys Ala
        275                 280                 285

Ala Trp Ala Phe Val Phe Ser Ser Leu Thr Leu Ala Cys Ile Ile Leu
    290                 295                 300
Thr Ala Gly Ile Ala Met Phe Pro Phe Val Met Pro Ser Ser Thr Met
305                 310                 315                 320
Met Asn Ala Ser Leu Thr Met Trp Asp Ala Thr Ser Ser Gln Leu Thr
                325                 330                 335
Leu Asn Val Met Thr Trp Val Ala Val Val Leu Val Pro Ile Ile Leu
                340                 345                 350
Leu Tyr Thr Ala Trp Cys Tyr Trp Lys Met Phe Gly Arg Ile Thr Lys
        355                 360                 365
Glu Asp Ile Glu Arg Asn Thr His Ser Leu Tyr
    370                 375

<210> 356
<211> 456
<212> PRT
<213> E. Coli

<400> 356
Met Glu Leu Ser Ser Leu Thr Ala Val Ser Pro Val Asp Gly Arg Tyr
1               5                   10                  15
Gly Asp Lys Val Ser Ala Leu Arg Gly Ile Phe Ser Glu Tyr Gly Leu
            20                  25                  30
Leu Lys Phe Arg Val Gln Val Glu Val Arg Trp Leu Gln Lys Leu Ala
        35                  40                  45
Ala His Ala Ala Ile Lys Glu Val Pro Ala Phe Ala Ala Asp Ala Ile
    50                  55                  60
Gly Tyr Leu Asp Ala Ile Val Ala Ser Phe Ser Glu Glu Asp Ala Ala
65                  70                  75                  80
Arg Ile Lys Thr Ile Glu Arg Thr Thr Asn His Asp Val Lys Ala Val
                85                  90                  95
Glu Tyr Phe Leu Lys Glu Lys Val Ala Glu Ile Pro Glu Leu His Ala
            100                 105                 110
Val Ser Glu Phe Ile His Phe Ala Cys Thr Ser Glu Asp Ile Asn Asn
        115                 120                 125
Leu Ser His Ala Leu Met Leu Lys Thr Ala Arg Asp Glu Val Ile Leu
    130                 135                 140
Pro Tyr Trp Arg Gln Leu Ile Asp Gly Ile Lys Asp Leu Ala Val Gln
145                 150                 155                 160
Tyr Arg Asp Ile Pro Leu Leu Ser Arg Thr His Gly Gln Pro Ala Thr
                165                 170                 175
Pro Ser Thr Ile Gly Lys Glu Met Ala Asn Val Ala Tyr Arg Met Glu
            180                 185                 190
Arg Gln Tyr Arg Gln Leu Asn Gln Val Glu Ile Leu Gly Lys Ile Asn
        195                 200                 205
Gly Ala Val Gly Asn Tyr Asn Ala His Ile Ala Ala Tyr Pro Glu Val
    210                 215                 220
Asp Trp His Gln Phe Ser Glu Glu Phe Val Thr Ser Leu Gly Ile Gln
225                 230                 235                 240
Trp Asn Pro Tyr Thr Thr Gln Ile Glu Pro His Asp Tyr Ile Ala Glu
                245                 250                 255
Leu Phe Asp Cys Val Ala Arg Phe Asn Thr Ile Leu Ile Asp Phe Asp
            260                 265                 270
Arg Asp Val Trp Gly Tyr Ile Ala Leu Asn His Phe Lys Gln Lys Thr
        275                 280                 285
Ile Ala Gly Glu Ile Gly Ser Ser Thr Met Pro His Lys Val Asn Pro
    290                 295                 300
Ile Asp Phe Glu Asn Ser Glu Gly Asn Leu Gly Leu Ser Asn Ala Val
305                 310                 315                 320
Leu Gln His Leu Ala Ser Lys Leu Pro Val Ser Arg Trp Gln Arg Asp

                325                 330                 335
Leu Thr Asp Ser Thr Val Leu Arg Asn Leu Gly Val Gly Ile Gly Tyr
            340                 345                 350
Ala Leu Ile Ala Tyr Gln Ser Thr Leu Lys Gly Val Ser Lys Leu Glu
        355                 360                 365
Val Asn Arg Asp His Leu Leu Asp Glu Leu Asp His Asn Trp Glu Val
    370                 375                 380
Leu Ala Glu Pro Ile Gln Thr Val Met Arg Arg Tyr Gly Ile Glu Lys
385                 390                 395                 400
Pro Tyr Glu Lys Leu Lys Glu Leu Thr Arg Gly Lys Arg Val Asp Ala
                405                 410                 415
Glu Gly Met Lys Gln Phe Ile Asp Gly Leu Ala Leu Pro Glu Glu Glu
            420                 425                 430
Lys Ala Arg Leu Lys Ala Met Thr Pro Ala Asn Tyr Ile Gly Arg Ala
        435                 440                 445
Ile Thr Met Val Asp Glu Leu Lys
    450                 455

<210> 357
<211> 61
<212> PRT
<213> E. Coli

<400> 357
Met Leu Ile Leu Thr Arg Arg Val Gly Glu Thr Leu Met Ile Gly Asp
1               5                   10                  15
Glu Val Thr Val Thr Val Leu Gly Val Lys Gly Asn Gln Val Arg Ile
            20                  25                  30
Gly Val Asn Ala Pro Lys Glu Val Ser Val His Arg Glu Glu Ile Tyr
        35                  40                  45
Gln Arg Ile Gln Ala Glu Lys Ser Gln Gln Ser Ser Tyr
    50                  55                  60

<210> 358
<211> 83
<212> RNA
<213> E. Coli

<400> 358
ggugaggugg ccgagaggcu gaaggcgcuc cccugcuaag ggaguaugcg gucaaaagcu 60
gcauccgggg uucgaauccc cgccucaccg cca 83

<210> 359
<211> 200
<212> PRT
<213> E. Coli

<400> 359
Meu Lys Asn Lys Ala Asp Asn Lys Lys Arg Asn Phe Leu Thr His Ser
1 5 10 15
Glu Ile Glu Ser Leu Leu Lys Ala Ala Asn Thr Gly Pro His Ala Ala
20 25 30
Arg Asn Tyr Cys Leu Thr Leu Leu Cys Phe Ile His Gly Phe Arg Ala
35 40 45
Ser Glu Ile Cys Arg Leu Arg Ile Ser Asp Ile Asp Leu Lys Ala Lys
50 55 60
Cys Ile Tyr Ile His Arg Leu Lys Lys Gly Phe Ser Thr Thr His Pro
65 70 75 80

Leu Leu Asn Lys Glu Val Gln Ala Leu Lys Asn Trp Leu Ser Ile Arg
                85                  90                  95
Thr Ser Tyr Pro His Ala Glu Ser Glu Trp Val Phe Leu Ser Arg Lys
                100                 105                 110
Gly Asn Pro Leu Ser Arg Gln Gln Phe Tyr His Ile Ile Ser Thr Ser
        115                 120                 125
Gly Gly Asn Ala Gly Leu Ser Leu Glu Ile His Pro His Met Leu Arg
    130                 135                 140
His Ser Cys Gly Phe Ala Leu Ala Asn Met Gly Ile Asp Thr Arg Leu
145                 150                 155                 160
Ile Gln Asp Tyr Leu Gly His Arg Asn Ile Arg His Thr Val Trp Tyr
                165                 170                 175
Thr Ala Ser Asn Ala Gly Arg Phe Tyr Gly Ile Trp Asp Arg Ala Arg
                180                 185                 190
Gly Arg Gln Arg His Ala Val Leu
        195                 200

<210> 360
<211> 198
<212> PRT
<213> E. Coli

<400> 360
Met Ser Lys Arg Arg Tyr Leu Thr Gly Lys Glu Val Gln Ala Met Met
 1               5                  10                  15
Gln Ala Val Cys Tyr Gly Ala Thr Gly Ala Arg Asp Tyr Cys Leu Ile
                20                  25                  30
Leu Leu Ala Tyr Arg His Gly Met Arg Ile Ser Glu Leu Leu Asp Leu
        35                  40                  45
His Tyr Gln Asp Leu Asp Leu Asn Glu Gly Arg Ile Asn Ile Arg Arg
    50                  55                  60
Leu Lys Asn Gly Phe Ser Thr Val His Pro Leu Arg Phe Asp Glu Arg
65                  70                  75                  80
Glu Ala Val Glu Arg Trp Thr Gln Glu Arg Ala Asn Trp Lys Gly Ala
                85                  90                  95
Asp Arg Thr Asp Ala Ile Phe Ile Ser Arg Arg Gly Ser Arg Leu Ser
                100                 105                 110
Arg Gln Gln Ala Tyr Arg Ile Ile Arg Asp Ala Gly Ile Glu Ala Gly
        115                 120                 125
Thr Val Thr Gln Thr His Pro His Met Leu Arg His Ala Cys Gly Tyr
    130                 135                 140
Glu Leu Ala Glu Arg Gly Ala Asp Thr Arg Leu Ile Gln Asp Tyr Leu
145                 150                 155                 160
Gly His Arg Asn Ile Arg His Thr Val Arg Tyr Thr Ala Ser Asn Ala
                165                 170                 175
Ala Arg Phe Ala Gly Leu Trp Glu Arg Asn Asn Leu Ile Asn Glu Lys
                180                 185                 190
Leu Lys Arg Glu Glu Val
        195

<210> 361
<211> 182
<212> PRT
<213> E. Coli

<400> 361
Met Lys Ile Lys Thr Leu Ala Ile Val Val Leu Ser Ala Leu Ser Leu
 1               5                  10                  15

Ser Ser Thr Ala Ala Leu Ala Ala Ala Thr Thr Val Asn Gly Gly Thr
            20                  25                  30
Val His Phe Lys Gly Glu Val Val Asn Ala Ala Cys Ala Val Asp Ala
        35                  40                  45
Gly Ser Val Asp Gln Thr Val Gln Leu Gly Gln Val Arg Thr Ala Ser
    50                  55                  60
Leu Ala Gln Glu Gly Ala Thr Ser Ser Ala Val Gly Phe Asn Ile Gln
65                  70                  75                  80
Leu Asn Asp Cys Asp Thr Asn Val Ala Ser Lys Ala Ala Val Ala Phe
                85                  90                  95
Leu Gly Thr Ala Ile Asp Ala Gly His Thr Asn Val Leu Ala Leu Gln
            100                 105                 110
Ser Ser Ala Ala Gly Ser Ala Thr Asn Val Gly Val Gln Ile Leu Asp
        115                 120                 125
Arg Thr Gly Ala Ala Leu Thr Leu Asp Gly Ala Thr Phe Ser Ser Glu
    130                 135                 140
Thr Thr Leu Asn Asn Gly Thr Asn Thr Ile Pro Phe Gln Ala Arg Tyr
145                 150                 155                 160
Phe Ala Thr Gly Ala Ala Thr Pro Gly Ala Ala Asn Ala Asp Ala Thr
                165                 170                 175
Phe Lys Val Gln Tyr Gln
            180

<210> 362
<211> 215
<212> PRT
<213> E. Coli

<400> 362
Met Leu Leu Met Arg Met Arg Pro Ser Arg Phe Ser Ile Asn Asn Leu
1               5                   10                  15
Pro Arg Phe Arg Asp Val Ile Thr Gly Arg Asp Ala His Pro Cys Ala
            20                  25                  30
Ile Lys Ile Thr Met Lys Arg Lys Arg Leu Phe Leu Leu Ala Ser Leu
        35                  40                  45
Leu Pro Met Phe Ala Leu Ala Gly Asn Lys Trp Asn Thr Thr Leu Pro
    50                  55                  60
Gly Gly Asn Met Gln Phe Gln Gly Val Ile Ile Ala Glu Thr Cys Arg
65                  70                  75                  80
Ile Glu Ala Gly Asp Lys Gln Met Thr Val Asn Met Gly Gln Ile Ser
                85                  90                  95
Ser Asn Arg Phe His Ala Val Gly Glu Asp Ser Ala Pro Val Pro Phe
            100                 105                 110
Val Ile His Leu Arg Glu Cys Ser Thr Val Val Ser Glu Arg Val Gly
        115                 120                 125
Val Ala Phe His Gly Val Ala Asp Gly Lys Asn Pro Asp Val Leu Ser
    130                 135                 140
Val Gly Glu Gly Pro Gly Ile Ala Thr Asn Ile Gly Val Ala Leu Phe
145                 150                 155                 160
Asp Asp Glu Gly Asn Leu Val Pro Ile Asn Arg Pro Pro Ala Asn Trp
                165                 170                 175
Lys Arg Leu Tyr Ser Gly Ser Thr Ser Leu His Phe Ile Ala Lys Tyr
            180                 185                 190
Arg Ala Thr Gly Arg Arg Val Thr Gly Gly Ile Ala Asn Ala Gln Ala
        195                 200                 205
Trp Phe Ser Leu Thr Tyr Gln
    210                 215

<210> 363
<211> 241
<212> PRT
<213> E. Coli

<400> 363
Met Ser Asn Lys Asn Val Asn Val Arg Lys Ser Gln Glu Ile Thr Phe
1               5                   10                  15
Cys Leu Leu Ala Gly Ile Leu Met Phe Met Ala Met Met Val Ala Gly
            20                  25                  30
Arg Ala Glu Ala Gly Val Ala Leu Gly Ala Thr Arg Val Ile Tyr Pro
        35                  40                  45
Ala Gly Gln Lys Gln Glu Gln Leu Ala Val Thr Asn Asn Asp Glu Asn
    50                  55                  60
Ser Thr Tyr Leu Ile Gln Ser Trp Val Glu Asn Ala Asp Gly Val Lys
65                  70                  75                  80
Asp Gly Arg Phe Ile Val Thr Pro Pro Leu Phe Ala Met Lys Gly Lys
                85                  90                  95
Lys Glu Asn Thr Leu Arg Ile Leu Asp Ala Thr Asn Asn Gln Leu Pro
            100                 105                 110
Gln Asp Arg Glu Ser Leu Phe Trp Met Asn Val Lys Ala Ile Pro Ser
        115                 120                 125
Met Asp Lys Ser Lys Leu Thr Glu Asn Thr Leu Gln Leu Ala Ile Ile
    130                 135                 140
Ser Arg Ile Lys Leu Tyr Tyr Arg Pro Ala Lys Leu Ala Leu Pro Pro
145                 150                 155                 160
Asp Gln Ala Ala Glu Lys Leu Arg Phe Arg Arg Ser Ala Asn Ser Leu
                165                 170                 175
Thr Leu Ile Asn Pro Thr Pro Tyr Tyr Leu Thr Val Thr Glu Leu Asn
            180                 185                 190
Ala Gly Thr Arg Val Leu Glu Asn Ala Leu Val Pro Pro Met Gly Glu
        195                 200                 205
Ser Thr Val Lys Leu Pro Ser Asp Ala Gly Ser Asn Ile Thr Tyr Arg
    210                 215                 220
Thr Ile Asn Asp Tyr Gly Ala Leu Thr Pro Lys Met Thr Gly Val Met
225                 230                 235                 240
Glu

<210> 364
<211> 878
<212> PRT
<213> E. Coli

<400> 364
Met Ser Tyr Leu Asn Leu Arg Leu Tyr Gln Arg Asn Thr Gln Cys Leu
1               5                   10                  15
His Ile Arg Lys His Arg Leu Ala Gly Phe Phe Val Arg Leu Val Val
            20                  25                  30
Ala Cys Ala Phe Ala Ala Gln Ala Pro Leu Ser Ser Ala Asp Leu Tyr
        35                  40                  45
Phe Asn Pro Arg Phe Leu Ala Asp Asp Pro Gln Ala Val Ala Asp Leu
    50                  55                  60
Ser Arg Phe Glu Asn Gly Gln Glu Leu Pro Pro Gly Thr Tyr Arg Val
65                  70                  75                  80
Asp Ile Tyr Leu Asn Asn Gly Tyr Met Ala Thr Arg Asp Val Thr Phe
                85                  90                  95
Asn Thr Gly Asp Ser Glu Gln Gly Ile Val Pro Cys Leu Thr Arg Ala
            100                 105                 110
Gln Leu Ala Ser Met Gly Leu Asn Thr Ala Ser Val Ala Gly Met Asn

115                 120                 125
Leu Leu Ala Asp Asp Ala Cys Val Pro Leu Thr Thr Met Val Gln Asp
    130                 135                 140
Ala Thr Ala His Leu Asp Val Gly Gln Gln Arg Leu Asn Leu Thr Ile
145                 150                 155                 160
Pro Gln Ala Phe Met Ser Asn Arg Ala Arg Gly Tyr Ile Pro Pro Glu
                165                 170                 175
Leu Trp Asp Pro Gly Ile Asn Ala Gly Leu Leu Asn Tyr Asn Phe Ser
            180                 185                 190
Gly Asn Ser Val Gln Asn Arg Ile Gly Gly Asn Ser His Tyr Ala Tyr
        195                 200                 205
Leu Asn Leu Gln Ser Gly Leu Asn Ile Gly Ala Trp Arg Leu Arg Asp
    210                 215                 220
Asn Thr Thr Trp Ser Tyr Asn Ser Ser Asp Arg Ser Ser Gly Ser Lys
225                 230                 235                 240
Asn Lys Trp Gln His Ile Asn Thr Trp Leu Glu Arg Asp Ile Ile Pro
                245                 250                 255
Leu Arg Ser Arg Leu Thr Leu Gly Asp Gly Tyr Thr Gln Gly Asp Ile
            260                 265                 270
Phe Asp Gly Ile Asn Phe Arg Gly Ala Gln Leu Ala Ser Asp Asp Asn
        275                 280                 285
Met Leu Pro Asp Ser Gln Arg Gly Phe Ala Pro Val Ile His Gly Ile
    290                 295                 300
Ala Arg Gly Thr Ala Gln Val Thr Ile Lys Gln Asn Gly Tyr Asp Ile
305                 310                 315                 320
Tyr Asn Ser Thr Val Pro Pro Gly Pro Phe Thr Ile Asn Asp Ile Tyr
                325                 330                 335
Ala Ala Gly Asn Ser Gly Asp Leu Gln Val Thr Ile Lys Glu Ala Asp
            340                 345                 350
Gly Ser Thr Gln Ile Phe Thr Val Pro Tyr Ser Ser Val Pro Leu Leu
        355                 360                 365
Gln Arg Glu Gly His Thr Arg Tyr Ser Ile Thr Ala Gly Glu Tyr Arg
    370                 375                 380
Ser Gly Asn Ala Gln Gln Glu Lys Thr Arg Phe Phe Gln Ser Thr Leu
385                 390                 395                 400
Leu His Gly Leu Pro Ala Gly Trp Thr Ile Tyr Gly Gly Thr Gln Leu
                405                 410                 415
Ala Asp Arg Tyr Arg Ala Phe Asn Phe Gly Ile Gly Lys Asn Met Gly
            420                 425                 430
Ala Leu Gly Ala Leu Ser Val Asp Met Thr Gln Ala Asn Ser Thr Leu
        435                 440                 445
Pro Asp Asp Ser Gln His Asp Gly Gln Ser Val Arg Phe Leu Tyr Asn
    450                 455                 460
Lys Ser Leu Asn Glu Ser Gly Thr Asn Ile Gln Leu Val Gly Tyr Arg
465                 470                 475                 480
Tyr Ser Thr Ser Gly Tyr Phe Asn Phe Ala Asp Thr Thr Tyr Ser Arg
                485                 490                 495
Met Asn Gly Tyr Asn Ile Glu Thr Gln Asp Gly Val Ile Gln Val Lys
            500                 505                 510
Pro Lys Phe Thr Asp Tyr Tyr Asn Leu Ala Tyr Asn Lys Arg Gly Lys
        515                 520                 525
Leu Gln Leu Thr Val Thr Gln Gln Leu Gly Arg Thr Ser Thr Leu Tyr
    530                 535                 540
Leu Ser Gly Ser His Gln Thr Tyr Trp Gly Thr Ser Asn Val Asp Glu
545                 550                 555                 560
Gln Phe Gln Ala Gly Leu Asn Thr Ala Phe Glu Asp Ile Asn Trp Thr
                565                 570                 575
Leu Ser Tyr Ser Leu Thr Lys Asn Ala Trp Gln Lys Gly Arg Asp Gln
            580                 585                 590
Met Leu Ala Leu Asn Val Asn Ile Pro Phe Ser His Trp Leu Arg Ser
        595                 600                 605

Asp Ser Lys Ser Gln Trp Arg His Ala Ser Ala Ser Tyr Ser Met Ser
    610                 615                 620
His Asp Leu Asn Gly Arg Met Thr Asn Leu Ala Gly Val Tyr Gly Thr
625                 630                 635                 640
Leu Leu Glu Asp Asn Asn Leu Ser Tyr Ser Val Gln Thr Gly Tyr Ala
                645                 650                 655
Gly Gly Gly Asp Gly Asn Ser Gly Ser Thr Gly Tyr Ala Thr Leu Asn
            660                 665                 670
Tyr Arg Gly Gly Tyr Gly Asn Ala Asn Ile Gly Tyr Ser His Ser Asp
        675                 680                 685
Asp Ile Lys Gln Leu Tyr Tyr Gly Val Ser Gly Gly Val Leu Ala His
    690                 695                 700
Ala Asn Gly Val Thr Leu Gly Gln Pro Leu Asn Asp Thr Val Val Leu
705                 710                 715                 720
Val Lys Ala Pro Gly Ala Lys Asp Ala Lys Val Glu Asn Gln Thr Gly
                725                 730                 735
Val Arg Thr Asp Trp Arg Gly Tyr Ala Val Leu Pro Tyr Ala Thr Glu
            740                 745                 750
Tyr Arg Glu Asn Arg Val Ala Leu Asp Thr Asn Thr Leu Ala Asp Asn
        755                 760                 765
Val Asp Leu Asp Asn Ala Val Ala Asn Val Val Pro Thr Arg Gly Ala
    770                 775                 780
Ile Val Arg Ala Glu Phe Lys Ala Arg Val Gly Ile Lys Leu Leu Met
785                 790                 795                 800
Thr Leu Thr His Asn Asn Lys Pro Leu Pro Phe Gly Ala Met Val Thr
                805                 810                 815
Ser Glu Ser Ser Gln Ser Ser Gly Ile Val Ala Asp Asn Gly Gln Val
            820                 825                 830
Tyr Leu Ser Gly Met Pro Leu Ala Gly Lys Val Gln Val Lys Trp Gly
        835                 840                 845
Glu Glu Glu Asn Ala His Cys Val Ala Asn Tyr Gln Leu Pro Pro Glu
    850                 855                 860
Ser Gln Gln Gln Leu Leu Thr Gln Leu Ser Ala Glu Cys Arg
865                 870                 875

<210> 365
<211> 176
<212> PRT
<213> E. Coli

<400> 365
Met Arg Asn Lys Pro Phe Tyr Leu Leu Cys Ala Phe Leu Trp Leu Ala
1               5                   10                  15
Val Ser His Ala Leu Ala Ala Asp Ser Thr Ile Thr Ile Arg Gly Tyr
            20                  25                  30
Val Arg Asp Asn Gly Cys Ser Val Ala Ala Glu Ser Thr Asn Phe Thr
        35                  40                  45
Val Asp Leu Met Glu Asn Ala Ala Lys Gln Phe Asn Asn Ile Gly Ala
    50                  55                  60
Thr Thr Pro Val Val Pro Phe Arg Ile Leu Leu Ser Pro Cys Gly Asn
65                  70                  75                  80
Ala Val Ser Ala Val Lys Val Gly Phe Thr Gly Val Ala Asp Ser His
                85                  90                  95
Asn Ala Asn Leu Leu Ala Leu Glu Asn Thr Val Ser Ala Ala Ser Gly
            100                 105                 110
Leu Gly Ile Gln Leu Leu Asn Glu Gln Gln Asn Gln Ile Pro Leu Asn
        115                 120                 125
Ala Pro Ser Ser Ala Leu Ser Trp Thr Thr Leu Thr Pro Gly Lys Pro
    130                 135                 140
Asn Thr Leu Asn Phe Tyr Ala Arg Leu Met Ala Thr Gln Val Pro Val

145                 150                 155                 160
Thr Ala Gly His Ile Asn Ala Thr Ala Thr Phe Thr Leu Glu Tyr Gln
                165                 170                 175

<210> 366
<211> 167
<212> PRT
<213> E. Coli

<400> 366
Met Lys Trp Cys Lys Arg Gly Tyr Val Leu Ala Ala Ile Leu Ala Leu
1               5                   10                  15
Ala Ser Ala Thr Ile Gln Ala Ala Asp Val Thr Ile Thr Val Asn Gly
            20                  25                  30
Lys Val Val Ala Lys Pro Cys Thr Val Ser Thr Thr Asn Ala Thr Val
        35                  40                  45
Asp Leu Gly Asp Leu Tyr Ser Phe Ser Leu Met Ser Ala Gly Ala Ala
    50                  55                  60
Ser Ala Trp His Asp Val Ala Leu Glu Leu Thr Asn Cys Pro Val Gly
65                  70                  75                  80
Thr Ser Arg Val Thr Ala Ser Phe Ser Gly Ala Ala Asp Ser Thr Gly
                85                  90                  95
Tyr Tyr Lys Asn Gln Gly Thr Ala Gln Asn Ile Gln Leu Glu Leu Gln
            100                 105                 110
Asp Asp Ser Gly Asn Thr Leu Asn Thr Gly Ala Thr Lys Thr Val Gln
        115                 120                 125
Val Asp Asp Ser Ser Gln Ser Ala His Phe Pro Leu Gln Val Arg Ala
    130                 135                 140
Leu Thr Val Asn Gly Gly Ala Thr Gln Gly Thr Ile Gln Ala Val Ile
145                 150                 155                 160
Ser Ile Thr Tyr Thr Tyr Ser
                165

<210> 367
<211> 300
<212> PRT
<213> E. Coli

<400> 367
Met Lys Arg Val Ile Thr Leu Phe Ala Val Leu Leu Met Gly Trp Ser
1               5                   10                  15
Val Asn Ala Trp Ser Phe Ala Cys Lys Thr Ala Asn Gly Thr Ala Ile
            20                  25                  30
Pro Ile Gly Gly Gly Ser Ala Asn Val Tyr Val Asn Leu Ala Pro Val
        35                  40                  45
Val Asn Val Gly Gln Asn Leu Val Val Asp Leu Ser Thr Gln Ile Phe
    50                  55                  60
Cys His Asn Asp Tyr Pro Glu Thr Ile Thr Asp Tyr Val Thr Leu Gln
65                  70                  75                  80
Arg Gly Ser Ala Tyr Gly Gly Val Leu Ser Asn Phe Ser Gly Thr Val
                85                  90                  95
Lys Tyr Ser Gly Ser Ser Tyr Pro Phe Pro Thr Thr Ser Glu Thr Pro
            100                 105                 110
Arg Val Val Tyr Asn Ser Arg Thr Asp Lys Pro Trp Pro Val Ala Leu
        115                 120                 125
Tyr Leu Thr Pro Val Ser Ser Ala Gly Gly Val Ala Ile Lys Ala Gly
    130                 135                 140

Ser Leu Ile Ala Val Leu Ile Leu Arg Gln Thr Asn Asn Tyr Asn Ser
145                 150                 155                 160
Asp Asp Phe Gln Phe Val Trp Asn Ile Tyr Ala Asn Asn Asp Val Val
                165                 170                 175
Val Pro Thr Gly Gly Cys Asp Val Ser Ala Arg Asp Val Thr Val Thr
            180                 185                 190
Leu Pro Asp Tyr Pro Gly Ser Val Pro Ile Pro Leu Thr Val Tyr Cys
        195                 200                 205
Ala Lys Ser Gln Asn Leu Gly Tyr Tyr Leu Ser Gly Thr Thr Ala Asp
    210                 215                 220
Ala Gly Asn Ser Ile Phe Thr Asn Thr Ala Ser Phe Ser Pro Ala Gln
225                 230                 235                 240
Gly Val Gly Val Gln Leu Thr Arg Asn Gly Thr Ile Ile Pro Ala Asn
                245                 250                 255
Asn Thr Val Ser Leu Gly Ala Val Gly Thr Ser Ala Val Ser Leu Gly
            260                 265                 270
Leu Thr Ala Asn Tyr Ala Arg Thr Gly Gly Gln Val Thr Ala Gly Asn
        275                 280                 285
Val Gln Ser Ile Ile Gly Val Thr Phe Val Tyr Gln
    290                 295                 300

<210> 368
<211> 521
<212> PRT
<213> E. Coli

<400> 368
Met Leu Ser Lys Leu Pro Arg Arg Leu Arg Ser Phe Gln Thr Tyr Cys
 1               5                  10                  15
Thr Ile Arg Val His Arg Gly Glu Asp Met Lys Ser Met Asp Lys Leu
            20                  25                  30
Thr Thr Gly Val Ala Tyr Gly Thr Ser Ala Gly Asn Ala Gly Phe Trp
        35                  40                  45
Ala Leu Gln Leu Leu Asp Lys Val Thr Pro Ser Gln Trp Ala Ala Ile
    50                  55                  60
Gly Val Leu Gly Ser Leu Val Phe Gly Leu Leu Thr Tyr Leu Thr Asn
65                  70                  75                  80
Leu Tyr Phe Lys Ile Lys Glu Asp Arg Arg Lys Ala Ala Arg Gly Glu
                85                  90                  95
Ser Asn Asp Ser Arg Leu Thr Gly Cys Glu Arg Ser Pro Phe Glu Ser
            100                 105                 110
Tyr Gly Asn Cys Ser Leu Thr Gly Gln Arg Thr Leu Arg Asn Phe Pro
        115                 120                 125
Gly Cys Arg His Gly Pro Cys Arg Ser Cys Ala Gly Val Leu Gly Ser
    130                 135                 140
Ser Gln Lys Glu Arg Pro Ala Ser Leu Pro Gly Ser Ser Arg Lys Ile
145                 150                 155                 160
Val Arg Lys Ser Val Leu Ser Ala Ala Ser Val Leu Leu Asp Lys Ser
                165                 170                 175
Cys Gln Ala Arg Ala Ser Ser Ser Ile Ser Met Asn Thr Lys Ile Arg
            180                 185                 190
Tyr Gly Leu Ser Ala Ala Val Leu Ala Leu Ile Gly Ala Gly Ala Ser
        195                 200                 205
Ala Pro Gln Ile Leu Asp Gln Phe Leu Asp Glu Lys Glu Gly Asn His
    210                 215                 220
Thr Met Ala Tyr Arg Asp Gly Ser Gly Ile Trp Thr Ile Cys Arg Gly
225                 230                 235                 240
Ala Thr Val Val Asp Gly Lys Thr Val Phe Pro Asn Met Lys Leu Ser
                245                 250                 255

Lys Glu Lys Cys Asp Gln Val Asn Ala Ile Glu Arg Asp Lys Ala Leu
            260                 265                 270
Ala Trp Val Glu Arg Asn Ile Lys Val Pro Leu Thr Glu Pro Gln Lys
        275                 280                 285
Ala Gly Ile Ala Ser Phe Cys Pro Tyr Asn Ile Gly Pro Gly Lys Cys
    290                 295                 300
Phe Pro Ser Thr Phe Tyr Lys Arg Leu Asn Ala Gly Asp Arg Lys Gly
305                 310                 315                 320
Ala Cys Glu Ala Ile Arg Trp Trp Ile Lys Asp Gly Gly Arg Asp Cys
                325                 330                 335
Arg Ile Arg Ser Asn Asn Cys Tyr Gly Gln Val Ile Arg Arg Asp Gln
            340                 345                 350
Glu Ser Ala Leu Thr Cys Trp Gly Ile Glu Gln Ile Arg Tyr Ser Trp
        355                 360                 365
Phe Phe Ser Cys Cys Gln Asp Leu Ser Ser Glu Met Ser Gly Ala Thr
    370                 375                 380
Glu Asp Gly Lys Lys Asn Gly Arg Asn Val Met Leu Pro His Tyr His
385                 390                 395                 400
Lys Arg Met Leu Asn Leu Leu Leu Glu Leu Asn Arg Gly Glu Leu Pro
                405                 410                 415
Val Met Arg Leu Leu Lys Met Arg Asn Arg Asn Leu Leu Lys Phe Leu
            420                 425                 430
Pro Gly Leu Leu Ile Cys Leu Ile Val Leu Thr Ser Cys Val Pro Lys
        435                 440                 445
Gln Lys Asn Met Pro Tyr Ala Leu Thr Gln Arg Ser Ile Pro Gln Ile
    450                 455                 460
Leu Pro Leu Pro Ser Glu Ala Lys Gln Pro Lys Pro Pro Lys Glu Cys
465                 470                 475                 480
Ser Pro Thr Cys Ser Glu Ile Leu Gln Gln Lys Leu Ser Phe Met Leu
                485                 490                 495
Lys Leu Leu Thr Asn Ala Thr Ser Gln Glu Leu Val Asn Arg Ser Met
            500                 505                 510
Asn Leu Glu Ile Lys Ser Ile Lys Cys
        515                 520

<210> 369
<211> 177
<212> PRT
<213> E. Coli

<400> 369
Met Asn Thr Lys Ile Arg Tyr Gly Leu Ser Ala Ala Val Leu Ala Leu
1               5                   10                  15
Ile Gly Ala Gly Ala Ser Ala Pro Gln Ile Leu Asp Gln Phe Leu Asp
            20                  25                  30
Glu Lys Glu Gly Asn His Thr Met Ala Tyr Arg Asp Gly Ser Gly Ile
        35                  40                  45
Trp Thr Ile Cys Arg Gly Ala Thr Val Val Asp Gly Lys Thr Val Phe
    50                  55                  60
Pro Asn Met Lys Leu Ser Lys Glu Lys Cys Asp Gln Val Asn Ala Ile
65                  70                  75                  80
Glu Arg Asp Lys Ala Leu Ala Trp Val Glu Arg Asn Ile Lys Val Pro
                85                  90                  95
Leu Thr Glu Pro Gln Lys Ala Gly Ile Ala Ser Phe Cys Pro Tyr Asn
            100                 105                 110
Ile Gly Pro Gly Lys Cys Phe Pro Ser Thr Phe Tyr Lys Arg Leu Asn
        115                 120                 125
Ala Gly Asp Arg Lys Gly Ala Cys Glu Ala Ile Arg Trp Trp Ile Lys
    130                 135                 140

Asp Gly Gly Arg Asp Cys Arg Ile Arg Ser Asn Asn Cys Tyr Gly Gln
145                 150                 155                 160
Val Ile Arg Arg Asp Gln Glu Ser Ala Leu Thr Cys Trp Gly Ile Glu
                165                 170                 175
Gln

<210> 370
<211> 103
<212> PRT
<213> E. Coli

<400> 370
Met Thr Gln Asp Tyr Glu Leu Val Val Lys Gly Val Arg Asn Phe Glu
1               5                   10                  15
Asn Lys Val Thr Val Thr Val Ala Leu Gln Asp Lys Glu Arg Phe Asp
            20                  25                  30
Gly Glu Ile Phe Asp Leu Asp Val Ala Met Asp Arg Val Glu Gly Ala
        35                  40                  45
Ala Leu Glu Phe Tyr Glu Ala Ala Ala Arg Arg Ser Val Arg Gln Val
    50                  55                  60
Phe Leu Glu Val Ala Glu Lys Leu Ser Glu Lys Val Glu Ser Tyr Leu
65                  70                  75                  80
Gln His Gln Tyr Ser Phe Lys Ile Glu Asn Pro Ala Asn Lys His Glu
                85                  90                  95
Arg Pro His His Lys Tyr Leu
            100

<210> 371
<211> 96
<212> PRT
<213> E. Coli

<400> 371
Met Leu Ser Lys Leu Pro Arg Arg Leu Arg Ser Phe Gln Thr Tyr Cys
1               5                   10                  15
Thr Ile Arg Val His Arg Gly Glu Asp Met Lys Ser Met Asp Lys Leu
            20                  25                  30
Thr Thr Gly Val Ala Tyr Gly Thr Ser Ala Gly Asn Ala Gly Phe Trp
        35                  40                  45
Ala Leu Gln Leu Leu Asp Lys Val Thr Pro Ser Gln Trp Ala Ala Ile
    50                  55                  60
Gly Val Leu Gly Ser Leu Val Phe Gly Leu Leu Thr Tyr Leu Thr Asn
65                  70                  75                  80
Leu Tyr Phe Lys Ile Lys Glu Asp Arg Arg Lys Ala Ala Arg Gly Glu
                85                  90                  95

<210> 372
<211> 71
<212> PRT
<213> E. Coli

<400> 372
Met Ser Asn Lys Met Thr Gly Leu Val Lys Trp Phe Asn Ala Asp Lys
1               5                   10                  15
Gly Phe Gly Phe Ile Ser Pro Val Asp Gly Ser Lys Asp Val Phe Val

20                  25                  30
His Phe Ser Ala Ile Gln Asn Asp Asn Tyr Arg Thr Leu Phe Glu Gly
        35                  40                  45
Gln Lys Val Thr Phe Ser Ile Glu Ser Gly Ala Lys Gly Pro Ala Ala
    50                  55                  60
Ala Asn Val Ile Ile Thr Asp
65                  70

<210> 373
<211> 338
<212> PRT
<213> E. Coli

<400> 373
Met Phe Val Ile Trp Ser His Arg Thr Gly Phe Ile Met Ser His Gln
1               5                   10                  15
Leu Thr Phe Ala Asp Ser Glu Phe Ser Ser Lys Arg Arg Gln Thr Arg
            20                  25                  30
Lys Glu Ile Phe Leu Ser Arg Met Glu Gln Ile Leu Pro Trp Gln Asn
        35                  40                  45
Met Val Glu Val Ile Glu Pro Phe Tyr Pro Lys Ala Gly Asn Gly Arg
    50                  55                  60
Arg Pro Tyr Pro Leu Glu Thr Met Leu Arg Ile His Cys Met Gln His
65                  70                  75                  80
Trp Tyr Asn Leu Ser Asp Gly Ala Met Glu Asp Ala Leu Tyr Glu Ile
                85                  90                  95
Ala Ser Met Arg Leu Phe Ala Arg Leu Ser Leu Asp Ser Ala Leu Pro
            100                 105                 110
Asp Arg Thr Thr Ile Met Asn Phe Arg His Leu Leu Glu Gln His Gln
        115                 120                 125
Leu Ala Arg Gln Leu Phe Lys Thr Ile Asn Arg Trp Leu Ala Glu Ala
    130                 135                 140
Gly Val Met Met Thr Gln Gly Thr Leu Val Asp Ala Thr Ile Ile Glu
145                 150                 155                 160
Ala Pro Ser Ser Thr Lys Asn Lys Glu Gln Gln Arg Asp Pro Glu Met
                165                 170                 175
His Gln Thr Lys Lys Gly Asn Gln Trp His Phe Gly Met Lys Ala His
            180                 185                 190
Ile Gly Val Asp Ala Lys Ser Gly Leu Thr His Ser Leu Val Thr Thr
        195                 200                 205
Ala Ala Asn Glu His Asp Leu Asn Gln Leu Gly Asn Leu Leu His Gly
    210                 215                 220
Glu Glu Gln Phe Val Ser Ala Asp Ala Gly Tyr Gln Gly Ala Pro Gln
225                 230                 235                 240
Arg Glu Glu Leu Ala Glu Val Asp Val Asp Trp Leu Ile Ala Glu Arg
                245                 250                 255
Pro Gly Lys Val Arg Thr Leu Lys Gln His Pro Arg Lys Asn Lys Thr
            260                 265                 270
Ala Ile Asn Ile Glu Tyr Met Lys Ala Ser Ile Arg Ala Arg Val Glu
        275                 280                 285
His Pro Phe Arg Ile Ile Lys Arg Gln Phe Gly Phe Val Lys Ala Arg
    290                 295                 300
Tyr Lys Gly Leu Leu Lys Asn Asp Asn Gln Leu Ala Met Leu Phe Thr
305                 310                 315                 320
Leu Ala Asn Leu Phe Arg Ala Asp Gln Met Ile Arg Gln Trp Glu Arg
                325                 330                 335
Ser His

<210> 374
<211> 157
<212> PRT
<213> E. Coli

<400> 374
Met Val Tyr Ile Ile Ile Val Ser His Gly His Glu Asp Tyr Ile Lys
1               5                   10                  15
Lys Leu Leu Glu Asn Leu Asn Ala Asp Asp Glu His Tyr Lys Ile Ile
            20                  25                  30
Val Arg Asp Asn Lys Asp Ser Leu Leu Leu Lys Gln Ile Cys Gln His
        35                  40                  45
Tyr Ala Gly Leu Asp Tyr Ile Ser Gly Gly Val Tyr Gly Phe Gly His
    50                  55                  60
Asn Asn Asn Ile Ala Val Ala Tyr Val Lys Glu Lys Tyr Arg Pro Ala
65                  70                  75                  80
Asp Asp Asp Tyr Ile Leu Phe Leu Asn Pro Asp Ile Ile Met Lys His
                85                  90                  95
Asp Asp Leu Leu Thr Tyr Ile Lys Tyr Val Glu Ser Lys Arg Tyr Ala
            100                 105                 110
Phe Ser Thr Leu Cys Leu Phe Arg Asp Glu Ala Lys Ser Leu His Asp
        115                 120                 125
Tyr Ser Val Arg Lys Phe Pro Val Leu Ser Asp Phe Ile Val Ser Phe
    130                 135                 140
Met Leu Gly Ile Lys Glu Gly Ala Asn Lys Ser Leu Ile
145                 150                 155

<210> 375
<211> 372
<212> PRT
<213> E. Coli

<400> 375
Met Gly Lys Ser Ile Val Val Val Ser Ala Val Asn Phe Thr Thr Gly
1               5                   10                  15
Gly Pro Phe Thr Ile Leu Lys Lys Phe Leu Ala Ala Thr Asn Asn Lys
            20                  25                  30
Glu Asn Val Ser Phe Ile Ala Leu Val His Ser Ala Lys Glu Leu Lys
        35                  40                  45
Glu Ser Tyr Pro Trp Val Lys Phe Ile Glu Phe Pro Glu Val Lys Gly
    50                  55                  60
Ser Trp Leu Lys Arg Leu His Phe Glu Tyr Val Val Cys Lys Lys Leu
65                  70                  75                  80
Ser Lys Glu Leu Asn Ala Thr His Trp Ile Cys Leu His Asp Ile Thr
                85                  90                  95
Ala Asn Val Val Thr Lys Lys Arg Tyr Val Tyr Cys His Asn Pro Ala
            100                 105                 110
Pro Phe Tyr Lys Gly Ile Leu Phe Arg Glu Ile Leu Met Glu Pro Ser
        115                 120                 125
Phe Phe Leu Phe Lys Met Leu Tyr Gly Leu Ile Tyr Lys Ile Asn Ile
    130                 135                 140
Lys Lys Asn Thr Ala Val Phe Val Gln Gln Phe Trp Met Lys Glu Lys
145                 150                 155                 160
Phe Ile Lys Lys Tyr Ser Ile Asn Asn Ile Ile Val Ser Arg Pro Glu
                165                 170                 175
Ile Lys Leu Ser Asp Lys Ser Gln Leu Thr Asp Asp Asp Ser Gln Phe
            180                 185                 190
Lys Asn Asn Pro Ser Glu Leu Thr Ile Phe Tyr Pro Ala Val Pro Arg

195                 200                 205
Val Phe Lys Asn Tyr Glu Leu Ile Ile Ser Ala Ala Arg Lys Leu Lys
    210                 215                 220
Glu Gln Ser Asn Ile Lys Phe Leu Leu Thr Ile Ser Gly Thr Glu Asn
225                 230                 235                 240
Ala Tyr Ala Lys Tyr Ile Ile Ser Leu Ala Glu Gly Leu Asp Asn Val
                245                 250                 255
His Phe Leu Gly Tyr Leu Asp Lys Glu Lys Ile Asp His Cys Tyr Asn
            260                 265                 270
Ile Ser Asp Ile Val Cys Phe Pro Ser Arg Leu Glu Thr Trp Gly Leu
        275                 280                 285
Pro Leu Ser Glu Ala Lys Glu Arg Gly Lys Trp Val Leu Ala Ser Asp
    290                 295                 300
Phe Pro Phe Thr Arg Glu Thr Leu Gly Ser Tyr Glu Lys Lys Ala Phe
305                 310                 315                 320
Phe Asp Ser Asn Asn Asp Asp Met Leu Val Lys Leu Ile Ile Asp Phe
                325                 330                 335
Lys Lys Gly Asn Leu Lys Lys Asp Ile Ser Asp Ala Asn Phe Ile Tyr
            340                 345                 350
Arg Asn Glu Asn Val Leu Val Gly Phe Asp Glu Leu Val Asn Phe Ile
        355                 360                 365
Thr Glu Glu His
    370

<210> 376
<211> 196
<212> PRT
<213> E. Coli

<400> 376
Met Ile Leu Lys Leu Ala Lys Arg Tyr Gly Leu Cys Gly Phe Ile Arg
1               5                   10                  15
Leu Val Arg Asp Val Leu Leu Thr Arg Val Phe Tyr Arg Asn Cys Arg
            20                  25                  30
Ile Ile Arg Phe Pro Cys Tyr Ile Arg Asn Asp Gly Ser Ile Asn Phe
        35                  40                  45
Gly Glu Asn Phe Thr Ser Gly Val Gly Leu Arg Leu Asp Ala Phe Gly
    50                  55                  60
Arg Gly Val Ile Phe Phe Ser Asp Asn Val Gln Val Asn Asp Tyr Val
65                  70                  75                  80
His Ile Ala Ser Ile Glu Ser Val Thr Ile Gly Arg Asp Thr Leu Ile
                85                  90                  95
Ala Ser Lys Val Phe Ile Thr Asp His Asn His Gly Ser Phe Lys His
            100                 105                 110
Ser Asp Pro Met Ser Ser Pro Asn Ile Pro Pro Asp Met Arg Thr Leu
        115                 120                 125
Glu Ser Ser Ala Val Val Ile Gly Gln Arg Val Trp Leu Gly Glu Asn
    130                 135                 140
Val Thr Val Leu Pro Gly Thr Ile Ile Gly Asn Gly Val Val Val Gly
145                 150                 155                 160
Ala Asn Ser Val Val Arg Gly Ser Ile Pro Glu Asn Thr Val Ile Ala
                165                 170                 175
Gly Val Pro Ala Lys Ile Ile Lys Lys Tyr Asn His Glu Thr Lys Leu
            180                 185                 190
Trp Glu Lys Ala
        195

<210> 377
<211> 330
<212> PRT

<213> E. Coli

<400> 377
Met Tyr Phe Leu Asn Asp Leu Asn Phe Ser Arg Arg Asp Ala Gly Phe
1               5                   10                  15
Lys Ala Arg Lys Asp Ala Leu Asp Ile Ala Ser Asp Tyr Glu Asn Ile
            20                  25                  30
Ser Val Val Asn Ile Pro Leu Trp Gly Gly Val Val Gln Arg Ile Ile
        35                  40                  45
Ser Ser Val Lys Leu Ser Thr Phe Leu Cys Gly Leu Glu Asn Lys Asp
    50                  55                  60
Val Leu Ile Phe Asn Phe Pro Met Ala Lys Pro Phe Trp His Ile Leu
65                  70                  75                  80
Ser Phe Phe His Arg Leu Leu Lys Phe Arg Ile Val Pro Leu Ile His
                85                  90                  95
Asp Ile Asp Glu Leu Arg Gly Gly Gly Gly Ser Asp Ser Val Arg Leu
            100                 105                 110
Ala Thr Cys Asp Met Val Ile Ser His Asn Pro Gln Met Thr Lys Tyr
        115                 120                 125
Leu Ser Lys Tyr Met Ser Gln Asp Lys Ile Lys Asp Ile Lys Ile Phe
    130                 135                 140
Asp Tyr Leu Val Ser Ser Asp Val Glu His Arg Asp Val Thr Asp Lys
145                 150                 155                 160
Gln Arg Gly Val Ile Tyr Ala Gly Asn Leu Ser Arg His Lys Cys Ser
                165                 170                 175
Phe Ile Tyr Thr Glu Gly Cys Asp Phe Thr Leu Phe Gly Val Asn Tyr
            180                 185                 190
Glu Asn Lys Asp Asn Pro Lys Tyr Leu Gly Ser Phe Asp Ala Gln Ser
        195                 200                 205
Pro Glu Lys Ile Asn Leu Pro Gly Met Gln Phe Gly Leu Ile Trp Asp
    210                 215                 220
Gly Asp Ser Val Glu Thr Cys Ser Gly Ala Phe Gly Asp Tyr Leu Lys
225                 230                 235                 240
Phe Asn Asn Pro His Lys Thr Ser Leu Tyr Leu Ser Met Glu Leu Pro
                245                 250                 255
Val Phe Ile Trp Asp Lys Ala Ala Leu Ala Asp Phe Ile Val Asp Asn
            260                 265                 270
Arg Ile Gly Tyr Ala Val Gly Ser Ile Lys Glu Met Gln Glu Ile Val
        275                 280                 285
Asp Ser Met Thr Ile Glu Thr Tyr Lys Gln Ile Ser Glu Asn Thr Lys
    290                 295                 300
Ile Ile Ser Gln Lys Ile Arg Thr Gly Ser Tyr Phe Arg Asp Val Leu
305                 310                 315                 320
Glu Glu Val Ile Asp Asp Leu Lys Thr Arg
                325                 330

<210> 378
<211> 388
<212> PRT
<213> E. Coli

<400> 378
Met Ile Tyr Leu Val Ile Ser Val Phe Leu Ile Thr Ala Phe Ile Cys
1               5                   10                  15
Leu Tyr Leu Lys Lys Asp Ile Phe Tyr Pro Ala Val Cys Val Asn Ile
            20                  25                  30
Ile Phe Ala Leu Val Leu Leu Gly Tyr Glu Ile Thr Ser Asp Ile Tyr
        35                  40                  45
Ala Phe Gln Leu Asn Asp Ala Thr Leu Ile Phe Leu Leu Cys Asn Val
    50                  55                  60

Leu Thr Phe Thr Leu Ser Cys Leu Leu Thr Glu Ser Val Leu Asp Leu
65                  70                  75                  80
Asn Ile Arg Lys Val Asn Asn Ala Ile Tyr Ser Ile Pro Ser Lys Lys
                85                  90                  95
Val His Asn Val Gly Leu Leu Val Ile Ser Phe Ser Met Ile Tyr Ile
            100                 105                 110
Cys Met Arg Leu Ser Asn Tyr Gln Phe Gly Thr Ser Leu Leu Ser Tyr
        115                 120                 125
Met Asn Leu Ile Arg Asp Ala Asp Val Glu Asp Thr Ser Arg Asn Phe
    130                 135                 140
Ser Ala Tyr Met Gln Pro Ile Ile Leu Thr Thr Phe Ala Leu Phe Ile
145                 150                 155                 160
Trp Ser Lys Lys Phe Thr Asn Thr Lys Val Ser Lys Thr Phe Thr Leu
                165                 170                 175
Leu Val Phe Ile Val Phe Ile Phe Ala Ile Ile Leu Asn Thr Gly Lys
            180                 185                 190
Gln Ile Val Phe Met Val Ile Ile Ser Tyr Ala Phe Ile Val Gly Val
        195                 200                 205
Asn Arg Val Lys His Tyr Val Tyr Leu Ile Thr Ala Val Gly Val Leu
    210                 215                 220
Phe Ser Leu Tyr Met Leu Phe Leu Arg Gly Leu Pro Gly Gly Met Ala
225                 230                 235                 240
Tyr Tyr Leu Ser Met Tyr Leu Val Ser Pro Ile Ile Ala Phe Gln Glu
                245                 250                 255
Phe Tyr Phe Gln Gln Val Ser Asn Ser Ala Ser Ser His Val Phe Trp
            260                 265                 270
Phe Phe Glu Arg Leu Met Gly Leu Leu Thr Gly Gly Val Ser Met Ser
        275                 280                 285
Leu His Lys Glu Phe Val Trp Val Gly Leu Pro Thr Asn Val Tyr Thr
    290                 295                 300
Ala Phe Ser Asp Tyr Val Tyr Ile Ser Ala Glu Leu Ser Tyr Leu Met
305                 310                 315                 320
Met Val Ile His Gly Cys Ile Ser Gly Val Leu Trp Arg Leu Ser Arg
                325                 330                 335
Asn Tyr Ile Ser Val Lys Ile Phe Tyr Ser Tyr Phe Ile Tyr Thr Phe
            340                 345                 350
Ser Phe Ile Phe Tyr His Glu Ser Phe Met Thr Asn Ile Ser Ser Trp
        355                 360                 365
Ile Gln Ile Thr Leu Cys Ile Ile Val Phe Ser Gln Phe Leu Lys Ala
    370                 375                 380
Gln Lys Ile Lys
385

<210> 379
<211> 367
<212> PRT
<213> E. Coli

<400> 379

<210> 380
<211> 371
<212> PRT
<213> E. Coli

<400> 380
Met Phe Pro Lys Ile Met Asn Asp Glu Asn Phe Phe Lys Lys Ala Ala
1               5                   10                  15
Ala His Gly Glu Glu Pro Pro Leu Thr Pro Gln Asn Glu His Gln Arg
            20                  25                  30
Ser Gly Leu Arg Phe Ala Arg Arg Val Arg Leu Pro Arg Ala Val Gly
        35                  40                  45
Leu Ala Gly Met Phe Leu Pro Ile Ala Ser Thr Leu Val Ser His Pro
    50                  55                  60
Pro Pro Gly Trp Trp Trp Leu Val Leu Val Gly Trp Ala Phe Val Trp
65                  70                  75                  80
Pro His Leu Ala Trp Gln Ile Ala Ser Arg Ala Val Asp Pro Leu Ser
                85                  90                  95
Arg Glu Ile Tyr Asn Leu Lys Thr Asp Ala Val Leu Ala Gly Met Trp
            100                 105                 110
Val Gly Val Met Gly Val Asn Val Leu Pro Ser Thr Ala Met Leu Met
        115                 120                 125
Ile Met Cys Leu Asn Leu Met Gly Ala Gly Gly Pro Arg Leu Phe Val

130                 135                 140
Ala Gly Leu Val Leu Met Val Val Ser Cys Leu Val Thr Leu Glu Leu
145                 150                 155                 160
Thr Gly Ile Thr Val Ser Phe Asn Ser Ala Pro Leu Glu Trp Trp Leu
                165                 170                 175
Ser Leu Pro Ile Ile Val Ile Tyr Pro Leu Leu Phe Gly Trp Val Ser
            180                 185                 190
Tyr Gln Thr Ala Thr Lys Leu Ala Glu His Lys Arg Arg Leu Gln Val
        195                 200                 205
Met Ser Thr Arg Asp Gly Met Thr Gly Val Tyr Asn Arg Arg His Trp
    210                 215                 220
Glu Thr Met Leu Arg Asn Glu Phe Asp Asn Cys Arg Arg His Asn Arg
225                 230                 235                 240
Asp Ala Thr Leu Leu Ile Ile Asp Ile Asp His Phe Lys Ser Ile Asn
                245                 250                 255
Asp Thr Trp Gly His Asp Val Gly Asp Glu Ala Ile Val Ala Leu Thr
            260                 265                 270
Arg Gln Leu Gln Ile Thr Leu Arg Gly Ser Asp Val Ile Gly Arg Phe
        275                 280                 285
Gly Gly Asp Glu Phe Ala Val Ile Met Ser Gly Thr Pro Ala Glu Ser
    290                 295                 300
Ala Ile Thr Ala Met Leu Arg Val His Glu Gly Leu Asn Thr Leu Arg
305                 310                 315                 320
Leu Pro Asn Thr Pro Gln Val Thr Leu Arg Ile Ser Val Gly Val Ala
                325                 330                 335
Pro Leu Asn Pro Gln Met Ser His Tyr Arg Glu Trp Leu Lys Ser Ala
            340                 345                 350
Asp Leu Ala Leu Tyr Lys Ala Lys Lys Ala Gly Arg Asn Arg Thr Glu
        355                 360                 365
Val Ala Ala
    370

<210> 381
<211> 467
<212> PRT
<213> E. Coli

<400> 381
Met Asp Val Asn Val Asp Gln Phe Asp Thr Glu Ala Phe Arg Thr Asp
1               5                   10                  15
Lys Leu Glu Leu Thr Ser Gly Asn Ile Ala Asp His Asn Gly Asn Val
            20                  25                  30
Val Ser Gly Val Phe Asp Ile His Ser Ser Asp Tyr Val Leu Asn Ala
        35                  40                  45
Asp Leu Val Asn Asp Arg Thr Trp Asp Thr Ser Lys Ser Asn Tyr Gly
    50                  55                  60
Tyr Gly Ile Val Ala Met Asn Ser Asp Gly His Leu Thr Ile Asn Gly
65                  70                  75                  80
Asn Gly Asp Val Asp Asn Gly Thr Glu Leu Asp Asn Ser Ser Val Asp
                85                  90                  95
Asn Val Val Ala Ala Thr Gly Asn Tyr Lys Val Arg Ile Asp Asn Ala
            100                 105                 110
Thr Gly Ala Gly Ala Ile Ala Asp Tyr Lys Asp Lys Glu Ile Ile Tyr
        115                 120                 125
Val Asn Asp Val Asn Ser Asn Ala Thr Phe Ser Ala Ala Asn Lys Ala
    130                 135                 140
Asp Leu Gly Ala Tyr Thr Tyr Gln Ala Glu Gln Arg Gly Asn Thr Val
145                 150                 155                 160
Val Leu Gln Gln Met Glu Leu Thr Asp Tyr Ala Asn Met Ala Leu Ser
                165                 170                 175
Ile Pro Ser Ala Asn Thr Asn Ile Trp Asn Leu Glu Gln Asp Thr Val

            180                 185                 190
Gly Thr Arg Leu Thr Asn Ser Arg His Gly Leu Ala Asp Asn Gly Gly
        195                 200                 205
Ala Trp Val Ser Tyr Phe Gly Gly Asn Phe Asn Gly Asp Asn Gly Thr
    210                 215                 220
Ile Asn Tyr Asp Gln Asp Val Asn Gly Ile Met Val Gly Val Asp Thr
225                 230                 235                 240
Lys Ile Asp Gly Asn Asn Ala Lys Trp Ile Val Gly Ala Ala Ala Gly
                245                 250                 255
Phe Ala Lys Gly Asp Met Asn Asp Arg Ser Gly Gln Val Asp Gln Asp
            260                 265                 270
Ser Gln Thr Ala Tyr Ile Tyr Ser Ser Ala His Phe Ala Asn Asn Val
        275                 280                 285
Phe Val Asp Gly Ser Leu Ser Tyr Ser His Phe Asn Asn Asp Leu Ser
    290                 295                 300
Ala Thr Met Ser Asn Gly Thr Tyr Val Asp Gly Ser Thr Asn Ser Asp
305                 310                 315                 320
Ala Trp Gly Phe Gly Leu Lys Ala Gly Tyr Asp Phe Lys Leu Gly Asp
                325                 330                 335
Ala Gly Tyr Val Thr Pro Tyr Gly Ser Val Ser Gly Leu Phe Gln Ser
            340                 345                 350
Gly Asp Asp Tyr Gln Leu Ser Asn Asp Met Lys Val Asp Gly Gln Ser
        355                 360                 365
Tyr Asp Ser Met Arg Tyr Glu Leu Gly Val Asp Ala Gly Tyr Thr Phe
    370                 375                 380
Thr Tyr Ser Glu Asp Gln Ala Leu Thr Pro Tyr Phe Lys Leu Ala Tyr
385                 390                 395                 400
Val Tyr Asp Asp Ser Asn Asn Asp Asn Asp Val Asn Gly Asp Ser Ile
                405                 410                 415
Asp Asn Gly Thr Glu Gly Ser Ala Val Arg Val Gly Leu Gly Thr Gln
            420                 425                 430
Phe Ser Phe Thr Lys Asn Phe Ser Ala Tyr Thr Asp Ala Asn Tyr Leu
        435                 440                 445
Gly Gly Gly Asp Val Asp Gln Asp Trp Ser Ala Asn Val Gly Val Lys
    450                 455                 460
Tyr Thr Trp
465

<210> 382
<211> 222
<212> PRT
<213> E. Coli

<400> 382
Met Pro Val Lys Asp Leu Thr Gly Ile Thr Ala Lys Asp Ala Gln Met
1               5                   10                  15
Leu Ser Val Val Lys Pro Leu Gln Glu Phe Gly Lys Leu Asp Lys Cys
            20                  25                  30
Leu Ser Arg Tyr Gly Thr Arg Phe Glu Phe Asn Asn Glu Lys Gln Val
        35                  40                  45
Ile Phe Ser Ser Asp Val Asn Asn Glu Asp Thr Phe Val Ile Leu Glu
    50                  55                  60
Gly Val Ile Ser Leu Arg Arg Glu Glu Asn Val Leu Ile Gly Ile Thr
65                  70                  75                  80
Gln Ala Pro Tyr Ile Met Gly Leu Ala Asp Gly Leu Met Lys Asn Asp
                85                  90                  95
Ile Pro Tyr Lys Leu Ile Ser Glu Gly Asn Cys Thr Gly Tyr His Leu
            100                 105                 110
Pro Ala Lys Gln Thr Ile Thr Leu Ile Glu Gln Asn Gln Leu Trp Arg
        115                 120                 125

Asp Ala Phe Tyr Trp Leu Ala Trp Gln Asn Arg Ile Leu Glu Leu Arg
    130                 135                 140
Asp Val Gln Leu Ile Gly His Asn Ser Tyr Glu Gln Ile Arg Ala Thr
145                 150                 155                 160
Leu Leu Ser Met Ile Asp Trp Asn Glu Glu Leu Arg Ser Arg Ile Gly
                165                 170                 175
Val Met Asn Tyr Ile His Gln Arg Thr Arg Ile Ser Arg Ser Val Val
            180                 185                 190
Ala Glu Val Leu Ala Ala Leu Arg Lys Gly Gly Tyr Ile Glu Met Asn
        195                 200                 205
Lys Gly Lys Leu Val Ala Ile Asn Arg Leu Pro Ser Glu Tyr
    210                 215                 220

<210> 383
<211> 84
<212> PRT
<213> E. Coli

<400> 383
Met Thr Asp Lys Ile Arg Thr Leu Gln Gly Arg Val Val Ser Asp Lys
1               5                   10                  15
Met Glu Lys Ser Ile Val Val Ala Ile Glu Arg Phe Val Lys His Pro
            20                  25                  30
Ile Tyr Gly Lys Phe Ile Lys Arg Thr Thr Lys Leu His Val His Asp
        35                  40                  45
Glu Asn Asn Glu Cys Gly Ile Gly Asp Val Val Glu Ile Arg Glu Cys
    50                  55                  60
Arg Pro Leu Ser Lys Thr Lys Ser Trp Thr Leu Val Arg Val Val Glu
65                  70                  75                  80
Lys Ala Val Leu

<210> 384
<211> 63
<212> PRT
<213> E. Coli

<400> 384

<210> 385
<211> 136
<212> PRT
<213> E. Coli

<400> 385

<210> 386
<211> 233
<212> PRT
<213> E. Coli

<400> 386
Met Gly Gln Lys Val His Pro Asn Gly Ile Arg Leu Gly Ile Val Lys
 1               5                  10                  15
Pro Trp Asn Ser Thr Trp Phe Ala Asn Thr Lys Glu Phe Ala Asp Asn
            20                  25                  30
Leu Asp Ser Asp Phe Lys Val Arg Gln Tyr Leu Thr Lys Glu Leu Ala
        35                  40                  45
Lys Ala Ser Val Ser Arg Ile Val Ile Glu Arg Pro Ala Lys Ser Ile
    50                  55                  60
Arg Val Thr Ile His Thr Ala Arg Pro Gly Ile Val Ile Gly Lys Lys
65                  70                  75                  80
Gly Glu Asp Val Glu Lys Leu Arg Lys Val Val Ala Asp Ile Ala Gly
                85                  90                  95
Val Pro Ala Gln Ile Asn Ile Ala Glu Val Arg Lys Pro Glu Leu Asp
            100                 105                 110
Ala Lys Leu Val Ala Asp Ser Ile Thr Ser Gln Leu Glu Arg Arg Val
        115                 120                 125
Met Phe Arg Arg Ala Met Lys Arg Ala Val Gln Asn Ala Met Arg Leu
    130                 135                 140
Gly Ala Lys Gly Ile Lys Val Glu Val Ser Gly Arg Leu Gly Gly Ala
145                 150                 155                 160
Glu Ile Ala Arg Thr Glu Trp Tyr Arg Glu Gly Arg Val Pro Leu His
                165                 170                 175
Thr Leu Arg Ala Asp Ile Asp Tyr Asn Thr Ser Glu Ala His Thr Thr
            180                 185                 190
Tyr Gly Val Ile Gly Val Lys Val Trp Ile Phe Lys Gly Glu Ile Leu
        195                 200                 205
Gly Gly Met Ala Ala Val Glu Gln Pro Glu Lys Pro Ala Ala Gln Pro
    210                 215                 220
Lys Lys Gln Gln Arg Lys Gly Arg Lys
225                 230

<210> 387
<211> 110
<212> PRT
<213> E. Coli

<400> 387

<210> 388
<211> 92
<212> PRT
<213> E. Coli

<400> 388
Met Pro Arg Ser Leu Lys Lys Gly Pro Phe Ile Asp Leu His Leu Leu
1               5                   10                  15
Met Lys Val Glu Lys Ala Val Glu Ser Gly Asp Lys Lys Pro Leu Arg
            20                  25                  30
Thr Trp Ser Arg Arg Ser Thr Ile Phe Pro Asn Met Ile Gly Leu Thr
        35                  40                  45
Ile Ala Val His Asn Gly Arg Gln His Val Pro Val Phe Val Thr Asp
    50                  55                  60
Glu Met Val Gly His Lys Leu Gly Glu Phe Ala Pro Thr Arg Thr Tyr
65                  70                  75                  80
Arg Gly His Ala Ala Asp Lys Lys Ala Lys Lys Lys
                85                  90

<210> 389
<211> 273
<212> PRT
<213> E. Coli

<400> 389

<210> 390
<211> 100
<212> PRT
<213> E. Coli

<400> 390

<210> 391
<211> 201
<212> PRT
<213> E. Coli

<400> 391

<210> 392
<211> 209
<212> PRT
<213> E. Coli

<400> 392

<210> 393
<211> 103
<212> PRT
<213> E. Coli

<400> 393
Met Gln Asn Gln Arg Ile Arg Ile Arg Leu Lys Ala Phe Asp His Arg
1               5                   10                  15
Leu Ile Asp Gln Ala Thr Ala Glu Ile Val Glu Thr Ala Lys Arg Thr
            20                  25                  30
Gly Ala Gln Val Arg Gly Pro Ile Pro Leu Pro Thr Arg Lys Glu Arg
        35                  40                  45
Phe Thr Val Leu Ile Ser Pro His Val Asn Lys Asp Ala Arg Asp Gln
    50                  55                  60
Tyr Glu Ile Arg Thr His Leu Arg Leu Val Asp Ile Val Glu Pro Thr
65                  70                  75                  80
Glu Lys Thr Val Asp Ala Leu Met Arg Leu Asp Leu Ala Ala Gly Val
                85                  90                  95
Asp Val Gln Ile Ser Leu Gly
            100

<210> 394
<211> 118
<212> PRT
<213> E. Coli

<400> 394

<210> 395
<211> 65
<212> PRT
<213> E. Coli

<400> 395

<210> 396
<211> 180
<212> PRT
<213> E. Coli

<400> 396
Met Lys Gly Gly Lys Arg Val Gln Thr Ala Arg Pro Asn Arg Ile Asn
1               5                   10                  15
Gly Glu Ile Arg Ala Gln Glu Val Arg Leu Thr Gly Leu Glu Gly Glu
            20                  25                  30
Gln Leu Gly Ile Val Ser Leu Arg Glu Ala Leu Glu Lys Ala Glu Glu
        35                  40                  45
Ala Gly Val Asp Leu Val Glu Ile Ser Pro Asn Ala Glu Pro Pro Val
    50                  55                  60
Cys Arg Ile Met Asp Tyr Gly Lys Phe Leu Tyr Glu Lys Ser Lys Ser
65                  70                  75                  80
Ser Lys Glu Gln Lys Lys Lys Gln Lys Val Ile Gln Val Lys Glu Ile
                85                  90                  95
Lys Phe Arg Pro Gly Thr Asp Glu Gly Asp Tyr Gln Val Lys Leu Arg
            100                 105                 110
Ser Leu Ile Arg Phe Leu Glu Glu Gly Asp Lys Ala Lys Ile Thr Leu
        115                 120                 125
Arg Phe Arg Gly Arg Glu Met Ala His Gln Gln Ile Gly Met Glu Val
    130                 135                 140
Leu Asn Arg Val Lys Asp Asp Leu Gln Glu Leu Ala Val Val Glu Ser
145                 150                 155                 160
Phe Pro Thr Lys Ile Glu Gly Arg Gln Met Ile Met Val Leu Ala Pro
                165                 170                 175
Lys Lys Lys Gln
            180

<210> 397
<211> 642
<212> PRT
<213> E. Coli

<400> 397
Met Pro Val Ile Thr Leu Pro Asp Gly Ser Gln Arg His Tyr Asp His
1               5                   10                  15
Ala Val Ser Pro Met Asp Val Ala Leu Asp Ile Gly Pro Gly Leu Ala
            20                  25                  30
Lys Ala Cys Ile Ala Gly Arg Val Asn Gly Glu Leu Val Asp Ala Cys
        35                  40                  45
Asp Leu Ile Glu Asn Asp Ala Gln Leu Ser Ile Ile Thr Ala Lys Asp
    50                  55                  60
Glu Glu Gly Leu Glu Ile Ile Arg His Ser Cys Ala His Leu Leu Gly
65                  70                  75                  80
His Ala Ile Lys Gln Leu Trp Pro His Thr Lys Met Ala Ile Gly Pro
                85                  90                  95
Val Ile Asp Asn Gly Phe Tyr Tyr Asp Val Asp Leu Asp Arg Thr Leu
            100                 105                 110
Thr Gln Glu Asp Val Glu Ala Leu Glu Lys Arg Met His Glu Leu Ala
        115                 120                 125
Glu Lys Asn Tyr Asp Val Ile Lys Lys Lys Val Ser Trp His Glu Ala
    130                 135                 140
Arg Glu Thr Phe Ala Asn Arg Gly Glu Ser Tyr Lys Val Ser Ile Leu
145                 150                 155                 160
Asp Glu Asn Ile Ala His Asp Asp Lys Pro Gly Leu Tyr Phe His Glu
                165                 170                 175

Glu Tyr Val Asp Met Cys Arg Gly Pro His Val Pro Asn Met Arg Phe
            180                 185                 190
Cys His His Phe Lys Leu Met Lys Thr Ala Gly Ala Tyr Trp Arg Gly
        195                 200                 205
Asp Ser Asn Asn Lys Met Leu Gln Arg Ile Tyr Gly Thr Ala Trp Ala
    210                 215                 220
Asp Lys Lys Ala Leu Asn Ala Tyr Leu Gln Arg Leu Glu Glu Ala Ala
225                 230                 235                 240
Lys Arg Asp His Arg Lys Ile Gly Lys Gln Leu Asp Leu Tyr His Met
                245                 250                 255
Gln Glu Glu Ala Pro Gly Met Val Phe Trp His Asn Asp Gly Trp Thr
            260                 265                 270
Ile Phe Arg Glu Leu Glu Val Phe Val Arg Ser Lys Leu Lys Glu Tyr
        275                 280                 285
Gln Tyr Gln Glu Val Lys Gly Pro Phe Met Met Asp Arg Val Leu Trp
    290                 295                 300
Glu Lys Thr Gly His Trp Asp Asn Tyr Lys Asp Ala Met Phe Thr Thr
305                 310                 315                 320
Ser Ser Glu Asn Arg Glu Tyr Cys Ile Lys Pro Met Asn Cys Pro Gly
                325                 330                 335
His Val Gln Ile Phe Asn Gln Gly Leu Lys Ser Tyr Arg Asp Leu Pro
            340                 345                 350
Leu Arg Met Ala Glu Phe Gly Ser Cys His Arg Asn Glu Pro Ser Gly
        355                 360                 365
Ser Leu His Gly Leu Met Arg Val Arg Gly Phe Thr Gln Asp Asp Ala
    370                 375                 380
His Ile Phe Cys Thr Glu Glu Gln Ile Arg Asp Glu Val Asn Gly Cys
385                 390                 395                 400
Ile Arg Leu Val Tyr Asp Met Tyr Ser Thr Phe Gly Phe Glu Lys Ile
                405                 410                 415
Val Val Lys Leu Ser Thr Arg Pro Glu Lys Arg Ile Gly Ser Asp Glu
            420                 425                 430
Met Trp Asp Arg Ala Glu Ala Asp Leu Ala Val Ala Leu Glu Glu Asn
        435                 440                 445
Asn Ile Pro Phe Glu Tyr Gln Leu Gly Glu Gly Ala Phe Tyr Gly Pro
    450                 455                 460
Lys Ile Glu Phe Thr Leu Tyr Asp Cys Leu Asp Arg Ala Trp Gln Cys
465                 470                 475                 480
Gly Thr Val Gln Leu Asp Phe Ser Leu Pro Ser Arg Leu Ser Ala Ser
                485                 490                 495
Tyr Val Gly Glu Asp Asn Glu Arg Lys Val Pro Val Met Ile His Arg
            500                 505                 510
Ala Ile Leu Gly Ser Met Glu Arg Phe Ile Gly Ile Leu Thr Glu Glu
        515                 520                 525
Phe Ala Gly Phe Phe Pro Thr Trp Leu Ala Pro Val Gln Val Val Ile
    530                 535                 540
Met Asn Ile Thr Asp Ser Gln Ser Glu Tyr Val Asn Glu Leu Thr Gln
545                 550                 555                 560
Lys Leu Ser Asn Ala Gly Ile Arg Val Lys Ala Asp Leu Arg Asn Glu
                565                 570                 575
Lys Ile Gly Phe Lys Ile Arg Glu His Thr Leu Arg Arg Val Pro Tyr
            580                 585                 590
Met Leu Val Cys Gly Asp Lys Glu Val Glu Ser Gly Lys Val Ala Val
        595                 600                 605
Arg Thr Arg Arg Gly Lys Asp Leu Gly Ser Met Asp Val Asn Glu Val
    610                 615                 620
Ile Glu Lys Leu Gln Gln Glu Ile Arg Ser Arg Ser Leu Lys Gln Leu
625                 630                 635                 640
Glu Glu

<210> 398
<211> 450
<212> PRT
<213> E. Coli

<400> 398
Met Thr Lys His Tyr Asp Tyr Ile Ala Ile Gly Gly Gly Ser Gly Gly
1               5                   10                  15
Ile Ala Ser Ile Asn Arg Ala Ala Met Tyr Gly Gln Lys Cys Ala Leu
            20                  25                  30
Ile Glu Ala Lys Glu Leu Gly Gly Thr Cys Val Asn Val Gly Cys Val
        35                  40                  45
Pro Lys Lys Val Met Trp His Ala Ala Gln Ile Arg Glu Ala Ile His
    50                  55                  60
Met Tyr Gly Pro Asp Tyr Gly Phe Asp Thr Thr Ile Asn Lys Phe Asn
65                  70                  75                  80
Trp Glu Thr Leu Ile Ala Ser Arg Thr Ala Tyr Ile Asp Arg Ile His
                85                  90                  95
Thr Ser Tyr Glu Asn Val Leu Gly Lys Asn Asn Val Asp Val Ile Lys
            100                 105                 110
Gly Phe Ala Arg Phe Val Asp Ala Lys Thr Leu Glu Val Asn Gly Glu
        115                 120                 125
Thr Ile Thr Ala Asp His Ile Leu Ile Ala Thr Gly Gly Arg Pro Ser
    130                 135                 140
His Pro Asp Ile Pro Gly Val Glu Tyr Gly Ile Asp Ser Asp Gly Phe
145                 150                 155                 160
Phe Ala Leu Pro Ala Leu Pro Glu Arg Val Ala Val Val Gly Ala Gly
                165                 170                 175
Tyr Ile Ala Val Glu Leu Ala Gly Val Ile Asn Gly Leu Gly Ala Lys
            180                 185                 190
Thr His Leu Phe Val Arg Lys His Ala Pro Leu Arg Ser Phe Asp Pro
        195                 200                 205
Met Ile Ser Glu Thr Leu Val Glu Val Met Asn Ala Glu Gly Pro Gln
    210                 215                 220
Leu His Thr Asn Ala Ile Pro Lys Ala Val Val Lys Asn Thr Asp Gly
225                 230                 235                 240
Ser Leu Thr Leu Glu Leu Glu Asp Gly Arg Ser Glu Thr Val Asp Cys
                245                 250                 255
Leu Ile Trp Ala Ile Gly Arg Glu Pro Ala Asn Asp Asn Ile Asn Leu
            260                 265                 270
Glu Ala Ala Gly Val Lys Thr Asn Glu Lys Gly Tyr Ile Val Val Asp
        275                 280                 285
Lys Tyr Gln Asn Thr Asn Ile Glu Gly Ile Tyr Ala Val Gly Asp Asn
    290                 295                 300
Thr Gly Ala Val Glu Leu Thr Pro Val Ala Val Ala Ala Gly Arg Arg
305                 310                 315                 320
Leu Ser Glu Arg Leu Phe Asn Asn Lys Pro Asp Glu His Leu Asp Tyr
                325                 330                 335
Ser Asn Ile Pro Thr Val Val Phe Ser His Pro Pro Ile Gly Thr Val
            340                 345                 350
Gly Leu Thr Glu Pro Gln Ala Arg Glu Gln Tyr Gly Asp Asp Gln Val
        355                 360                 365
Lys Val Tyr Lys Ser Ser Phe Thr Ala Met Tyr Thr Ala Val Thr Thr
    370                 375                 380
His Arg Gln Pro Cys Arg Met Lys Leu Val Cys Val Gly Ser Glu Glu
385                 390                 395                 400
Lys Ile Val Gly Ile His Gly Ile Gly Phe Gly Met Asp Glu Met Leu
                405                 410                 415
Gln Gly Phe Ala Val Ala Leu Lys Met Gly Ala Thr Lys Lys Asp Phe
            420                 425                 430

Asp Asn Thr Val Ala Ile His Pro Thr Ala Ala Glu Glu Phe Val Thr
        435                 440                 445
Met Arg
    450

<210> 399
<211> 2894
<212> RNA
<213> E. Coli

<400> 399
aagguuaagc cucacgguuc auuaguaccg guuagcucaa cgcaucgcug cgcuuacaca 60
cccggccuau caacgucguc gucuucaacg uuccuucagg acccuuaaag ggucagggag 120
aacucaucuc ggggcaaguu ucgugcuuag augcuuucag cacuuaucuc uuccgcauuu 180
agcuaccggg cagugccauu ggcaugacaa cccgaacacc agugaugcgu ccacuccggu 240
ccucucguac uaggagcagc cccccucagu ucuccagcgc ccacggcaga uagggaccga 300
acugucucac gacguucuaa acccagcucg cguaccacuu uaaauggcga acagccauac 360
ccuugggacc uacuucagcc ccaggaugug augagccgac aucgaggugc caaacaccgc 420
cgucgauaug aacucuuggg cgguaucagc cuguuauccc cggaguaccu uuuauccguu 480
gagcgauggc ccuuccauuc agaaccaccg gaucacuaug accugcuuuc gcaccugcuc 540
gcgccgucac gcucgcaguc aagcuggcuu augccauugc acuaaccucc ugauguccga 600
ccaggauuag ccaaccuucg ugcuccuccg uuacucuuua ggaggagacc gccccaguca 660
aacuacccac cagacacugu ccgcaacccg gauuacgggu caacguuaga acaucaaaca 720
uuaaagggug guauuucaag gucggcucca ugcagacugg cguccacacu ucaaagccuc 780
ccaccuaucc uacacaucaa ggcucaaugu ucagugucaa gcuauaguaa agguucacgg 840
ggucuuuccg ucuugccgcg gguacacugc aucuucacag cgaguucaau uucacugagu 900
cucgggugga gacagccugg ccaucauuac gccauucgug caggucggaa cuuacccgac 960
aaggaauuuc gcuaccuuag gaccguuaua guuacggccg ccguuuaccg gggcuucgau 1020
caagagcuuc gcuugcgcua accccaucaa uuaaccuucc ggcaccgggc aggcgucaca 1080
ccguauacgu ccacuuucgu guuugcacag ugcuguguuu uuaauaaaca guugcagcca 1140
gcugguaucu ucgacugauu ucagcuccau ccgcgaggga ccucaccuac auaucagcgu 1200
gccuucuccc gaaguuacgg caccauuuug ccuaguuccu ucacccgagu ucucucaagc 1260
gccuugguau ucucuaccug accaccugug ucgguuuggg guacgauuug auguuaccug 1320
augcuuagag gcuuuuccug gaagcagggc auuuguugcu ucagcaccgu agugccucgu 1380
caucacgccu cagccuugau uuuccggauu ugccuggaaa accagccuac acgcuuaaac 1440
cgggacaacc gucgcccggc caacauagcc uucuccgucc ccccuucgca guaacaccaa 1500
guacaggaau auuaaccugu uucccaucga cuacgccuuu cggccucgcc uuaggggucg 1560
acucacccug ccccgauuaa cguuggacag gaacccuugg ucuuccggcg agcgggcuuu 1620
ucacccgcuu uaucguuacu uaugucagca uucgcacuuc ugauaccucc agcaugccuc 1680
acagcacacc uucgcaggcu uacagaacgc uccccuaccc aacaacgcau aagcgucgcu 1740
gccgcagcuu cggugcaugg uuuagccccg uuacaucuuc cgcgcaggcc gacucgacca 1800
gugagcuauu acgcuuucuu uaaaugaugg cugcuucuaa gccaacaucc uggcugucug 1860
ggccuuccca caucguuucc cacuuaacca ugacuuuggg accuuagcug gcggucuggg 1920
uuguuucccu cuucacgacg gacguuagca cccgccgugu gucucccgug auaacauucu 1980
ccgguauucg caguuugcau cggguuggua agucgggaug acccccuugc cgaaacagug 2040
cucuaccccc ggagaugaau ucacgaggcg cuaccuaaau agcuuucggg gagaaccagc 2100
uaucucccgg uuugauuggc cuuucacccc cagccacaag ucauccgcua auuuuucaac 2160
auuagucggu ucgguccucc aguuaguguu acccaaccuu caaccugccc auggcuagau 2220
caccggguuu cgggucuaua cccugcaacu uaacgcccag uuaagacucg guuucccuuc 2280
ggcuccccua uucgguuaac cuugcuacag aauauaaguc gcugacccau uauacaaaag 2340
guacgcaguc acacgccuaa gcgugcuccc acugcuugua cguacacggu uucagguucu 2400
uuuucacucc ccucgccggg guucuuuucg ccuuucccuc acgguacugg uucacuaucg 2460
gucagucagg aguauuuagc cuuggaggau ggucccccca uauucagaca ggauaccacg 2520
ugucccgccc uacucaucga gcucacagca ugugcauuuu uguguacggg gcugucaccc 2580
uguaucgcgc gccuuuccag acgcuuccac uaacacacac acugauucag gcucugggcu 2640
gcuccccguu cgcucgccgc uacuggggga aucucgguug auuucuuuuc cucgggguac 2700
uuagauguuu caguuccccc gguucgccuc auuaaccuau ggauucaguu aaugauagug 2760
ugucgaaaca cacuggguuu ccccauucgg aaaucgccgg uuauaacggu ucauaucacc 2820
uuaccgacgc uuaucgcaga uuagcacguc cuucaucgcc ucugacugcc agggcaucca 2880

ccguguacgc uuagucgcuu aacc 2894

<210> 400
<211> 120
<212> RNA
<213> E. Coli

<400> 400
augccuggca guucccuacu cucgcauggg gagaccccac acuaccaucg gcgcuacggc 60
guuucacuuc ugaguucggc auggggucag gugggaccac cgcgcuacgg ccgccaggca 120

<210> 401
<211> 76
<212> RNA
<213> E. Coli

<400> 401
guccccuucg ucuagaggcc caggacaccg cccuuucacg gcgguaacag ggguucgaau 60
ccccuagggg acgcca 76

<210> 402
<211> 1549
<212> RNA
<213> E. Coli

<400> 402
aaauugaaga guuugaucau ggcucagauu gaacgcuggc ggcaggccua acacaugcaa 60
gucgaacggu aacaggaagc agcuugcugc uucgcugacg aguggcggac gggugaguaa 120
ugucugggaa gcugccugau ggagggggau aacuacugga aacgguagcu aauaccgcau 180
aaugucgcaa gaccaaagag ggggaccuuc gggccucuug ccaucggaug ugcccagaug 240
ggauuagcuu guuggugggg uaacggcuca ccaaggcgac gaucccuagc uggucugaga 300
ggaugaccag ccacacugga acugagacac gguccagacu ccuacgggag gcagcagugg 360
ggaauauugc acaaugggcg caagccugau gcagccaugc cgcguguaug aagaaggccu 420
ucggguugua aaguacuuuc agcggggagg aagggaguaa aguuaauacc uuugcucauu 480
gacguuaccc gcagaagaag caccggcuaa cuccgugcca gcagccgcgg uaauacggag 540
ggugcaagcg uuaaucggaa uuacugggcg uaaagcgcac gcaggcgggu ugguuaaguc 600
agaugugaaa uccccgggcu caaccuggga acugcaucug auacuggcaa gcuugagucu 660
cguagagggg gguagaauuc cagguguagc ggugaaaugc guagagaucu ggaggaauac 720
cgguggcgaa ggcggccccc uggacgaaga cugacgcuca ggugcgaaag cguggggagc 780
aaacaggauu agauacccug guaguccacg ccguaaacga ugucgacuug gagguugugc 840
ccuugaggcg uggcuuccgg agcuaacgcg uuaagucgac cgccugggga guacggccgc 900
aagguuaaaa cucaaaugaa uugacggggg cccgcacaag cgguggagca ugugguuuaa 960
uucgaugcaa cgcgaagaac cuuaccuggu cuugacaucc acggaaguuu ucagagauga 1020
gaaugugccu ucgggaaccg ugagacaggu gcugcauggc ugucgucagc ucguguugug 1080
aaauguuggg uuaagucccg caacgagcgc aacccuuauc cuuuguugcc agcgguccgg 1140
ccgggaacuc aaaggagacu gccagugaua aacuggagga agguggggau gacgucaagu 1200
caucauggcc cuuacgacca gggcuacaca cgugcuacaa uggcgcauac aaagagaagc 1260
gaccucgcga gagcaagcgg accucauaaa gugcgucgua guccggauug gagucugcaa 1320
cucgacucca ugaagucgga aucgcuagua aucguggauc agaaugccac ggugaauacg 1380
uucccgggcc uuguacacac cgcccgucac accaugggag uggguugcaa aagaaguagg 1440
uagcuuaacc uucgggaggg cgcuuaccac uuugugauuc augacugggg ugaagucgua 1500
acaagguaac cguaggggaa ccugcgguug gaucaccucc uuaccuuaa 1549

<210> 403
<211> 17
<212> DNA
<213> Artificial
<220>
<223> Primer Oligonucleotide

<400> 403
tgtttatcag accgctt                                                    17

<210> 404
<211> 18
<212> DNA
<213> Artificial
<220>
<223> Primer Oligonucleotide

<400> 404
acaatttcac acagcctc                                                   18

<210> 405
<211> 159
<212> DNA
<213> Escherichia coli

<400> 405
caggtggtat ggaaacccaa aatggagacg ggaagctgaa ccagatagtt actggaggtg      60
atcaccagca gatgaaataa cgataaccag aacaacgcct tatagcgttg agtttgcgag     120
aaaacgttca tattgtacct ttttgattaa ccattgggg                            159

<210> 406
<211> 640
<212> DNA
<213> Escherichia coli

<220>
<221> misc_feature
<222> (1)...(640)
<223> n = A,T,C or G

<400> 406
ggggnccaaa gtgtttgggn cgggcaactg gaggccaacc ttaanttngg ggaaattttt      60
aanaaaaggc ggggatttgt nagccacggg ngattanttt anaataaatt aagtgttgcc     120
ataaggggac aaagngaagg aagtggntat taanggannc gccaatgcga nttagggcag     180
accattcggc cattcgcctt cttggttatc gaagttcatc cagatagccg ttgccngacc     240
gaccagattc gcttcnggca caaagcccca gtaacggctg tccgcgctgt tgtcgcggtt     300
gtcgcccatc atgaagtatt gtcccggagg aacaatccag gttgccagtt gttgccctgg     360
ctgctggtaa tacatcccca cctgatcctg cgcaatcggc actgtcagaa tgcggtgcgt     420
cacatcaccc agtgtctctt tacgctcgga aagacgaatt ccattttctt tggtttcgtt     480
tttcggcact tcaaagaatc cgctggtcgc ttccccacca ttacggcgtg agaaggtctg     540
aacgaaatcg ctcggttcca cgtttgagta ggtgaccggc agcgcgtttt cacacgcctg     600
gccggaactg catcccggtt gaatcgtcag ctcttttgag                           640

<210> 407
<211> 682
<212> DNA
<213> Escherichia coli

<220>
<221> misc_feature

<222> (1)...(682)
<223> n = A,T,C or G

<400> 407
cctgcagggt aatgtcgcca ttaaactggc gcaggcagcc aaagagttgc tccgcttcta 60
cccagtcggc agcgacaact tgcgttaaag tcgcaaaatt atcatctgca ctcactgcgt 120
gacgtaagcg gatggagtgg ccggaaacct catagtgacc gcccaccagt tggcctgcat 180
cgctttgtag cgtacgcgcg gcattggcaa taagattcag atactcagac tcttccgggg 240
ccttcgccag cataaaagag gaggatgctc gcgtatgcag caactgctcc agcgcaaatt 300
gcagccgcgg ttgagtatca ctgaataaag gatcgttttc gtcaatcaaa tgtggctgag 360
caaatatttc ctgatagcta tcggtatcag gaaccaggtc acgccatgca agtttcgtaa 420
tggtcaaagt tgatgttttt tagtctgttg tcaaagccgc nattataccn gtaaccggca 480
ctacagcaca cgtagaaagc acccgacaat actcctggca tgggcgttaa agctcacagg 540
atggagatct tttcttcact ggcctaaaaa gctgatattc tgtaaagagt tacacngtaa 600
cattgagatc gctatgaaat atcaacaact tggaaaatct tgnaaagcng gttggaaaat 660
ggaaagtatc tggttaagaa gc 682

<210> 408
<211> 309
<212> DNA
<213> Escherichia coli

<400> 408
ggggatccgg cagaatttta cgctgaccaa tgacgcgacg acgtggcatg gaaatactcc 60
gttgttaatt caggattgtc caaaactcta cgagtttagt ttgacattta agttaaaacg 120
tttggcctta cttaacggag aaccattaag ccttaggacg cttcacgcca tacttggaac 180
gagcctgctt acggtcttta acgccggagc agtcaagcgc accacgtacg gtgtggtaac 240
gaacacccgg gaggtcttta acacgaccgc cacggatcag gatcacggag tgctcctgca 300
gccaagctt 309

<210> 409
<211> 1167
<212> DNA
<213> Escherichia coli

<400> 409
gtcgacccat ctgtccattg agcggacagt ttgtgcaaca ctattttgtt gaccggaaaa 60
tggaacactt tccgcaatgc ctgttgctat cacgcttaaa ccatttcatt gcgatttaca 120
cagaacggac gtcctgtcgc agtatattaa gtcgtcgata gaaacaagca ttgaaaggca 180
cagcagtagt caaacagtgt gaaacgctac tggcgcctta cagcgcaaaa aggctggtga 240
ctaaaaagtc accagccatc agcctgattt ctcaggctgc aaccggaagg gttggcttat 300
ttaacttcaa cttcagcgcc agcttcttcc agagcttttt tcagtgcttc tgcgtcgtct 360
ttgctcacgc cttctttcag agcagccggt gcagattcta ccaggtcttt agcttctttc 420
agacccaggc cagttgcgcc acgtactgct ttgataacag caactttgtt agcgccagca 480
gctttcagaa ttacgtcgaa ttcagttttt tcttcagcag cttcaaccgg gccagcagct 540
acagctacag cagcagcagc ggaaacaccg aatttttctt ycattgcaga gatcaagttc 600
tacaacgtcc attacagaca tagctgcaac tgcttcaatg awttgatctt tagtgataga 660
catttaaatk gttcctgaat atcagaataa gtttatacgt aagcgaatgc gttaaaaaga 720
taactgcgaw taagcagctt ytttcgcatc gcgtacagma gccagagtac gaaccagttt 780
gccagccgaa gcttctttca tggttgccat caggcgtgca attgcttctt cgtaggtcgg 840
cagagttgcc aggcggtcga tctgagacgc cgggatcagc tcaccttcaa aggcagcggc 900
tttgacctca aattttgcat tcgctttcgc gaactctttg aacagacgag cagcagcgcc 960
cgggtgttcc atagagtatg caatcagggt cggaccaaca aacgcgtctt tcaggcactc 1020
gaacggagta ccttcaacag cacggcgcag cagggtgtta cgaacaacac gcatgtatac 1080
gccagcttcg cgacctgctt tacgcagttc agtcatttta tctacagtta cgcccacggg 1140
aatccgcaac tactgcaagc caagctt 1167

<210> 410
<211> 404
<212> DNA
<213> Escherichia coli

<400> 410
caacmctatt ttgktggacc ggaaaakgga acactttccg cawkgcctgt tgctatcacg      60
cttaaaccat ttcattgcga tttacacaga acggacgtcc tgtcgcagta tattaagtcg     120
tcgatagaaa caagcattga aaggcacagc agtagtcaaa cagtgtgaaa cgctactggc     180
gccttacagc gcaaaaaggc tggtgactaa aaagtcacca gccatcagcc tgatttctca     240
ggctgcaacc ggaagggttg gcttatttaa cttcaacttc agcgccagct tcttccagag     300
cttttttcag tgcttctgcg tcgtctttgc tcacgccttc tttcagagca gccggtgcag     360
attctaccag gtctttagct tctttcagac ccaggccagt tgcg                      404

<210> 411
<211> 152
<212> DNA
<213> Escherichia coli

<400> 411
agagcttttt tcagtgcttc tgcgtcgtct ttgctcacgc cttctttcaa gagcagcccg      60
gtgcagattc taccaggtct ttagcttctt tcagacccag gccagttgcg ccacgtactg     120
ctttgataac agcaactttg ttagcgccag ca                                   152

<210> 412
<211> 825
<212> DNA
<213> Escherichia coli

<220>
<221> misc_feature
<222> (1)...(825)
<223> n = A,T,C or G

<400> 412
gatccgtcga cccatctgtc cattgagcgg acagtttgtg caacactatt ttgttgaccg      60
gaaaatggaa cactttccgc aatgcctgtt gctatcacgc ttaamccatt tcattgcgat     120
ttacacagaa cggacgtcct gtcgcagtat attaagtcgt cgatagaaac aagcattgaa     180
aggcacagca gtagtcaaac agtgtgaaac gctactggcg ccttacagcg caaaaaggct     240
ggtgactaaa aagtcaccag ccatcagcct gatttctcag gctgcaaccg gaagggttgg     300
cttatttaac ttcaacttca gcgccagctt cttccagagc ttttttcagt gcttctgcgt     360
cgtctttgct cacgccttct ttcagagcag ccgggtgcag attctaccag gtctttagct     420
tctttcagac ccaggccagt tgcgccacgt actgctttga taacagcaac tttgttagcg     480
ccagcagctt tcagaattac gtcgaattca agttttttct tcagcagctt caaccgggcc     540
agcagctaca gctacagcag cagcagcgga aacaccgaat ttttcttyca ttggcagaga      600
tcaagttcta caacgtccat tacagacata gctgcaactg cttcaatgat tkgatcttwa     660
gtgatagaca tttaaattgt tcctgaatat cagaataagt ttatacgtaa gcgaatgcgt     720
taaaaagata actgcgatta agcagcttct ttcgcatcgc gtacagcagc cagaggtcga     780
accagtttgc cagccgaagg ttggcttttc agcctnnncn natta                     825

<210> 413
<211> 425
<212> DNA
<213> Escherichia coli

<400> 413
agtagtcaaa caggtgkgra acgctactgg cgccttacag cgcaaaaagg ctggtgacta      60
aaaagtcacc agccatcarc ctgatttctc aggctgcaac ccggaagggt tggcttattt     120
aacttcaact tcagcgccag cttcttccag agcttttttc agtgcttctg cgtcgtcttt     180
gctcacgcct tctttcagag cagccggtgc agattctacc aggtctttag cttctttcag     240
acccaggcca gttgcgccac gtactgcttt gataacagca actttgttag cgccagcagc     300
tttcagaatt acgtcgaatt cagttttttc ttcagcagct tcaaccgggc cagcagctac     360
agctacagca gcagcagcgg aaacacccga atttttcttc cattgcagag atcaagttct     420
acaac                                                                 425

<210> 414
<211> 126
<212> DNA
<213> Escherichia coli

<400> 414
agagcttttt tcagtgcttc tgcgtcgtct ttgctcacgc cttctttcag agcagccggt 60
gcagattcta ccaggtcttt agcttctttc agacccaggc cagttgcgcc acgtactgct 120
ttrata 126

<210> 415
<211> 264
<212> DNA
<213> Escherichia coli

<400> 415
ctgcmacccg gargggttgg cttatttaac ttcaacttca gcgccagctt cttycagagc 60
ttttttcaag tgcttctgcg tcgtctttgc tcacgccttc tttcagagca gccggtgcag 120
attctaccag gtctttagct tctttcagac ccaggccagt tgcgccacgt actgctttga 180
taacagcaac tttgttagcg ccagcagctt tcagaattac gtcgaattca gttttttctt 240
cagcagcttc aaccgggcca gcag 264

<210> 416
<211> 201
<212> DNA
<213> Escherichia coli

<400> 416
cgcataccct gcagcatcgg cccgatggag atcaggtcgg cagaacgctg taccgctttg 60
taggtggtgt taccggtgtt cagatccggg aagatgaaca cggtagcgcg acctgcaacc 120
ggagagttcg gcgctttgga tttcgcaacg tcagccatta ccgcagcgtc gtactgcagc 180
ggaccgtcga tcatcaggtc a 201

<210> 417
<211> 239
<212> DNA
<213> Escherichia coli

<400> 417
aattcagcag ttgacagtgg cataaacgta actggtgact tttgcccggc atgacgccgg 60
gcttttttta ttattccgtg acttccagcg tagtgaaggc aaacttctcg ccatcaaata 120
gcccctgact ggttagtttt agcgcgggga tcactggcag agaaagaaac gccatctgaa 180
taaacggctc atcgggtaac ggaccgcatt cacgggcggc ggctttcaag gcgtcaatt 239

<210> 418
<211> 223
<212> DNA
<213> Escherichia coli

<400> 418
ttcttttttt cgtcaacggt gtccagaatc attttattta cctcgggtac ttatgctgat 60
ttttattatt atggggaagg tgttatttat gagtttcatt tatgccgtaa cgacaatgaa 120
ctcgggaatt agtataagca gcgcgagaat aataatcatt gtgcaaatgc taatttaatt 180
aatactattt aaatattatt ttgagcatat gcacataagg ttg 223

<210> 419
<211> 223
<212> DNA
<213> Escherichia coli

<400> 419

ttcttttttt cgtcaacggt gtccagaatc attttattta cctcgggtac ttatgctgat 60
ttttattatt atggggaagg tgttatttat gagtttcatt tatgccgtaa cgacaatgaa 120
ctcgggaatt agtataagca gcgcgagaat aataatcatt gtgcaaatgc taatttaatt 180
aatactattt aaatattatt ttgagcatat gcacataagg ttg 223

<210> 420
<211> 212
<212> DNA
<213> Escherichia coli

<400> 420
aatagcgggt atgcacgcct ttcttttttt cgtcaacggt gtccagaatc attttattta 60
cctcgggtac ttatgctgat ttttattatt atggggaagg tgttatttat gagtttcatt 120
tatgccgtaa cgmcaatgaa ctcgggaatt agtataagca gcgcgagaat aataatcatt 180
gtgcaaatgc taatttaatt aatactattt aa 212

<210> 421
<211> 438
<212> DNA
<213> Escherichia coli

<400> 421
ccctgtaaat tatcgcccgt ggcataaaaa ctgcgtccaa acgccgtctt tgccagcagc 60
caggccataa atgccaccag aattatcgtc aaccaaccaa ttgctgaaac gccaagcagc 120
agcggggcgg agagctgttt cagttcggcg ggtaaccctt caatccattt gccgccagtc 180
cacagcaaca tgatgcctct gtacaaccct aacgtgccaa gggtggcaac aatggcaggg 240
atctttagcc acgcgaccag gacaccgttg aaaaatcccg cgagcaaacc aagcagtaaa 300
gtcgcgacac aagcaacagg tagtgaatat cctgcgttca gtaacatccc caacagcacc 360
gcgcacattc cgggtaatcg aaccccactt gaaacatcaa tattgsgsgt aagcattwcc 420
aagcgttcgs gcccatkg 438

<210> 422
<211> 682
<212> DNA
<213> Escherichia coli

<400> 422
aattcccggg gatccgtcga ccgtgcgctt ccggttgtgg caacccgcga aatggcgcgg 60
cggtaagtat ggcggggtta ttccttcccc gttgaggaca ccgggttgtc aggttgacca 120
tacgcttaag tgacaacccc gctgcaacgc cctctgttat caattttctg gtgacgtttg 180
gcggtatcag ttttactccg tgactgctct gccgcccttt ttaaagtgaa ttttgtgatg 240
tggtgaatgc ggctgagcgc acgcggaaca gttaaaacca aaaacagtgt tatgggtgga 300
ttctctgtat ccggcgttaa ttgttaactg gttaacgtca cctggaggca ccaggcactg 360
catcacaaaa ttcattgttg aggacgcgat aatgaaaacg ttattaccaa acgttaatac 420
gtctgaaggt tgttttgaaa ttggtgtcac tatcagtaac ccagtattta ctgaagatgc 480
cattaacaag agaaaacaag aacgggagct attaaataaa atatgcattg tttcaatgct 540
ggctcgttta cgtctgatgc caaaaggatg tgcacaatga attcagcatt tgtgcttgtt 600
ctgacagttt ttcttgtttc cggagagcca gttgatattg cagtcagtgt tcacaggaca 660
atgcaggagt gatgactgca gc 682

<210> 423
<211> 600
<212> DNA
<213> Escherichia coli

<400> 423
ggggatccga ttgtgactgc tctgccgccc tttttaaagt gaattttgtg atgtggtgaa 60
tgcggctgag cgcacgcgga acagttaaaa ccaaaaacag tgttatgggt ggattctctg 120
tatccggcgt taattgttaa ctggttaacg tcacctggag gcaccaggca ctgcatcaca 180
aaattcattg ttgaggacgc gataatgaaa acgttattac caaacgttaa tacgtctgaa 240
ggttgttttg aaattggtgt cactatcagt aacccagtat ttactgaaga tgccattaac 300

aagagaaaac aagaacggga gctattaaat aaaatatgca ttgtttcaat gctggctcgt 360
ttacgtctga tgccaaaagg atgtgcacaa tgaattcagc atttgtgctt gttctgacag 420
tttttcttgt ttccggagag ccagttgata ttgcagtcag tgttcacagg acaatgcagg 480
agtgtatgac tgcagcaacc gaacagaaaa ttcccggtaa ctgttacccg gtcgataaag 540
ttattcacca ggataatatc gaaatcccgg caggtcttta aacagttccg taataaataa 600

<210> 424
<211> 100
<212> DNA
<213> Escherichia coli

<400> 424
gggatccagc aagaagatgc ggttgtaccg tcatcacgca gatgcgcaaa gctactcagc 60
aactgacctt tcttcgcaat aagcacgcca ttagcgtcat 100

<210> 425
<211> 465
<212> DNA
<213> Escherichia coli

<400> 425
tcgcgtgttt accttcaaca tcggtaactt tctggcggat agtttcacgg taagcaacct 60
gcggtttacc tacgttcgct tcaacgttga attcacgctt catacggtca acgatgatgt 120
cgaggtgcag ttcgcccata cccgcgatga tggtctggtt agattcttcg tcagtccata 180
cacggaaaga cgggtcttct ttagccagac ggcccagagc cagacccatt ttttcctggt 240
cagctttggt tttcggttca actgcgatgg agattaccgg ctcagggaat tccatacgtt 300
ccagaatgat cggcgcatcc gggtcacaca gggtgtcacc agtggttacg tctttcagac 360
cgatagcagc agcgatgtcg cccgcgcgaa cttctttgat ctcttcacgt ttgttagcgt 420
gcatctgaac gatacgaccg aaacgctcac gtgcagcttt cacgg 465

<210> 426
<211> 653
<212> DNA
<213> Escherichia coli

<220>
<221> misc_feature
<222> (1)...(653)
<223> n = A,T,C or G

<400> 426
tgatcggctc aagcagaact ggtttcgctt tcttaaagcc ttctttaaag gcgatagaag 60
cagccagttt aaacgccagt tcagaggagt caacgtcatg gtaagaaccg aagtgcagac 120
gaatacccat gtctactacc gggtagcctg ccagcggacc tgctttcagc tgttcctgga 180
tacctttatc aacggccggg atgtattcgc cagggattac accaccttta atgtcgttga 240
tgaactcgta gcctttcggg tttgaacccg gctccagcgg gtacatgtcg ataacaacat 300
gaccatactg accacgacca ccagactgtt tcgcgtgttt accttcaaca tcggtaactt 360
tctggcggat agtttcacgg taagcaacct gcggtttacc tacgttcgct tcaacgttga 420
attcacgctt catacggtca acgatgatgt cgaggtgcag ttcgccatac ccgcgatgat 480
ggctgggtag attcttcgtc agtccataca cggnaagacg ggtcttnttt agccagacgg 540
gccagagnca gacccatttt tttctggcag ctttggnttc ggtcaactgc gatggaaata 600
cccggctcaa ggaattcata cgtttcanaa tgatcggggc attccgggtc aca 653

<210> 427
<211> 268
<212> DNA
<213> Escherichia coli

<400> 427
ctttcttaaa gccttcttta aaggcgatag aagcagccag tttaaacgcc agttcagagg 60
agtcaacgtc atggtaagaa ccgaagtgca gacgaatacc catgtctact accgggtagc 120

ctgccagcgg acctgctttc agctgttcct ggataccttt atcaacggcc gggatgtatt 180
cgccagggat tacaccacct ttaatgtcgt tgatgaactc gtagcctttc gggtttgaac 240
ccggctccag cgggtacatg tcgataac 268

<210> 428
<211> 330
<212> DNA
<213> Escherichia coli

<400> 428
gttttgggga gatgtaaggg ctaatctgaa tggctgcatt ccttgtttaa ggaaaaacga 60
atgactgatt gccgatacct gattaaacgg gtcatcaaaa tcatcattgc tgttttacag 120
ctgatccttc tgttcttata acacaaggaa acgtacttaa ggtgcgtccg gtgaaccagt 180
cggacgcacc tttaataact ataaataagt gtctgggcag atactatata aattaactta 240
gtgaatgatt atgctaatgt catcaattaa ataaatataa tggcgttaag gcttcccagt 300
aatataatta atactctact tccagagtag 330

<210> 429
<211> 465
<212> DNA
<213> Escherichia coli

<400> 429
gttttgggga gatgtaaggg ctaatctgaa tggctgcatt ccttgtttaa ggaaaaacga 60
atgactgatt gccgatacct gattaaacgg gtcatcaaaa tcatcattgc tgttttacag 120
ctgatccttc tgttcttata acacaaggaa acgtacttaa ggtgcgtccg gtgaaccagt 180
cggacgcacc tttaataact ataaataagt gtctgggcag atactatata aattaactta 240
gtgaatgatt atgctaatgt catcaattaa ataaatataa tggcgttaag gcttcccagt 300
aatataatta atactctact tccagagtag aatattaaat tttatccgcg tggtgcatca 360
gcacaaattt atcccacaac tgttcttctg tctcgacatg cgccggatct ttcacaatag 420
tattggggat cgggcacacc ttctggcagg ttggtgtctc gtagt 465

<210> 430
<211> 379
<212> DNA
<213> Escherichia coli

<400> 430
aatctgaatg gctgcattcc ttgtttaagg aaaaacgaat gactgattgc cgatacctga 60
ttaaacgggt catcaaaatc atcattgctg ttttacagct gatccttctg ttcttataac 120
acaaggaaac gtacttaagg tgcgtccggt gaaccagtcg gacgcacctt taataactat 180
aaataagtgt ctgggcagat actatataaa ttaacttagt gaatgattat gctaatgtca 240
tcaattaaat aaatataatg gcgttaaggc ttcccagtaa tataattaat actctacttc 300
cagagtagaa tattaaattt tatccgcgtg gtgcatcagc acaaatttat cccacaactg 360
ttcttctgtc tcgacatgc 379

<210> 431
<211> 443
<212> DNA
<213> Escherichia coli

<400> 431
aagatgatgt gatgagaaag tcaatttgaa taagacaata ttaagagcta aaaaaatgtc 60
aaaaaacact aaatcaaaaa ataatggcat tagaaaatat aatgcgaaaa cggaggtgaa 120
attagtttat ttcaaatgag gaaaatctcc cggcgaaaaa accgggagat gaaagtgtga 180
tgggtatcaa ataaacaaca gaggagaaat ttttaacgca gccattcagg caaatcgttt 240
aatcccattg cctggcggat aagttgcggc ttaacgccag gaagcgtgtc ggccagtttc 300
aaaccaatat cacgcagcag ttttttcgcc ggattggtac cggaaaacag atcgcggaat 360
ccctgcatac cagccagcat caacgccgca ctgtgcttgc ggctacgctc atagcgacgc 420
agataaatgt actgcccgat gtc 443

<210> 432
<211> 638
<212> DNA
<213> Escherichia coli

<400> 432
cagggggttt gttgtgggca atgatgcatt taagttatcg tctgcagata gaggagatat 60
tacaataaac aacgaatcag ggcatttgat agtcaatacc gcaattctat caggagatat 120
agtcactcta agaggaggag aaattaggtt ggtattatag cttgtgcgcg ccatgattgg 180
cgcgcaattt aaacttagtg ctttacatcg ctattgtctt gatttctttg aattatttta 240
taaattaaaa aaacgactgt tatgtataag caaaggtccg aacgaaaaat acattccaaa 300
taaatgcttg cttaaatctc tatatccttc cccgaaaaat gacacataaa attgagatat 360
tccaaaaaga gatactacaa ataaagatgc ctttatttta ttatttctaa taaaaataga 420
agcaataaaa aataataaca atgatataaa tctaatgttt ttaaatatat tgtcttttat 480
gttagtaata gtcgttagta tgtttgattc tccatatatt acgtgtagtt ttttatatac 540
atggaaataa ttttctttat actgagacat cacaccatca tcaaatggaa gtttgaagat 600
ggtgcttggt ttgctaacca ataaaaagag tgcattcg 638

<210> 433
<211> 299
<212> DNA
<213> Escherichia coli

<400> 433
ctttacctgg catgatccac ttcgccagaa taccggcaat aagcccaaaa ataatccatg 60
acagaatgcc cattgtttcc tcacttatct gttttgcatt agcgggttag tcgctgataa 120
aaagcatagc acaacatcgg gagggcaaga tttgtgacga gcatcacgga ggtttttttg 180
cgatggcgca gaaattgcgc catcaacgat cagtgataat taccaaccac aaacatcatg 240
ttcgttttcc gtgtcataag aacgtacggt attcaccaga tcttttatca cttcagccg 299

<210> 434
<211> 388
<212> DNA
<213> Escherichia coli

<400> 434
gaaaaaggag gcaatatcgg gtaaaggcat tagcccgacg aatacgtcgg gctacaaata 60
ttattgtgct gcaggtgttt tagcgggttg ttgatccaca ggttctaact ggaagaccac 120
atcgacctga tcatcaaact gaatagcggc ctgctcgtaa gtttcctggg cggacaccgg 180
cgcggcatcg gctttcatca tccgcaccat tgggctgggc tgatagttgg aaacatggta 240
gcgcacgcta tataccggcc ccagtttacg atgaaagccg ttcgccagtt cctgcgcctg 300
atgaatcgcg ttatcaatcg ctgccttacg cgctttgtct ttataggcat ccggctgcgc 360
cacgcccagc gacacagaac gaattccc 388

<210> 435
<211> 351
<212> DNA
<213> Escherichia coli

<400> 435
ctatccttga tgaaaccgcg agcaaagata ggtgattacg tcatggtttt acagaaaatt 60
acagaaaaag gaggcaatat cgggtaaagg cattagcccg acgaatacgt cgggctacaa 120
atattattgt gctgcaggtg ttttagcggg ttgttgatcc acaggttcta actggaagac 180
cacatcgacc tgatcatcaa actgaatagc ggcctgctcg taagtttcct gggcggacac 240
cggcgcggca tcggctttca tcatccgcac cattgggctg ggctgatagt tggaaacatg 300
gtagcgcacg ctatataccg gccccagttt acgatgaaag ccgttcgcca g 351

<210> 436
<211> 762
<212> DNA
<213> Escherichia coli

<220>
<221> misc_feature
<222> (1)...(762)
<223> n = A,T,C or G

<400> 436
aattatgaaa cactgtctgg aatcgtctga atgacgggca catttgcgag cacgcatcca 60
gtaataacac aggaaactat tttatctacg cgttagcgat agactgcttg catggcgaaa 120
ggaggtaagc cgacgatttc agcgggacgc tgaaacggga aagcccctcc cgaggaaggg 180
gccataaata aggaaagggt catgatgaag ctactcatca tcgtggtgct cttagtcata 240
agcttccccg cttactaaga ctaccagggc gggggaaacc ccgctctacc ctcactcctg 300
aaagtatgcc ttcacgataa gattgtcaat ccgcaggctt tgtagtctgc gatcctgcca 360
gcaaatattc tttgcgagtc gttacgcaat aatcacagag gaaactattt tattcacgcg 420
ttagcgatag actgcattca gggcgaaagg aggtaagccg atgatttcag cgggacgctg 480
aaacgggaaa gcctctcccg gagaagaggg cttttaataa ggaaagggtt atgatgaagc 540
acgtcatcat actggtgata ctcttagtga ttagcttcca ggcttactaa gaacaccagg 600
gggaggggga aacctcttcc taaccctcac ttctgaaatt gggtgctatg acgctggcgt 660
tactgcttan cgctaccagt ttgtctgccc tggcggttgt aacgccagat cggtacccgt 720
ttggatattt taatgaaagc cgacaaatca atcancgtga cg 762

<210> 437
<211> 292
<212> DNA
<213> Escherichia coli

<400> 437
cacatttgcg agcacgcatc cagtaataac acaggaaact attttatcta cgcgttagcg 60
atagactgct tgcatggcga aaggaggtaa gccgacgatt tcagcgggac gctgaaacgg 120
gaaagcccct cccgaggaag gggccataaa taaggaaagg gtcatgatga agctactcat 180
catcgtggtg ctcttagtca taagcttccc cgcttactaa gactaccagg gcgggggaaa 240
ccccgctcta ccctcactcc tgaaagtatg ccttcacgat aagattgtca at 292

<210> 438
<211> 631
<212> DNA
<213> Escherichia coli

<400> 438
atttacactt tttacgaaat catgggatca ctaacaaaat atcgcttgtc agttatattg 60
tatggcagga aagatatgcg actgatatta cagatcccca aagtggagag tttatgacca 120
ttaaaaataa gatgttgctg ggtgcgcttt tgctggttac cagtgccgcc tgggccgcac 180
cagccaccgc gggttcgacc aatacctcgg gaatttctaa gtatgagtta agtagtttca 240
ttgctgactt taagcatttc aaaccagggg acaccgtacc agaaatgtac cgtaccgatg 300
agtacaacat taagcagtgg cagttgcgta acctgcccgc gcctgatgcc gggacgcact 360
ggacctatat gggtggcgcg tacgtgttga tcagcgacac cgacggtaaa atcattaaag 420
cctacgacgg tgagattttt tatcatcgct aaaaaaagcc ccctcatcat gagggggaaa 480
tgcagacacc ttgttatttt ttattattag ccacttgctc gtcttgcttg ttattagtcg 540
tatttcacgt tgattaatgc ggttgcctcc agtgcgccag atttaacttt gtttgtatcg 600
tagacgtagt aactggctgt tatcggaatt g 631

<210> 439
<211> 566
<212> DNA
<213> Escherichia coli

<400> 439
tatggcagga aagatatgcg actgatatta cagatcccca aagtggagag tttatgacca 60
ttaaaaataa gatgttgctg ggtgcgcttt tgctggttac cagtgccgcc tgggccgcac 120
cagccaccgc gggttcgacc aatacctcgg gaatttctaa gtatgagtta agtagtttca 180
ttgctgactt taagcatttc aaaccagggg acaccgtacc agaaatgtac cgtaccgatg 240

agtacaacat taagcagtgg cagttgcgta acctgcccgc gcctgatgcc gggacgcact 300
ggacctatat gggtggcgcg tacgtgttga tcagcgacac cgacggtaaa atcattaaag 360
cctacgacgg tgagattttt tatcatcgct aaaaaaagcc ccctcatcat gagggggaaa 420
tgcagacacc ttgttatttt ttattattag ccacttgctc gtcttgcttg ttattagtcg 480
tatttcacgt tgattaatgc ggttgcctcc agtgcgccag atttaacttt gtttgtatcg 540
tagacgtagt aactggctgt atcgaa 566

<210> 440
<211> 339
<212> DNA
<213> Escherichia coli

<400> 440
cgtattcaca tccttttgat tggtgataac atgcgaatcg gtattatttt tccggttgta 60
atcttcatta cagcggtcgt atttttagca tggtttttta ttggcggcta tgctgccccg 120
ggagcataaa gatgaaaaaa acaacgatta ttatgatggg tgtggcgatt attgtcgtac 180
tcggcactga gctgggatgg tggtaacgtc acctctaaaa aatagcaaag gctgcctgtg 240
tgcagccttt gtgcaattta agcgttaact tttaatcttc ctgtagataa atagcacgac 300
aatcgcacca ataacggcaa ccacgaagct gccaaaatt 339

<210> 441
<211> 376
<212> DNA
<213> Escherichia coli

<400> 441
catgaatatt taaaaaggaa aacgacatga aaccgaagca cagaatcaac attctccaat 60
cataaaatat ttccgtggag cattttatta ttgaatatag aggtttaact ccggtaaaaa 120
acaaagaagc attgaatgca gggaaaaata atatggccat aaaaaacatc gaaagaaact 180
cttttaattt aacatgtaaa cgcatggtta atcctcatat cacgggtgga gtgttaagaa 240
catacataaa tggagtcatg ttttcccttt tccatttatc aagttcctgt tgccgtttta 300
gtccatctct aattgcatat tttaattttt ctgataaatg gcattgagca tcgatttcat 360
ttaaaacaac tgtaca 376

<210> 442
<211> 446
<212> DNA
<213> Escherichia coli

<400> 442
   ttacgatagc tattagtaaa aatataagag ttagctgtat tgttatgtct gtggcgaaat 60
   tgactacctt cgtttttttg attaagaatg attttattat cgtaagtaaa attacatgaa 120
   tatttaaaaa ggaaaacgac atgaaaccga agcacagaat caacattctc caatcataaa 180
   atatttccgt ggagcatttt attattgaat atagaggttt aactccggta aaaaacaaag 240
   aagcattgaa tgcagggaaa aataatatgg ccataaaaaa catcgaaaga aactctttta 300
   atttaacatg taaacgcatg gttaatcctc atatcacggg tggagtgtta agaacataca 360
   taaatggagt catgttttcc cttttccatt tatcaagttc ctgttgccgt tttagtccat 420
   ctctaattgc atattttaat ttttct 446

<210> 443
<211> 388
<212> DNA
<213> Escherichia coli

<220>
<221> misc_feature
<222> (1)...(388)
<223> n = A,T,C or G

<400> 443
tcaccccggt gccgattttc aggcatcctg atttaactta gcacccgcaa cttaactaca 60

ggaaaacaaa gagataaatg tctaatcctg atgcaaatcg agccgatttt ttaatcttta 120
cggactttta cccgcctggt ttattaattg cactgtnatc cgggcgttcg cccgctttaa 180
tcacaatagg ctgtgtagcc tgggcctgtt tctctttcac ccgcgccaga gcggcagcaa 240
tcgcatcttt atctttggct gcaggttgaa cggctgcgct cttatgtcgt tcaaggcgag 300
ccgctttttc gcgctccaga cgagcctggc gcgcttcgaa acgcgctttg gcttctgcgg 360
cncgcttttc ttcctgacga atagccgc 388

<210> 444
<211> 209
<212> DNA
<213> Escherichia coli

<400> 444
aattttaata acgctatctg cggataaagc agaataggtg gttaacccca gacataaacc 60
gaggaaaata atgttattgt atttcataat ctattgttcc ttagcgacag attgctgtct 120
gctggttcag taaggtacca ggagaaactt caggaagctt gtactcgaca atacagtttg 180
agtttttatc tttgccccat gaaacctgt 209

<210> 445
<211> 341
<212> DNA
<213> Escherichia coli

<400> 445
catcctcaat accgttaaat gcaacccgaa cccccgttgt ccctttgctg cattcactta 60
acgtaatctg aaaagggacg gctggacttg tgctaccggt cgttggaaat tgtctggcac 120
tgtttttttg gagatctacg gtaaaattaa gcgaatccga tgagactgtg cagccataat 180
cgaggacgcg cccgctaatt ttaataacgc tatctgcgga taaagcagaa taggtggtta 240
accccagaca taaaccgagg aaaataatgt tattgtattt cataatctat tgttccttag 300
cgacagattg ctgtctgctg gttcagtaag gtaccaggag a 341

<210> 446
<211> 697
<212> DNA
<213> Escherichia coli

<400> 446
agatttactg ccaatttccg gcagatcgga aagggttaam ccatattgat ccataagggt 60
acgaatcmcg ggctataccg ccaggcatgg cttgagccat ggcattaaat tccgcaaatt 120
cgggcgctga ttcttcccac gcggttattt tggcacacac cagatccagc aaggggtttt 180
caggatcgtt gagcagcaga tgatctacca gttccagcgc ctgggtgtat tgttcctcgt 240
tctgaatacc cgccagaaaa ggtgccacag cagttagctt ttctcctgct tgcaagatgt 300
cggcaatcgc aatcattttt tccccttagt acgatgaaca gcggtaaaga aatcgtattc 360
tttatgcgtc ataacttcac gtatgtagca cttttgcgat tcaaaaaaga ccattgctac 420
aacacgtaat tcattgcccc caacattgaa aacataatgc ttatccagat atttgaagtt 480
atccagagat gggaatactg cttttaatga ctcaggtttt ttgaaatatc ccttagcaat 540
cgtgktcccc agagccacca actccgtttt atgttgcggg tatttttccg cagcatcttt 600
caatgctttt tgagttatca ggtgcattct tcatcacgtc cgtkgmcaaa ttggcaatat 660
gataacatcc gttgccagat tggcacggat gaattat 697

<210> 447
<211> 215
<212> DNA
<213> Escherichia coli

<400> 447
aattaataac ttttcgttag gcagttttgg gtgtgagttg caagagggga gactactgaa 60
taactcaagt tttataatcg aggggaaaat ggtgatggcg ttcatagcaa aacgccctca 120
accataaagg tcgagggcgc ttaagatgtt aaaaacccgc tatccgttaa aaaacaatgt 180
tcaactaagg tcagtgacat tgcgctaaaa aagcg 215

<210> 448
<211> 395
<212> DNA
<213> Escherichia coli

<400> 448
gcattattca tgagaaatgt gtatcgtaaa tcaactgaaa ttaacgcaac catttgttat 60
ttaaggttta attatctgtg tgtgatattt tattgaatgt tttaaatatt gtttttattg 120
gcattgctat aatattggtt atcatttgct gaatggattc agtcttaatg agtgggtttt 180
taagggacag gcatagagta atgatacgta tgcataacca acatctttac tcattatgtc 240
attgaatgtt gacgctatgt gtttatgagg gagaggtatt ttcagttgat ctggattgtt 300
aaattcatat aatgcgcctt tgctcatgaa tggatgccag tatgtagtgg gaaattataa 360
atattgaaat agtccaacta cttctttatt accaa 395

<210> 449
<211> 641
<212> DNA
<213> Escherichia coli

<220>
<221> misc_feature
<222> (1)...(641)
<223> n = A,T,C or G

<400> 449
ataatcaggt aagaaaaggt gcgcggagat taccgtgtgt tgcgatatat tttttagttt 60
cgcgtggcaa tacatcagtg gcaataaaac gacatatcca gaaaaatata cactaagtga 120
atgatatctt ccgatttatc ttaatcgttt atggataacg gcaaagggct tcgttttttc 180
ctatacttat tcagcactca caaataaagg aacgccaatg aaaattatac tctgggctgt 240
attgattatt ttcctgattg ggctactggt ggtgactggc gtatttaaga tgatatttta 300
aaattaatta atgtcatcag gtccgaaaat aacgagaata tttcagtctc tcatcctgtt 360
gcgctcctgt catgtgcatt gcttcatata atcactggcg caaggagcgc cgcaggcgna 420
gnntgcncgn cgncccacct naccccatgc cgaacttcag aantgaaaac nccntaacnc 480
cgatngtcgg cgggngcctc cccatgcnan agtangggaa ntgccangcg ncnnattaaa 540
cgaaaggctn attncaaaga ctgggccttn cntttatctg atgtttgtcg gagaacgctc 600
tcctgagnan gacaaatncc gccgggagcg gatttgaacn t 641

<210> 450
<211> 314
<212> DNA
<213> Escherichia coli

<220>
<221> misc_feature
<222> (1)...(314)
<223> n = A,T,C or G

<400> 450
gaactacgag taagaatagc tncgaattcc cgtttatgga taacggcaaa gggcttcgtt 60
ttttcctata cttattcagc actcacaaat aaaggaacgc caatgaaaat tatactctgg 120
gctgtattga ttattttcct gattgggcta ctggtggtga ctggcgtatt taagatgata 180
ttttaaaatt aattaatgtc atcaggtccg aaaataacga gaatatttca gtctctcatc 240
ctgttgcgct cctgtcatgt gcattgcttc atataatcac tggcgcaagg agcgcgcagg 300
gggntntnnt cttt 314

<210> 451
<211> 236
<212> DNA
<213> Escherichia coli

<400> 451

atatacacta agtgaatgat atcttccgat ttatcttaat cgtttatgga taacggcaaa 60
gggcttcgtt ttttcctata cttattcagc actcacaaat aaaggaacgc caatgaaaat 120
tatactctgg gctgtattga ttattttcct gattgggcta ctggtggtga ctggcgtatt 180
taagatgata ttttaaaatt aattaatgtc atcaggtccg aaaataacga gaatat 236

<210> 452
<211> 418
<212> DNA
<213> Escherichia coli

<400> 452
cggagattac cgtgtgttgc gatatatttt ttagtttcgc gtggcaatac atcagtggca 60
ataaaacgac atatccagaa aaatatacac taagtgaatg atatcttccg atttatctta 120
atcgtttatg gataacggca aagggcttcg ttttttccta tacttattca gcactcacaa 180
ataaaggaac gccaatgaaa attatactct gggctgtatt gattattttc ctgattgggc 240
tactggtggt gactggcgta tttaagatga tattttaaaa ttaattaatg tcatcaggtc 300
cgaaaataac gagaatattt cagtctctca tcctgttgcg ctcctgtcat gtgcattgct 360
tcatataatc actggcgcaa ggagcgcgca gggggcggcc aatcgccgcc gccccctg 418

<210> 453
<211> 551
<212> DNA
<213> Escherichia coli

<400> 453
   aacaatttgc ccatgcgctc ggtcatgcgc tgcatcgccc ggccattttg sgcgtccccg 60
   cgaccgccat tcgactgtta atgggcgaat cttcagtact ggtattaggt ggacaacgcg 120
   cgctgcctaa acggctggaa gaagcgggtt ttgcgtttcg ctggtacgat ttagaagagg 180
   cgctggcgga tgtcgttcgc tgatgtggtt tacagcaaac atccgccagt taactcccgg 240
   tgttacagga ttagtggctt tgcgcgataa gatcgtctgg tgaaagtcgg gtcaccatca 300
   taactaactc tctgtctaaa cctctatcca gcatctcctg agcaatacgc agggcttctt 360
   cgtgtttgcc ctgcattgcg ccttcttcac gtaatctgtc agcaatggtc atcaagtttc 420
   tccttttctt gtggtgcgcg ttccgctatc tcaccaataa atgcacgaaa acgctgggca 480
   tcccctgttt gtaatacgta attaaacagg gcttttagct gtctgtcatt agtgktccct 540
   gtaactagca g 551

<210> 454
<211> 93
<212> DNA
<213> Escherichia coli

<400> 454
tggcatctcg gtgttgccga tcttcatgat atccagcccg ccggaaactt cttcccaaac 60
ggttttgctg ttatccattg agtcacggaa ctg 93

<210> 455
<211> 232
<212> DNA
<213> Escherichia coli

<400> 455
cgtgccgaga tgatcctgta accatcatca gttgtgaagt agtgattcac gacttcaagg 60
cgcttttcaa aagggtattt tggctttgac atattagggg ctattccatt tcatcgtcca 120
acaaaatggg tgcagtacat actcgttgga aatcaacaca ggaggctggg aatgccgcag 180
aaatatagat tactttcttt aatagtgatt tgtttcacgc ttttattttt ca 232

<210> 456
<211> 713
<212> DNA
<213> Escherichia coli

<220>
<221> misc_feature
<222> (1)...(713)
<223> n = A,T,C or G

<400> 456
ttagnggatn naangcccac ancctcgang gatctaggag gtagaatagc ttcgaattcc 60
ccagcagagc gcggccttct tcgtcagatt tcgcagtagt ggtaatggta atatccaaac 120
cacgaacgcg gtcgacttta tcgtagtcga tttctgggaa gatgatctgc tcacggacac 180
ccatgctgta gttaccacga ccgtcgaaag acttagcgga caggccacgg aagtcacgga 240
tacgaggtac agcaatagtg atcaggcgct caaagaactc ccacatgcgt tcgccacgca 300
gagttacttt acagccgatc ggatagccct gacggatttt gaagcctgca acagatttgc 360
gtgctttggt gatcagcggt ttttgaccgg agattgctgc caggtctgct gctgcgttat 420
ccagcagttt tttgtcagcg atcgcttcac caacacccat gttcagggtg atcttctcga 480
cccgagggac ttgcatgaca gaattgtagt taaactcagt catgagtttt ttaactactt 540
cgtctttgta gtaatcatgc agtttcgcca tcgtactact ccatgtcggt gaacgctctc 600
ctgagtagga caaatccgcc ggagccggat ttaacgttgc gaacaaccgn cccggagggg 660
tggnggcagg accccgccat aactggcagc attaaattaa gcagaaggcc atc 713

<210> 457
<211> 292
<212> DNA
<213> Escherichia coli

<400> 457
tgaacagcag agatacggcc agtgcggcca atgttttttg tcctttaaac ataacagagt 60
cctttaagga tatagaatag gggtatagct acgccagaat atcgtatttg attattgcta 120
gtttttagtt ttgcttaaaa atattgttag ttttattaaa tgcaaaacta aattattggt 180
atcatgaatt tgttgtatga tgaataaaat ataggggggt atagatagac gtcattttca 240
tagggttata aatgcgacta ccatgaagtt tttaattgaa agtattgggt tg 292

<210> 458
<211> 282
<212> DNA
<213> Escherichia coli

<400> 458
ttattaaatg caaaactaaa ttattggtat catgaatttg ttgtatgatg aataaaatat 60
aggggggtat agatagacgt cattttcata gggttataaa tgcgactacc atgaagtttt 120
taattgaaag tattgggttg ctgataattt gagctgttct attcttttta aatatctata 180
taggtctgtt aatggatttt atttttacaa ttttttgtgt ttaggcatat aaaaatcaac 240
ccgccatatg aacggcgggt taaaatattt acaacttagc aa 282

<210> 459
<211> 300
<212> DNA
<213> Escherichia coli

<400> 459
tctgcgttcc gctaaaaggt gcaaatgctc aggacgttgc agcgttttgc gtgaccgctc 60
ggggaaggca aaattgcctc tgggaaagca ttgcgcgggg tccggcgctc atcaacaatc 120
ggggggcagc aaggggctga aacgggaaag cccctcccga agaaggggcc ttgtataagg 180
aaagggttat gatgaagctc gtcatcatac tggttgtgtt gttactgtta agtttcccga 240
cttactaaca actcatcaga ggggggagaa atcctccctt acccttgttc ctttactcta 300

<210> 460
<211> 293
<212> DNA
<213> Escherichia coli

<400> 460

cggggtccgg cgctcatcaa caatcggggg gcagcaaggg gctgaaacgg gaaagcccct 60
cccgaagaag gggccttgta taaggaaagg gttatgatga agctcgtcat catactggtt 120
gtgttgttac tgttaagttt cccgacttac taacaactca tcagaggggg gagaaatcct 180
cccttaccct tgttccttta ctctaggttg aaaaaacaac agcgtcaata ggcctgccat 240
gtacgaagcg agatctgtga accgctttcc ggttagcctt ttttatcctg ttg 293

<210> 461
<211> 359
<212> DNA
<213> Escherichia coli

<400> 461
caacacagga ggctgggaat gccgcagaaa tatagattac tttctttaat agtgatttgt 60
ttcacgcttt tatttttcac ctggatgata agagattcac tgtgtgaatt gcatattaaa 120
caggagagtt atgagctggc ggcgttttta gcctgcaaat tgaaagagta agagtcttcg 180
gcgggaaatt attcccgcct tacttacggc gttgcgcatt ctcattgcac ccaaatttat 240
tcttcacaaa aataataata gattttatta cgcgatcgat tatttatttc ctgaaaacaa 300
ataaaaaaat ccccgccaaa tggcagggat cttagattct gtgcttttaa gcagagatt 359

<210> 462
<211> 673
<212> DNA
<213> Escherichia coli

<400> 462
gcaacccatg tcctgacctg ggttcggggg acaccaaaac gtgccgagat gatcctgtaa 60
ccatcatcag ttgtgaagta gtgattcacg acttcaaggc gcttttcaaa agggtatttt 120
ggctttgaca tattaggggc tattccattt catcgtccaa caaaatgggt gcagtacata 180
ctcgttggaa atcaacacag gaggctggga atgccgcaga aatatagatt actttcttta 240
atagtgattt gtttcacgct tttatttttc acctggatga taagagattc actgtgtgaa 300
ttgcatatta aacaggagag ttatgagctg gcggcgtttt tagcctgcaa attgaaagag 360
taagagtctt cggcgggaaa ttattcccgc cttacttacg gcgttgcgca ttctcattgc 420
acccaaattt attcttcaca aaaataataa tagattttat tacgcgatcg attatttatt 480
tcctgaaaac aaataaaaaa atccccgcca aatggcaggg atcttagatt ctgtgctttt 540
aagcagagaa tacaggctgg ttacgttacc agctgccggg cctttagcgc cgctttcgat 600
ggtgaaggac actttctgac cttcgtccag agatttgtaa ccatcgttct ggatagcaga 660
gaagtgtacg aac 673

<210> 463
<211> 630
<212> DNA
<213> Escherichia coli

<400> 463
tggtggcatt ggttgctgga gagagaaaac ccccgcacgt tgcaggtatg cacctgacaa 60
caccacgggg gctaatcttg actctagacc actcaagaat agccgcgaaa cgttgtcatt 120
acaacacagg cggctatatg acgttcgcag agctgggcat ggccttctgg catgatttag 180
cggctccggt cattgctggc attcttgcca gtatgatcgt gaactggctg aacaagcgga 240
agtaacgtgt catgcgggcg tcaggctgcc gtaatggcaa tttgcgcccg gaccaggccg 300
caggggggaa actctgcggc ctttttcgtt cttactgcgg gtaaggcacc cagtcgccgc 360
cgttcaggcg aacgtacggt ttatcctggt attgaataac tactgcattt gagttctcgg 420
agaccggtgc tgtttgtggc aacccactgg tgagtttttt ccagtcaaca ttgtcttcgg 480
tgaaaatctt gccatcgaga acgcgaacca ccagatcgga gatagccagg aagctgctcg 540
gttgttcgat gacaatcggt gccccctgat gcggtgcctt catgccgaag aatttcaccc 600
caacggggac gtcggtgata gacgggctag 630

<210> 464
<211> 391
<212> DNA
<213> Escherichia coli

<400> 464
ctcaggctgc tgattgtttt tttgtgcaat ggcgcggtat tagcgtcgtt gctgtcgatg 60
gagagaatca taaacgtggt gaatgatgat tgttagcaag gaaaactgtc aaaaatcttc 120
aaaaaatttg agggataagg ccggaatggc tccggccaga gggaagttaa ccgcgaagct 180
gttgctgctt gagggtcgtt ttaaccagac gccaggcgct ccatacgcca aaaccgcgtc 240
tggcccagcg gaccagcata ttaggatggc gaatcgtcca gatcgccatc acgctactgc 300
caaccagcgc ccaggagcgc agacttagca gcatattcca gcgacgatcg taagcgcctg 360
ttgtctccag ccattcacga cgactggcgg a 391

<210> 465
<211> 625
<212> DNA
<213> Escherichia coli

<400> 465
aacacaccac accataaacg gaggcaaata atgctgggta atatgaatgt tttaatggcc 60
gtactgggaa taattttatt ttctggtttt ctggccgcgt atttcagcca caaatgggat 120
gactaatgaa cggagataat ccctcaccta accggcccct tgttacagtt gtgtacaagg 180
ggcctgattt ttatgacggc gaaaaaaaac cgccagtaaa ccggcggtga atgcttgcat 240
ggatagattt gtgttttgct tttacgctaa caggcatttt cctgcactga taacgaatcg 300
ttgacacagt agcatcagtt ttctcaatga atgttaaacg gagcttaaac tcggttaatc 360
acattttgtt cgtcaataaa catgcagcga tttcttccgg tttgcttacc ctcatacatt 420
gcccggtccg ctcttccaat gaccacatcc agaggctctt caggaaatgc gcgactcaca 480
cctgctgtca cggtaatgtt gatatgccct tcagaatgtg tgatggcatg gttatcgact 540
aactggcaaa ttctgacacc tgcacgacat gcttcttcat cattagccgc tttgacaata 600
atgataaatt cttcgccccc gtagc 625

<210> 466
<211> 623
<212> DNA
<213> Escherichia coli

<400> 466
tgcttttgaa tatgtgctcg caatcttgag aaggaaatgg cgaccacgaa agaaaaggca 60
aaaacgataa tctgaaagag ccaaggtatt tcagtataag cattgaatgc gacagtaaac 120
tctttcggta tcagccagag agtgagacca aaaatgataa tcgtatacat aagtctttcg 180
agtggctcgt tagcaaaaag tttcaacaat ggagtaaata catccaacat atcaataact 240
ctcaactgta agggtattga aatgttaaca caagctctcg ctgtaggggt atagccgaga 300
ccaccgaagc ccggaggtgg tgaaataaaa ccgggcacaa cacgaaggcg catttccgat 360
atccataaag agtcggtctt gtctgttaaa tttaaatggt gggagtgcgc ctccggttgt 420
aaataacgac attgctgtgt gtagtcctgg cggcatcagt ttttttcttg aagttcggct 480
gatgtccgcc ctttttaaag tgaattttgt gatgcggtga atgcggctaa gcgcacgtgg 540
cacagttaaa agtcatgtta gtccttattg gtttgggtgg gaaagccgac tgtaattgtt 600
aactggttgc agtcacctgg agg 623

<210> 467
<211> 234
<212> DNA
<213> Escherichia coli

<400> 467
tgtttactta caagagattc atctttgtat aaataaagat aagtaattac gcataaaaca 60
acaatgatta taatagcaaa aataaatatt atcatctttg atagattact tgagatagcc 120
agcatcttgt aaagccttta tcgttttttt atgctctgga ttaatataat cactacatct 180
atctgagcaa tctgttgttg atggacatgt caacccatgg tcatttacag ccaa 234

<210> 468
<211> 529
<212> DNA
<213> Escherichia coli

<400> 468
attagctatt tcggctaaaa tagagactac atgtcttcgg tccatctcac ttaaggagtg 60
tagttccgtt gtaagttttt ccatagcttg cactgctaaa tttcgaacaa ggaattttct 120
gctggtaatc tctaaaaaga tggcatggtt tacaatgatt tttgtttcct tttgattatt 180
atgaacaact gtccatgatt tcgtttaaga atgaagagaa atcactaaac gaactgaata 240
tattttctgt gccaatatta tctctaattt caaaaaagtt acttttaatg tcggtaatga 300
ctccaactta ttgatagtgt tttatgttca gataatgccc gatgactttg tcatgcagct 360
ccaccgattt tgagaacgac agcgacttcc gtcccagccg tgccaggtgc tgcctcagat 420
tcaggttatg ccgctcaatt cgctgcgtat atcgcttttc cttatcagtt cgttgatgtc 480
agtggttttg accacgaggg agcttcacgc gagttattga aaaccctga 529

<210> 469
<211> 261
<212> DNA
<213> Escherichia coli

<400> 469
caaagaacct tcaacatgaa aaatatccat ttgtttgcaa aaaaagatta ttaggaagga 60
aattaatgca attatcgaaa attcaaaaaa tatccaaaaa tagtatactt tattccagaa 120
gagttcaata taatgtttgt cttcaatttt tcttacttca gggtaatata gattgctcat 180
tacattgtga gcttcatctt tatttaattt tctgttgact ccagctctcc gtgataacgg 240
ttttataatt agatgcttat c 261

<210> 470
<211> 98
<212> DNA
<213> Escherichia coli

<400> 470
agatgattgc cgggaacttg ttagcggcac gcaggcggcg gctcgcaccc ttaccctgct 60
ctttacgtac ttctgcgttg atagtaaaca tttctttc 98

<210> 471
<211> 259
<212> DNA
<213> Escherichia coli

<400> 471
agcgcgaacg aagtcgatgt gctgcagctt cggtttgtac gggtgacgct gtacgtcctg 60
agctttaact ttgatttctt taccgtcaac aacgatggtc agaacttcgc tgtagaattc 120
agctttagct tgcatgttca tgactttgtc gtgatccagc tcgatagcca gcggcgcttc 180
tttgccaccg tagatgattg ccgggaactt gttagcggca cgcaggcggc ggctcgcacc 240
cttaccctgc tctttacgt 259

<210> 472
<211> 94
<212> DNA
<213> Escherichia coli

<400> 472
aaaaacggcg taaagaaagg atgcaaacat gttaataaaa actcaaattg atcccacgta 60
tatattacgc cgcaaaatcc ttacaataaa cagg 94

<210> 473
<211> 174
<212> DNA
<213> Escherichia coli

<400> 473
ttaattatta aaatagtgta acgcgattat gtggttatgg gggtaaacat taaataaacc 60
agcggggagg ggaggtaaag tgaaaaaata aaaagcggat aatcttaata agcaggccgg 120

acagcatcgc catccggcac tgatacgagg tttatttcag ctcatcaacc atcg 174

<210> 474
<211> 138
<212> DNA
<213> Escherichia coli

<400> 474
ctgtaaaaac gtcaaaaaga gtgttttatc aacagaagaa tggaggtctg acagatagta 60
gtaatgcaaa aaaatggaga cttaagttga atgaacggga gtaaagcgaa aagactatag 120
agtgaaggag aaattccc 138

<210> 475
<211> 191
<212> DNA
<213> Escherichia coli

<400> 475
tttgttggct taatattcta ttgttatctt tatttataga tgtttatatt gcatgaggtg 60
gtttttggag agaagaatga ggaagatgcg tcgagccaca gaaacgttag ctttacatat 120
agcggaggtg atgtgaaatt aatttacaat agaaataatt tacatatcaa acagttagat 180
gctttttgtc g 191

<210> 476
<211> 245
<212> DNA
<213> Escherichia coli

<400> 476
cggccattta tacaggaaaa gcctatgtca gaacgtaaaa actcaaaatc acgccgtaat 60
tatctcgtta aatgttcctg cccaaactgc acccaagagt cagaacacag tttttcaaga 120
gtacaaaaag gtgccctttt gatctgccct cattgcaaca aagtattcca gacaaatctt 180
aaagctgtag cctgattgat tttattagta acaagtattt tttatatttt aataatatat 240
ttaaa 245

<210> 477
<211> 319
<212> DNA
<213> Escherichia coli

<400> 477
aaattttcag gtaccttgtc accatacttt tttttctgag cattaatgat attttgagct 60
tcttgaggat ctttaactcc ccacatttgg tggaaagtat tcatattaaa aggaaggttg 120
aataatttgt ctttataaat cgccagtgga gaattagtaa aacgattaaa ttctactaaa 180
tcattaacgt aatcccatat atatttatca ttggtatgaa aaatatgtgc accatattta 240
tgaatctgga taccctcaca gtcctctgtg tacgcatttc caccgatatg atttcttttc 300
tcaatcacta aaactttttt 319

<210> 478
<211> 149
<212> DNA
<213> Escherichia coli

<400> 478
gcagtgatcg aagcgatgac gaagtgtatg gaaaaatcag aaaaactcag caaatcctga 60
tgactttcgc cggacgtcag gccgccactt cggtgcggtt acgtccggct ttctttgctt 120
tgtaaagcgc caaatctgcc gatttcaac 149

<210> 479
<211> 330
<212> DNA

<213> Escherichia coli

<400> 479
gaaagtatct tcgttattga catcactgga aaatataact tgcttttcat tattaaactc 60
gaagcgcgta ccgtatctgg acaaacattt atcgagctta ccaaattcct gaagaggttt 120
aactacagat aacatttgcg cgtcctttgc agtaatgccc gtcaaatcct tgacgggcat 180
tatttagatt aaattaccag tatttcttcg gagtgaagaa tattaccagg tatatttaac 240
acccacgttc gcggaccagt cttgatctac gtcaccacca ccgaggtagt tagcatcggt 300
ataggcgctg aagttcttgg tgaagctaaa 330

<210> 480
<211> 191
<212> DNA
<213> Escherichia coli

<400> 480
tttttttcca gcaacggagc aaaaggtttg cccttgtgca gctcagggtt aaccacttta 60
actacgtggc gacgacccgg agatgtcggt ttacatttaa caactgccat tgtattactc 120
ctccgactta ctcagcgccg ccaacgaagt ccagattctg gccttctttc agggtgacgt 180
aagctttttt c 191

<210> 481
<211> 188
<212> DNA
<213> Escherichia coli

<400> 481
tccctttaac taccagggtg ttaacgactt cgacttcgac ttcaaacagt ttctgcacag 60
cagctttgat ttctgctttg gtcgcgtctt tagcaacttt gagtacgatg gtgttggatt 120
tttccatcgc agtagacgct ttttcagaaa cgtgcggtgc acgcagcacc ttcagcagac 180
gttcttca 188

<210> 482
<211> 172
<212> DNA
<213> Escherichia coli

<400> 482
caaaggcgaa caaagcctgt gaagcccgaa ggctccacag acagtgctac ttgaaggcct 60
tactgtttct tcttaggagc gagcaccatg atcatctggc ggccttcgat cttcgttggg 120
aaggattcga ccactgccag ttcttgcaaa tcgtctttca cgcgattaag ca 172

<210> 483
<211> 266
<212> DNA
<213> Escherichia coli

<400> 483
tggagaaaac gggtgattga taaagcaatc atcgttctag gggcgttaat tgcgctgctg 60
gaactgatcc gctttctgct tcagcttctg aactgatagc ggaaacgtaa ttaagggcta 120
agagcacact actcttagcc ctttaacatt taacgcattg tcacgaactc ttctgccgcc 180
gttgggtgaa tggcgacggt attgtcgaag tcttttttgg ttgcccccat cttcagcgcc 240
accgcgaagc cctgcaacat ttcgtc 266

<210> 484
<211> 259
<212> DNA
<213> Escherichia coli

<400> 484
cgcaggcagc tgatggtcaa caggatgaga gaaacccaga gacaggttaa tcacattgcc 60

WO 00/44906 PCT/US00/02200

tttaaccgct gcacggtaac ctacaccaac cagctgcagc ttcttagtga agccttcggt 120
aacaccgata accattgagt tcagcagggc acgcgcggta ccagcctgtg cccaaccgtc 180
tgcgtaacca tcacgcggac cgaaggtcag ggtattatct gcatgtttaa cttcaacagc 240
atcgttgaga gtacgagtc 259

<210> 485
<211> 73
<212> DNA
<213> Escherichia coli

<400> 485
caggtcggaa cttacccgac aaggaatttc gctaccttag gaccgttata gttacggccg 60
ccgtttaccg ggg 73

## Description

### BACKGROUND OF THE INVENTION

Since the discovery of penicillin, the use of antibiotics to treat the ravages of bacterial infections has saved millions of lives. With the advent of these "miracle drugs," for a time it was popularly believed that humanity might, once and for all, be saved from the scourge of bacterial infections. In fact, during the 1980s and early 1990s, many large pharmaceutical companies cut back or eliminated antibiotics research and development. They believed that infectious disease caused by bacteria finally had been conquered and that markets for new drugs were limited. Unfortunately, this belief was overly optimistic.

The tide is beginning to turn in favor of the bacteria as reports of drug resistant bacteria become more frequent. The United States Centers for Disease Control announced that one of the most powerful known antibiotics, vancomycin, was unable to treat an infection of the common *Staphylococcus aureus* (staph). This organism is commonly found in our environment and is responsible for many nosocomial infections. The import of this announcement becomes clear when one considers that vancomycin was used for years to treat infections caused by stubborn strains of bacteria, like staph. In short, the bacteria are becoming resistant to our most powerful antibiotics. If this trend continues, it is conceivable that we will return to a time when what are presently considered minor bacterial infections are fatal diseases.

There are a number of causes for the predicament in which practitioners of medical arts find themselves. Over-prescription and improper prescription habits by some physicians have caused an indiscriminate increase in the availability of antibiotics to the public. The patient is also partly responsible, for even in instances where an antibiotic is the appropriate treatment, patients will often improperly use the drug, the result being yet another population of bacteria that is resistant, in whole or in part, to traditional antibiotics.

The bacterial scourges that have haunted humanity remain, in spite of the development of modern scientific practices to deal with the diseases that they cause. Drug resistant bacteria are now advancing on the health of humanity. A new generation of antibiotics to once again deal with the pending health threat that bacteria present is required.

### Discovery of New Antibiotics

As more and more bacterial strains become resistant to the panel of available antibiotics, new compounds are required. In the past, practitioners of pharmacology would have to rely upon traditional methods of drug discovery to generate novel, safe and efficacious compounds for the treatment of disease. Traditional drug discovery methods involve blindly testing potential drug candidate-molecules, often selected at random, in the hope that one might prove to be an effective treatment for some disease. The process is painstaking and laborious, with no guarantee of success. Today, the average cost to discover and develop a new drug is nearly US $500 million, and the average time is 15 years from laboratory to patient. Improving this process, even incrementally, would represent a huge advance in the generation of novel antimicrobial agents.

Newly emerging practices in drug discovery utilize a number of biochemical techniques to provide for directed approaches to creating new drugs, rather than discovering them at random. For example, gene sequences and proteins encoded thereby that are required for the proliferation of an organism make for excellent targets since exposure of bacteria to compounds active against these targets would result in the inactivation of the organism. Once a target is identified, biochemical analysis of that target can be used to discover or to design molecules that interact with and alter the functions of the target. Using physical and computational techniques, to analyze structural and biochemical targets in order to derive compounds that interact with a target is called rational drug design and offers great future potential. Thus, emerging drug discovery practices use molecular modeling techniques, combinatorial chemistry approaches, and other means to produce and screen and/or design large numbers of candidate compounds.

Nevertheless, while this approach to drug discovery is clearly the way of the future, problems remain. For example, the initial step of identifying molecular targets for investigation can be an extremely time consuming task. It may also be difficult to design molecules that interact with the target by using computer modeling techniques. Furthermore, in cases where the function of the target is not known or is poorly understood, it may be difficult to design assays to detect molecules that interact with and alter the functions of the target. To improve the rate of novel drug discovery and development, methods of identifying important molecular targets in pathogenic microorganisms and methods for identifying molecules that interact with and alter the functions of such molecular targets are urgently required.

*Escherichia coli* represents an excellent model system to understand bacterial biochemistry and physiology. The estimated 4288 genes scattered along the 4.6 x 10⁶ base pairs of the *Escherichia coli* (*E*. *coli*) chromosome offer tremendous promise for the understanding of bacterial biochemical processes. In turn, this knowledge will assist in the development of new tools for the diagnosis and treatment of bacteria-caused human disease. The entire *E*. *coli* genome has been sequenced, and this body of information holds a tremendous potential for application to the discovery and development of new antibiotic compounds. Yet, in spite of this accomplishment, the general functions or roles of many of these genes are still unknown. For example, the total number of proliferation-required genes contained within the E. coli genome is unknown, but has been variously estimated at around 200 to 700 (Armstrong, K.A. and Fan, D.P. Essential Genes in the *metB-malB* Region of *Escherichia coli* K12, 1975, J. Bacteriol. 126:48-55).

Novel, safe and effective antimicrobial compounds are needed in view of the rapid rise of antibiotic resistant microorganisms. However, prior to this invention, the characterization of even a single bacterial gene was a painstaking process, requiring years of effort. Accordingly, there is an urgent need for more novel methods to identify and characterize bacterial genomic sequences that encode gene products required for proliferation and for methods to identify molecules that interact with and alter the functions of such genes and gene products.

### SUMMARY OF THE INVENTION

One embodiment of the present invention is a purified or isolated nucleic acid sequence consisting essentially of one of SEQ ID NOs: 1-81, 405-485, wherein said nucleic acid inhibits microorganism proliferation. The nucleic acid sequence may be complementary to at least a portion of a coding sequence of a gene whose expression is required for microorganism proliferation. The nucleic acid sequence may comprise a fragment of one of SEQ ID NOs. 1-81, 405. 485, said fragment selected from the group consisting of fragments comprising at least 10, at least 20, at least 25, at least 30, at least 50 or more than 50 consecutive bases of one of SEQ ID NOs: 1-81, 405-485. The nucleic acid sequence may be complementary to a coding sequence of a gene whose expression is required for microorganism proliferation.

Another embodiment of the present invention is a vector comprising a promoter operably linked to a nucleic acid comprising a sequence selected from the group consisting of SEQ ID NOs. 1-81, 405-485. The promoter may be active in an organism selected from the group consisting of *Escherichia coli, Staphylococcus aureus, Pseudomonas aeruginosa, Enterobacter cloacae, Helicobacter pylori, Neisseria gonorrhoeae, Enterococcus faecalis, Streptococcus pneumoniae, Haemophilus influenzae, Salmonella typhimurium, Saccharomyces cerevisiae, Candida albicans, Cryptococcus neoformans, Aspergillus fumigatus, Klebsiella pneumoniae, Salmonella typhi, Salmonella paratyphi, Salmonella cholerasuis, Staphylococcus epidermidis, Mycobacterium tuberculosis, Mycobacterium leprae, Treponema pallidum*, *Bacillus anthracis, Yersinia pestis, Clostridium botulinum, campylobacter jejuni, Chlamydia trachomatus, Chlamydia pneumoniae* or any species falling within the genera of any of the above species.

Another embodiment of the present invention is a host cell containing the vectors described above.

Another embodiment of the present invention is a purified or isolated nucleic acid consisting essentially of the coding sequence of one of SEQ ID NOs: 82-88, 90-242. One aspect of this embodiment is a fragment of the nucleic acid comprising at least 10, at least 20, at least 25, at least 30, at least 50 or more than 50 consecutive bases of one of SEQ ID NOs: 82-88, 90-242.

Another embodiment of the present invention isa vector comprising a promoter operably linked to the nucleic acids of the preceding embodiment.

Another aspect of the present invention is a purified or isolated nucleic acid comprising a nucleic acid sequence complementary to at least a portion of an intragenic sequence, intergenic sequence, sequences spanning at least a portion of two or more genes, 5' noncoding region, or 3' noncoding region within an operon encoding a polypeptide comprising a sequence selected from the group consisting of SEQ ID NOs: 243-357, 359-398.

Another embodiment of the present invention is a purified or isolated nucleic acid comprising a nucleic acid having at least 70% homology to a sequence selected from the group consisting of SEQ ID NOs 1-81, 405-485, 82-88, 90-242 or the sequences complementary thereto as determined using BLASTN version 2.0 with the default parameters. The nucleic acid may be from an organism selected from the group consisting of *Staphylococcus aureus, Pseudomonas aeruginosa, Enterobacter cloacae, Helicobacter pylori, Neisseria gonorrhoeae, Enterococcus faecalis, Streptococcus pneumoniae, Haemophilus influenzae, Salmonella typhimurium, Saccharomyces cerevisiae, Candida albicans, Cryptococcus neoformans, Aspergillus fumigatus, Klebsiella pneumoniae, Salmonella typhi, Salmonella paratyphi*, *Salmonella cholerasuis*, *Staphylococcus epidermidis, Mycobacterium tuberculosis, Mycobacterium leprae, Treponema pallidum, Bacillus anthracis, Yersinia pestis, Clostridium botulinum, campylobacter jejuni, and Chlamydia trachomatus, Chlamydia pneumoniae* or any species falling within the genera of any of the above species.

Another embodiment of the present invention is a purified or isolated nucleic acid consisting essentially of a nucleic acid encoding a polypeptide having a sequence selected from the group consisting of SEQ ID NOs.: 243-357, 359-398.

Another embodiment of the present invention is a vector comprising a promoter operably linked to a nucleic acid encoding a polypeptide having a sequence selected from the group consisting of SEQ ID NOs.: 243-357, 359-398.

Another embodiment of the present invention is a host cell containing the vector of the preceding embodiment.

Another embodiment of the present invention is purified or isolated polypeptide comprising the sequence of one of SEQ ID NOs: 243-357, 359-398.

Another embodiment of the present invention is purified or isolated polypeptide comprising a fragment of one of the polypeptides of SEQ ID NOs. 243-357, 359-398, said fragment selected from the group consisting of fragments comprising at least 5, at least 10, at least 20, at least 30, at least 40, at least 50, at least 60 or more than 60 consecutive amino acids of one of the polypeptides of SEQ ID NOs.: 243-357, 359-398.

Another embodiment of the present invention is an antibody capable of specifically binding the polypeptide of the preceding embodiment.

Another embodiment of the present invention is method of producing a polypeptide, comprising introducing a vector comprising a promoter operably linked to a nucleic acid encoding a polypeptide having a sequence selected from the group consisting of SEQ ID NOs. 243-357, 359-398into a cell. The method may further comprise the step of isolating said protein.

Another embodiment of the present invention is a method of inhibiting proliferation comprising inhibiting the activity or reducing the amount of a polypeptide having a sequence selected from the group consisting of SEQ ID NOs. 243-357, 359-398 or inhibiting the activity or reducing the amount of a nucleic acid encoding said polypeptide.

Another embodiment of the present invention is method for identifying compounds which influence the activity of a polypeptide required for proliferation comprising:
contacting a polypeptide comprising a sequence selected from the group consisting of 243-357, 359-398with a candidate compound; and
determining whether said compound influences the activity of said polypeptide.

The activity may be an enzymatic activity. The activity may be a carbon compound catabolism activity. The activity may be a biosynthetic activity. The activity may be a transporter activity. The activity may be a transcriptional activity. The activity may be a DNA replication activity. The activity may be a cell division activity.

Another embodiment of the present invention is a compound identified using the above method.

Another embodiment of the present invention is method for assaying compounds for the ability to reduce the activity or level of a polypeptide required for proliferation, comprising:
providing a target, wherein said target comprises the coding sequence of a sequence selected from the group consisting of SEQ ID NOs. 82-88, 90-242;
contacting said target with a candidate compound; and
measuring an activity of said target.

The target may be a messenger RNA molecule transcribed from a coding region of one of SEQ ID. NOs.: 82-88, 90-242 and said activity is translation of said messenger RNA. The target may be a coding region of one of SEQ ID. NOs. 82-88, 90-242 and said activity is transcription of said messenger RNA.

Another embodiment of the present invention is a compound identified using the method above.

Another embodiment of the present invention is a method for identifying compounds which reduce the activity or level of a gene product required for cell proliferation comprising the steps of:
expressing an antisense nucleic acid against a nucleic acid encoding said gene product in a cell to reduce the activity or amount of said gene product in said cell, thereby producing a sensitized cell;
contacting said sensitized cell with a compound; and
determining whether said compound inhibits the growth of said sensitized cell to a greater extent than said compound inhibits the growth of a nonsensitized cell.

The cell may be selected from the group consisting of bacterial cells, fungal cells, plant cells, and animal cells. The cell may be an *E*. *coli* cell. The cell may be from an organism selected from the group consisting of *Staphylococcus aureus, Pseudomonas aeruginosa, Enterobacter cloacae, Helicobacter pylori, Neisseria gonorrhoeae, Enterococcus faecalis, Streptococcus pneumoniae, Haemophilus influenzae, Salmonella typhimurium, Saccharomyces cerevisiae, Candida albicans, Cryptococcus neoformans, Aspergillus fumigatus, Klebsiella pneumoniae, Salmonella typhi, Salmonella paratyphi, Salmonella cholerasuis, Staphylococcus epidermidis, Mycobacterium tuberculosis, Mycobacterium leprae, Treponema pallidum, Bacillus anthracis, Yersinia pestis, Clostridium botulinum, campylobacter jejuni, and Chlamydia trachomatus, Chlamydia pneumoniae* or any species falling within the genera of any of the above species. The antisense nucleic acid may be transcribed from an inducible promoter. The method may, further comprise the step of contacting said cell with a concentration of inducer which induces said antisense nucleic acid to a sublethal level. The sub-lethal concentration of said inducer may be such that growth inhibition is 8% or more. The inducer may be isopropyl-1-thio-β-D-galactoside. The growth inhibition may be measured by monitoring optical density of a culture growth solution. The gene product may be a polypeptide. The gene product may be an RNA. The gene product may comprise a polypeptide having a sequence selected from the group consisting of SEQ ID NOs.: 243-357, 359-398.

Another embodiment of the present invention is a compound identified using the method above.

Another embodiment of the present invention is a method for inhibiting cellular proliferation comprising introducing a compound with activity against a gene corresponding to one of SEQ ID NOs.: 82-88, 90-242 or with activity against the product of said gene into a population of cells expressing a gene. The compound may be an antisense oligonucleotide comprising a sequence selected from the group consisting of SEQ ID NOs.: 1-81, 405-485, or a proliferation-inhibiting portion thereof. The proliferation inhibiting portion of one of SEQ ID NOs. 1-81, 405-485 may be a fragment comprising at least 10, at least 20, at least 25, at least 30, at least 50 or more than 50 consecutive bases of one of SEQ ID NOs: 1-81, 405-485. The compound may be a triple helix oligonucleotide.

Another embodiment of the present invention is a preparation comprising an effective concentration of an antisense oligonucleotide comprising a sequence selected from the group consisting of SEQ ID NOs.: 1-81, 405-485, or a proliferation-inhibiting portion thereof in a pharmaceutically acceptable carrier. The proliferation-inhibiting portion of one of SEQ ID NOs. 1-81, 405-485 may comprise at least 10, at least 20, at least 25, at least 30, at least 50 or more than 50 consecutive bases of one of SEQ ID NOs: 1-81, 405-485.

Another embodiment of the present invention is a method for inhibiting the expression of a gene in an operon required for proliferation comprising contacting a cell in a cell population with an antisense nucleic acid, said cell expressing a gene corresponding to one of SEQ ID NOs.: 82-88, 90-242, wherein said antisense nucleic acid comprises at least a proliferation-inhibiting portion of said operon in an antisense orientation that is effective in inhibiting expression of said gene. The antisense nucleic acid may be complementary to a sequence of a gene comprising one or more of SEQ ID NOs.: 82-88, 90-242. The antisense nucleic acid may be a sequence of one of SEQ ID NOs.: 1-81, 405-485, or a portion thereof. The cell may be contacted with said antisense nucleic acid by introducing a plasmid which expresses said antisense nucleic acid into said cell population. The cell may be contacted with said antisense nucleic acid by introducing a phage which expresses said antisense nucleic acid into said cell population. The cell may be contacted with said antisense nucleic acid by introducing a sequence encoding said antisense nucleic acid into the chromosome of said cell into said cell population. The cell may be contacted with said antisense nucleic acid by introducing a retron which expresses said antisense nucleic acid into said cell population. The cell may be contacted with said antisense nucleic acid by introducing a ribozyme into said cell-population, wherein a binding portion of said ribozyme is complementary to said antisense oligonucleotide. The cell may be contacted with said antisense nucleic acid by introducing a liposome comprising said antisense oligonucleotide into said cell. The cell may be contacted with said antisense nucleic acid by electroporation. The antisense nucleic acid may be a fragment comprising at least 10, at least 20, at least 25, at least 30, at least 50 or more than 50 consecutive bases of one of SEQ ID NOs: 82-88, 90-242. The antisense nucleic acid may be an oligonucleotide.

Another embodiment of the present invention is a method for identifying bacterial strains comprising the steps of:
providing a sample containing a bacterial species; and
identifying a bacterial species using a species specific probe having a sequence selected from the group consisting of SEQ 10 NOs. 1-81,405-485, 82-88, 90-242.

Another embodiment of the present invention is a method for identifying a gene in a microorganism required for proliferation comprising:
(a) identifying an inhibitory nucleic acid which inhibits the activity of a gene or gene product required for proliferation in a first microorganism;
(b) contacting a second microorganism with said inhibitory nucleic acid;
(c) determining whether said inhibitory nucleic acid from said first microorganism inhibits proliferation of said second microorganism; and
(d) identifying the gene in said second microorganism which is inhibited by said inhibitory nucleic acid.

Another embodiment of the present invention is a method for assaying a compound for the ability to inhibit proliferation of a microorganism comprising:
(a) identifying a gene or gene product required for proliferation in a first microorganism;
(b) identifying a homolog of said gene or gene product in a second microorganism;
(c) identifying an inhibitory nucleic acid sequence which inhibits the activity of said homolog in said second microorgansim;
(d) contacting said second microorganism with a proliferation-inhibiting amount of said inhibitory nucleic acid, thus sensitizing said second microorganism;
(e) contacting the sensitized microorganism of step (dl with a compound; and
(f) determining whether said compound inhibits proliferation of said sensitized microorganism to a greater extent than said compound inhibits proliferation of a nonsensitized microorganism.

The step of identifying a gene involved in proliferation in a first microorganism may comprise:
introducing a nucleic acid comprising a random genomic fragment from said first microorganism operably linked to a promoter wherein said random genomic fragment is in the antisense orientation; and
comparing the proliferation of said first microorganism transcribing a first level of said random genomic fragment to the proliferation of said first microorganism transcribing a lower level of said random genomic fragment, wherein a difference in proliferation indicates that said random genomic fragment comprises a gene involved in proliferation.

The step of identifying a homolog of said gene in a second microorganism may comprise identifying a homologous nucleic acid or a nucleic acid encoding a homologous polypeptide in a database using an algorithm selected from the group consisting of BLASTN version 2.0 with the default parameters and FASTA version 3.0t78 algorithm with the default parameters. The step of identifying a homolog of said gene in a second microorganism may comprise identifying a homologous nucleic acid or a nucleic acid encoding a homologous polypeptide by identifying nucleic acids which hybridize to said first gene. The step of identifying a homolog of said gene in a second microorganism may comprise expressing a nucleic acid which inhibits the proliferation of said first microorganism in said second microorganism. The inhibitory nucleic acid may be an antisense nucleic acid. The inhibitory nucleic acid may comprise an antisense nucleic acid to a portion of said homolog. The inhibitory nucleic acid may comprise an antisense nucleic acid to a portion of the operon encoding said homolog. The step of contacting the second microorganism with a proliferation-inhibiting amount of said nucleic acid sequence may comprise directly contacting said second microorganism with said nucleic acid. The step of contacting the second microorganism with a proliferation-inhibiting amount of said nucleic acid sequence may comprise expressing an antisense nucleic acid to said homolog in said second microorganism.

Another embodiment of the present invention is a compound identified using the method above.

Another embodiment of the present invention is a method of assaying a compound for the ability to inhibit proliferation comprising:
(a) identifying an inhibitory nucleic acid sequence which inhibits the activity of a gene or gene product required for proliferation in a first microorgansim;
(b) contacting a second microorganism with a proliferation-inhibiting amount of said inhibitory nucleic acid, thus sensitizing said second microorganism;
(c) contacting the proliferation-inhibited microorganism of step (b) with a compound; and
(d) determining whether said compound inhibits proliferation of said sensitized second microorganism to a greater extent than said compound inhibits proliferation of a nonsensitized second microorganism.

The inhibitory nucleic acid may be an antisense nucleic acid which inhibits the proliferation of said first microorganism. The inhibitory nucleic acid may comprise a portion of an antisense nucleic acid which inhibits the proliferation of said first microorganism. The inhibitory nucleic acid may comprise an antisense molecule against the entire coding region of the gene involved in proliferation of the first microorganism. The inhibitory nucleic acid may comprise an antisense nucleic acid to a portion of the operon encoding the gene involved in proliferation of the first microorganism.

Another embodiment of the present invention is a compound identified using the method above.

Another embodiment of the present invention is a method for assaying compounds for activity against a biological pathway required for proliferation comprising:
sensitizing a cell by expressing an antisense nucleic acid against a nucleic acid encoding a gene product required for proliferation in a cell to reduce the activity or amount of said gene product;
contacting the sensitized cell with a compound; and
determining whether said compound inhibits the growth of said sensitized cell to a greater extent than said compound inhibits the growth of an nonsensitized cell.

The cell may be selected from the group consisting of bacterial cells, fungal cells, plant cells, and animal cells. The cell may be an *E*. *coli* cell. The cell may be an organism selected from the group consisting of *Staphylococcus aureus, Pseudomonas aeruginosa, Enterobacter cloacae, Helicobacter pylori, Neisseria gonorrhoeae, Enterococcus faecalis, Streptococcus pneumoniae, Haemophilus influenzae, Salmonella typhimurium, Saccharomyces cerevisiae, Candida albicans, Cryptococcus neoformans, Aspergillus fumigatus, Klebsiella pneumoniae, Salmonella typhi, Salmonella paratyphi, Salmonella cholerasuis, Staphylococcus epidermidis, Mycobacterium tuberculosis, Mycobacterium leprae, Treponema pallidum. Bacillus anthracis, Yersinia pestis, Clostridium botulinum, campylobacter jejuni, and Chlamydia trachomatus, Chlamydia pneumoniae* or any species falling within the genera of any of the above species. The antisense nucleic acid may be transcribed from an inducible promoter. The method may further comprise contacting the cell with an agent which induces expression of said antisense nucleic acid from said inducible promoter, wherein said antisense nucleic acid is expressed at a sublethal level. The sublethal level of said antisense nucleic acid may inhibit proliferation by 8% or more. The agent may be isopropyl-1-thio-β-D-galactoside (IPTG). The inhibition of proliferation may be measured by monitoring the optical density of a liquid culture. The gene product may comprise a polypeptide having a sequence selected from the group consisting of SEQ ID NOs: 243-357, 359-398.

Another embodiment of the present invention is a compound identified using the method above.

Another embodiment of the present invention is a method for assaying a compound for the ability to inhibit cellular proliferation comprising:
contacting a cell with an agent which reduces the activity or level of a gene product required for proliferation of said cell;
contacting said cell with said compound; and
determining whether said compound reduces proliferation to a greater extent than said compound reduces proliferation of cells which have not been contacted with said agent.

The agent which reduces the activity or level of a gene product required for proliferation of said cell may comprise an antisense nucleic acid to a gene or operon required for proliferation. The agent which reduces the activity or level of a gene product required for proliferation of said cell may comprise an antibiotic. The cell may contain a temperature sensitive mutation which reduces the activity or level of said gene product required for proliferation of said cell. The antisense nucleic acid may be directed against the same functional domain of said gene product required for proliferation of said cell to which said antisense nucleic acid is directed. The antisense nucleic acid may be directed against a different functional domain of said gene product required for proliferation of said cell than the fucntional domain to which said antisense nucleic acid is directed.

Another embodiment of the present invention is a compound identified using the method above.

Another embodiment of the present invention is a method for identifying the pathway in which a proliferation-required nucleic acid or its gene product lies comprising:
expressing a sublethal level of an antisense nucleic acid directed against said proliferation-required nucleic acid in a cell;
contacting said cell with an antibiotic, wherein the a biological pathway on which said antibiotic acts is known; and
determining whether said cell has a substantially greater sensitivity to said antibiotic than a cell which does not express said sublethal level of said antisense nucleic acid.

Another embodiment of the present invention is a method for determining the pathway on which a test compound acts comprising:
(a) expressing a sublethal level of an antisense nucleic acid directed against a proliferation-required nucleic acid in a cell, wherein the biological pathway in which said proliferation-required nucleic acid lies is known,
(b) contacting said cell with said test compound; and
(c) determining whether said cell has a substantially greater sensitivity to said test compound than a cell which does not express said sublethal level of said antisense nucleic acid.
   The method may further comprise:
(d) expressing a sublethal level of a second antisense nucleic acid directed against a second proliferation-required nucleic acid in said cell, wherein said second proliferation-required nucleic acid is in a different biological pathway than said proliferation-required nucleic acid in step (a); and
(e) determining whether said cell has a substantially greater sensitivity to said test compound than a cell which does not express said sublethal level of said second antisense nucleic acid.

Another embodiment of the present invention is a purified or isolated nucleic acid consisting essentially of one of SEQ ID NOs: 358, 399-402.

Another embodiment of the present invention is a purified or isolated nucleic acid comprising a sequence selected from the group consisting of 1-81, 405-485, 82-88, 90-242, 358, 399-402.

Another embodiment of the present invention is a compound which interacts with the gene or gene product of a nucleic acid comprising a sequence of one of SEQ ID NOs: 82-88, 90-242 to inhibit proliferation.

Another embodiment of the present invention compound which interacts with a polypeptide comprising one of SEQ ID NOs. 243-357, 359-398 to inhibit proliferation.

Another embodiment of the present invention is a compound which interacts with a nucleic acid comprising one of SEQ ID NOs: 358, 399-402 to inhibit proliferation.

### BRIEF DESCRIPTION OF THE DRAWINGS

Figure 1 is an IPTG dose response curve in *E*. *coli* transformed with an IPTG-inducible plasmid containing either an antisense clone to the *E*. *coli* ribosomal protein rplW (AS-rplW) which is required for protein synthesis and essential cell proliferation, or an antisense clone to the elaD (AS-elaD) gene which is not known to be involved in protein synthesis and which is also essential for proliferation.
Figure 2A is a tetracycline dose response curve in *E. coli* transformed with an IPTG-inducible plasmid containing antisense to rplW(AS-rplW) in the presence of 0, 20 or 50 µM IPTG.
Figure 2B is a tetracycline dose response curve in *E*. *coli* transformed with an IPTG-inducible plasmid containing antisense to elaD (AS-elaD) in the presence of 0, 20 or 50 µM IPTG.
Figure 3 is a graph showing the fold increase in tetracycline sensitivity of *E. coli* transfected with antisense clones to essential ribosomal proteins L23 (AS-rplW) and L7/L12 and L10 (AS-rplLrplJ). Antisense clones to genes known not to be involved in protein synthesis (atpB/E(AS-atpB/E ), visC (AS-visC, elaD (AS-elaD), yohH (AS-yohH) are much less sensitive to tetracycline.

### Definitions

By "biological pathway" is meant any discrete cell function or process that is carried out by a gene product or a subset of gene products. Biological pathways include enzymatic, biochemical and metabolic pathways as well as pathways involved in the production of cellular structures such cell walls. Biological pathways that are usually required for proliferation of microorganisms include, but are not limited to, cell division, DNA synthesis & replication, RNA synthesis (transcription), protein synthesis (translationl, protein processing, protein transport, fatty acid biosynthesis, cell wall synthesis, cell membrane synthesis & maintenance, etc.

By "inhibit activity against a gene or gene product" is meant having the ability to interfere with the function of a gene or gene product in such a way as to decrease expression of the gene or to reduce the level or activity of a product of the gene. Agents which have activity against a gene include agents that inhibit transcription of the gene and agents that inhibit translation of the mRNA transcribed from the gene. In microorganisms, agents which have activity against a gene can act to decrease expression of the operon in which the gene resides or alter the processing of operon RNA such as to reduce the level or activity of the gene product. The gene product can be a non-translated RNA such as ribosomal RNA, a translated RNA (mRNA) or the protein product resulting from translation of the gene mRNA. Of particular utility to the present invention are anti-sense RNAs that have activities against the operons or genes to which they specifically hybridze.

By "activity against a gene product" is meant having the ability to inhibit the function or to reduce the level or activity of the gene product in a cell.

By "activity against a protein" is meant having the ability to inhibit the function or to reduce the level or activity of the protein in a cell.

By "activity against nucleic acid" is meant having the ability to inhibit the function or to reduce the level or activity of the nucleic acid in a cell.

As used herein, "sublethal" means a concentration of an agent below the concentration required to inhibit all cell growth.

### DETAILED DESCRIPTION OF THE INVENTION

The present invention describes a group of *E*. *coli* genes and gene families required for growth and/or proliferation. A proliferation-required gene or gene family is one where, in the absence of a gene transcript and/or gene product, growth or viability of the microorganism is reduced or eliminated. Thus, as used herein the terminology "proliferation-required" or "required for proliferation" encompasses sequences where the absence of a gene transcript and/or gene product completely eliminates cell growth as well as sequences where the absence of a gene transcript and/or gene product merely reduces cell growth. These proliferation-required genes can be used as potential targets for the generation of new antimicrobial agents. To achieve that goal, the present invention also encompasses novel assays for analyzing proliferation-required genes and for identifying compounds which interact with the gene products of the proliferation-required genes. In addition, the present invention contemplates the expression of genes and the purification of the proteins encoded by the nucleic acid sequences identified as required proliferation genes and reported herein. The purified proteins can be used to generate reagents and screen small molecule libraries or other candidate compound libraries for compounds that can be further developed to yield novel antimicrobial compounds. The present invention also describes methods for identification of homologous genes in organisms other than *E*. *coil.*

The present invention utilizes a novel method to identify proliferation-required *E*. *coli* sequences. Generally, a library of nucleic acid sequences from a given source are subcloned or otherwise inserted into an inducible expression vector, thus forming an expression library. Although the insert nucleic acids may be derived from the chromosome of the organism into which the expression vector is to be introduced, because the insert is not in its natural chromosomal location, the insert nucleic acid is an exogenous nucleic acid for the purposes of the discussion herein. The term expression is defined as the production of an RNA molecule from a gene, gene fragment, genomic fragment, or operon. Expression can also be used to refer to the process of peptide or polypeptide synthesis. An expression vector is defined as a vehicle by which a ribonucleic acid (RNA) sequence is transcribed from a nucleic acid sequence carried within the expression vehicle. The expression vector can also contain features that permit translation of a protein product from the transcribed RNA message expressed from the exogenous nucleic acid sequence carried by the expression vector. Accordingly, an expression vector can produce an RNA molecule as its sole product or the expression vector can produce a RNA molecule that is ultimately translated into a protein product.

Once generated, the expression library containing the exogenous nucleic acid sequences is introduced into an *E*. *coli* population to search for genes that are required for bacterial proliferation. Because the library molecules are foreign to the population of *E*. *coli*, the expression vectors and the nucleic acid segments contained therein are considered exogenous nucleic acid.

Expression of the exogenous nucleic acid fragments in the test population of *E*. *coli* containing the expression vector library is then activated. Activation of the expression vectors consists of subjecting the cells containing the vectors to conditions that result in the expression of the exogenous nucleic acid sequences carried by the expression vector library. The test population of *E*. *coli* cells is then assayed to determine the effect of expressing the exogenous nucleic acid fragments on the test population of cells. Those expression vectors that, upon activation and expression, negatively impact the growth of the *E. coil* screen population were identified, isolated, and purified for further study.

A variety of assays are contemplated to identify nucleic acid sequences that negatively impact growth upon expression. In one embodiment, growth in *E. coli* cultures expressing exogenous nucleic acid sequences and growth in cultures not expressing these sequences is compared. Growth measurements are assayed by examining the extent of growth by measuring optical densities. Alternatively, enzymatic assays can be used to measure bacterial growth rates to identify exogenous nucleic acid sequences of interest. Colony size, colony morphology, and cell morphology are additional factors used to evaluate growth of the host cells. Those cultures that failed to grow or grow with reduced efficiency under expression conditions are identified as containing an expression vector encoding a nucleic acid fragment that negatively affects a proliferation-required gene.

Once exogenous nucleic acid sequences of interest are identified, they are analyzed. The first step of the analysis is to acquire the nucleic acid sequence of the nucleic acid fragment of interest. To achieve this end, the insert in those expression vectors identified as containing a sequence of interest is sequenced, using standard techniques well known in the art. The next step of the process is to determine the source of the nucleic acid sequence.

Determination of sequence source is achieved by comparing the obtained sequence data with known sequences in various genetic databases. The sequences identified are used to probe these gene databases. The result of this procedure is a list of exogenous nucleic acid sequences corresponding to a list that includeds novel bacterial genes required for proliferation as well as genes previously identified as required for proliferation.

The number of DNA and protein sequences available in database systems has been growing exponentially for years. For example, at the end of 1998, the complete sequences of *Caenorhabditis elegans, Saccharomyces cerevisiae* and nineteen bacterial genomes, including *E*. *coli* were available. This sequence information is stored in a number of databanks, such as GenBank (the National Center for Biotechnology Information (NCBI), and is publicly available for searching.

A variety of computer programs are available to assist in the analysis of the sequences stored within these databases. FastA, (W. R. Pearson (1990) "Rapid and Sensitive Sequence Comparison with FASTP and FASTA" Methods in Enzymology 183:63- 98), Sequence Retrieval System (SRS), (Etzold & Argos, SRS an indexing and retrieval tool for flat file data libraries. Comput. Appl. Biosci. 9:49-57, 1993) are two examples of computer programs that can be used to analyze sequences of interest. In one embodiment of the present invention, the BLAST family of computer programs, which includes BLASTN version 2.0 with the default parameters, or BLASTX version 2.0 with the default parameters, is used to analyze nucleic acid sequences.

BLAST, an acronym for "Basic Local Alignment Search Tool," is a family of programs for database similarity searching. The BLAST family of programs includes: BLASTN, a nucleotide sequence database searching program, BLASTX, a protein database searching program where the input is a nucleic acid sequence; and BLASTP, a protein database searching program. BLAST programs embody a fast algorithm for sequence matching, rigorous statistical methods for judging the significance of matches, and various options for tailoring the program for special situations. Assistance in using the program can be obtained by e-mail at blast@ncbi.nlm.nih.gov.

Bacterial genes are often transcribed in polycistronic groups. These groups comprise operons, which are a collection of genes and intergenic sequences. The genes of an operon are co-transcribed and are often related functionally. Given the nature of the screening protocol, it is possible that the identified exogenous nucleic acid sequence corresponds to a gene or portion thereof with or without adjacent noncoding sequences, an intragenic sequence (i.e. a sequence within a gene), an intergenic sequence (i.e. a sequence between genes), a sequence spanning at least a portion of two or more genes, a 5' noncoding region or a 3' noncoding region located upstream or downstream from the actual sequence that is required for bacterial proliferation. Accordingly, determining which of the genes that are encoded within the operons are individually required for proliferation is often desirable.

In one embodiment of the present invention, an operon is dissected to determine which gene or genes are required for proliferation. For example, the RegulonDB DataBase described by Huerta et al. (*Nucl. Acids Res.* **26**:55-59, 1998), which may also be found on the website http://www.cifn.unam.mx/Computational_Biology/regulondb/, may be used. to identify the boundaries of operons encoded within microbial genomes. A number of techniques that are well known in the art can be used to dissect the operon. In one aspect of this embodiment, gene disruption by homologous recombination is used to individually inactivate the genes of an operon that is thought to contain a gene required for proliferation.

Several gene disruption techniques have been described for the replacement of a functional gene with a mutated, non-functional (null) allele. These techniques generally involve the use of homologous recombination. The method described by Link et al. (J. Bacteriol 1997 179:6228; incorporated herein by reference in it's entirety) serves as an excellent example of these methods as applicable to disruption of genes in *E*. *coli*. This technique uses crossover PCR to create a null allele with an in-frame deletion of the coding region of a target gene. The null allele is constructed in such a way that sequences adjacent to the wild type gene (ca. 500 bp) are retained. These homologous sequences surrounding the deletion null allele provide targets for homologous recombination so that the wild type gene on the *E*. *coli* chromosome can be replaced by the constructed null allele.

The crossover PCR amplification product is subcloned into the vector pK03, the features of which include a chloramphenicol resistance gene, the counter-selectable marker *sacB*, and a temperature sensitive autonomous replication function. Following transformation of an *E*. *coil* cell population with such a vector, selection for cells that have undergone homologous recombination of the vector into the chromosome is achieved by growth on chloramphenicol at the non-permissive temperature of 43°C. Under these conditions, autonomous replication of the plasmid cannot occur and cell are resistant to chloramphinicol only if the chloramphenicol resistance gene has been integrated into the chromosome. Usually a single crossover event is responsible for this integration event such that the *E*. *coli* chromosome now contains a tandem duplication of the target gene consisting of one wild type allele and one deletion null allele separated by vector sequence.

This new *E*. *coli* strain containing the tandem duplication can be maintained at permissive temperatures in the presence of drug selection (chloramphenicol). Subsequently, cells of this new strain are cultured at the permissive temperature 30°C without drug selection. Under these conditions, the chromosome of some of the cells within the population will have undergone an internal homologous recombination event resulting in removal of the plasmid sequences. Subsequent culturing of the strain in growth medium lacking chloramphenicol but containing sucrose is used to select for such recombinative resolutions. In the presence of the counter-selectable marker *sacB*, sucrose is rendered into a toxic metabolite. Thus, cells that survive this counter-selection have lost both the plasmid sequences from the chromosome and the autonomously replicating plasmid that results as a byproduct of recombinative resolution.

There are two possible outcomes of the above recombinative resolution via homologous recombination. Either the wild type copy of the targeted gene is retained on the chromosome or the mutated null allele is retained on the chromosome. In the case of an essential gene, a single copy of the null allele would be lethal and such cells should not be obtained by the above procedure when applied to essential genes. In the case of a non-essential gene, roughly equal numbers of cells containing null alleles and cells containing wild type alleles should be obtained. Thus, the method serves as a test for essentiality of the targeted gene: when applied to essential genes, only cells with a wild type allele on the chromosome will be obtained.

Other techniques have also been described for the creation of disruption mutations in *E. coli*. For example, Link et al. also describe inserting an in-frame sequence tag concommitantly with an in-frame deletion in order to simplify analysis of recombinants obtained. Further, Link et al. describe disruption of genes with a drug resistance marker such as a kanamycin resistance gene. Arigoni et al., (Arigoni, F. et al. A Genome-based Approach for the Identification of Essential Bacterial Genes, Nature Biotechnology 16: 851-856, the disclosure of which is incorporated herein by reference in its entirety) describe the use of gene disruption combined with engineering a second copy of a test gene such that the expression of the gene is regulated by and inducible promoter such as the arabinose promoter to test the essentiality of the gene. Many of these techniques result in the insertion of large fragments of DNA into the gene of interest, such as a drug selection marker. An advantage of the technique described by Link et al. is that it does not rely on an insertion into the gene to cause a functional defect, but rather results in the precise removal of the coding region. This insures the lack of polar effects on the expression of genes downstream from the target gene.

Recombinant DNA techniques can be used to express the entire coding sequences of the gene identified as required for proliferation, or portions thereof. The over-expressed proteins can be used as reagents for further study. The identified exogenous sequences are isolated, purified, and cloned into a suitable expression vector using methods well known in the art. If desired, the nucleic acids can contain the sequences encoding a signal peptide to facilitate secretion of the expressed protein.

Expression of fragments of the bacterial genes identified as required for proliferation is also contemplated by the present invention. The fragments of the identified genes can encode a polypeptide comprising at least 5, at least 10, at least 15, at least 20, at least 25, at least 30, at least 35, at least 40, at least 45, at least 50, at least 55, at least 60, at least 65, at least 75, or more than 75 consecutive amino acids of a gene complementary to one of the identified sequences of the present invention. The nucleic acids inserted into the expression vectors can also contain sequences upstream and downstream of the coding sequence.

When expressing the coding sequence of an entire gene identified as required for bacterial proliferation or a fragment thereof, the nucleic acid sequence to be expressed is operably linked to a promoter in an expression vector using conventional cloning technology. The expression vector can be any of the bacterial, insect, yeast, or mammalian expression systems known in the art. Commercially available vectors and expression systems are available from a variety of suppliers including Genetics Institute (Cambridge, MA), Stratagene (La Jolla, California), Promega (Madison, Wisconsin), and Invitrogen (San Diego, California). If desired, to enhance expression and facilitate proper protein folding, the codon usage and codon bias of the sequence can be optimized for the particular expression organism in which the expression vector is introduced, as explained by Hatfield, et al., U.S. Patent No. 5,082,767, incorporated herein by this reference. Fusion protein expression systems are also contemplated by the present invention.

Following expression of the protein encoded by the identified exogenous nucleic acid sequence, the protein is purified. Protein purification techniques are well known in the art. Proteins encoded and expressed from identified exogenous nucleic acid sequences can be partially purified using precipitation techniques, such as precipitation with polyethylene glycol. Chromatographic methods usable with the present invention can include ion-exchange chromatography, gel filtration, use of hydroxyapaptite columns, immobilized reactive dyes, chromatofocusing, and use of high-performance liquid chromatography. Electrophoretic methods such one-dimensional gel electrophoresis, high-resolution two-dimensional polyacrylamide electrophoresis, isoelectric focusing, and others are contemplated as purification methods. Also, affinity chromatographic methods, comprising antibody columns, ligand presenting columns and other affinity chromatographic matrices are contemplated as purification methods in the present invention.

The purified proteins produced from the gene coding sequences identified as required for proliferation can be used in a variety of protocols to generate useful antimicrobial reagents. In one embodiment of the present invention, antibodies are generated against the proteins expressed from the identified exogenous nucleic acid sequences. Both monoclonal and polyclonal antibodies can be generated against the expressed proteins. Methods for generating monoclonal and polyclonal antibodies are well known in the art. Also, antibody fragment preparations prepared from the produced antibodies discussed above are contemplated.

Another application for the purified proteins of the present invention is to screen small molecule libraries for candidate compounds active against the various target proteins of the present invention. Advances in the field of combinatorial chemistry provide methods, well known in the art, to produce large numbers of candidate compounds that can have a binding, or otherwise inhibitory effect on a target protein. Accordingly, the screening of small molecule libraries for compounds with binding affinity or inhibitory activity for a target protein produced from an identified gene sequence is contemplated by the present invention.

The present invention further contemplates utility against a variety of other pathogenic organisms in addition to *E*. *coli*. For example, the invention has utility in identifying genes required for proliferation in prokaryotes and eukaryotes. For example, the invention has utility with protists, such as *Plasmodium* spp.; plants; animals, such as *Entamoeba* spp. *and Contracaecum* spp; and fungi including *Candida* spp., (e.g., *Candida albicans*), *Saccharomyces cerevisiae*, *Cryptococcus neoformans,* and *Aspergillus fumigatus*. In one embodiment of the present invention, monera, specifically bacteria are probed in search of novel gene sequences required for proliferation. This embodiment is particularly important given the rise of drug resistant bacteria.

The numbers of bacterial species that are becoming resistant to existing antibiotics are growing. A partial list of these organisms includes: *Staphylococcus* spp., such as *S*, *aureus*; *Enterococcus* spp., such as *E*. *faecalis; Pseudomonas* spp., *such as P. aeruginosa, Clostridium* spp., such as *C*. *botulinum*, *Haemophilus* spp., such as *H. influenzae, Enterobacter* spp., such as *E*. *cloacae, Vibrio* spp., such as *V. cholera; Moraxala* spp., such as *M. catarrhalis*; *Streptococcus* spp., such as *S. pneumoniae*, *Neisseria* spp., such as *N. gonorrhoeae; Mycoplasma* spp., such as *Mycoplasma pneumoniae; Salmonella typhimurium; Helicobacter pylori; Escherichia coli;* and *Mycobacterium tuberculosis*. The sequences identified as required for proliferation in the present invention can be used to probe these and other organisms to identify homologous required proliferation genes contained therein.

In one embodiment of the present invention, the nucleic acid sequences disclosed herein are used to screen genomic libraries generated from bacterial species of interest other than *E*. *coli.* For example, the genomic library may be from *Staphylococcus aureus, Pseudomonas aeruginosa, Enterobacter cloacae, Heilcobacter pylori, Neisseria gonorrhoeae, Enterococcus faecalis, Streptococcus pneumoniae, Haemophilus influenzae, Salmonella typhimurium, Saccharomyces cerevisiae, Candida albicans, Cryptococcus neoformans, Aspergillus fumigatus, Klebsiella pneumoniae, Salmonella typhi, Salmonella paratyphi, Salmonella cholerasuis, Staphylococcus epidermidis, Mycobacterium* *tuberculosis, Mycobacterium leprae, Treponema pallidum, Bacillus anthracis, Yersinia pestis, Clostridium botulinum, Campylobacter jejuni, Chlamydia trachomatus, Chlamydia pneumoniae* or any species falling within the genera of any of the above species. Standard molecular biology techniques are used to generate genomic libraries from various microorganisms. In one aspect, the libraries are generated and bound to nitrocellulose paper. The identified exogenous nucleic acid sequences of the present invention can then be used as probes to screen the libraries for homologous sequences. The homologous sequences identified can then be used as targets for the identification of new, antimicrobial compounds with activity against more than one organism.

For example, the preceding methods may be used to isolate nucleic acids having a sequence with at least 97%, at least 95%, at least 90%, at least 85%, at least 80%, or at least 70% identity to a nucleic acid sequence selected from the group consisting of one of the sequences of SEQ ID NOS. 1-81, 405-485, 82-88, 90-242, fragments comprising at least 10, 15, 20, 25, 30, 35, 40, 50, 75, 100, 150, 200, 300, 400, or 500 consecutive bases thereof, and the sequences complementary thereto. Identity may be measured using BLASTN version 2.0 with the default parameters. (Altschul, S.F. et al. Gapped BLAST and PSI-BLAST: A New Generation of Protein Database Search Programs, Nucleic Acid Res. 25: 3389-3402 (1997), the disclosure of which is incorporated herein by reference in its entirety). For example, the homologous polynucleotides may have a coding sequence which is a naturally occurring allelic variant of one of the coding sequences described herein. Such allelic variants may have a substitution, deletion or addition of one or more nucleotides when compared to the nucleic acids of SEQ ID NOs: 1-81, 405-485, 82-88, 90-242 or the sequences complementary thereto.

Additionally, the above procedures may be used to isolate nucleic acids which encode polypeptides having at least 99%, 95%, at least 90%, at least 85%, at least 80%, at least 70%, at least 60%, at least 50%, or at least 40% identity or similarity to a polypeptide having the sequence of one of SEQ ID NOs: 243-357, 359-398 or fragments comprising at least 5, 10, 15, 20, 25, 30, 35, 40, 50, 75, 100, or 150 consecutive amino acids thereof as determined using the FASTA version 3.0t78 algorithm with the default parameters. Alternatively, protein identity or similarity may be identified using BLASTP with the default parameters, BLASTX with the default parameters, or TBLASTN with the default parameters. (Alschul, S.F. et al. Gapped BLAST and PSI-BLAST: A New Generation of Protein Database Search Programs, Nucleic Acid Res. 25: 3389-3402 (1997), the disclosure of which is incorporated herein by reference in its entirety).

Alternatively, homologous nucleic acids or polypeptides may be identified by searching a database to identify sequences having a desired level of homology to a nucleic acid or polypeptide involved in proliferation or an antisense nucleic acid to a nucleic acid involved in microbial proliferation. A variety of such databases are available to those skilled in the art, including GenBank and GenSeq. In some embodiments, the databases are screened to identify nucleic acids or polypeptides having at least 97%, at least 95%, at least 90%, at least 85%, at least 80%, at least 70%, at least 60%, or at least 50%, at least 40% identity or similarity to a nucleic acid or polypeptide involved in proliferation or an antisense nucleic acid involved in proliferation. For example, the database may be screened to identify nucleic acids homologous to one of SEQ ID Nos. 1-81, 405-485, 82-88, 90-242 or polypeptides homologous to SEQ ID NOs. 243-357, 359-398. In some embodiments, the database may be screened to identify homologous nucleic acids or polypeptides from organisms other than *E. coli*, including organisms such as *Staphylococcus aureus*, *Pseudomonas aeruginosa, Enterobacter cloacae, Helicobacter pylori, Neisseria gonorrhoeae, Enterococcus faecalis, Streptococcus pneumoniae, Haemophilus influenzae*, *Salmonella typhimurium*, *Saccharomyces cerevisiae*, *Candida albicans*, *Cryptococcus neoformans*, *Aspergillus fumigatus*, *Klebsiella pneumoniae*, *Salmonella typhi*, *Salmonella paratyphi*, *Salmonella cholerasuis*, *Staphylococcus epidermidis*, *Mycobacterium tuberculosis, Mycobacterium leprae, Treponema pallidum*, *Bacillus anthracis, Yersinia pestis, Clostridium botulinum, Campylobacter jejuni, Chlamydia trachomatus, Chlamydia pneumoniae* or any species falling within the genera of any of the above species.

In another embodiment, gene expression arrays and microarrays can be employed. Gene expression arrays are high density arrays of DNA samples deposited at specific locations on a glass chip, nylon membrane, or the like. Such arrays can be used by researchers to quantify relative gene expression under different conditions. Gene expression arrays are used by researchers to help identify optimal drug targets, profile new compounds, and determine disease pathways. An example of this technology is found in U.S. Patent No. 5807522, which is hereby incorporated by reference.

It is possible to study the expression of all genes in the genome of a particular microbial organism using a single array. For example, the arrays from Genosys consist of 12 x 24 cm nylon filters containing PCR products corresponding to 4290 ORFs from *E*. *coli.* 10 ngs of each are spotted every 1.5 mm on the filter. Single stranded labeled cDNAs are prepared for hybridization to the array (no second strand synthesis or amplification step is done) and placed in contact with the filter. Thus the labeled cDNAs are of "antisense" orientation. Quantitative analysis is done by phosphorimager.

Hybridization of cDNA made from a sample of total cell mRNA to such an array followed by detection of binding by one or more of various techniques known to those in the art results in a signal at each location on the array to which cDNA hybridized. The intensity of the hybridization signal obtained at each location in the array thus reflects the amount of mRNA for that specific gene that was present in the sample. Comparing the results obtained for mRNA isolated from cells grown under different conditions thus allows for a comparison of the relative amount of expression of each individual gene during growth under the different conditions.

Gene expression arrays may be used to analyze the total mRNA expression pattern at various time points after induction of an antisense nucleic acid against a proliferation-required gene. Analysis of the expression pattern indicated by hybridization to the array provides information on whether or not the target gene of the antisense nucleic acid is being affected by antisense induction, how quickly the antisense is affecting the target gene, and for later timepoints, what other genes are affected by antisense expression. For example, if the antisense is directed against a gene for ribosomal protein L7/L12 in the 50S subunit, its targeted mRNA may disappear first and then other mRNAs may be observed to increase, decrease or stay the same. Similarly, if the antisense is directed against a different 50S subunit ribosomal protein mRNA (e.g. L25), that mRNA may disappear first followed by changes in mRNA expression that are similar to those seen with the L7/L12 antisense expression. Thus, the mRNA expression pattern observed with an antinsense nucleic acid against a proliferation required gene may identify other proliferation-required nucleic acids in the same pathway as the target of the antisense nucleic acid. In addition, the mRNA expression patterns observed with candidate drug compounds may be compared to those observed with antisense nucleic acids against a proliferation-required nucleic acid. If the mRNA expression pattern observed with the candidate drug compound is similar to that observed with the antisense nucleic acid, the drug compound may be a promising therapeutic candidate. Thus, the assay would be useful in assisting in the selection of candidate drug compounds for use in screening methods such as those described below.

In cases where the source of nucleic acid deposited on the array and the source of the nucleic acid being hybridized to the array are from two different organisms, gene expression arrays can identify homologous genes in the two organisms.

The present invention also contemplates additional methods for screening other microorganisms for proliferation-required genes. In this embodiment, the conserved portions of sequences identified as proliferation-required can be used to generate degenerate primers for use in the polymerase chain reaction (PCR). The PCR technique is well known in the art. The successful production of a PCR product using degenerate probes generated from the sequences identified herein would indicate the presence of a homologous gene sequence in the species being screened. This homologous gene is then isolated, expressed, and used as a target for candidate antibiotic compounds. In another aspect of this embodiment, the homologous gene is expressed in an autologous organism or in a heterologous organism in such a way as to alter the level or activity of a homologous gene required for proliferation in the autologous or heterologus organism. In still another aspect of this embodiment, the homologous gene or portion is expressed in an antisense orientation in such a way as to alter the level or activity of a nucleic acid required for proliferation of an autologous or heterologous organism.

The homologous sequences to proliferation-required genes identified using the techniques described herein may be used to identify proliferation-required genes of organisms other than *E*. *coli*, to inhibit the proliferation of organisms other than *E*. *coli* by inhibiting the activity or reducing the amount of the identified homologous nucleic acid or polypeptide in the organism other than *E*. *coli*, or to identify compounds which inhibit the growth of organisms other than E. coli as described below.

in another embodiment of the present invention, *E*. *coli* sequences identified as required for proliferation are transferred to expression vectors capable of function within non-*E coli* species. As would be appreciated by one of ordinary skill in the art, expression vectors must contain certain elements that are species specific. These elements can include promoter sequences, operator sequences, repressor genes, origins of replication, ribosomal binding sequences, termination sequences, and others. To use the identified exogenous sequences of the present invention, one of ordinary skill in the art would know to use standard molecular biology techniques to isolate vectors containing the sequences of interest from cultured bacterial cells, isolate and purify those sequences, and subclone those sequences into an expression vector adapted for use in the species of bacteria to be screened.

Expression vectors for a variety of other species are known in the art. For example, Cao et al. report the expression of steroid receptor fragments in *Staphylococcus aureus.* **J. Steroid Biochem Mol Biol.** 44(1):1-11 (1993). Also, Pla et al. have reported an expression vector that is functional in a number of relevant hosts including: *Salmonella typhimurium, Pseudomonas putida, and Pseudomonas aeruginosa.* **J. Bacteriol.** 172(8):4448-55 (1990). These examples demonstrate the existence of molecular biology techniques capable of constructing expression vectors for the species of bacteria of interest to the present invention.

Following the subcloning of the identified nucleic acid sequences into an expression vector functional in the microorganism of interest, the identified nucleic acid sequences are conditionally transcribed to assay for bacterial growth inhibition. Those expression vectors found to contain sequences that, when transcribed, inhibit bacterial growth are compared to the known genomic sequence of the pathogenic microorganism being screened or, if the homologous sequence from the organism being screened is not known, it may be identified and isolated by hybridization to the proliferation-required *E*. *coli* sequence interest or by amplification using primers based on the proliferation-required *E. coli* sequence of interest as described above.

The antisense sequences from the second organism which are identified as described above may then be operably linked to a promoter, such as an inducible promoter, and introduced into the second organism. The techniques described herein for identifying *E*. *coli* genes required for proliferation may thus be employed to determine whether the identified sequences from a second organism inhibit the proliferation of the second organism.

Antisense nucleic acids required for the proliferation of organisms other than *E*. *coli* or the genes corresponding thereto, may also be hybridized to a microarray containing the *E*. *coli* ORFs to gauge the homology between the *E*. *coli* sequences and the proliferation-required nucleic acids from other organisms. For example, the proliferation-required nucleic acid may be from *Staphylococcus aureus, Pseudomonas aeruginosa, Enterobacter cloacae, Helicobacter pylori, Neisseria gonorrhoeae, Enterococcus faecalis, Streptococcus pneumoniae, Haemophilus influenzae, Salmonella typhimurium, Saccharomyces cerevisiae, Candida albicans, Cryptococcus neoformans, Aspergillus fumigatus, Klebsiella pneumoniae, Salmonella typhi*, *Salmonella paratyphi, Salmonella cholerasuis, Staphylococcus epidermidis, Mycobacterium tuberculosis, Mycobacterium leprae, Treponema pallidum, bacillus anthracis, Yersinia pestis, Clostridium botulinum, Campylobacter jejuni or Chlamydia trachomatus, Chlamydia pneumoniae* or any species falling within the genera of any of the above species. The proliferation-required nucleic acids from an organism other than *E*. *coli* may be hybridized to the array under a variety of conditions which permit hybridization to occur when the probe has different levels of homology to the sequence on the microarray. This would provide an indication of homology across the organisms as well as clues to other possible essential genes in these organisms.

In still another embodiment, the exogenous nucleic acid sequences of the present invention that are identified as required for bacterial growth or proliferation can be used as antisense therapeutics for killing bacteria. The antisense sequences can be directed against the proliferation-required genes whose sequence corresponds to the exogenous nucleic acid probes identified here (i.e. the antisense nucleic acid may hybridize to the gene or a portion thereof). Alternatively, antisense therapeutics can be directed against operons in which proliferation-required genes reside (i.e. the antisense nucleic acid may hybridize to any gene in the operon in which the proliferation-required genes reside). Further, antisense therapeutics can be directed against a proliferation-required gene or portion thereof with or without adjacent noncoding sequences, an intragenic sequence (i.e. a sequence within a gene), an intergenic sequence (i.e. a sequence between genes), a sequence spanning at least a portion of two or more genes, a 5' noncoding region or a 3' noncoding region located upstream or downstream from the actual sequence that is required for bacterial proliferation or an operon containing a proliferation-required gene.

In addition to therapeutic applications, the present invention encompasses the use of nucleic acid sequences complementary to sequences required for proliferation as diagnostic tools, for example, nucleic acid probes complementary to proliferation-required sequences that are specific for particular species of microorganisms can be used as probes to identify particular microorganism species in clinical specimens. This utility provides a rapid and dependable method by which to identify the causative agent or agents of a bacterial infection. This utility would provide clinicians the ability to prescribe species specific antimicrobial compounds to treat such infections. In an extension of this utility, antibodies generated against proteins translated from mRNA transcribed from proliferation-required sequences can also be used to screen for specific microorganisms that produce such proteins in a species-specific manner.

The following examples teach the genes of the present invention and a subset of uses for the *E. coli* genes identified as required for proliferation. These examples are illustrative only and are not intended to limit the scope of the present invention.

### EXAMPLES

The following examples are directed to the identification and exploitation of *E. coli* genes required for proliferation. Methods of gene identification are discussed as well as a variety of methods to utilize the identified sequences.

### Genes Identified as Required for Proliferation of E. coli

Exogenous nucleic acid sequences were cloned into an inducible expression vector and assayed for growth inhibition activity. Example 1 describes the examination of a library of exogenous nucleic acid sequences cloned into IPTG-inducible expression vectors. Upon activation or induction, the expression vectors produced an RNA molecule corresponding to the subcloned exogenous nucleic acid sequences. The RNA product was in an antisense orientation with respect to the *E*. *coli* genes from which it was originally derived. This antisense RNA then interacted with sense mRNA produced from various *E*. *coli* genes and interfered with or inhibited the translation of the sense messenger RNA (mRNA) thus preventing protein production from these sense mRNA molecules. In cases where the sense mRNA encoded a protein required for the proliferation, bacterial cells containing an activated expression vector failed to grow or grew at a substantially reduced rate.

### EXAMPLE 1

### Inhibition of Bacterial Proliferation after IPTG induction

To study the effects of transcriptional induction in liquid medium, growth curves were carried out by back diluting cultures 1:200 into fresh media with or without 1 mM IPTG and measuring the OD₄₅₀ every 30 minutes (min). To study the effects of transcriptional induction on solid medium, 10², 10³, 10⁴, 10⁵, 10⁶, 10⁷ and 10⁸ fold dilutions of overnight cultures were prepared. Aliquots of from 0.5 to 3 µl of these dilutions were spotted on selective agar plates with or without 1 mM IPTG. After overnight incubation, the plates were compared to assess the sensitivity of the clones to IPTG.

Of the numerous clones tested, some clones were identified as a containing sequence that inhibited *E*. *coli* growth after IPTG induction. Accordingly, the gene to which the inserted nucleic acid sequence corresponds, or a gene within the operon containing the inserted nucleic acid, may be required for proliferation in *E*. *coli.*

### Characterization of Isolated Clones Negatively Affecting E. coli Proliferation

Following the identification of those expression vectors that, upon expression, negatively impacted *E*. *coli* growth or proliferation, the inserts or nucleic acid fragments contained in those expression vectors were isolated for subsequent characterization. Expression vectors of interest were subjected to nucleic acid sequence determination.

### EXAMPLE 2

### Nucleic Acid Sequence Determination of Identified Clones Expressing Nucleic Acid Fraqments with Detrimental Effects of E. coli Proliferation

The nucleotide sequences for the exogenous identified sequences were determined using plasmid DNA isolated using QIAPREP (Qiagen, Valencia, CA) and methods supplied by the manufacturer. The primers used for sequencing the inserts were 5' - TGTTTATCAGACCGCTT - 3' (SEQ ID NO: 403) and 5'- ACAATTTCACACAGCCTC - 3' (SEQ ID NO: 404). These sequences flank the polylinker in pLEX5BA. Sequence identification numbers (SEQ ID NOs) for the identified inserts are listed in Table I and discussed below.

### EXAMPLE 3

### Comparison Of Isolated Sequences to Known Sequences

The nucleic acid sequences of the subcloned fragments obtained from the expression vectors discussed above were compared to known *E*. *coli* sequences in GenBank using BLAST version 1.4 or version 2.0.6 using the following default parameters: Filtering off, cost to open a gap=5, cost to extend a gap=2, penalty for a mismatch in the blast portion of run=-3, reward for a match in the blast portion of run=1, expectation value (e)=10.0, word size=11, number of one-line descriptions=100, number of alignments to show (B)=100. BLAST is described in Altschul, J Mol Biol. 215:403-10 (1990), the disclosure of which is incorporated herein by reference in its entirety. Expression vectors were found to contain nucleic acid sequences in both the sense and antisense orientations. The presence of known genes, open reading frames, and ribosome binding sites was determined by comparison to public databases holding genetic information and various computer programs such as the Genetics Computer Group programs FRAMES and CODONPREFERENCE. Clones were designated as "antisense" if the cloned fragment was oriented to the promoter such that the RNA transcript produced was complementary to the expressed mRNA from a chromosomal locus. Clones were designated as "sense" if they coded for an RNA fragment that was identical to a portion of a wild type mRNA from a chromosomal locus.

The sequences described in Examples 1-2 that inhibited bacterial proliferation and contained gene fragments in an antisense orientation are listed in Table I. This table lists each identified sequence by: a sequence identification number; a Molecule Number; a gene to which the identified sequence corresponds, listed according to the National Center for Biotechnology Information (NCBI), Blattner (Science 277:1453-1474(1997); also contains the *E*. *coli* K-12 genome sequence), or Rudd (Micro. and Mol. Rev. 62:985-1019 (1998)), (both papers are hereby incorporated by reference) nomenclatures. The CONTIG numbers for each identified sequence is shown, as well as the location of the first and last base pairs located on the *E*. *coli* chromosome. A Molecule Number with a "**" indicates a clone corresponding to an intergenic sequence.

The sequences of the nucleic acid inserts of SEQ ID NOs: 1-81 from U.S. Provisional Patent Application No. 60/117,405 which inhibited proliferation were further analyzed. The reanalyzed sequences corresponding to SEQ ID NOs. 1-81 of U.S. Provisional Patent Application No. 60/117,405 have SEQ ID NOs. 405-485 in the present application.

SEQ ID NOs: 82-242 in U.S. Provisional Patent Application No. 60/117,405 are identical to SEQ ID NOs: 82-242 of the present application with the following exceptions. SEQ ID NO: 148 in the present application is the complementary strand of SEQ ID NO: 148 in U.S. Provisional Patent Application No. 60/117,405. Accordingly, the protein of SEQ ID NO: 308 which is encoded by SEQ ID NO: 148 has also been revised. SEQ ID NO: 163 in the present application is the complementary strand of SEQ ID NO: 163 in U.S. Provisional Patent Application No. 60/117,405. Accordingly, the protein of SEQ ID NO: 323 which is encoded by SEQ ID NO: 163 has also been revised.

The target gene of SEQ ID NOs. 18 and 19 of U.S. Provisional Patent Application No. 60/117,405 (SEQ ID NOs. 18, 19, 422, 423 of the present application) has been revised from dicF to ftsZ to reflect the fact that these SEQ ID NOs. include natural antisense molecules which inhibit ftsZ expression.

The gene products of the nucleic acids of SEQ ID NOs. 198 and 239-242 in U.S. Provisional Patent Application No. 60/117,405 and in the present application (SEQ ID NOs. 358 and 399-402 of the present application) have been revised to reflect the fact that these nucleic acids encode nontranslated tRNAs and rRNAs. Tables I and II have been revised accordingly. The SEQ ID NDs. in Table II were also revised to reflect the fact that SEQ ID NOs: 89 and 402 were identical in U.S. Provisional Patent Application No. 60/117,405.

**TABLE I**

| Identified Clones with Corresponding Genes and Operons | | | | | |
|---|---|---|---|---|---|
| **SEQ ID NO.** | **Molecule No.** | **Gene (NCBI)** | **Gene (Blattner)** | **Gene (Rudd)** | **CONTIG** |
| 1, 405 | EcXA001 | *yhhQ* | *b3471* | *yhhQ* | AE000423 |
| 2, 406 | EcXA002 | *lepB* | *lepB* | *lepB* | AE000343 |
| 3, 407 | EcXA003 | *f586* | *b0955* | *ycbZ* | AE000197 |
| 4, 408 | EcXA004 | *rpsG, rpsL* | *b3341* | *rpsG, rpsL* | AE000410 |
| 5, 409 | EcXA005a | *rplL, rplJ* | *b3986* | *rplL, rplJ* | AE000472 |
| 6, 410 | EcXA005b | *rplL* | *rplL* | *rplL* | AE000472 |
| 7, 411 | EcXA005c | *rplL, rplJ* | *rplL, rplJ* | *rplL, rplJ* | AE000472 |
| 8, 412 | EcXA005d | *rplL, rplJ* | *rplL, rplJ* | *rplL, rplJ* | AE000472 |
| 9, 413 | EcXA005e | *rplL* | *rplL* | *rplL* | AE000472 |
| 10,414 | EcXA005f | *rplL* | *rplL* | *rplL* | AE000472 |
| 11,415 | EcXA005g | *rplL* | *rplL* | *rplL* | AE000472 |
| 12,416 | EcXA006 | *pta* | *b2297* | *pta* | AE000319 |
| 13,417 | EcXA007 | *yicP* | *b3666* | *yicP* | AE000444 |
| 14,418 | EcXA008a | *yhaU* | *b3127* | *yhaU* | AE000394 |
| 15,419 | EcXA008b | *yhaU* | *yhaU* | *yhaU* | AE000394 |
| 16,420 | EcXA008c | *yhaU* | *yhaU* | *yhaU* | AE000394 |
| 17,421 | EcXA009 | *ydeY* | *ydeY* | *ydeY* | AE000249 |
| 18,422 | EcXA010a (natural as) | *dicF* | *b1575* | *dicF* | AE000253 |
| 19,423 | EcXA010b | *dicF* | *dicF* | *dicF* | AE000253 |
| 20,424 | EcXA011 | *fdnG* | *b1474* | *fdnG* | AE000244 |
| 21,425 | EcXA012a | *fusA* | *b3340* | *fusA* | AE000410 |
| 22,426 | EcXA012b | *fusA* | *fusA* | *fusA* | AE000410 |
| 23,427 | EcXA012c | *fusA* | *fusA* | *fusA* | AE000410 |
| 24,428 | EcXA013a | *o86* | *b2562* | *yfhL* | AE000342 |
| 25,429 | EcXA013b | *o86* | *b2562* | *yfhL* | AE000342 |
| 26,430 | EcXA013c | *o86* | *b2562* | *yfhL* | AEOQ0342 |
| 27,431 | EcXA014 | *visC* | *b2906* | *visC* | AE000374 |
| 28,432 | EcXA015 | *yfdI* | *yfdI* | *yfdI* | AE000323 |
| 29,433 | EcXA016 | *yeaQ* | *yeaQ* | *yeaQ* | AE000274 |
| | | *yoaG* | *yoaG* | *yoaG* | |
| 30,434 | EcXA017a | *yggE* | *b2922* | *yggE* | AE000375 |
| 31,435 | EcXA017b | *yggE* | *yggE* | *yggE* | AE000375 |
| 32,436 | EcXA018a | *o464* | *b2074* | *yegM* | AE000297 |
| 33,437 | EcXA018b | *o464* | *b2074* | *yegM* | AE000297 |
| 34,438 | EcXA019a | *yehA* | *yehA* | *yehA* | AE000300 |
| | | | | | AE000299 |
| 35,439 | EcXA019b | *o172,yehA* | *o172,yehA* | *o172,yehA* | AE000299 |
| 36,440 | EcXA020 | *o384,f82* | *b1794,b1795* | *yeaP,yeaQ* | AE000274 |
| 37,441 | EcXA021a | *f112* | *b0218* | *yafU* | AE000130 |
| 38,442 | EcXA021b | *f112* | *b0218* | *yafU* | AE000130 |
| 39,443 | EcXA022 | *o740* | *b1629* | *ydgN* | AE000258 |
| 40,444 | EcXA023a | *f176,f382* | *b1504,h1505* | *ydeS,ydeT* | AE000247 |
| 41,445 | EcXA023b | *f176,f382* | *b1504,b1505* | *ydeS,ydeT* | AE000247 |
| 42,446 | EcXA024 | *ygjM,ygjN* | *b3082* | *ygjM,ygjN* | AE000390 |
| 43,447 | EcXA025 | *02383* | *b1878* | *yeeJ* | AE000289 |
| 44,448 | EcXA026 | *o61* | *Unpre-dicted* | *Unpre-dicted* | AE000138 |
| 45,449 | EcXA027a | *yohH* | *yohH* | *yohH* | AE000303 |
| 46,450 | EcXA027b | *yohH* | *yohH* | *yohH* | AE000303 |
| 47,451 | EcXA027c | *yohH* | *yohH* | *yohH* | AE000303 |
| | | *yohl* | *yohl* | *yohl* | |
| 48,452 | EcXA027d | *yohH* | *yohH* | *yohH* | AE000303 |
| 49,453 | EcXA028 | *f296* | *b2305* | *yfc/* | AE000319 |
| 50,454 | EcXA029 | *yjjK* | *b4391* | *yjjK* | AE000509 |
| 51,455 | EcXA030 | *yi5A* | *b3557* | *yi5A* | AE000433 |
| 52,456 | EcXA031 | *rplE* | *B3308* | *rplE* | AE000408 |
| 53,457 | EcXA032a | *ybgD* | *ybgD* | *ybgD* | AE000175 |
| 54,458 | EcXA032b** | *ybgD* | *ybgD* | *ybgD* | AE000175 |
| | | *gltA* | *gltA* | *gltA* | |
| 55,459 | EcXA033a | *f477(as)* | *b3052* | *waaE* | AE000387 |
| | | | | | AE000386 |
| 56,460 | EcXA033b | *f477* | *b3052* | *waaE* | AE000387 |
| 57,461 | EcXA034a | *cspA* | *b3556* | *cspA* | AE000433 |
| 58,462 | EcXA034b | *cspA* | *b3556* | *cspA* | AE000433 |
| 59,463 | EcXA035 | *yhjU* | *yhjU* | *yhjU* | AE000431 |
| 60,464 | EcXA036 | *yqiF* | *b3101* | *yqjF* | AE000392 |
| | | *ogg* | *b3100,* | *yqjK* | |
| 61,465 | EcXA037 | *ydeH* | *b1535* | *ydeH* | AE000251 |
| 62,466 | EcXA038 | *sieB* | *b1353* | *sieB* | AE000233 |
| 63,467 | EcXA039 | *ybbD* | | *ybbD* | AE000156 |
| 64,468 | EcXA040 | *InsB 6* | *b3445* | *insB 6* | AE000420 |
| 65,469 | EcXA041 | *f234* | *b1138* | *ymfE* | AE000214 |
| 66,470 | EcXA042a | *rplY* | *rplY* | *rplY* | AE000308 |
| 67,471 | EcXA042b | *rplY* | *rplY* | *rplY* | AE000308 |
| 68,472 | EcXA043 | *ybgB* | *ybgB* | *ybgB* | AE000176 |
| | | *cydA* | *cydA* | *cydA* | |
| 69,473 | EcXA044 | *purB* | *b1131* | *purB* | AE000213 |
| 70,474 | EcXA045** | *csrA* | *csrA* | *csrA* | AE000353 |
| | | *serV* | *serV* | *serV* | |
| 71,475 | EcXA046** | *fimE, fimA* | *b4313* | *fimE, fimA* | AE000502 |
| 72,476 | EcXA047** | *f96, cspB* | *f96, cspB* | *cspB, ydfS* | AE000252 |
| 73,477 | EcXA048 | *yefE* | *yefE* | *yefE* | AE000294 |
| 74,478 | EcXA049 | *yaiC* | *b0385* | *yaiC* | AE000145 |
| 75,479 | EcXA050 | *o467, o222* | *yaiU,yaiV* | *yaiU, yaiV* | AE000144 |
| 76,480 | EcXA051a | *rplB, rplW* | *rplB, rplW* | *rplB, rplW* | AE000408 |
| 77,481 | EcXA051b | *rplW* | *rplW* | *rplW* | AE000408 |
| 78,482 | EcXA052 | *infC* | *infC* | *infC* | AE000267 |
| | | | | | AE000266 |
| 79,483 | EcXA053 | *gor* | *gor* | *gor* | AE000426 |
| 80,484 | EcXA054 | *rplF* | *rplF* | *rplF* | AE000408 |
| 81,485 | EcXA055 | *rrlG* | *rrlG* | *rrlG* | AE000345 |

### EXAMPLE 4

### Identification of Genes and their Corresponding Operons Affected by Antisense Inhibition

The sequencing of the entire E. coli genome is described in Blattner et al., Science 277:1453-1474(1997) the entirety of which is hereby incorporated by reference and the sequence of the genome is listed in GenBank Accession No.U00096, the disclosure of which is incorporated herein by reference in its entirety. The operons to which the proliferation-inhibiting nucleic acids correspond were identified using RegulonDB and information in the literature. The coordinates of the boundaries of these operons on the E. coli genome are listed in Table III. Table II lists the molecule numbers of the inserts containing the growth inhibiting nucleic acid fragments, the genes in the operons corresponding to the inserts, the SEQ ID NOs of the genes containing the inserts, the SEQ ID NOs of the proteins encoded by the genes, the start and stop points of the genes on the E. coli genome, the orientation of the genes on the genome, whether the operons are predicted or documented, and the predicted functions of the genes. The identified operons, their putative functions, and whether or not the genes are presently thought to be required for proliferation are discussed below.

Functions for the identified genes were determined by using either Blattner functional class designations or by comparing identified sequence with known sequences in various databases. A variety of biological functions were noted for the genes to which the clones of the present invention correspond. The functions for the genes of interest appear in Table II.

The proteins that are listed in Table II are involved in a wide range of biological functions.

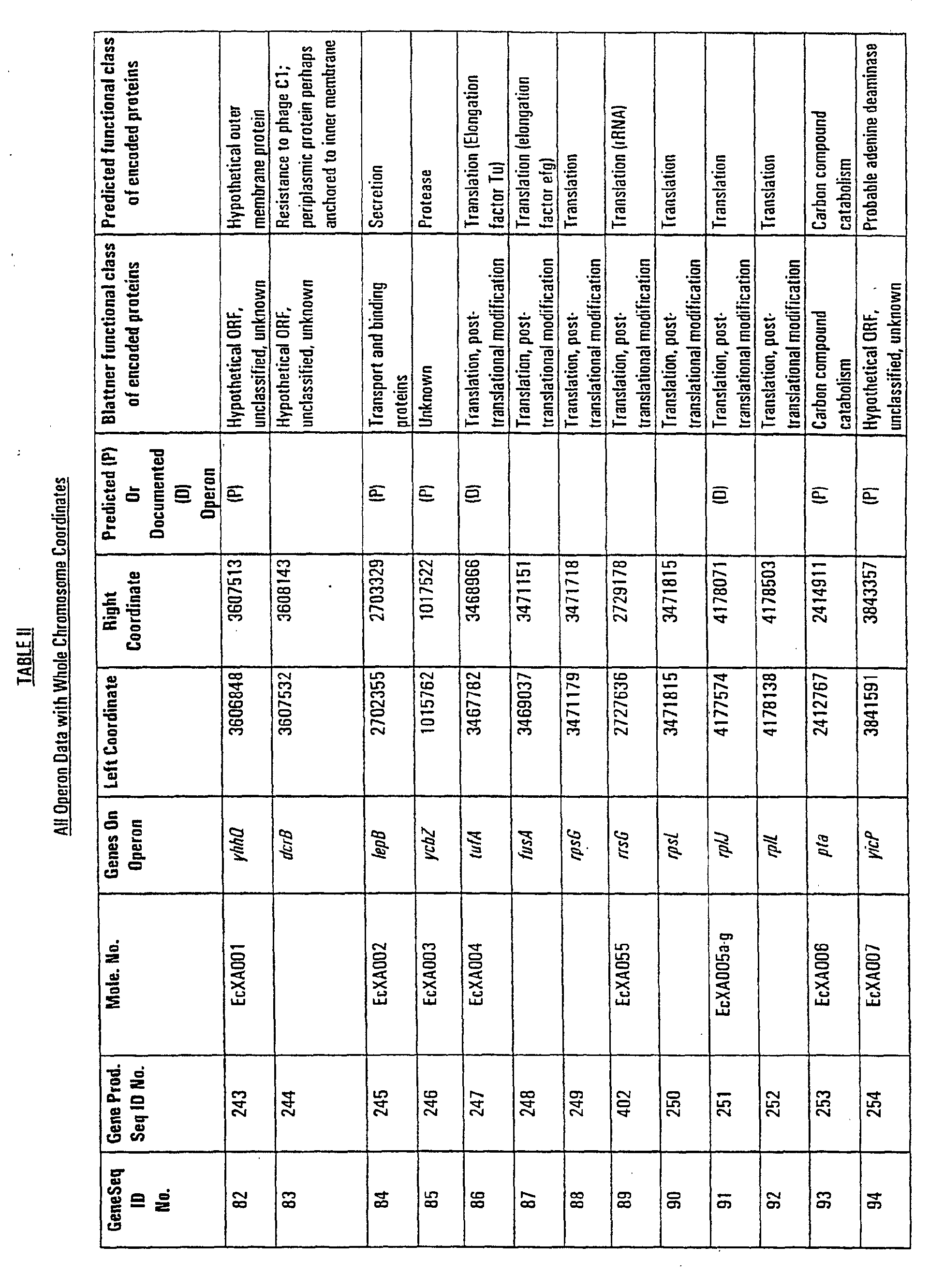

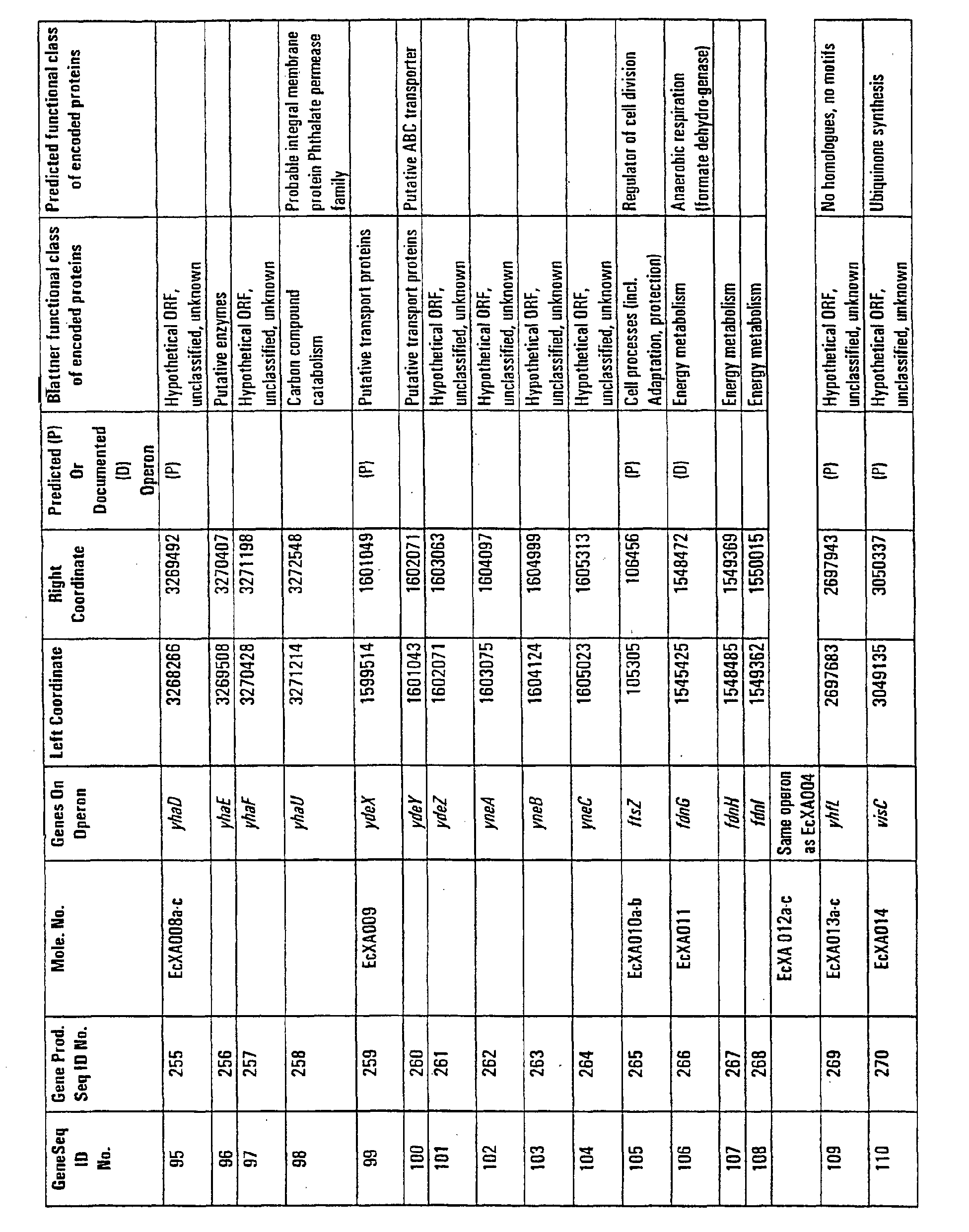

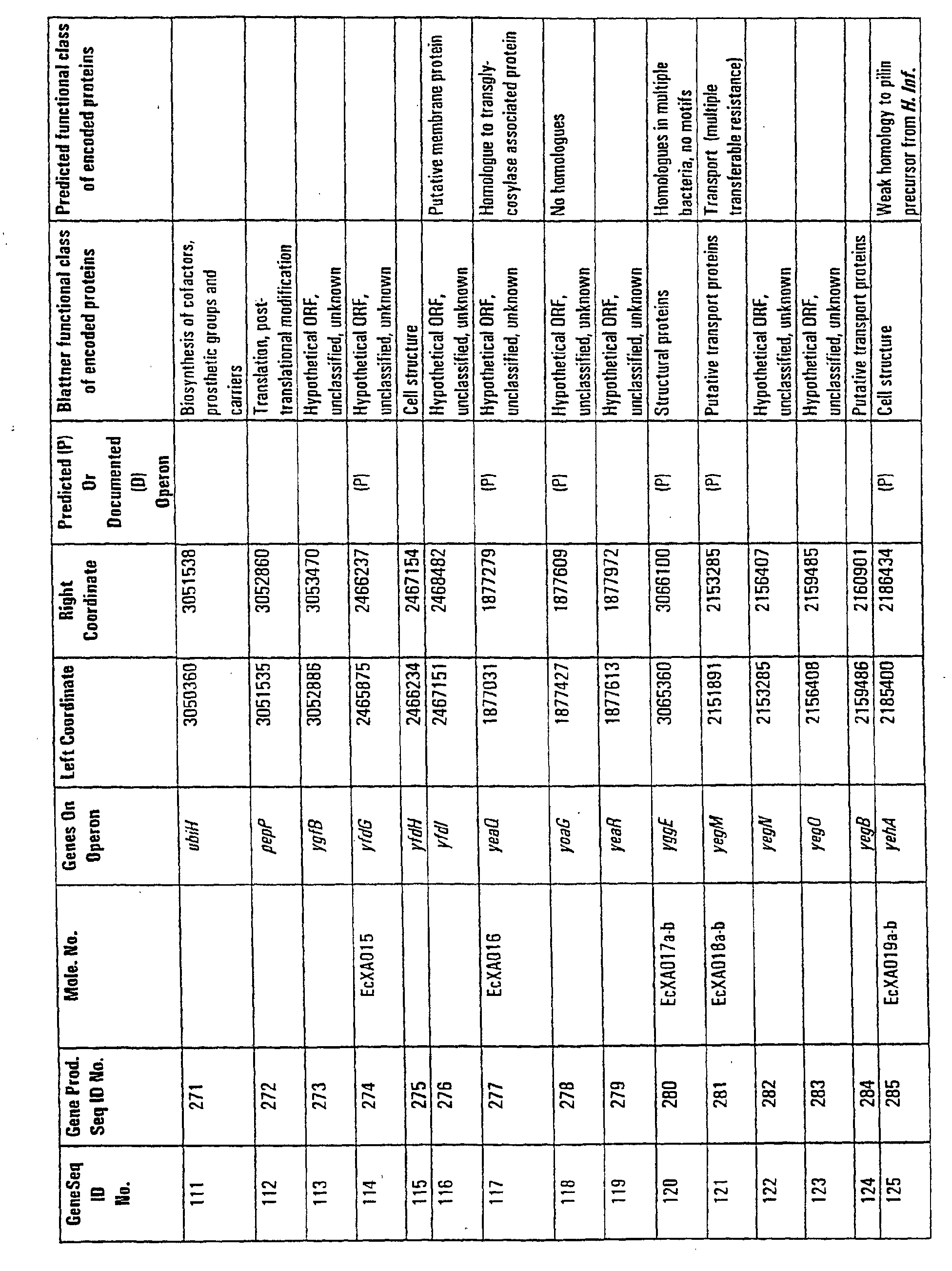

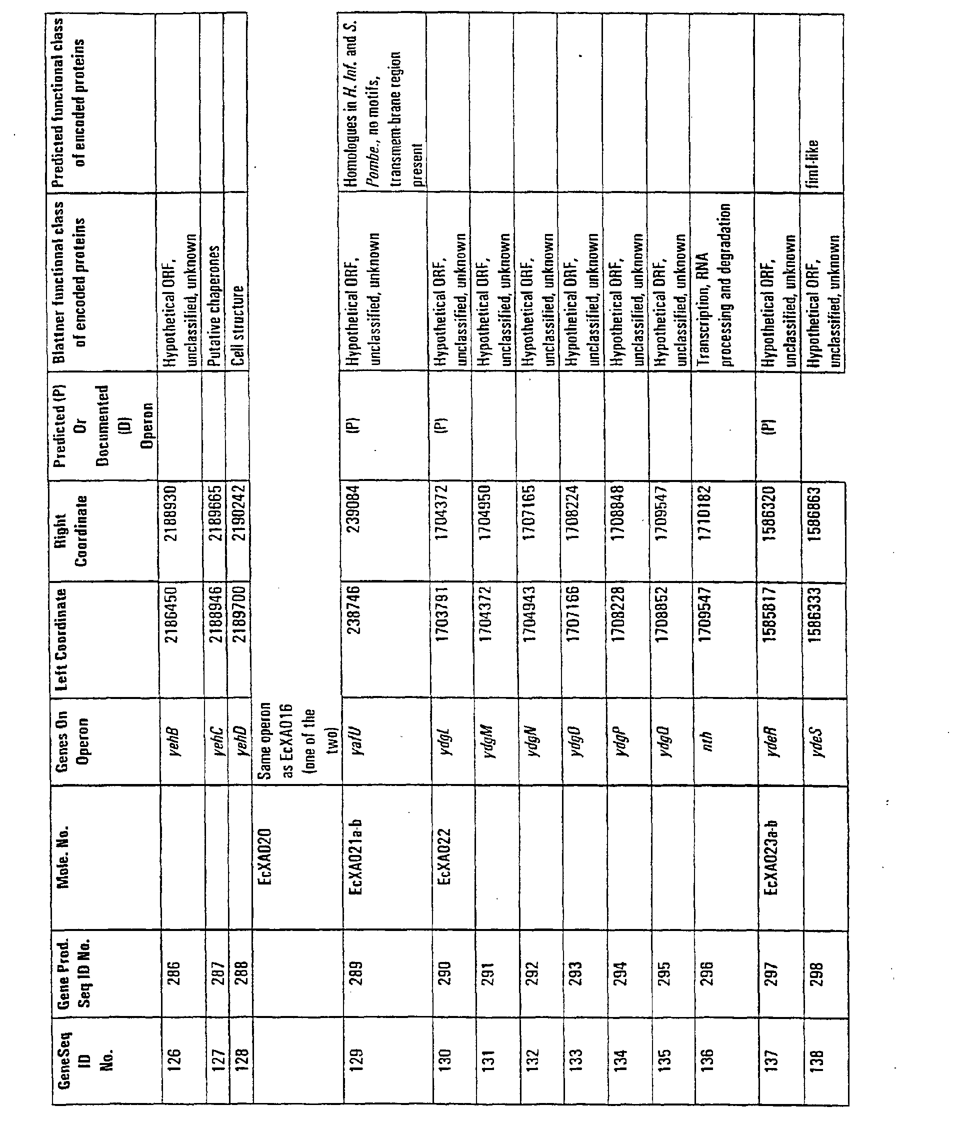

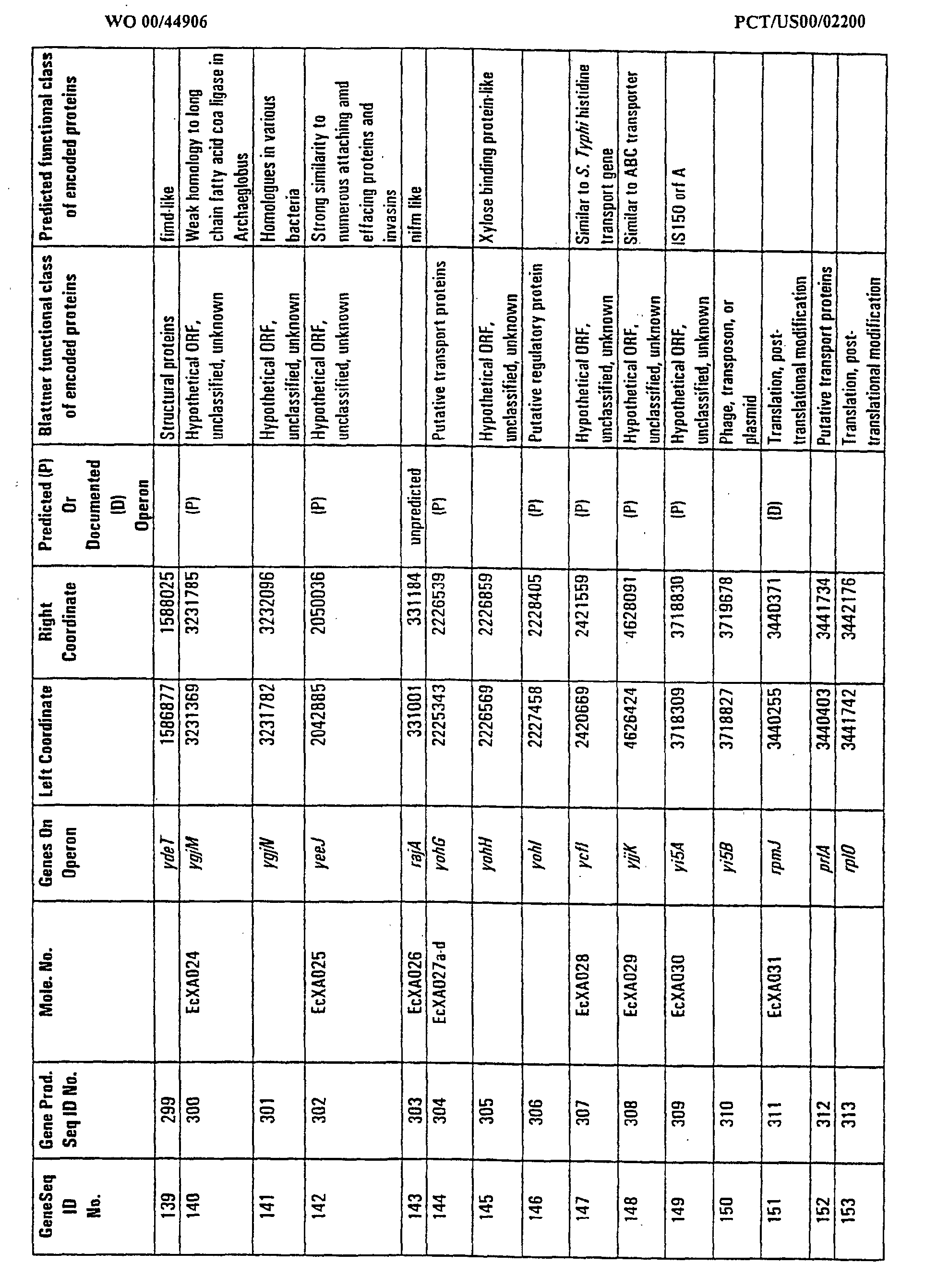

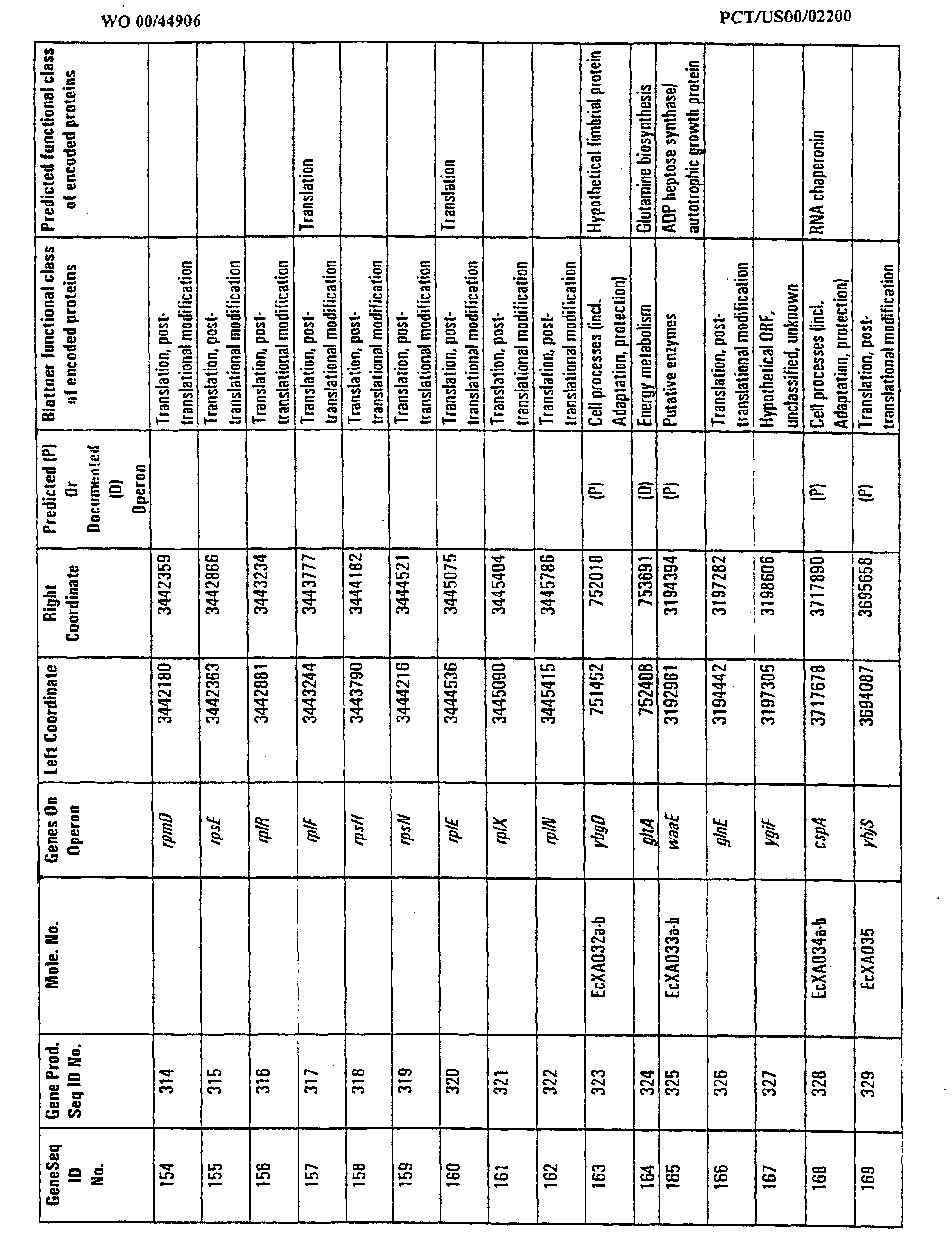

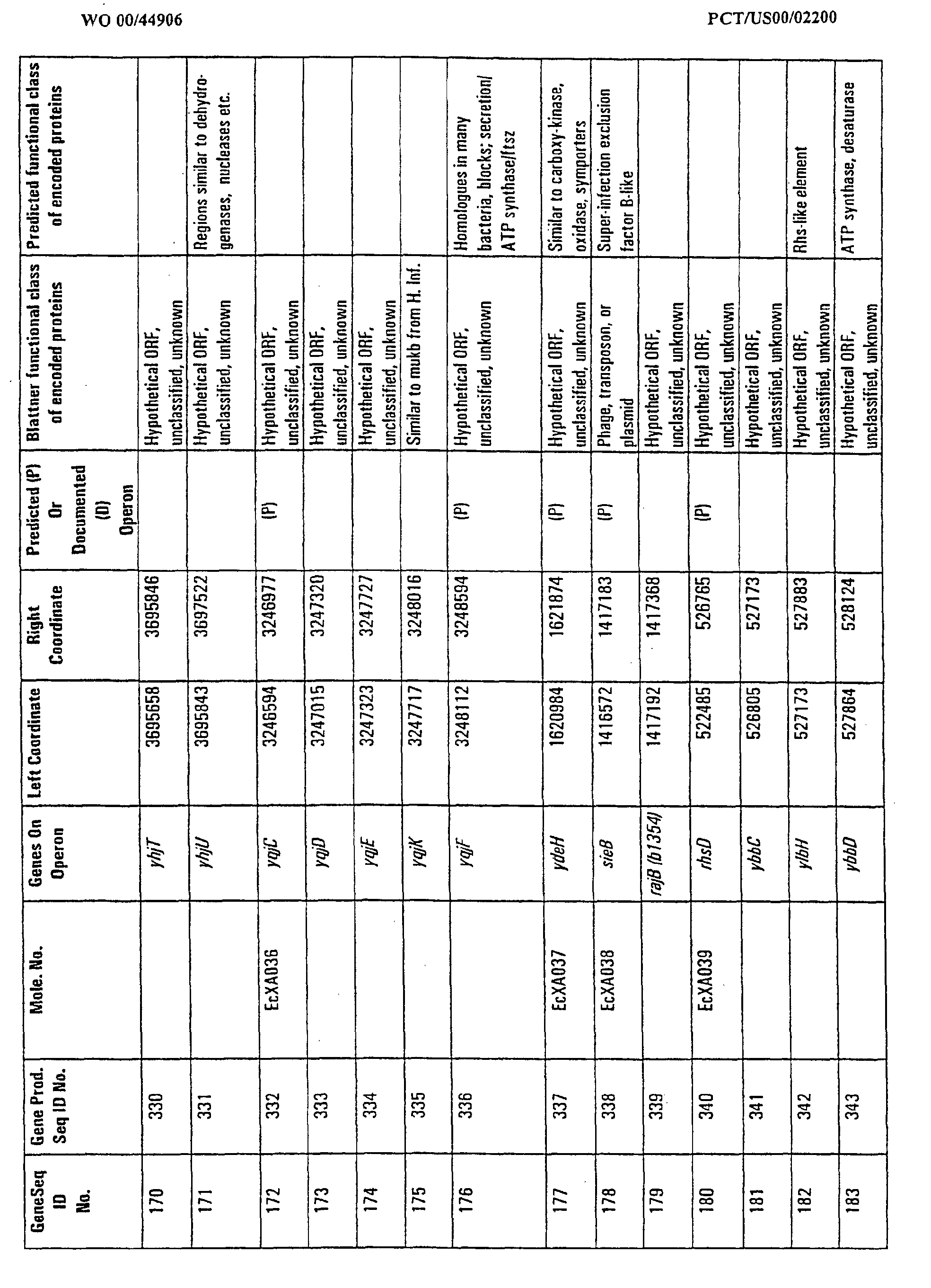

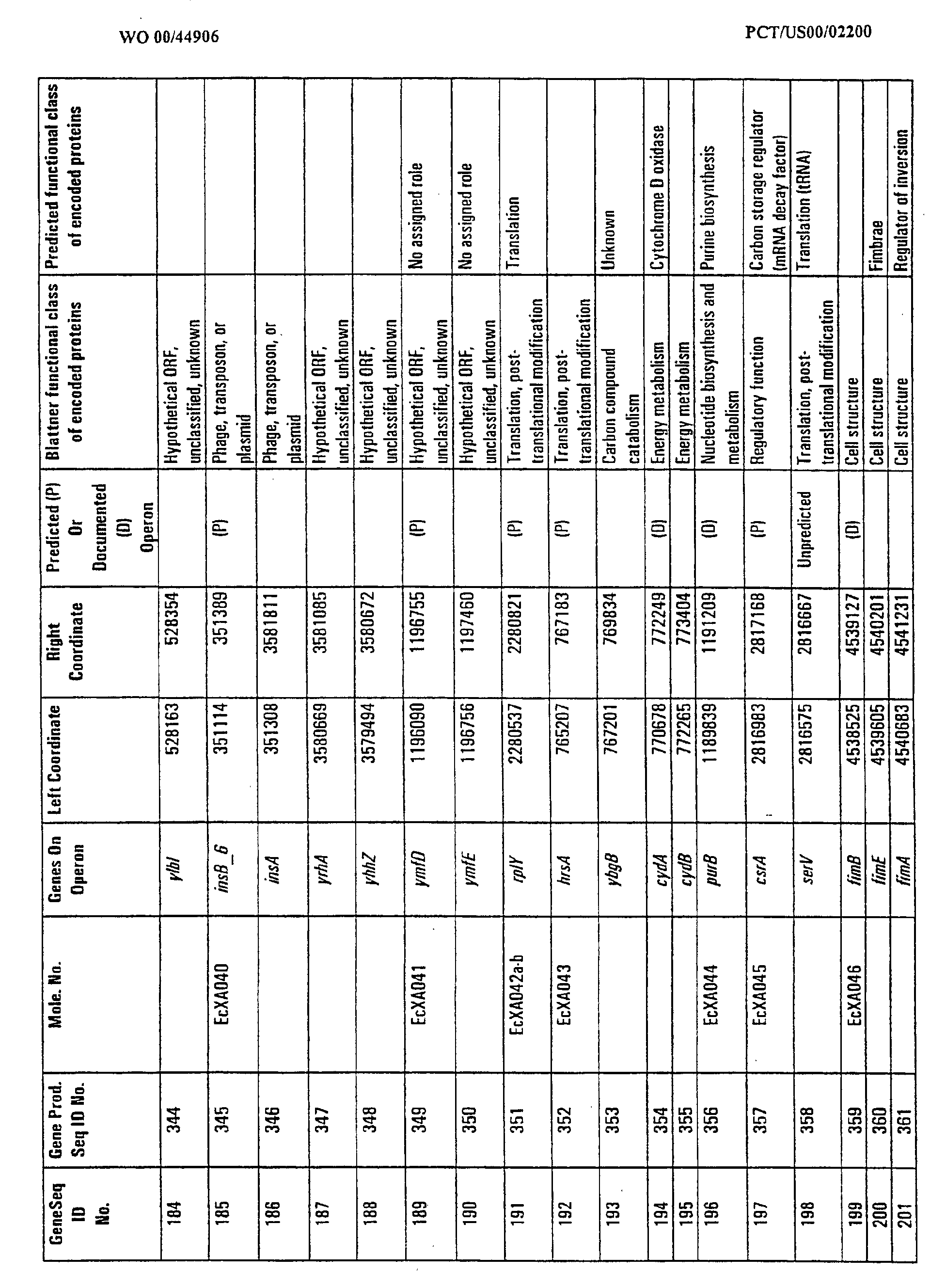

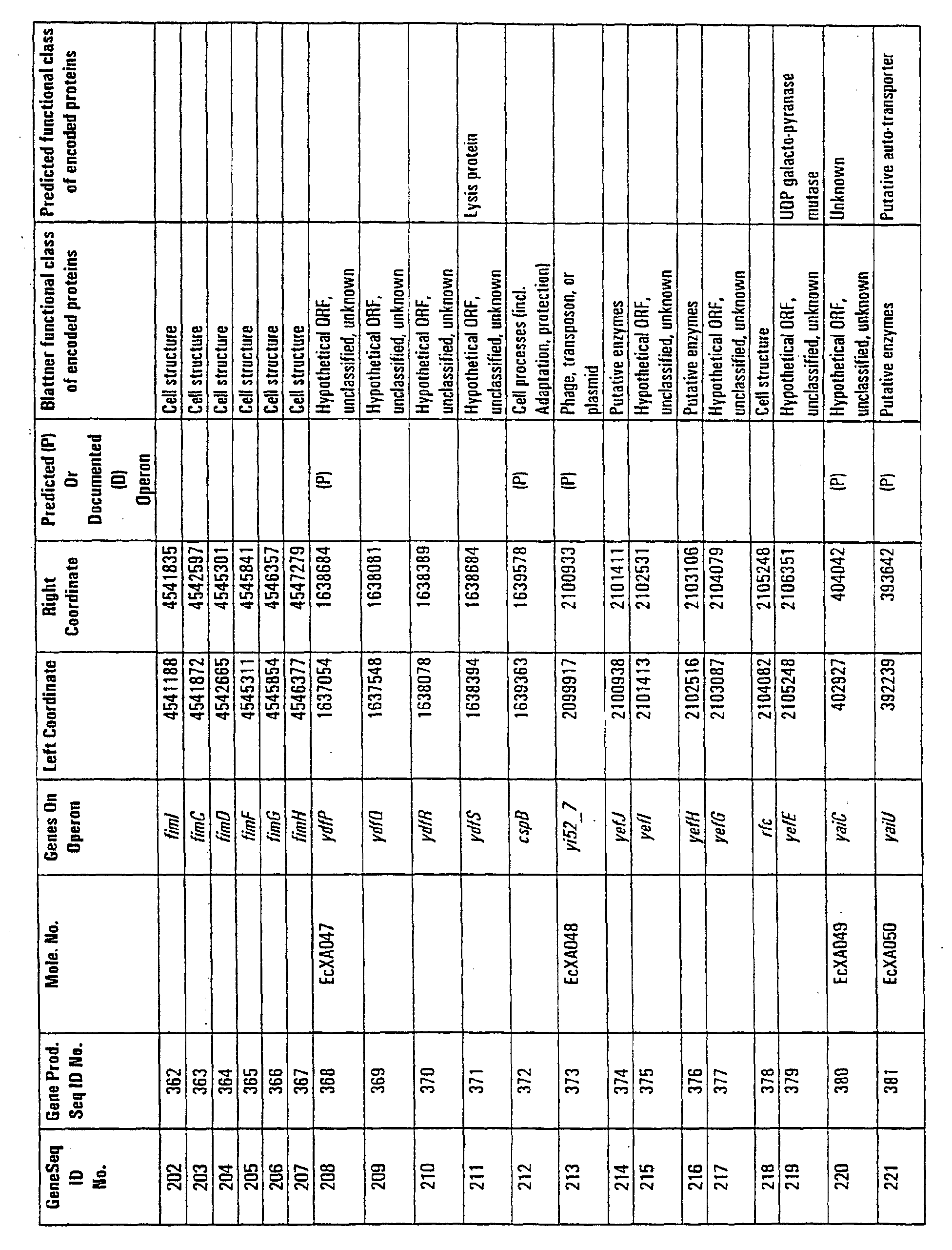

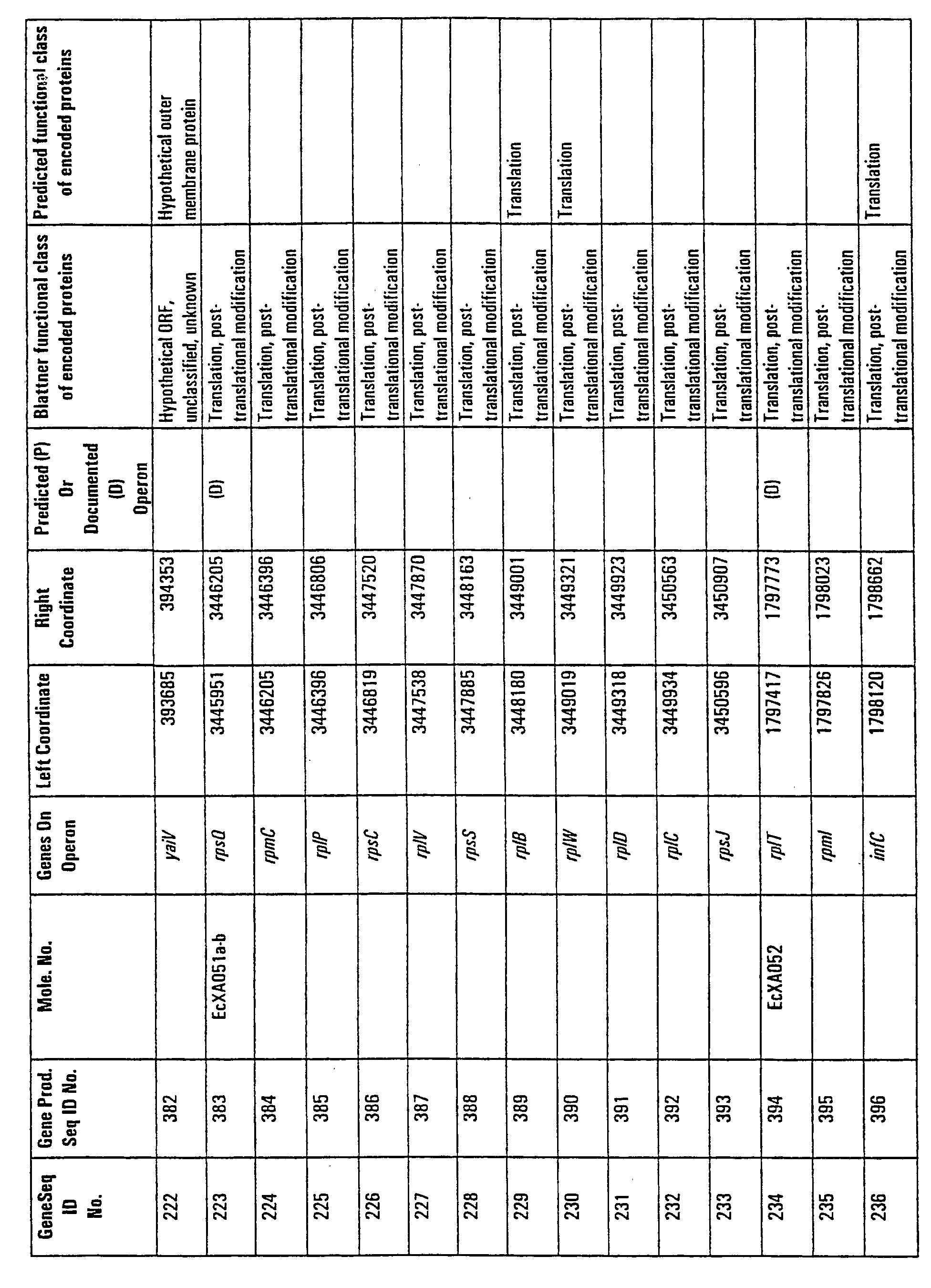

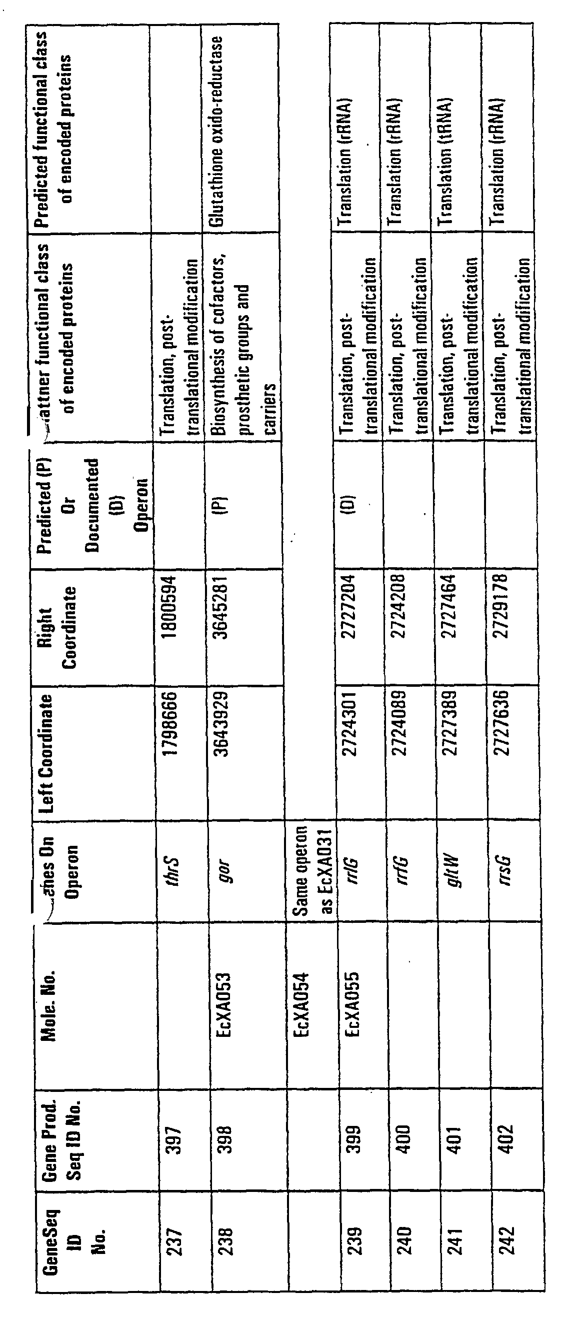

Several of the expression vectors contain fragments that correspond to genes of unknown function or if the function is known, it is not known whether the gene is essential. For example, EcXA001, 003, 007, 008, 013, 015, 016, 017, 018, 019, 020, 021, 022, 023, 024, 025, 026, 027, 028, 029, 030, 032, 033, 034, 035, 036, 037, 038, 039, 040, 041, 047, 048, 049 and 050 are all exogenous nucleic acid sequences that correspond to *E. coli* proteins that have no known function or where the function has not been shown to be essential or nonessential.

The present invention reports a number of novel *E. coli* genes and operons that are required for proliferation. From the list clone sequences identified here, each was identified to be a portion of a gene in an operon required for the proliferation of *E. coli*. Cloned sequences corresponding to genes already known to be required for proliferation in *E*. *coli* include EcXA002, 004, 005, 010, 012, 014, 031, 02, 043, 045, 051, 052, 054, and 055. The remaining identified sequences correspond to *E*. *coli* genes previously undesignated as required for proliferation in the art.

An interesting observation of the present invention is that there are also several sequence fragments that correspond to *E*. *coli* genes that are not thought to be required for *E*. *coli* proliferation. Nevertheless, under the conditions described above, the antisense expression of these gene fragments causes a reduction in cell growth. This result implies that the genes corresponding to the identified sequences are actually required for proliferation. Molecule Nos. corresponding to these genes are EcXA006, 044, 046, and 053.

Following identification of the sequences of interest, these sequences were localized into operons. Since bacterial genes are expressed in a polycistronic manner, the antisense inhibition of a single gene in an operon might effect the expression of all the other genes on the operon or the genes down stream from the single gene identified. In order to determine which of the gene products in an operon are required for proliferation, each of the genes contained within an operon may be analyzed for their effect on viability as described below.

**TABLE III**

| Operon Boundaries | | |
|---|---|---|
| **Mole. No.** | **Left Coordinate** | **Right Coordinate** |
| EcXA001 | 3606848 | 3608143 |
| EcXA002 | 2702355 | 2703329 |
| EcXA003 | 1015762 | 1017522 |
| EcXA004 | 3467782 | 3472189 |
| EcXA005 | 4177574 | 4178503 |
| EcXA006 | 2412767 | 2414911 |
| EcXA007 | 3841591 | 3843357 |
| EcXA008 | 3268266 | 3272548 |
| EcXA009 | 1599514 | 1605313 |
| EcXA010 | 1647406 | 1647458 |
| EcXA011 | 1545425 | 1550015 |
| EcXA012 | 3467782 | 3472189 |
| EcXA013 | 2697683 | 2697943 |
| EcXA014 | 3049135 | 3053470 |
| EcXA015 | 2465875 | 2468482 |
| EcXA016 | 1877031 | 1877972 |
| EcXA017 | 3065360 | 3066100 |
| EcXA018 | 2151891 | 2160901 |
| EcXA019 | 2185400 | 2190242 |
| EcXA020 | 1877031 | 1877972 |
| EcXA021 | 238746 | 239084 |
| EcXA022 | 1703791 | 1710182 |
| EcXA023 | 1585817 | 1588025 |
| EcXA024 | 3231369 | 3232096 |
| EcXA025 | 2042885 | 2050036 |
| EcXA026 | 331001 | 331184 |
| EcXA027c | 2225343 | 2228405 |
| EcXA028 | 2420669 | 2421559 |
| EcXA029 | 4626424 | 4628091 |
| EcXA030 | 3718309 | 3719678 |
| EcXA031 | 3440255 | 3445786 |
| EcXA032b | 751452 | 753691 |
| EcXA033 | 3192961 | 3198606 |
| EcXA034 | 3717678 | 3717890 |
| EcXA035 | 3694087 | 3697522 |
| EcXA036 | 3246594 | 3248594 |
| EcXA037 | 1620984 | 1621874 |
| EcXA038 | 1416572 | 1417368 |
| EcXA039 | 522485 | 528354 |
| EcXA040 | 3580669 | 3580672 |
| EcXA041 | 1196090 | 1197460 |
| EcXA042 | 2280537 | 2280821 |
| EcXA043 | 765207 | 773404 |
| EcXA044 | 1189839 | 1191209 |
| EcXA045 | 2816575 | 2817168 |
| EcXA046 | 4538525 | 4547279 |
| EcXA047 | 1637054 | 1639578 |
| EcXA048 | 2099917 | 2106351 |
| EcXA049 | 402927 | 404042 |
| EcXA050 | 392239 | 394353 |
| EcXA051 | 3445951 | 3450907 |
| EcXA052 | 1797417 | 1800594 |
| EcXA053 | 3643929 | 3645281 |
| EcXA054 | 3440255 | 3445786 |
| EcXA055 | 2724301 | 2729178 |

### EXAMPLE 5

### Identification of Individual Genes within an Operon Required for Proliferation

The following example illustrates a method for determining which gene in an operon is required for proliferation. The clone insert corresponding to Molecule No. EcXA004 possesses nucleic acid sequence homology to the *E*. *coli* genes *rspG* and *rspL*. This molecule corresponds to an operon containing two additional genes *fusA* and *tufA*. The *rpsL* gene is the first gene in the operon. To determine which gene or genes in this operon are required for proliferation, each gene is selectively inactivated using homologous recombination. Gene *rpsL* is the first gene to be inactivated.

Deletion inactivation of a chromosomal copy of a gene in *E*. *coli* can be accomplished by integrative gene replacement. The principle of this method (Hamilton, C. M., et al 1989. *J. Bacteriol.* **171**: 4617-4622) is to construct a mutant allele of the targeted gene, introduce that allele into the chromosome using a conditional suicide vector, and then force the removal of the native wild type allele and vector sequences. This will replace the native gene with a desired mutation(s) but leave promoters, operators, etc. intact. Essentiality of a gene is determined either by deduction from genetic analysis or by conditional expression of a wild type copy of the targeted gene (trans complementation).

The first step is to generate a mutant *rpsL* allele using PCR amplification. Two sets of PCR primers are chosen to produce a copy of *rpsL* with a large central deletion to inactivate the gene. In order to eliminate polar effects, it is desirable to construct a mutant allele comprising an in-frame deletion of most or all of the coding region of the *rpsL* gene. Each set of PCR primers is chosen such that a region flanking the gene to be amplified is sufficiently long to allow recombination (typically at least 500 nucleotides on each side of the deletion). The targeted deletion or mutation will be contained within this fragment. To facilitate cloning of the PCR product, the PCR primers may also contain restriction endonuclease sites found in the cloning region of a conditional knockout vector such as pK03 (Link, et al 1997 *J*. *Bacteriol.* **179** (20): 6228-6237). Suitable sites include Notl, Sall, BamHl and Smal. The *rpsL* gene fragments are produced using standard PCR conditions including, but not limited to, those outlined in the manufacturers directions for the Hot Start Taq PCR kit (Qiagen, Inc., Valencia, CA). The PCR reactions will produce two fragments that can be fused together. Alternatively, crossover PCR can be used to generate a desired deletion in one step (Ho, S. N., et al 1989. *Gene* **77**:51-59, Horton, R. M., et al 1989. *Gene* **77**: 61-68). The mutant allele thus produced is called a "null" allele because it cannot produce a functional gene product.

The mutant allele obtained from PCR amplification is cloned into the multiple cloning site of pK03. Directional cloning of the *rpsL* null allele is not necessary. The pK03 vector has a temperature-sensitive origin of replication derived from pSC101. Therefore, clones are propagated at the permissive temperature of 30°C. The vector also contains two selectable marker genes: one that confers resistance to chloramphenicol and another, the *Bacillus subtilis sacB* gene, that allows for counter-selection on sucrose containing growth medium. Clones that contain vector DNA with the null allele inserted are confirmed by restriction endonuclease analysis and DNA sequence analysis of isolated plasmid DNA. The plasmid containing the *rpsL* null allele insert is known as a knockout plasmid.

Once the knockout plasmid has been constructed and its sequence verified, it is transformed into a Rec⁺ *E*. *coli* host cell. Transformation can be by any standard method such as electroporation. In some fraction of the transformed cells, plasmids will integrate into the *E. coil* chromosome by homologous recombination between the *rpsL* null allele in the plasmid and the *rpsL* gene in the chromosome. Transformant colonies in which such an event has occurred are readily selected by growth at the non-permissive temperature of 43°C and in the presence of choramphenicol. At this temperature, the plasmid will not replicate as an episome and will be lost from cells as they grow and divide. These cells are no longer resistant to chloramphenicol and will not grow when it is present. However, cells in which the knockout plasmid has integrated into the *E*. *coli* chromosome remain resistant to chloramphenicol and propagate.

Cells containing integrated knock-out plasmids are usually the result of a single crossover event that creates a tandem repeat of the mutant and native wild type alleles of *rpsL* separated by the vector sequences. A consequence of this is that *rpsL* will still be expressed in these cells. In order to determine if the gene is essential for growth, the wild type copy must be removed. This is accomplished by selecting for plasmid excision, a process in which homologous recombination between the two alleles results in looping out of the plasmid sequences. Cells that have undergone such an excision event and have lost plasmid sequences including *sacB* gene are selected for by addition of sucrose to the medium. The sacB gene product converts sucrose to a toxic molecule. Thus counter selection with sucrose ensures that plasmid sequences are no longer present in the cell. Loss of plasmid sequences is further confirmed by testing for sensitivity to chloramphenicol (loss of the chloramphenicol resistance gene). The latter test is important because occasionally a mutation in the *sacB* gene can occur resulting in a loss of *sacB* function with no effect on plasmid replication (Link, et. al., 1997 *J. Bacteriol.* **179** (20): 6228-6237). These artifact clones retain plasmid sequences and are therefore still resistant to chloramphenicol.

In the process of plasmid excision, one of the two *rpsL* alleles is lost from the chromosome along with the plasmid DNA. In general, it is equally likely that the null allele or the wild type allele will be lost. Therefore, if the *rpsL* gene is not essential, half of the clones obtained in this experiment will have the wild type allele on the chromosome and half will have the null allele. However, if the *rpsL* gene is essential, cells containing the null allele will not be obtained as a single copy of the null allele would be lethal.

To determine the essentiality of *rpsL*, a statistically significant number of the resulting clones, at least 20, are analyzed by PCR amplification of the *rpsL* gene. Since the null allele is missing a significant portion of the *rpsL* gene, its PCR product is significantly shorter than that of the wild type gene and the two are readily distinguished by gel electrophoretic analysis. The PCR products may also be subjected to sequence determination for further confirmation by methods well known to those in the art.

The above experiment is generally adequate for determining the essentiality of a gene such as *rpsL*. However, it may be necessary or desirable to more directly confirm the essentiality of the gene. There are several methods by which this can be accomplished. In general, these involve three steps: 1) construction of an episome containing a wild type allele, 2) isolation of clones containing a single chromosomal copy of the mutant null allele as described above but in the presence of the episomal wild type allele, and then 3) determining if the cells survive when the expression of the episomal allele is shut off. In this case, the trans copy of wild type *rpsL* is made by PCR cloning of the entire coding region of *rpsL* and inserting it in the sense orientation downstream of an inducible promoter such as the *E*. *coli lac* promoter. Transcription of this allele of *rpsL* will be induced in the presence of IPTG which inactivates the *lac* repressor. Under IPTG induction *rpsL* protein will be expressed as long as the recombinant gene also possesses a ribosomal binding site, also known as a "Shine-Dalgarno Sequence". The trans copy of *rpsL* is cloned on a plasmid that is compatible with pSC101. Compatible vectors include p15A, pBR322, and the pUC plasmids, among others. Replication of the compatible plasmid will not be temperature-sensitive. The entire process of integrating the null allele of *rpsL* and subsequent plasmid excision is carried out in the presence of IPTG to ensure the expression of functional *rpsL* protein is maintained throughout. After the null *rpsL* allele is confirmed as integrated on the chromosome in place of the wild type *rpsL* allele, then IPTG is withdrawn and expression of functional *rpsL* protein shut off.. If the *rpsL* gene is essential, cells will cease to proliferate under these conditions. However, if the *rpsL* gene is not essential, cells will continue to proliferate under these conditions. In this experiment, essentiality is determined by conditional expression of a wild type copy of the gene rather than inability to obtain the intended chromosomal disruption.

An advantage of this method over some other gene disruption techniques is that the targeted gene can be deleted or mutated without the introduction of large segments of foreign DNA. Therefore, polar effects on downstream genes are eliminated or minimized. There are methods described to introduce inducible promoters upstream of potential essential bacterial genes. However in such cases, polarity from multiple transcription start points can be a problem. One way of preventing this is to insert a gene disruption cassette that contains strong transcriptional terminators upstream of the integrated inducible promoter (Zhang, Y, and Cronan, J. E. 1996 *J. Bacteriol*. **178** (12): 3614-3620). The described techniques will all be familiar to one of ordinary skill in the art.

Following the analysis of the *rpsL* gene, the other genes of the operon are investigated to determine if they are required for proliferation.

### EXAMPLE 6

### Expression of the Proteins Encoded by Genes Identified as Required for E. coli Proliferation

The following is provided as one exemplary method to express the proliferation-required proteins encoded by the identified sequences described above. First, the initiation and termination codons for the gene are identified. If desired, methods for improving translation or expression of the protein are well known in the art. For example, if the nucleic acid encoding the polypeptide to be expressed lacks a methionine codon to serve as the initiation site, a strong Shine-Delgamo sequence, or a stop codon, these sequences can be added. Similarly, if the identified nucleic acid sequence lacks a transcription termination signal, this sequence can be added to the construct by, for example, splicing out such a sequence from an appropriate donor sequence. In addition, the coding sequence may be operably linked to a strong promoter or an inducible promoter if desired. The identified nucleic acid sequence or portion thereof encoding the polypeptide to be expressed is obtained by PCR from the bacterial expression vector or genome using oligonucleotide primers complementary to the identified nucleic acid sequence or portion thereof and containing restriction endonuclease sequences for *Nco*I incorporated into the 5' primer and *Bgl*II at the 5' end of the corresponding 3'-primer, taking care to ensure that the identified nucleic acid sequence is positioned in frame with the termination signal. The purified fragment obtained from the resulting PCR reaction is digested with *Nco*I and *Bgl*II, purified and ligated to an expression vector.

The ligated product is transformed into DH5α or some other *E*. *coli* strain suitable for the over expression of potential proteins. Transformation protocols are well known in the art. For example, transformation protocols are described in: **Current Protocols in Molecular Biology**, Vol. 1, Unit 1.8, (Ausubel, et al., Eds.) John Wiley & Sons, Inc. (1997). Positive transformants are selected after growing the transformed cells on plates containing 50-100 µg/ml Ampicillin (Sigma, St. Louis, Missouri). In one embodiment, the expressed protein is held in the cytoplasm of the host organism. In an alternate embodiment, the expressed protein is released into the culture medium. In still another alternative, the expressed protein can be sequestered in the periplasmic space and liberated therefrom using any one of a number of cell lysis techniques known in the art. For example, the osmotic shock cell lysis method described in Chapter 16 of **Current Protocols in Molecular Biology,** Vol. 2, (Ausubel, et al., Eds.) John Wiley & Sons, Inc. (1997). Each of these procedures can be used to express a proliferation-required protein.

Expressed proteins, whether in the culture medium or liberated from the periplasmic space or the cytoplasm, are then purified or enriched from the supernatant using conventional techniques such as ammonium sulfate precipitation, standard chromatography, immunoprecipitation, immunochromatography, size exclusion chromatography, ion exchange chromatography, and HPLC. Alternatively, the secreted protein can be in a sufficiently enriched or pure state in the supernatant or growth media of the host to permit it to be used for its intended purpose without further enrichment. The purity of the protein product obtained can be assessed using techniques such as Coomassie or silver staining or using antibodies against the control protein. Coomassie and silver staining techniques are familiar to those skilled in the art.

Antibodies capable of specifically recognizing the protein of interest can be generated using synthetic peptides using methods well known in the art. See, **Antibodies: A Laboratory Manual**, (Harlow and Lane, Eds.) Cold Spring Harbor Laboratory (1988). For example, 15-mer peptides having a sequence encoded by the appropriate identified gene sequence of interest or portion thereof can be chemically synthesized. The synthetic peptides are injected into mice to generate antibodies to the polypeptide encoded by the identified nucleic acid sequence of interest or portion thereof. Alternatively, samples of the protein expressed from the expression vectors discussed above can be purified and subjected to amino acid sequencing analysis to confirm the identity of the recombinantly expressed protein and subsequently used to raise antibodies. An Example describing in detail the generation of monoclonal and polyclonal antibodies appears in Example 7.

The protein encoded by the identified nucleic acid sequence of interest or portion thereof can be purified using standard immunochromatography techniques. In such procedures, a solution containing the secreted protein, such as the culture medium or a cell extract, is applied to a column having antibodies against the secreted protein attached to the chromatography matrix. The secreted protein is allowed to bind the immunochromatography column. Thereafter, the column is washed to remove non-specifically bound proteins. The specifically bound secreted protein is then released from the column and recovered using standard techniques. These procedures are well known in the art.

In an alternative protein purification scheme, the identified nucleic acid sequence of interest or portion thereof can be incorporated into expression vectors designed for use in purification schemes employing chimeric polypeptides. In such strategies the coding sequence of the identified nucleic acid sequence of interest or portion thereof is inserted in-frame with the gene encoding the other half of the chimera. The other half of the chimera can be maltose binding protein (MBP) or a nickel binding polypeptide encoding sequence. A chromatography matrix having antibody to MBP or nickel attached thereto is then used to purify the chimeric protein. Protease cleavage sites can be engineered between the MBP gene or the nickel binding polypeptide and the identified expected gene of interest, or portion thereof. Thus, the two polypeptides of the chimera can be separated from one another by protease digestion.

One useful expression vector for generating maltose binding protein fusion proteins is pMAL (New England Biolabs), which encodes the *malE* gene. In the pMal protein fusion system, the cloned gene is inserted into a pMal vector downstream from the *malE* gene. This results in the expression of an MBP-fusion protein. The fusion protein is purified by affinity chromatography. These techniques as described are well known to those skilled in the art of molecular biology.

### EXAMPLE 7

### Production of an Antibody to an isolated E. coli Protein

Substantially pure protein or polypeptide is isolated from the transformed cells as described in Example 6. The concentration of protein in the final preparation is adjusted, for example, by concentration on a 10,000 molecular weight cut off AMICON filter device (Millipore, Bedford, MA), to the level of a few micrograms/ml. Monoclonal or polyclonal antibody to the protein can then be prepared as follows:

### Monoclonal Antibody Production by Hybridoma Fusion

Monoclonal antibody to epitopes of any of the peptides identified and isolated as described can be prepared from murine hybridomas according to the classical method of Kohler, G. and Milstein, C., **Nature 256**:495 (1975) or any of the well-known derivative methods thereof. Briefly, a mouse is repetitively inoculated with a few micrograms of the selected protein or peptides derived therefrom over a period of a few weeks. The mouse is then sacrificed, and the antibody producing cells of the spleen isolated. The spleen cells are fused by means of polyethylene glycol with mouse myeloma cells, and the excess unfused cells destroyed by growth of the system on selective media comprising aminopterin (HAT media). The successfully fused cells are diluted and aliquots of the dilution placed in wells of a microtiter plate where growth of the culture is continued. Antibody-producing clones are identified by detection of antibody in the supernatant fluid of the wells by immunoassay procedures, such as ELISA, as described by Engvall, E., "Enzyme immunoassay ELISA and EMIT," **Meth. Enzymol. 70**:419 (1980), and derivative methods thereof. Selected positive clones can be expanded and their monoclonal antibody product harvested for use. Detailed procedures for monoclonal antibody production are described in Davis, L et al. **Basic Methods in Molecular Biology** Elsevier, New York. Section 21-2.

### Polyclonal Antibody Production by Immunization

Polyclonal antiserum containing antibodies to heterogeneous epitopes of a single protein or a peptide can be prepared by immunizing suitable animals with the expressed protein or peptides derived therefrom described above, which can be unmodified or modified to enhance immunogenicity. Effective polyclonal antibody production is affected by many factors related both to the antigen and the host species. For example, small molecules tend to be less immunogenic than larger molecules and can require the use of carriers and adjuvant. Also, host animals vary in response to site of inoculations and dose, with both inadequate or excessive doses of antigen resulting in low titer antisera. Small doses (ng level) of antigen administered at multiple intradermal sites appears to be most reliable. An effective immunization protocol for rabbits can be found in Vaitukaitis, J. et al. **J. Clin. Endocrinol.** Metab. 33:988-991 (1971).

Booster injections can be given at regular intervals, and antiserum harvested when antibody titer thereof, as determined semi-quantitatively, for example, by double immunodiffusion in agar against known concentrations of the antigen, begins to fall. See, for example, Ouchterlony, 0. et al., Chap. 19 in: **Handbook of Experimental Immunology D.** Wier (ed) Blackwell (1973). Plateau concentration of antibody is usually in the range of 0.1 to 0.2 mg/ml of serum (about 12 M). Affinity of the antisera for the antigen is determined by preparing competitive binding curves, as described, for example, by Fisher, D., Chap. 42 in: **Manual of Clinical Immunology,** 2d Ed. (Rose and Friedman, Eds.) Amer. Soc. For Microbiol., Washington, D.C. (1980).

Antibody preparations prepared according to either protocol are useful in quantitative immunoassays which determine concentrations of antigen-bearing substances in biological samples; they are also used semi-quantitatively or qualitatively to identify the presence of antigen in a biological sample. The antibodies can also be used in therapeutic compositions for killing bacterial cells expressing the protein.

### EXAMPLE 8

### Screening Chemical Libraries

### A. Protein-Based Assays

Having isolated and expressed bacterial proteins shown to be required for bacterial proliferation, the present invention further contemplates the use of these expressed proteins in assays to screen libraries of compounds for potential drug candidates. The generation of chemical libraries is well known in the art. For example combinatorial chemistry can be used to generate a library of compounds to be screened in the assays described herein. A combinatorial chemical library is a collection of diverse chemical compounds generated by either chemical synthesis or biological synthesis by combining a number of chemical "building blocks" reagents. For example, a linear combinatorial chemical library such as a polypeptide library is formed by combining amino acids in every possible combination to yield peptides of a given length. Millions of chemical compounds theoretically can be synthesized through such combinatorial mixings of chemical building blocks. For example, one commentator observed that the systematic, combinatorial mixing of 100 interchangeable chemical building blocks results in the theoretical synthesis of 100 million tetrameric compounds or 10 billion pentameric compounds. (Gallop et al., "Applications of Combinatorial Technologies to Drug Discovery, Background and Peptide Combinatorial Libraries," **Journal of Medicinal Chemistry**, Vol. 37, No. 9, 1233-1250 (1994). Other chemical libraries known to those in the art may also be used, including natural product libraries.

Once generated, combinatorial libraries can be screened for compounds that possess desirable biological properties. For example, compounds which may be useful as drugs or to develop drugs would likely have the ability to bind to the target protein identified, expressed and purified as discussed above. Further, if the identified target protein is an enzyme, candidate compounds would likely interfere with the enzymatic properties of the target protein. Any enzyme can be a target protein. For example, the enzymatic function of a target protein can be to serve as a protease, nuclease, phosphatase, dehydrogenase, transporter protein, transcriptional enzyme, and any other type of enzyme known or unknown. Thus, the present invention contemplates using the protein products described above to screen combinatorial chemical libraries.

Those in the art will appreciate that a number of techniques exist for characterizing target proteins in order to identify molecules useful for the discovery and development of therapeutics. For example, some techniques involve the generation and use of small peptides to probe and analyze target proteins both biochemically and genetically in order to identify and develop drug leads. Such techniques include the methods described in PCT publications No. W09935494, W09819162, W09954728, the disclosures of which are incorporated herein by reference in their entireties.

In another example, the target protein is a serine protease and the substrate of the enzyme is known. The present example is directed towards the analysis of libraries of compounds to identify compounds that function as inhibitors of the target enzyme. First, a library of small molecules is generated using methods of combinatorial library formation well known in the art. U.S. Patent NOs. 5,463,564 and 5,574, 656, to Agrafiotis, et al., entitled "System and Method of Automatically Generating Chemical Compound with Desired Properties," are two such teachings. Then the library compounds are screened to identify library compounds that possess desired structural and functional properties. U.S. Patent No. 5,684,711 also discusses a method for screening libraries.

To illustrate the screening process, the combined target and chemical compounds of the library are exposed to and permitted to interact with the purified enzyme. A labeled substrate is added to the incubation. The label on the substrate is such that a detectable signal is emitted from metabolized substrate molecules. The emission of this signal permits one to measure the effect of the combinatorial library compounds on the enzymatic activity of target enzymes. The characteristics of each library compound is encoded so that compounds demonstrating activity against the enzyme can be analyzed and features common to the various compounds identified can be isolated and combined into future iterations of libraries.

Once a library of compounds is screened, subsequent libraries are generated using those chemical building blocks that possess the features shown in the first round of screen to have activity against the target enzyme. Using this method, subsequent iterations of candidate compounds will possess more and more of those structural and functional features required to inhibit the function of the target enzyme, until a group of enzyme inhibitors with high specificity for the enzyme can be found. These compounds can then be further tested for their safety and efficacy as antibiotics for use in mammals.

It will be readily appreciated that this particular screening methodology is exemplary only. Other methods are well known to those skilled in the art. For example, a wide variety of screening techniques are known for a large number of naturally-occurring targets when the biochemical function of the target protein is known.

### B. Cell Based Assays

Current cell-based assays used to identify or to characterize compounds for drug discovery and development frequently depend on detecting the ability of a test compound to inhibit the activity of a target molecule located within a cell or located on the surface of a cell. Most often such target molecules are proteins such as enzymes, receptors and the like. However, target molecules may also include other molecules such as DNAs, lipids, carbohydrates and RNAs including messenger RNAs, ribosomal RNAs, tRNAs and the like. A number of highly sensitive cell-based assay methods are available to those of skill in the art to detect binding and interaction of test compounds with specific target molecules. However, these methods are generally not highly effective when the test compound binds to or otherwise interacts with its target molecule with moderate or low affinity. In addition, the target molecule may not be readily accessible to a test compound in solution, such as when the target molecule is located inside the cell or within a cellular compartment such as the periplasm of a bacterial cell. Thus, current cell-based assay methods are limited in that they are not effective in identifying or characterizing compounds that interact with their targets with moderate to low affinity or compounds that interact with targets that are not readily accessible.

Cell-based assay methods of the present invention have substantial advantages over current cell-based assays practiced in the art. These advantages derive from the use of sensitized cells in which the level or activity of a proliferation-required gene product (the target molecule) has been specifically reduced to the point where the presence or absence of its function becomes a rate-determining step for cellular proliferation. Bacterial, fungal, plant, or animal cells can all be used with the present method. Such sensitized cells become much more sensitive to compounds that are active against the affected target molecule. Thus, cell-based assays of the present invention are capable of detecting compounds exhibiting low or moderate potency against the target molecule of interest because such compounds are substantially more potent on sensitized cells than on non-sensitized cells. The affect may be such that a test compound may be two to several times more potent, at least 10 times more potent or even at least 100 times more potent when tested on the sensitized cells as compared to the non-sensitized cells.

Due in part to the increased appearance of antibiotic resistance in pathogenic microorganisms and to the significant side-effects associated with some currently used antibiotics, novel antibiotics acting at new targets are highly sought after in the art. Yet, another limitation in the current art related to cell-based assays is the problem of identifying hits against the same kinds of target molecules in the same limited set of biological pathways over and over again. This may occur when compounds acting at such new targets are discarded, ignored or fail to be detected because compounds acting at the "old" targets are encountered more frequently and are more potent than compounds acting at the new targets. As a result, the majority of antibiotics in use currently interact with a relatively small number of target molecules within an even more limited set of biological pathways.

The use of sensitized cells of the current invention provides a solution to the above problem in two ways. First, desired compounds acting at a target of interest, whether a new target or a previously known but poorly exploited target, can now be detected above the "noise" of compounds acting at the "old" targets due to the specific and substantial increase in potency of such desired compounds when tested on the sensitized cells of the current invention. Second, the methods used to sensitize cells to compounds acting at a target of interest may also sensitize these cells to compounds acting at other target molecules within the same biological pathway. For example, expression of an antisense molecule to a gene encoding a ribosomal protein is expected to sensitize the cell to compounds acting at that ribosomal protein and may also sensitize the cells to compounds acting at any of the ribosomal components (proteins or rRNA) or even to compounds acting at any target which is part of the protein synthesis pathway. Thus an important advantage of the present invention is the ability to reveal new targets and pathways that were previously not readily accessible to drug discovery methods.

Sensitized cells of the present invention are prepared by reducing the activity or level of a target molecule. The target molecule may be a gene product, such as an RNA or polypeptide produced from the proliferation-required nucleic acids described herein. Alternatively, the target may be a gene product such as an RNA or polypeptide which is produced form a sequence within the same operon as the proliferation-required nucleic acids described herein. In addition, the target may be an RNA or polypeptide in the same biological pathway as the proliferation-required nucleic acids described herein. Such biological pathways include, but are not limited to, enzymatic, biochemical and metabolic pathways as well as pathways involved in the production of cellular structures such the cell wall.

Current methods employed in the arts of medicinal and combinatorial chemistries are able to make use of structure-activity relationship information derived from testing compounds in various biological assays including direct binding assays and cell-based assays. Occasionally compounds are directly identified in such assays that are sufficiently potent to be developed as drugs. More often, initial hit compounds exhibit moderate or low potency. Once a hit compound is identified with low or moderate potency, directed libraries of compounds are synthesized and tested in order to identify more potent leads. Generally these directed libraries are combinatorial chemical libraries consisting of compounds with structures related to the hit compound but containing systematic variations including additions, subtractions and substitutions of various structural features. When tested for activity against the target molecule, structural features are identified that either alone or in combination with other features enhance or reduce activity. This information is used to design subsequent directed libraries containing compounds with enhanced activity against the target molecule. After one or several iterations of this process, compounds with substantially increased activity against the target molecule are identified and may be further developed as drugs. This process is facilitated by use of the sensitized cells of the present invention since compounds acting at the selected targets exhibit increased potency in such cell-based assays, thus; more compounds can now be characterized providing more useful information than would be obtained otherwise.

Thus, it is now possible using cell-based assays of the present invention to identify or characterize compounds that previously would not have been readily identified or characterized including compounds that act at targets that previously were not readily exploited using cell-based assays. The process of evolving potent drug leads from initial hit compounds is also substantially improved by the cell-based assays of the present invention because, for the same number of test compounds, more structure-function relationship information is likely to be revealed.

The method of sensitizing a cell entails selecting a suitable gene or operon. A suitable gene or operon is one whose expression is required for the proliferation of the cell to be sensitized. The next step is to introduce into the cells to be sensitized, an antisense RNA capable of hybridizing to the suitable gene or operon or to the RNA encoded by the suitable gene or operon. Introduction of the antisense RNA can be in the form of an expression vector in which antisense RNA is produced under the control of an inducible promoter. The amount of antisense RNA produced is limited by varying the inducer concentration to which the cell is exposed and thereby varying the activity of the promoter driving transcription of the antisense RNA. Thus, cells are sensitized by exposing them to an inducer concentration that results in a sub-lethal level of antisense RNA expression.

In one embodiment of the cell-based assays, the identified exogenous *E*. *coli* nucleotide sequences of the present invention are used to inhibit the production of a proliferation-required protein. Expression vectors producing antisense RNA against identified genes required for proliferation are used to limit the concentration of a proliferation-required protein without severly inhibiting growth. To achieve that goal, a growth inhibition dose curve of inducer is calculated by plotting various doses of inducer against the corresponding growth inhibition caused by the antisense expression. From this curve, various percentages of antisense induced growth inhibition, from 1 to 100% can be determined. If the promoter contained in the expression vector contains a *lac* operator the transcription is regulated by *lac* repressor and expression from the promoer is inducible with IPTG. For example, the highest concentration of the inducer IPTG that does not reduce the growth rate (0% growth inhibition) can be predicted from the curve. Cellular proliferation can be monitored by growth medium turbidity via OD measurements. In another example, the concentration of inducer that reduces growth by 25% can be predicted from the curve. In still another example, a concentration of inducer that reduces growth by 50% can be calculated. Additional parameters such as colony forming units (cfu) can be used to measure cellular viability.

Cells to be assayed are exposed to the above-determined concentrations of inducer. The presence of the inducer at this sub-lethal concentration reduces the amount of the proliferation required gene product to the lowest amount in the cell that will support growth. Cells grown in the presence of this concentration of inducer are therefore specifically more sensitive to inhibitors of the proliferation-required protein or RNA of interest or to inhibitors of proteins or RNAs in the same biological pathway as the proliferation-required protein or RNA of interest but not to inhibitors of unrelated proteins or RNAs.

Cells pretreated with sub-inhibitory concentrations of inducer and thus containing a reduced amount of proliferation-required target gene product are then used to screen for compounds that reduce cell growth. The sub-lethal concentration of inducer may be any concentration consistent with the intended use of the assay to identify candidate compounds to which the cells are more sensitive. For example, the sub-lethal concentration of the inducer may be such that growth inhibition is at least about 5%, at least about 8%, at least about 10%, at least about 20%, at least about 30%, at least about 40%, at least about 50%, at least about 60% at least about 75%, or more. Cells which are pre-sensitized using the preceding method are more sensitive to inhibitors of the target protein because these cells contain less target protein to inhibit than wild-type cells.
In another embodiment of the cell based assays of the present invention, the level or activity of a proliferation required gene product is reduced using a temperature sensitive ...mutation in the proliferation-required sequence and an antisense nucleic acid against the proliferation-required sequence. Growing the cells at an intermediate temperature between the permissive and restrictive temperatures of the temperature sensitive mutant where the mutation is in a proliferation-required gene produces cells with reduced activity of the proliferation-required gene product. The antisense RNA directed against the proliferation-required sequence further reduces the activity of the proliferation required gene product. Drugs that may not have been found using either the temperature sensitive mutation or the antisense nucleic acid alone may be identified by determining whether cells in which expression of the antisense nucleic acid has been induced and which are grown at a temperature between the permissive temperature and the restrictive temperature are substantially more sensitive to a test compound than cells in which expression of the antisense nucleic acid has not been induced and which are grown at a permissive temperature. Also drugs found previously from either the antisense nucleic acid alone or the temperature sensitive mutation alone may have a different sensitivity profile when used in cells combining the two approaches, and that sensitivity profile may indicate a more specific action of the drug in inhibiting one or more activities of the gene product.

Temperature sensitive mutations may be located at different sites within the gene and correspond to different domains of the protein. For example, the *dnaB* gene of *Escherichia coli* encodes the replication fork DNA helicase. DnaB has several domains, including domains for oligomerization, ATP hydrolysis, DNA binding, interaction with primase, interaction with DnaC, and interaction with DnaA [(Biswas, E.E. and Biswas, S.B. 1999. Mechanism and DnaB helicase of *Escherichia coli*: structural domains involved in ATP hydrolysis, DNA binding, and oligomerization. Biochem. **38**:10919-10928; Hiasa, H. and Marians, K.J. 1999. Initiation of bidirectional replication at the chromosomal origin is directed by the interaction between helicase and primase. J. Biol. Chem. **274**:27244-27248; San Martin, C., Radermacher, M., Wolpensinger, B., Engel, A., Miles, C.S., Dixon, N.E., and Carazo, J.M. 1998. Three-dimensional reconstructions from cryoelectron microscopy images reveal an intimate complex between helicase DnaB and its loading partner DnaC. Structure **6**:501-9; Sutton, M.D., Carr, K.M., Vicente, M., and Kaguni, J.M. 1998. *Escherichia coli* DnaA protein. The N-terminal domain and loading of DnaB helicase at the *E*. *coli* chromosomal. J. Biol. Chem. **273**:34255-62.), the disclosures of which are incorporated herein by reference in their entireties]. Temperature sensitive mutations in different domains of DnaB confer different phenotypes at the restrictive temperature, which include either an abrupt stop or slow stop in DNA replication with or without DNA breakdown (Wechsler, J.A. and Gross, J.D. 1971. *Escherichia coli* mutants temperature-sensitive for DNA synthesis. Mol. Gen. Genetics **113**:273-284, the disclosure of which is incorporated herein by reference in its entirety) and termination of growth or cell death. Combining the use of temperature sensitive mutations in the *dnaB* gene that cause cell death at the restrictive temperature with an antisense to the *dnaB* gene could lead to the discovery of very specific and effective inhibitors of one or a subset of activities exhibited by DnaB.

When screening for antimicrobial agents against a gene product required for proliferation, growth inhibition of cells containing a limiting amount of that proliferation-required gene product can be assayed. Growth inhibition can be measured by directly comparing the amount of growth, measured by the optical density of the growth medium, between an experimental sample and a control sample. Alternative methods for assaying cell proliferation include measuring green fluorescent protein (GFP) reporter construct emissions, various enzymatic activity assays, and other methods well known in the art.

It will be appreciated that the above method may be performed in solid phase, liquid phase or a combination of the two. For example, cells grown on nutrient agar containing the inducer of the antisense construct may be exposed to compounds spotted onto the agar surface. A compound's effect may be judged from the diameter of the resulting killing zone, the area around the compound application point in which cells do not grow. Multiple compounds may be transferred to agar plates and simultaneously tested using automated and semi-automated equipment including but not restricted to multi-channel pipettes (for example the Beckman Multimek) and multi-channel spotters (for example the Genomic Solutions Flexys). In this way multiple plates and thousands to millions of compounds may be tested per day.

The compounds may also be tested entirely in liquid phase using microtiter plates as described below. Liquid phase screening may be performed in microtiter plates containing 96, 384, 1536 or more wells per microtiter plate to screen multiple plates and thousands to millions of compounds per day. Automated and semi-automated equipment may be used for addition of reagents (for example cells and compounds) and determination of cell density.

### EXAMPLE 9

The effectiveness of the above cell based assay was validated using constructs expressing antisense RNA to *E*. *coli* genes rplL, rplJ, and rplW encoding ribosomal proteins L7/L12, L10 and L23 respectively. These proteins are part of the protein synthesis apparatus of the cell and as such are required for proliferation. These constructs were used to test the effect of antisense expression on cell sensitivity to antibiotics known to bind to the ribosome and thereby inhibit protein synthesis. Constructs expressing antisense RNA to several other genes (elaD, visC, yohH, and aptE/B), the products of which are not involved in protein synthesis were used for comparison.

First expression vectors containing antisense constructs to either rplW or to elaD were introduced into separate *E*. *coli* cell populations. Vector introduction is a technique well known to those of ordinary skill in the art. The expression vectors of this example contain IPTG inducible promoters that drive the expression of the antisense RNA in the presence of the inducer. However, those skilled in the art will appreciate that other inducible promoters may also be used. Suitable expression vectors are also well known in the art. The *E. coli* antisense clones encoding ribosomal proteins L7/L12, L10 and L23 were used to test the effect of antisense expression on cell sensitivity to the antibiotics known to bind to these proteins. First, expression vectors containing antisense to either the genes encoding L7/L12 and L10 or L23 were introduced into separate E. coli cell populations.

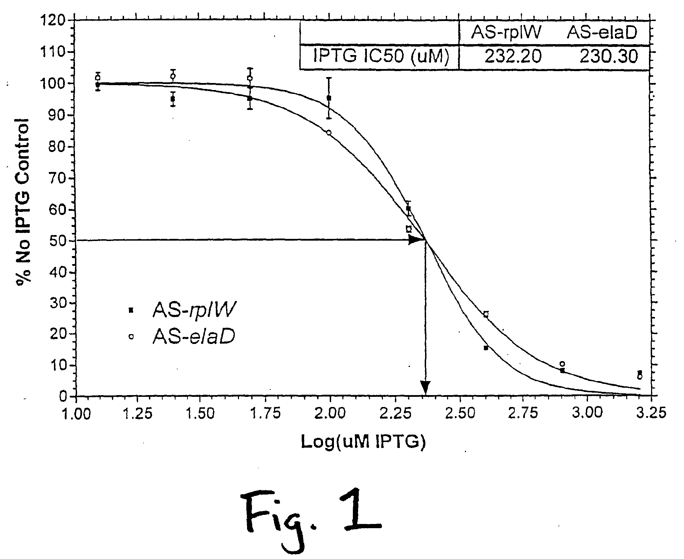

The cell populations were exposed to a range of IPTG concentrations in liquid medium to obtain the growth inhibitory dose curve for each clone (Fig. 1). First, seed cultures were grown to a particular turbidity that is measured by the optical density (OD) of the growth solution. The OD of the solution is directly related to the number of bacterial cells contained therein. Subsequently, sixteen 200 ul liquid medium cultures were grown in a 96 well microtiter plate at 37 C with a range of IPTG concentrations in duplicate two-fold serial dilutions from 1600 uM to 12.5 uM (final concentration). Additionally, control cells were grown in duplicate without IPTG. These cultures were started from equal amounts of cells derived from the same initial seed culture of a clone of interest. The cells were grown for up to 15 hours and the extent of growth was determined by measuring the optical density of the cultures at 600 nm. When the control culture reached mid-log phase the percent growth of the control for each of the IPTG containing cultures was plotted against the log concentrations of IPTG to produce a growth inhibitory dose response curve for the IPTG. The concentration of IPTG that inhibits cell growth to 50% (IC₅₀) as compared to the 0 mM IPTG control (0% growth inhibition) was then calculated from the curve. Under these conditions, an amount of antisense RNA was produced that reduced the expression levels of rplW and elaD to a degree such that growth was inhibited by 50%.

Alternative methods of measuring growth are also contemplated. Examples of these methods include measurements of proteins, the expression of which is engineered into the cells being tested and can readily be measured. Examples of such proteins include green fluorescent protein (GFP) and various enzymes.

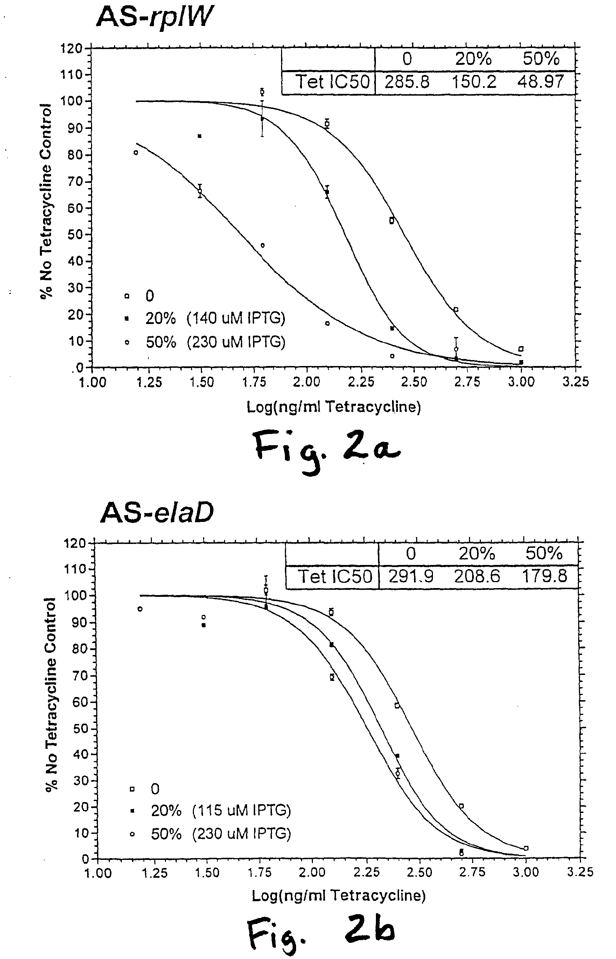

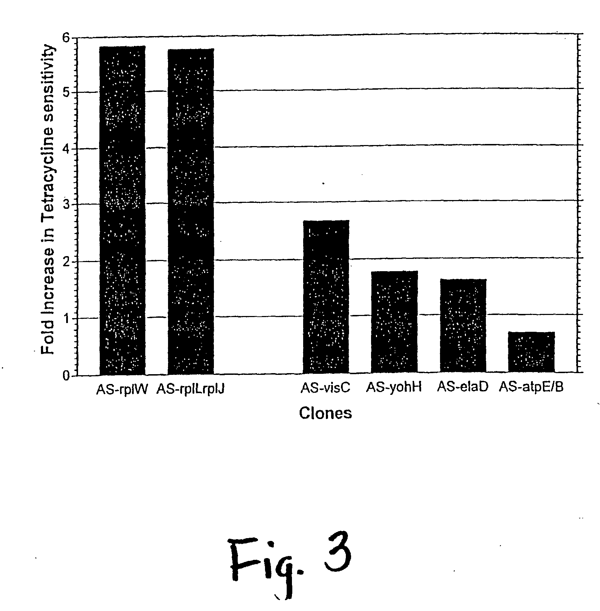

Cells were pretreated with the selected concentration of IPTG and then used to test the sensitivity of cell populations to tetracycline, erythromycin and other protein synthesis inhibitors. An example of a tetracycline dose response curve is shown in Figures 2A and 2B for the rplW and elaD genes, respectively. Cells were grown to log phase and then diluted into media alone or media containing IPTG at concentrations which give 20% and 50% growth inhibition as determined by IPTG dose response curves. After 2.5 hours, the cells were diluted to a final OD600 of 0.002 into 96 well plates containing (1) +/- IPTG at the same concentrations used for the 2.5 hour pre-incubation; and (2) serial two-fold dilutions of tetracycline such that the final concentrations of tetracycline range from 1 µg/ml to 15.6 ng/ml and 0 µg/ml. The 96 well plates were incubated at 37°C and the OD600 was read by a plate reader every 5 minutes for up to 15 hours. For each IPTG concentration and the no IPTG control, tetracycline dose response curves were determined when the control (absence of tetracycline) reached 0.1 OD600. To compare tetracycline sensitivity with and without IPTG, tetracycline IC50s were determined from the dose response curves (Figs. 2A-B). Cells with reduced levels of L23 (rplW) showed increased sensitivity to tetracycline (Fig. 2A) as compared to cells with reduced levels of elaD (Fig. 2B). Figure 3 shows a summary bar chart in which the ratios of tetracycline IC50s determined in the presence of IPTG which gives 50% growth inhibition versus tetracycline IC50s determined without IPTG (fold increase in tetracycline sensitivity) were plotted. Cells with reduced levels of either L7/L12 (genes *rplL, rplJ*) or L23 (*rp/W*) showed increased sensitivity to tetracycline (Fig. 3). Cells expressing antisense to genes not known to be involved in protein synthesis (*atpB/E, visC, elaD, yohH*) did not show the same increased sensitivity to tetracycline, validating the specificity of this assay (Fig. 3).

In addition to the above, it has been observed in initial experiments that clones expressing antisense RNA to genes involved in protein synthesis (including genes encoding ribosomal proteins L7/L12 & L10, L7/L12 alone, L22, and L18, as well as genes encoding rRNA and Elongation Factor G) have increased sensitivity to the macrolide, erythromycin, whereas clones expressing antisense to the non-protein synthesis genes elaD, atpB/E and visC do not. Furthermore, the clone expressing antisense to rplL and rplJ does not show increased sensitivity to nalidixic acid and ofloxacin, antibiotics which do not inhibit protein synthesis.

The results with the ribosomal protein genes rplL, rplJ, and rplW as well as the initial results using various other antisense clones and antibiotics show that limiting the concentration of an antibiotic target makes cells more sensitive to the antimicrobial agents that specifically interact with that protein. The results also show that these cells are sensitized to antimicrobial agents that inhibit the overall function in which the protein target is involved but are not sensitized to antimicrobial agents that inhibit other functions.

The cell based assay described above may also be used to identify the biological pathway in which a proliferation-required nucleic acid or its gene product lies. In such methods, cells expressing a sub-lethal level of antisense to a target proliferation-required nucleic acid and control cells in which expression of the antisense has not been induced are contacted with a panel of antibiotics known to act in,various pathways. If the antibiotic acts in the pathway in which the target proliferation-required nucleic acid or its gene product lies, cells in which expression of the antisense has been induced will be more sensitive to the antibiotic than cells in which expression of the antisense has not been induced.

As a control, the results of the assay may be confirmed by contacting a panel of cells expressing antisense nucleic acids to many different proliferation-required genes including the target proliferation-required gene. If the antibiotic is acting specifically, heightened sensitivity to the antibiotic will be observed only in the cells expressing antisense to a target proliferation-required gene (or cells expressing antisense to other proliferation-required genes in the same pathway as the target proliferation-required gene) but will not be observed generally in all cells expressing antisense to proliferation-required genes.

Similarly, the above method may be used to determine the pathway on which a test antibiotic acts. A panel of cells, each of which expresses antisense to a proliferation-required nucleic acid in a known pathway, is contacted with a compound for which it is desired to determine the pathway on which it acts. The sensitivity of the panel of cells to the test compound is determined in cells in which expression of the antisense has been induced and in control cells in which expression of the antisense has not been induced. If the test antibiotic acts on the pathway on which an antisense nucleic acid acts, cells in which expression of the antisense has been induced will be more sensitive to the antibiotic than cells in which expression of the antisense has not been induced. In addition, control cells in which expression of antisense to proliferation-required genes in other pathways has been induced will not exhibit heightened sensitivity to the antibiotic. In this way, the pathway on which the test antibiotic acts may be determined.

The Example below provides one method for performing such assays.

### EXAMPLE 10

### Identification of thePathway in which a Proliferation-Required Gene Lies or the Pathway on which an Antibiotic Acts

### A. Preparation of Bacterial Stocks for Assay

To provide a consistent source of cells to screen, frozen stocks of host bacteria containing the desired antisense construct are prepared using standard microbiological techniques. For example, a single clone of the organism can be isolated by streaking out a sample of the original stock onto an agar plate containing nutrients for cell growth and an antibiotic for which the antisense construct contains a gene which confers resistance. After overnight growth an isolated colony is picked from the plate with a sterile needle and transferred to an appropriate liquid growth media containing the antibiotic required for maintenance of the plasmid. The cells are incubated at 30°C to 37°C with vigorous shaking for 4 to 6 hours to yield a culture in exponential growth. Sterile glycerol is added to 15% (volume to volume) and 100µL to 500 µL aliquots are distributed into sterile cryotubes, snap frozen in liquid nitrogen, and stored at -80°C for future assays.

### B. Growth of Bacteria for Use in the Assay

A day prior to an assay, a stock vial is removed from the freezer, rapidly thawed (37°C water bath) and a loop of culture is streaked out on an agar plate containing nutrients for cell growth and an antibiotic to which the antisense construct confers resistance. After overnight growth at 37°C, ten randomly chosen, isolated colonies are transferred from the plate (sterile inoculum loop) to a sterile tube containing 5 mL of LB medium containing the antibiotic to which the antisense vector confers resistance. After vigorous mixing to form a homogeneous cell suspension, the optical density of the suspension is measured at 600 nm (OD600) and if necessary an aliquot of the suspension is diluted into a second tube of 5 mL, sterile, LB medium plus antibiotic to achieve an OD600 ≤ 0.02 absorbance units. The culture is then incubated at 37° C for 1-2 hrs with shaking until the OD600 reaches OD 0.2 - 0.3. At this point the cells are ready to be used in the assay.

### C. Selection of Media to be Used in Assay

Two fold dilution series of the inducer are generated in culture media containing the appropriate antibiotic for maintenance of the antisense construct. Several media are tested side by side and three to four wells are used to evaluate the effects of the inducer at each concentration in each media. For example, M9 minimal media, LB broth, TBD broth and Muller-Hinton media may be tested with the inducer IPTG at the following concentrations, 50 µM, 100 µM, 200 µM, 400 µM, 600 µM, 800 µM and 1000 µM. Equal volumes of test media-inducer and cells are added to the wells of a 384 well microtiter plate and mixed. The cells are prepared as described above and diluted 1:100 in the appropriate media containing the test antibiotic immediately prior to addition to the microtiter plate wells. For a control, cells are also added to several wells of each media that do not contain inducer, for example 0 M IPTG. Cell growth is monitored continuously by incubation at 37°C in a microtiter plate reader monitoring the OD600 of the wells over an 18-hour period. The percent inhibition of growth produced by each concentration of inducer is calculated by comparing the rates of logarithmic growth against that exhibited by cells growing in media without inducer. The medium yielding greatest sensitivity to inducer is selected for use in the assays described below.

### D. Measurement of Test Antibiotic Sensitivity in the Absence of Antisense Construct Induction

Two-fold dilution series of antibiotics of known mechanism of action are generated in the culture media selected for further assay development that has been supplemented with the antibiotic used to maintain the construct. A panel of test antibiotics known to act on different pathways is tested side by side with three to four wells being used to evaluate the effect of a test antibiotic on cell growth at each concentration. Equal volumes of test antibiotic and cells are added to the wells of a 384 well microtiter plate and mixed. Cells are prepared as described above using the media selected for assay development supplemented with the antibiotic required to maintain the antisense construct and are diluted 1:100 in identical media immediately prior to addition to the microtiter plate wells. For a control, cells are also added to several wells that contain the solvent used to dissolve the antibiotics but no antibiotic. Cell growth is monitored continuously by incubation at 37°C in a microtiter plate reader monitoring the OD600 of the wells over an 18-hour period. The percent inhibition of growth produced by each concentration of antibiotic is calculated by comparing the rates of logarithmic growth against that exhibited by cells growing in media without antibiotic. A plot of percent inhibition against loglantibiotic concentration] allows extrapolation of an IC₅₀ value for each antibiotic.

### E. Measurement of Test Antibiotic Sensitivity in the Presence of Antisense Construct Inducer

The culture media selected for use in the assay is supplemented with inducer at concentrations shown to inhibit cell growth by 50 and 80% as described above and the antibiotic used to maintain the construct. Two fold dilution series of the panel of test antibiotics used above are generated in each of these media. Several antibiotics are tested side by side with three to four wells being used to evaluate the effects of an antibiotic on cell growth at each concentration, in each media. Equal volumes of test antibiotic and cells are added to the wells of a 384 well microtiter plate and mixed. Cells are prepared as described above using the media selected for use in the assay supplemented with the antibiotic required to maintain the antisense construct. The cells are diluted 1:100 into two 50 mL aliquots of identical media containing concentrations of inducer that have been shown to inhibit cell growth by 50% and 80 % respectively and incubated at 37°C with shaking for 2.5 hours. Immediately prior to addition to the microtiter plate wells, the cultures are adjusted to an appropriate OD₆₀₀ (typically 0.002) by dilution into warm (37°C) sterile media supplemented with identical concentrations of the inducer and antibiotic used to maintain the antisense construct. For a control, cells are also added to several wells that contain solvent used to dissolve test antibiotics but which contain no antibiotic. Cell growth is monitored continuously by incubation at 37°C in a microtiter plate reader monitoring the OD600 of the wells over an 18-hour period. The percent inhibition of growth produced by each concentration of antibiotic is calculated by comparing the rates of logarithmic growth against that exhibited by cells growing in media without antibiotic. A plot of percent inhibition against log[antibiotic concentration] allows extrapolation of an IC₅₀ value for each antibiotic.

### F. Determining the Specificity of the Test Antibiotics

A comparison of the IC₅₀s generated by antibiotics of known mechanism of action under antisense induced and non-induced conditions allows the pathway in which a proliferation-required nucleic acid lies to be identified. If cells expressing an antisense nucleic acid against a proliferation-required gene are selectively sensitive to an antibiotic acting via a particular pathway, then the gene against which the antisense acts is involved in the pathway in which the antibiotic acts.

### G. Identification of Pathway in which a Test Antibiotic Acts

As discussed above, the cell based assay may also be used to determine the pathway against which a test antibiotic acts. In such an analysis, the pathways against which each member of a panel of antisense nucleic acids acts are identified as described above. A panel of cells, each containing an inducible antisense vector against a gene in a known proliferation-required pathway, is contacted with a test antibiotic for which it is desired to determine the pathway on which it acts under inducing an non-inducing conditions. If heightened sensitivity is observed in induced cells expressing antisense against a gene in a particular pathway but not in induced cells expressing antisense against genes in other pathways, then the test antibiotic acts against the pathway for which heightened sensitivity was observed.

One skilled in the art will appreciate that further optimization of the assay conditions, such as the concentration of inducer used to induce antisense expression andlor the growth conditions used for the assay (for example incubation temperature and media components) may further increase the selectivity and/or magnitude of the antibiotic sensitization exhibited.

The following example confirms the effectiveness of the methods described above.

### EXAMPLE 11

### Identification of the Pathway in which a Proliferation-Required Gene Lies

Antibiotics of various chemical classes and modes of action were purchased from Sigma Chemicals (St. Louis, MO). Stock solutions were prepared by dissolving each antibiotic in an appropriate aqueous solution based on information provided by the manufacturer. The final working solution of each antibiotic contained no more than 0.2% (w/v) of any organic solvent. To determine their potency against a bacterial strain engineered for expression of an antisense against a proliferation-required 50S ribosomal protein, each antibiotic was serially diluted two or three fold in growth medium supplemented with the appropriate antibiotic for maintenance of the anti-sense construct. At least ten dilutions were prepared for each antibiotic. 25 µL aliquots of each dilution were transferred to discrete wells of a 384-welt microplate (the assay plate) using a multi-channel pipette. Quadruplicate wells were used for each dilution of an antibiotic under each treatment condition (plus and minus inducer). Each assay plate contained twenty wells for cell growth controls (growth media replacing antibiotic), ten wells for each treatment (plus and minus inducer, in this example IPTG). Assay plates were usually divided into the two treatments: half the plate containing induced cells and an appropriate concentrations of inducer (in this example IPTG) to maintain the state of induction, the other half containing non-induced cells in the absence of IPTG.

Cells for the assay were prepared as follows. Bacterial cells containing a construct, from which expression of antisense nucleic acid against rplL and rplJ, which encode proliferation-required 50S ribosomal subunit proteins, is inducible in the presence of IPTG, were grown into exponential growth (OD₆₀₀ 0.2 to 0.3) and then diluted 1:100 into fresh media containing either 400 µM or 0 µM inducer (IPTG). These cultures were incubated at 37° C for 2.5 hr. After a 2.5 hr incubation, induced and non-induced cells were respectively diluted into an assay medium at a final OD₆₀₀ value of 0.0004. The medium contained an appropriate concentration of the antibiotic for the maintenance of the anti-sense construct. In addition, the medium used to dilute induced cells was supplemented with 800 µM IPTG so that addition to the assay plate would result in a final IPTG concentration of 400 µM. Induced and non-induced cell suspensions were dispensed (25 µl/well) into the appropriate wells of the assay plate as discussed previously. The plate was then loaded into a plate reader, incubated at constant temperature, and cell growth was monitored in each well by the measurement of light scattering at 595 nm. Growth was monitored every 5 minutes until the cell culture attained a stationary growth phase. For each concentration of antibiotic, a percentage inhibition of growth was calculated at the time point corresponding to mid-exponential growth for the associated control wells (no antibiotic, plus or minus IPTG). For each antibiotic and condition (plus or minus IPTG), a plot of percent inhibition versus log of antibiotic concentration was generated and the IC50 determined. A comparison of the IC₅₀ for each antibiotic in the presence and absence of IPTG revealed whether induction of the antisense construct sensitized the cell to the mechanism of action exhibited by the antibiotic. Cells which exhibited a significant (standard statistical analysis) numerical decrease in the IC₅₀ value in the presence of inducer were considered to have an increased sensitivity to the test antibiotic.

The results are provided in the table below, which lists the classes and names of the antibiotics used in the analysis, the targets of the antibiotics, the IC50 in the absence of IPTG, the IC50 in the presence of IPTG, the concentration units for the IC50s, the fold increase in IC50 in the presence of IPTG, and whether increased sensitivity was observed in the presence of IPTG.

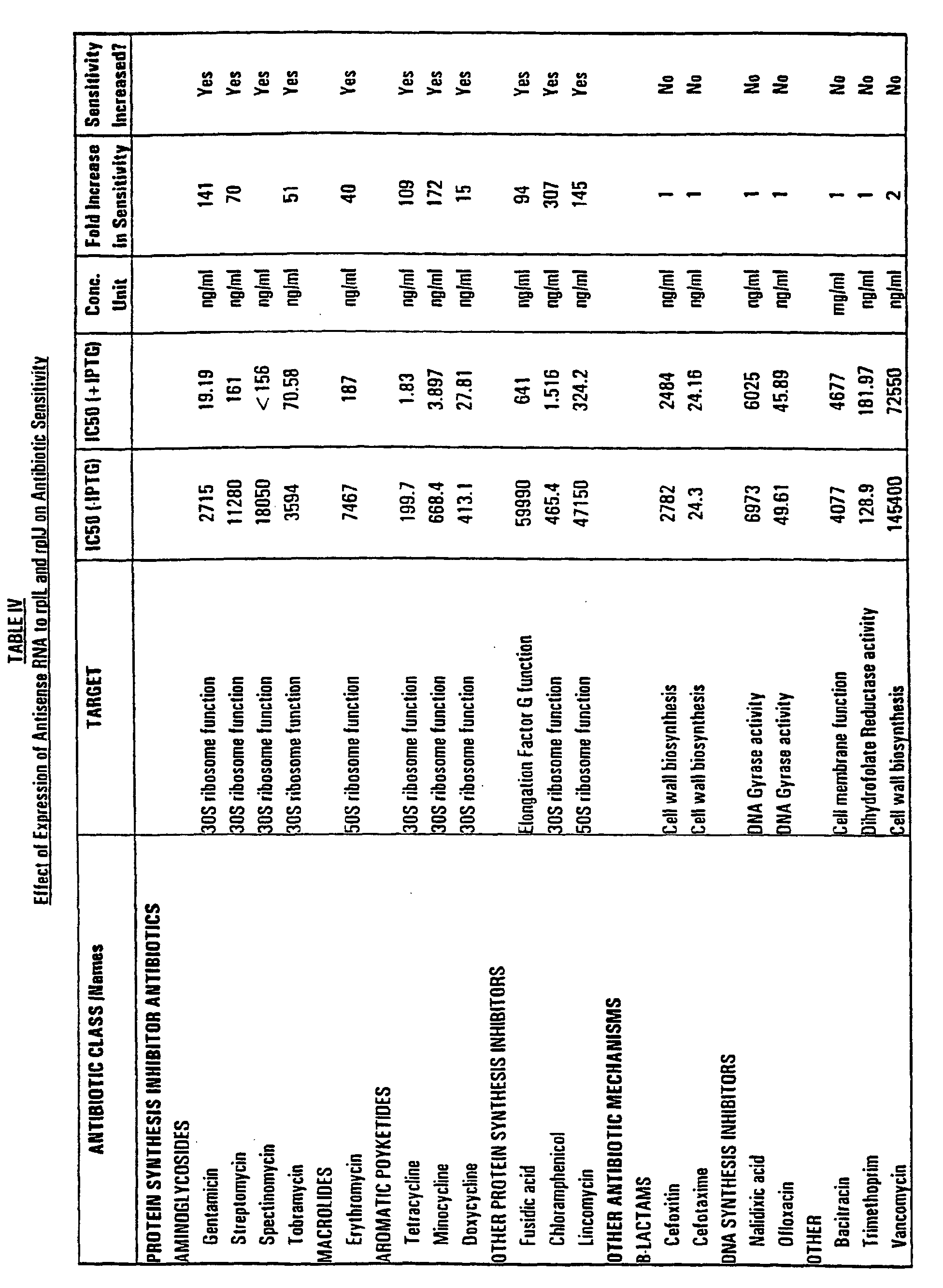

The above results demonstrate that induction of an antisense RNA to genes encoding 50S ribosomal subunit proteins results in a selective and highly significant sensitization of cells to antibiotics that inhibit ribosomal function and protein synthesis. The above results further demonstrate that induction of an antisense construct to an essential gene sensitizes an organism to compounds that interfere with that gene products' biological role. This sensitization is restricted to compounds that interfere with pathways associated with the targeted gene and it's product.

Assays utilizing antisense constructs to essential genes can be used to identify compounds that specifically interfere with the activity of multiple targets in a pathway. Such constructs can be used to simultaneously screen a sample against multiple targets in one pathway in one reaction (Combinatorial HTS).

Furthermore, as discussed above, panels of antisense construct containing cells may be used to characterize the point of intervention of any compound affecting an essential biological pathway including antibiotics with no known mechanism of action.

Another embodiment of the present invention is a method for determining the pathway against which a test antibiotic compound is active in which the activity of target proteins or nucleic acids involved in proliferation-required pathways is reduced by contacting cells with a sublethal concentration of a known antibiotic which acts against the target protein or nucleic acid. In one embodiment, the target protein or nucleic acid is a target protein or nucleic acid corresponding to a proliferation-required nucleic acid identified using the methods described above. The method is similar to those described above for determining which pathway a test antibiotic acts against except that rather than reducing the activity or level of a proliferation-required gene product using a sublethal level of antisense to a proliferation-required nucleic acid, the activity or level of the proliferation-required gene product is reduced using sublethal level of a known antibiotic which acts against the proliferation required gene product.

Interactions between drugs which affect the same biological pathway has been described in the literature. For example, Mecillinam (Amdinocillin) binds to and inactivates the penicillin binding protein 2 (PBP2, product of the *mrdA* in *E*. *coli*). This antibiotic inteacts with other antibiotics that inhibit PBP2 as well as antibiotics that inhibit other penicillin binding proteins such as PBP3 [(Gutmann, L., Vincent, S., Billot-Klein, D., Acar, J.F., Mrena, E., and Williamson, R. (1986) Involvement of penicillin-binding protein 2 with other penicillin-binding proteins in lysis of *Escherichia coli* by some betalactam antibiotics alone and in synergistic lytic effect of amdinocillin (mecillinam). Antimicrobial Agents & Chemotherapy, 30:906-912), the disclosure of which is incorporated herein by reference in its entirety]. Interactions between drugs could, therefore, involve two drugs that inhibit the same target protein or nucleic acid or inhibit different proteins or nucleic acids in the same pathway [(Fukuoka, T., Domon, H., Kakuta, M., Ishii, C., Hirasawa, A., Utsui, Y., Ohya, S., and Yasuda, H. (1997) Combination effect between panipenem and vancomycin on highly methicillin-resistant Staphylococcus aureus. Japan. J. Antibio. 50:411-419; Smith, C.E., Foleno, B.E., Barrett, J.F., and Frosc, M.B. (1997) Assessment of the synergistic interactions of levofloxacin and ampicillin against Enterococcus faecium by the checkerboard agar dilution and time-kill methods. Diagnos. Microbiol. Infect. Disease 27:85-92; den Hollander, J.G., Horrevorts, A.M., van Goor, M.L, Verbrugh, H.A., and Mouton, J.W. (1997) Synergism between tobramycin and ceftazidime against a resistant Pseudomonas aeruginosa strain, tested in an in vitro pharmacokinetic model. Antimicrobial Agents & Chemotherapy. 41:95-110), the disclosure of all of which are incorporated herein by reference in their entireties].

Two drugs may interact even though they inhibit different targets. For example, the proton pump inhibitor, Omeprazole, and the antibiotic, Amoxycillin, two synergistic compounds acting together, can cure *Helicobacter pylori* infection [( Gabryelewicz, A., Laszewicz, W., Dzieniszewski, J., Ciok, J., Marlicz, K., Bielecki, D., Popiela, T., Legutko, J., Knapik, Z., Poniewierka, E. (1997) Multicenter evaluation of dual-therapy (omeprazol and amoxycillin) for *Helicobacter pylori*-associated duodenal and gastric ulcer (two years of the observation). J. Physiol. Pharmacol. 48 Suppl 4:93-105), the disclosure of which is incorporated herein by reference in its entirety].

The growth inhibition from the sublethal concentration of the known antibiotic may be at least about 5%, at least about 8%, at least about 10%, at least about 20%, at least about 30%, at least about 40%, at least about 50%, at least about 60%, or at least about 75%, or more.

Alternatively, the sublethal concentration of the known antibiotic may be determined by measuring the activity of the target proliferation-required gene product rather than by measuring growth inhibition.

Cells are contacted with a combination of each member of a panel of known antibiotics at a sublethal level and varying concentrations of the test antibiotic. As a control, the cells are contacted with varying concentrations of the test antibiotic alone. The IC₅₀ of the test antibiotic in the presence and absence of the known antibiotic is determined. If the IC50s in the presence and absence of the known drug are substantially similar, then the test drug and the known drug act on different pathways. If the IC₅₀s are substantially different, then the test drug and the known drug act on the same pathway.

Another embodiment of the present invention is a method for identifying a candidate compound for use as an antibiotic in which the activity of target proteins or nucleic acids involved in proliferation-required pathways is reduced by contacting cells with a sublethal concentration of a known antibiotic which acts against the target protein or nucleic acid. In one embodiment, the target protein or nucleic acid is a target protein or nucleic acid corresponding to a proliferation-required nucleic acid identified using the methods described above. The method is similar to those described above for identifying candidate compounds for use as antibiotics except that rather than reducing the activity or level of a proliferation-required gene product using a sublethal level of antisense to a proliferation-required nucleic acid, the activity or level of the proliferation-required gene product is reduced using a sublethal level of a known antibiotic which acts against the proliferation required gene product.

The growth inhibition from the sublethal concentration of the known antibiotic may be at least about 5%, at least about 8%, at least about 10%, at least about 20%, at least about 30%, at least about 40%, at least about 50%, at least about 60%, or at least about 75%, or more.

Alternatively, the sublethal concentration of the known antibiotic may be determined by measuring the activity of the target proliferation-required gene product rather than by measuring growth inhibition.

In order to characterize test compounds of interest, cells are contacted with a panel of known antibiotics at a sublethal level and one or more concentrations of the test compound. As a control, the cells are contacted with the same concentrations of the test compound alone. The IC₅₀ of the test compound in the presence and absence of the known antibiotic is determined. If the IC₅₀ of the test compound is substantially different in the presence and absence of the known drug then the test compound is a good candidate for use as an antibiotic. As discussed above, once a candidate compound is identified using the above methods its structure may be optimized using standard techniques such as combinatorial chemistry.

Representative known antibiotics which may be used in each of the above methods are provided in the table below. However, it will be appreciated that other antibiotics may also be used.

| **ANTIBIOTIC** | **INHIBITS/TARGET** | **RESISTANT MUTANTS** |
|---|---|---|
| **Inhibitors of Transcription** | | |
| Rifamycin, 1959 Rifampicin Rifabutin Rifaximin | Inhibits initiation of transcription/β-subunit RNA polymerase, *rpoB* | *rpoB, crp, cyaA* |
| Streptolydigin | Accelerates transcription chain termination/β. subunit RNA polymerase | *rpoB* |
| Streptovaricin | an acyclic ansamycin, inhibits RNA polymerase | *rpoB* |
| Actinomycin D+EDTA | Intercalates between 2 successive G-C pairs, *rpoB*, inhibits RNA synthesis | *pldA* |

| **Inhibitors of Nucleic Acid Metabolism** | | |
|---|---|---|
| Quinolones, 1962 Nalidixic acid | subunit gyrase andlor topoisomerase IV, *gyrA* | |
| Oxolinic acid | | *gyrAorB*, *icd*, *sloB* |
| Fluoroquinolones Ciprofloxacin, 1983 Norfloxacin | subunit gyrase, *gyrA* andlor topoisomerase IV (probable target in Staph) | *gyrA* |
| | | *norA* (efflux in Staph) |
| | | *hipQ* |
| Coumerins Novobiocin | Inhibits ATPase activity of β-subunit gyrase, *gyrB* | *gyrB, cysB, cysE, nov, ompA* |
| Coumermycin | Inhibits ATPase activity of β-subunit gyrase, *gyrB* | *gyrB, hisW* |
| Albicidin | DNA synthesis | *tsx* (nucleoside channel) |
| Metronidazole | Causes single-strand breaks in DNA | *nar* |

| **Inhibitors of Metabolic Pathways** | | |
|---|---|---|
| Sulfonamides, 1932 Sulfanilamide | blocks synthesis of dihydrofolate,dihydropteroate synthesis, *folP* | *folP, gpt, pabA, pabB*, *pabC* |
| Trimethoprim, 1962 | Inhibits dihydrofolate reductase, *folA* | *folA, thyA* |
| Showdomycin | Nucleoside analogue capable of alkylating | *nupC, pnp* |
| | sulfhydryl groups, inhibitor of thymidylate synthetase | |
| Thiolactomycin | type II fatty acid synthase inhibitor | *emrB* |
| | | *fadB, emrB* due to gene dosage |
| Psicofuranine | Adenosine glycoside antibiotic, target is GMP synthetase | *guaA,B* |
| Triclosan | Inhibits fatty acid synthesis | *fabl(envM)* |
| Diazoborines Isoniazid, Ethionamide | heterocyclic, contains boron, inhibit fatty acid synthesis, enoyl-ACP reductase, *fabl* | *fabl(envM)* |

| **Inhibitors of Translation** | | |
|---|---|---|
| Phenylpropanoids Chloramphenicol, 1947 | Binds to ribosomal peptidyl transfer center preventing peptide translocation/ binds to S6, L3, L6, L14, L16, L25, L26, L27, but preferentially to L16 | *rrn, cmlA, marA, ompF, ompR* |
| Tetracyclines, 1948, type II | Binding to 30S ribosomal subunit, "A" site on 30S subunit, blocks peptide elongation, strongest binding to S7 | *clmA (cmr), mar, ompF* |
| polyketides | | |
| Minocycline | | |
| Doxycycline | | |
| Macrolides (type I polyketides) Erythromycin, 1950 | Binding to 50 S ribosomal subunit, 23S rRNA, blocks peptide translocation, L15, L4, L12 | |
| Carbomycin, Spiramycin | | *rrn, rplC, rplD, rplV, mac* |
| etc | | |
| Aminoglycosides Streptomycin, 1944 | Irreversible binding to 30S ribosomal subunit, prevents translation or causes mistranslation of | *rpsL*, *strC,M, ubiF* |
| Neomycin | mRNA/16S rRNA | *atpA-E, ecfB, hemAC,D,E,G, topA,* |
| Spectinomycin Kanamycin | | *rpsC,D,E, rrn, spcB atpA-atpE, cpxA, ecfB, hemA,B,L, topA* |
| Kasugamycin | | *ksgA,B,C,D, rplB,K, rpsl,N,M,R* |
| Gentamicin, 1963 | | *rplF*, *ubiF* |
| Amikacin | | *cpxA* |
| Paromycin | | *rpsL* |
| Lincosamides Lincomycin, 1955 Clindamycin | Binding to 50 S ribosomal subunit, blocks peptide translocation | *linB, rplN,0, rpsG* |
| Streptogramins Virginiamycin, 1955 Pristinamycin | 2 components, Streptogramins A&B, bind to the 50S ribosomal subunit blocking peptide | |
| Synercid: quinupristin /dalfopristin | translocation and peptide bond formation | |
| Fusidanes Fusidic Acid | Inhibition of elongation factor G (EF.G) prevents peptide translocation | *fusA* |
| Kirromycin (Mocimycin) | Inhibition of elongation factor TU (EF-Tu), prevents peptide bond formation | *tufA,B* |
| Pulvomycin | Binds to and inhibits EF-TU | |
| Thiopeptin | Sulfur-containing antibiotic, inhibits protein synthesis,EF-G | *rplE* |
| Tiamulin | Inhibits protein synthesis | *iplC, rplD* |
| Negamycin | Inhibits termination process of protein synthesis | *prfB* |
| Oxazolidinones Linezolid Isoniazid | 23S rRNA | |
| | | *pdx* |
| Nitrofurantoin | Inhibits protein synthesis, nitroreductases convert nitrofurantoin to highly reactive electrophilic intermediates which attack bacterial ribosomal proteins non-specifically | *nfnA,B* |
| Pseudomonic Acids Mupirocin (Bactroban) | Inhibition of isoleucyl tRNA synthetase-used for Staph, topical cream, nasal spray | *ileS* |
| Indolmycin | Inhibits tryptophanyl-tRNA synthetase | *trpS* |
| Viomycin | | *rrmA* (23S rRNA |
| | | methyltransferase; |
| | | mutant has slow |
| | | growth rate, slow chain |
| | | elongation rate, and |
| | | viomycin resistance) |
| Thiopeptides | Binds to L11-23S RNA complex | |
| Thiostrepton | Inhibits GTP hydrolysis by EF-G | |
| Micrococcin | Stimulates GTP hydrolysis by EF-G | |

| **Inhibitors of Cell Walls/Membranes** | | |
|---|---|---|
| β-lactams | Inhibition of one or more cell wall | |
| Penicillin, 1929 Ampicillin | transpeptidases, endopeptidases, and glycosidases (PBPs), of the 12 PBPs only 2 are essential: *mrdA* (PBP2) and *ftsl (pbpB,* PBP3) | *ampC, ampD, ampE,* |
| Methicillin, 1960 | | *envZ, galU, hipA,* |
| | | *hipQ, ompC, ompF,* |
| | | *ompR, ptsI, rfa, tolD,* |
| | | *tolE* |
| Cephalosporins, 1962 | | *tonB* |
| Mecillinam (amdinocillin) | Binds to and inactivates PBP2 (*mrdA*) Inactivates PBP3 (*ftsl*) Inactivates PBP3 (*ftsl*) | *alaS, argS, crp, cyaA,* *envB, mrdA,B*, *envB, mrdA,B,* |
| Aztreonam (Furazlocillin) | | *mreB,C,D* |
| Bacilysin, Tetaine | Dipeptide, inhib glucosamine synthase | *dppA* |
| Glycopeptides Vancomycin, 1955 | Inhib G+ cell wall syn, binds to terminal D-ala-D-ala of pentapeptide, | |
| Polypeptides Bacitracin | Prevents dephosphorylation and regeneration of lipid carrier | *rfa* |
| Cyclic lipopeptide Daptomycin, 1980 | Disrupts multiple aspects of membrane | |
| | function, including peptidoglycan synthesis, lipoteichoic acid synthesis, and the bacterial membrane potential | |
| Cyclic polypeptides Polymixin, 1939 | Surfactant action disrupts cell membrane lipids, binds lipid A mioety of LPS | *pmrA* |
| Fosfomycin, 1969 i | Analogue of P-enolpyruvate, inhibits 1^{st} step in peptidoglycan synthesis - UOP-N- acetylglucosamine enolpyruvyl transferase, *murA*. Also acts as Immunosuppressant | *murA*, *crp*, *cyaA glpT,* *hipA, ptsI, uhpT* |
| Cycloserine | Prevents formation of D-ala dimer, inhibits D-ala ligase, *ddlA,B* | *hipA, cycA* |
| Alafosfalin | phosphonodipeptide, cell wall synthesis inhibitor, potentiator of lactams | *pepA, tpp* |

| **Inhibitors of Protein Processing/Transport** | | |
|---|---|---|
| Globomycin | Inhibits signal peptidase II (cleaves prolipoproteins subsequent to lipid modification. *lspA* | *lpp, dnaE* |

### EXAMPLE 12

### Transfer of Exogenous Nucleic Acid Sequences to other Bacterial Species Using the E. coli Expression Vectors or Expression Vectors Functional in Bacterial Species other than E. coli.

The above methods were validated using antisense nucleic acids which inhibit the growth of *E*. *coli* which were identified using methods similar to those described above. Expression vectors which inhibited growth of E. coli upon induction of antisense RNA expression with IPTG were transformed directly into *Enterobacter cloacae, Klebsiella pneumonia* or *Salmonella typhimurium*. The transformed cells were then assayed for growth inhibition according to the method of Example 1. After growth in liquid culture, cells were plated at various serial dilutions and a score determined by calculating the log difference in growth for INDUCED vs. UNINDUCED antisense RNA expression as determined by the maximum 10 fold dilution at which a colony was observed. The results of these experiments are listed below in Table VI. If there was no effect of antisense RNA expression in an organism, the clone is minus in Table VI. In contrast, a positive in Table VI means that at least 10 fold more cells were required to observe a colony on the induced plate than on the non-induced plate under the conditions used and in that organism.

Sixteen of the construts were found to inhibit growth in all the organisms tested upon induction of antisense RNA expression with IPTG. Those skilled in the art will appreciate that a negative result in a heterologous organism does not mean that that organism is missing that gene nor does it mean that the gene is unessential. However, a positive result means that the heterologous organism contains a homologous gene which is required for proliferation of that organism. The homologous gene may be obtained using the methods described herein. Those cells that are inhibited by antisense may be used in cell based assays as described herein for the identification and characterization of compounds in order to develop antibiotics effective in these organisms. Those skilled in the art will appreciate that an antisense molecule which works in the organism from which it was obtained will not always work in a heterologous organism.

**TABLE VI**

| Sensitivity of Other Microorganisms to Antisense Nucleic Acids That Inhibit Proliferation in *E. coli* | | | |
|---|---|---|---|
| **Mol. No.** | ***S*. *typhimurium*** | ***E. cloacae*** | ***K. pneumoniae*** |
| EcXA001 | + | + | - |
| EcXA004 | - | - | - |
| EcXA005 | + | + | + |
| EcXA006 | - | - | - |
| EcXA007 | - | + | - |
| EcXA008 | + | - | + |
| EcXA010 | + | + | + |
| EcXA011 | - | + | - |
| EcXA012 | - | + | - |
| EcXA013 | + | + | + |
| EcXA014 | + | + | - |
| EcXA015 | - | + | + |
| EcXA016 | + | + | + |
| EcXA017 | + | + | + |
| EcXA018 | + | + | + |
| EcXA019 | + | + | + |
| EcXA020 | + | + | + |
| EcXA021 | + | + | + |
| EcXA023 | + | + | + |
| EcXA024 | + | - | + |
| EcXA025 | - | - | - |
| EcXA026 | + | + | - |
| EcXA027 | + | + | + |
| EcXA028 | + | - | - |
| EcXA029 | - | - | - |
| EcXA030 | + | + | + |
| EcXA031 | + | - | - |
| EcXA032 | + | - | - |
| EcXA033 | + | + | + |
| EcXA034 | + | + | + |
| EcXA035 | - | - | - |
| EcXA036 | + | - | + |
| EcXA037 | - | + | - |
| EcXA038 | + | + | - |
| EcXA039 | + | - | - |
| EcXA041 | + | + | + |
| EcXA042 | - | + | + |
| EcXA044 | - | - | - |
| EcXA045 | - | + | - |
| EcXA046 | - | - | - |
| EcXA047 | + | + | - |
| EcXA048 | - | - | - |
| EcXA049 | + | - | - |
| EcXA050 | - | - | - |
| EcXA051 | + | - | - |
| EcXA052 | + | - | - |
| EcXA053 | + | + | + |
| EcXA054 | - | - | + |
| EcXA055 | + | - | - |

### EXAMPLE 13

### Use of Identified Exogenous Nucleic Acid Sequences as Probes

The identified sequence of the present invention can be used as probes to obtain the sequence of additional genes of interest from a second organism. For example, probes to potential bacterial target proteins may be hybridized to nucleic acids from other organisms including other bacteria and higher organisms, to identify homologous sequences. Such hybridization might indicate that the protein encoded by the gene to which the probe corresponds is found in humans and therefore not necessarily a good drug target. Alternatively, the gene can be conserved only in bacteria and therefore would be a good drug target for a broad spectrum antibiotic or antimicrobial.

Probes derived from the identified nucleic acid sequences of interest or portions thereof can be labeled with detectable labels familiar to those skilled in the art, including radioisotopes and non-radioactive labels, to provide a detectable probe. The detectable probe can be single stranded or double stranded and can be made using techniques known in the art, including *in vitro* transcription, nick translation, or kinase reactions. A nucleic acid sample containing a sequence capable of hybridizing to the labeled probe is contacted with the labeled probe. If the nucleic acid in the sample is double stranded, it can be denatured prior to contacting the probe, In some applications, the nucleic acid sample can be immobilized on a surface such as a nitrocellulose or nylon membrane. The nucleic acid sample can comprise nucleic acids obtained from a variety of sources, including genomic DNA, cDNA libraries, RNA, or tissue samples.

Procedures used to detect the presence of nucleic acids capable of hybridizing to the detectable probe include well known techniques such as Southern blotting, Northern blotting, dot blotting, colony hybridization, and plaque hybridization. In some applications, the nucleic acid capable of hybridizing to the labeled probe can be cloned into vectors such as expression vectors, sequencing vectors, or in vitro transcription vectors to facilitate the characterization and expression of the hybridizing nucleic acids in the sample. For example, such techniques can be used to isolate, purify and clone sequences from a genomic library, made from a variety of bacterial species, which are capable of hybridizing to probes made from the sequences identified in Examples 5 and 6.

### EXAMPLE 14

### Preparation of PCR Primers and Amplification of DNA

The identified E. coli genes corresponding directly to or located within the operon of nucleic acid sequences required for proliferation or portions thereof can be used to prepare PCR primers for a variety of applications, including the identification or isolation of homologous sequences from other species, for example *S*. *typhimurium, E*. *cloacae, and Klebsiella pneumoniae,* which contain part or all of the homologous genes. Because homologous genes are related but not identical in sequence, those skilled in the art will often employ degenerate sequence PCR primers. Such degenerate sequence primers are designed based on conserved sequence regions, either known or suspected, such as conserved coding regions. The successful production of a PCR product using degenerate probes generated from the sequences identified herein would indicate the presence of a homologous gene sequence in the species being screened. The PCR primers are at least 10 bases, and preferably at least 20 bases in length. More preferably, the PCR primers are at least 20-30 bases in length. In some embodiments, the PCR primers can be more than 30 bases in length. It is preferred that the primer pairs have approximately the same G/C ratio, so that melting temperatures are approximately the same. A variety of PCR techniques are familiar to those skilled in the art. For a review of PCR technology, see Molecular Cloning to Genetic Engineering White, B.A. Ed, in **Methods in Molecular Biology** 67: Humana Press, Totowa 1997. When the entire coding sequence of the target gene is known, the 5' and 3' regions of the target gene can be used as the sequence source for PCR probe generation. In each of these PCR procedures, PCR primers on either side of the nucleic acid sequences to be amplified are added to a suitably prepared nucleic acid sample along with dNTPs and a thermostable polymerase such as Taq polymerase, Pfu polymerase, or Vent polymerase. The nucleic acid in the sample is denatured and the PCR primers are specifically hybridized to complementary nucleic acid sequences in the sample. The hybridized primers are extended. Thereafter, another cycle of denaturation, hybridization, and extension is initiated. The cycles are repeated multiple times to produce an amplified fragment containing the nucleic acid sequence between the primer sites.

### EXAMPLE 15

### Inverse PCR

The technique of inverse polymerase chain reaction can be used to extend the known nucleic acid sequence identified in Examples 5 and 6. The inverse PCR reaction is described generally by Ochman et al., in Ch. 10 of **PCR Technology: Principles and Applications for DNA Amplification**, (Henry A. Erlich, Ed.) W.H. Freeman and Co. (1992). Traditional PCR requires two primers that are used to prime the synthesis of complementary strands of DNA. In inverse PCR, only a core sequence need be known.

Using the sequences identified as relevant from the techniques taught in Examples 5 and 6 and applied to other species of bacteria, a subset of exogenous nucleic sequences are identified that correspond to genes or operons that are required for bacterial proliferation. In species for which a genome sequence is not known, the technique of inverse PCR provides a method for obtaining the gene in order to determine the sequence or to place the probe sequences in full context to the target sequence to which the identified exogenous nucleic acid sequence binds.

To practice this technique, the genome of the target organism is digested with an appropriate restriction enzyme so as to create fragments of nucleic acid that contain the identified sequence as well as unknown sequences that flank the identified sequence. These fragments are then circularized and become the template for the PCR reaction. PCR primers are designed in accordance with the teachings of Example 15 and directed to the ends of the identified sequence are synthesized. The primers direct nucleic acid synthesis away from the known sequence and toward the unknown sequence contained within the circularized template. After the PCR reaction is complete, the resulting PCR products can be sequenced so as to extend the sequence of the identified gene past the core sequence of the identified exogenous nucleic acid sequence identified. In this manner, the full sequence of each novel gene can be identified. Additionally the sequences of adjacent coding and noncoding regions can be identified.

### EXAMPLE 16

### Identification of Genes Required for Staphylococcus aureus Proliferation

Genes required for proliferation in *Staphylococcus aureus* are identified according to the methods described above.

### EXAMPLE 17

### Identification of Genes Reouired for Neisseria gonorrhoeae Proliferation

Genes required for proliferation in *Neisseria gonorrhoeae* are identified according to the methods described above.

### EXAMPLE 18

### Identification of Genes Required for Pseudomonas aeruginosa Proliferation

Genes required for proliferation in *Pseudomonas aeruginosa* are identified according to the methods described above.

### EXAMPLE 19

### Identification of Genes Required for Enterococcus faecalis Proliferation

Genes required for proliferation in *Enterococcus faecalis* are identified according to the methods described above.

### EXAMPLE 20

### Identification of Genes Required for Haemophilus influenzae Proliferation

Genes required for proliferation in *Haemophilus influenzae* are identified according to the methods described above.

### EXAMPLE 21

### Identification of Genes Reouired for Salmonella typhimurium Proliferation

Genes required for proliferation in *Salmonella typhimurium* are identified according to the methods described above.

### EXAMPLE 22

### Identification of Genes Reouired for Helicobacter pylori Proliferation

Genes required for proliferation in *Helicobacter pylori* are identified according to the methods described above.

### EXAMPLE 23

### Identification of Genes Required for Mycoplasma pneumoniae Proliferation

Genes required for proliferation in *Mycoplasma pneumoniae* are identified according to the methods described above.

### EXAMPLE 24

### Identification of Genes Required for Plasmodium ovale Proliferation

Genes required for proliferation in *Plasmodium ovate* are identified according to the methods described above.

### EXAMPLE 25

### Identification of Genes Required for Saccharomyces cerevisiae Proliferation

Genes required for proliferation in *Saccharomyces cerevisiae* are identified according to the methods described above.

### EXAMPLE 26

### Identification of Genes Required for Entamoeba histolytica Proliferation

Genes required for proliferation in *Entamoeba histolytica* are identified according to the methods described above.

### EXAMPLE 27

### Identification of Genes Required for Candida albicans Proliferation

Genes required for proliferation in *Candida albicans* are identified according to the methods described above.

### EXAMPLE 28

### Identification of Genes Reouired forKlebsiella pneumoniae Proliferation

Genes required for proliferation in *KlebsieKlebsiella pneumoniae Proliferationlla pneumoniae* are identified according to the methods described above.

### EXAMPLE 29

### Identification of Genes Reouired for Salmonella typhi Proliferation

Genes required for proliferation in *Salmonella typhi* are identified according to the methods described above.

### EXAMPLE 30

### Identification of Genes Required for Salmonella paratyphi Proliferation

Genes required for proliferation in *Salmonella paratyphi* are identified according to the methods described above.

### EXAMPLE 31

### Identification of Genes Required for Salmonella cholerasuis Proliferation

Genes required for proliferation in *Salmonella cholerasuis* are identified according to the methods described above.

### EXAMPLE 32

### Identification of Genes Required for Staphylococcus epidermis Proliferation

Genes required for proliferation in *Staphylococcus epidermis* are identified according to the methods describ above.

### EXAMPLE 33

### Identification of Genes Required for Mycobacterium tuberculosis Proliferation

Genes required for proliferation in *Mycobacterium tuberculosis* are identified according to the methods describ above.

### EXAMPLE 34

### Identification of Genes Required for Mycobacterium leprae Proliferation

Genes required for proliferation in *Mycobacterium leprae* are identified according to the methods described above.

### EXAMPLE 35

### Identification of Genes Required for Treponema pallidum Proliferation

Genes required for proliferation in *Treponema pallidum* are identified according to the methods described above.

### EXAMPLE 36

### Identification of Genes Required for Bacillus anthracis Proliferation

Genes required for proliferation in *Bacillus anthracis* are identified according to the methods described above.

### EXAMPLE 37

### Identification of Genes Reouired for Yersinia pestis Proliferation

Genes required for proliferation in *Yersinia pestis* are identified according to the methods described above.

### EXAMPLE 38

### Identification of Genes Required for Clostridium botulinum Proliferation

Genes required for proliferation in *Clostridium botulinum* are identified according to the methods described above.

### EXAMPLE 39

### Identification of Genes Required for Campylobacter jejuni Proliferation

Genes required for proliferation in *Campylobacter jejuni* are identified according to the methods described above.

### EXAMPLE 40

### Identification of Genes Required for Chlamydia trachomatis Proliferation

Genes required for proliferation in *Chlamydia trachomatis* are identified according to the methods described above.

### Use of Isolated Exogenous Nucleic Acid Fragments as Antisense Antibiotics

In addition to using the identified sequences to enable screening of molecule libraries to identify compounds useful to identify antibiotics, the sequences themselves can be used as therapeutic agents. Specifically, the identified exogenous sequences in an antisense orientation can be provided to an individual to inhibit the translation of a bacterial target gene.

### Generation of Antisense Therapeutics from Identified Exogenous Sequences

The sequences of the present invention can be used as antisense therapeutics for the treatment of bacterial infections or simply for inhibition of bacterial growth *in vitro* or *in vivo*. The therapy exploits the biological process in cells where genes are transcribed into messenger RNA (mRNA) that is then translated into proteins. Antisense RNA technology contemplates the use of antisense oligonucleotides directed against a target gene that will bind to its target and decrease or inhibit the translation of the target mRNA. In one embodiment, antisense oligonucleotides can be used to treat and control a bacterial infection of a cell culture containing a population of desired cells contaminated with bacteria. In another embodiment, the antisense oligonucleotides can be used to treat an organism with a bacterial infection.

Antisense oligonucleotides can be synthesized from any of the sequences of the present invention using methods well known in the art. In a preferred embodiment, antisense oligonucleotides are synthesized using artificial means. Uhlmann & Peymann, Chemical Rev. 90:543-584 (1990) review antisense oligonucleotide technology in detail. Modified or unmodified antisense oligonucleotides can be used as therapeutic agents. Modified antisense oligonucleotides are preferred since it is well known that antisense oligonucleotides are extremely unstable. Modification of the phosphate backbones of the antisense oligonucleotides can be achieved by substituting the internucleotide phosphate residues with methylphosphonates, phosphorothioates, phosphoramidates, and phosphate esters. Nonphosphate internucleotide analogs such as siloxane bridges, carbonate brides, thioester bridges, as well as many others known in the art. The preparation of certain antisense oligonucleotides with modified internucleotide linkages is described in U.S. Patent No. 5,142,047, hereby incorporated by reference.

Modifications to the nucleoside units of the antisense oligonucleotides are also contemplated. These modifications can increase the half-life and increase cellular rates of uptake for the oligonucleotides *in vivo*. For example, α-anomeric nucleotide units and modified bases such as 1,2-dideoxy-d-ribofuranose, 1,2-dideoxy-1-phenylribofuranose, and *N*⁴,*N*⁴-ethano-5-methyl-cytosine are contemplated for use in the present invention.

An additional form of modified antisense molecules is found in peptide nucleic acids. Peptide nucleic acids (PNA) have been developed to hybridize to single and double stranded nucleic acids. PNA are nucleic acid analogs in which the entire deoxyribose-phosphate backbone has been exchanged with a chemically completely different, but structurally homologous, polyamide (peptide) backbone containing 2-aminoethyl glycine units. Unlike DNA, which is highly negatively charged, the PNA backbone is neutral. Therefore, there is much less repulsive energy between complementary strands in a PNA-DNA hybrid than in the comparable DNA-DNA hybrid, and consequently they are much more stable. PNA can hybridize to DNA in either a Watson/Crick or Hoogsteen fashion (Demidov et al., *Proc. Natl. Acad*. *Sci*. *U.S.A.* **92**:2637-2641, 1995; Egholm, *Nature* **365**:566-568, 1993; Nielsen et al., *Science* **254**:1497-1500, 1991; Dueholm et al., *New J. Chem.* **21**:19.31, 1997).

Molecules called PNA "clamps" have been synthesized which have two identical PNA sequences joined by a flexible hairpin linker containing three 8-amino-3,6-dioxaoctanoic acid units. When a PNA clamp is mixed with a complementary homopurine or homopyrimidine DNA target sequence, a PNA-DNA-PNA triplex hybrid can form which has been shown to be extremely stable (Bentin et al., *Biochemistry* **35**:8863-8869, 1996; Egholm et al., *Nucleic Acids Res.* **23:**217-222, 1995; Griffith et al., *J. Am*. *Chem. Soc.* **117**:831-832, 1995).

The sequence-specific and high affinity duplex and triplex binding of PNA have been extensively described (Nielsen et al., *Science* **254**:1497-1500, 1991; Egholm et al., *J*. *Am*. *Chem. Soc*. **114**:9677-9678, 1992; Egholm et al., *Nature* **365**:566-568, 1993; Almarsson et al., *Proc. Natl. Acad. Sci. U.S.A.* **90**:9542-9546, 1993; Demidov et al., *Proc. Natl. Acad*. *Sci. U.S.A.* **92**:2637-2641, 1995). They have also been shown to be resistant to nuclease and protease digestion (Demidov et al., *Biochem*. *Pharm.* **48**:1010-1313, 1994). PNA has been used to inhibit gene expression (Hanvey at al., *Science* **258**:1481-1485,1992; Nielsen et al., *Nucl. Acids. Res.,* **21**:197-200, 1993; Nielsen et al., *Gene* **149**:139-145, 1994; Good & Nielsen, Science, 95: 2073-2076, 1998; all of which are hereby incorporated by reference), to block restriction enzyme activity (Nielsen et al., *supra.,* 1993), to act as an artificial transcription promoter (Mollegaard, *Proc*. *Natl. Acad. Sci. U.S.A.* **91**:38923895, 1994) and as a pseudo restriction endonuclease (Demidov et al., *Nucl. Acids*. *Res.* **21**:2103-2107, 1993). Recently, PNA has also been shown to have antiviral and antitumoral activity mediated through an antisense mechanism (Norton, *Nature Biotechnol.,* **14**:615-619, 1996; Hirschman et al., *J*. *Investig. Med*. **44**:347-351, 1996). PNAs have been linked to various peptides in order to promote PNA entry into cells (Basu et al., *Bioconj. Chem.* **8**:481-488, 1997; Pardridge et al., *Proc*. *Natl. Acad. Sci. U.S.A.* **92**:5592-5596, 1995).

The antisense oligonucleotides contemplated by the present invention can be administered by direct application of oligonucleotides to a target using standard techniques well known in the art. The antisense oligonucleotides can be generated within the target using a plasmid, or a phage. Alternatively, the antisense nucleic acid may be expressed from a sequence in the chromosome of the target cell. It is further contemplated that contemplated that the antisense oligonucleotide contemplated are incorporated in a ribozyme sequence to enable the antisense to specifically bind and cleave its target mRNA. For technical applications of ribozyme and antisense oligonucleotides see Rossi et al., **Pharmacol. Ther. 50(2)**:245-254, (1991), which is hereby incorporated by reference. The present invention also contemplates using a retron to introduce an antisense oligonucleotide to a cell. Retron technology is exemplified by U.S. Patent No. 5,405,775, which is hereby incorporated by reference. Antisense oligonucleotides can also be delivered using liposomes or by electroporation techniques which are well known in the art.

The antisense nucleic acids of the present invention can also be used to design antibiotic compounds comprising nucleic acids which function by intracellular triple helix formation. Triple helix oligonucleotides are used to inhibit transcription from a genome. The sequences identified as required for proliferation in the present invention, or portions thereof, can be used as templates to inhibit microorganism gene expression in individuals infected with such organisms. Traditionally, homopurine sequences were considered the most useful for triple helix strategies. However, homopyrimidine sequences can also inhibit gene expression. Such homopyrimidine oligonucleotides bind to the major groove at homopurine:homopyrimidine sequences. Thus, both types of sequences based on the sequences of the present invention that are required for proliferation are contemplated for use as antibiotic compound templates.

The antisense oligonucleotides of this example employ the identified sequences of the present invention to induce bacterial cell death or at least bacterial stasis by inhibiting target gene translation. Antisense oligonucleotides containing from about 8 to 40 bases of the sequences of the present invention have sufficient complementary to form a duplex with the target sequence under physiological conditions.

To kill bacterial cells or inhibit their growth, the antisense oligonucleotides are applied to the bacteria or to the target cells under conditions that facilitate their uptake. These conditions include sufficient incubation times of cells and oligonucleotides so that the antisense oligonucleotides are taken up by the cells. In one embodiment, an incubation period of 7-10 days is sufficient to kill bacteria in a sample. An optimum concentration of antisense oligonucleotides is selected for use.

The concentration of antisense oligonucleotides to be used can vary depending on the type of bacteria sought to be controlled, the nature of the antisense oligonucleotide to be used, and the relative toxicity of the antisense oligonucleotide to the desired cells in the treated culture. Antisense oligonucleotides can be introduced to cell samples at a number of different concentrations preferably between 1x10⁻¹⁰M to 1x10⁻⁴M. Once the minimum concentration that can adequately control gene expression is identified, the optimized dose is translated into a dosage suitable for use *in vivo*. For example, an inhibiting concentration in culture of 1x10⁻⁷ translates into a dose of approximately 0.6 mg/kg body weight. Levels of oligonucleotide approaching 100 mglkg body weight or higher may be possible after testing the toxicity of the oligonucleotide in laboratory animals. It is additionally contemplated that cells from the subject are removed, treated with the antisense oligonucleotide, and reintroduced into the subject. This range is merely illustrative and one of skill in the art are able to determine the optimal concentration to be used in a given case.

After the bacterial cells have been killed or controlled in a desired culture, the desired cell population may be used for other purposes.

### EXAMPLE 41

The following example demonstrates the ability of an *E*. *coli* antisense oligonucleotide to act as a bactericidal or bacteriostatic agent to treat a contaminated cell culture system. The application of the antisense oligonucleotides of the present invention are thought to inhibit the translation of bacterial gene products required for proliferation.

The antisense oligonucleotide of this example corresponds to a 30 base phophorothioate modified oligodeoxynucelotide complementary to a nucleic acid involved in proliferation, such as Molecule Number EcXA001. A sense oligodeoxynucelotide complementary to the antisense sequence is synthesized and used as a control. The oligonucleotides are synthesized and purified according to the procedures of Matsukura, et al., Gene 72:343 (1988). The test oligonucleotides are dissolved in a small volume of autoclaved water and added to culture medium to make a 100 micromolar stock solution.

Human bone marrow cells are obtained from the peripheral blood of two patients and cultured according standard procedures well known in the art. The culture is contaminated with the K-12 strain of *E. coli* and incubated at 37°C overnight to establish bacterial infection.

The control and antisense oligonucleotide containing solutions are added to the contaminated cultures and monitored for bacterial growth. After a 10 hour incubation of culture and oligonucleotides, samples from the control and experimental cultures are drawn and analyzed for the translation of the target bacterial gene using standard microbiological techniques well known in the art. The target *E*. *coli* gene is found to be translated in the control culture treated with the control oligonucleotide, however, translation of the target gene in the experimental culture treated with the antisense oligonucleotide of the present invention is not detected or reduced.

### EXAMPLE 42

A subject suffering from an *E*. *coli* infection is treated with the antisense oligonucleotide preparation of Example 39. The antisense oligonucleotide is provided in a pharmaceutically acceptable carrier at a concentration effective to inhibit the translation of the target gene. The present subject is treated with a concentration of antisense oligonucleotide sufficient to achieve a blood concentration of about 100 micromolar. The patient receives daily injections of antisense oligonucleotide to maintain this concentration for a period of 1 week. At the end of the week a blood sample is drawn and analyzed for the presence or absence using standard techniques well known in the art. There is no detectable evidence of E. coli and the treatment is terminated.

### EXAMPLE 43

### Preparation and use of Triple Helix Probes

The sequences of microorganism genes required for proliferation of the present invention are scanned to identify 10-mer to 20-mer homopyrimidine or homopurine stretches that could be used in triple-helix based strategies for inhibiting gene expression. Following identification of candidate homopyrimidine or homopurine stretches, their efficiency in inhibiting gene expression is assessed by introducing varying amounts of oligonucleotides containing the candidate sequences into a population of bacterial cells that normally express the target gene. The oligonucleotides may be prepared on an oligonucleotide synthesizer or they may be purchased commercially from a company specializing in custom oligonucleotide synthesis, such as GENSET, Paris, France.

The oligonucleotides can be introduced into the cells using a variety of methods known to those skilled in the art, including but not limited to calcium phosphate precipitation, DEAE-Dextran, electroporation, liposome-mediated transfection or native uptake.

Treated cells are monitored for a reduction in proliferation using techniques such as monitoring growth levels as compared to untreated cells using optical density measurements. The oligonucleotides that are effective in inhibiting gene expression in cultured cells can then be introduced in *vivo* using the techniques well known in that art at a dosage level shown to be effective.

In some embodiments, the natural (beta) anomers of the oligonucleotide units can be replaced with alpha anomers to render the oligonucleotide more resistant to nucleases. Further, an intercalating agent such as ethidium bromide, or the like, can be attached to the 3 end of the alpha oligonucleotide to stabilize the triple helix. For information on the generation of oligonucleotides suitable for triple helix formation see Griffin et al. (**Science 245**:967-971 (1989), which is hereby incorporated by this reference).

### EXAMPLE 44

### Identification of Bacterial Strains from Isolated Specimens by PCR

Classical bacteriological methods for the detection of various bacterial species are time consuming and costly. These methods include growing the bacteria isolated from a subject in specialized media, cultivation on selective agar media, followed by a set of confirmation assays that can take from 8 to 10 days or longer to complete. Use of the identified sequences of the present invention provides a method to dramatically reduce the time necessary to detect and identify specific bacterial species present in a sample.

In one exemplary method, bacteria are grown in enriched media and DNA samples are isolated from specimens of, for example, blood, urine, stool, saliva or central nervous system fluid by conventional methods. A panel of PCR primers based on identified sequences unique to various species of microorganisms are then utilized in accordance with Example 12 to amplify DNA of approximately 100-200 bases in length from the specimen. A separate PCR reaction is set up for each pair of PCR primers and after the PCR reaction is complete, the reaction mixtures are assayed for the presence of PCR product. The presence or absence of bacteria from the species to which the PCR primer pairs belong is determined by the presence or absence of a PCR product in the various test PCR reaction tubes.

Although the PCR reaction is used to assay the isolated sample for the presence of various bacterial species, other assays such as the Southern blot hybridization are also contemplated.

## Claims

1. A purified or isolated nucleic acid consisting essentially of the nucleotide sequence of SEQ ID NO:405, wherein said nucleic acid inhibits microorganism proliferation.

2. The nucleic acid sequence of Claim 1, wherein said nucleic acid is complementary to at least a portion of a coding sequence of a gene whose expression is required for microorganism proliferation.

3. A fragment of comprising at least 10, at least 20, at least 25, at least 30, at least 50 or more than 50 consecutive bases of the nucleotide sequence of SEQ ID NO: 405.

4. The fragment of Claim 3, wherein said fragment is complementary to a coding sequence of a gene whose expression is required for microorganism proliferation.

5. A vector comprising a promoter operably linked to the nucleotide sequence of SEQ ID NO: 405.

6. The vector of Claim 5, wherein said promoter is active in an organism selected from the group consisting of *Escherichia coli, Staphylococcus aureus, Pseudomonas aeruginosa, Enterobacter cloacae, Helicobacter pylori, Neisseria gonorrhoeae, Enterococcus faecalis, Streptococcus pneumoniae, Haemophilus influenzae, Salmonella typhimurium, Saccharomyces cerevisiae, Candida albicans, Cryptococcus neoformans, Aspergillus fumigatus, Klebsiella pneumoniae, Salmonella typhi, Salmonella paratyphi, Salmonella cholerasuis, Staphylococcus epidermidis, Mycohacterium tuberculosis, Mycobacterium leprae, Treponema pallidum, Bacillus anthracis, Yersinia pestis, Clostridium botulinum, campylobacter jejuni, Chlamydia trachomatus, Chlamydia pneumoniae* or any species falling within the genera of any of the above species.

7. A host cell containing the vector of Claim 5 or Claim 6.

8. A purified or isolated nucleic acid comprising a nucleic acid having at least 70% homology to the nucleotide sequence of SEQ ID NO: 405 or the nucleotide sequence complementary thereto as determined using BLASTN version 2.0 with the default parameters.

9. The nucleic arid of Claim 8, wherein said nucleic acid is from an organism selected from the group consisting of *Staphylococcus aureus, Pseudomonas aeruginosa, Enterobacter cloacae, Helicobacter pylori, Neisseria gonorrhoeae, Enterococcus faecalis, Streptococcus pneumoniae, Haemophilus influenzae, Salmonella typhimurium, Saccharomyces cerevisiae, Candida albicans, Cryptococcus neoformans, Aspergillus fumigatus, Klebsiella pneumoniae, Salmonella typhi, Salmonella paratyphi, Salmonella cholerasuis, Staphylococcus epidermidis, Mycobacterium tuberculosis, Mycobacterium leprae, Treponema pallidum, Bacillus anthracis, Yersinia pestis, Clostridium botulinum, campylobacter jejuni, and Chlamydia trachomatus, Chlamydia pneumoniae* or any species falling within the genera of any of the above species.

10. A purified or isolated nucleic acid consisting essentially of the coding sequence of SEQ ID NO: 82.

11. A fragment of the nucleic acid of Claim 10, said fragment comprising at least 10, at least 20, at least 25, at least 30, at least 50 or more than 50 consecutive nucleotides of the nucleotide sequence of SEQ ID NO: 82.

12. A vector comprising a promoter operably linked to the nucleic acid of Claim 10 or Claim 11.

13. A purified or isolated nucleic acid comprising a nucleotide sequence complementary to at least a portion of an intragenic sequence, intergenic sequence, sequences spanning at least a portion of two or more genes, 5' noncoding region, or 3' noncoding region within an operon encoding a polypeptide comprising the amino acid sequence of SEQ ID NO: 243.

14. A purified or isolated nucleic acid consisting essentially of a nucleic acid encoding a polypeptide comprising the amino acid sequence of SEQ ID NO: 243.

15. A vector comprising a promoter operably linked to a nucleic acid encoding a polypeptide comprising the amino acid sequence of SEQ ID NO: 243.

16. A host cell containing the vector of Claim 15.

17. A purified or isolated polypeptide comprising the amino acid sequence of SEQ ID NO: 243.

18. A purified or isolated polypeptide comprising a fragment of the amino acid sequence of SEQ ID NO: 243, said fragment selected from the group consisting of fragments comprising at least 5, at least 10, at least 20, at least 30, at least 40, at least 50, at least 60 or more than 60 consecutive amino acids of the amino acid sequence of SEQ ID NO: 243.

19. An antibody capable of specifically binding the polypsptide of Claim 17 or Claim 18.

20. A method of producing a polypeptide, comprising introducing a vector comprising a promoter operably linked to a nucleic acid encoding a polypeptide having the amino acid sequence of SEQ ID NO: 243 into a cell.

21. The method of Claim 20, further comprising the step of isolating said protein.

22. A method of inhibiting proliferation comprising inhibiting the activity or reducing the amount of a polypeptide comprising the amino acid sequence of SEQ ID NO: 243 or inhibiting the activity or reducing the amount of a nucleic acid encoding said polypeptide.

23. A method for identifying compounds which influence the activity of a polypeptide required for proliferation comprising:
contacting a polypeptide comprising the amino acid-sequence of SEQ ID NO: 243 with a candidate compound; and
determining whether said compound influences the activity of said polypeptide.

24. The method of Claim 23, wherein said activity is an enzymatic activity.

25. The method of Claim 23, wherein said activity is a carbon compound catabolism activity.

26. The method of Claim 23, wherein said activity is a biosynthetic activity.

27. The method of Claim 23, wherein said activity is a transporter activity,

28. The method of Claim 23, wherein said activity is a transcriptional activity,

29. The method of Claim 23, wherein said activity is a DNA replication activity.

30. The method of Claim 23, wherein said activity is a cell division activity.

31. A method for assaying compounds for the ability to reduce the activity or level of a polypeptide required for proliferation, comprising:
providing a target, wherein said target comprises the coding sequence of SEQ ID NO: 82;
contacting said target with a candidate compound; and
measuring an activity of said target.

32. The method of Claim 31, wherein said terget is a messenger RNA molecule transcribed from a coding region comprising the nucleotide sequence of SEQ ID. NOs,: B2 and said activity is translation of said messenger RNA.

33. The method of Claim 31, wherein said target is the coding region comprising the nucleotide sequence of SEQ ID. NO: 82 and said activity is transcription of said messenger RNA.

34. A compound identified using the method of Claim 31.

35. A method for identifying compounds which reduce the activity or level of a gene product required for cell proliferation comprising the steps of:
expressing an antisense nucleic acid against a nucleic acid encoding a gene product comprising the amino acid sequence of SEQ ID NO: 243 in a cell to reduce the activity or amount of said gene product in said cell, thereby producing a sensitized cell;
contacting said sensitized cell with a compound; and
determining whether said compound inhibits the growth of said sensitized cell to a greater extent than said compound inhibits the growth of a nonsensitized cell.

36. The method of Claim 35, wherein said cell is selected from the group consisting of bacterial cells, fungal cells, plant cells, and animal cells.

37. The method of Claim 36 wherein said cell is an *E*. *coli* cell.

38. The method of Claim 38, wherein said cell is from an organism selected from the group consisting of *Staphylococcus aureus, Pseudomonas aeruginosa, Enterobacter cloacae, Helicobacter pylori, Neisseria gonorrhoeae, Enterococcus faecalis, Streptococcus pneumoniae, Haemophilus influenzae, Salmonella typhimurium, Saccharomyces cerevisiae, Candida albicans, Cryptococcus neoformans, Aspergillus fumigatus, Klebsiella pneumoniae, Salmonella typi, Salmonella paratyphi, Salmonella cholerasuis, Staphylococcus epidermidis, Mycobacterium tuberculosis, Mycobacterium leprae, Treponema pallidum, Bacillus anthracis, Yersinia pestis, Clostridium botulinum, campylobacter jejuni, and Chlamydia trachomatus, Chlamydia pneumoniae* or any species falling within the genera of any of the above species.

39. The method of Claim 35, wherein said antisense nucleic acid is transcribed from an inducible promoter.

40. The method of Claim 39, further comprising the step of contacting said cell with a concentration of inducer which induces said antisense nucleic acid to a sublethal level.

41. The method of Claim 40, wherein said sub-lethal concentration of said inducer is such that growth inhibition is 8% or more.

42. The method of Claim 40, wherein said inducer is isopropyl-1-thio-β-D-galactoside.

43. The method of Claim 35, wherein growth inhibition Is measured by monitoring optical density of a culture growth solution.

44. A compound identified using the method of Claim 35.

45. A method for inhibiting cellular proliferation comprising introducing a compound with activity against a gene comprising the nucleotide sequence of SEQ ID NO: 82 or with activity against the product of said gene into a population of cells expressing a gene.

46. The method of Claim 45, wherein said compound is an antisense nucleic acid comprising the nucleotide sequence of SEQ ID NO: 405 or a proliferation-inhibiting portion thereof.

47. The method of Claim 46, wherein said proliferation inhibiting portion of the nucleotide sequence of SEQ ID NO: 405 is a fragment comprising at least 10, at least 20, at least 25, at least 30, at least 50 or more than 50 consecutive nucleotides of SEQ ID NO; 405.

48. The method of Claim 45 wherein said compound is a triple helix oligonucleotide.

49. A preparation comprising an effective concentration of an antisense nucleic acid comprising the nucleotide sequence of SEQ ID ND: 405 or a proliferation-inhibiting portion thereof in a pharmaceutically acceptable carrier.

50. The preparation of Claim 49, wherein said proliferation-inhibiting portion of SEQ ID NO: 405 comprises at least 10, at least 20, at least 25, at least 30, at least 50 or more than 50 consecutive nucleotides of SEQ ID NO: 405.

51. A method for inhibiting the expression of a gene In an opeton required for proliferation comprising contacting a cell in a cell population with an antisense nucleic acid, said cell expressing a gene comprising the nucleotide sequence of SEQ ID NOs: 82, wherein said antisense nuclelc add comprises at least a proliferation-inhibiting portion of said operon in an antisense orientation that is effective in inhibiting expression of said gene.

52. The method of Claim 51, wherein said antisense nucleic acid comprises the nucleotide sequence of SEQ ID ND: 405, or a portion thereof.

53. The method of Claim 51, wherein said cell is contacted with said antisense nucleic acid by introducing a plasmid which expresses said antisense nucleic acid into said cell population.

54. The mathod of Claim 51, wherein said cell is contacted with said antisense nucleic acid by introducing a phage which expresses said antisense nucleic acid into said cell population.

55. The method of Claim 51, wherein said cell is contacted with said antisense nucleic acid by introducing a nucleotide sequence encoding said antisense nucleic acid into the chromosome of said cell in said cell population.

56. The method of Claim 51, wherein said cell is contacted with said antisense nucleic acid by introducing a retron which expresses said antisense nucleic acid into said cell population.

57. The method of Claim 51, wherein said cell is contacted with said antisense nucleic acid by introducing a ribozyme into said cell-population, wherein a binding portion of said ribozyme is complementary to said antisense nucleic acid.

58. The method of Claim 51, wherein said cell is contacted with said antisense nucleic acid by introducing a liposome comprising said antisense nucleic acid into said cell.

59. The method of Claim 51, wherein said cell is contacted with said antisense nucleic acid by electroporation.

60. The method of Claim 51, wherein said antisense nucleic acid is a fragment comprising at least 10, at least 20, at least 25, at least 30, at least 50 or more than 50 consecutive nucleotides of the nucleotide sequence of SEQ ID ND: 405.

61. The method of Claim 51 wherein said antisense nucleic acid is an oligonuclentide.

62. A method for Identifying bacterial strains comprising the steps of:
providing a sample containing a bacterial species; and
identifying a bacterial species using a species specific probe comprising the nucleotide sequence of SEQ ID NO: 405.

63. A method for identifying a gene in a microorganism required for proliferation comprising:
(a) identifying an inhibitory nucleic acid which inhibits the activity of a gene or gene product required for proliferation in a first microorganism, wherein said gene comprises the nucleotide sequence of SEQ ID NO; 82 and said gene product comprises the polypeptide sequence of SEQ ID NO: 243;
(b) contacting a second microorganism with said inhibitory nucleic acid;
(c) determining whether said inhibitory nucleic acid from said first microorganism inhibits proliferation of said second microorganism; and
(d) identifying the gene in said second microorganism which is inhibited by said inhibitory nucleic acid.

64. A method for assaying a compound for the ability to inhibit proliferation of a microorganism comprising:
(a) identifying a homolog of the nucleotide sequence of SEQ ID NO: 82 or the amino acid sequence of SEQ ID NO: 243 in a microorganism other than *Escherichia coli*;
(b) identifying an inhibitory nucleic acid sequence which inhibits the activity of said homolog in said second microorgansim;
(c) contacting said second microorganism with a proliferation-inhibiting amount of said inhibitory nucleic acid, thus sensitizing said second microorganism;
(d) contacting the sensitized microorganism of step (c) with a compound; and
(e) determining whether said compound inhibits proliferation of said sensitized microorganism to a greater extent than sald compound inhibits proliferation of a nonsensitized microorganism.

65. The method of Claim 64, wherein said step of identifying a homolog of said gene in a second microorganism comprises identifying a homologous nucleic acid or a nucleic acid encoding a homologous polypeptide in a database using an algorithm selected from the group consisting of BLASTN version 2.0 with the default paramaters and FASTA version 3.0t78 algorithm with the default parameters.

66. The method of Claim 64, wherein said step of identifying a homolog of said gene in a second microorganism comprises identlfying a homologous nucleic acid or a nucleic acid encoding a homologous polypeptide by identifying nucleic aclds which hybridize to said first gene.

67. The method of Claim 64, wherein the step of identifying a homolog of said gene in a second microorganism comprises expressing a nucleic add which inhibits the proliferation of said first microorganism in said second microorganlsm.

68. The method of Claim 64, wherein said Inhibitory nucleic acid is an antisense nucleic acid,

69. The method of Claim 64, wherein said inhibitory nucleic acid comprises an antisense nucleic acid to a portion of said homolog.

70. The method of Claim 64, wherein said inhibitory nucleic acid comprises an antisense nucleic acid to a portion of the operon encoding said homolog.

71. The method of Claim 64, wherein the step of contacting the second microorganism with a proliferation-inhibiting amount of said nucleic acid sequence comprises directly contacting said second microorganism with said nucleic acid.

72. The method of Claim 64, wherein the step of contacting the second microorganism with a proliferation-inhibiting amount of said nucleic acid sequence comprises expressing an antisensa nucleic acid to said homolog in said second microorganism.

73. A compound identified using the method of Claim 64.

74. A method of assaying a compound for the ability to inhibit proliferation comprising:
(a) identifying an inhibitory nucleic acid sequence which inhibits the activity of a gene or gene product wherein said gene comprises the nucleotide sequence of SEQ ID NO: 82 and said gene product comprises the amino acid sequence of SEQ ID NO: 243;
(b) contacting a microorganism other than *Escherichia coli* with a proliferation-inhibiting amount of said inhibitory nucleic acid, thus sensitizing said microorganism;
(c) contacting the proliferation-inhibited microorganism of step (b) with a compound; and
(d) determining whether said compound inhibits proliferation of said sensitized microorganism to a greater extent than said compound inhibits proliferation of a nonsensitized microorganism.

75. The method of Claim 74, wherein said inhibitory nucleic acid is an antisense nucleic acid which inhibits the proliferation of *Escherichia coli.*

76. The method of Claim 74, wherein said inhibitory nucleic acid comprises a portion of an antisense nucleic acid which inhibits the proliferation of *Escherichia coli*.

77. The method of Claim 74, wherein said inhibitory nucleic acid comprises an antisense molecule against the entire coding region of the nucleotide sequence of SEQ 10 NO: 82.

78. The method of Claim 74, wherein said inhibitory nucleic acid comprises an antisense nucleic acid to a portion of the operon comprising the nucleotide sequence of SEQ ID NO: 82.

79. A compound identified using the method of Claim 74.

80. A method far assaying compounds for activity against a biological pathway required for proliferation comprising:
sensitizing a cell by expressing an antlsense nucleic add complementary to the nucleotide sequence of SEQ ID NO: 82 or a portion thereof which inhibits the proliferation of said cell;
contacting the sensitized cell with a compound; and
determining whether said compound inhibits the growth of said sensitized cell to a greater extent than said compound Inhibits the growth of an nonsensitized cell.

81. The method of Claim 80, wherein said cell is selected from the group consisting of bacterial cells, fungal cells, plant cells, and animal cells.

82. The method of Claim 80, wherein said cell is an *E*. *coli* cell.

83. The method of Claim 80, wherein said cell is from an organism selected from the group consisting of *Staphylococcus aureus, Pseudomonas aeruginosa, Enterobacter cloacae, Helicobacter pylori, Neisseria gonorrhoeae, Enterococcus faecalis, Streptococcus pneumoniae, Haemophilus influenzae, Salmonella typhimurium, Saccharomyces cerevisiae, Candida albicans, Cryptococcus neoformans, Aspergillus fumigatus*, *Klebsiella pneumoniae, Salmonella typhi, Salmonella paratyphi, Salmonella cholerasuis, Staphylococcus epidermidis, Mycobacterium tuberculosis, Mycobacterium leprae, Treponema pallidum, Bacillus anthracis, Yersinia pestis, Clostridium botulinum, campylobacter jejuni, and Chlamydie trachomatus, Chlamydia pneumoniae* or any species falling within the genera of any of the above species.

84. The method of Claim 80, wherein said antisense nucleic acid is transcribed from an inducible promoter.

85. The method of Claim 84, further comprising contacting the cell with an agent which induces expression of said antisense nucleic acid from said inducible promoter, wherein said antisense nucleic acid is expressed at a sublethal level.

86. The method of Claim 85, wherein said sublethal level of said antisense nucleic acid inhibits proliferation by 8% or more.

87. The method of Claim 85, wherein said agent is isopropyl-1-thio-β-D-galactoside (IPTG).

88. The method of Claim 86, wherein inhibition of proliferation is measured by monitoring the optical density of a liquid culture.

89. The method of Claim 80, wherein said anitsense nucleic acid reduces the level or activity of a polypeptide comprising the amino acid sequence of SEQ ID NO: 243 in said cell.

90. A compound identified using the method of Claim 80.

91. A method for assaying a compound for the ability to inhibit cellular proliferation comprising:
contacting a cell with an agent which reduces the activity or level of a polypeptide comprsing the amino acid sequence of SEQ ID NO: 243;
contacting said cell with said compound; and
determining whether said compound reduces proliferation to a greater extent than said compound reduces proliferation of cells which have not been contacted with said agent.

92. The method of Claim 91, wherein said agent comprises an antisense nucleic acid.

93. The method of Claim 91, wherein said agent comprises an antibiotic.

94. The method of Claim 91, wherein said cell contains a temperature sensitive mutation which raduces the activity or level of a polypeptide comprising the amino acid sequence of SEQ ID NO: 243.

95. The method of Claim 93, further comprising contacting said cell with an antisense nucleic acid, wherein said antisense nucleic acid is directed against the nucleic acid encoding the same functional domaln of the polypeptide of SEQ ID NO: 243 as the functional domain on which said antibiotic acts.

96. The method of Claim 93, further comprising contacting said cell with an antisense nucleic acid, wherein said antisense nucleic acid is directed against the nucleic acid encoding the same functional domain of the polypeptide of SEQ ID NO: 243 as the functional domain on which said antibiotic acts.

97. A compound identified using the method of Claim 91.

98. A method for identifying the pathway in which a polypeptide comprising the amino acid sequence of SED ID NO: 243 lies:
expressing a sublethal level of an antisense nucleic acid directed against a nucleic acid encoding said palypeptide in a cell:
contacting said cell with an antibiotic, wherein the biological pathway on which said antibiotic acts is known; and
determining whether said cell has a substantially greater sensitivity to said antibiotic than a cell which does not express said sublethal level of said antisense nucleic acid.

99. A method for determining the pathway on which a test compound acts comprising;
(a) identifying the biological pathway in which a polypeptide comprising the amino acid sequence of SEQ ID NO: 243 is involved;
(b) expressing a sublethal level of an antisense nucleic acid directed against a nucleic acid encoding said polypeptide,
(c) contacting said cell with said test compound; and
(d) determining whether said cell has a substantially greater sensitivity to said test compound than a cell which does not express said sublethal level of said antisense nucleic acid.

100. The method of Claim 99, further comprising;
(e) expressing a sublethal level of a second antisense nucleic acid directed against a second proliferation-required nucleic acid in a second cell, wherein said second proliferation-required nucleic acid is in a different biological pathway than the polypeptide of step (a); and
(f) determining whether said cell has a substantially greater sensitivity to said test compound than a cell which does not express said sublethal level of said second antisense nucleic acid.

101. A compound identified using the method of Claim 23.

102. A compound which interacts with a nucleic acid comprising the nucleotide sequence of SEQ ID NO: 82 to inhibit proliferation.

103. A compound which interacts with a polypeptide comprising the amino acid sequence of SEQ ID NO: 243 to inhibit proliferation.
